# EUROPEAN PATENT SPECIFICATION

(11) **EP 0 610 334 B1**
(45) Date of publication and mention of the grant of the patent: **24.01.1996**
(21) Application number: 92922468.1
(22) Date of filing: 15.10.1992
(51) Int. Cl.: C07D 243/14, A61K 31/55, C07D 223/16, C07D 267/14

(54) **NONPEPTIDYL INTEGRIN INHIBITORS HAVING SPECIFICITY FOR THE GPII B III A RECEPTOR**
NICHTPEPTIDYL INTEGRIN INHIBITOREN MIT SPEZIFITÄT FÜR DEN GPIIB IIIA REZEPTOR
INHIBITEURS NON PEPTIDYLES A BASE D'INTEGRINE SPECIFIQUES DU RECEPTEUR GPII B III A

(30) Priority: 18.10.1991 US 781477; 10.04.1992 US 866931
(43) Date of publication of application: 17.08.1994
(73) Proprietor: GENENTECH, INC., South San Francisco, CA 94080-4990 (US)
(72) Inventor: BLACKBURN, Brent, San Francisco, CA 94124 (US); BARKER, Peter, El Granada, CA 94018 (US); GADEK, Thomas, Oakland, CA 94611 (US); McDOWELL, Robert, San Francisco, CA 94124 (US); McGEE, Lawrence, Pacifica, CA 94044 (US); SOMERS, Todd, Montara, CA 94037 (US); WEBB, Rob, Moss Beach, CA 94035 (US); ROBARGE, Kirk, San Francisco, CA 94117 (US)
(74) Representative: Armitage, Ian Michael
(86) International application number: PCT/US92/08788
(87) International publication number: WO 93/08174

(56) References cited:
- EP-A- 0 268 148
- WO-A-91/09024

## Description

### FIELD OF THE INVENTION

The present invention relates to nonpeptidyl inhibitors of integrin receptors. Specifically, the invention is directed to antagonists of the final common pathway of platelet aggregation. These antagonists act as potent antithrombotics. The invention further relates to therapeutic applications of these nonpeptidyl inhibitors in diseases for which blocking platelet aggregation is indicated.

### BACKGROUND OF THE INVENTION

### A. Platelets

Platelets are particles found in whole blood that initiate and provide the structural basis for the hemostatic plug necessary to stop bleeding. Platelets depend on adhesive interactions with extracellular proteins and other cells for proper function (see Hawiger, J. *Atherosclerosis Reviews* 21:165-186 (1990) and Roth J. R. *Immunology Today* 13(2):100-105 (1992)). The external platelet plasma membrane surface is covered with a variety of membrane bound glycoproteins, many of which have recognition and adhesive functions. Perhaps the most abundant platelet membrane adhesive proteins belong to the integrin superfamily which include the glycoproteins; GPII_{b}IIIₐ, GPIₐIIₐ, GPI_{c}IIₐ, GPl_{b}lX, and the fibronectin and vitronectin receptors (Hynes, R. O., *Cell,* 48: 549 (1987). Each integrin receptor is an αβ heterodimer displaying characteristic affinity and specificity toward various protein ligands found in serum and /or the extracellular matrix including; von Willebrand factor (vWF), collagen, entactin, tenascin, fibronectin (Fn), vitronectin (Vn), and laminin, as well as fibrinogen (Fg) and thrombospondin (see Kieffer *et al*., *Ann. Rev. Cell Biol.* 6:329-357(1990) and Ruoslahti, *J*. *Clin. Invest.,* 87:1-5 (1991)). The most abundant integrin found on the surface of normal platelets is GPII_{b}IIIₐ comprising about 50,000 molecules per platelet and representing about 2% of the total platelet protein. GPII_{b}IIIₐ is a non-covalent, calcium ion dependent heterodimeric complex (Jennings, *et al., J. Biol. Chem.* 257: 10458 (1982)) that is restricted in distribution to platelets and other cells of the megakaryocytic lineage (Kieffer *et al*., *supra ).* On activated platelets, GPII_{b}IIIₐ promiscuously binds a number of protein ligands with varying affinities, including; fibrinogen, fibronectin, von Willebrand factor, vitronectin and thrombospondin (Plow *et al*. , *Biochemistry of Platelets,* Phillips and Shuman eds., p. 225-256, Orlando: Academic Press [1986]). Each of these protein ligands contain at least one tripeptide sequence Arg-Gly-Asp (RGD) which is commonly referred to as the "recognition sequence". It is believed the most important interactions mediating platelet aggregation involve GPII_{b}IIIₐ binding with the trinodular fibrinogen and, to a lesser extent, with the filamentous von Willebrand factor (Kieffer *et al*. , *supra* and Albeda *et al*., *The FASEB Journal*, 4:2868-2880 [1990]).

GPII_{b}IIIₐ binding to its natural ligands can be inhibited to varying degrees by peptides and proteins containing the amino acid recognition sequences; Arg-Gly-Asp (Ruoslahti, *supra* and EPO 368 486, assigned to Merck & Co.)_{,} Lys-Gly-Asp (KGD), and the fibrinogen γ-chain carboxy-terminal dodecapeptide HHLGGAKQAGDV and analogues thereof (Timmons *et al., Biochemistry* , 28:2919-2922 [1989]).

### B. The Hyperthrombotic State

Many common human disorders are characteristically associated with a hyperthrombotic state leading to intravascular thrombi and emboli. These are a major cause of medical morbidity, leading to infarction, stroke and phlebitis, and of mortality from stroke and pulmonary and cardiac emboli. Patients with atherosclerosis are predisposed to arterial thromboembolic phenomena for a variety of reasons. Atherosclerotic plaques form niduses or platelet plugs and thrombi that lead to vascular narrowing and occlusion, resulting in myocardial and cerebral ischemic disease. This may happen spontaneously or following procedures such as angioplasty or endarterectomy. Thrombi that break off and are released into the circulation cause infarction of different organs, especially the brain, extremities, heart and kidneys.

In addition to being involved in arterial thrombosis, platelets may also play a role in venous thrombosis. A large percentage of such patients have no antecedent risk factors and develop venous thrombophlebitis and subsequent pulmonary emboli without a known cause. Other patients who form venous thrombi have underlying diseases known to predispose them to these syndromes. Some of these patients may have genetic or acquired deficiencies of factors that normally prevent hypercoagulability, such as antithrombin-3. Others have mechanical obstructions to venous flow, such as tumor masses, that lead to low flow states and thrombosis. Patients with malignancy have a high incidence of thrombotic phenomena for unclear reasons. Antithrombotic therapy in this situation with currently available agents is dangerous and often ineffective.

Patients whose blood flows over artificial surfaces, such as prosthetic synthetic cardiac valves or through extracorporeal perfusion devices, are also at risk for the development of platelet plugs, thrombi and emboli. It is standard practice that patients with artificial cardiac valves be treated chronically with anti-coagulants. However, in all instances, platelet activation and emboli formation may still occur despite adequate anticoagulation treatment.

Thus, a large category of patients, including those with atherosclerosis, coronary artery disease, artificial heart valves, cancer, and a history of stroke, phlebitis, or pulmonary emboli, are candidates for limited or chronic antithrombotic therapy. The number of available therapeutic agents is limited and these, for the most part, act by inhibiting or reducing levels of circulating clotting factors. These agents are frequently not effective against the patient's underlying hematologic problem, which often concerns an increased propensity for platelet aggregation and adhesion. They also cause the patient to be susceptible to abnormal bleeding. Available antiplatelet agents, such as aspirin, inhibit only part of the platelet activation process and are therefore often inadequate for therapy and also cause the patient to be susceptible to abnormal bleeding. An ideal anti-thrombotic drug would have many properties currently not available (see e.g. Sixma, *et al*. *Thrombosis Research* 67: 305-311 [1992]).

### C. Therapeutic Agents

An agent which effectively inhibits the final common pathway of platelet activation, namely fibrinogen binding to the GP II_{b}IIIₐ receptor, should accordingly be useful in a large group of disorders characterized by a hyperthrombotic state as described above.

Such agents include anti-thrombotic peptides and pseudopeptides capable of inhibiting platelet aggregation. Ruoslahti *et al*. (U.S. patent no. 4,578,079) suggest that tetrapeptides containing the RGD sequence may be used to inhibit platelet aggregation. Zimmerman *et al*. (U.S. patent no. 4,683,291) disclose that positively charged amino acid residues (e. g. Arg and Lys) and homologues located before or toward the amino terminus of the RGD sequence are superior for inhibiting fibrinogen-platelet binding. Adams *et al*. (US. patent no. 4,857,508) describe superior results for *in vitro* inhibition of human platelet aggregation in platelet-rich plasma for linear tetrapeptides containing O-methyl-Tyr-amide immediately following the RGD (or homo-RGD) sequence. Barker *et al*., WO 90/01331, disclose substantially rigid RGD cyclic peptides possessing high affinity for the GP II_{b}IIIₐ receptor. A particularly efficacious rigid RGD cyclic peptide described by Barker *et al*. is represented by the following structure: 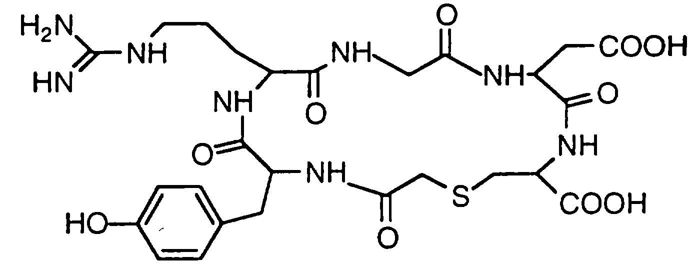 Tjoeng *et al*. (U.S. patent no. 4,879,313) describe peptide mimetic platelet aggregation inhibitors in which the first two residues of the RGD sequence are replaced by the pseudodipeptidyl 8-guanidino-octanoyl moiety. Other peptidomimetics in which the Arg of the RGD sequence has been altered include; WO89/07609 (homo-Arg[Har]), EP 341 915 (Har and alkyl-Arg), WO90/15620 (Har and amidino derivatives e. g. imidazolinyl, imidazolyl, and substituted imidazolyl), EP 422 937 (aryl-, arylalkyl-, and cycloalkyl-amines), WO9l /07976 (alkylamidino and alkylamino derivatives), and WO91/04247 (alkylamino and alkylguanidino proline derivatives). See also EP 384 362 (glycine derivatives) and EP 381 033.

Complete replacement of all residues in the RGD sequence has been described in EP 372 486, assigned to Hoffmann La Roche, where platelet aggregation inhibitors that are derivatives of benzoic and phenylacetic acid are presented. A benzoic acid derivative inhibitor having a particular low IC₅₀ in an ELISA measurement of fibrinogen GPII_{b}IIIₐ binding has the following structure: 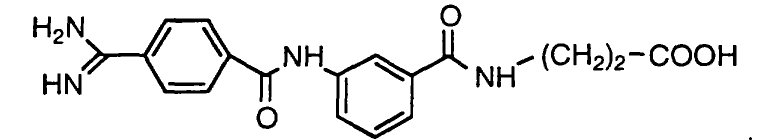 Fibrinogen receptor antagonists possessing similar structures can be found in EP 478 362, EP 478 363, and EP 478 328, all assigned to Merck. A representative Merck compound has the following structure: 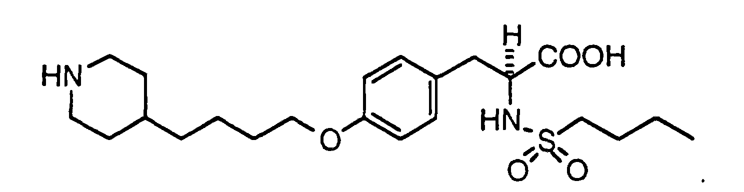 Also of interest are biphenyl derivatives described in EP 483 667 and EP 496 378, the latter publication providing a representative compound having the structure; 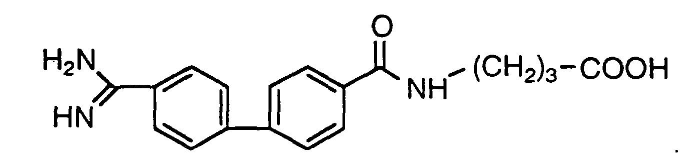 Quinazoline-3-alkanoic acid derivatives are also reported to have an inhibitory effect on platelet aggregation(although possibly by a different mechanism) in EP 456 835 A1. A generic formula representing these compounds is given by: 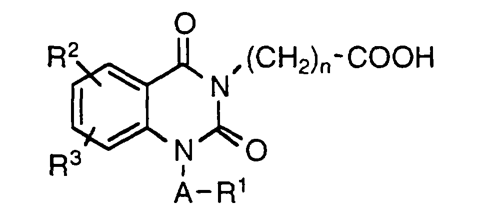 where n is 1 to 3 and R and R³ may be *interalia* hydrogen, lower alkyl, lower alkoxy, aralkyl groups that may be substituted with *interalia* -NR⁴R⁵, where R⁴ or R⁵ may be hydrogen, lower alkyl, or connected with each other to make five- or six-membered heterocycles which may contain another heteroatom, and A-R¹ may be lower alkyl.

### D. Benzodiazepines

It is well established that benzodiazepines and related ligands interact with a specific site commonly referred to as the "benzodiazepine receptor" that is associated with a neuro-inhibitory postsynaptic GABA receptor and a chloride ionophore channel (see eg. Watjen *et al*., *J. Med. Chem.* 32:2282-2291[1989]). Binding of ligands to this receptor is known to produce a wide variety of neuro-physiological effects. Benzodiazepines have not been reported to have platelet aggregation inhibition activity. The preparation and therapeutic use of benzodiazepines is described in, for example; EP 0 059 390, EP 0 059 386, and EP 0 394 101.

Benzodiazepinediones have been employed as intermediates in the synthesis of various anti-HIV-1 compounds. For example Kukla, M. J. *et al*., *J. Med. Chem.* 34:3187-3197 (1991) reduce the dilactam to either the corresponding "-one" or diamine in the preparation of various TIBO derivatives having anti-HIV activity. Pyridodiazepines are also described as useful for treatment of HIV infection in U.S. Patent No. 5,087,625.

### E. Objects

It is an object of this invention to produce nonpeptidyl compounds having potent antithrombotic activity. It is another object of the invention to produce such compounds that are essentially free of amide bonds, substantially rigid, and stable to degradation. It is a further object to produce potent nonpeptidyl antithrombotics that specifically inhibit the GPII_{b}IIIₐ-Fg interaction but do not strongly inhibit other RGD sensitive integrin interactions including the Vn-VnR, Fn-FnR, and GPII_{b}IIIₐ-vWF interactions. It is still a further object to produce potent nonpeptidyl platelet aggregation inhibitors that do not significantly increase cutaneous bleeding time or diminish other hemodynamic factors. It is also an object of this invention to produce nonpeptidyl compounds having a long half-life and a large therapeutic range.

It is still a further object to produce nonpeptidyl compounds that are capable of inhibiting other integrin interactions such as the Vn-VnR interaction.

These and other objects of this invention will be apparent from consideration of the invention as a whole.

### SUMMARY OF THE INVENTION

The objects of this invention are accomplished by providing a nonpeptidyl compound represented by structural formula I: 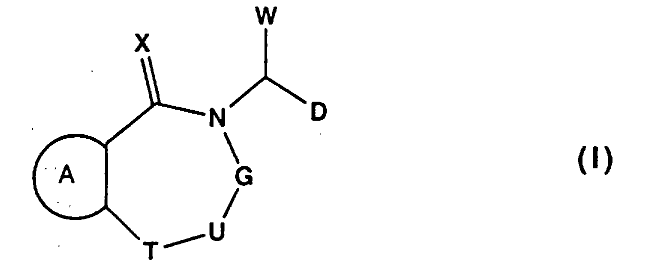 where the partial structure 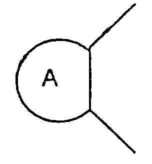 represents; 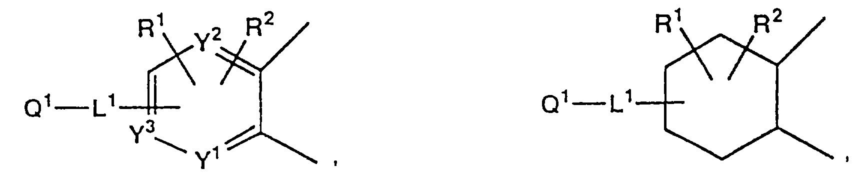 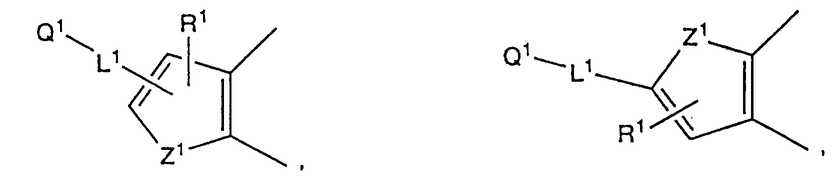 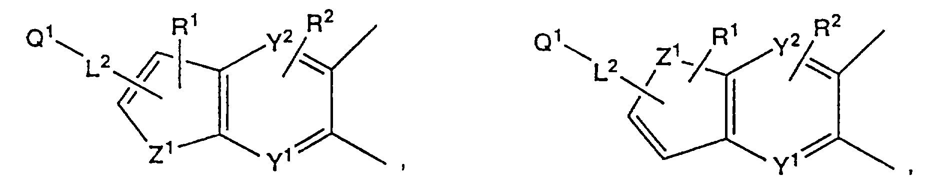 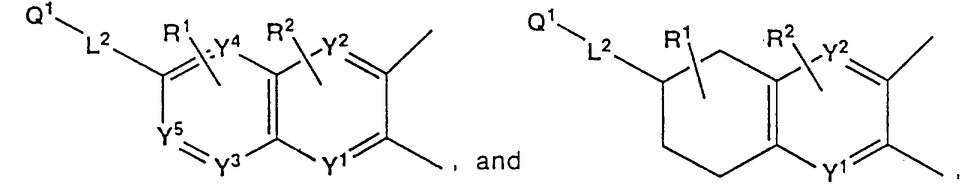 where R¹ and R are one to three optional groups typically selected from; hydrogen, halo(F, Cl, Br, I), cyano carboxamido, carbamoyloxy, formyloxy, formyl, azido, nitro, ureido, thioureido, thiocyanato, hydroxy, mercapto, sulfonamido, and an optionally substituted radical selected from; C₁ -C₁₂ alkyl, C₂-C₁₂ alkenyl, C₃-C₁₂ alkynyl, C₃-C₁₂ cycloalkyl, C₆-C₁₄ aryl, C₆-C₁₀ aryl-C₁-C₈ alkyl, C₁-C₁₂ alkyloxy, C₆-C₁₄ aryloxy, C₁-C₁₂ acylamino, N,N-di(C₁-C₁₂)acylamino, N-(C₁C₁₂)alkyl-N-(C₁-C₁₂)-acylamino, C₁-C₁₂ alkylsulfonamido, N-(C₁-C₁₂)-alkyl-N-(C₁-C₁₂)alkyl-sulfonylamino, C₁-C₁₂ alkylthiocarbonyl, C₁-C₁₂ alkylthio, C₁-C₁₂ alkylsulfinyl, C₁C₁₂ alkylsulfonyl, C₁-C₁₂ alkylsulfonato, N-(C₁-C₁₂)alkylsulfonamido, N,N-di-(C₁-C₁₂) alkyl sulfonamido, N-(C₁-C₁₂) alkyl-N-thioformylamino, C₁-C₁₂ thioacylamino, N-(C₁-C₁₂)alkyl-N-(C₁-C₁₂) thioacylamino, C₁-C₁₂ alkylsulfinamido, N-(C₁-C₁₂)alkyl-N-(C₁-C₁₂)alkylsulfinylamino, C₁-C₁₂ carbalkoxy, C₁-C₁₂ alkylcarbonyl, C₁-C₁₂ alkanoyloxy, N-(C₁-C₁₂)alkylcarboxamido, N,N-di-(C₁-C₁₂)carboxamido, N-(C₁-C₁₂) alkylcarbamoyloxy, N,N-di-(C₁-C₁₂)carbamoyloxy, and heterocycloalkyl or heteroaryl having from 1 to 3 rings, each ring having from 5 to 7 atoms with from 0-3 heteroatoms selected from N, O, and S, provided that at least one ring contains a heteroatom, where the substituents are typically selected from halo (F, Cl, Br, I), cyano, azido, nitro, hydroxy, mercapto, sulfonamido, ureido, thioureido, carboxamido, carbamoyloxy, formyloxy, formyl, C₁-C₄alkyl, C₁-C₄alkoxy, phenyl, and phenoxy. Optionally, R¹ and R when bonded to adjacent carbon atoms may join to form an unsubstituted or substituted aryl ring, where the substituents are typically selected from halo (F, Cl, Br, I), cyano, azido, nitro, hydroxy, mercapto, sulfonamido, ureido, thioureido, carboxamido, carbamoyloxy, formyloxy, formyl, C₁-C₄alkyl, C₁-C₄alkoxy, phenyl, and phenoxy.

Y¹, Y, Y³, Y⁴ and Y⁵ are independently selected from CH, CR¹, CR, and N; X is O, S, =NCN, or =NO(C₁-C₃)alkyl; and Z¹ is selected from CH₂, NH, S, and O;

Q¹ is a substituted or unsubstituted positively charged nitrogen-containing moiety. Preferably Q¹ is selected from:
(A) an amino group including;
   (1) -NH₂,
   (2) -NR³H,
   (3) -NR³R⁴, and
   (4) -NR³R⁴R⁵,
   where R³, R⁴, and R⁵ are typically selected from; (i) an optionally substituted radical selected from (a) -NR⁶R⁷, (b) -C(=NR⁸)-NR⁶R⁷, (c) -N=CR⁹-NR⁶R⁷, (d) -NR¹⁰-CR⁹=NR⁸, and (e) -NR¹⁰-C(=NR⁸)-NR6R7 where each R⁶, R⁷, R⁸, R⁹, and R¹⁰ is independently selected from hydrogen, C₁-C₄ alkoxy, C₁-C₄ alkyl, and halo(F, Cl, Br, I)-C₁-C₄ alkyl, (ii) optionally substituted C₁-C₁₂ alkyl, (iii) optionally substituted C₃-C₇ alkenyl, (iv) optionally substituted C₃-C₇ alkynyl, (v) optionally substituted C₃-C₁₂ cycloalkyl, (vi) optionally substituted C₅-C₁₂ cycloalkenyl, (vii) optionally substituted C₆-C₁₄ aryl, (viii) optionally substituted C₁-C₆ alkyl-C₆-C₁₄-aryl, (ix) optionally substituted C₃-C₆ alkenyl-C₆-C₁₀ aryl, (x) optionally substituted heterocyclyl, (xi) optionally substituted C₁-C₆ alkyl-heterocyclyl, (xii) optionally substituted C₁-C₈ alkoxy, (xiii) optionally substituted C₁-C₈ thioalkoxy, (xiv) optionally substituted C₃-C₁₀ alkenoxy, and (xv) optionally substituted C₆-C₁₄ aryloxy, where the substituents are usually one to three R¹¹, each R¹¹ typically selected from (a) optionally substituted C₆-C₁₂ aryloxy, (b) optionally substituted C₆-C₁₂ arylamino, (c) optionally substituted C₆-C₁₂ aroyl, (d) optionally substituted C₆-C₁₂ arylthio, where the substituents are usually one to three R¹, each R¹ typically selected from nitro, amino, C₁-C₈ alkylamino, di-(C₁-C₈) alkylamino, amidino, aminomethyleneimino, imino, imino-C₁-C₄ alkyl, iminomethyleneamino, guanidino, C₆-C₁₀ arylamino, C₁-C₈ acylamino, C₁-C₄ alkylsulfonamino, azido, cyano, hydroxy, hydroxy-C₁-C₈-alkyl, C₁-C₈-alkoxy, phenyloxy, C₁-C₈ alkanoyloxy, C₁-C₈ alkanoyl, C₆-C₁₂ aroyl, benzamido, phenyl, halo(F, Cl, Br, I), halo-C₁-C₈-alkyl, and C₁-C₈-alkyl, (e) C₁-C8 alkoxy (f) C₁-C₈alkthio (g) halo(F, Cl, Br, l), (h) hydroxy, (i) mercapto, (j) C₁-C₈ alkylcarbonyl, (k) carbamoyl, (I) formyl, (m) formyloxy, (n) carboxy, (o) carb-C₁-C₈ alkyloxy, (p) C₁-C₈ alkanoyloxy, (q) N-(C₁-C₈)-alkylcarboxamido, (r) N-(C₁-C₈), N-(C₁C₈)-dialkylcarboxamido, (s) carbamoyloxy, (t) N-(C₁.C₈) alkylcarbamoyloxy, (u) N-(C₁-C₈), N-(C₁-C₈)dialkylcarbamoyloxy, (v) C₁-C₈ alkylsulfinyl, (w) C₁-C₈ alkylsulfonyl, (x) C₁-C₈ alkylsulfonato, (y) sulfo, (z) sulfonamido, (aa) N-(C₁-C₈) alkylsulfonamido, (ab) N-(C₁-C₈), N-(C₁-C₈) dialkylsulfonamido, (ac) amino, (ad) C₁-C₈ alkylamino, (ae) C₁-C₈ dialkylamino, (af) C₁-C₈ acylamino, (ag) N-(C₁-C₈), N-(C₁-C₈)-diacylamino, (ah) N-(C₁-C₈)-alkyl-N-(C₁-C₈)-acylamino, (ai) formylamino, (aj) ureido, (ak) isothioureido, (al) amino-C₂-C₈ alkylthio, (am) amino-C₂-C₈ alkloxy, (an) amidino,(ao) guanidino, (ap) aminomethyleneimino, (aq) imino, (ar) imino-C₁-C₄ alkyl, (as) iminomethyleneamino, (at) glycylamino, (au) glycyl,(av) phthalimido, (aw) succinimido, (ax) morpholino, (ay) C₁-C₈ alkylsulfonamido, (az) N-(C₁-C₈)-alkyl-N-(C₁-C₈) alkyl sulfonoylamino, (ba) C₁-C₈ alkylsulfinamino, (bb) N-(C₁-C₈) alkyl-N-(C₁-C₈) alkylsulfinamino, (bc) C₁-C₈ alkoxyamino, (bd) C₁-C₈ alkoxyamino, (be) N-(C₁-C₈) alkyl-N-(C₁-C₈) alkoxyamino, (bf) C₃-C₇ cycloalkyl, (bg) oxo, and (bh) heterocyclyl, optionally any one or two pairs of R³-R¹⁰ may independently be joined to form one or two optionally substituted heterocyclic rings, each ring optionally fused with one or two optionally substituted homocyclic or heterocyclic rings of from four to seven atoms where any heterocyclic ring contains from one to four heteroatoms selected from N, O, and S and where any ring may be substituted with from one to three R¹,
(B) an amidino (aminoiminomethyl) group including;
   (1) -C(=NH)-NH₂,
   (2) -C(=NH)-NHR³,
   (3) -C(=NR⁴)-NHR³,
   (4) -C(=NH)-NR³R⁴, and
   (5) -C(=NR⁵)-NR³R⁴,
   where R³, R⁴, and R⁵ are defined above,
(C) an aminoalkyleneamino group including;
   (1) -N=CH-NH₂,
   (2) -N=CH-NHR³,
   (3) -N=CH-NR³R⁴, and
   (4) -N=CR⁵-NR³R⁴,
   where R³, R⁴, and R⁵ are defined above,
(D) an iminoalkyleneamino group, including;
   (1) -NH-CH=NH,
   (2) -NH-CH=NR³,
   (3) -NH-CR⁴=NR³, and
   (4) -NR⁵-CR⁴=NR³,
   where R³, R⁴, and R⁵ are defined above,
(E) a guanidino (aminoiminomethyleneamino) group including;
   (1) -NH-C(=NH)-NH₂,
   (2) -NH-C(=NH)-NR³H,
   (3) -NH-C(=NH)-NR³R⁴,
   (4) -NH-C(=NR⁵)-NR³R⁴,
   (5) -NR³-C(=NR³)-NR³R⁴,
   (6) -NR³-C(=NH)-NR³R⁴,
   (7) -NR³-C(=NR³)-NH₂,
   (8) -NR³-C(=NH)-NH₂,
   (9) -NR³-C(=NR³)-NHR⁴, and
   (10) -NR³-C(=NH)-NHR⁴,
   where R³, R⁴, and R⁵ are defined above,
(F) an optionally substituted saturated heterocyclic group including; 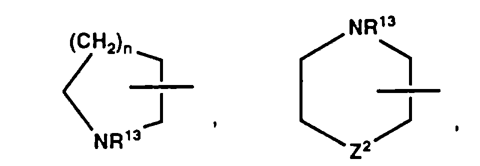 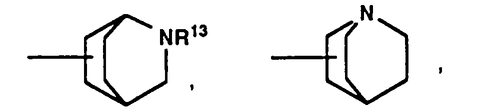 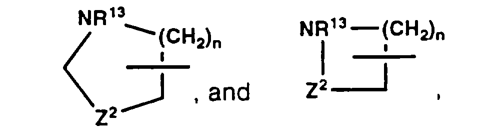 where (1) n is 0, 1, 2, or 3, (2) R¹³ is selected from; R⁶, -CR⁹=NR⁸, -CR⁹(=NR⁸)-NR⁶R⁷, -C(=NR⁸)-NR⁶R⁷, -N=CR⁹-NR⁶R⁷, -NR¹⁰-CR⁹=NR⁸, and -NR¹⁰-(C=NR⁸)-NR⁶R⁷ where R⁶-R¹⁰ are defined above, (3) Z is O, S, or NR¹³, and (4) the substituents are independently one to three R¹,
(G) an optionally substituted unsaturated (nonaromatic) heterocyclyl including; 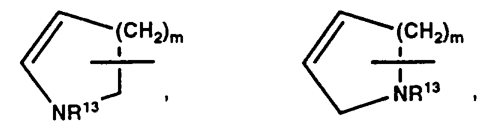 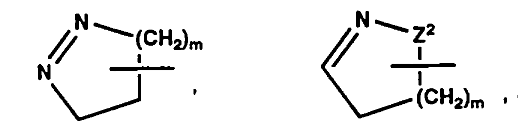 and 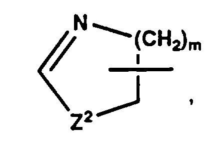 where (1) m is 1, 2, or 3, (2) Z and R¹³ are defined above, and (3) the substituents are typically one to three R¹,
(H) an optionally substituted unsaturated (aromatic) heterocyclyl including; 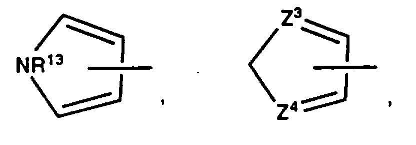 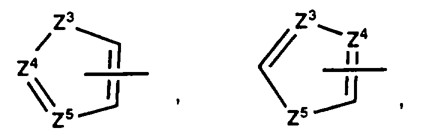 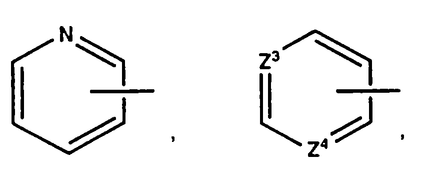 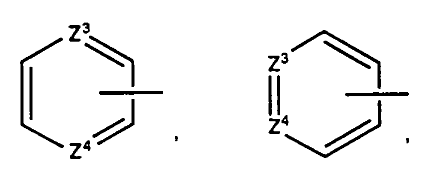 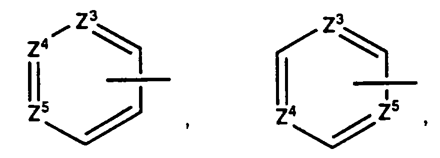 and 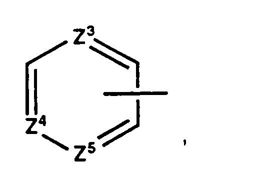 where (1) Z³, Z⁴, and Z⁵ are typically selected from O,S,N, and NH, provided at least one Z³, Z⁴, or Z⁵ is N or NH, (2) R¹³ is defined above, and the substituents are independently one to three R¹,
(I) an optionally substituted bicycloheterocyclic group including; 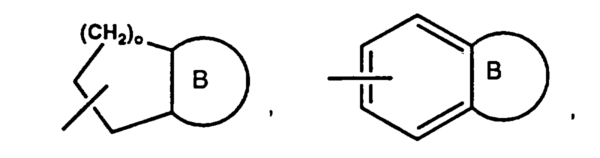 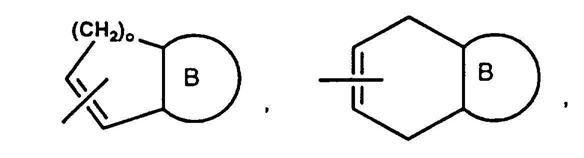 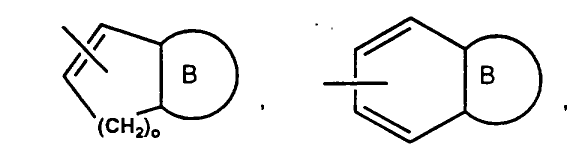 and 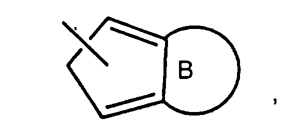 where the partial structure 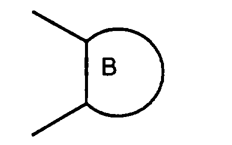 represents 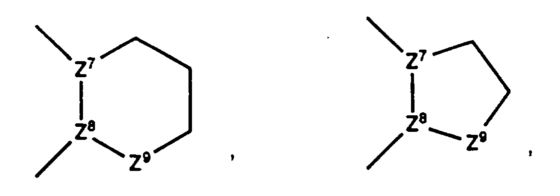 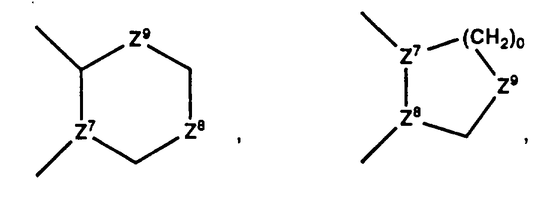 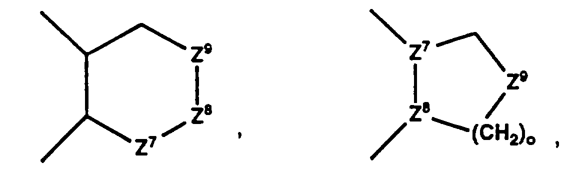 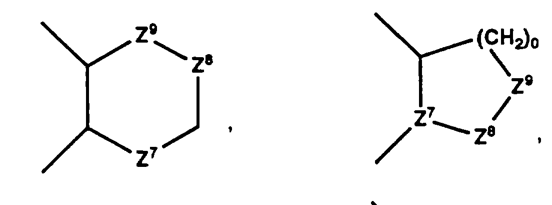 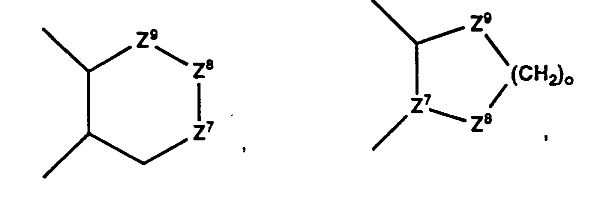 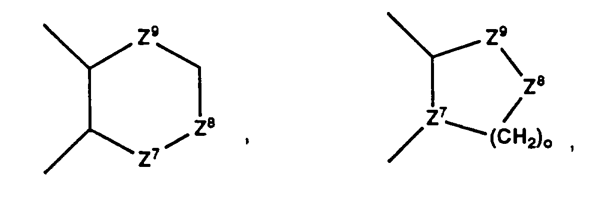 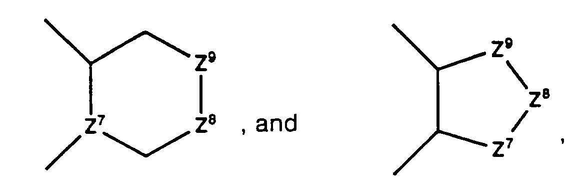
   where Z⁷, Z⁸, and Z⁹are independently selected from;

   -CH₂- ,

   -CH=,

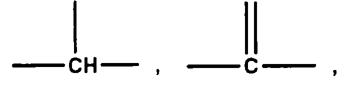

   -O-_{,}

   -S-,

   -N=,

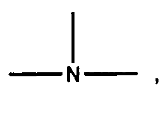

   and

   -NR¹³-

   provided that at least one Z⁷, Z⁸, or Z⁹ is

   -N=,

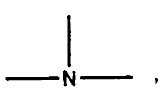

   or

   -NR¹³-,

   where (1) o is 0, 1, or 2, (2) R¹³ is defined above, and (3) the substituents are independently one to three R¹. Exemplary heterocyclic Q¹ groups include; isoindolinyl, quinuclidinyl, morpholinyl, and 1,3-diazacyclohex-4-ene. Optionally, any of the nitrogen containing heterocycles described above may be substituted with amino, imino, amidino, aminomethyleneimino, iminomethyleneamino, guanidino, N^{G}-aminoguanidino, alkylamino, dialkylamino, trialkylamino, or alkylideneamino groups.

L¹ is a bivalent radical containing from 3 to 9 methylene groups where any methylene group or groups may be replaced with one or more alkene, alkyne, aryl, or functional groups containing the heteroatoms selected from the group N, O, and S. Preferably, L¹ is a saturated or unsaturated, linear, branched, or cyclic bivalent radical separating Q from the 7 carbon of the benzodiazepinedione nucleus. Typically, the heteroatoms (N, O, and S) will comprise from 0-5 of the atoms separating Q from the benzodiazepinedione nucleus. Preferably, L¹ is typically an optionally substituted bivalent radical including; (A) C₃-C₇-alkylene, (B) C₃-C₇-cycloalkylene, (C) C₃-C₇-alkenylene, (D) C₃-C₇-alkadienylene, (E) C₃-C₇-alkynylene, (F) C₄-C₇-alkadiynylene, (G) C₄-C₇-alkenynylene, (H) C₆-C₁₄-arylene, (I) C₆-C₁₄-aryl-C₂-C₄-alkynylene, (J) C₁-C₃-alkyl-C₆-C₁₄-aryl-C₂-C₄-alkynylene, (K) C₆-C₁₄-aryl-C₂-C₄-alkenylene, (L) C₁-C₃-alkyl-C₆-C₁₄-arylene, (M) C₁-C₃-alkyl-C₆-C₁₄-aryl-C₂-C₄-alkenylene, (N) C₆-C₁₄-aryl-C₁-C₃-alkylene, (O) C₆-C₁₄-aryl-C₁-C₃-alkyloxyene, (P) C₁-C₂-alkyl-C₆-C₁₄-aryl-C₁-C₂-alkylene, (Q) C₁-C₃-alkyloxy-C₆-C₁₄-arylene, (R) C₂-C₆-alkyloxyene, (S) C₁-C₅-alkyloxy-C₁-C₅-alkylene, (T) C₆-C₁₀-aryloxyene, (U) C₆-C₁₀-aryloxy-C₁-C₅-alkylene, (V) C₂-C₆-alkylthioene, (W) C₁-C₅-alkylthio-C₁-C₅-alkylene, (X) C₆-C₁₀-arylthioene, (Y) C₆-C₁₀-arylthio-C₁-C₅-alkylene, (Z) C₁-C₅-alkylsulfoxide-C₁-C₅-alkylene, ( AA) C₁-C₅-alkylsulfone-C₁-C₅-alkylene,

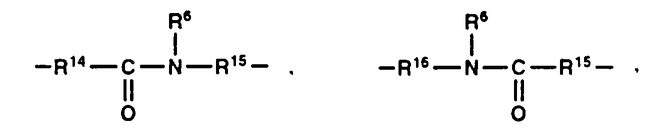

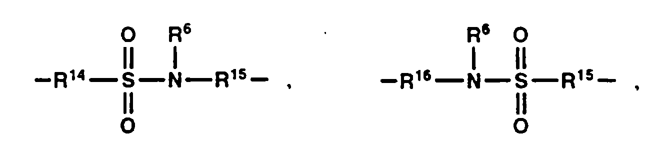

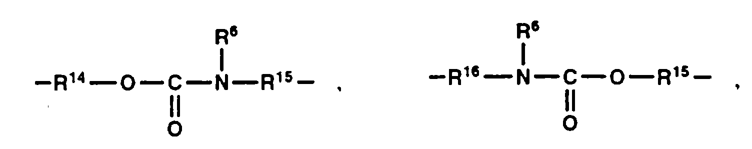

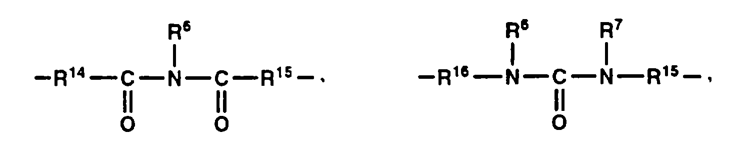

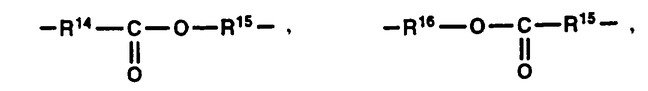

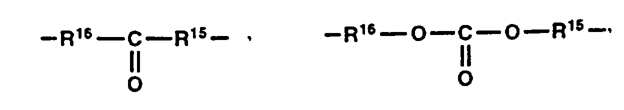

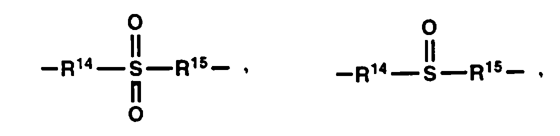

-R¹⁴-S-R¹⁵- ,

-R¹⁴-O-R¹⁵- ,

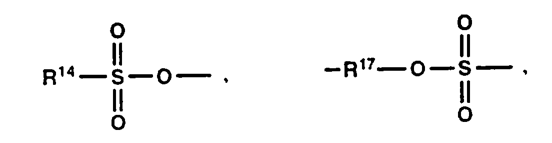

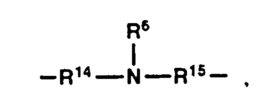

and

-HET-R¹⁵-,

where (1) R¹⁴ is selected from; (i) a chemical bond, (ii) C₁-C₅-alkyl, (iii) C₃-C₇-cycloalkyl, (iv) C₂-C₅-alkenyl, (v) C₃-C₅-alkynyl, (vi) C₆-C₁₀-aryl, (vii) C₁-C₂-alkyl-C₆-C₁₀-aryl-C₁-C₂-alkyl, (viii) C₁ -C₃-alkyl-C₆-C₁₂-aryl, (ix) C₆-C₁₀-aryl-C₁-C₂-alkyl, (x) C₆-C₁₀-aryloxy-C₁-C₂-alkyl, and (xi) piperizinyl, (2) R¹⁵ is selected from; (i) a chemical bond, (ii) C₁-C₄-alkyl, (iii) C₂-C₄-alkenyl, (iv) C₂-C₄-alkynyl, (v) C₁-C₃-alkyl-C₆-C₁₂-aryl, and (vi) C₆-C₁₀-aryl, (3) R¹⁶ is selected from; (i) a chemical bond, (ii) C₁-C₅-alkyl, (iii) C₃-C₇-cycloalkyl, (iv) C₃-C₅-alkenyl, (v) C₃-C₅-alkynyl, (vi) C₆-C₁₀-aryl, (vii) C₁-C₃-alkyl-C₆-C₁₂-aryl, (viii) C₆-C₁₀-aryl-C₁-C₂-alkyl, and (ix) piperizinyl, (4) R¹⁷ is selected from; (i) C₃-C₄-alkenyl, (ii) C₃-C₄-alkynyl, (iii) C₆-C₁₀-aryl, and (iv) benzyl, (5) HET is a saturated or unsaturated heterocycle having from 5-14 atoms in the cycle(s) and from 1-3 heteroatoms selected from N, O, and S, where the substituents are selected from one to three R¹.
L is typically an optionally substituted bivalent radical including but not limited to;

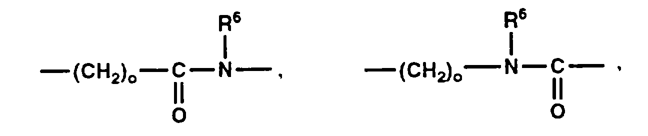

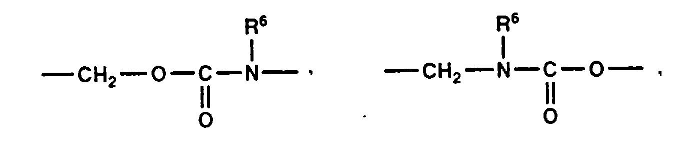

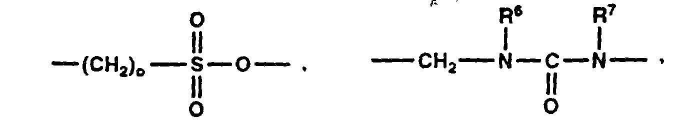

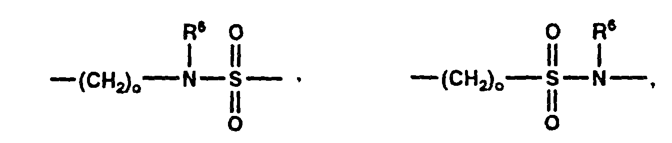

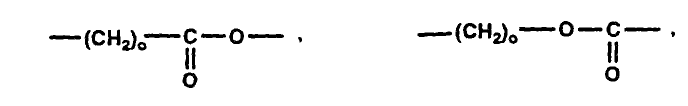

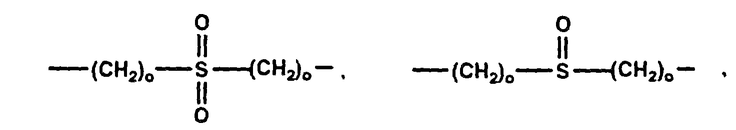

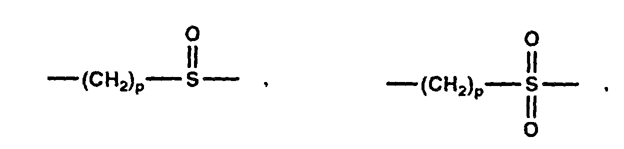

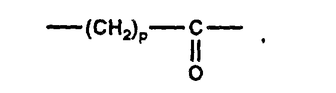

-(CH₂)ₚ-S- ,

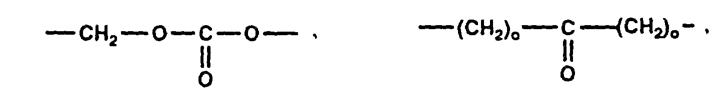

-(CH₂)ₒ-S-(CH₂)ₒ- ,

-(CH₂)ₒ-O-(CH₂)ₒ-,

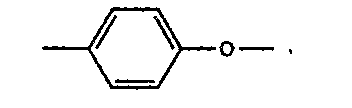

-(CH₂)ₚ-O- ,

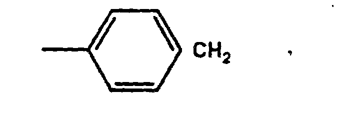

- (CH₂)ₚ - ,

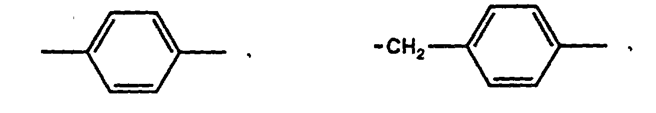

-(CH₂)ₒ-C≡C- ,

-(CH₂)ₒ-CH=CH- ,

-O-(CH₂)ₚ- ,

and

-HET- ,

where; (1) p is 1, 2, 3 or 4, (2) R⁶, R⁷, o, and HET are defined above, and (3) the substituents are typically selected from one to three R¹.
T-U-G is selected from; 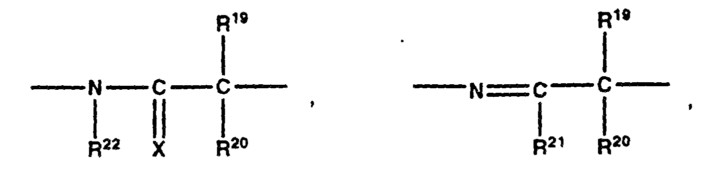 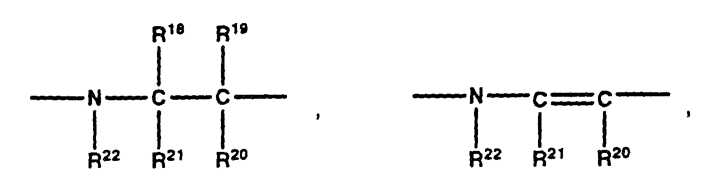 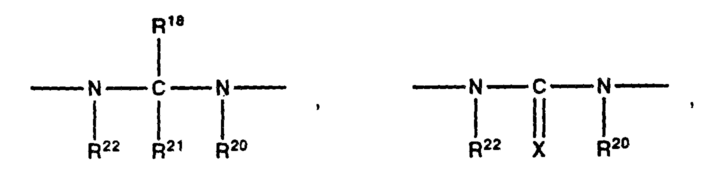 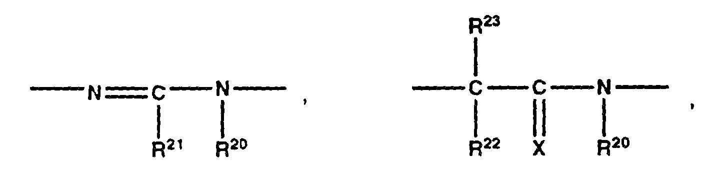 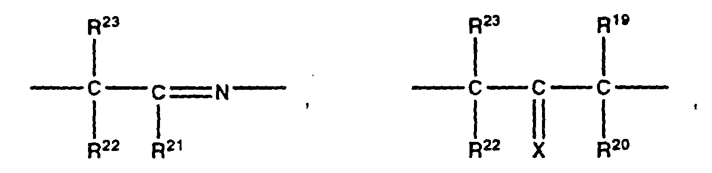 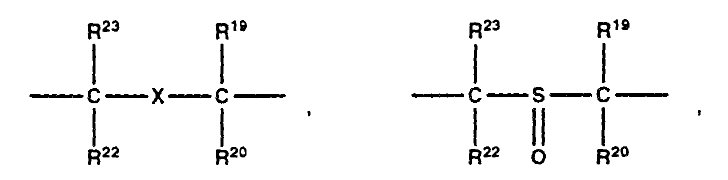 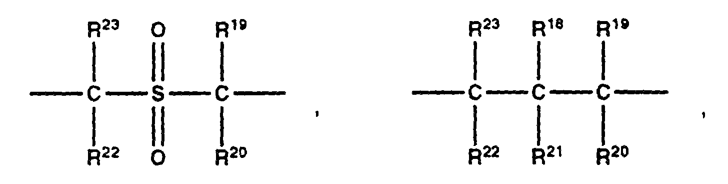 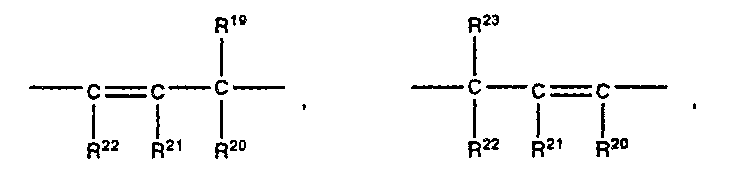 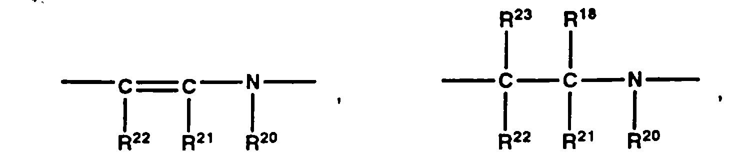 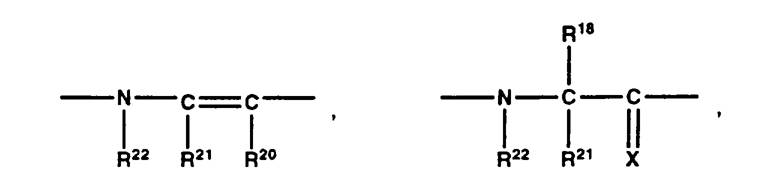 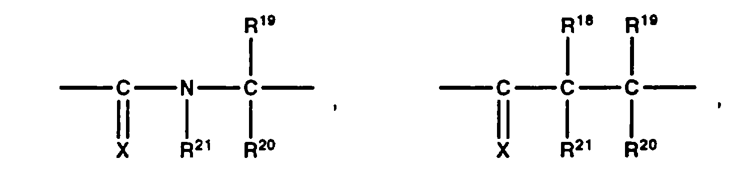 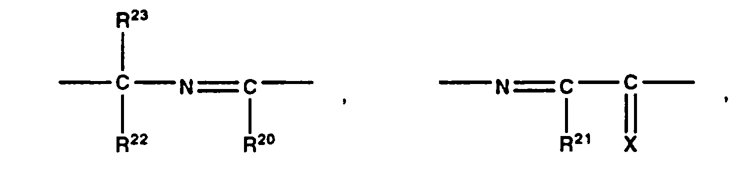 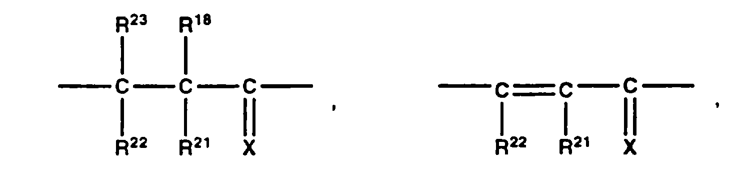 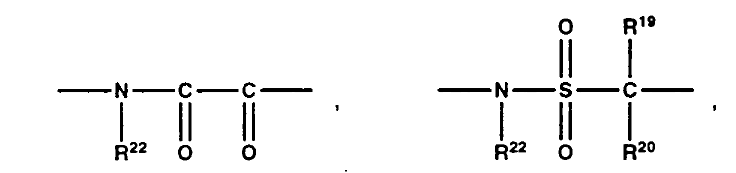 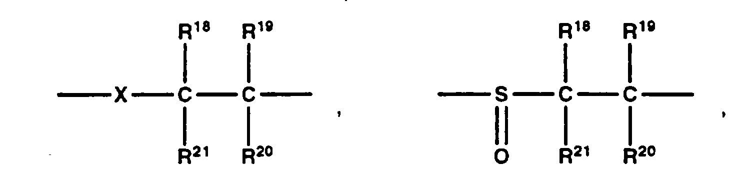 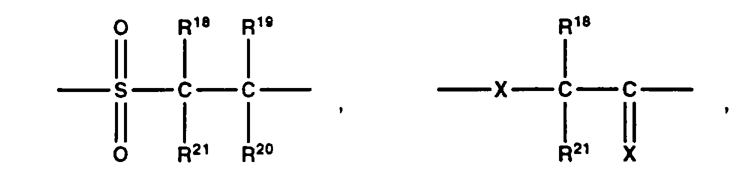 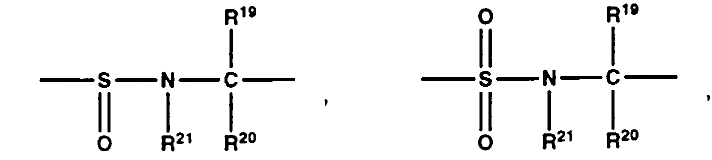 and 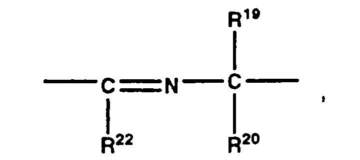 where; (1) X is defined above, (2) R¹⁸, R¹⁹, R⁰, R³, are typically selected from (i) hydrogen, (ii) optionally substituted C₁-C₁₂ alkyl, (iii) optionally substituted C₃-C₁₂ alkenyl, (iv) optionally substituted C₃-C₁₄ cycloalkyl, (v) optionally substituted C₁-C₁₂ alkyl-C₆-C₁₄ aryl, (vi) optionally substituted C₆-C₁₄ aryl, (vii) optionally substituted C₁-C₄ alkylphenyl, and (viii) optionally substituted C₁ -C₁₂ alkoxy, (3) R¹ and R are typically selected from (i) Q-L³- where Q is selected from hydrogen and Q¹, and L³ is either a chemical bond, L¹ or L, (ii) optionally substituted C₁-C₁₂-alkyl, (iii) optionally substituted C₆-C₁₄-aryl, (iv) optionally substituted C₃-C₁₄-cycloalkyl, (v) optionally substituted C₁-C₁₂-alkyl-C₆-C₁₄ aryl, and (vi) optionally substituted C₁-C₁₂-alkyl-C₃-C₁₄-cycloalkyl, where the substituents are usually (a) halo (F, Cl, Br, I), (b) nitro, (c) hydroxy, (d) carboxy, (e) tetrazole, (f) hydroxamate, (g) sulfonamide, (h) trifluoroimide, (i) phosphonate, (j) C₁-C₆-alkyl, (k) C₆-C₁₄-aryl, (l) benzyl, (m) C₃-C₁₄-cycloalkyl, (n) COR⁴ where R⁴ is selected from C₁ -C₈-alkoxy, C₃-C₁₂-alkenoxy, C₆-C₁₂-aryloxy, di-C₁-C₈-alkylamino-C₁-C₈-alkoxy, acylamino-C₁-C₈-alkoxy selected from acetylaminoethoxy, nicotinoylaminoethoxy, succinamidoethoxy, and pivaloyloxyethoxy, and C₆-C₁₂-aryl-C₁-C₈-alkoxy where the aryl group is unsubstituted or substituted with one to three of the groups nitro, halo (F, Cl, Br, I), C₁ -C₄-alkoxy, amino, hydroxy, hydroxy-C₂-C₈-alkoxy, and dihydroxy-C₃-C₈-alkoxy, and (o) CONR⁵R⁶ where R⁵ and R⁶ are typically selected from hydrogen, C₁-C₁₀-alkyl, C₃-C₁₀-alkenyl, C₆-C₁₄-aryl, C₁*-*C₆*-*alkyl-C₆-C₁₀-aryl. Optionally, R⁵ and R⁶ taken together may form trimethylene, tetramethylene, pentamethylene, and 3-oxopentamethylene. Also, optionally, R¹ and R taken together may form an optionally substituted 5, 6, or 7 member saturated or unsaturated homocyclic or heterocyclic ring containing 0-3 heteroatoms selected from O, S, and N, where the substituents are selected from R¹.

D is typically (A) R¹, or (B) -(C=O)-Xaa, where Xaa is one to three D or L α-amino acid residues.

W is -R⁷-w.

R⁷ is selected from (a) a covalent bond, (b) substituted or unsubstituted methylene, and (c) substituted or unsubstituted ethylene where the substituents are selected from (i) nitro, (ii) halo(F, Cl, Br, l), (iii) C₁-C₆ alkyl, (iv) halo(F, Cl, Br, l)-C₁-C₆ alkyl, and (v) substituted or unsubstituted phenyl where the substituents are selected from (1) C₁-C₆ alkyl, (2) C₁-C₆ alkoxy, (3) halo(F, Cl, Br, I), and (4) CF₃.

"w" is selected from (a) -COR⁸, (b) -SO₃R³¹, (c) -NHSO₂R³ , (d) -PO(OR³¹)₂, (e) -SO₂NHR³, (f) -CONHOR³¹, (g) -C(OH)R³³PO(OR³³)₂, (h) -CN, (i) -SO₂NH-heteroaryl where the heteroaryl is a 5- or 6-member aromatic ring containing 1 to 3 heteroatoms selected from O, N, and S and where the heteroaryl is unsubstituted or substituted with one or two substituents selected from the group (i) -OH, (ii) -SH, (iii) -(C₁-C₄alkyl), (iv) -C₁-C₄alkoxyl, (v) CF₃, (vi) halo(F, Cl, Br, l), (vii) NO₂, (viii) -COOH, (ix) -COO-(C₁C₄alkyl), (x) -NH₂, (xi) -NH(C₁-C₄alkyl), or (xii) -N(C₁-C₄alkyl)₂, (j) -CH₂SO₂NH-heteroaryl, (k) -SO₂NHCOR³³, (l) -CH₂SO₂NHCOR³, (m) -CONHSO₂R³³, (n) -CH₂CONHSO₂R³³, (o) -NHCONHSO₂R³³, (p) -NHSO₂NHCOR³³, (q) -CONHNHSO₂CF₃, (r) CON(OH)R³¹, (s) -CONHCOCF₃, (t) -CONHSO₂R⁸, (u) -CONHSO₂R⁹, (v) -CONHSO₂R³⁰,

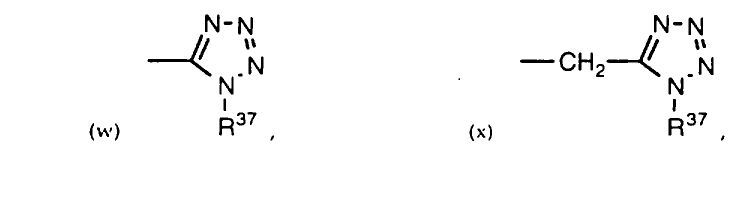

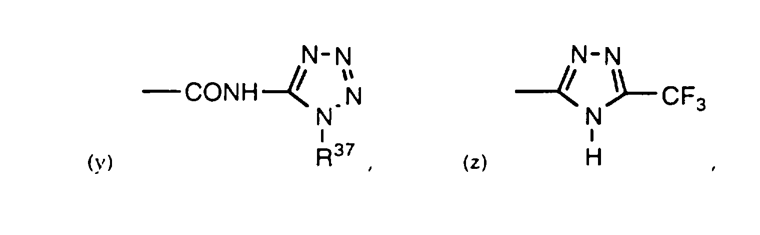

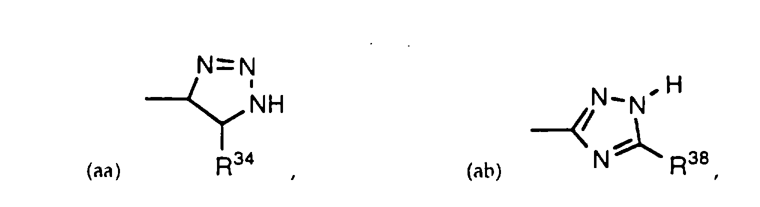

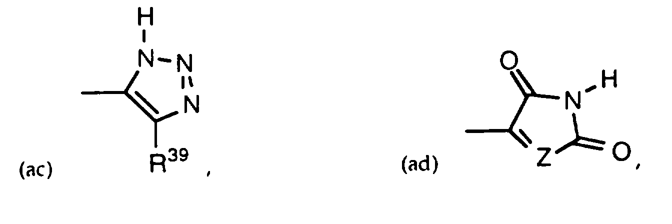

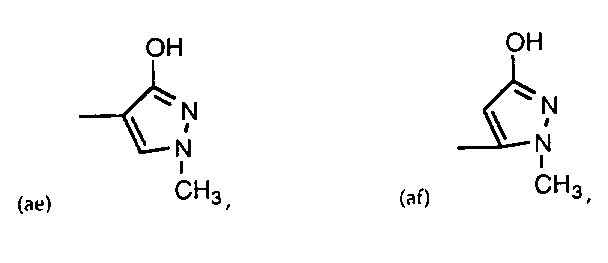

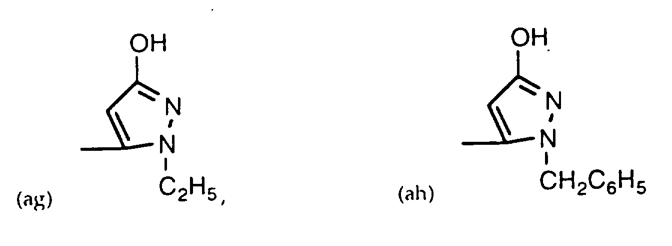

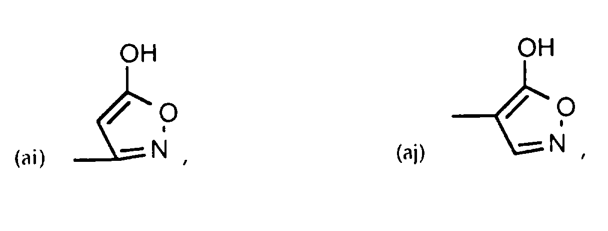

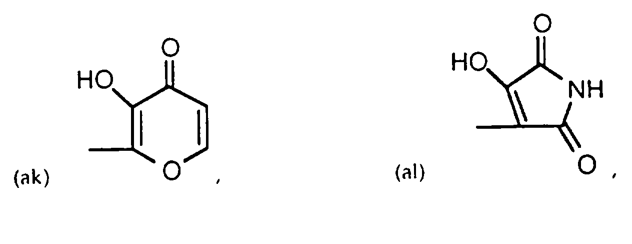

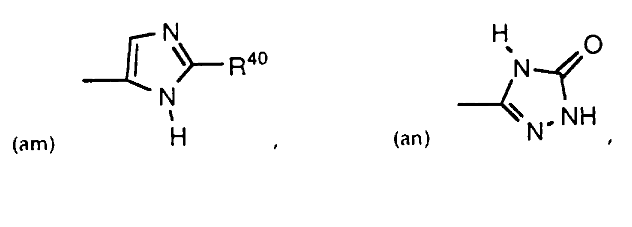

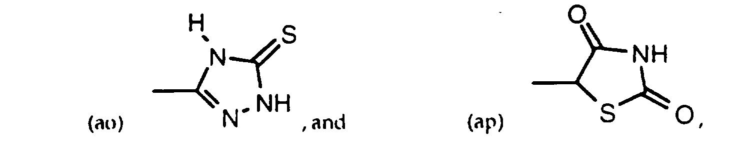

R⁸ is selected from the group consisting of (a) hydroxy, (b) C₁ -C₈-alkoxy, (c) C₃-C₁₂-alkenoxy, (d) C₆-C₁₂-aryloxy, (e) C₁-C₆-alkyl-C₆-C₁₂-aryloxy, (f) di-C₁-C₈-alkylamino-C₁-C₈-alkoxy, (g) acylamino-C₁-C₈-alkoxy selected from the group (i) acetylaminoethoxy, (ii) nicotinoylaminoethoxy, and (iii) succinamidoethoxy, (h) C₁-C₈-alkoyloxy-C₁-C₈-alkoxy, (i) C₆-C₁₂-aryl-C₁-C₈-alkoxy where the aryl group is unsubstituted or substituted with one to three of the groups (i) nitro, (ii) halo (F, Cl, Br, l), (iii) C₁-C₄-alkoxy, and (iv) amino, (j) hydroxy-C₂-C₈-alkoxy, (k) dihydroxy-C₃-C₈-alkoxy, and (l) NR⁹R³⁰.

R⁹ and R³⁰ are independently selected from the group consisting of (a) hydrogen, (b) C₁-C₈-alkyl, (c) C₃-C₈-alkenyl, (d) C₆-C₁₂-aryl where the aryl group is unsubstituted or substituted with one to three of the groups (i) nitro, (ii) halo (F, Cl, Br, I), (iii) C₁ -C₄-alkoxy, and (iv) amino, and (e) C₆-C₁₂-aryl-C₁-C₈-alkyl where the aryl group is unsubstituted or substituted with one to three of the groups (i) nitro, (ii) halo (F, Cl, Br, I), and (iii) C₁-C₄-alkoxy.

R³¹ is selected from the group consisting of (a) H, (b) C₁-C₆ alkyl, (c) halo(F, Cl, Br, I)-C₁-C₆ alkyl, (d) phenyl, (e) benzyl, and (f) -CH₂-O-COCH₃.

R³ is selected from the group consisting of (a) H, (b) benzyl and (c) -CH(R³⁵)-O-C(O)R³⁵.

R³³ is selected from the group consisting of (a) aryl, (b) heteroaryl, (c) (C₃-C₇)-cycloalkyl, (d) (C₁-C₄)-alkyl, unsubstituted or substituted with a substituent selected from the group consisting of (i) aryl, (ii) heteroaryl, (iii) -OH, (iv) -SH, (v) (C₁-C₄)-alkyl, (vi) (C₁-C₄)-alkoxy, (vii) (C₁-C₄)-alkylthio, (viii) -CF_{3,} (ix) halo (F, Cl, Br, l), (x) -NO₂, (xi) -CO₂H, (xii) CO₂-(C₁-C₄)-alkyl, (xiii) -NH₂, (xiv)-N[(C₁-C₄)-alkyl]₂, (xv) -NH[(C₁-C₄)-alkyl], (xvi) -PO₃H or (xvii) PO(OH)(C₁-C₄)-alkoxy, or (e) (C₁-C₄)-perfluoroalkyl.

R³⁴ is selected from the group consisting of (a) -CN, (b) -NO₂, (c) -COOR³¹, (d) C₁-C₆-perfluoroalkyl, and (e) CF₃.

R³⁵ is independently selected from the group consisting of (a) H, (b) (C₁-C₆)-alkyl, (C₂-C₆)-alkenyl, (C₂-C₆)-alkynyl or (C₃-C₈)-cycloalkyl, each of which is unsubstituted or substituted with: (i) OH, (ii) (C₁-C₄)-alkoxy, (iii) CO₂R³³, (iv) OCOR³³, (v) CONHR³³, (vi) CON(R³³)₂, (vii) N(R³³)C(O)R³³, (viii) NH₂, (ix) (C₁-C₄)-alkylamino, (x) di[(C₁-C₄)-alkyl]amino, (xi) aryl, (xii) heteroaryl, (c) -C(O)-aryl, (d) -NO₂, (e) halo(Cl, Br, I, F), (f) -OH, (g) -OR³⁶, (h) (C₁-C₄)-perfluoroalkyl, (i) -SH, (j) -S(O)*₁₋₂* (C₁-C₄)-alkyl, (k) CO₂R³³, (l) -SO₃H, (m) -NR³³R³⁶, (n) -NR³³C(O)R³⁶, (o) -NR³³COOR³, (p) -SO₂NHR³, (q) -SO₂NR³³R³³, (r) -NHSO₂R³, (s) -C(O)NHSO₂R³, (t) aryl, (u) heteroaryl, (v) morpholin-4-yl, (w) CONH₂, or (y) 1H-tetrazol-5-yl.

R³⁶ is selected from the group consisting of (a) H or (b) (C₁-C₄)-alkyl unsubstituted or substituted with (i) NH₂, (ii) NH[(C₁-C₄)-alkyl], (iii) N[(C₁-C₄)-alkyl]₂, (iv) CO₂H, (v) CO₂(C₁-C₄)-alkyl, (vi) OH, (vii) SO₃H, or (viii) SO₂NH₂.

R³⁷ is selected from the group consisting of (a) H, (b) (C₁-C₆)-alkyl, (c) (C₂-C₆)-alkenyl, (d) (C₁-C₆)-alkoxyalkyl, (e) -CH₂-O-COCH_{3,} or (f) -CH₂-phenyl, where the phenyl is unsubstituted or substituted with a substituent selected from -NO₂, -NH₂, -OH, or -OCH₃.

R³⁸,R³⁹, and R⁴⁰ are each independently selected from H, Cl, CN, NO₂, CF₃, C₂F₅, C₃F₇, CHF₂, CH₂F, CO₂CH₃, CO₂C₂H₅, SO₂CH₃, SO₂CF₃ and SO₂C₆F₅, where Z is selected from O, S, NR⁴¹ and CH₂.

R⁴¹ is selected from hydrogen, CH₃, and CH₂C₆H₅ or a tautomer thereof.

The invention further provides a pharmaceutical composition comprising a pharmaceutically, acceptable excipient and/or pharmaceutically acceptable hydrates, solvates, and salts of I.

The invention provides a method for inhibiting platelet aggregation or reducing platelet aggregation in a mammal comprising administering a platelet aggregation inhibiting amount of the pharmaceutical composition of I. Optionally, the method includes administering the pharmaceutical composition in combination with a peptidyl antithrombotic and/or a thrombolytic agent and/or an anticoagulant.

### DETAILED DESCRIPTION OF THE INVENTION

### A. Definitions

Terms used in the claims and specification are defined as set forth below unless otherwise specified.

As used herein, the term "positively charged" when used to describe the moiety Q (either Q¹ or Q) means at least 10% of the Q groups are positively charged at physiological pH. This will normally mean that the pK_{b} of the nitrogen containing moiety will be about 6.9 or higher. The phrase "positively charged Q moiety;" as used to define Q, is intended to embrace chemical groups which, when attached to the linking moiety L of Formula I, confers basic character to the compound of Formula I. "Basic character" means proton accepting or electron donating capability, that is, the capacity of the compound of Formula I to be a proton acceptor in the presence of a proton donor, such as water. Optionally, Q will be a prodrug that is capable of being converted, *in vivo,* into a positively charged basic moiety. An example of a basic group having "basic character" is the guanidino (H₂N-C(=NH)-NH-) group. There are many examples of other equivalent basic groups other than the guanidino group that can be substituted into Q to produce biologically equivalent compounds of Formula I. Such other basic groups are collectively referred to as "bioisosteres of the guanidino group" or "basic bioisoteres". Representative examples of such "basic bioisoteres" are set forth above under Q (A) through (I).

The term "alkyl" means a branched or unbranched, saturated aliphatic hydrocarbon radical, having the number of carbon atoms specified, or if no number is specified, having up to 12 carbon atoms. Examples of alkyl radicals include methyl, ethyl, n-propyl, isopropyl, n-butyl, iso-butyl, sec-butyl, tert-butyl, n-pentyl, 2-methylbutyl, 2,2-dimethylpropyl, n-hexyl, 2-methylpentyl, 2,2-dimethylbutyl, n-heptyl, 2-methylhexyl, and the like. The terms "lower alkyl" "C₁-C₆ alkyl" and "alkyl of 1 to 6 carbon atoms" are synonymous and used interchangeably. A preferred "C₁-C₆ alkyl" group is methyl.

The term "substituted Cₙ-Cₘ alkyl" where m and n are integers identifying the range of carbon atoms contained in the alkyl group, denotes the above alkyl groups that are substituted by one, two or three halogen, hydroxy, protected hydroxy, amino, protected amino, C₁-C₇ acyloxy, nitro, carboxy, protected carboxy, carbamoyl, carbamoyloxy, cyano, methylsulfonylamino or C₁-C₄ alkoxy groups. The substituted alkyl groups may be substituted once, twice or three times with the same or with different substituents.

Examples of the above substituted alkyl groups include but are not limited to; cyanomethyl, nitromethyl, hydroxymethyl, trityloxymethyl, propionyloxymethyl, aminomethyl, carboxymethyl, alkyloxycarbonylmethyl, allyloxycarbonylaminomethyl, carbamoyloxymethyl, methoxymethyl, ethoxymethyl, t-butoxymethyl, acetoxymethyl, chloromethyl, bromomethyl, iodomethyl, trifluromethyl, 6-hydroxyhexyl, 2,4-dichloro(n-butyl), 2-amino(iso-propyl), 2-carbamoyloxyethyl and the like. A preferred group of examples within the above "C₁ -C₁₂ substituted alkyl" group includes the substituted methyl group, e.g. a methyl group substituted by the same substituents as the "substituted Cₙ-Cₘ alkyl" group. Examples of the substituted methyl group include groups such as hydroxymethyl, protected hydroxymethyl (e.g. tetrahydropyranyloxymethyl), acetoxymethyl, carbamoyloxymethyl, trifluoromethyl, chloromethyl, bromomethyl and iodomethyl.

The terms "C₁-C₁₂ alkyloxy" or "C₁-C₁₂ alkoxy" are used interchangeably herein and denote groups such as methoxy, ethoxy, n-propoxy, isopropoxy, n-butoxy, t-butoxy and like groups.

The terms "C₁-C₁₂ acyloxy" or "C₁-C₁₂ alkanoyloxy" are used interchangeably and denote herein groups such as formyloxy, acetoxy, propionyloxy, butyryloxy, pentanoyloxy, hexanoyloxy, heptanoyloxy, and the like.

The terms "C₁-C₁₂ alkylcarbonyl", "C₁-C₁₂ alkanoyl" and "C₁-C₁₂ acyl" are used interchangeably herein encompass groups such as formyl, acetyl, propionyl, butyryl, pentanoyl, hexanoyl, heptanoyl, benzoyl and the like.

The term "cycloalkyl" as used herein refers to a mono-, bi-, or tricyclic aliphatic ring having 3 to 14 carbon atoms and preferably 3 to 7 carbon atoms.

The term "alkenyl" means a branched or unbranched hydrocarbon radical having the number of carbon atoms designated containing one or more carbon-carbon double bonds, each double bond being independently cis, trans, or a nongeometric isomer.

The term "alkynyl" means a branched or unbranched hydrocarbon radical having the number of carbon atoms designated containing one or more carbon-carbon triple bonds.

The terms "C₁-C₁₂ alkylthio" and "C₁-C₁₂ substituted alkylthio" denote C₁-C₁₂ alkyl and C₁-C₁₂ substituted alkyl groups, respectively, attached to a sulfur which is in turn the point of attachment for the alkylthio or substituted alkylthio group to the group or substituent designated.

The term "aryl" when used alone means a homocyclic aromatic radical whether or not fused having the number of carbon atoms designated. Preferred aryl groups include phenyl, napthyl, biphenyl, phenanthrenyl, naphthacenyl, and the like (see e.g. *Lang's Handbook of Chemistry* (Dean, J. A., ed) 13^{th} ed. Table 7-2 [1985]).

The term "substituted phenyl" or "substituted aryl" denotes a phenyl group or aryl group substituted with one, two or three substituents chosen from halogen(F, Cl, Br, I), hydroxy, protected hydroxy, cyano, nitro, C₁-C₆ alkyl, C₁-C₆ alkoxy, carboxy, protected carboxy, carboxymethyl, protected carboxymethyl, hydroxymethyl, protected hydroxymethyl, aminomethyl, protected aminomethyl, trifluoromethyl N-(methylsulfonylamino) or other groups specified.

Examples of the term "substituted phenyl" includes but is not limited to a mono- or di(halo)phenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono or di(alkoxy)phenyl group, for example, 2,6-dimethoxyphenyl, 4-methoxyphenyl, 3-ethoxyphenyl, 4-(isopropoxy)phenyl, 4-(t-butoxy)phenyl, 3-ethoxy-4-methoxyphenyl and the like; 3- or 4- trifluoromethylphenyl; a mono- or dicarboxyphenyl or (protected carboxy)phenyl group such 4-carboxyphenyl; a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-( N-methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl and the like. Preferred substituted phenyl groups include the 2- and 3-trifluoromethylphenyl, the 4-hydroxyphenyl, the 2-aminomethylphenyl and the 3-(N-(methylsulfonylamino))phenyl groups.

The term "arylalkyl" means one, two, or three aryl groups having the number of carbon atoms designated, appended to an alkyl radical having the number of carbon atoms designated including but not limited to; benzyl, napthylmethyl, phenethyl, benzhydryl (diphenylmethyl), trityl, and the like. A preferred arylalkyl group is the benzyl group.

The term "substituted C₆-C₁₀aryl-C₁-C₈alkyl" denotes a C₁-C₈alkyl group substituted at any carbon with a C₆-C₁₀aryl group bonded to the alkyl group through any aryl ring position and substituted on the C₁-C₈alkyl portion with one, two or three groups chosen from halogen (F, Cl, Br, l), hydroxy, protected hydroxy, amino, protected amino, C₁-C₇acyloxy, nitro, carboxy, protected carboxy, carbamoyl, carbamoyloxy, cyano, C₁-C₆alkylthio, N-(methylsulfonylamino) or C₁-C₄alkoxy. Optionally the aryl group may be substituted with one, two, or three groups chosen from halogen, hydroxy, protected hydroxy, nitro, C₁-C₆alkyl, C₁-C₄alkoxy, carboxy, protected carboxy, carboxymethyl, protected carboxymethyl, hydroxymethyl, protected hydroxymethyl, aminomethyl, protected aminomethyl, or an N-(methylsulfonylamino) group. As before, when either the C₁-C₈alkyl portion or the aryl portion or both are disubstituted, the substituents can be the same or different.

Examples of the term "substituted C₆-C₁₀aryl-C₁-C₈alkyl" include groups such as 2-phenyl-1 -chloroethyl, 2-(4-methoxyphenyl)ethyl, 2,6-dihydroxy-4-phenyl(n-hexyl), 5-cyano-3-methoxy-2-phenyl(n-pentyl), 3-(2,6-dimethylphenyl)n-propyl, 4-chloro-3-aminobenzyl, 6-(4-methoxyphenyl)-3-carboxy(n-hexyl), 5-(4-aminomethyl phenyl)-3-(aminomethyl)(n-pentyl), and the like.

The term "carboxy-protecting group" as used herein refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl,4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, β-(trimethylsilyl)ethyl, β-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the benzodiazepinedione molecule and can be removed at the appropriate point without disrupting the remainder of the molecule. In particular, it is important not to subject the carboxy-protected benzodiazepinedione molecule to strong nucleophilic bases or reductive conditions employing highly activated metal catalysts such as Raney nickel. (Such harsh removal conditions are also to be avoided when removing amino-protecting groups and hydroxy-protecting groups, discussed below.) Preferred carboxylic acid protecting groups are the allyl and p-nitrobenzyl groups. Similar carboxy-protecting groups used in the cephalosporin, penicillin and peptide arts can also be used to protect a carboxy group substituents of the benzodiazepinedione. Further examples of these groups are found in E. Haslam, "Protective Groups in Organic Chemistry", J. G. W. McOmie, Ed., Plenum Press, New York, N.Y., 1973, Chapter 5, and T.W. Greene, "Protective Groups in Organic Synthesis", John Wiley and Sons, New York, NY, 1981, Chapter 5. The term "protected carboxy" refers to a carboxy group substituted with one of the above carboxy-protecting groups.

As used herein the term "amide-protecting group" refers to any group typically used in the peptide art for protecting the peptide nitrogens from undesirable side reactions. Such groups include p-methoxyphenyl, 3,4-dimethoxybenzyl, benzyl, O-nitrobenzyl, di-(p-methoxyphenyl)methyl, triphenylmethyl, (p-methoxyphenyl)diphenylmethyl, diphenyl-4-pyridylmethyl, m-2-(picolyl)-N'-oxide, 5-dibenzosuberyl, trimethylsilyl, t-butyl dimethylsilyl, and the like. Further descriptions of these protecting groups can be found in "Protective Groups in Organic Synthesis", by Theodora W. Greene, 1981, John Wiley and Sons, New York.

Unless otherwise specified, the terms "heterocyclic group" or "heterocyclic" or "HET" or "heterocyclyl" are used interchangeably as used herein refer to any mono-, bi-, or tricyclic saturated, unsaturated, or aromatic ring having the number of atoms designated where at least one ring is a 5-, 6- or 7-membered ring containing from one to four heteroatoms selected from the group nitrogen, oxygen, and sulfur preferably at least one heteroatom is nitrogen (*Lang's Handbook of Chemistry, supra*). Typically, the 5-membered ring has 0 to 2 double bonds and the 6- or 7-membered ring has 0 to 3 double bonds and the nitrogen or sulfur heteroatoms may optionally be oxidized, and any nitrogen heteroatom may optionally be quaternized. Included in the definition are any bicyclic groups where any of the above heterocyclic rings are fused to a benzene ring. Heterocyclics in which nitrogen is the heteroatom are preferred.

The following ring systems are examples of the heterocyclic (whether substituted or unsubstituted) radicals denoted by the term "heterocylic": thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, thiazinyl, oxazinyl, triazinyl, thiadiazinyl, oxadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl, tetrazolo[1,5-b]pyridazinyl and purinyl, as well as benzo-fused derivatives, for example benzoxazolyl, benzthiazolyl, benzimidazolyl and indolyl.

Heterocyclic 5-membered ring systems containing a sulfur or oxygen atom and one to three nitrogen atoms are also suitable for use in the instant invention. Examples of such preferred groups include thiazolyl, in particular thiazol-2-yl and thiazol-2-yl N-oxide, thiadiazolyl, in particular 1,3,4-thiadiazol-5-yl and 1,2,4-thiadiazol-5-yl, oxazolyl, preferably oxazol-2-yl, and oxadiazolyl, such as 1,3,4-oxadiazol-5-yl, and 1,2,4-oxadiazol-5-yl. A group of further preferred examples of 5-membered ring systems with 2 to 4 nitrogen atoms include imidazolyl, preferably imidazol-2-yl; triazolyl, preferably 1,3,4-triazol-5-yl; 1,2,3-triazol-5-yl, 1,2,4-triazol-5-yl, and tetrazolyl, preferably 1H-tetrazol-5-yl. A preferred group of examples of benzo-fused derivatives are benzoxazol-2-yl, benzthiazol-2-yl and benzimidazol-2-yl.

Further suitable specific examples of the above heterocylic ring systems are 6-membered ring systems containing one to three nitrogen atoms. Such examples include pyridyl, such as pyrid-2-yl, pyrid-3-yl, and pyrid-4-yl; pyrimidyl, preferably pyrimid-2-yl and pyrimid-4-yl; triazinyl, preferably 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl radicals, are a preferred group.

The substituents for the optionally substituted heterocyclic ring systems, and further examples of the 5- and 6-membered ring systems discussed above can be found in W. Druckheimer *et al*., U.S*.* Patent No. 4,278,793.

A particularly preferred group of "heterocyclics" or "HET" include; 1,3-thiazol-2-yl, 4-(carboxymethyl)-5-methyl-1,3-thiazol-2-yl, 4-(carboxymethyl)-5-methyl-1,3-thiazol-2-yl sodium salt, 1,2,4-thiadiazol-5-yl, 3-methyl-1,2,4-thiadiazol-5-yl, 1,3,4-triazol-5-yl, 2-methyl-1,3,4-triazol-5-yl, 2-hydroxy-1,3,4-triazol-5-yl, 2-carboxy-4-methyl-1,3,4-triazol -5-yl sodium salt, 2-carboxy-4-methyl-1,3,4-triazol-5-yl, 1,3-oxazol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-thiol-1,3,4-thiadiazol-5-yl, 2-(methylthio)-1,3,4-thiadiazol-5-yl, 2-amino-1,3,4-thiadiazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1 H-tetrazol-5-yl sodium salt, 2-methyl-1H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(n-oxide)-pyridaz-3-yl, 6-hydroxypyridaz-3-yl, 1 -methylpyrid-2-yl, 1-methylpyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-astriazin-3-yl, 2,5-dihydro-5-oxo-6-hydroxy-as-triazin-3-yl sodium salt, 2,5-dihydro-5-oxo-6-hydroxy-2-methyl-astriazin-3-yl sodium salt, 2,5-dihydro-5-oxo-6-hydroxy-2-methyl-as-triazin-3-yl, 2,5-dihydro-5-oxo-6-methoxy-2-methyl-as-triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5b]pyridazin-6-yl and 8-aminotetrazolo[1,5-b]-pyridazin-6-yl.

An alternative group of "heterocyclics" includes; 4-(carboxymethyl)-5-methyl-1,3-thiazol-2-yl, 4-(carboxymethyl)-5-methyl-1,3-thiazol-2-yl sodium salt, 1,3,4-triazol-5-yl, 2-methyl-1,3,4-triazol-5-yl, 1H-tetrazol-5-yl, 1-methyl-1 H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1 H-tetrazol-5-yl sodium salt, 1-(methylsulfonic acid)-1H-tetrazol-5-yl, 1-(methylsulfonic acid)-1H-tetrazol-5-yl sodium salt, 1,2,3-triazol-5-yl, 1,4,5,6-tetrahydro-5,6-dioxo-4-methyl-as-triazin-3-yl, 1,4,5,6-tetrahydro-4-(2-formylmethyl)-5,6-dioxo-as-triazin-3-yl, 2,5-dihydro-5-oxo-6-hydroxy-2-methyl-as-triazin-3-yl sodium salt, 2,5-dihydro-5-oxo-6-hydroxy-2-methyl-as-triazin-3-yl, tetrazolo[1,5-b]pyridazin-6-yl, and 8-aminotetrazolo[1,5-b]pyridazin-6-yl.

Bivalent radicals L (either L¹, L, or L³) whether branched or unbranched, derived from alkanes, alkenes, alkadienes, alkynes, alkadiynes, and arenes optionally containing O, N and/or S atoms, or homo- and heterocycles either aromatic or aliphatic, are designated by adding the suffix "ene" to the corresponding monovalent radical. Atoms bearing the free valences may include any C, O, N or S.

The phrase "negatively charged acidic moiety" as used to define the W moiety, is intended to embrace chemical groups which, when attached to the 7-member lactam ring of Formula I, confers acidic character to the compound of Formula I. "Acidic character" means proton-donor capability, that is, the capacity of the compound of Formula I to be a proton donor in the presence of a proton acceptor, such as water. Typically, the acidic group should be selected to have proton donor capability such that the compound of Formula I has a pKₐ in a range from about one to about twelve. More typically, the Formula I compound would have a pKₐ in a range from about two to about seven. Also typically, W will be a group that contains at least one acidic hydrogen. Optionally, W will be a prodrug that is capable of being converted, *in vivo,* into a negatively charged acidic moiety. An example of an acidic group containing at least one acidic hydrogen atom is the carboxyl group (-COOH). A preferred example of W in Formula I containing a carboxyl group is where R⁷ is -CH₂- and "w" is -COOH. There are many examples of equivalent acidic groups other than carboxyl group, that can be substituted into W to produce biologically equivalent compounds of Formula I. Such other acidic groups are collectively referred to as "bioisosteres of the carboxylate" or "acidic bioisoteres". Representative examples of such "basic bioisoteres" are set forth above under "w" (a) through (ap). Other specific examples of such acidic bioisoteres may be independently selected from acidic moieties consisting of CO₂H, CO₂CH₃, SH, CH₂SH, C₂H₄SH, PO₃H₂, NHSO₂CF₃, NHSO₂C₆F₅, SO₃H, CONHNH₂, CONHNHSO₂CF₃, CONHOCH₃, CONHOC₂H₅, CONHCF₃, OH, CH₂OH, C₂H₄OH, OPO₃H₂,OSO₃H, 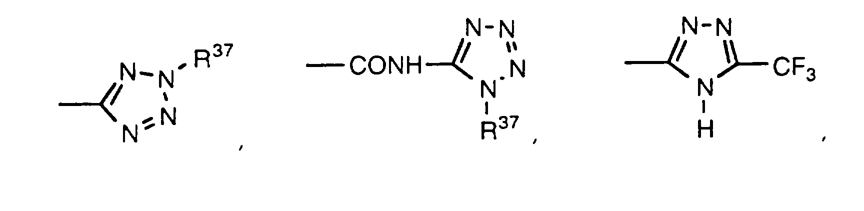 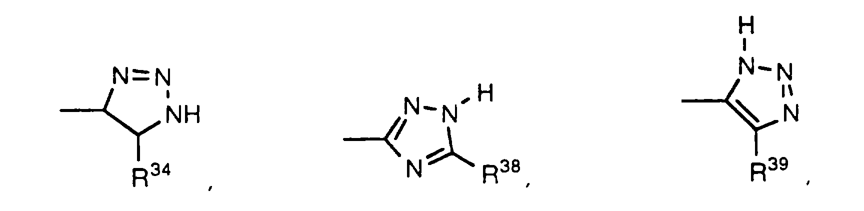 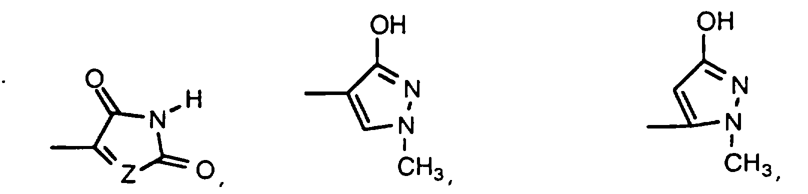 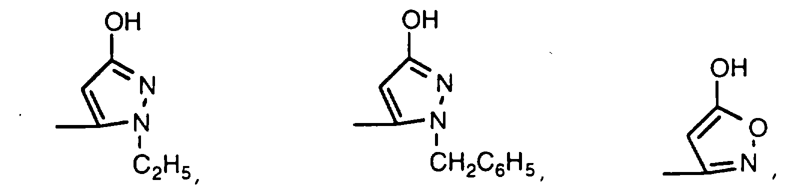 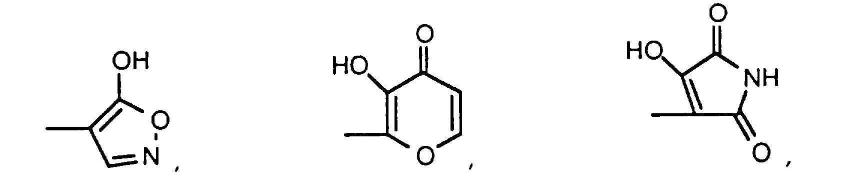 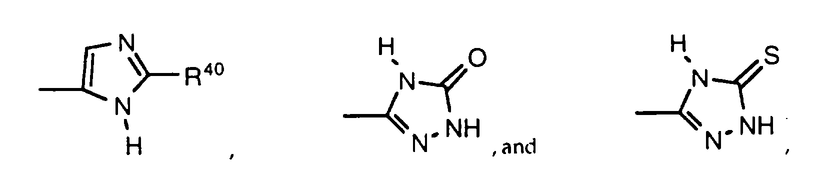 wherein each of R³⁷, R³⁸ R³⁹, and R⁴⁰ is independently selected from H, Cl, CN, NO₂, CF₃, C₂F₅, C₃F₇, CHF₂, CH₂F, CO₂CH₃, CO₂C₂H₅, SO₂CH₃, SO₂CF₃ and SO₂C₆F₅; wherein Z is selected from O, S, NR⁴¹ and CH₂; wherein R⁴¹ is selected from hydrido, CH₃ and CH₂C₆H₅; or a tautomer thereof or a pharmaceutically-acceptable salt thereof.

"Pharmaceutically acceptable salts" include both acid and base addition salts.

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic non-toxic bases are isopropylamine, diethylamine, ethanolamine, trimethamine, dicyclohexylamine, choline, and caffeine.

The term "prodrug" as used herein means a derivative of a parent drug molecule that enhances pharmaceutically desirable characteristics or properties (e.g. transport, bioavailablity, pharmacodynamics, etc.) and that requires biotransformation, either spontaneous or enzymatic, within the organism to release the active parent drug.

### B. Utility

The present invention is the result of the unexpected discovery that substituted fused 7-membered "lactam" ring compounds defined by formula I, especially including substituted 3,4-dihydro-1*H*-1,4-benzodiazepine-2,5-diones (referred to hereafter as substituted benzodiazepinediones) are capable of acting as fibrinogen receptor antagonists, that is inhibiting both platelet aggregation and binding of fibrinogen to the platelet receptor, GPII_{b}IIIₐ, at concentrations comparable to many of the more potent anti-aggregatory peptides of the prior art ( see e.g. Ali *et al*. EP 341 915). Thus, the instant fibrinogen receptor antagonists are particularly useful when they inhibit the binding of fibrinogen to the GPII_{b}IIIₐ receptor with an lC₅₀ in a Fg/GPII_{b}IIIₐ ELISA as described herein of at least about 35nM and an lC₅₀ in a platelet aggregation (PA) assay, also described herein, of at least about 3µM. Since there is some variability in the values obtained for ELISA and PA assays in different laboratories, preferred fibrinogen receptor antagonists represented by Formulae I-VI will have IC₅₀'s for Fg/GPII_{b}IIIₐ ELISA's and PA assays equal to or greater than that of the prior art compound N-[3-(4-amidinobenzamido)benzoyl]-3-aminoproprionoic acid,

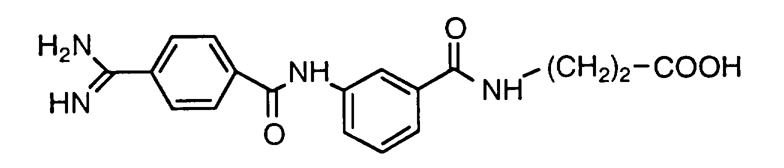

This compound is used as an internal standard and has been found to have an Fg/GPII_{b}IIIₐ ELISA lC₅₀ of about 25 nM and a PA inhibition assay lC₅₀ of about 3µM when measured according to the assays described herein. Most preferred fibrinogen receptor antagonists represented by Formulae l-VI have lC₅₀s in Fg/GPII_{b}IIIₐ ELISA of at least about 15 nM and in platelet aggregation inhibition assays of at least about 1µM or three times more potent in a platelet aggregation inhibition assay than the reference compound N-[3-(4-amidinobenzamido)benzoyl]-3-aminoproprionoic acid. This reference compound is readily prepared by standard methods from commercially available precursors (e.g. β-alanine, *m*-aminobenzoic acid, and *p*-amidinobenzoic acid) or may be synthesized according to the procedures set forth in EP 372 486 A2.

The compounds described in the present invention may therefore inhibit the binding of fibrinogen to its receptor on platelets, GPII_{b}IIIₐ, and thus prevent the aggregation of platelets and the formation of platelet plugs, emboli and thrombi in the circulatory system in mammals. Thromboembolic disorders have been shown to be directly related to the susceptibility of blood platelets to aggregate. Mammals exposed to medical procedures such as angioplasty and thrombolytic therapy are particularly susceptible to thrombus formation. The compounds of the present invention can be used to inhibit thrombus formation following angioplasty. They may also be used in combination with antithrombolytic agents such as tissue plasminogen activator and its derivatives (US patents 4,752,603; 4,766,075; 4,777,043; EP 199 574; EP 238 304; EP 228 862; EP 297 860; PCT WO89/04368; PCT WO89/00197), streptokinase and its derivatives, or urokinase and its derivatives to prevent arterial reocclusion following thrombolytic therapy. When used in combination with the above thrombolytic agents, the compounds of the present invention may be administered prior to, simultaneously with, or subsequent to the antithrombolytic agent. Mammals exposed to renal dialysis, blood oxygenation, cardiac catheterization and similar medical procedures as well as mammals fitted with certain prosthetic devices are also susceptible to thromboembolic disorders. Physiologic conditions, with or without known cause may also lead to thromboembolic disorders. Thus, the compounds described herein may be useful in treating thromboembolic disorders in mammals. The compounds described herein may also be used as adjuncts to anticoagulant therapy, for example in combination with aspirin, heparin or warfarin and other anticoagulant agents. The application of the compounds described herein for these and related disorders will be apparent to those skilled in the art.

Compounds represented by Formula I are also capable of antagonizing the interaction between vitronectin(Vn) and the vitronectin receptor (VnR) and thus are useful in situations where such antagonism is indicated. Vn-VnR antagonism is particularly pronounced when substituent Q¹-L¹- is bonded to position 8 or 9 of the substituted benzodiazapinedione, as represented by Formula Vla below. Thus, it is contemplated the instant compounds are useful in treating bone disorders such as osteoporosis.

Compounds of this invention are also useful as intermediates generally, or as precursors of inhibitors to GPII_{b}IIIₐ or other integrin receptors and thus in addition to treating cardiovascular and bone disease, these compounds may be usefully employed in metastatic disease, or for any disease where antagonism of RGD binding integrin receptors is indicated.

### C. Preferred Embodiments

### 1. Nonpeptidyl GPII_{b}IIIₐ Inhibitors

One embodiment of the invention comprises a compound represented by formula I capable of inhibiting binding of the platelet GPII_{b}IIIₐ receptor to its native *in vivo* ligands. Preferred nonpeptidyl inhibitors include compounds represented by structural formulae II-VI: 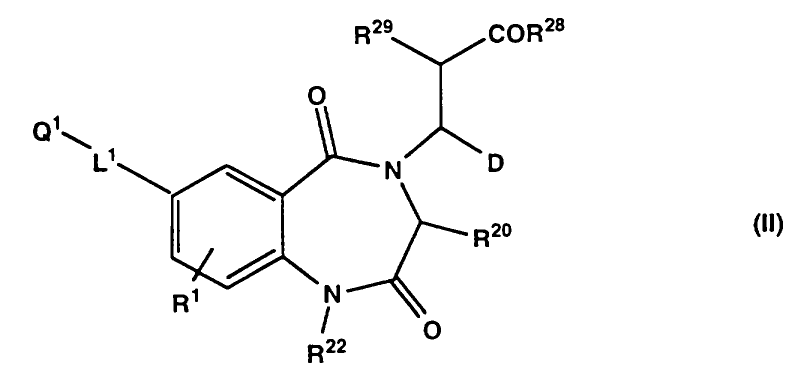 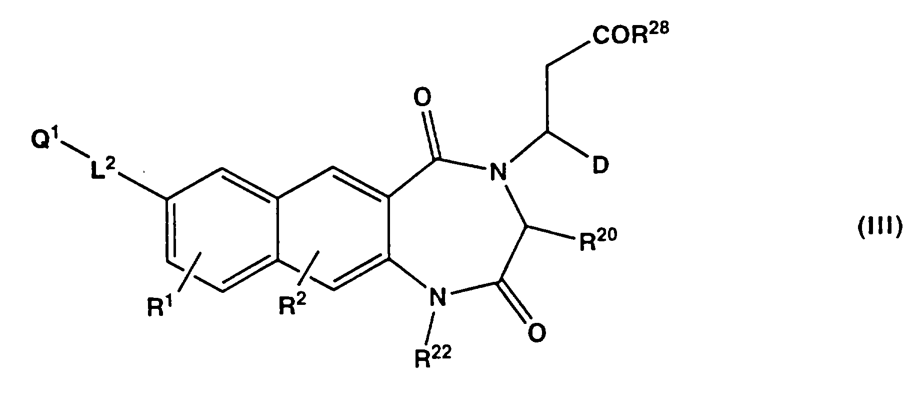 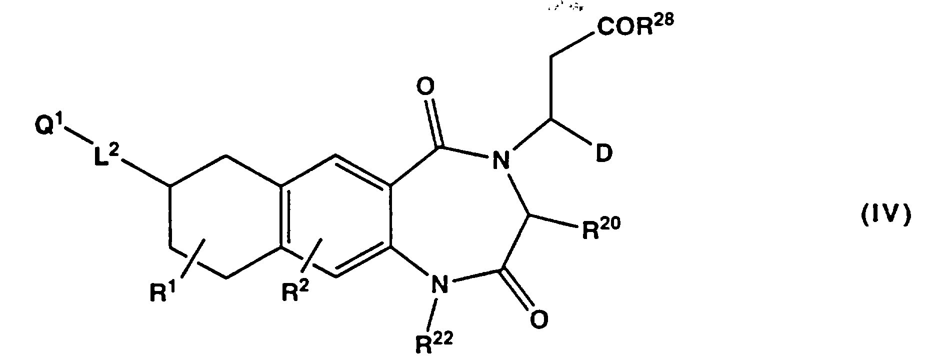 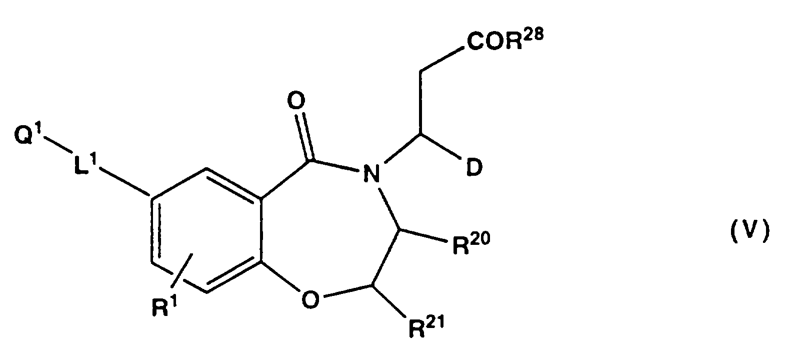 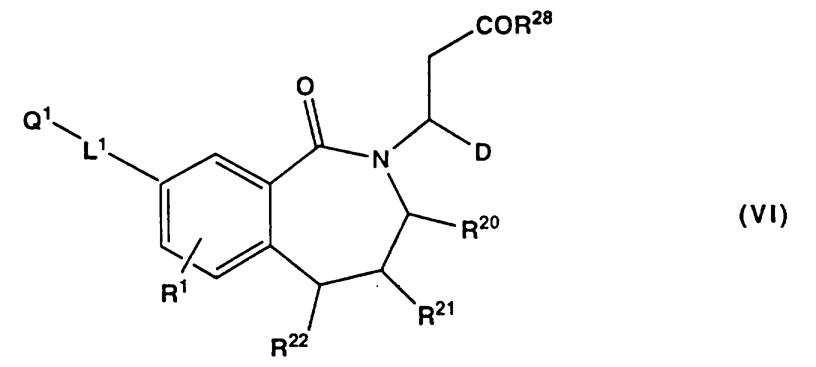 where R¹, R, R⁰, R¹,R, R⁸, R⁹, Q¹,L¹ and L are defined above.

Referring to formulae I-VI the following structural features of the instant nonpeptidyl antithrombotic inhibitors can be identified:
a. The positively charged Q moiety;
b. The linking moiety L;
c. The flat (usually aromatic) ring A;
d. The 7-member "lactam" ring containing TUG;
e. Substituents of T U G especially R;
f. The amino acid linking moiety D; and
g. The negatively charged acidic moiety W.

### a. Positively charged Q

Suitable groups Q (either Q¹ or Q) contain one or more nitrogen atoms and have a pK_{b} sufficiently high so that they are at least 10% positively charged at physiological pH. Q may be one or more primary, secondary, tertiary, or quartinary amines or imines either isolated or conjugated with other nitrogen atoms to form groups including but not limited to; aminomethyleneimino, amidino, and guanidino groups and multiples thereof. Alternatively, Q may be a saturated or unsaturated (including aromatic) heterocyclic group provided the group bears a positive charge at physiological pH. In one embodiment of the invention, Q is preferably selected from; amino (H₂N-), imino (=NH), amidino (H₂NC(=NH)-), aminomethyleneamino (H₂N-CH=N-), iminomethylamino (HN=CH-NH-), guanidino (H₂N-C(=NH)-NH-), N^{G}-aminoguanidino (H₂N-HN-C(=NH)-NH-), alkylamino (R¹NH-), dialkylamino (R¹₂N-), trialkylamino (R¹₃N-), alkylideneamino (R¹₂C=N-), pyranyl, pyrrolyl, imadazolyl, pyrazolyl, pyridyl, pyrazinyl, pyrimidinyl, pyridazinyl, indolizinyl, isoindolyl, 3H-indolyl, indolyl, 1H-indazolyl, purinyl, 4H-quinolizinyl, isoquinolyl, quinolyl, phthalazinyl, naphthyridinyl, quinoxalinyl, quinazolinyl, cinnolinyl, pteridinyl, 4aH-carbazolyl, carbazolyl, b-carbolinyl, phenanthridinyl, acridinyl, perimidinyl, phenanthrolinyl, phenazinyl, phenarsazinyl, phenothiazinyl, pyrrolinyl, imidazolidinyl, imidazolinyl, pyrazolidinyl, pyrazolinyl, piperidyl, piperazinyl, indolinyl, isoindolinyl, quinuclidinyl, morpholinyl, and 1,3-diazacyclohex-4-ene, where R¹ is selected from; hydrogen, C₁-C₆-alkyl, C₆-C₁₀-aryl, and C₃-C₁₀-cycloalkyl. Optionally, any of the nitrogen containing heterocycles described above may be substituted with amino, imino, amidino, aminomethyleneamino, iminomethylamino, guanidino, N^{G}-amino-guanidino, alkylamino, dialkylamino, trialkylamino, or alkylideneamino groups.

Exemplary preferred Q¹ groups include the following:
(A) Amino and ammonio groups;

   -NH₂ ,

   -NHCH₃, 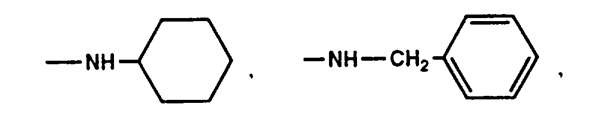 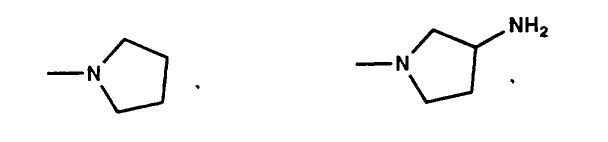 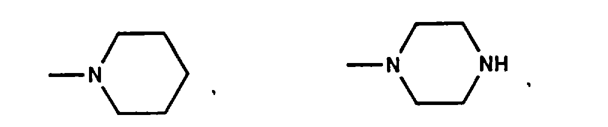 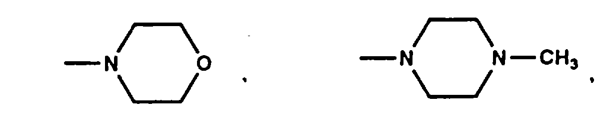 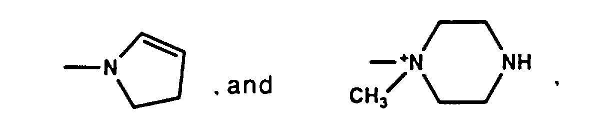
(B) Amidino groups; 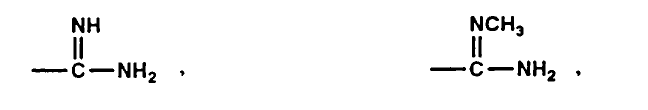 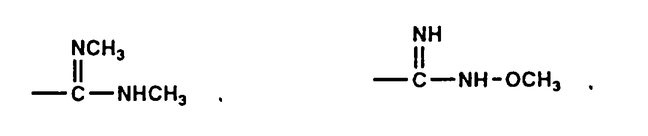 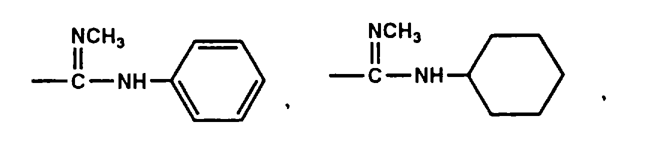 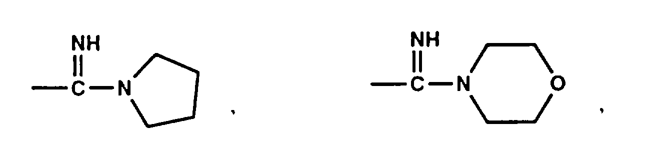 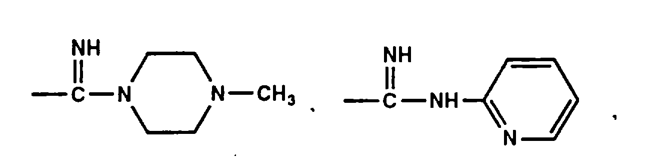 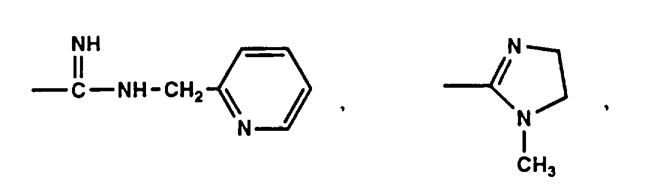 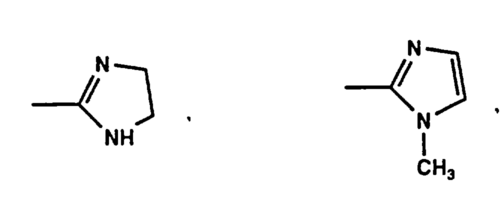 and 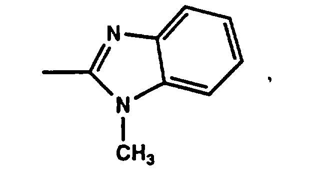
(C) Aminoalkyleneimino groups; 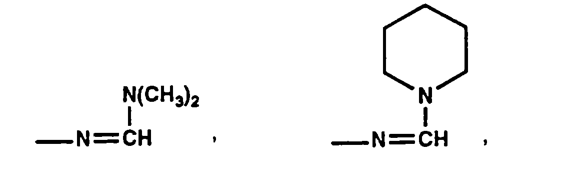 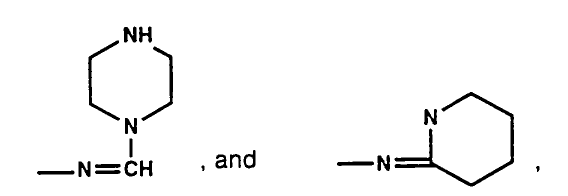
(D) Iminoalkyleneamino groups; 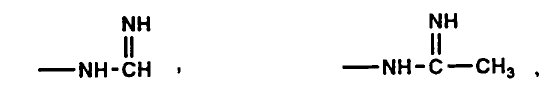 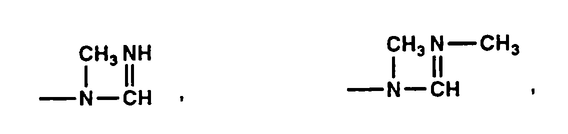 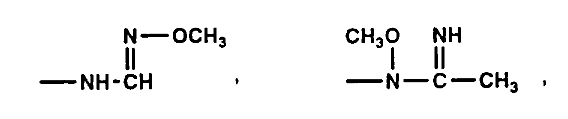 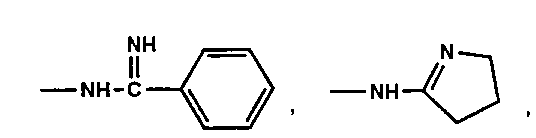 and 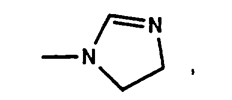
(E) Guanidino groups; 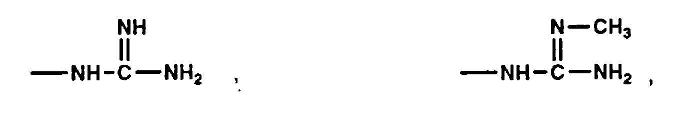 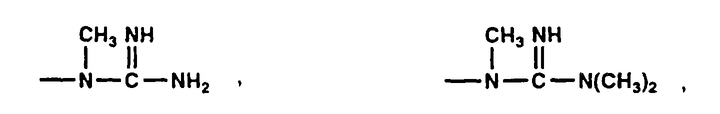 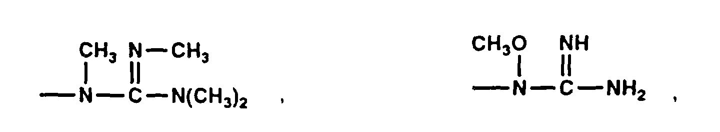 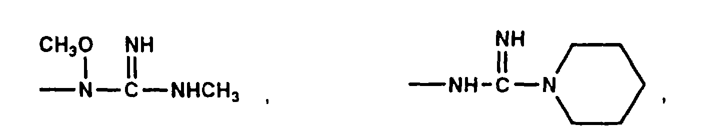 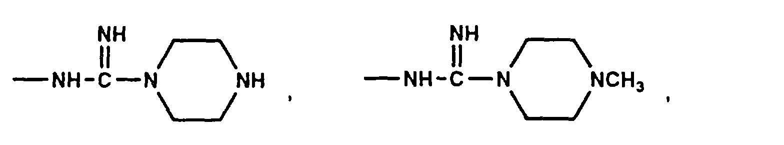 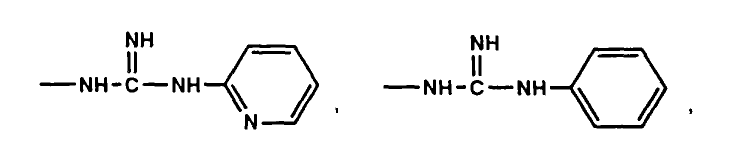 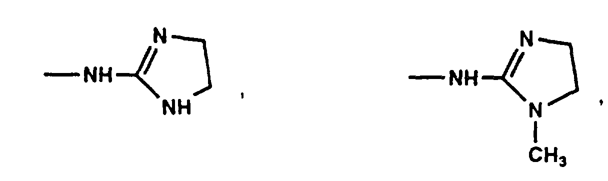 and 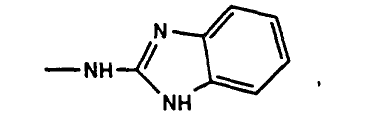
(F) Saturated heterocyclic groups; 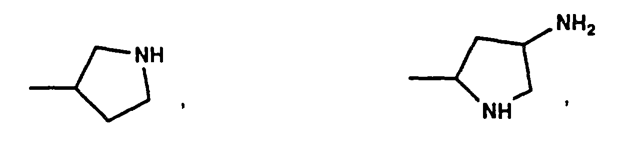 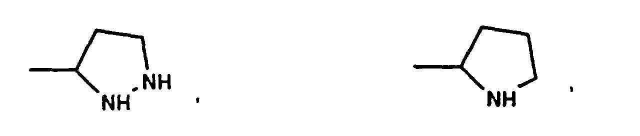 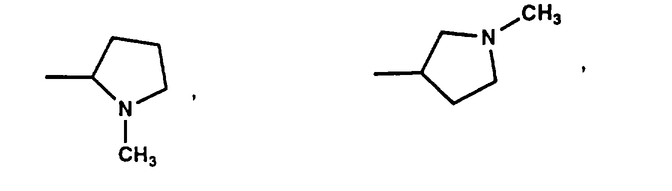 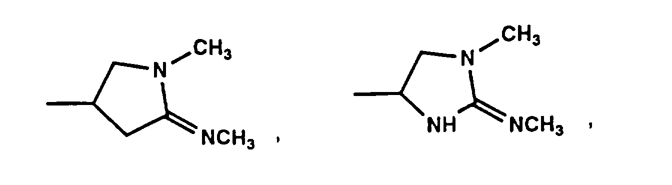 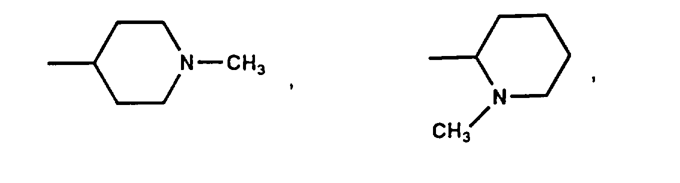 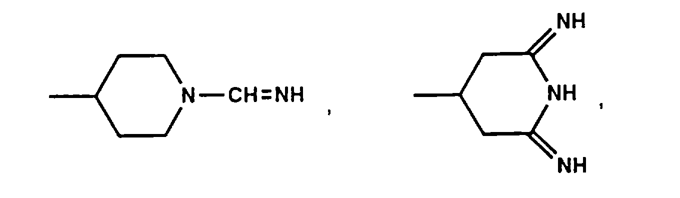 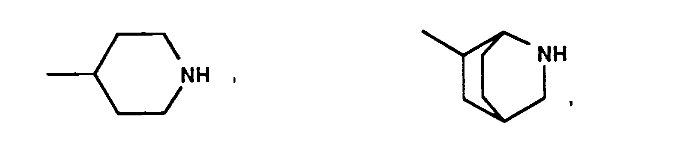 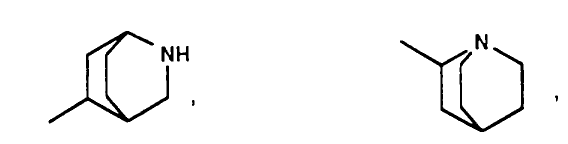 and 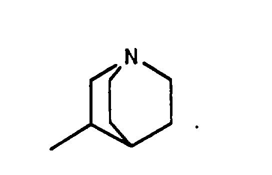
(G) Unsaturated (nonaromatic) heterocyclic groups; 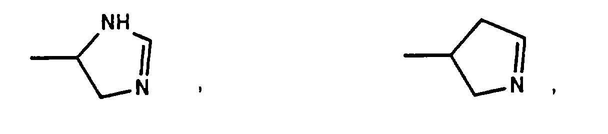 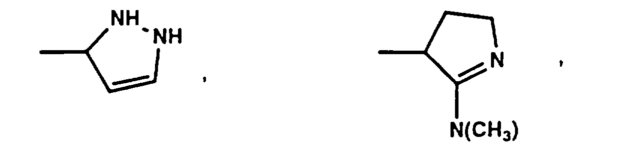 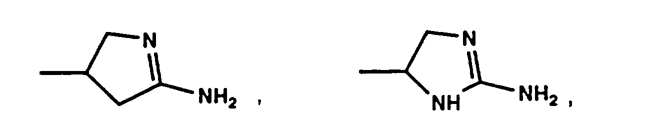 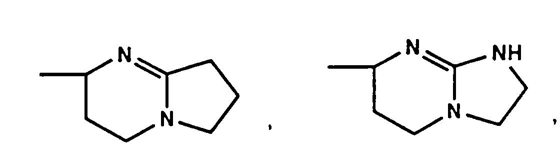 and 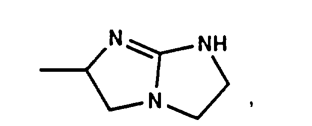
(H) Unsaturated aromatic heterocyclic groups: 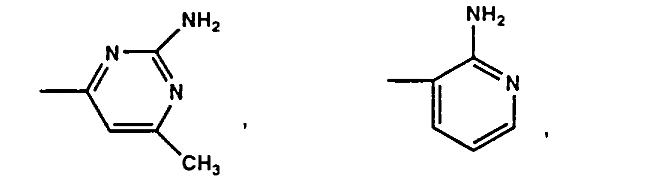 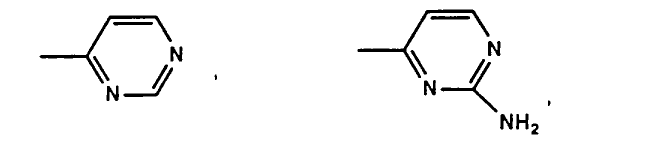 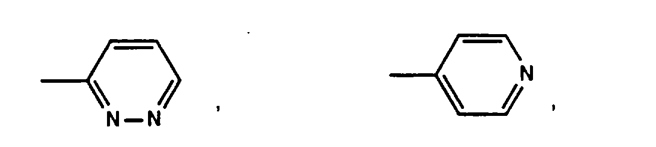 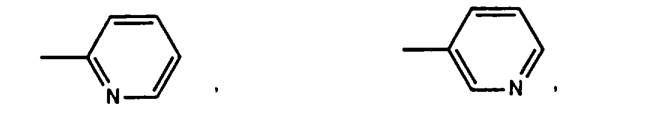 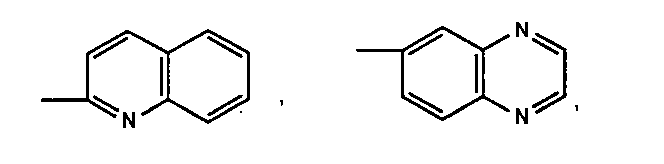 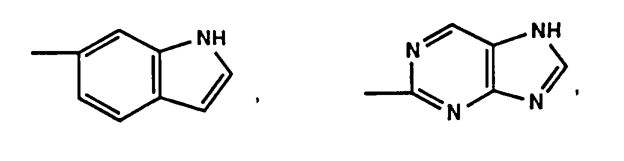 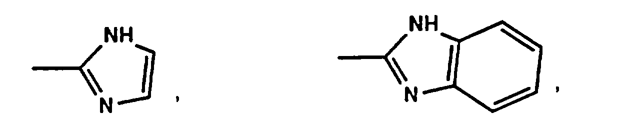 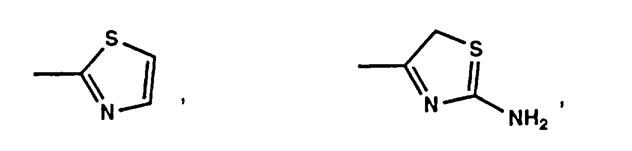 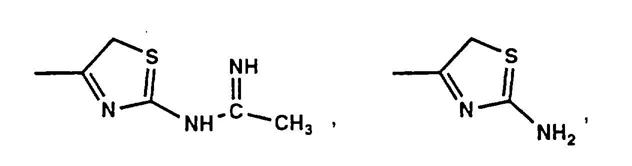 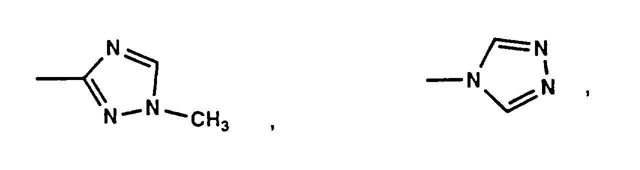 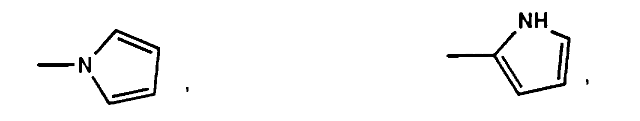 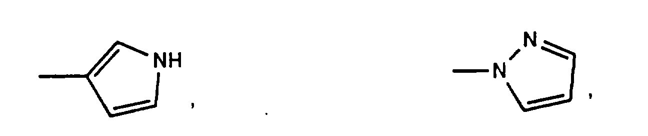 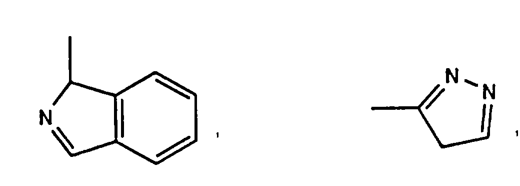 and 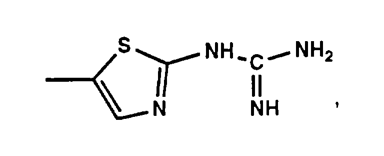
(I) Bicycloheterocyclic groups; 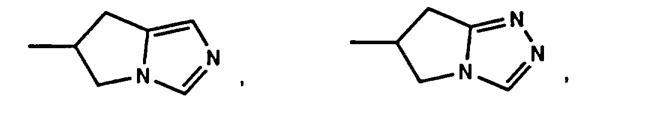 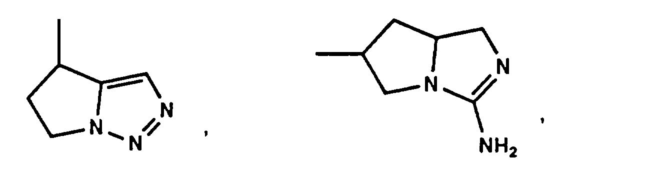 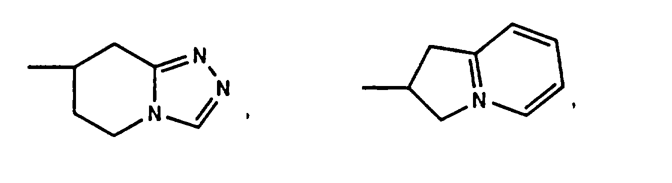 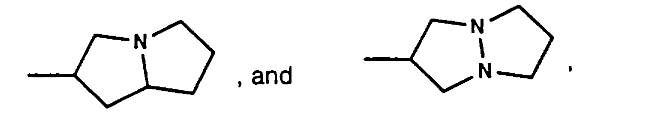
(J) Multiple amino, guanidino, and amidino groups;

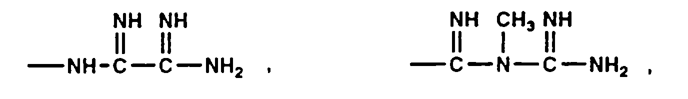

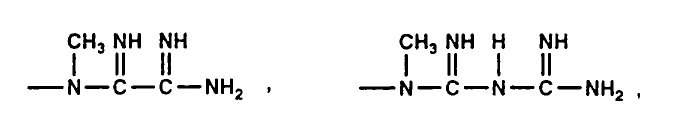

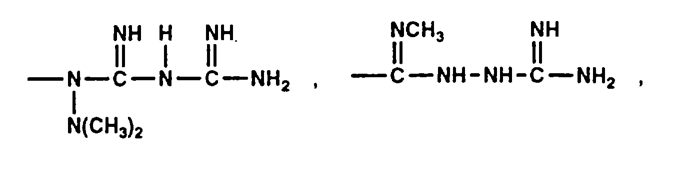

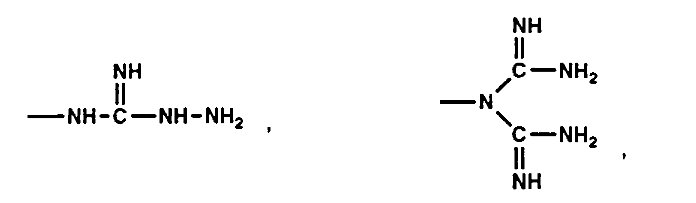

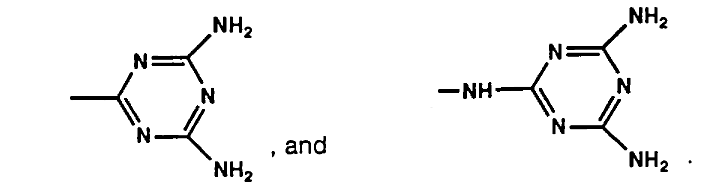

Most preferred Q groups are amino, amidino, and guanidino groups.

### b. The linking group L

The length of the bivalent radical L appears to be important to biological activity. By length is meant the distance between the A ring and the first charge bearing N of substituent Q. For example, when the A moiety is a 5 or 6-member ring [e.g. the benzene moiety of the benzodiazepinedione nucleus (carbon 7 of Formula II)], suitable lengths for bivalent radical L¹ range from about 3 to about 9 methylene equivalents. L¹ is therefore a bivalent radical containing from 3 to 9 methylene groups connecting Q to the number 7 carbon of the benzodiazepinedione nucleus (or an equivalent position on other 5 or 6-member A rings) where any methylene group or groups may be replaced with one or more or a combination of; alkene, alkyne, aryl, heterocycle or a functional group or groups containing the heteroatoms N, O, and S, so long as the overall length is equivalent to from 3 to 9 methylene groups. These functional groups include one or more of the following:

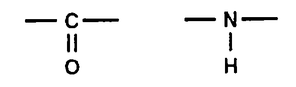

-O-

-S-

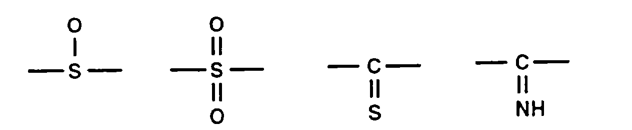

and may be isolated within the linker ( e.g. forming ethers, thioethers, ketones, sulfoxides and the like) or combined in any combination, provided only that the compounds so produced are stable in aqueous solution and do not exceed the above stated length requirements. For example, combining these functional groups produces esters, amides, ureidos, carbamates, carbonates, sulfonamides, sulfoxides, sulfones, and the like. Preferred lengths for L¹ are from 4 to 6 while most preferred lengths are about 5 methylene equivalents. In counting atoms comprising L¹, only those atoms sequentially linking Q with ring A are counted except when a homo- or heterocycle comprises L¹ in which case the fewest number of atoms separating these moieties are counted. Furthermore, given that a bivalent radical is of appropriate length, it is preferred that it be somewhat rigid, that is, contain one or more sp or sp atoms.

In the description that follows, the free valence to the left of the page is bonded to Q, while the free valence to the right is bonded to carbon 7 of the compound of formula II (or an equivalent position on other 5 or 6-member A rings) Preferred L¹'s are selected from substituted or unsubstituted; C₃-C₇-alkylene, C₃-C₇-cycloalkylene, C₃-C₇-alkenylene, C₄-C₇-cycloalkenylene, C₅-C₈-cycloalkadienylene, C₃-C₇-alkadienylene, C₃-C₇-alkynylene, C₄-C₇-alkenynylene, C₆-C₁₄-arylene, C₆-C₁₄-aryl-C₂-C₄-alkynylene, C₁-C₃-alkyl-C₆-C₁₄-aryl-C₂-C₄-alkynylene, C₆-C₁₄-aryl-C₂-C₄-alkenylene, C₁-C₃-alkyl-C₆,-C₁₄-arylene, C₁-C₃-alkyl-C₆-C₁₄-aryl-C₂-C₄-alkenylene, C₆-C₁₄-aryl-C₁-C₃-alkylene, C₆-C₁₄-aryl-C₁-C₃-alkyloxyene, C₁-C₃-alkyl-C₆-C₁₄-aryl-C₁-C₂-alkylene, C₁-C₃-alkyloxy-C₆-C₁₄-arylene, C₂-C₈-alkyloxyene, C₁-C₅-alkyloxy-C₁-C₅-alkylene, C₆-C₁₀-aryloxyene, C₆-C₁₀-aryloxy-C₁-C₅-alkylene, C₆-C₁₀-arylthio-C₁-C₅-alkylene,

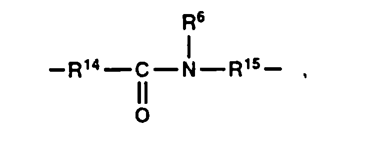

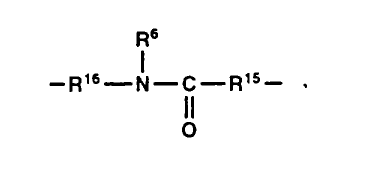

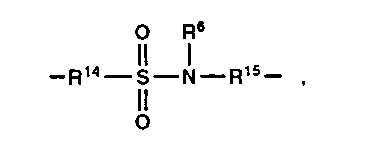

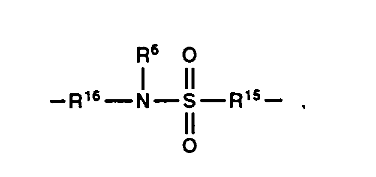

-R¹⁴-S-R¹⁵-,

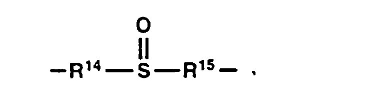

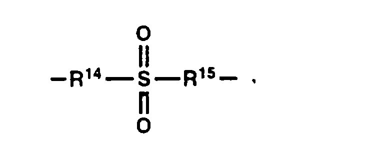

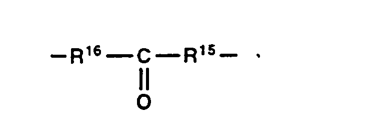

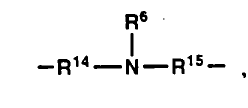

and

-R¹⁴-O-R¹⁵- ,

where R¹⁴ is selected from; a chemical bond, C₁-C₈-alkyl, C₃-C₇-cycloalkyl C₂-C₅-alkenyl, C₃-C₅-alkynyl, C₆-C₁₀-aryl, C₁-C₃-alkyl-C₆-C₁₂-aryl, C₁-C₂-alkyl-C₆-C₁₀-aryl-C₁-C₂-alkyl, C₆-C₁₀-aryl-C₁-C₂-alkyl, and C₆-C₁₀-aryloxy-C₁-C₂-alkyl. R¹⁵ is selected from; a chemical bond, C₁-C₄-alkyl, C₂-C₄-alkenyl, C₂-C₄-alkynyl, C₆-C₁₀-aryl, and C₁-C₃-alkyl-C₆-C₁₂-aryl. R¹⁶ is selected from; a chemical bond, C₁-C₅-alkyl, C₃-C₇-cycloalkyl C₃-C₅-alkenyl, C₃-C₅-alkynyl, C₆-C₁₀-aryl, C₁-C₃-alkyl-C₆-C₁₂-aryl, and C₆-C₁₀-aryl-C₁-C₂-alkyl. The substituents are preferably selected from one to three R¹ defined above.

More preferred bivalent radicals L¹ are selected from the following groups:

### Group 1

- CH₂- C≡C-,

- CH₂-CH₂-C≡C-,

- CH₂-CH₂-CH₂- C≡C - ,

- CH₂-CH₂-CH₂-CH₂- C≡C- ,

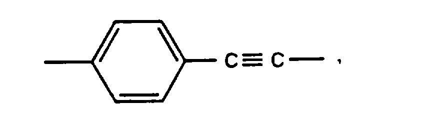

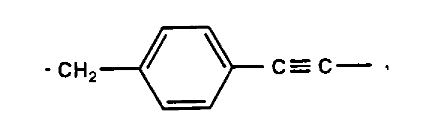

### Group 2

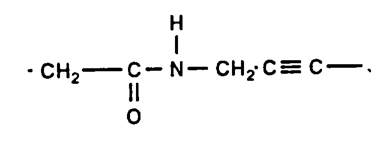

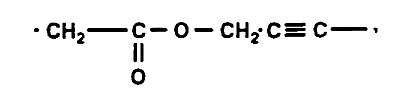

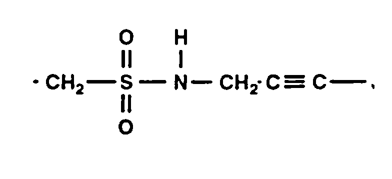

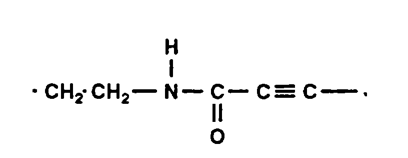

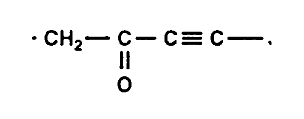

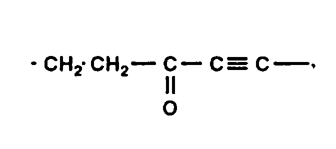

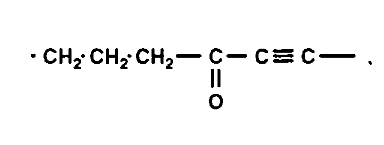

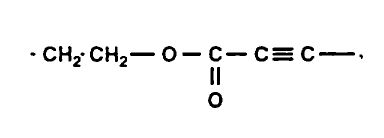

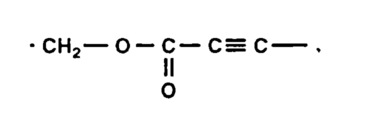

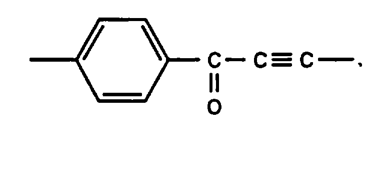

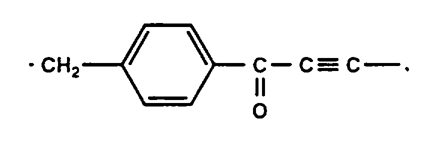

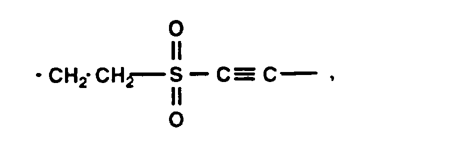

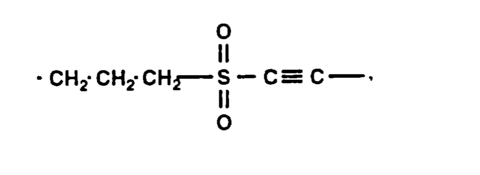

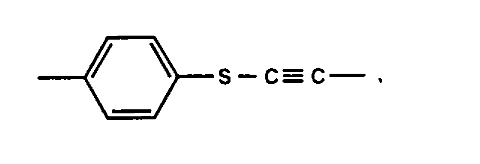

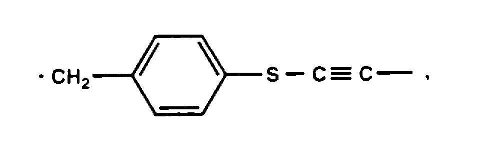

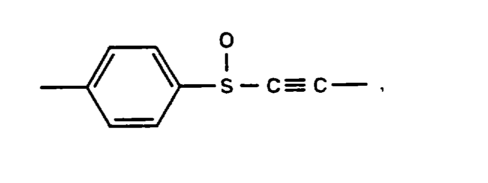

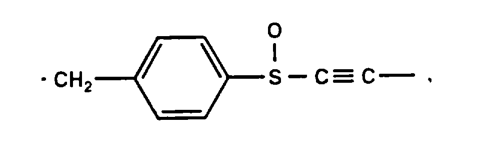

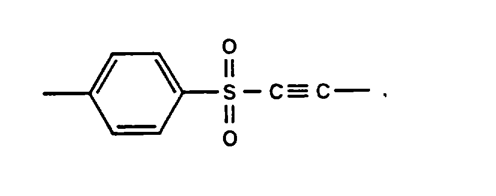

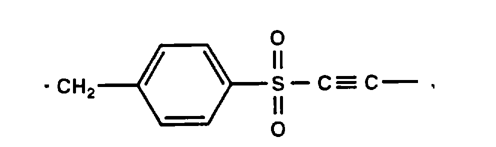

### Group 3

- CH₂-CH₂-CH₂- ,

- CH₂-CH₂-CH₂-CH₂-,

- CH₂-CH₂-CH₂-CH₂-CH₂-,

- CH₂-CH₂-CH₂-CH₂-CH₂-CH₂-,

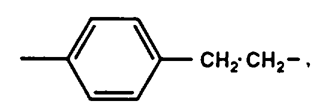

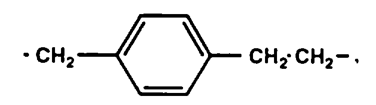

### Group 4

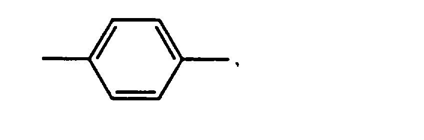

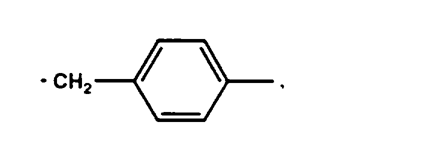

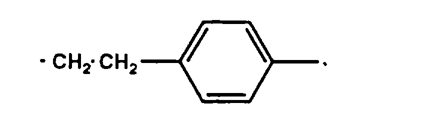

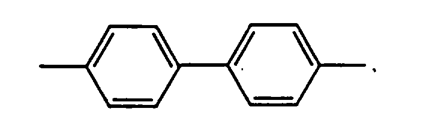

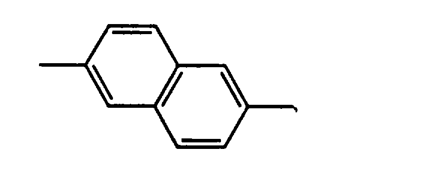

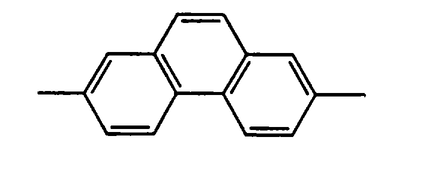

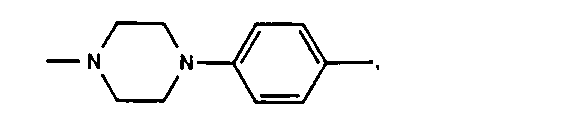

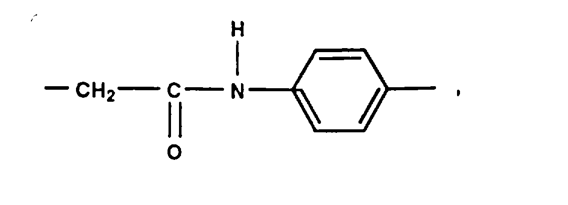

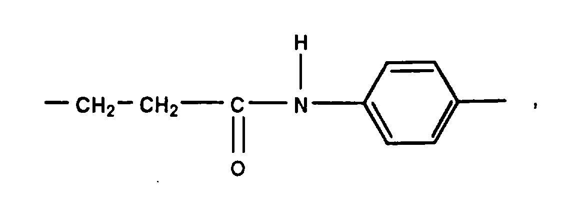

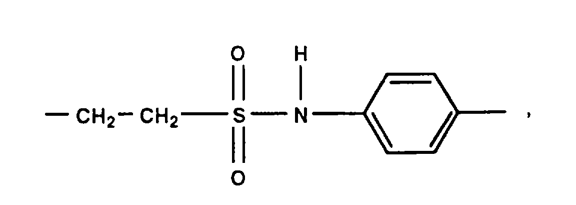

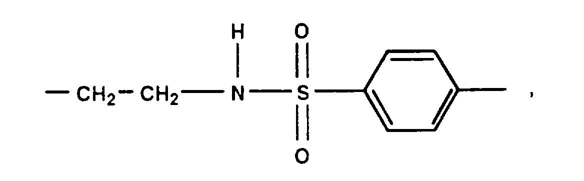

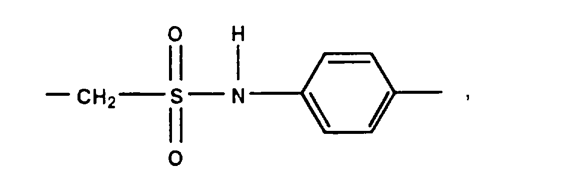

### Group 5

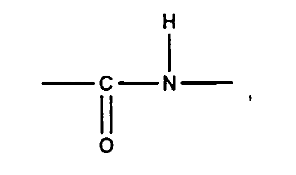

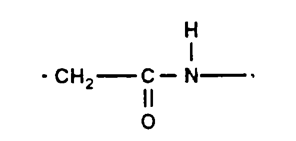

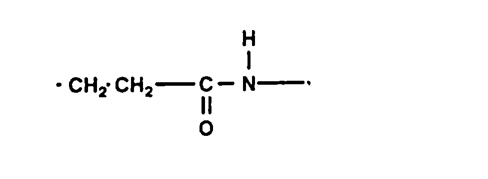

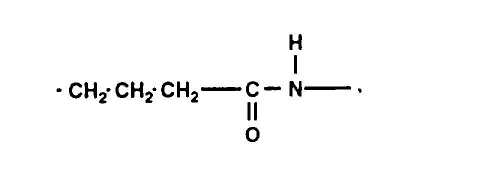

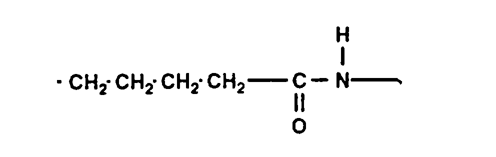

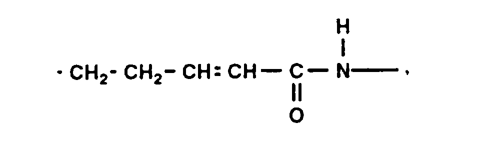

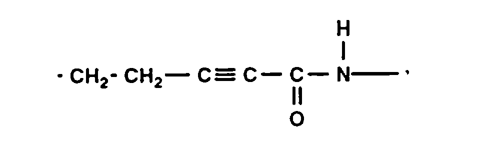

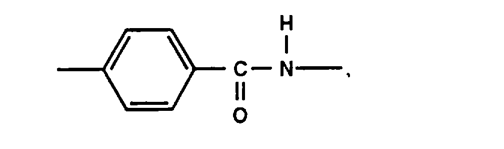

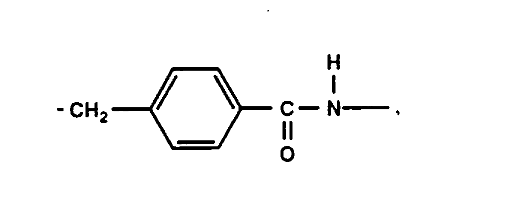

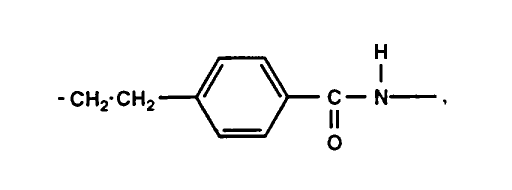

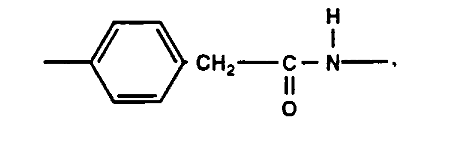

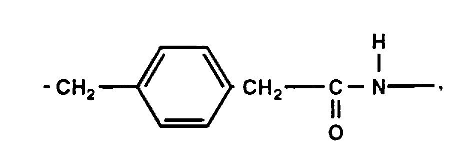

### Group 6

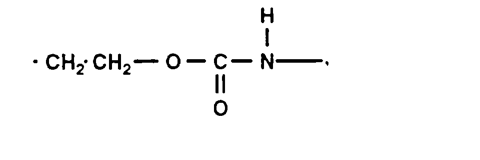

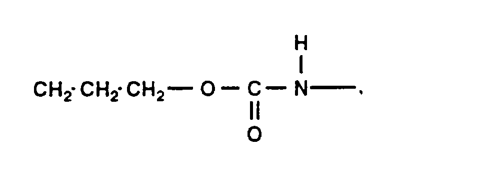

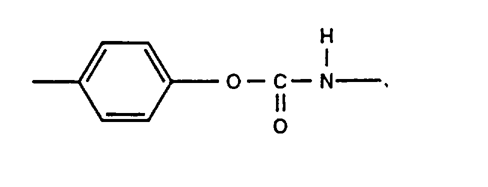

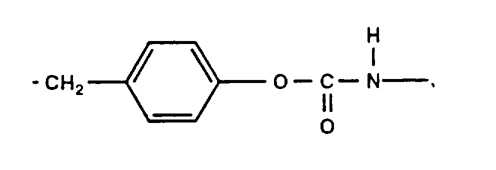

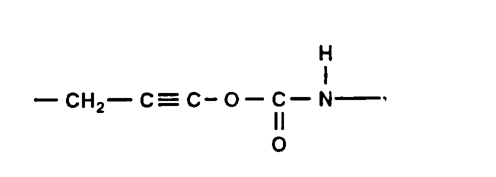

### Group7

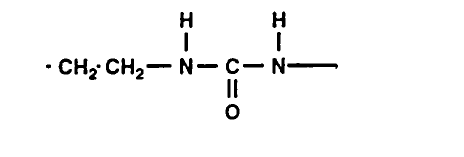

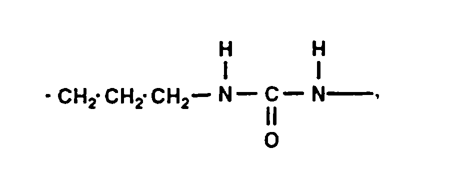

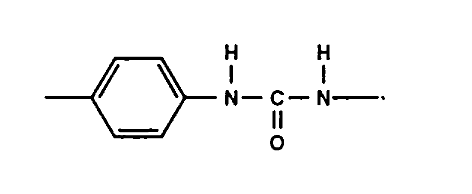

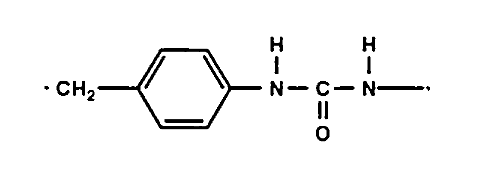

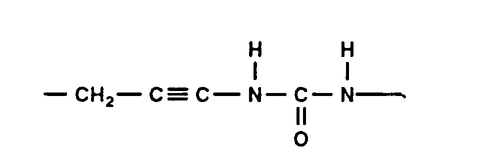

### Group 8

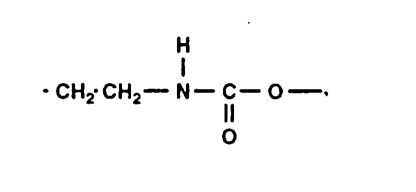

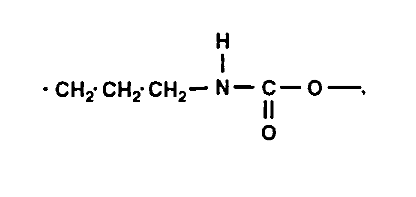

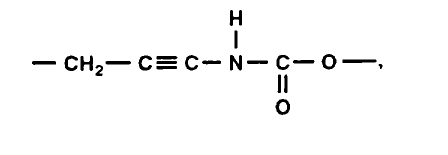

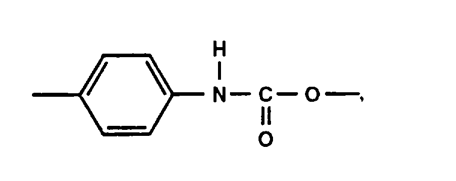

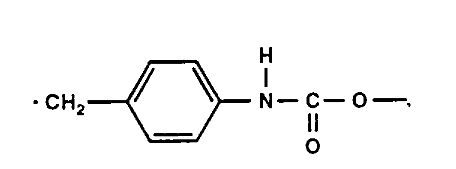

### Group 9

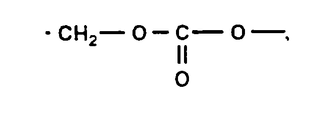

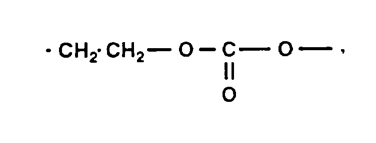

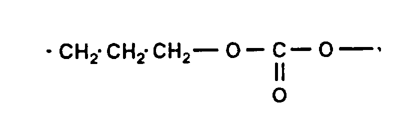

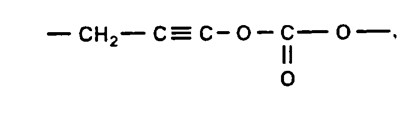

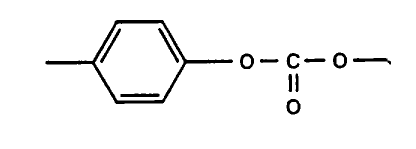

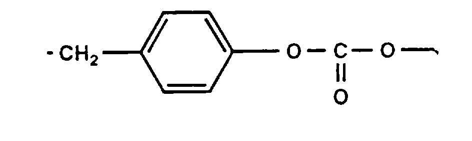

### Group 10

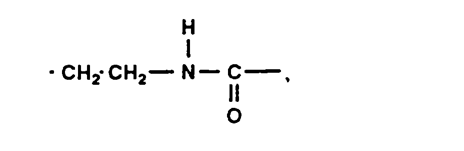

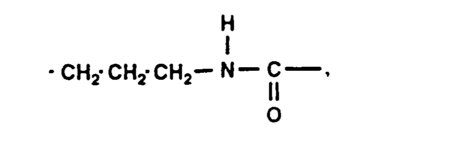

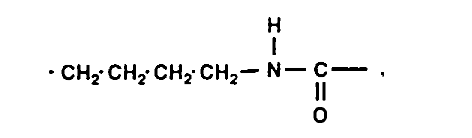

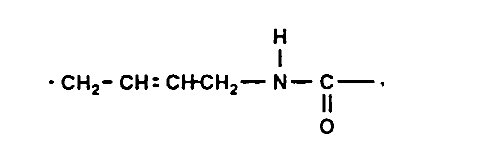

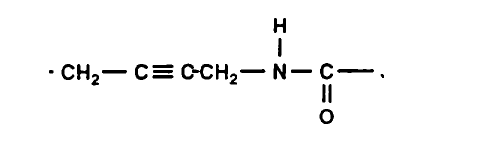

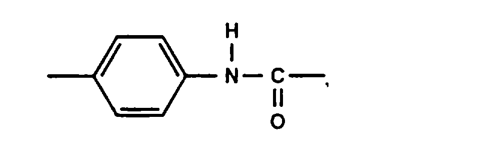

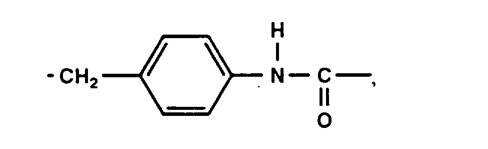

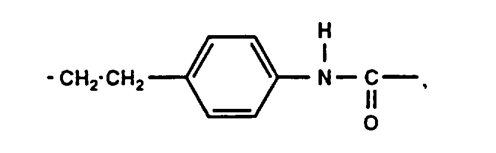

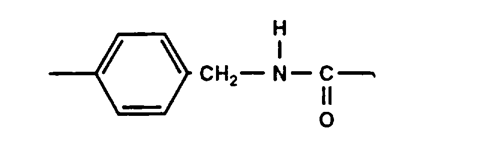

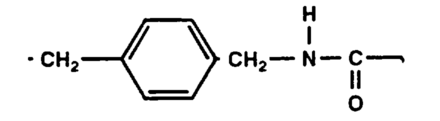

### Group 11

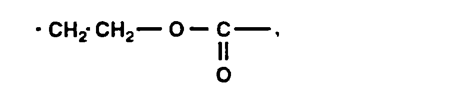

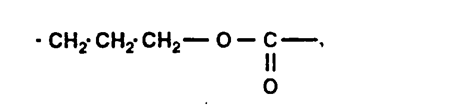

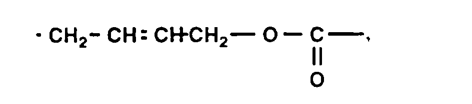

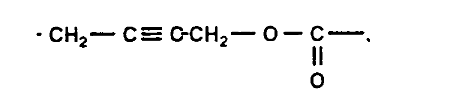

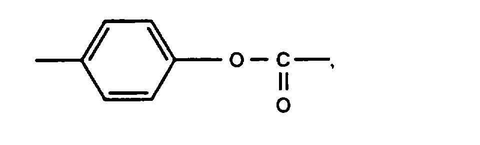

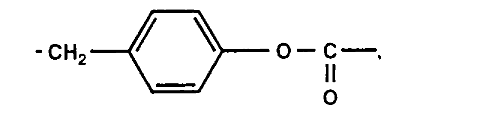

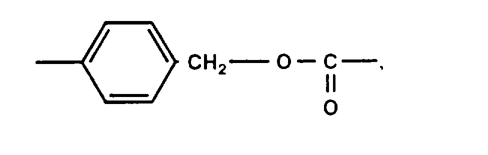

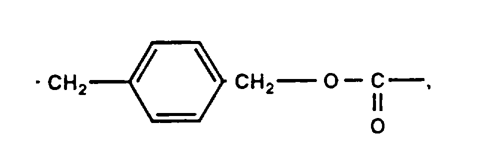

### Group 12

- CH₂- CH₂- O -,

- CH₂- CH₂- CH₂- O -,

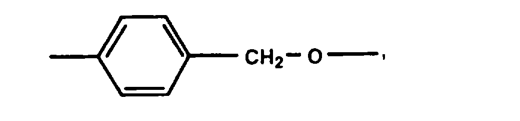

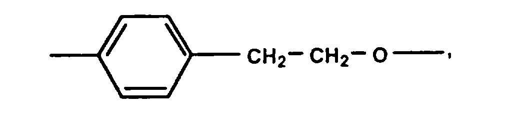

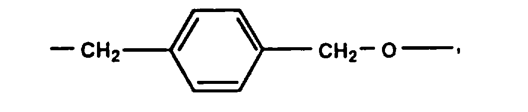

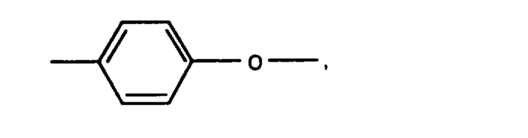

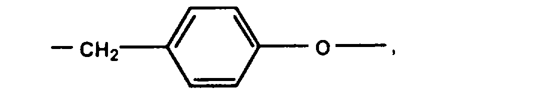

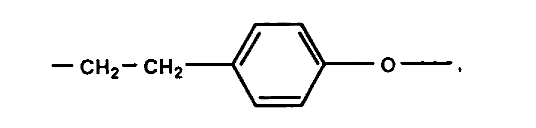

### Group 13

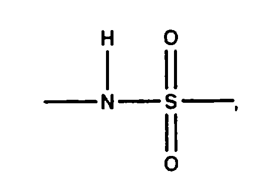

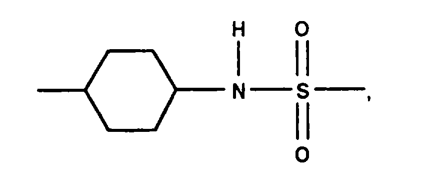

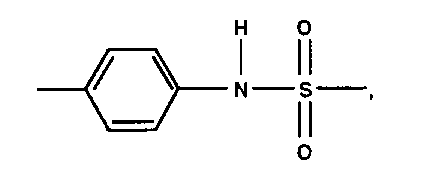

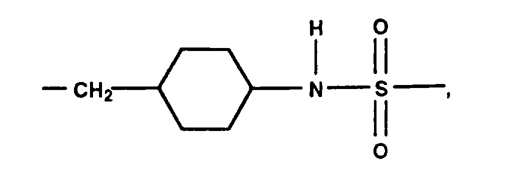

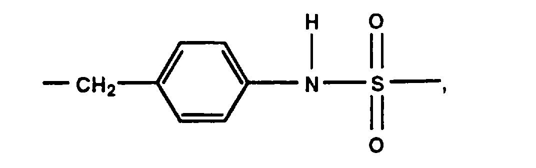

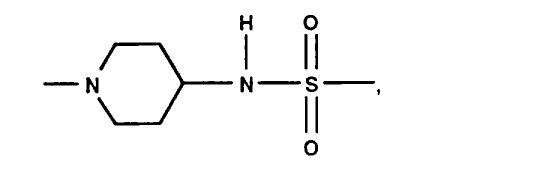

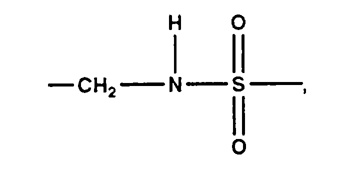

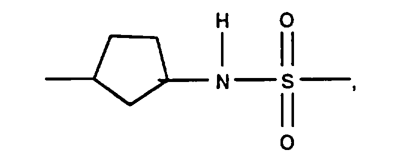

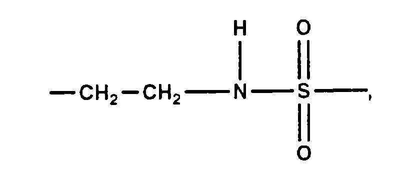

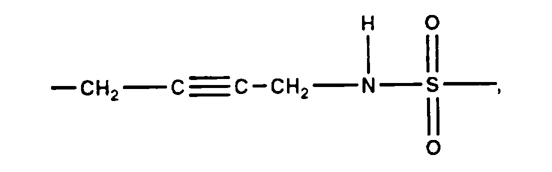

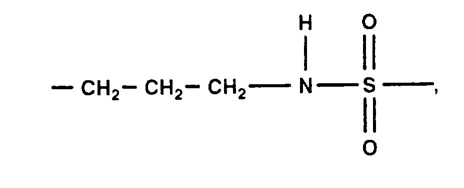

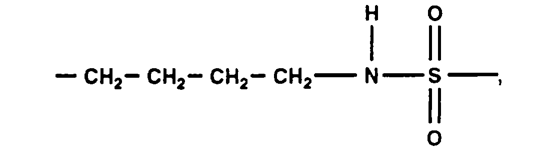

### Group 14

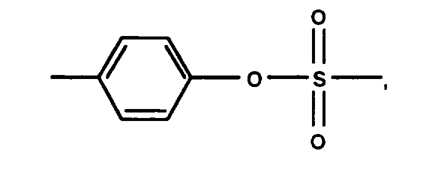

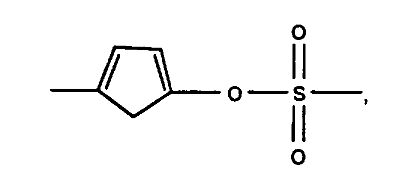

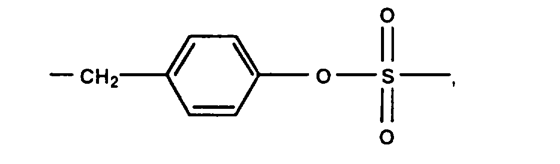

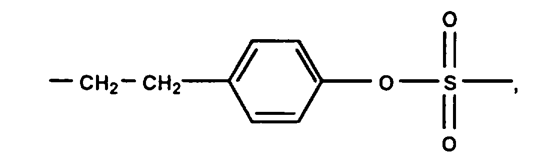

### Group 15

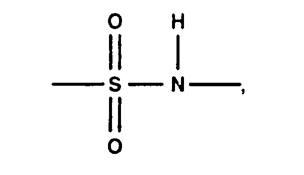

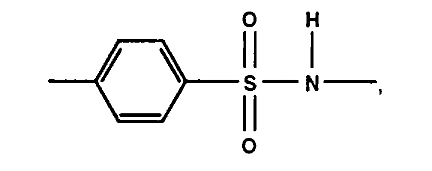

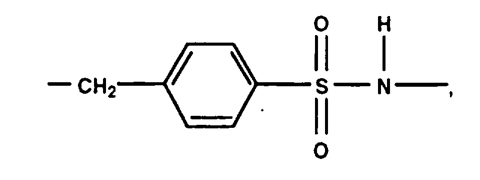

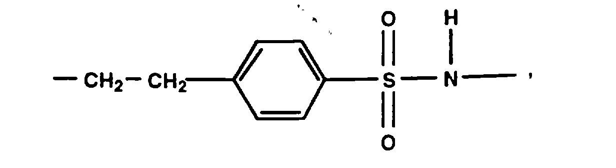

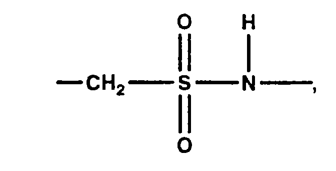

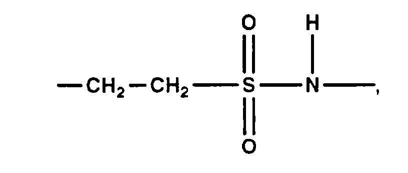

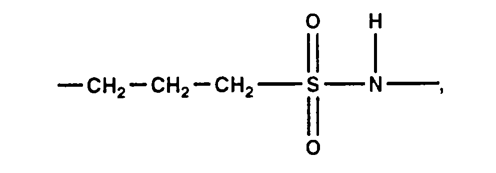

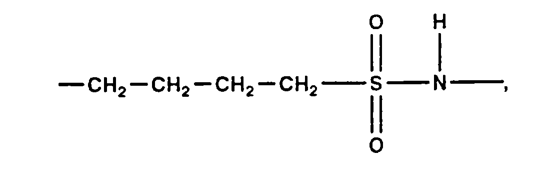

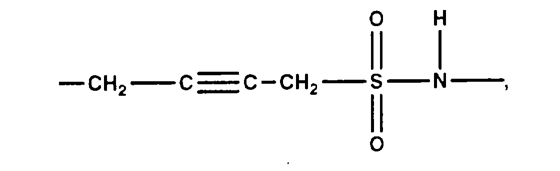

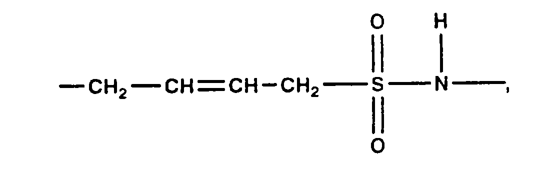

### Group 16

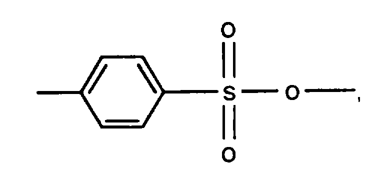

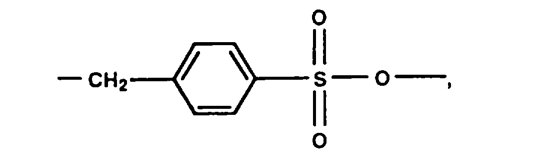

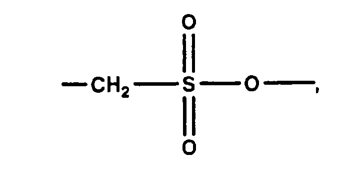

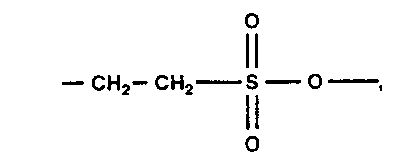

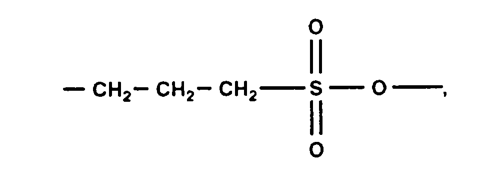

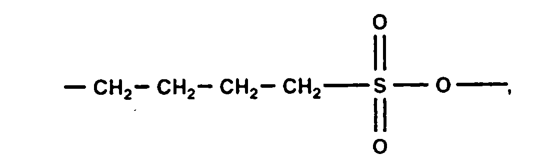

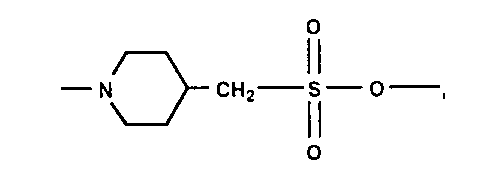

### Group 17

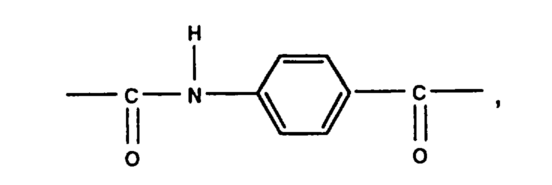

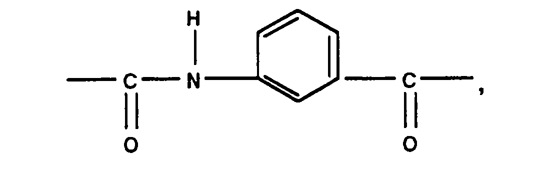

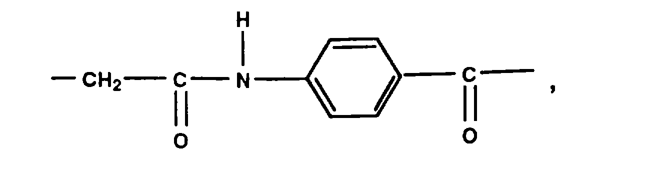

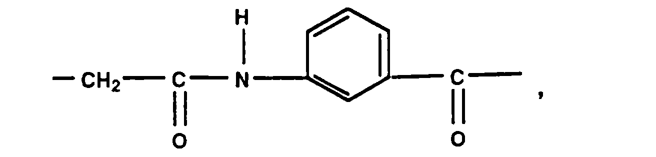

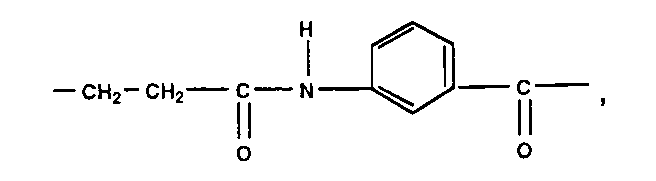

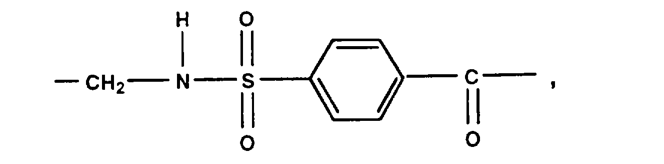

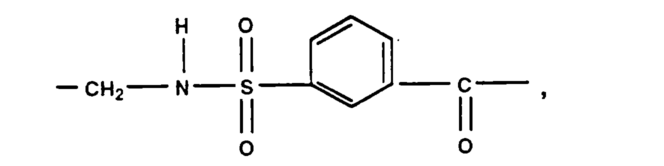

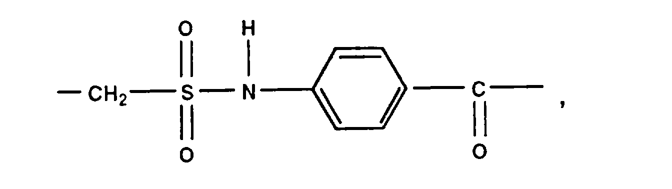

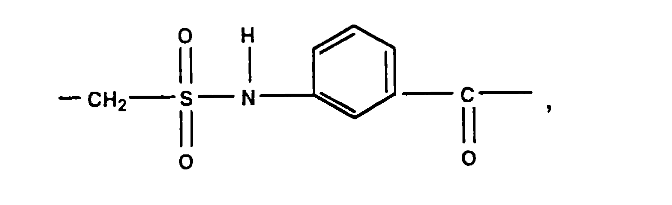

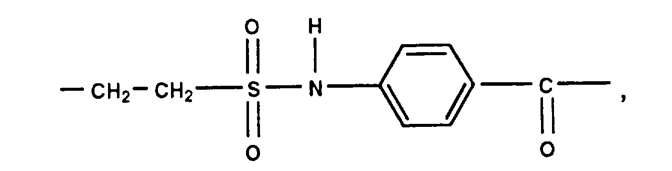

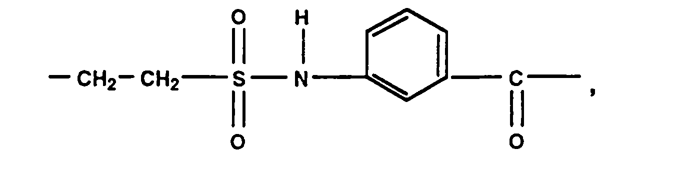

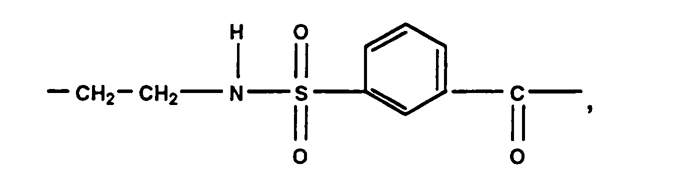

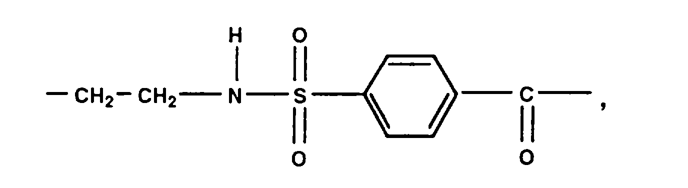

### Group 18

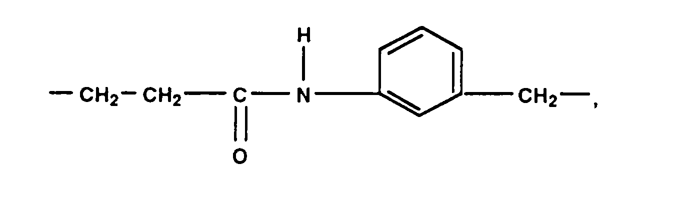

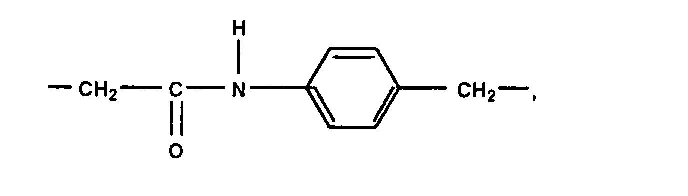

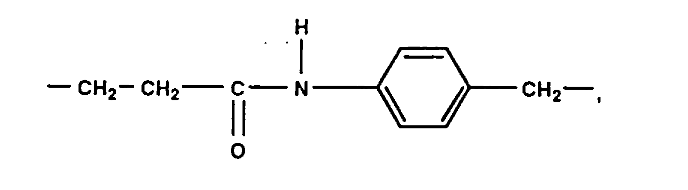

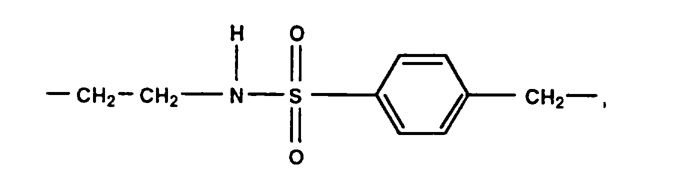

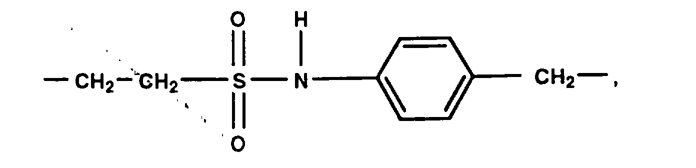

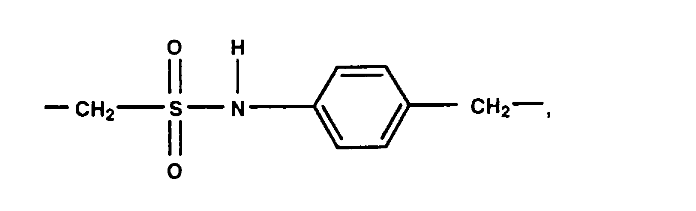

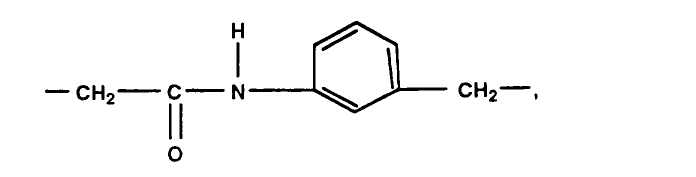

Most preferred L¹'s are selected from;

-(CH₂)_{q}-R¹⁵- ,

-(CH₂)ᵣ-C≡C-R¹⁵- ,

-(CH₂)ᵣ-CH=CH-R¹⁵- ,

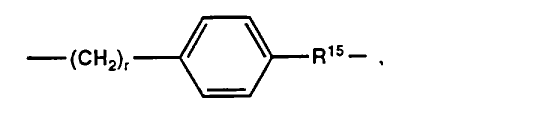

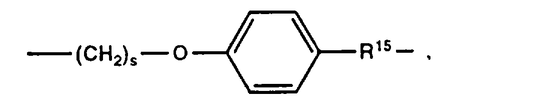

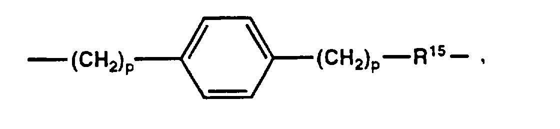

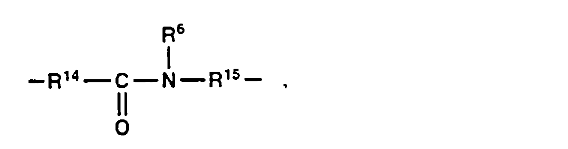

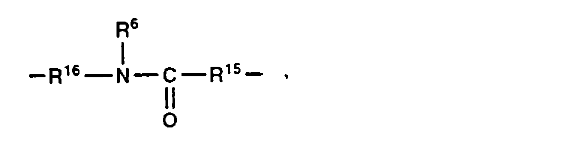

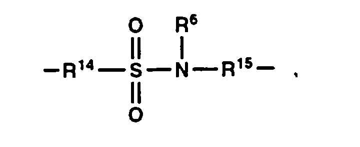

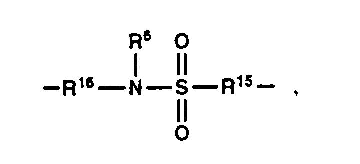

-R¹⁴-S-R¹⁵-,

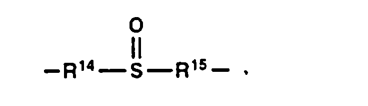

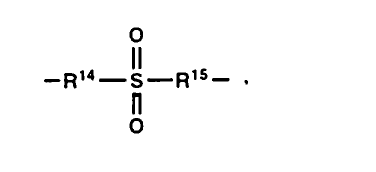

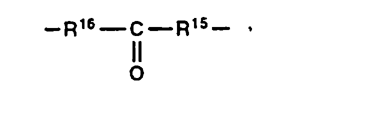

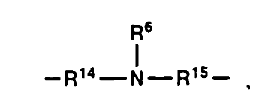

and

-R¹⁴-O-R¹⁵- _{,}

where p is 1, 2, 3, or 4; q is 3, 4, 5, 6 or 7; r is 1, 2, 3, 4, or 5; s is 2 or 3. R¹⁴ is selected from; a chemical bond, C₁-C₅-alkyl, C₃-C₇-cycloalkyl, C₂-C₅-alkenyl, C₃-C₅-alkynyl, C₆-C₁₀-aryl, C₁-C₂-alkyl-C₆-C₁₂-aryl, C₁-C₂-alkyl-C₆-C₁₀-aryl-C₁-C₂-alkyl, C₆-C₁₀-aryl-C₁-C₂-alkyl, C₆-C₁₀-aryloxy-C₁-C₂-alkyl, and piperizinyl. R¹⁵ is a chemical bond connecting L¹ to position 7 of the benzodiazepinedione. R¹⁶ is selected from; C₁-C₅-alkyl, C₃-C₇-cycloalkyl, C₃-C₅-alkenyl, C₃-C₅-alkynyl, C₆-C₁₀-aryl, C₁-C₃-alkyl-C₆-C₁₂-aryl, C₆-C₁₀-aryl-C₁-C₂-alkyl, and piperizinyl. For the foregoing most preferred L¹'s R¹⁴ and R¹⁶ bond L¹ to Q¹.

Most preferred Q¹-L¹- combinations are selected from;

H₂N-(CH₂)₃- ,

H₂N-(CH₂)₄- _{,}

H₂N-(CH₂)₅-,

H₂N-(CH₂)₆- ,

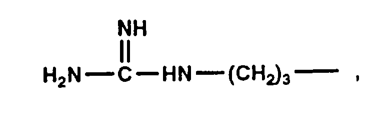

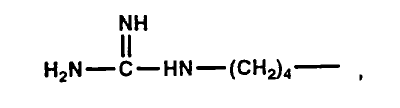

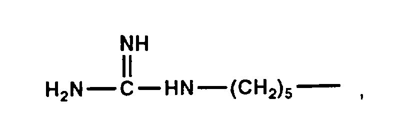

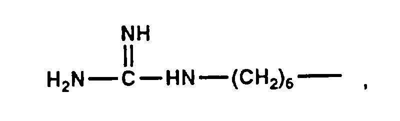

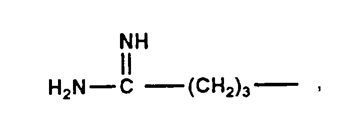

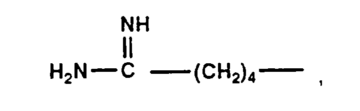

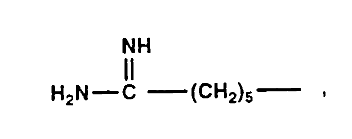

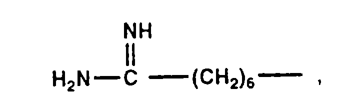

H₂N-(CH₂)₂-O- ,

H₂N-(CH₂)₃-O- ,

H₂N-(CH₂)₄-O- ,

H₂N-(CH₂)₅-O- ,

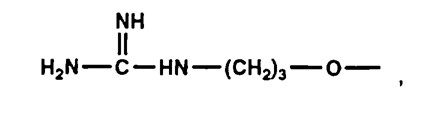

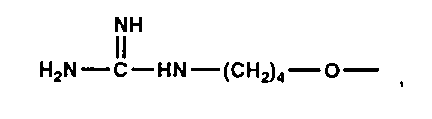

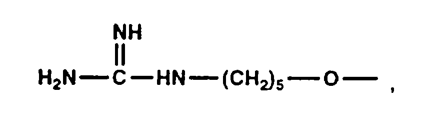

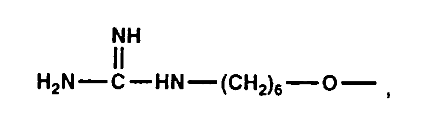

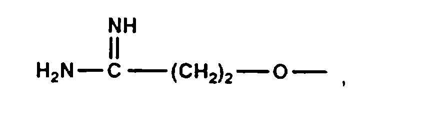

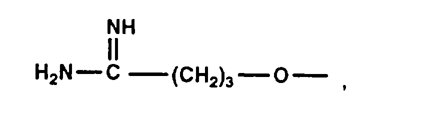

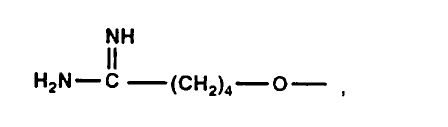

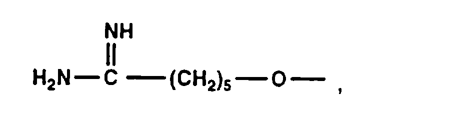

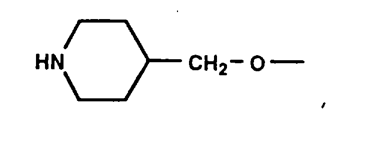

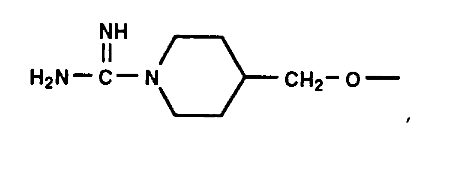

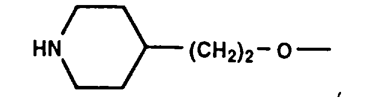

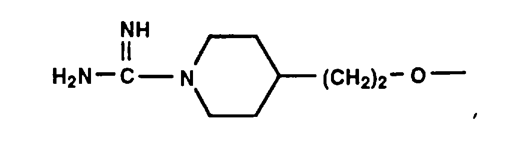

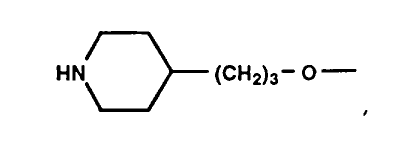

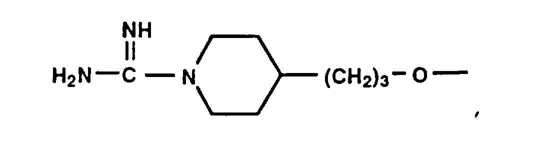

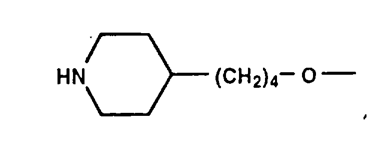

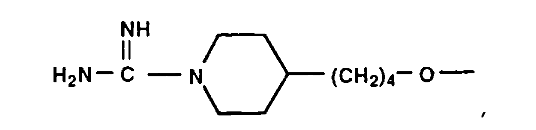

H₂N-CH₂-C≡C- ,

H₂N-(CH₂)₂-C≡C- ,

H₂N-(CH₂)₃-C≡C- ,

H₂N-(CH₂)₄-C≡C- ,

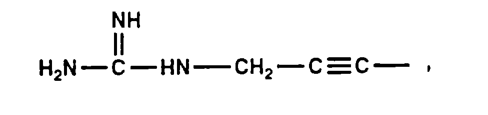

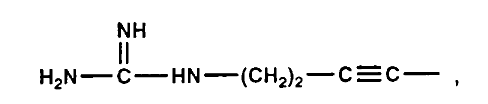

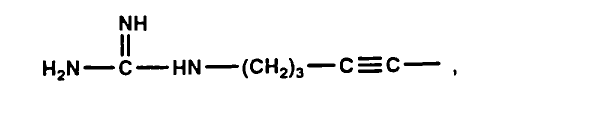

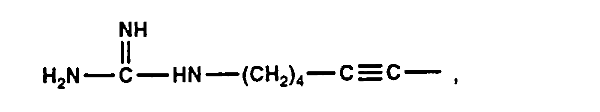

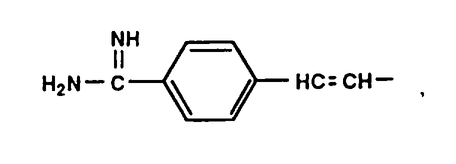

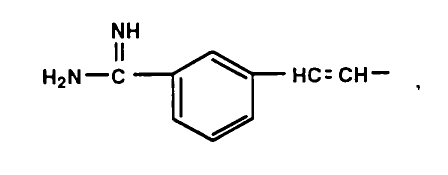

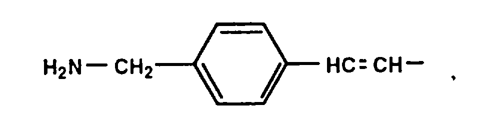

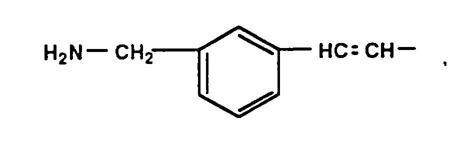

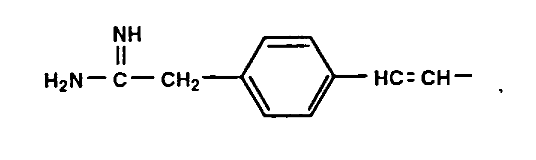

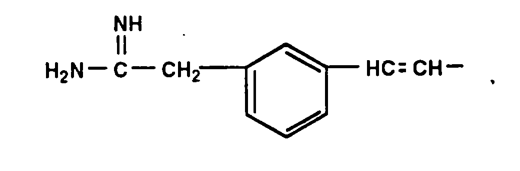

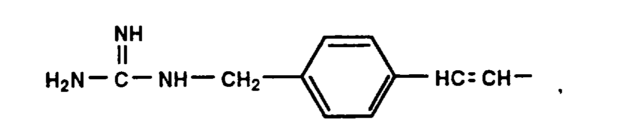

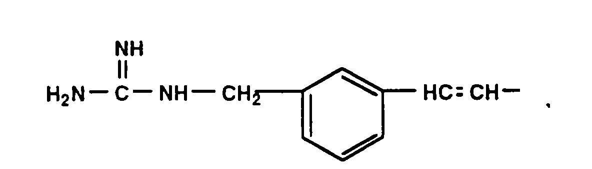

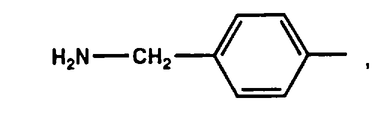

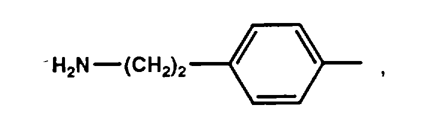

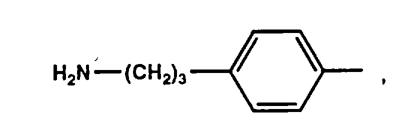

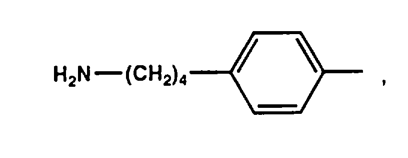

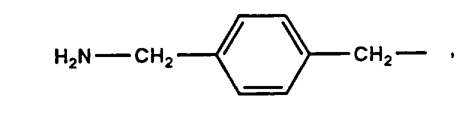

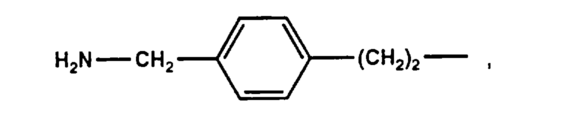

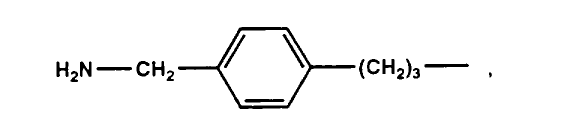

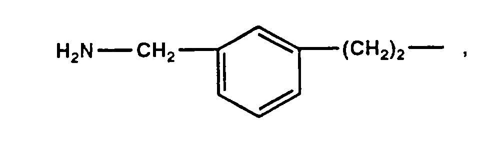

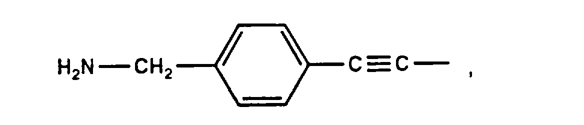

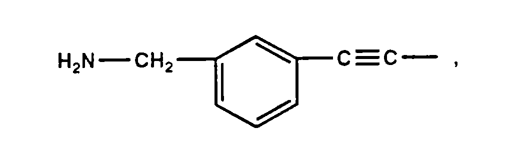

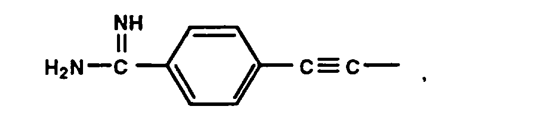

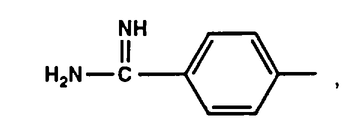

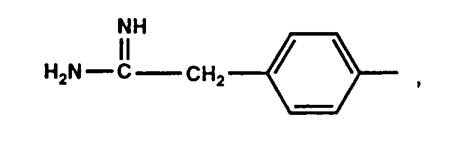

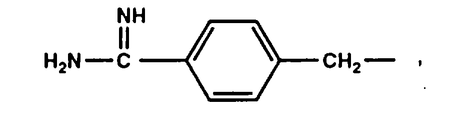

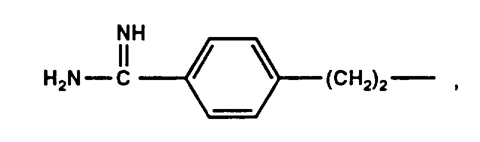

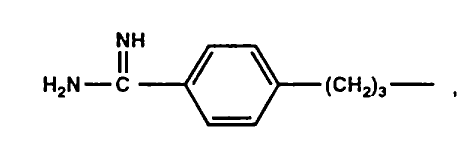

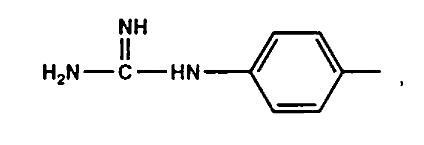

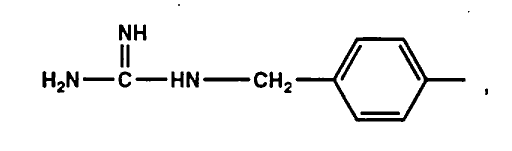

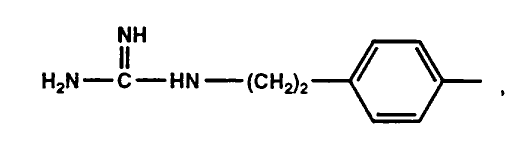

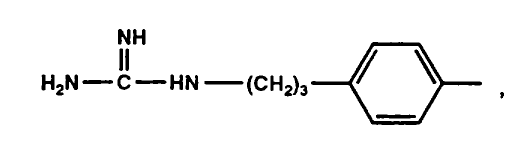

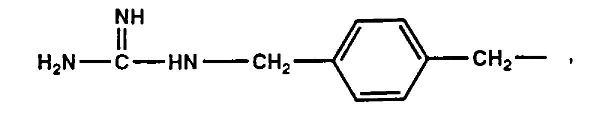

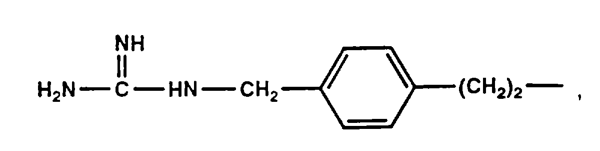

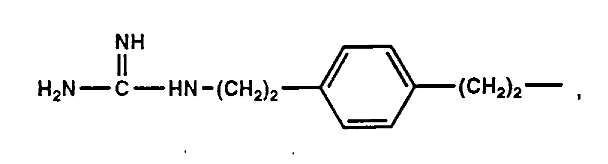

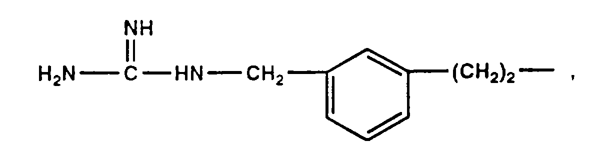

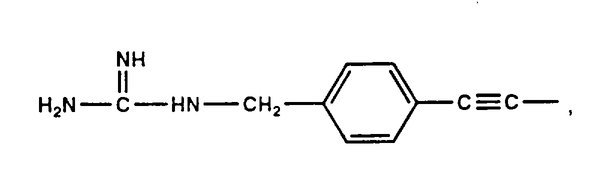

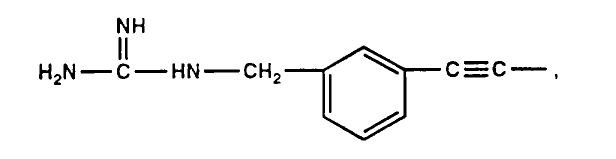

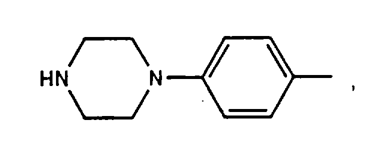

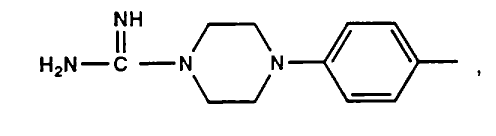

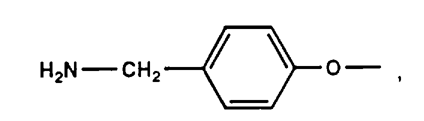

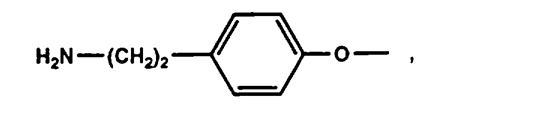

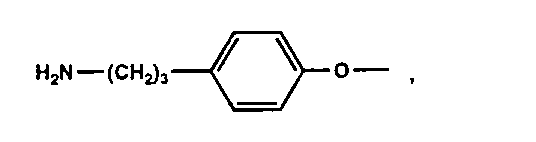

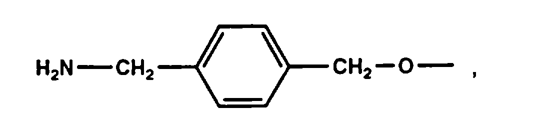

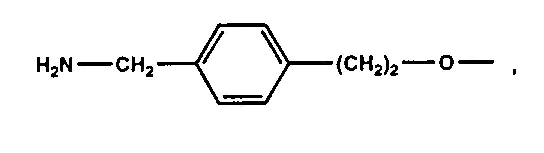

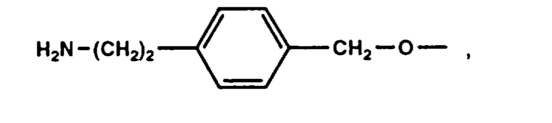

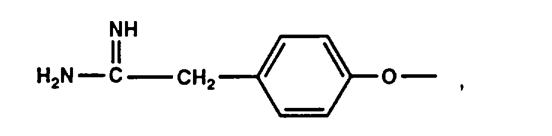

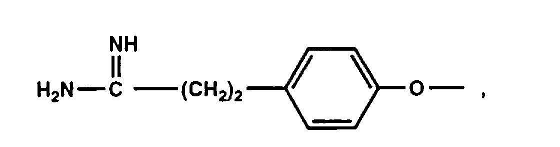

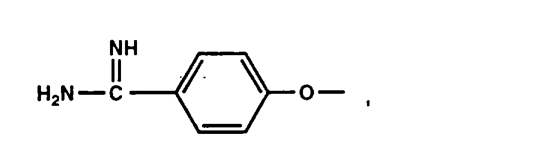

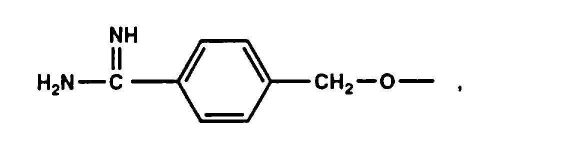

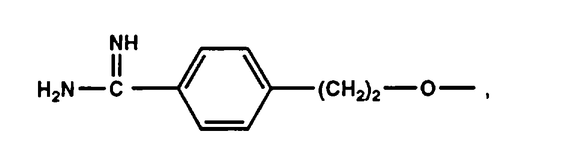

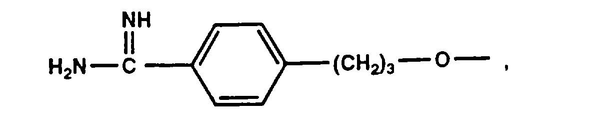

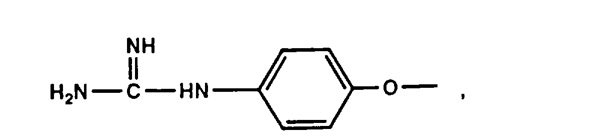

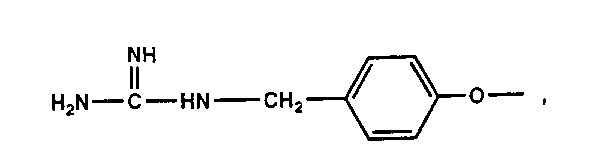

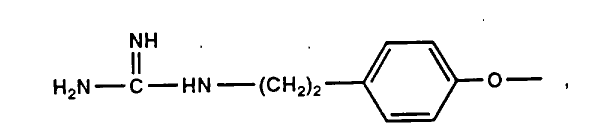

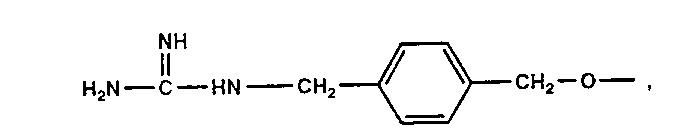

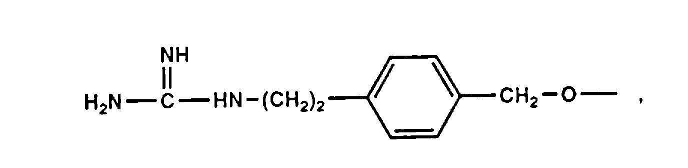

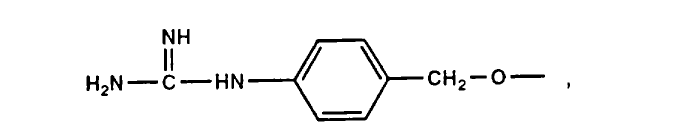

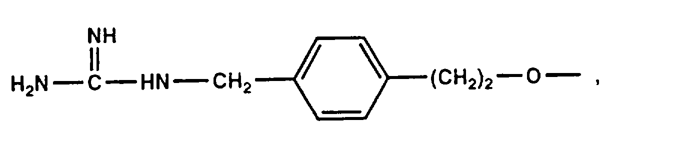

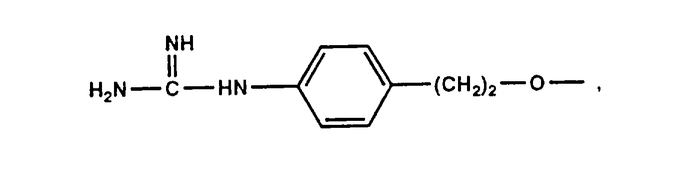

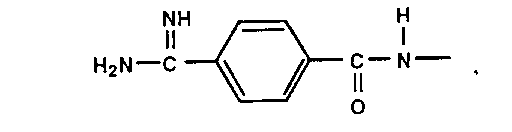

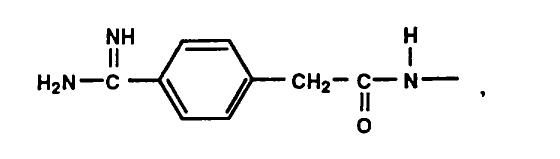

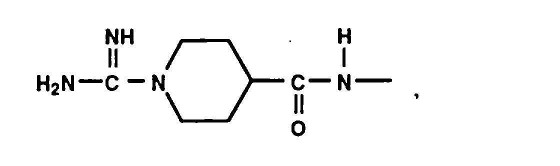

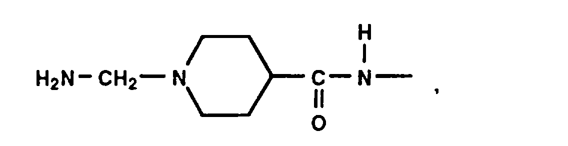

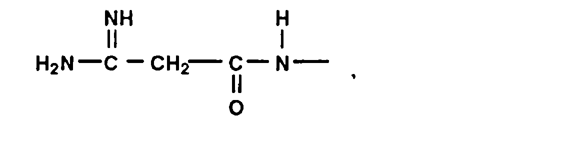

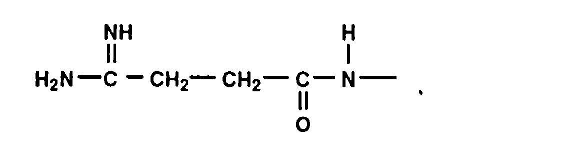

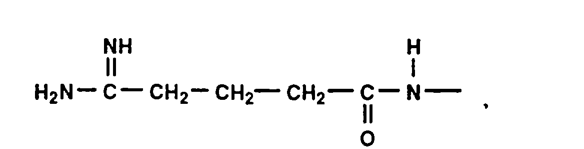

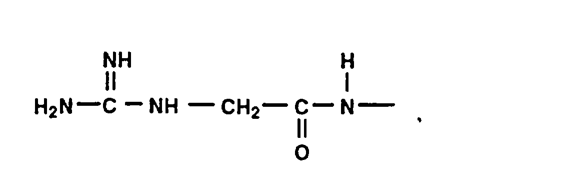

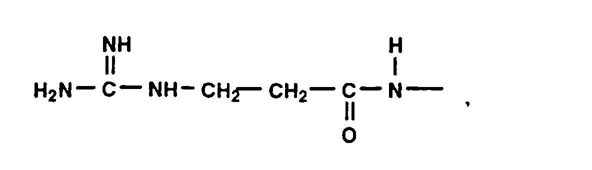

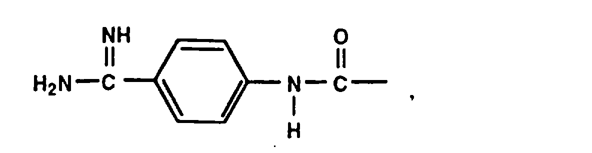

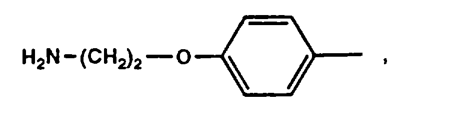

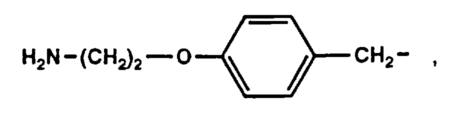

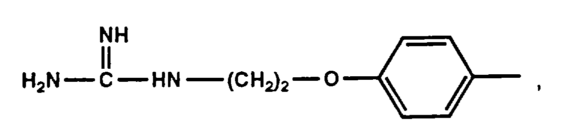

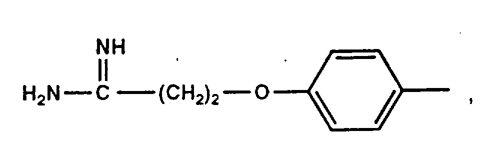

and

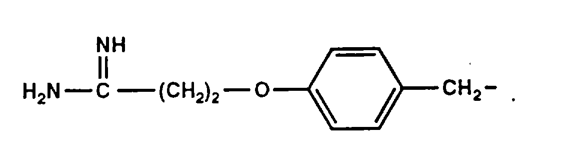

Very most preferred Q¹-L¹- combinations are selected from; 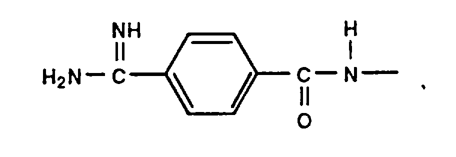 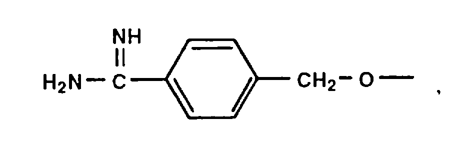 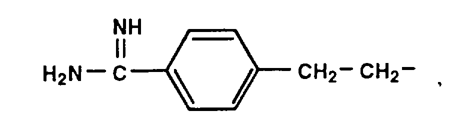 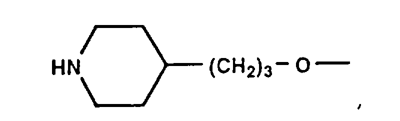 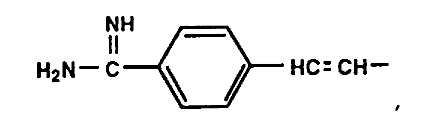 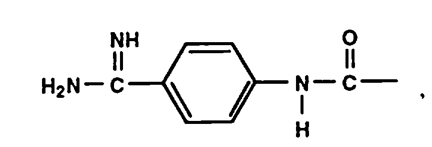 and

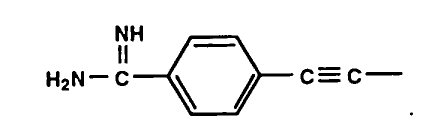

The length of the bivalent radical L is also important to biological activity. Here, when the A moiety is a 10-member fused ring [e.g. the napthalene moiety of the napthadiazepinedione nucleus (carbon 8 of Formula III)], suitable lengths for bivalent radical L range from about 1 to about 7 methylene equivalents. L is therefore a bivalent radical containing from 1 to 7 methylene groups connecting Q¹ to the number 8 carbon of the napthadiazepinedione nucleus (or an equivalent position on other 10-member A rings) where any methylene group or groups may be replaced with one or more alkene, alkyne, aryl, heterocycle or functional groups containing the heteroatoms N, O, and S, so long as the overall length is equivalent to 1 to 7 methylene groups. Here, the functional groups are the same as described above for L¹ and may be combined in any combination provided again that the compounds produced are stable in aqueous solution and do not exceed the stated length requirements for L. Preferred lengths for L are from 2 to 4 while most preferred lengths are about 3 methylene equivalents.

Preferred L's are optionally substituted bivalent radicals selected from;

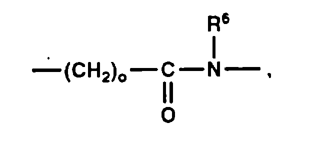

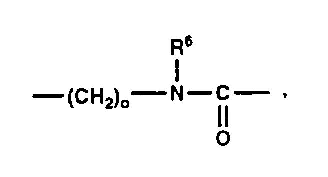

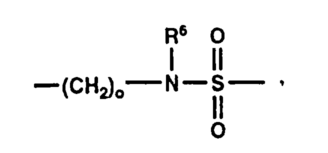

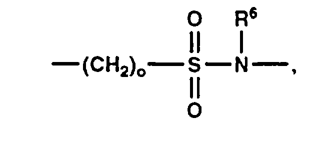

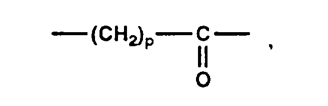

-(CH₂)ₚ-S- ,

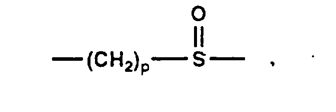

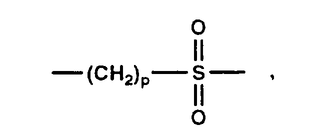

-(CH₂)ₒ-O-(CH₂)ₒ- ,

-(CH₂)ₚ-O-,

-(CH₂)ₚ-,

-(CH₂)ₒ-CH=CH- ,

and

-(CH₂)ₒ-C≡C- ,

where o is 0, 1 or 2, p is 1, 2, 3 or 4, R⁶ is defined above, and where the substituents are selected from one to three R¹ groups defined above.

### c. Ring A

Preferred A rings include; benzene, napthalene, tetrahydronapthalene, cyclohexane, thiophene, pyridine, pyrazine, pyrimidine, quinoline, isoquinoline, quinoxaline, quinazoline, pteridine, and naphthyridine The most preferred A ring is benzene.

Preferred substituents of rings A are R¹ and R, where each R¹ and R, is independently selected from hydrogen, halogen(F, Cl, Br, l), cyano, nitro, carboxyl, mercaptocarbonyl, mercapto, pthalimido and a substituent having from 1 to 12 carbon atoms selected from: alkyl, hydroxyalkyl, formyl, haloalkyl, cycloalkyl, cycloalkylalkyl, cycloalkylhaloalkyl, cycloalkylcarbonyl, alkoxy, aralkyl, aralkylhaloalkyl, aryl, aroyl, aryloxy, aryloxyalkyl, aralkoxy, alkoxyalkyl, alkylcarbonyl, alkylcarbonylalkyl, alkoxycarbonyl, alkenyl, cycloalkenyl, alkylnyl, carboxylalkyl, alkylcarbonyloxy, alkylcarbonyloxyalkyl, alkoxycarbonylalkyl, aralkoxycarbonylalkyl, aralkylcarbonyloxyalkyl, mercaptothiocarbonyl, mercaptoalkyl, alkoxycarbonyloxy, alkylthio, cycloalkylthio, cycloalkylalkylthio, alkylthiocarbonyl, alkylcarbonylthio, alkylthiocarbonyloxy, alkylthiocarbonylthio, alkylthiothiocarbonyl, alkylthiothiocarbonylthio, arylthio, arylthiocarbonyl, arylcarbonylthio, arylthiocarbonyloxy, arylthiocarbonylthio, arylthiothiocarbonyl, arylthiothiocarbonylthio, aralkylthio, aralkylthiocarbonyl, aralkylcarbonylthio, aralkylthiocarbonyloxy, aralkylthiocarbonylthio, alkylthiocarbonyl, aralkylthiocarbonylthio, alkylsulfinyl, alkylsulfonyl, aralkylsulfinyl, aralkylsulfonyl, arylsulfinyl, arylsulfonyl, pthalimidoalkyl, heteroaryl, heteroarylalkyl, cycloheteroalkyl, cycloheteroalkylalkyl, and cycloheteroalkylcarbonylalkyl where each heteroaryl- and cyclohetero-containing groups has one or more ring hetero atoms selected from oxygen, sulfur and nitrogen. Optionally, each R¹ and R also may be independently selected from sulfonyl, sulfonylamido, amino and amido radicals of the formula 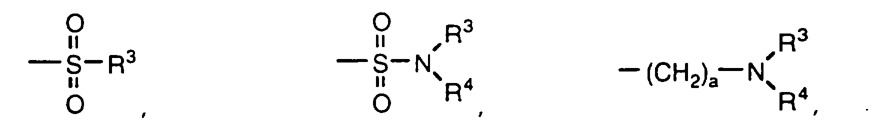 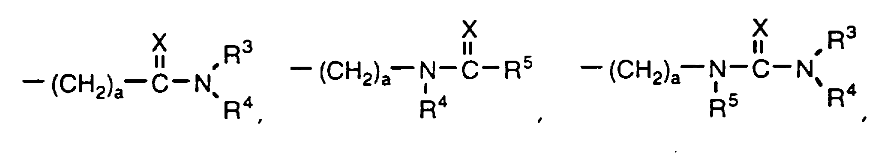 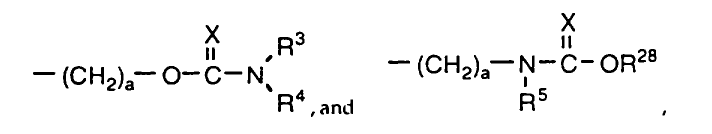 where X is oxygen or sulfur, each "a" is a number independently selected from zero to six, inclusive, and where each of R³ through R⁵ is independently selected from the groups described above. Optionally, R³ and R⁴ taken together, R⁴ and R⁵ taken together and R³ and R⁵ taken together may each form heterocyclic having from five to seven ring members including the hetero atom of the sulfonyl, amino or amido radical and which heterocyclic may further contain one or more hetero atoms as ring members selected from oxygen, nitrogen, and sulfur and which heterocyclic group may be saturated or partially saturated. R⁸ is selected from the groups defined below.

### d. The 7-member "lactam" ring containing TUG;

Preferred seven member rings are those of formula I where T bears the substituent R. Thus T is preferably sp³ carbon or nitrogen. Also preferably G is a methylene group. The most preferred "lactam ring" is a diazepinedione.

### e. Substituents of T-U-G

R¹⁹ and R⁰ are preferably hydrogen or halogen. R¹⁸ and R¹ are preferably hydrogen or halogen or taken together form oxo. R is preferably selected from; hydrogen, optionally substituted C₁-C₆-alkyl, where the substituents are selected from amino, hydroxy, halo (F, Cl, Br, I), carboxy, and C₁-C₄-alkoxycarbonyl, optionally substituted C₆-C₁₀-aryl, and optionally substituted C₆-C₁₂-aryl-C₁-C₄-alkyl, where the substituents on any aryl group are selected from amino, nitro, halo (F, Cl, Br, l), halo(F, Cl, Br, l)-C₁-C₆-alkyl, C₁-C₆-alkoxy, C₆-C₁₀-aryloxy, amino-C₁-C₆-acylamino, amino-C₁-C₆-acyl, and guanidino C₆-C₁₀-aroylamino.

### f. The amino acid linking moiety D

D is preferably hydrogen, phenyl, or lower alkyl. Optionally, D is -(C=O)-Xaa, where Xaa is one to three natural, unnatural, or modified α-amino acid residues, preferably one or two naturally occurring amino acid residues, and most preferably a naturally occurring hydrophobic amino acid residue. Optionally, when Xaa is two amino acid residues, the terminal residue is Arg or Lys amide. Common naturally occurring a-amino acids are described by the standard three letter amino acid code when referring to amino acids or residues. When the three-letter code begins with a lower-case letter, it is understood the amino acid is the unnatural or D-isomeric form. Standard abbreviations are listed in The Merck Index, 10th Edition, pp Misc-2 - Misc-3, Modified or unusual α-amino acids such as norleucine (Nle) and ornithine (Orn) are designated as described in U.S. Patent and Trademark Office Official Gazette 1114TMOG, May 15, 1990.

### g. The negatively charged acidic W moiety

W is -R⁷-w.

R⁷ is preferably selected from (a) a covalent bond, (b) substituted or unsubstituted methylene, and (c) substituted or unsubstituted ethylene, where the substituents are independently selected from one or more of the groups (i) nitro, (ii) halo(F, Cl, Br,l), (iii) C₁-C₆alkyl, (iv) halo(F, Cl, Br, l)-C₁-C₆alkyl, and (v) substituted or unsubstituted phenyl where the substituents are selected from (1) C₁-C₆alkyl, (2) C₁-C₆alkoxy, (3) halo(F, Cl, Br, I), and (4) CF₃.

"w" is selected from (a) -COR⁸, (b) -SO₃R³¹, (c) -NHSO₂R³ (d) -PO(OR³¹)₂, (e) -SO₂NHR³, (f) -CONHOR³¹, (g) -C(OH)R³³PO(OR³³)₂, (h) -CN, (i) -SO₂NH-heterocycle where the heterocycle is a 5- or 6-member aromatic ring containing 1 to 3 heteroatoms selected from O, N, and S and where the heterocycle is unsubstituted or substituted with one or two substituents selected from the group (i) -OH, (ii) -SH, (iii) -(C₁-C₄alkyl), (iv) -(C₁-C₄alkoxyl), (v) CF₃, (vi) halo(F, Cl, Br, I), (vii) NO₂, (viii) -COOH, (ix) -COO-(C₁-C₄alkyl), (x) -NH₂, (xi) -NH(C₁ -C₄alkyl), or (xii)-N(C₁-C₄alkyl)₂, (j) -CH₂SO₂NH-heterocycle, (k) -SO₂NHCOR³³, (l) -CH₂SO₂NHCOR³ʼ (m) -CONHSO₂R³³, (n) -CH₂CONHSO₂R³³, (o) -NHCONHSO₂R³³, (p) -NHSO₂NHCOR³³, (q) -CONHNHSO₂CF₃, (r) CON(OH)R³¹, (s) -CONHCOCF₃, (t) -CONHSO₂R⁸,(u) -CONHSO₂R⁹, (v) -CONHSO₂R³⁰,

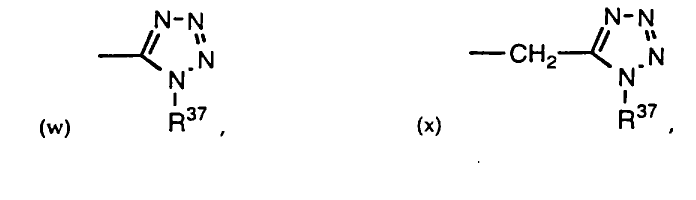

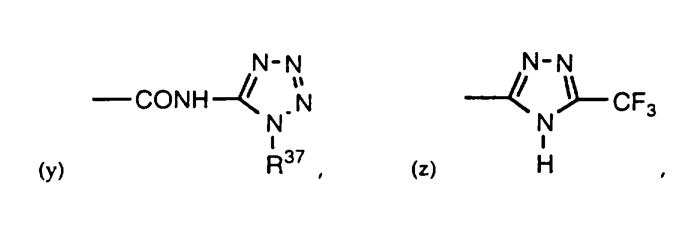

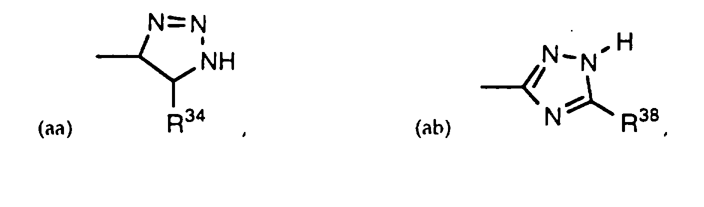

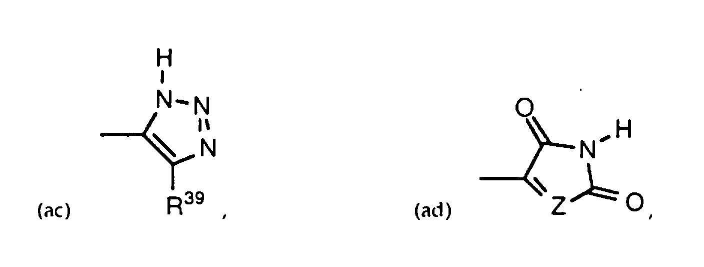

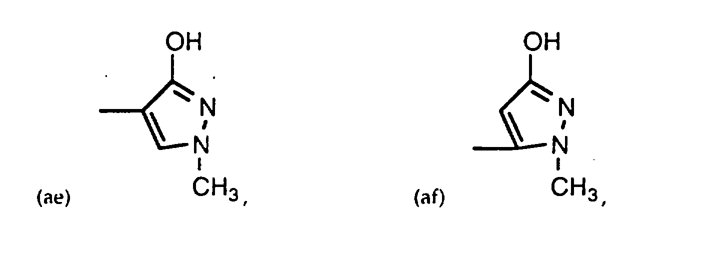

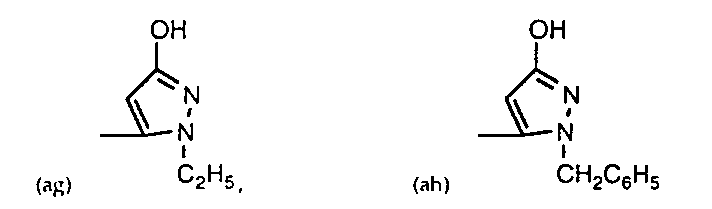

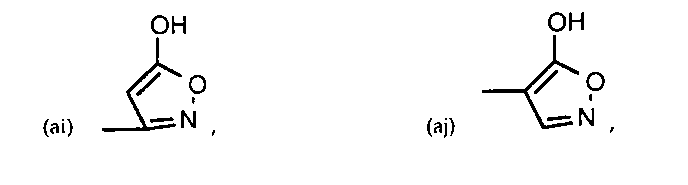

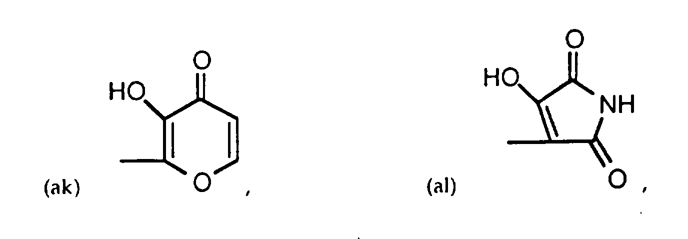

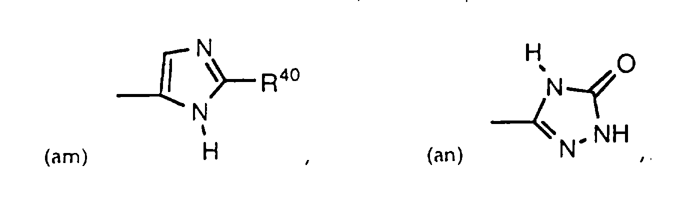

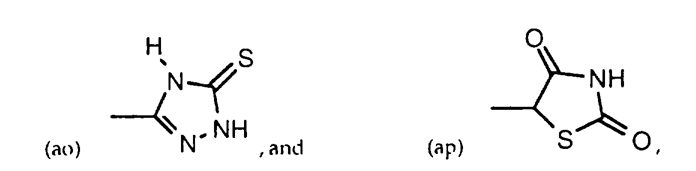

R⁸ is selected from the group consisting of (a) hydroxy, (b) C₁-C₈-alkoy, (c) C₃-C₁₂-alkenoxy, (d)C₆-C₁₂,-aryloxy, (e) C₁-C₆-alkyl-C₆-C₁₂-aryloxy, (f) di-C₁-C₈-alkylamino-C₁-C₈-alkoxy, (g)acylamino-C₁-C₈-alkoxy selected from the group (i) acetylaminoethoxy, (ii) nicotinoylaminoethoxy, and (iii) succinamidoethoxy, (h) C₁-C₈-alkoxy, (i) C₆-C₁₂-aryl-C₁-C₈-alkoxy where the aryl group is unsubstituted or substituted with one to three of the groups (i) nitro, (ii) halo (F, Cl, Br, I), (iii) ) C₁-C₄-alkoxy, and (iv) amino, (j) hydroxy-C₂-C₈-alkoxy, (k) dihydroxy-C₃-C₈-alkoxy, and (I) NR⁹R³⁰.

R⁹ and R³⁰ are independently selected from the group (a) hydrogen, (b) C₁-C₈-alkyl, (c) C₃-C₈-alkenyl, (d) C₆-C₁₂-aryl where the aryl group is unsubstituted or substituted with one to three of the groups (i) nitro, (ii) halo (F, Cl, Br, I), (iii) C₁-C₄-alkoxy, and (iv) amino, and (e) C₆-C₁₂-aryl-C₁-C₈-alkyl where the aryl group is unsubstituted or substituted with one to three of the groups (i) nitro, (ii) halo (F, Cl, Br, I), and (iii) C₁-C₄-alkoxy.

R³¹ is selected from the group consisting of (a) H, (b) C₁-C₆ alkyl, (c) halo(F, Cl, Br, I)-C₁-C₆ alkyl, (d) phenyl, (e) benzyl, and (f) -CH₂-O-COCH₃.

R³ is selected from the group consisting of (a) H, (b) benzyl, and (c) -CH(R³⁵)-O-C(O)R³⁵.

R³³ is selected from the group consisting of (a) aryl, (b) heterocycle, (c) (C₃-C₇)-cycloalkyl, (d) (C₁-C₄)-alkyl, unsubstituted or substituted with a substituent selected from the group consisting of (i) aryl, (ii) heterocycle, (iii) -OH, (iv) -SH, (v) (C₁-C₄)-alkyl, (vi) (C₁-C₄)-alkoxy, (vii) (C₁-C₄)-alkylthio, (viii) -CF_{3,} (ix) halo (F, Cl, Br, I), (x) -NO₂, (xi)-CO₂H, (xii) CO₂-(C₁-C₄)alkyl, (xiii) -NH₂, (xiv) -N[(C₁-C₄)-alkyl)-]₂, (xv) -NH[(C₁-C₄)alkyl], (xvi) -PO₃H, or (xvii) PO(OH)(C₁-C₄)-alkoxy, or (e) (C₁-C₄)-perfluoroalkyl.

R³⁴ is selected from the group consisting of (a) -CN, (b) -NO₂, (c) -COOR³¹, (d) C₁-C₆-perfluoroalkyl, and (e) CF₃.

R³⁵ is independently selected from the group consisting of (a) H, (b) (C₁-C₆)-alkyl, (C₂-C₆)-alkenyl, (C₂-C₆)-alkynyl or (C₃-C₈)-cycloalkyl, each of which is unsubstituted or substituted with: (i) OH, (ii) (C₁-C₄)-alkoxy, (iii) CO₂R³³, (iv) OCOR³³, (v) CONHR³³, (vi) CON(R³³)₂, (vii) N(R³³)C(O)R³³, (viii) NH₂, (ix) (C₁-C₄)-alkylamino, (x) di[(C₁-C₄)-alkyl]amino, (xi) aryl, (xii) heteroaryl, (c) -C(O)-aryl, (d) -NO₂, (e) halo(Cl, Br, I, F), (f) -OH, (g) -OR³⁶, (h) (C₁-C₄)-perfluoroalkyl, (i) -SH, (j) -S(O)*₁*₋₂ (C₁-C₄)-alkyl, (k) CO₂R³³,(I)-SO₃H, (m) -NR³³R³⁶, (n) -NR³³C(O)R³⁶, (o) -NR³³COOR³, (p) -SO₂NHR³, (q) -SO₂NR³³R³³,(r) -NHSO₂R³,(s) -C(O)NHSO₂R³, (t) aryl, (u) heteroaryl, (v) morpholin-4-yl, (w) CONH₂, or (y) 1H-tetrazol-5-yl.

R³⁶ is selected from the group (a) H; or (b) (C₁-C₄)-alkyl unsubstituted or substituted with (i) NH₂, (ii) NH[(C₁-C₄)-alkyl], (iii) N[(C₁-C₄)-alkyl]₂, (iv) CO₂H, (v) CO₂(C₁-C₄)-alkyl, (vi) OH, (vii) SO₃H, or (viii) SO₂NH₂.

R³⁷ is selected from the group consisting of (a) H, (b) (C₁-C₆)-alkyl, (c) (C₂-C₆)-alkenyl, (d) (C₁-C₆)-alkoxyalkyl, (e) -CH₂-O-COCH₃ or (f) -CH₂-phenyl, where the phenyl is unsubstituted or substituted with a substituent selected from -NO₂, -NH₂, -OH, or -OCH₃.

R³⁸, R³⁹, and R⁴⁰ are each independently selected from H, Cl, CN, NO₂, CF₃, C₂F₅, C₃F₇, CHF₂, CH₂F, CO₂CH₃, CO₂C₂H₅, SO₂CH₃, SO₂CF₃ and SO₂C₆F₅; wherein Z is selected from O, S, NR⁴¹ and CH₂.

R⁴¹ is selected from hydrogen, CH₃, and CH₂C₆H₅ or a tautomer or pharmaceutically acceptable salt thereof.

Preferred "w"'s are selected from; -COR⁸.-NHSO₂CF₃,

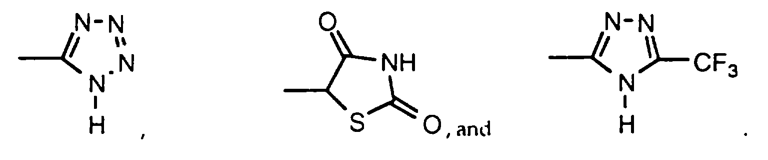

Most preferred W's are either a tetrazole or a carboxylate. Preferably these groups are represented by; 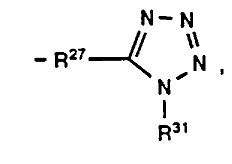 and 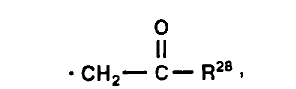 where R⁷ is methylene, and R⁸ and R³¹ are hydroxy and hydrogen respectively. Optionally, R⁸ and R³¹ are substituents that transform compounds represented by structural formulae I- VI into prodrugs. When W contains a carboxylate, for example, preferred prodrug forms include simple esters, α-acyloxyalkyl esters, and amides. Preferred esters include compounds where R⁸ is selected from;

-O-CH₂-CH₃ ,

-O-CH₂-CH₂-CH₂-CH₃ ,

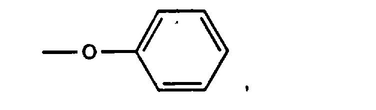

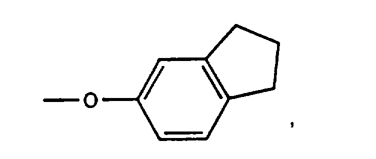

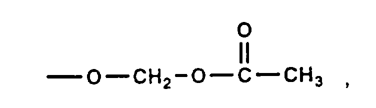

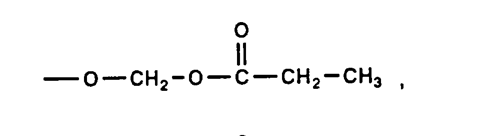

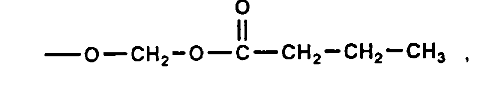

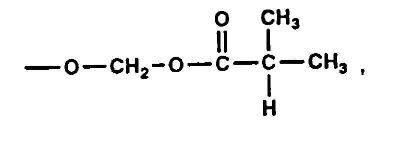

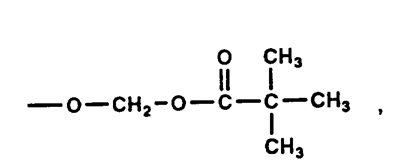

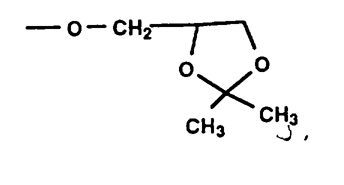

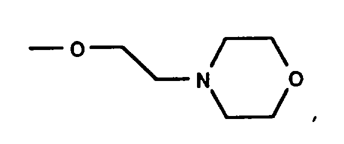

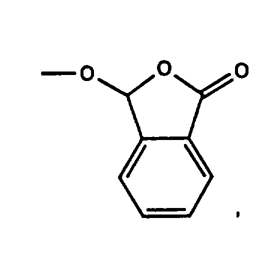

and

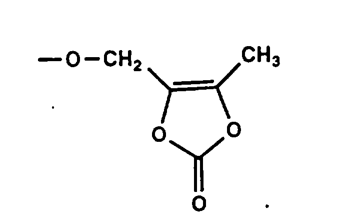

The most preferred compounds of this invention are represented by structural formula II and are selected from the following:
1-(4-chlorophenyl)methyl-4-(2-carboxyethyl)-7-(4-(1-piperizin)-phenyl)3,4-dihydro-1H-1,4-benzodiazapine-2,5-dione.
1-(diphenyl)methyl-4-(2-carboxyethyl)-7-(4-(1-piperizin)-phenyl)-3,4-dihydro-1H-1,4-benzodiazapine-2,5-dione.
1-(*m*-trifluromethyl)phenyl-4-(2-carboxyethyl)-7-(4-(1-piperizin)-phenyl)-3,4-dihydro-1H-1,4-benzodiazapine-2,5-dione.
1-methyl-4-(2-carboxyethyl)-7-(4-(1-piperizin)-phenyl)-3,4-dihydro-1H-1,4-benzodiazapine-2,5-dione.
1-(1-methyl)ethyl-4-(2-carboxyethyl)-7-(4-(1-piperizin)-phenyl)-3,4-dihydro-1H-1,4-benzodiazapine-2,5-dione.
1-(2-methyl)propyl-4-(2-carboxyethyl)-7-(4-(1-piperizin)-phenyl)-3,4-dihydro-1H-1,4-benzodiazapine-2,5-dione.
1-(4-chlorophenyl)methyl-4-(2-carboxyethyl)-7-(4-(2-aminoethoxy)-phenyl)-3,4-dihydro-1H-1,4-benzodiazapine-2,5-dione.
1-(diphenyl)methyl-4-(2-carboxyethyl)-7-(4-(2-aminoethoxy)-phenyl)-3,4-dihydro-1H-1,4-benzodiazapine-2,5-dione.
1-(*m*-trifluromethyl)phenyl-4-(2-carboxyethyl)-7-(4-(2-aminoethoxy)-phenyl)-3,4-dihydro-1H-1,4-benzodiazapine-2,5-dione.
1-methyl-4-(2-carboxyethyl)-7-(4-(2-aminoethoxy)-phenyl)-3,4-dihydro-1H-1,4-benzodiazapine-2,5-dione.
1-(1-methyl)ethyl-4-(2-carboxyethyl)-7-(4-(2-aminoetoxy)-phenyl)-3,4-dihydro-1H-1,4-benzodiazapine-2,5-dione.
1-(2-methyl)propyl-4-(2-carboxyethyl)-7-(4-(2-aminoethoxy)-phenyl)-3,4-dihydro-1H-1,4-benzodiazapine-2,5-dione.
1-(diphenyl)methyl-4-(2-carboxyethyl)-7-(6-aminohexyl)-3,4-dihydro-1H-1,4-benzodiazapine-2,5-dione.
1-(diphenyl)methyl-4-(2-carboxyethyl)-7-(6-aminohex-1-ynyl)-3,4-dihydro-1H-1,4-benzodiazapine-2,5-dione.
1-(diphenyl)methyl-4-(2-carboxyethyl)-7-(5-aminopentoxy)-3,4-dihydro-1H-1,4-benzodiazapine-2,5-dione.
1-(diphenyl)methyl-4-(2-carboxyethyl)-7-(5-guanidinopentyl)-3,4-dihydro-1H-1,4-benzodiazapine-2,5-dione.
1-(diphenyl)methyl-4-(2-carboxyethyl)-7-(4-guanidinobutoxy)-3,4-dihydro-1H-1,4-benzodiazapine-2,5-dione.
1-(diphenyl)methyl-4-(2-carboxyethyl)-7-(5-aminopent-1-ynyl)-3,4-dihydro-1H-1,4-benzodiazapine-2,5-dione.
1-(diphenyl)methyl-4-(2-carboxyethyl)-7-(5-guanidinopent-1-ynyl)-3,4-dihydro-1H-1,4-benzodiazapine-2,5-dione.
1-(diphenyl)methyl-4-(2-carboxyethyl)-7-(4-amidino)benzamido-3,4-dihydro-1H-1,4-benzodiazapine-2,5-dione.
1-(diphenyl)methyl-4-(2-carboxyethyl)-7-(4-amidino)benzyloxy-3,4-dihydro-1H-1,4-benzodiazapine-2,5-dione.
1-(diphenyl)methyl-4-(2-carboxyethyl)-7-[2-(4-amidinophenyl)]ethenyl-3,4-dihydro-1H-1,4-benzodiazapine-2,5-dione.
1-(diphenyl)methyl-4-(2-carboxyethyl)-7-[2-(4-amidinophenyl)]ethynyl-3,4-dihydro-1H-1,4-benzodiazapine-2,5-dione.
1-(4-chlorophenyl)methyl-4-(2-carboxyethyl)-7-(6-aminohexyl)-3,4-dihydro-1H-1,4-benzodiazapine-2,5-dione.
1-(4-chlorophenyl)methyl-4-(2-carboxyethyl)-7-(5-aminopentoxy)-3,4-dihydro-1H-1,4-benzodiazapine-2,5-dione.
1-(4-chlorophenyl)methyl-4-(2-carboxyethyl)-7-(4-amidino)benzamido-3,4-dihydro-1H-1,4-benzodiazapine-2,5-dione.
1-(4-chlorophenyl)methyl-4-(2-carboxyethyl)-7-(4-amidino)benzyloxy-3,4-dihydro-1H-1,4-benzodiazapine-2,5-dione.
1-(4-chlorophenyl)methyl-4-(2-carboxyethyl)-7-[2-(4-amidinophenyl)]ethenyl-3,4-dihydro-1H-1,4-benzodiazapine-2,5-dione.
1-(4-chlorophenyl)methyl-4-(2-carboxyethyl)-7-[2-(4-amidinophenyl)]ethynyl-3,4-dihydro-1H-1,4-benzodiazapine-2,5-dione.
1-methyl-4-(2-carboxyethyl)-7-(4-amidino)benzamido-3,4-dihydro-1H-1,4-benzodiazapine-2,5-dione.
1-methyl-4-(2-carboxyethyl)-7-(4-amidino)benzyloxy-3,4-dihydro-1H-1,4-benzodiazapine-2,5-dione.
1-methyl-4-(2-carboxyethyl)-7-[2-(4-amidinophenyl)]ethenyl-3,4-dihydro-1H-1,4-benzodiazapine-2,5-dione.
1-methyl-4-(2-carboxyethyl)-7-[2-(4-amidinophenyl)]ethynyl-3,4-dihydro-1H-1,4-benzadiazapine-2,5-dione.
1-(1-methyl)ethyl-4-(2-carboxyethyl)-7-(4-amidino)benzamido-3,4-dihydro-1H-1,4-benzodiazapine-2,5-dione.
1-(1-methyl)ethyl-4-(2-carboxyethyl)-7-(4-amidino)benzyloxy-3,4-dihydro-1H-1,4-benzodiazapine-2,5-dione.
1-(1-methyl)ethyl-4-(2-carboxyethyl)-7-[2-(4-amidinophenyl)]ethenyl-3,4-dihydro-1H-1,4-benzodiazapine-2,5-dione.
1-(2-methyl)ethyl-4-(2-carboxyethyl)-7-[2-(4-amidinophenyl)]ethynyl-3,4-dihydro-1H-1,4-benzodiazapine-2,5-dione.
1-(2-methyl)propyl-4-(2-carboxyethyl)-7-(4-amidino)benzamido-3,4-dihydro-1H-1,4-benzodiazapine-2,5-dione.
1-(2-methyl)propyl-4-(2-carboxyethyl)-7-(4-amidino)benzyloxy-3,4-dihydro-1H-1,4-benzodiazapine-2,5-dione.
1-(2-methyl)propyl-4-(2-carboxyethyl)-7-[2-(4-amidinophenyl)]ethenyl-3,4-dihydro-1H-1,4-benzodiazapine-2,5-dione.
1-(2-methyl)propyl-4-(2-carboxyethyl)-7-[2-(4-amidinophenyl)]ethynyl-3,4-dihydro-1H-1,4-benzodiazapine-2,5-dione.
1-(2,4-diflurophenyl)methyl-4-(2-carboxyethyl)-7-(4-(1-piperizin)-phenyl)-3,4-dihydro-1H-1,4-benzodiazapine-2,5-dione.
1-(2,4-diflurophenyl)methyl-4-(2-carboxyethyl)-7-(4-(2-aminoethoxy)-phenyl)-3,4-dihydro-1H-1,4-benzodiazapine-2,5-dione.
1-(2,4-diflurophenyl)methyl-4-(2-carboxyethyl)-7-(6-aminohexyl)-3,4-dihydro-1H-1,4-benzodiazapine-2,5-dione.
1-(2,4-diflurophenyl)methyl-4-(2-carboxyethyl)-7-(5-aminopentoxy)-3,4-dihydro-1H-1,4-benzodiazapine-2,5-dione.
1-(2,4-diflurophenyl)methyl-4-(2-carboxyethyl)-7-(4-amidino)benzamido-3,4-dihydro-1H-1,4-benzodiazapine-2,5-dione.
1-(2,4-diflurophenyl)methyl-4-(2-carboxyethyl)-7-(4-amidino)benzyloxy-3,4-dihydro-1H-1,4-benzodiazapine-2,5-dione.
1-(2,4-diflurophenyl)methyl-4-(2-carboxyethyl)-7-[2-(4-amidinophenyl)]ethenyl-3,4-dihydro-1H-1,4-benzodiazapine-2,5-dione.
1-(2,4-diflurophenyl)methyl-4-(2-carboxyethyl)-7-[2-(4-amidinophenyl)]ethynyl-3,4-dihydro-1H-1,4-benzodiazapine-2,5-dione.
1-(4-chlorophenyl)methyl-4-(2-carboxyethyl)-7-[2-(4-piperidinyl)ethyloxy]-3,4-dihydro-1H-1,4-benzodiazapine-2,5-dione.
1-(diphenyl)methyl-4-(2-carboxyethyl)-7-[2-(4-piperidinyl)ethyloxy]3,4-dihydro-1H-1,4-benzodiazapine-2,5-dione.
1-(*m*-trifluromethyl)phenyl-4-(2-carboxyethyl)-7-[2-(4-piperidinyl)ethyIoxy]-3,4-dihydro-1H-1,4-benzodiazapine-2,5-dione.
1-methyl-4-(2-carboxyethyl)-7-[2-(4-piperidinyl)ethyloxy]-3,4-dihydro-1H-1,4-benzodiazapine-2,5-dione.
1-(1-methyl)ethyl-4-(2-carboxyethyl)-7-[2-(4-piperidinyl)ethyloxy]-3,4-dihydro-1H-1,4-benzodiazapine-2,5-dione.
1-(2-methyl)propyl-4-(2-carboxyethyl)-7-[2-(4-piperidinyl)ethyloxy]-3,4-dihydro-1H-1,4-benzodiazapine-2,5-dione.
1-(2,4-diflurophenyl)methyl-4-(2-carboxyethyl)-7-[2-(4-piperidinyl)ethyloxy]-3,4-dihydro-1H-1,4-benzodiazapine-2,5-dione.
1-(4-chlorophenyl)methyl-4-(3-butanoyl)-7-(4-(1-piperizin)-phenyl)-3,4-dihydro-1H-1,4-benzodiazapine-2,5-dione.
1-(diphenyl)methyl-4-(3-butanoyl)-7-(4-(1-piperizin)-phenyl)-3,4-dihydro-1H-1,4-benzodiazapine-2,5-dione.
1-(*m*-trifluromethyl)phenyl-4-(3-butanoyl)-7-(4-(1-piperizin)-phenyl)-3,4-dihydro-1H-1,4-benzodiazapine-2,5-dione.
1-methyl-4-(3-butanoyl)-7-(4-(1-piperizin)-phenyl)-3,4-dihydro-1H-1,4-benzodiazapine-2,5dione.
1-(1-methyl)ethyl-4-(3-butanoyl)-7-(4-(1-piperizin)-phenyl)-3,4-dihydro-1H-1,4-benzodiazapine-2,5-dione.
1-(2-methyl)propyl-4-(3-butanoyl)-7-(4-(1-piperizin)-phenyl)-3,4-dihydro-1H-1,4-bezodiazapine-2,5-dione.
1-(4-chlorophenyl)methyl-4-(3-butanoyl)-7-(4-(2-aminoethoxy)-phenyl)-3,4-dihydro-1H-1,4-benzodiazapine-2,5-dione.
1-(diphenyl)methyl-4-(3-butanoyl)-7-(4-(2-aminoethoxy)-phenyl)-3,4-dihydro-1H-1,4-benzodiazapine-2,5-dione.
1-(*m*-trifluromethyl)phenyl-4-(3-butanoyl)-7-(4-(2-aminoethoxy)-phenyl)-3,4-dihydro-1H-1,4-benzodiazapine-2,5-dione.
1-methyl-4-(3-butanoyl)-7-(4-(2-aminoethoxy)-phenyl)-3,4-dihydro-1H-1,4-berzodiazapine-2,5-dione.
1-(1-methyl)ethyl-4-(3-butanoyl)-7-(4-(2-aminoethoxy)-phenyl)-3,4-dihydro-1H-1,4-benzodiazapine-2,5-dione.
1-(2-methyl)propyl-4-(3-butanoyl)-7-(4-(2-aminoethoxy)-phenyl)-3,4-dihydro-1H-1,4-benzodiazapine-2,5-dione.
1-(diphenyl)methyl-4-(3-butanoyl)-7-(6-aminohexyl)-3,4-dihydro-1H-1,4-benzodiazapine-2,5-dione.
1-(diphenyl)methyl-4-(3-butanoyl)-7-(6-aminohex-1-ynyl)-3,4-dihydro-1H-1,4-benzodiazapine-2,5-dione.
1-(diphenyl)methyl-4-(3-butanoyl)-7-(5-aminopentoxy)-3,4-dihydro-1H-1,4-benzodiazapine-2,5-dione.
1-(diphenyl)methyl-4-(3-butanoyl)-7-(5-guanidinopentyl)-3,4-dihydro-1H-1,4-benzodiazapine-2,5-dione.
1-(diphenyl)methyl-4-(3-butanoyl)-7-(4-guanidinobutoxy)-3,4-dihydro-1H-1,4-benzodiazapine-2,5-dione.
1-(diphenyl)methyl-4-(3-butanoyl)-7-(5-aminopent-1-ynyl)-3,4-dihydro-1H-1,4-benzodiazapine-2,5-dione.
1-(diphenyl)methyl-4-(3-butanoyl)-7-(5-guanidinopent-1-ynyl)-3,4-dihydro-1H-1,4-benzodiazapine-2,5-dione.
1-(diphenyl)methyl-4-(3-butanoyl)-7-(4-amidino)benzamido-3,4-dihydro-1H-1,4-benzodiazapine-2,5-dione.
1-(diphenyl)methyl-4-(3-butanoyl)-7-(4-amidino)benzyloxy-3,4-dihydro-1H-1,4-benzodiazapine-2,5-dione.
1-(diphenyl)methyl-4-(3-butanoyl)-7-[2-(4-amidinophenyl)]ethenyl-3-dihydro-1H-1,4-benzodiazapine-2,5-dione.
1-(diphenyl)methyl-4-(3-butanoyl)-7-[2-(4-amidinophenyl)]ethynyl-3,4-dihydro-1H-1,4-benzodiazapine-2,5-dione.
1-(4-chlorophenyl)methyl-4-(3-butanoyl)-7-(6-aminohexyl)-3,4-dihydro-1H-1,4-benzodiazapine-2,5-dione.
1-(4-chlorophenyl)methyl-4-(3-butanoyl)-7-(5-aminopentoxy)-3,4-dihydro-1H-1,4-benzodiazapine-2,5-dione.
1-(4-chlorophenyl)methyl-4-(3-butanoyl)-7-(4-amidino)benzamido-3,4-dihydro-1H-1,4-benzodiazapine-2,5-dione.
1-(4-chlorophenyl)methyl-4-(3-butanoyl)-7-(4-amidino)benzyloxy-3,4-dihydro-1H-1,4-benzodiazapine-2,5-dione.
1-(4-chlorophenyl)methyl-4-(3-butanoyl)-7-[2-(4-amidinophenyl)]ethenyl-3,4-dihydro-1H-1,4-benzodiazapine-2,5-dione.
1-(4-chlorophenyl)methyl-4-(3-butanoyl)-7-[2-(4-amidinophenyl)]ethynyl-3,4-dihydro-1H-1,4-benzodiazapine-2,5-dione.
1-methyl-4-(3-butanoyl)-7-(4-amidino)benzamido-3,4-dihydro-1H-1,4-benzodiazapine-2,5-dione.
1-methyl-4-(3-butanoyl)-7-(4-amidino)benzyloxy-3,4-dihydro-1H-1,4-benzodiazapine-2,5-dione.
1-methyl-4-(3-butanoyl)-7-[2-(4-amidinophenyl)]ethenyl-3,4-dihydro-1H-1,4-benzodiazapine-2,5-dione.
1-methy-1-4-(3-butanoyl)-7-[2-(4-amidinophenyl)]ethynyl-3,4-dihydro-1H-1,4-benzodiazapine-2,5-dione.
1-(1-methyl)ethyl-4-(3-butanoyl)-7-(4-amidino)benzamido-3,4-dihydro-1H-1,4-benzodiazapine-2,5-dione.
1-(1-methyl)ethyl-4-(3-butanoyl)-7-(4-amidino)benzyloxy-3,4-dihydro-1H-1,4-benzodiazapine-2,5-dione.
1-(1-methyl)ethyl-4-(3-butanoyl)-7-[2-(4-amidinophenyl)]ethenyl-3,4-dihydro-1H-1,4-benzodiazapine-2,5-dione.
1-(2-methyl)ethyl-4-(3-butanoyl)-7-[2-(4-amidinophenyl)]ethynyl-3,4-dihydro-1H-1,4-benzodiazapine-2,5-dione.
1-(2-methyl)propyl-4-(3-butanoyl)-7-(4-amidino)benzamido-3,4-dihydro-1H-1,4-benzodiazapine-2,5-dione.
1-(2-methyl)propyl-4-(3-butanoyl)-7-(4-amidino)benzyloxy-3,4-dihydro-1H-1,4-benzodiazapine-2,5-dione.
1-(2-methyl)propyl-4-(3-butanoyl)-7-[2-(4-amidinophenyl)]ethenyl-3,4-dihydro-1H-1,4-benzodiazapine-2,5-dione.
1-(2-methyl)propyl-4-(3-butanoyl)-7-[2-(4-amidinophenyl)]ethynyl-3,4-dihydro-1H-1,4-benziodiazapine-2,5-dione.
1-(2,4-diflurophenyl)methyl-4-(3-butanoyl)-7-(4-(1-piperizin)-phenyl)-3,4-dihydro-1H-1,4-benzodiazapine-2,5-dione.
1-(2,4-diflurophenyl)methyl-4-(3-butanoyl)-7-(4-(2-aminoethoxy)-phenyl)-3,4-dihydro-1H-1,4-benzodiazapine-2,5-dione.
1-(2,4-diflurophenyl)methyl-4-(3-butanoyl)-7-(6-aminohexyl)-3,4-dihydro-1H-1,4-benzodiazapine-2,5-dione.
1-(2,4-diflurophenyl)methyl-4-(3-butanoyl)-7-(5-aminopentoxy)-3,4-dihydro-1H-1,4-benzodiazapine-2,5-dione.
1-(2,4-diflurophenyl)methyl-4-(3-butanoyl)-7-(4-amidino)benzamido-3,4-dihydro-1H-1,4-benzodiazapine-2,5-dione.
1-(2,4-diflurophenyl)methyl-4-(3-butanoyl)-7-(4-amidino)benzyloxy-3,4-dihydro-1H-1,4-benzodiazapine-2,5-dione.
1-(2,4-diflurophenyl)methyl-4-(3-butanoyl)-7-[2-(4-amidinophenyl)]ethenyl-3,4-dihydro-1H-1,4-benzodiazapine-2,5-dione.
1-(2,4-diflurophenyl)methyl-4-(3-butanyl)-7-[2-(4-amidinophenyl)]ethynyl-3,4-dihydro-1H-1,4-benzodiazapine-2,5-dione.
1-(4-chlorophenyl)methyl-4-(3-butanoyl)-7-[2-(4-piperidinyl)ethyloxy]-3,4-dihydro-1H-1,4-benzodiazapine-2,5-dione.
1-(diphenyl)methyl-4-(3-butanoyl)-7-[2-(4-piperidinyl)ethyloxy]3,4-dihydro-1H-1,4-benzodiazapine-2,5-dione.
1-(*m*-trifluromethyl)phenyl-4-(3-butanoyl)-7-[2-(4-piperidinyl)ethyloxy]-3,4-dihydro-1H-1,4-benzodiazapine-2,5-dione.
1-methyl-4-(3-butanoyl)-7-[2-(4-piperidinyl)ethyloxy]-3,4-dihydro-1H-1,4-benzodiazapine-2,5-dione.
1-(1-methyl)ethyl-4-(3-butanoyl)-7-[2-(4-piperidinyl)ethyloxy]-3,4-dihydro-1H-1,4-benzodiazapine-2,5-dione.
1-(2-methyl)propyl-4-(3-butanoyl)-7-[2-(4-piperidinyl)ethyloxy]-3,4-dihydro-1H-1,4-benzodiazapine-2,5-dione.
1-(2,4-diflurophenyl)methyl-4-(3-butanoyl)-7-[2-(4-piperidinyl)ethyloxy]-3,4-dihydro-1H-1,4-benzodiazapine-2,5-dione.
1-(4-chlorophenyl)methyl-4-[(2-methyl)carboxyethyl]-7-(4-(1-piperizin)-phenyl-3,4-dihydro-1H-1,4-benzodiazapine-2,5-dione.
1-(diphenyl)methyl-4-[(2-methyl)carboxyethyl]-7-(4-(1-piperizin)-phenyl)-3,4-dihydro-1H-1,4-benzodiazapine-2,5-dione.
1-(*m*-trifluromethyl)phenyl-4-[(2-methyl)carboxyethyl]-7-(4-(1-pipenzin)-phenyl)-3,4-dihydro-1H-1,4-benzodiazapine-2,5-dione.
1-methyl-4-[(2-methyl)carboxyethyl]-7-(4-(1-piperizin)-phenyl)-3,4-dihydro-1H-1,4-benzodiazapine-2,5-dione.
1-(1-methyl)ethyl-4-[(2-methyl)carboxyethyl]-7-(4-(1-piperizin)-phenyl)-3,4-dihydro-1H-1,4-benzodiazapine-2,5-dione.
1-(2-methyl)propyl-4-[(2-methyl)carboxyethyl]-7(4-(1-piperizin)-phenyl-3,4-dihydro-1H-1,4-benzodiazapine-2,5-dione.
1-(4-chlorophenyl)methyl-4-[(2-methyl)carboxyethyl]-7-(4-(2-aminoethoxy)-phenyl)-3,4-dihydro-1H-1,4-benzodiazapine-2,5-dione.
1-(diphenyl)methyl-4-[(2-methyl)carbioxyethyl]-7-(4-(2-aminoethoxy)-phenyl)-3,4-dihydro-1H-1,4-benzodiazapine-2,5-dione.
1-(*m*-trifluromethyl)phenyl-4-[(2-methyl)carboxyethyl]-7-(4-(2-aminoethoxy)-phenyl)-3,4-dihydro-1H-1,4-benzodiazapine-2,5-dione.
1-methyl-4-[(2-methyl)carboxyethyl]-7-(4-(2-aminoethoxy)-phenyl)-3,4-dihydro-1H-1,4-benzodiazapine-2,5-dione.
1-(1-methyl)ethyl-4-[(2-methyl)carboxyethyl]-7-(4-(2-aminoethoxy)-phenyl)-3,4-dihydro-1H-1,4-benzodiazapine-2,5-dione.
1-(2-methyl)propyl-4-[(2-methyl)carboxyethyl]-7-(4-(2-aminoethoxy)-phenyl)-3,4-dihydro-1H-1,4-benzodiazapine-2,5-dione.
1-(diphenyl)methyl-4-[(2-methyl)carboxyethyl]-7-(6-aminohexyl)-3,4-dihydro-1H-1,4-benzodiazapine-2,5-dione.
1-(diphenyl)methyl-4-[(2-methyl)carboxyethyl]-7-(6-aminohex-1-ynyl)-3,4-dihydro-1H-1,4-benzodiazapine-2,5-dione.
1-(diphenyl)methyl-4-[(2-methyl)carboxyethyl]-7-(5-aminopentoxy)-3,4-dihydro-1H-1,4-benzodiazapine-2,5-dione.
1-(diphenyl)methyl-4-[(2-methyl)carboxyethyl]-7-(5-guanidinopentyl)-3,4-dihydro-1H-1,4-benzodiazapine-2,5-dione.
1-(diphenyl)methyl-4-[(2-methyl)carboxyethyl)-7-(4-guanidinobutoxy)-3,4-dihydro,-1H-1,4-benzodiazapine-2,5-dione.
1-(diphenyl)methyl-4-[(2-methyl)carboxyethyl]-7-(5-aminopent-1-ynyl)-3,4-dihydro-1H-1,4-benzodiazapine-2,5-dione.
1-(diphenyl)methyl-4-[(2-methyl)carboxyethyl]-7-(5-guanidinopent-1-ynyl)-3,4-dihydro-1H-1,4-benzodiazapine-2,5-dione.
1-(diphenyl)methyl-4-[(2-methyl)cartoxyethyl]-7-(4-amidino)benzamido-3,4-dihydro-1H-1,4-benzodiazapine-2,5-dione.
1-(diphenyl)methyl-4-[(2-methyl)carboxyethyl]-7-(4-amidino)benzyloxy-3,4-dihydro-1H-1,4-benzodiazapine-2,5-dione.
1-(diphenyl)methyl-4-[(2-methyl)carboxyethyl]-7-[2-(4-amidinophenyl)]ethenyl-3,4-dihydro-1H-1,4-benzodiazapine-2,5-dione.
1-(diphenyl)methyl-4-[(2-methyl)carboxyethyl]-7-[2-(4-amidinophenyl)]ethynyl-3,4-dihydro-1H-1,4-benzodiazapine-2,5-dione.
1-(4-chlorophenyl)methyl-4-[(2-methyl)carboxyethyl]-7-(6-aminohexyl)-3,4-dihydro-1H-1,4-benzediazapine-2,5-dione.
1-(4-chlorophenyl)methyl-4-[(2-methyl)carboxyethyl]-7-(5-aminopentoxy)-3,4-dihydro-1H-1,4-benzodiazapine-2,5-dione.
1-(4-chlorophenyl)methyl-4-(2-methyl)carboxyethyl]-7-(4-amidino)benzamido-3,4-dihydro-1H-1,4-benzodiazapine-2,5-dione.
1-(4-chlorophenyl)methyl-4-[(2-methyl)carboxyethyl]-7-(4-amidino)benzyloxy-3,4-dihydro-1H-1,4-benzodiazapine-2,5-dione.
1-(4-chlorophenyl)methyl-4-[(2-methyl)carboxyethyl]-7-[2-(4-amidinophenyl)]ethenyl-3,4-dihydro-1H-1,4-benzodiazapine-2,5-dione.
1-(4-chlorophenyl)methyl-4-[(2-methyl)carboxyethyl]-7-[2-(4-amidinophenyl)]ethynyl-3,4-dihydro-1H-1,4-benzodiazapine-2,5-dione.
1-methyl-4-[(2-methyl)carboxyethyl]-7-(4-amidino)benzamido-3,4-dihydro-1H-1,4-benzodiazapine-2,5-dione.
1-methyl-4-[(2-methyl)carboxyethyl]-7-(4-amidino)benzyloxy-3,4-dihydro-1H-1,4-benzodiazapine-2,5-dione.
1-methyl-4-(2-methyl)carboxyethyl]-7-[2-(4-amidinophenyl)]ethenyl-3,4-dihydro-1H-1,4-benzodiazapine-2,5-dione.
1-methyl-4-[(2-methyl)carboxyethyl]-7-[2-(4-amidinophenyl)]ethynyl-3,4-dihydro-1H-1,4-benzodiazapine-2,5-dione.
1-(1-methyl)ethyl-4-[(2-methyl)carboxyethyl]-7-(4-amidino)benzamido-3,4-dihydro-1H-1,4-benzodiazapine-2,5-dione.
1-(1-methyl)ethyl-4-[(2-methyl)carboxyethyl]-7-(4-amidino)benzyloxy-3,4-dihydro-1H-1,4-benzodiazapine-2,5-dione.
1-(1-methyl)ethyl4-[(2-methyl)carboxyethyl]-7-[2-(4-amidinophenyl)]ethenyl-3,4-dihydro-1H-1,4-benzodiazapine-2,5-dione.
1-(2-methyl)ethyl-4-[(2-methyl)carboxyethyl]-7-[2-(4-amidinophenyl)]ethynyl-3,4-dihydro-1H-1,4-benzodiazapine-2,5-dione.
1-(2-methyl)propyl-4-[(2-methyl)carboxyethyl]-7-(4-amidino)benzamido-3,4-dihydro-1H-1,4-benzodiazapine-2,5-dione.
1-(2-methyl)propyl-4-[(2-methyl)carboxyethyl]-7-(4-amidino)benzyloxy-3,4-dihydro-1H-1,4-benzodiazapine-2,5-dione.
1-(2-methyl)propyl-4-[(2-methyl)carboxyethyl]-7-[2-(4-amidinophenyl)]ethenyl-3,4-dihydro-1H-1,4-benzodiazapine-2,5-dione.
1-(2-methyl)propyl-4-[(2-methyl)carboxyethyl]-7-[2-(4-amidinophenyl)]ethynyl-3,4-dihydro-1H-1,4-benzadiazapine-2,5-dione.
1-(2,4-diflurophenyl)methyl-4-[(2-methyl)carboxyethyl]-7-(4-(1-piperizin)-phenyl)-3,4-dihydro-1H-1,4-benzodiazapine-2,5-dione.
1-(2,4-diflurophenyl)methyl-4-[(2-methyl)carboxyethyl]-7-(4-(2-aminoethoxy)-phenyl)-3,4-dihydro-1H-1,4-benzodiazapine-2,5-dione.
1-(2,4-diflurophenyl)methyl-4-[(2-methyl)carboxyethyl]-7-(6-aminohexyl)-3,4-dihydro-1H-1,4-benzodiazapine-2,5-dione.
1-(2,4-diflurophenyl)methyl-4-[(2-methyl)carboxyethyl]-7-(5-aminopentoxy)-3,4-dihydro-1H-1,4-benzodiazapine-2,5-dione.
1-(2,4-diflurophenyl)methyl-4-[(2-methyl)carboxyethyl]-7-(4-amidino)benzamido-3,4-dihydro-1H-1,4-benzodiazapine-2,5-dione.
1-(2,4-diflurophenyl)methyl-4-[(2-methyl)carboxyethyl]-7-(4-amidino)benzyloxy-3,4-dihydro-1H-1,4-benzodiazapine-2,5-dione.
1-(2,4-diflurophenyl)methyl-4-[(2-methyl)carboxyethyl]-7-[2-(amidinophenyl)]ethenyl-3,4-dihydro-1H-1,4-benzodiazapine-2,5-dione.
1-(2,4-diflurophenyl)methyl-4-[(2-methyl)carboxyethyl]-7-[2-(4-amidinophenyl)]ethynyl-3,4-dihydro-1H-1,4-benzodiazapine-2,5-dione.
1-(4-chlorophenyl)methyl-4-[(2-methyl)carhoxyethyl]-7-[2-(4-piperidinyl)ethyloxy]-3,4-dihydro-1H-1,4-benzodiazapine-2,5-dione.
1-(diphenyl)methyl-4-[(2-methyl)carboxyethyl]-7-[2-(4-piperidinyl)ethyloxy]3,4-dihydro-1H-1,4-benzodiazapine-2,5-dione.
1-(*m*-trifluromethyl)phenyl-4-[(2-methyl)carboxyethyl]-7-[2-(4-piperidinyl)ethyloxy]-3,4-dihydro-1H-1,4-benzodiazapine-2,5-dione.
1-methyl-4-[(2-methyl)carboxyethyl]-7-[2-(4-piperidinyl)ethyloxy]-3,4-dihydro-1H-1,4-benzodiazapine-2,5-dione.
1-(1-methyl)ethyl-4-[(2-methyl)carboxyethyl]-7-[2-(4-piperidinyl)ethyloxy]-3,4-dihydro-1H-1,4-benzodiazapine-2,5-dione.
1-(2-methyl)propyl-4-[(2-methyl)carboxyethyl]-7-[2-(4-piperidinyl)ethyloxy]-3,4-dihydro-1H-1,4-benzodiazapine-2,5-dione.
1-(2,4-diflurophenyl)methyl-4-[(2-methyl)carboxyethyl]-7-[2-(4-piperidinyl)ethyloxy]-3,4-dihydro-1H-1,4-benzodiazapine-25-dione.
1-(3-(butyryl)aminophenyl)methyl-4-(2-carboxyethyl)-7-(6-aminohexyl)-3,4-dihydro-1H-1,4-benzodiazapine-2,5-dione.
1-(3-(butyryl)aminophenyl)methyl-4-(2-carboxyethyl)-7-(5-guanidinopentyl)-3,4-dihydro-1H-1,4-benzodiazapine-2,5-dione.
1-(3-(4-aminobutyryl)aminophenyl)methyl-4-(2-carboxyethyl)-7-(6-aminohexyl)-3,4-dihydro-1H-1,4-benzodiazapine-2,5-dione.
1-(3-(4-guanidinobutyryl)aminophenyl)methyl-4-(2-carboxyethyl)-7-(5-guanidinopentyl)-3,4-dihydro-1H-1,4-benzodiazapine-2,5-dione.
1-(3-(6-aminohexoyl)aminophenyl)methyl-4-(2-carboxyethyl)-7-(6-aminohexyl)-3,4-dihydro-1H-1,4-benzodiazapine-2,5-dione.
1-(3-(6-guanidinohexoyl)aminophenyl)methyl-4-(2-carboxyethyl)-7-(5-guanidinopentyl)-3,4-dihydro-1H-1,4-benzodiazapine-2,5-dione.
1-(3-(6-aminopentoyl)aminophenyl)methyl-4-(2-carboxyethyl)-7-(6-aminohexyl)-3,4-dihydro-1H-1,4-benzodiazapine-2,5-dione.
1-(3-(6-guanidinopentoyl)aminophenyl)methyl-4-(2-carboxyethyl)-7-(5-guanidinopentyl)-3,4-dihydro-1H-1,4-benzodiazapine-2,5-dione.
1-(3-(6-aminopropionyl)aminophenyl)methyl-4-(2-carboxyethyl)-7-(6-aminohexyl)-3,4-dihydro-1H-1,4-benzodiazapine-2,5-dione.
1-(3-(6-guanidinopropionyl)aminophenyl)methyl-4-(2-carboxyethyl)-7-(5-guanidinopentyl)-3,4-dihydro-1H-1,4-benzodiazapine-2,5-dione.
1-(3-(6-aminoacetyl)aminophenyl)methyl-4-(2-carboxyethyl)-7-(6-aminohexy)-3,4-dihydro-1H-1,4-benzodiazapine-2,5-dione.
1-(3-(6-guanidinoacetyl)aminophenyl)methyl-4-(2-carboxyethyl)-7-(5-guanidinopentyl)-3,4-dihydro-1H-1,4-benzodiazapine-2,5-dione.
1-(3-(4-guanidinobenzoyl)aminophenyl)methyl-4-(2-carboxyethyl)-7-(6-aminohexyl)-3,4-dihydro-1H-1,4-benzodiazapine-2,5-dione.
1-(4-(6-aminopropionyl)aminophenyl)methyl-4-(2-carboxyethyl)-7-(6-aminohexyl)-3,4-dihydro-1H-1,4-benzodiazapine-2,5-dione.
1-(3-(4-aminobutyl)phenyl)methyl-4-(2-carboxyethyl)-7-(6-aminohexyl)-3,4-dihydro-1H-1,4-benzodiazapine-2,5-dione.
1-(4-carboxyphenyl)methyl-4-(2-carhoxyethyl)-7-(6-aminohexyl)-3,4-dihydro-1H-1,4-benzodiazapine-2,5-dione.

### 2. Nonpeptidyl αᵥβ₃ inhibitors

An alternative embodiment of the invention comprises a compound represented by formula VII capable of inhibiting binding of the osteoclast αᵥβ₃ receptor to its native *in vivo* ligands. 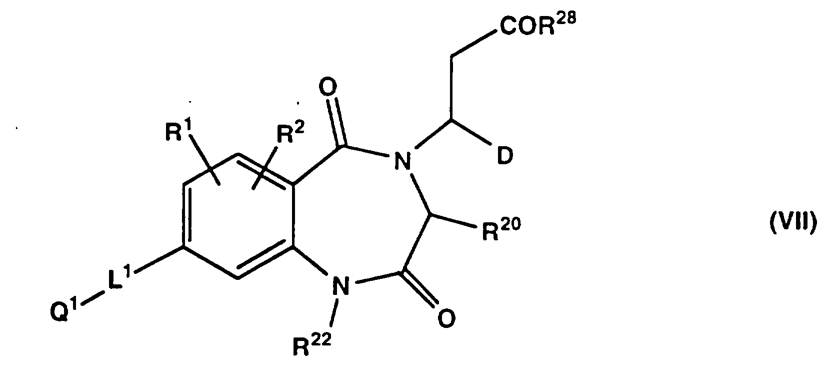 where R¹, R, R⁰, R, R⁸ Q¹, and L¹ are defined above.

Compounds represented by formula VII are potent inhibitors of the vitronectin-vitronectin receptor (Vn-VnR) interaction, typically yielding IC₅₀'s in a Vn-VnR ELISA of between 150 and 200 nM. These compounds show roughly a 20-fold selectivity when compared with the GPII_{b}IIIₐ-Fg inhibition.

### D. Methods of Making

Compounds of the present invention can be prepared by many methods, employing standard chemical methodologies described and referenced in standard textbooks (e.g. March, J. "Advanced Organic Chemistry" McGraw-Hill, New York, 1977; Collman, J.P., Hegedus, L.S., Norton, J.R., Finke, R.G. "Principles and Applications of Organotransition Metal Chemistry" University Science, Mill Valley, 1987; Larock, R.C. "Comprehensive Organic Transformations" Verlag, New York, 1989). In the description that follows standard abbreviations as recommended by the *Journal of Organic Chemistry* (see "Guidelines for Authors" in any volume) are employed unless otherwise specified.

### 1. The Q Group

The nitrogen containing substituents Q or a precursor thereof may added to the linker L and the combination Q-L-, usually in protected form, may be bonded to ring A. Alternatively, Q or Q plus a portion of L may be added to L or a portion thereof after ring A and the rest of the molecule have been formed. Q itself may be prepared and bonded to ring A by standard methods published in both the scientific and patent literature (see e.g. US patents; 4,992,542, 4,997,936,4,194,047,5,003,076,5,063,207,5,063,208, and 5,079,357, and references cited therein).

In the description immediately following, addition of Q-L- to the benzene moiety of 3,4-dihydro-1*H*-1,4-benzodiazepine-2,5-dione is specified. It will be understood that these same procedures may be applied to other ring systems encompassed by formula I.

### 2. The benzodiazepinedione

The key intermediate is the substituted 3,4-dihydro-1*H*-1,4-benzodiazepine-2,5-dione 1. The benzodiazepinedione 1 (R≠H) may be prepared using a triply convergent approach from the isatoic anhydride 2, the b-alanine ester 3, and the a-haloacetyl halide 4 (Scheme 1). The benzodiazepinedione 1 (R=H) may be prepared using a doubly convergent approach from the isatoic anhydride 2 (R=H) and the substituted N-carboxymethyl b-alanine ester 5 (Scheme 2).

Methods for preparation of the isatoic anhydrides 2, b-alanine esters 3, and a-halo acetyl halides 4 (Scheme 1), are known in the art and a number of them are available from commercial sources such as Aldrich Chemical Co. A reaction sequence, similar to that shown in Scheme 1 to prepare 3,4-dihydro-1*H*-1,4-benzodiazepine-2,5-diones has been previously described (Lee, C.M. *J*. *Heterocyclic Chem.* 1: 235 (1964). Briefly, the isatoic anhydrides are converted to N-(2-aminobenzoyl)-b-alanine esters 6 by allowing 2 to react with 3, or its salt, in the presence an organic base. The isatoic anhydride 2 may be substituted or unsubstituted, but the 5-iodo isatoic anhydride is preferred (2, R^{a}=I). The b-alanine ester 3 may also be substituted or unsubstituted as the free amine or, more conveniently, as its salt (e.g. HCI). For example, the reaction may be carried out with b-alanine ethyl ester hydrochloride, ethyl 3-phenyl-3-aminopropionate p-tosylate, aspartyl-valine dibenzyl ester hydrochloride, or the like. Generally, the reaction is conducted in a dry polar aprotic solvent, such as dimethylformamide or the like, in the presence of an equimolar amount, or up to 30% excess, of a tertiary amine as the organic base, e.g. triethylamine. Alternatively, the reaction may be catalysed by dimethylaminopyridine (Venuti, M.C. *Synthesis* 266 (1982)). The reaction requires between about 0.5 to 4 hours at temperatures between about room temperature and 100°C . Preferably, the reaction is conducted at 50°C for about 2 hours in dimethylformamide as the solvent.

The products are isolated and purified by conventional methods, typically by solvent extraction into a compatible solvent. The product may be further purified by column chromatography or other appropriate methods.

If the anilino nitrogen in N-(2-aminobenzoyl)-b-alanine esters 6a is unsubstituted such that R=H, this position can be alkylated by allowing 6b (R=H) to react with a molar amount, but up to 50% excess, of an alkyl halide to produce 6 (R≠H). Generally, the reaction can be accomplished in a polar aprotic solvent, in the presence of a molar amount, or up to 50% excess, of an organic base. Preferably, the reaction will be conducted in dimethylformamide as solvent and 2,6-lutidine as the organic base. The reaction is allowed to proceed at temperatures between about room temperature to 200°C for about 0.5 hours to 2 days. Preferably, the reaction will be run at 80°C for 16 hours. The products are isolated and purified by conventional methods, typically by solvent extraction into a compatible solvent. The product may be further purified by column chromatography or other appropriate methods.

Alternatively, 6 (R≠H) may be prepared by allowing 6a to react with an aldehyde or ketone, under dehydrating conditions, in the presence of a catalytic amount of acid followed by reduction with a trialkylsilane and a strong acid. Generally, the first of these reactions can be accomplished in a non-polar aprotic solvent, such as toluene, and an organic acid, such as p-toluenesulphonic acid at temperatures between 80-160°C for times between 1 to 60 mins. Preferably, the reaction will be conducted at reflux for 10 mins. The product may be isolated by crystallization or carried on to reduction without further purification. Reduction can be achieved by the addition of an excess of trialkylsilane, such as triethylsilane, in the presence of an excess of a strong acid, such as trifluoroacetic acid, at temperatures between -30 to 30°C for times between 0.5 to 48 hours with a non-reactive organic solvent, such as dichloromethane. Preferably, reduction is run at 0°C for 2 hours with dichloromethane as a co-solvent. The products are isolated and purified by conventional methods, typically by crystallization from suitable solvents. The product may be otherwise purified by solvent extraction into a compatible solvent followed by column chromatography or other appropriate methods.

Conversion of N-(2-aminobenzoyl)-b-alanine esters 6 to the benzodiazepinedione 1 (R≠H) involves first the acylation of the anilino nitrogen, followed by a base promoted ring formation. The acylation reaction can be achieved by either reaction of 6 with an a-haloacyl halide in the presence of an equimolar amount, or up to 30% excess, of an organic base, such as triethylamine, or with an a-haloacid in the presence of an amide coupling reagent, such as dicyclohexylcarbodiimide Acylation of 6 with an a-haloacyl halide may also be conducted in the absence of an organic base and can be run with water in a biphasic reaction system. Generally, these reactions are conducted in a pre-dried, non-reactive organic solvent, such as methylene chloride, diethyl ether, or tetrahydrofuran under a dry, inert atmosphere, such as nitrogen. Preferably, acylation with an a-halo acylhalide will be run in methylene chloride, whereas acylation with an a-halo-acid will be run in tetrahydrofuran. The reactions are allowed to run at temperatures between about 0°C to room temperature for times between about 0.5 to 24 hours. Preferably, the reactions will be allowed to run for 2 hours at room temperature. The products are isolated and purified by conventional methods, typically by solvent extraction into a compatible solvent. Generally, further purification of the acylated products 7 is not required.

Cyclization of the acylated N-(2-acylaminobenzoyl)-b-alanine esters *7* may be achieved by reaction with a base such as an alkali metal alkoxide, hydride, or carbonate in a polar solvent at temperatures between about 0 and 100°C for about 0.5 to 2 hours. For example, a solution of the N-(2-aminobenzoyl)-b-alanine ester 7 is commonly added over a period of approximately 15 to 60 minutes to a slurry of an alkali metal hydride in an appropriate solvent, cooled to 0°C. 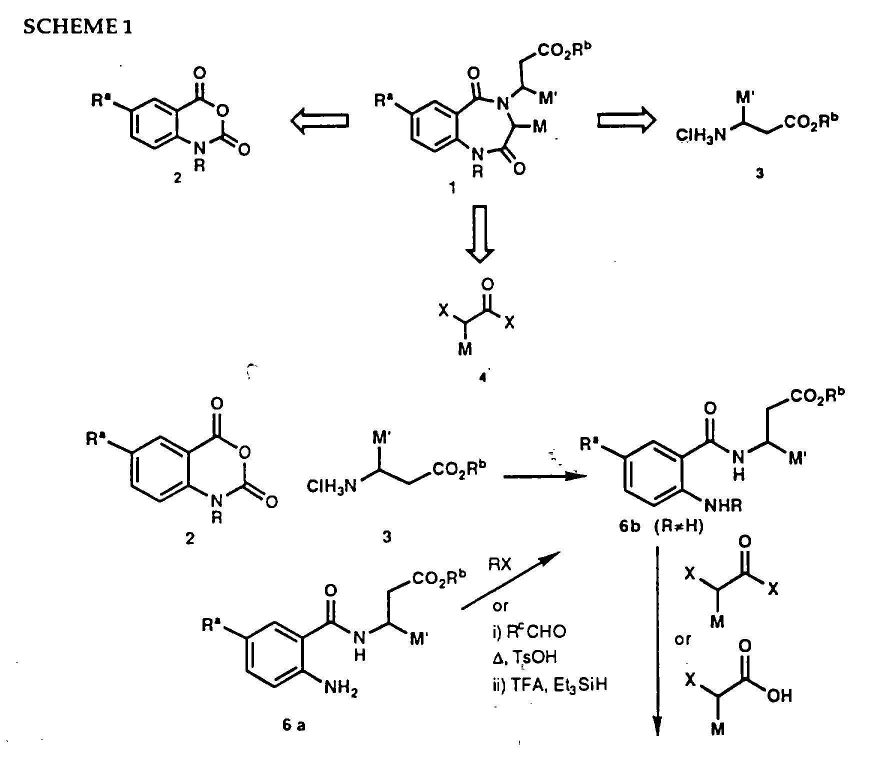 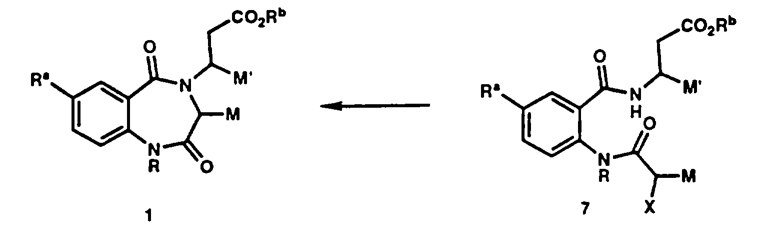 It is preferable that the solvent will be a polar aprotic solvent such as dimethylfomamide and the alkali metal hydride be sodium hydride or calcium carbonate. Once the addition is complete, the reaction mixture is generally allowed to warm to room temperature and run for an additional 60 to 105 minutes after which the reaction is neutralized by the addition of a solution of an acid, such as 10% citric acid or the like, and the solvent evaporated. The product is then isolated by solvent extraction and further purified by column chromatography.

In Scheme 2, the substituted N-carboxymethyl b-alanine esters 5 are also known in the art and may be prepared in two steps by first reaction of an acrylate ester and a-amino acid ester to give 8, followed by selective conversion of the glycine ester group to its corresponding acid. For example, allowing glycine benzyl ester to react with ethyl acrylate in an alcoholic solvent such as methanol for about 1 day yields N-(carboxymethyl benzyl ester)-b-alanine ethyl ester. Removal of the benzyl ester by hydrogenation in the presence of a catalytic amount 10% palladium on carbon provides N-carboxymethyl-b-alanine ester 5 (M'=H, R^{b}=ethyl).

Preparation of the benzodiazepinedione 1 (R=H) may be accomplished by allowing 5 to react with 2 (R=H). The reaction can be conducted in either a 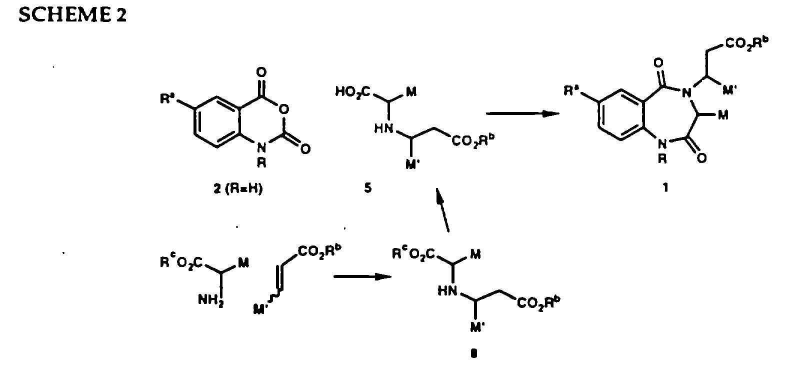 polar aprotic solvent, such as dimethyl sulfoxide, or weak organic base, such as pyridine, at temperatures of 100-200 °C for about 2-86 hours. Preferably, the reaction will be run in pyridine at refluxing temperature for about 24 hours. The product is then isolated by dilution with an appropriate organic solvent, such as ethyl acetate, and washed with aqueous acid, such as 10% citric acid, and aqueous base, such as saturated sodium bicarbonate. The products can be further purified by column chromatography or crystallization from an appropriate organic solvent to provide the compounds 1 (R=H).

Reaction Scheme 3 outlines the preparation of the compounds of the present invention from compound 9 [1 (R^{a}=I)].

Conversion of the compounds having the structural formula 9 to compounds having the structural formula 10, provides those compounds classified in Groups 1 and 2. This may be accomplished by allowing 9 to react with an alkyne 11 in the presence of a palladium (II) salt, a copper (I) salt, and an organic base. Preferably, the alkyne 11 is substituted in a way such that the Y group can be synthetically converted to the positively charged Q group of structural Formula I. More preferably, the Y group is a protected form of the positively charged Q group of structural Formula I. For example, the alkyne may be a N-Boc-amino alkyne, a benzo or alkylnitrile alkyne, a nitrobenzo alkyne, or the like. L' is L less the functional groups shown below in scheme 3.

Generally, the reaction is allowed to run in a dry organic polar aprotic solvent, such as ethyl acetate or the like, to the exclusion of oxygen at temperatures between about 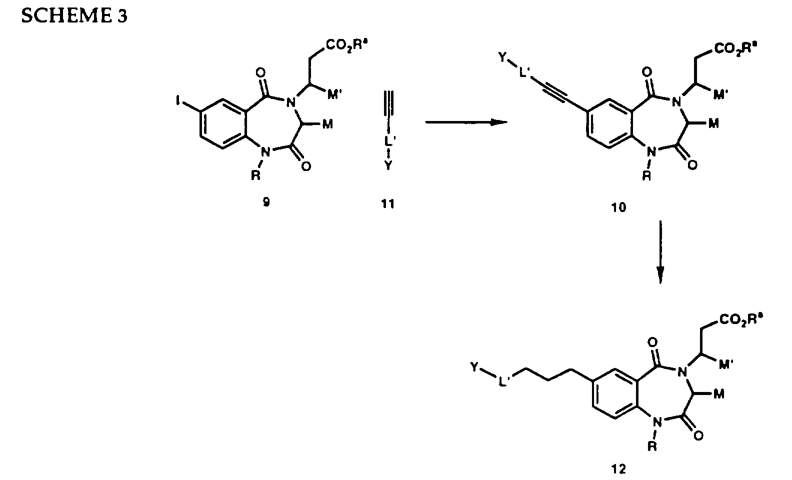 room temperature and 180°C for times between about 2 and 48 hours. Preferably, the reaction will run with a catalytic amount of palladium(II) salt at 10 molar percent and cupric(I) salt at 5 molar percent. A 2 fold excess of the alkyne 11 and a 5 fold excess of a tertiary amine as the organic base, such as triethylamine or the like, is preferred. For example, a mixture of the iodo arene 9, a 2 fold excess of the alkyne 11, 10 molar percent of bis(triphenylphosphine)palladium dichloride, 5 molar percent cupric iodide, 5 fold excess of triethylamine, and ethyl acetate under a dry, inert atmosphere, such as nitrogen, is allowed run for about 2 hours. The product can be isolated by solvent extraction in to a suitable organic solvent, such as ethyl acetate, and washed with a solution of 10% ethylenediaminetetraacetic acid and the solvent evaporated. The products may be further purified by column chromatography.

Preparation of the compounds classified in Groups 3 and 4 may be achieved by reduction of the alkyne moiety in compounds of the general formula 10 to yield the saturated compounds 12 by allowing the alkyne to stir under an atmosphere of hydrogen in the presence of a small amount of palladium on carbon. Typically, the reaction is run in an inert solvent, such as ethyl acetate, with a 5-10 molar percent by weight of 10% palladium on carbon at temperatures between about room temperature and 50°C for times between about 15 - 240 minutes. Preferably, the reaction is carried out at room temperature for 1 hour. The products are isolated by filtration of the mixture through a filter agent, such as Celite®, and evaporation of solvent.

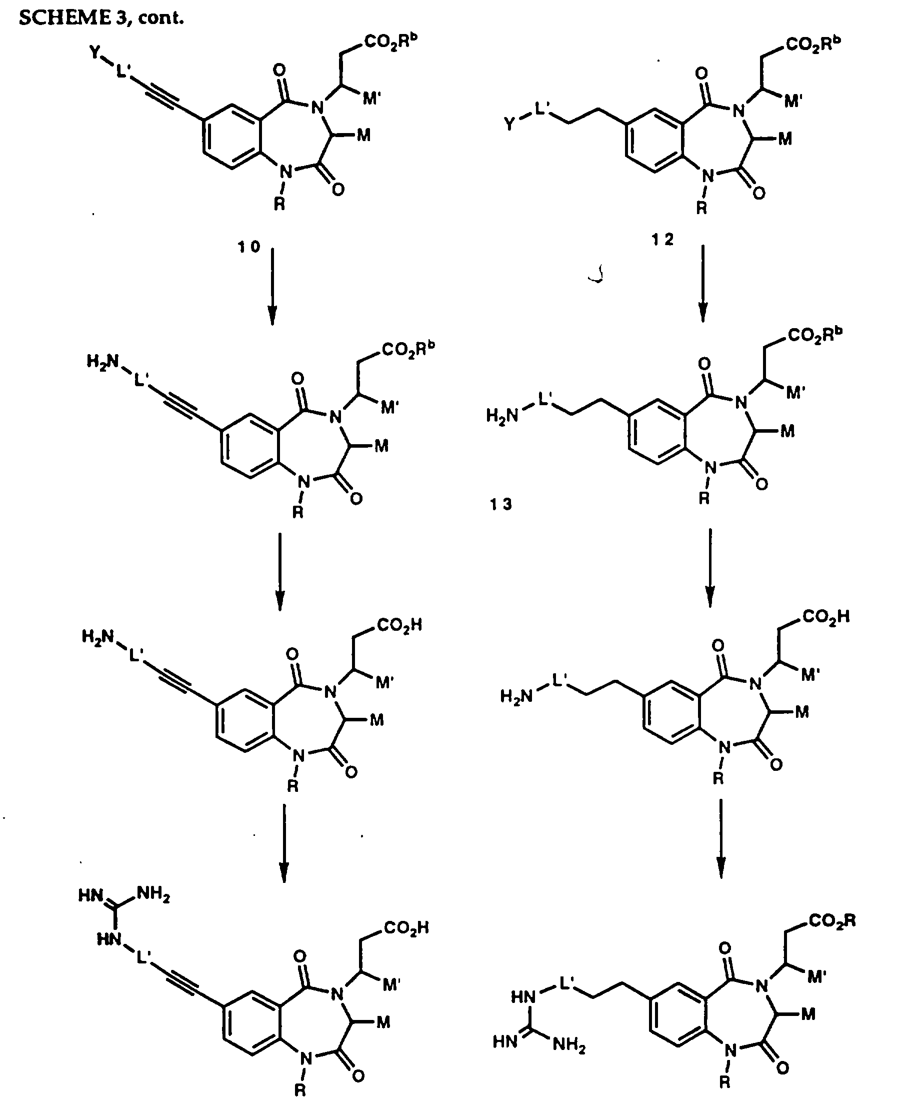

Conversion of the compounds of the general formula 10 or 12 in which Y is a N-(*tert*-butoxycarbonyl) amino protected moiety (N-BOC) to the amino esters 13 can be accomplished by allowing the material to react with a strong acid. Generally, the reaction is carried out by mixing the N-BOC amino ester with a large excess of concentrated solution of a strong acid, such as hydrogen chloride, dissolved in an appropriate inert solvent, such as ethyl acetate. The reaction can be conducted at a temperature between about minus 30°C and room temperature for about 0.5 to 24 hours. The reaction can be run in the presence of a molar amount of trialkylsilane, such as triethylsilane or the like. Preferably, the reaction will be carried out at room temperature for 2 hours with a molar equivalent of triethylsilane in ethyl acetate. The solvent is evaporated and the products can be further purified by high-pressure liquid chromatography using a reverse phase column.

Conversion of the compounds of the general formula 10 or 12 in which the Y functional group is an arylnitrile to the amino esters 13 can be achieved by allowing the arylnitriles 10 or 12 to react with dicobalt octacarbonyl in the presence of trimethylsilane. The reduction of a benzonitrile to a benzylamine is a known reaction (Murai, T.; Sakane, T.; Kato, S. *Tetrahedron Lett*. 26: 5145-5148[1985]) and is generally carried out in an inert solvent, such as toluene or the like, at 60°C for 20 hours with an 8 molar percent of dicobalt octacarbonyl and a 10 fold excess of trimethylsilane. The solvent is evaporated and the resulting material diluted with methanol and allowed to react with a 5 fold excess of potassium fluoride. The products are then isolated by solvent extraction and further purified high-pressure liquid chromatography using a reverse phase column.

Conversion of the compounds of the general formula 10 or 12 in which the Y functional group is a nitroarene, to the amino ester 13 can be executed by a selective reduction (Bellamy, F.D.; Ou, K. *Tetrahedron Lett*. 25: 839-842[1984]). The reaction is generally run with a five molar excess of stannous chloride dihydrate in either ethyl acetate or ethanol as the solvent at temperatures between about 50 and 100°C for times between about 15-120 minutes under an inert atmosphere, such as nitrogen. Preferably, the reaction is carried out at 70°C in ethanol for approximately 30 minutes. The products are then isolated by solvent extraction and further purified by high-pressure liquid chromatography using a reverse phase column.

Conversion of the amino esters to their corresponding amino acids of the structural formula I involves saponification using well known conditions and reagents. For example, an aqueous solution of a strong alkali metal base, such as sodium hydroxide, lithium hydroxide or the like, is added to an alcoholic solution of the ester. Alcohols which may be used as the solvent for this reaction may include, for example, methanol, ethanol, and isopropanol, but, methanol is preferred. The preferred base is sodium hydroxide at a concentration between about 1 to 6N, though 2N is preferred. The reaction may be conducted at a temperature between about 0 to 50°C for times between about 10 to 60 minutes. Preferably, the reaction is carried out at room temperature for 30 minutes after which the reaction is neutralized with a concentrated solution of a strong acid, such as hydrochloric acid or the like, and the solvent evaporated. The products are isolated by high-pressure liquid chromatography using a reverse phase column.

The conversion of the amino acids to their corresponding guanidino acids of the structural formula I is a known reaction (Kim, K. Lin, Y.-L.; Mosher, H.S. *Tetrahedron Lett.* 3183- 3186 [1988]). The reaction can be accomplished by allowing the amino acid to react with aminoiminomethanesulfonic acid. Generally, the reaction can be conducted with a equimolar to a 10 fold molar excess of aminoiminomethanesulfonic acid at temperatures between 0 to 50°C for times between about 15 to 120 minutes in a polar protic solvent, such as methanol, water or the like. The solution, prior to the addition of aminoiminomethanesulfonic acid, may be made neutral or basic by the addition of weak base, such as an alkali metal carbonate. Preferably, for alkyl amines the amino acid will be allowed to react with a 5 fold excess aminoiminomethanesulfonic acid at room temperature for 30 minutes with 5% potassium bicarbonate in water as the reaction medium, whereas for aryl amines the amino acid will be allowed to react with an equimolar amount of aminoiminomethanesulfonic acid at room temperature for 1 hour in methanol. Generally, the reaction mixture will be made acidic by the addition of a dilute solution of an acid, such as acetic acid and the solvent evaporated. The products are isolated by high-pressure liquid chromatography using a reverse phase column.

Preparation of the compounds classified as Groups 4-11 may also be prepared from the key intermediate 9. Conversion of the iodoarene 9 to a synthetic precursors of those compounds found in Groups 5-11 namely compounds 15-18, may be accomplished by a multistep reaction sequence (Scheme 4). First, the iodoarene is carbonylated in an alcoholic solvent (R'OH) under a carbon monoxide atmosphere utilizing a palladium (0) catalyst. The alcoholic solvent (R'OH) must be chosen so as to allow for the selective removal of the R' group of the diester 14. For example, when R' = CH₃ and R^{b}= *tert*-butyl, the alcoholic solvent is methanol, and this methyl ester functionality in 14 can be removed by mild basic hydrolysis to afford acid 15. The treatment of 15 with a molar equivalent of diphenylphosphoryl azide and a molar equivalent of a tertiary amine such as triethylamine at room temperature in a non-protic solvent such as dichloromethane affords the isocyanate 16. This may be hydrolyzed to the amine 17 by the addition of water to a solution of 16 in tetrahydrofuran at room temperature. Finally, conversion of the aniline 17 to its corresponding diazonium salt by treatment with nitrous acid and subsequent heating of the diazonium salt in the presence of water produces the phenol 18.

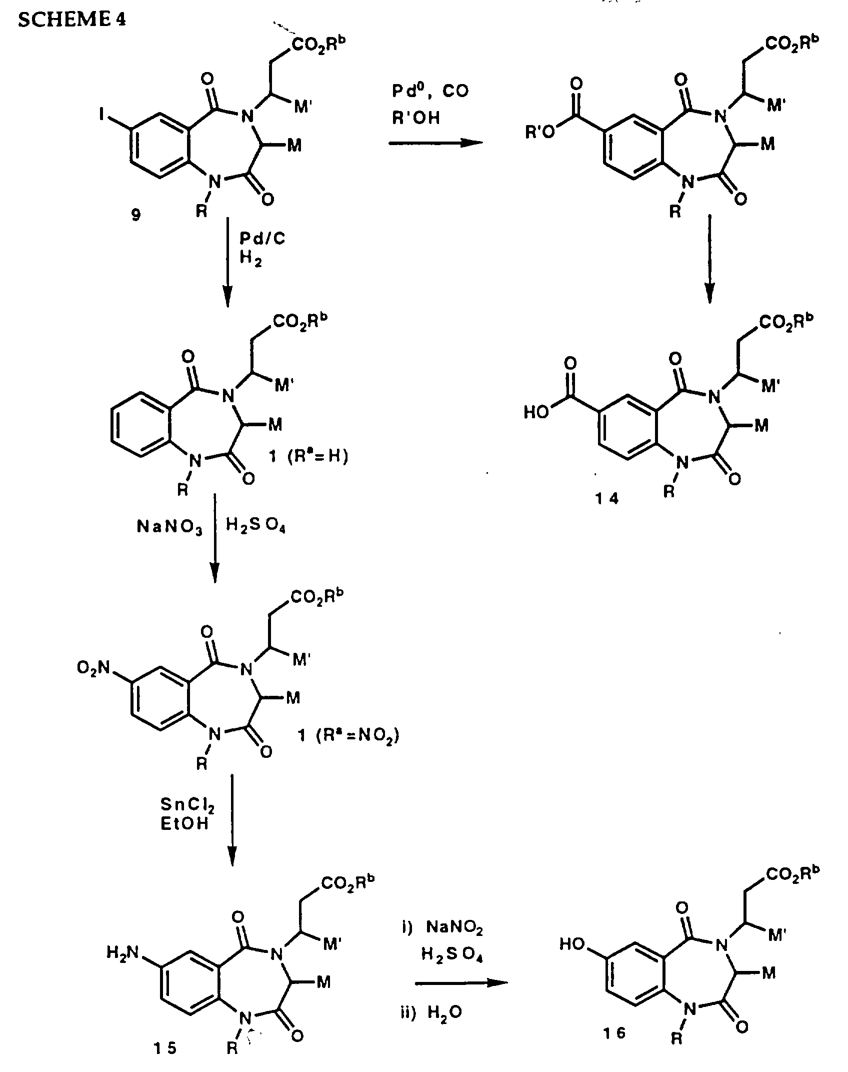

Schemes 5 and 6 depict methods that may be used to introduce the linking groups of compounds classified as Groups 4-11 onto the benzodiazepinedione nucleus. For example, preparation of the biaryl adducts 19, precursors of Group 5 type compounds, may be accomplished by allowing 9 to react with substituted aryl molecules 20, where Y and L' are defined as above, in the presence of palladium(0). Allowing the carboxylic acid 15 to react with an amine 21 or alcohol 22 in the presence of a dehydrating reagent, such as dicyclohexylcarbodiimide (DCC) or the like, will yield the amide 23 and ester 24, forerunners of Group 10 and 11 type compounds, respectively. Similarly, 25 can be prepared from the aniline 17 by reaction with a carboxylic acid 26 in the presence of a coupling reagent, such as DCC or the like. Compounds of the general formula 25 are precursors of Group 5 type compounds.

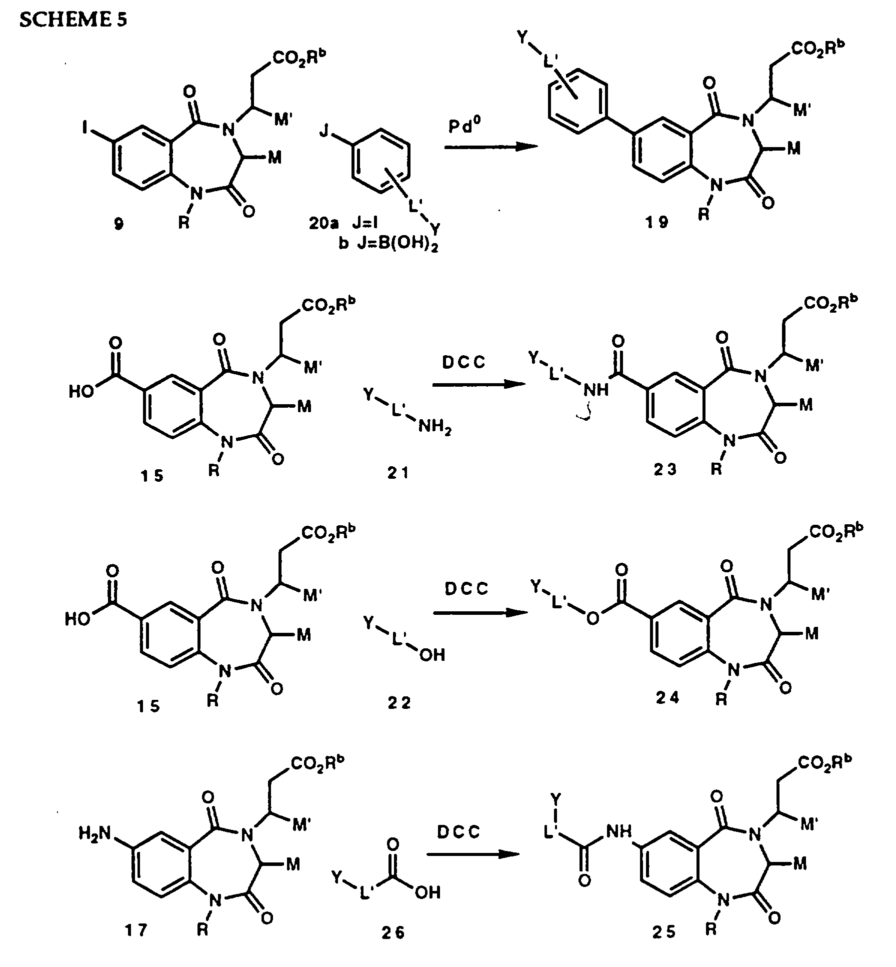

Conversion of aniline 17 into its corresponding urea 27 or carbamate 28 (Scheme 6) may be accomplished by reaction of the isocyanate16 in the presence of an amine 21 or alcohol 22, respectively. Treatment of the phenol 18 with phosgene and an amine 21 or alcohol 22 yields the carbamate 29 and carbonate 30, respectively. Reaction of a sulfonylhalide 32 with either the aniline 17 or phenol 18 would produce the corresponding sulfonamide 33 and sufinyl ester 34, respectively. Allowing the phenol 18 to react with an alcohol 22 in the presence of diethylazodicarboxylate and triphenylphophine will furnish 31, a precursor to Group 12 type compounds. The product 27 is a synthetic precursor to those compounds classified as Group 7, product 28 is a forerunner to Group 6 type compounds, product 29 will give rise to Group 8 type compounds, whereas, product 30 is a precursor to Group 9 type compounds. Products 33 and 34 are precursors to Group 15 and 16, respectively. 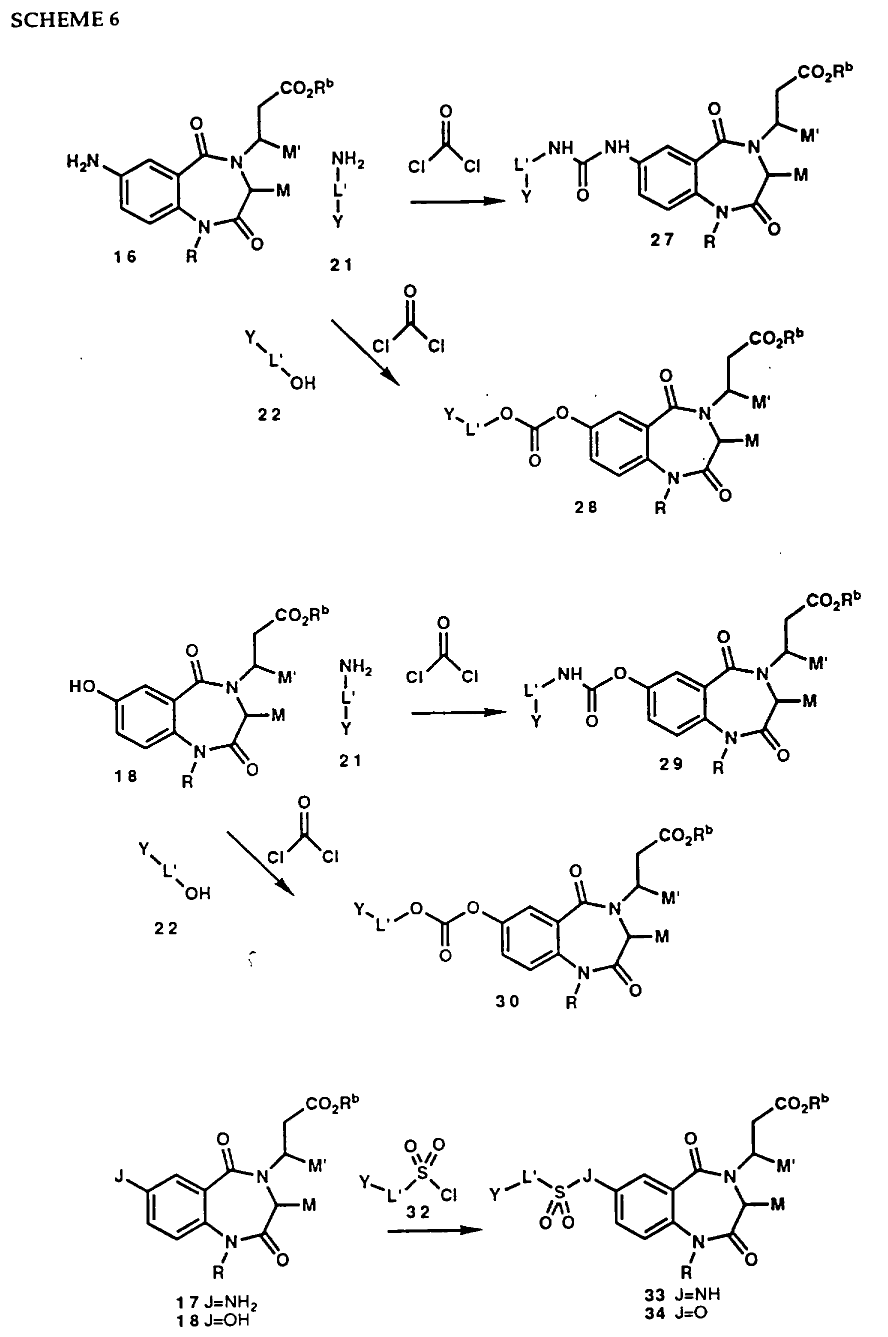 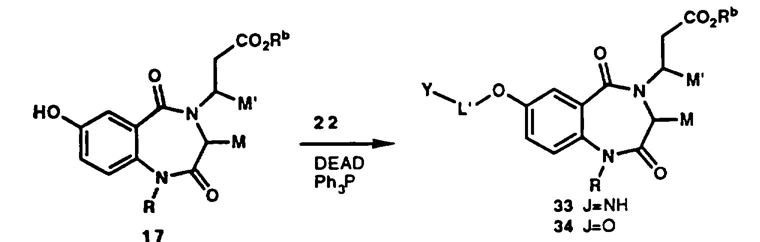 Scheme 7 depict methods that may be used in the preparation of the compounds classified as Groups 13,14, 17 and 18. Diazotization of the aniline 17, by the reaction of 17 with nitrous acid, followed by treatment with sulfur dioxide in the presence of cupric chloride (Gilbert, *Synthesis,* 1969, 1-10) yields the sulfonyl chloride 35. Reaction of 35 with either the amine 21 or the alcohol 22 will provide the synthetic precursor to Groups 13 and 14, respectively, namely, the suphonamide 36 and sulfinyl ester 37. The diazonium salt prepared by diazotization of 17 when allowed to react with oximes in the presence of copper sulphate and sodium sufite will give the corresponding oxime 38, which can be readily hydrolyzed to yield the aryl ketone 39 (Beech, *J. Chew. Soc,* 1954, 1297). *Clemmensen reduction* of the ketone 39 using zinc amalgam and aqueous hydrogen chloride gives rise to the benzyl adduct 40 (Vedejs, *Org. React.* 1975, 22, 401). The products 39 and 40 are precursors to the compounds classified as Groups 17 and 18, respectively.

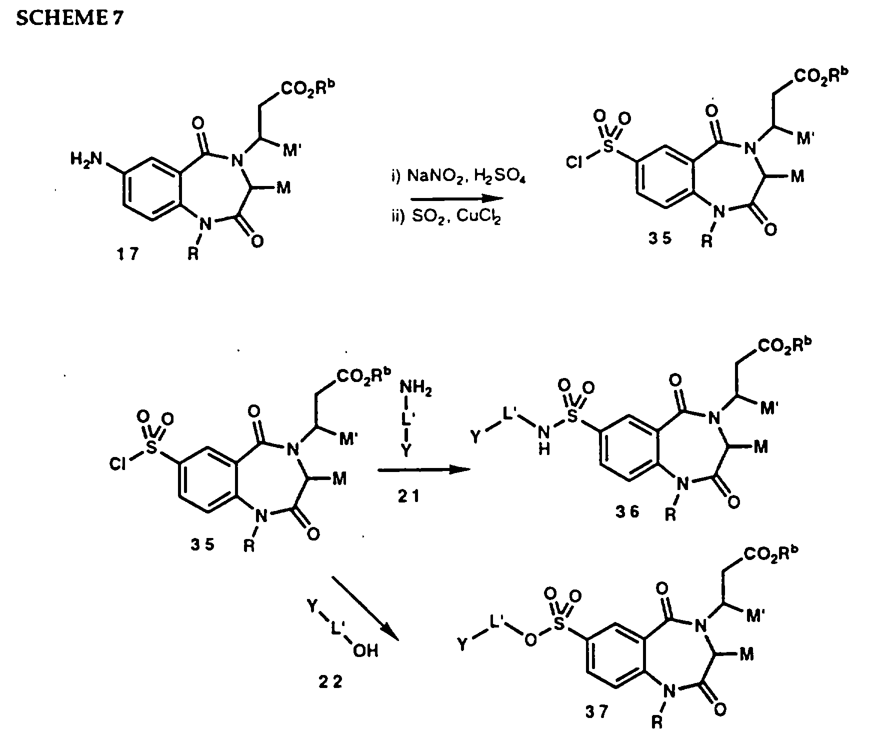

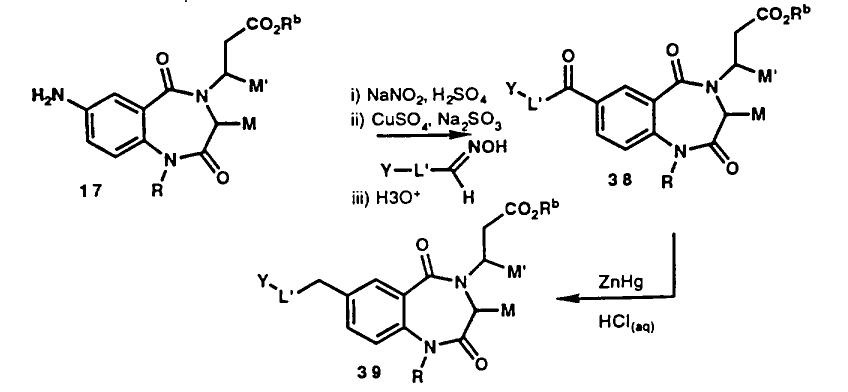

Each of the protected precursors 19, 23, 24, 25, 27, 28, 29, 30, 31, 33, 34, 36. 37, 39, and 40 may be treated in a similar way as described above for compounds 10 and 12 (Scheme 3) to prepare the compounds encompassed by this invention.

### 3. Other "6-7" Fused Ring Systems

Other "6-7" fused ring systems where each T, U, or G of formula I is independently selected from NH, N, NR, C=X, CR₂, CHR, CH₂, CR, CH, O, S, SO, or SO₂ can be prepared by methods known in the art. To provide guidance, however, specific representative synthetic routes are provided in scheme 8. It will be understood that other synthetic routes may be preferred when specific substituents or T-U-G are targeted.

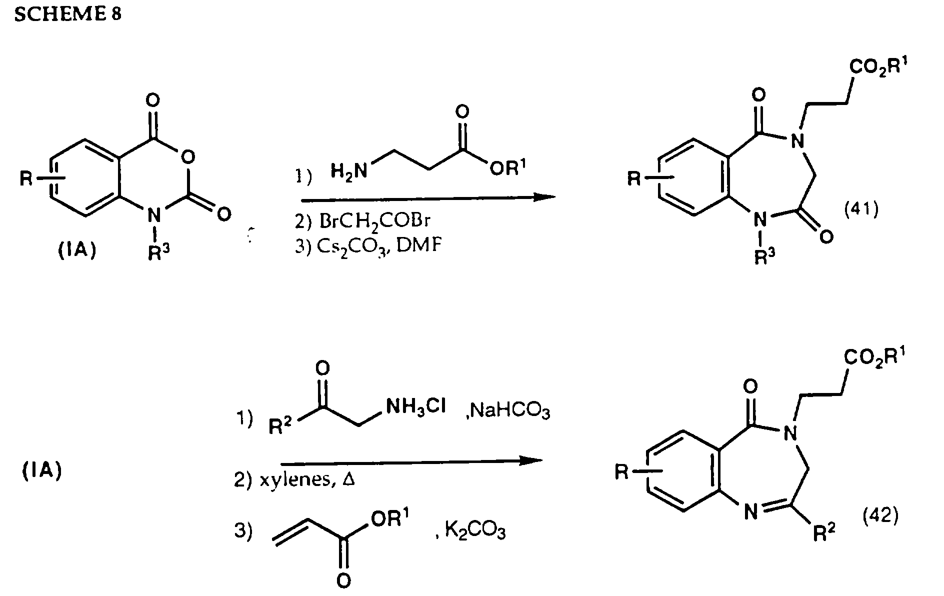

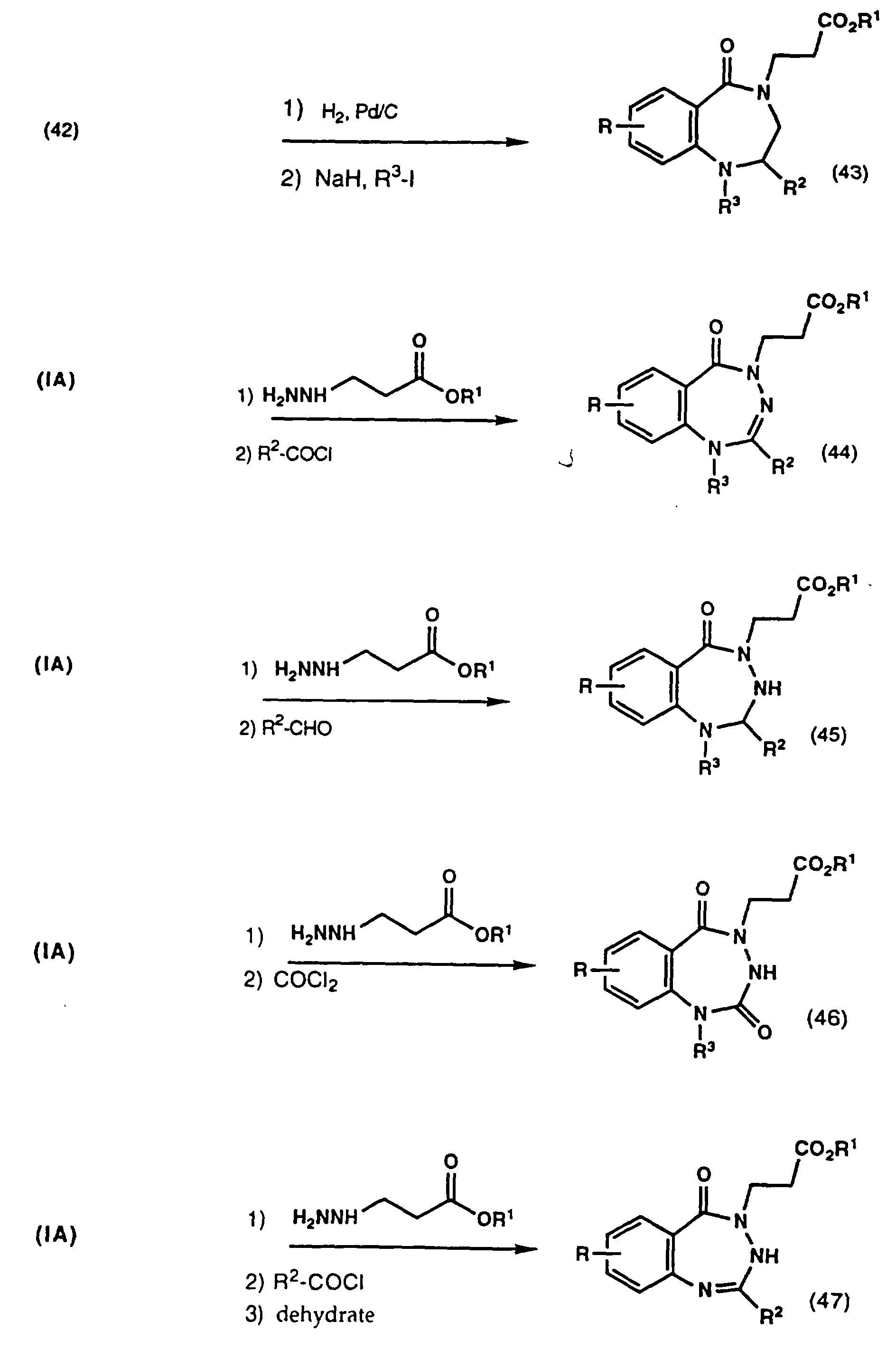

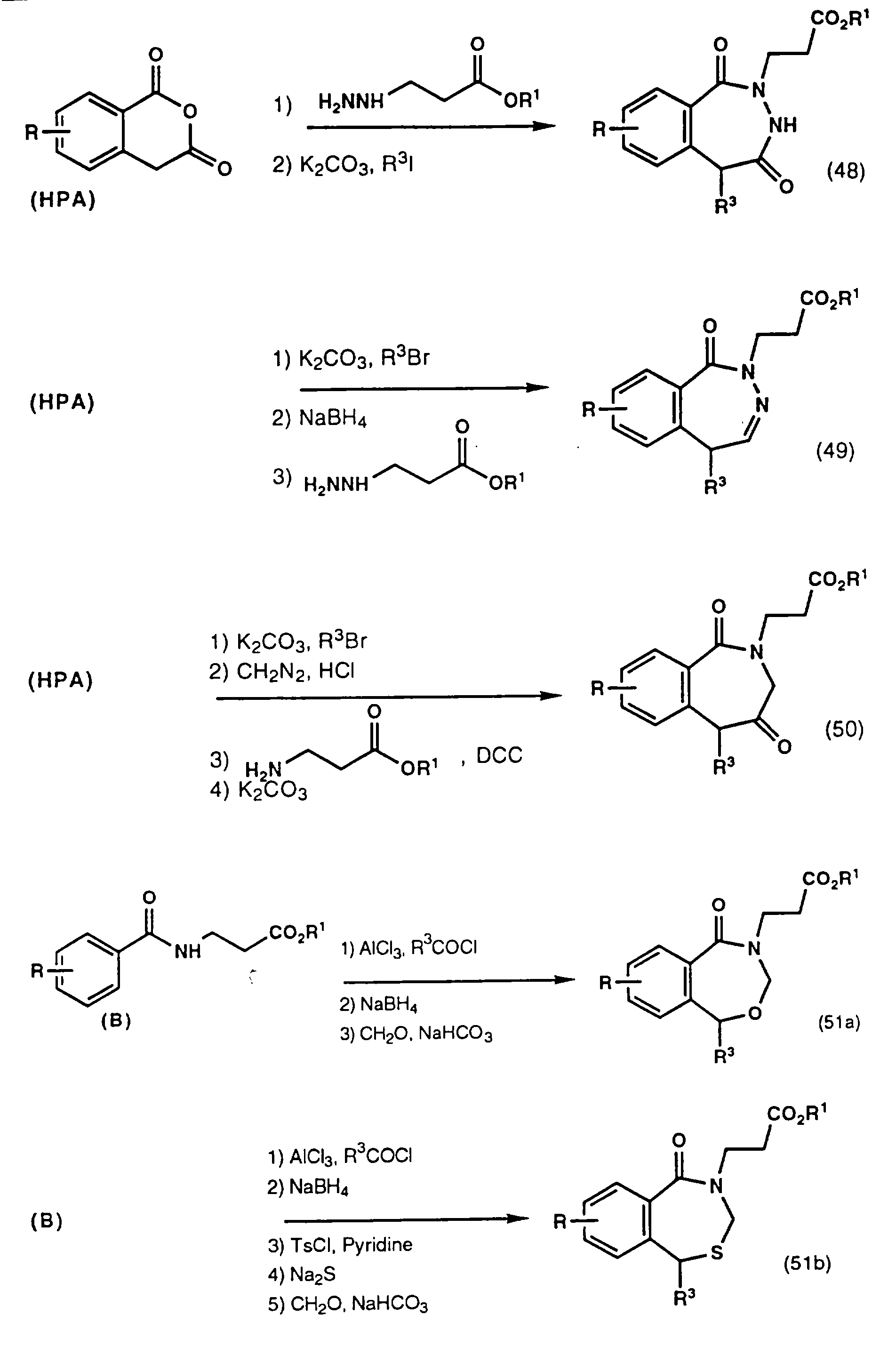

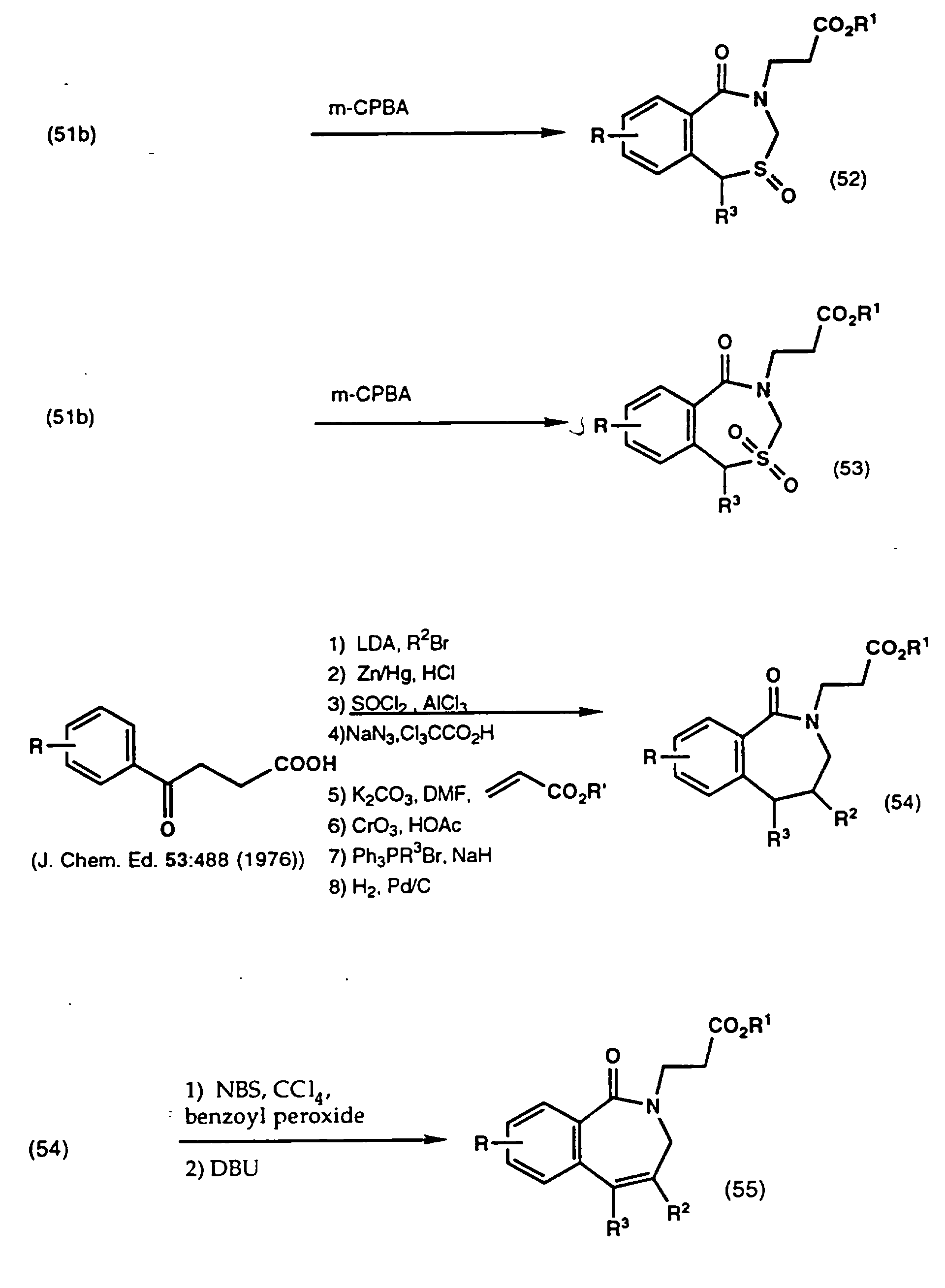

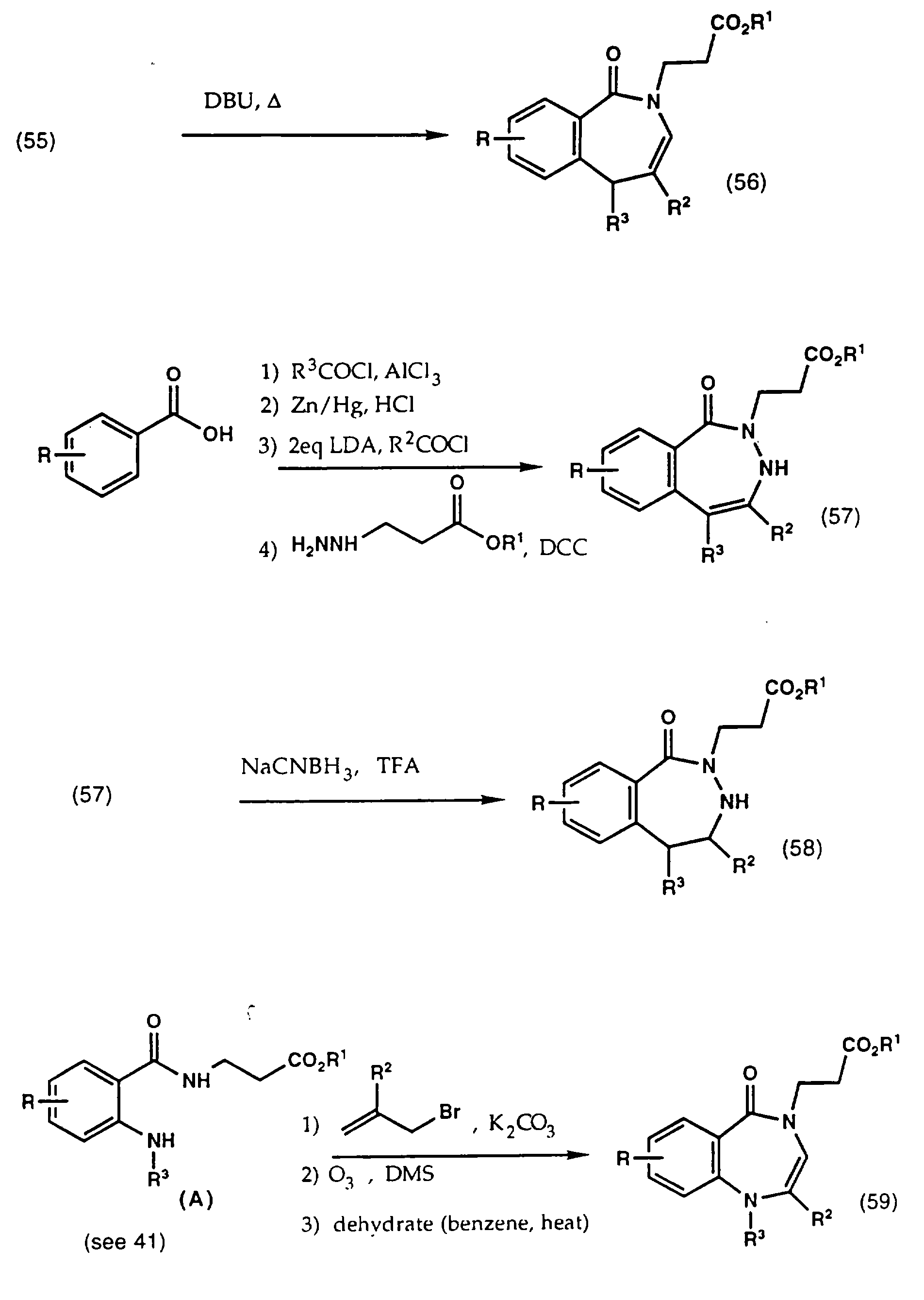

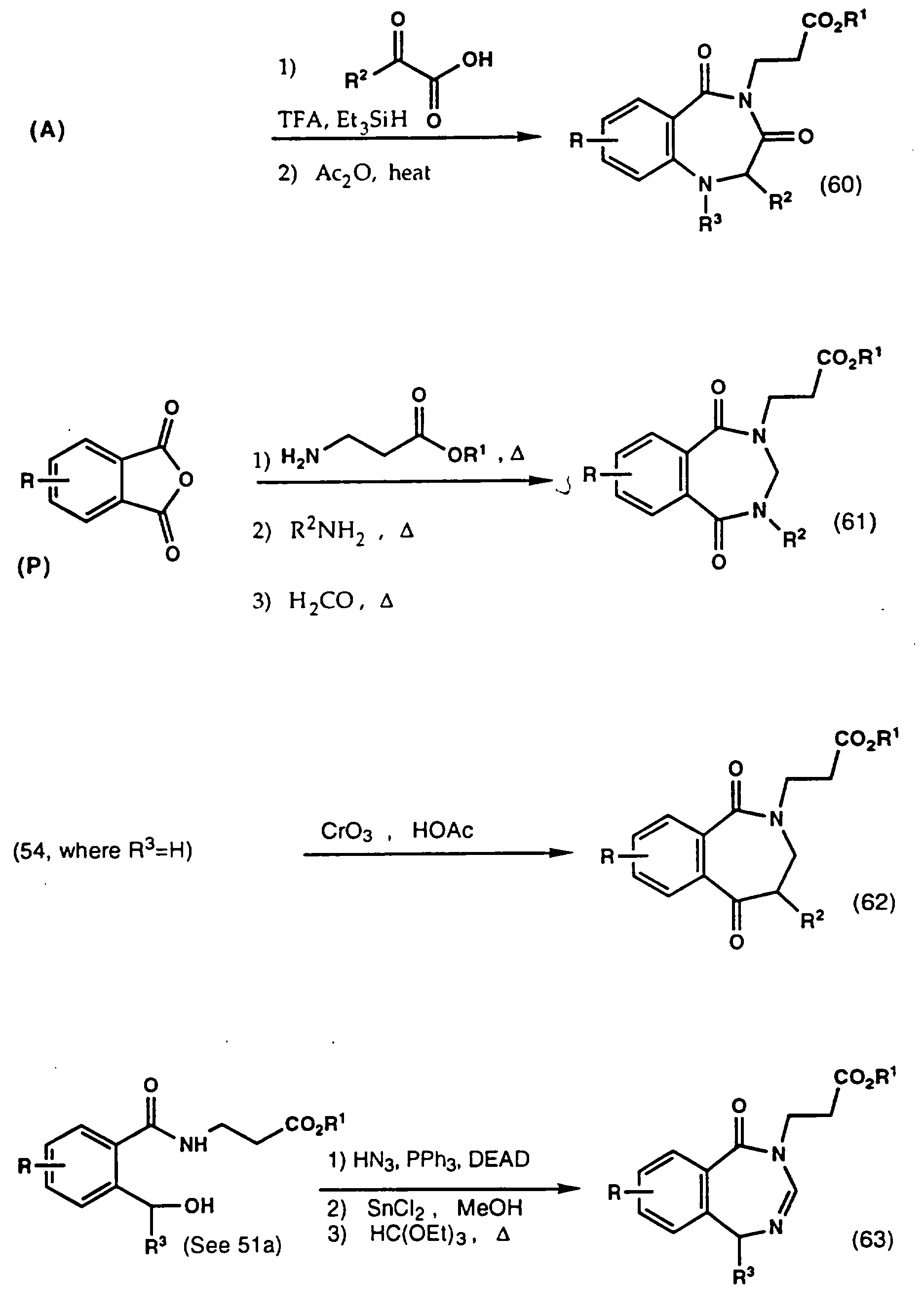

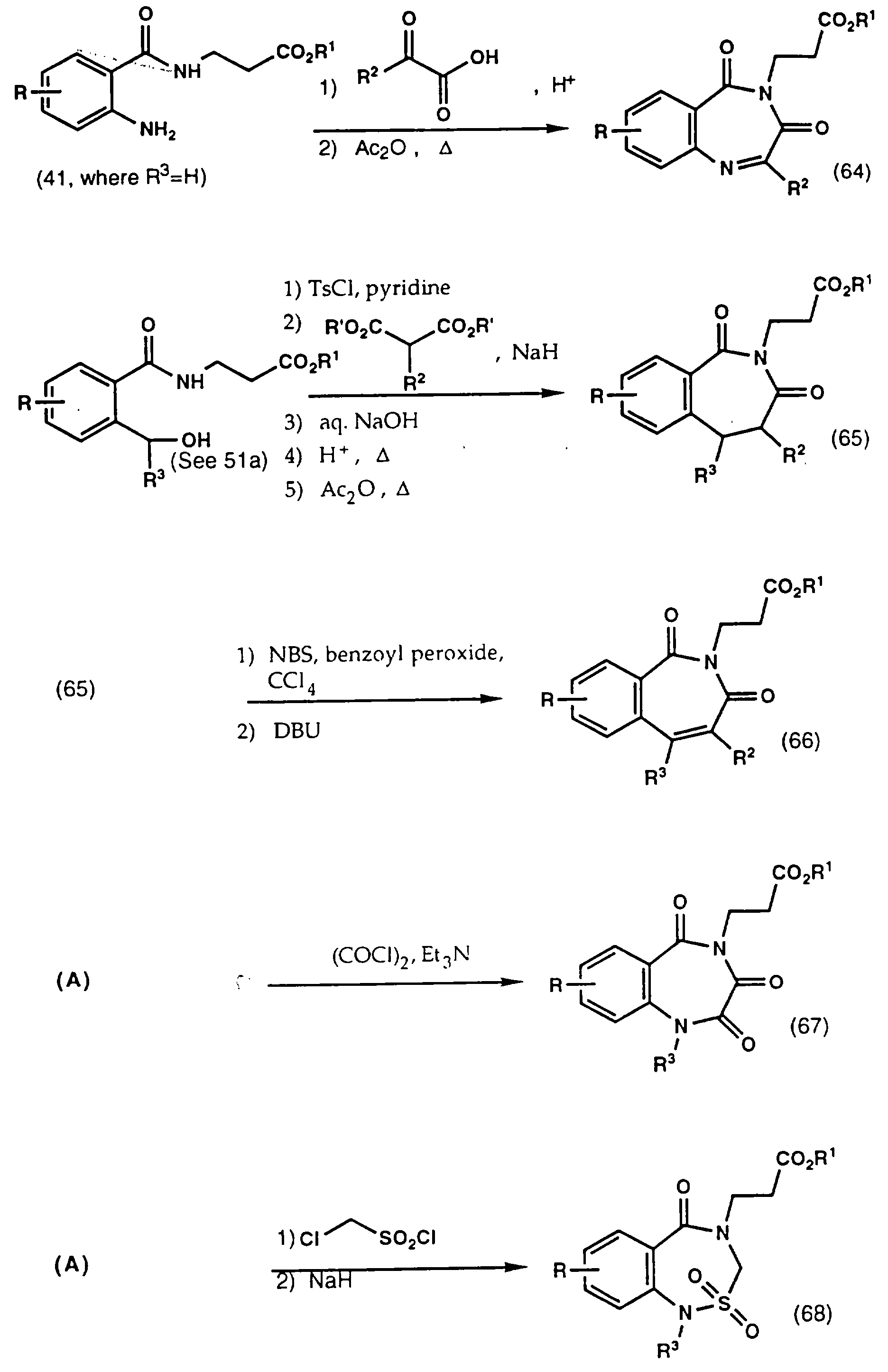

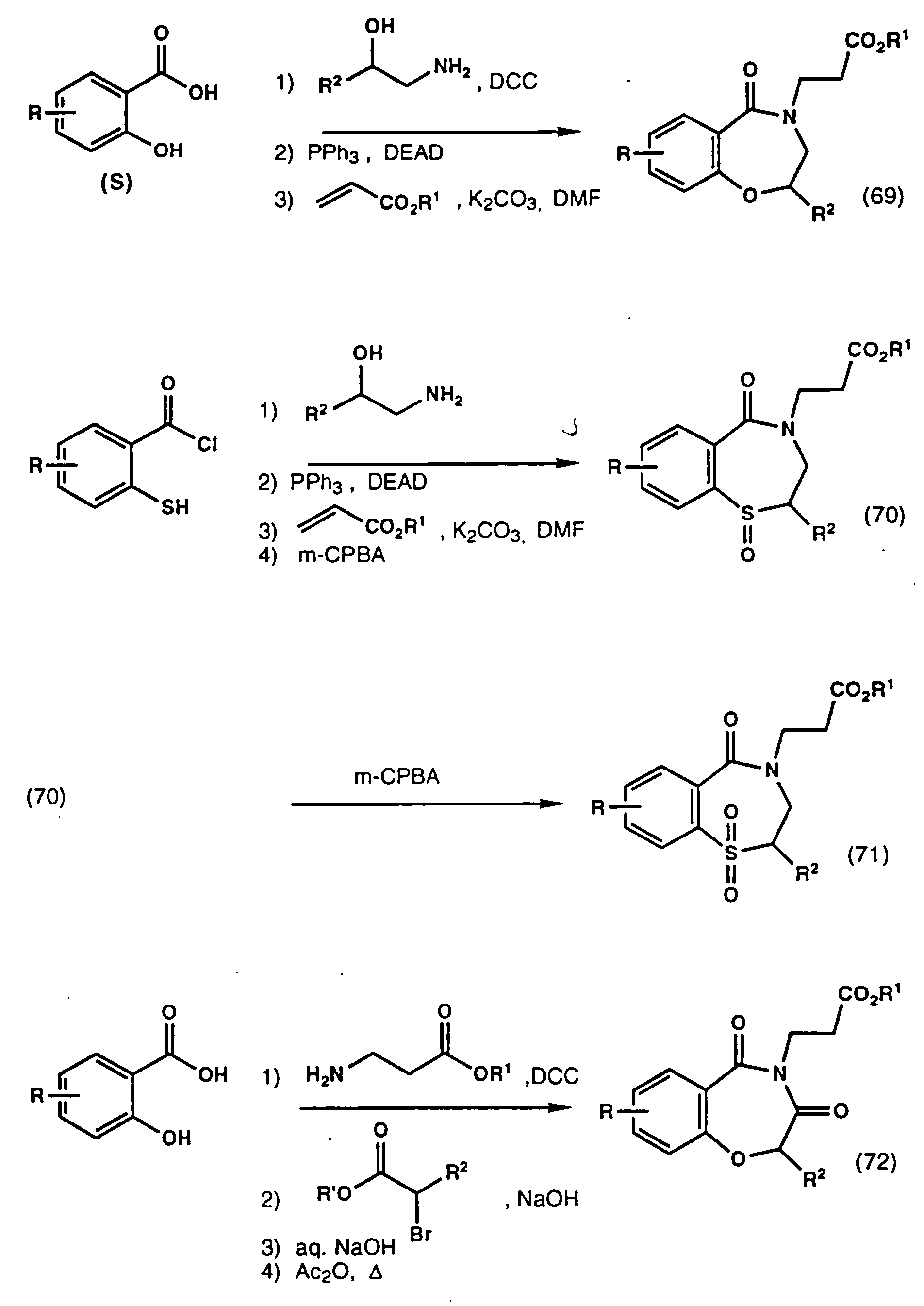

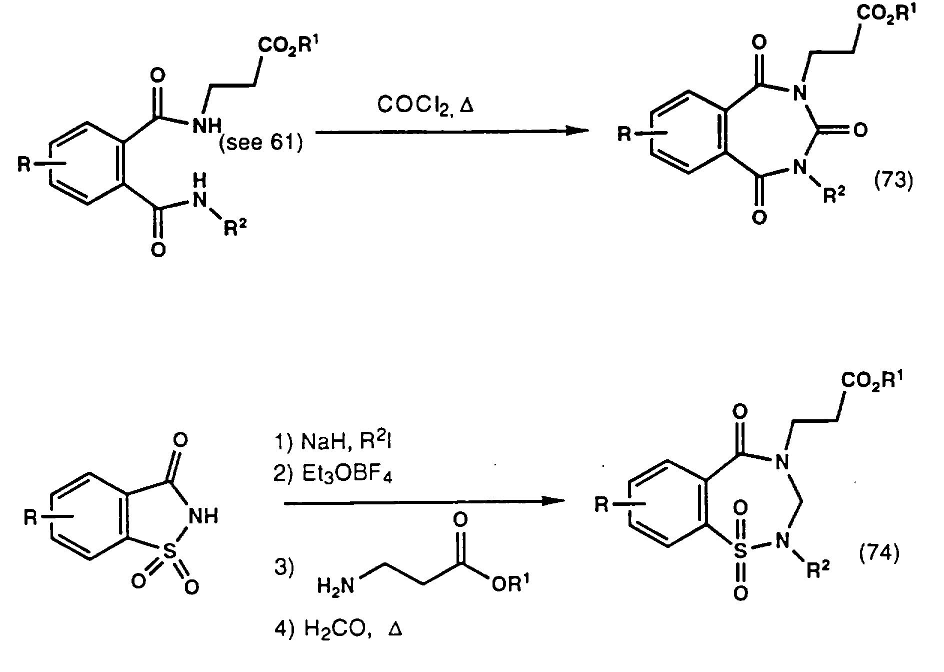

Starting reagents employed in scheme 8 are either commercially available or readily synthesized by known procedures.

### 4. Isomeric Products

In products of Formula I carbon atoms bonded to four nonidentical substituents are asymmetric. Accordingly, the compounds may exist as diastereoisomers, enantiomers or mixtures thereof. The syntheses described above may employ racemates, enantiomers or diastereomers as starting materials or intermediates. Diastereomeric products resulting from such syntheses may be separated by chromatographic or crystallization methods. Likewise, enantiomeric product mixtures may be separated using the same techniques or by other methods known in the art. Each of the asymmetric carbon atoms, when present in compounds of Formula I, may be in one of two configurations (R or S) and both are within the scope of the present invention.

### E. Pharmaceutical Compositions

The compounds described in this invention may be isolated as the free acid or base or converted to salts of various inorganic and organic acids and bases. Such salts are within the scope of this invention. Examples of such salts include ammonium, metal salts like sodium, potassium, calcium and magnesium; salts with organic bases like dicyclohexylamine, N- methyl-D-glucamine and the like; and salts with amino acids like arginine or lysine. Salts with inorganic and organic acids may be likewise prepared, for example, using hydrochloric, hydrobromic, sulfuric, phosphoric, trifluoroacetic, methanesulfonic, malic, maleic, fumaric and the like. Non-toxic and physiologically compatible salts are particularly useful although other less desirable salts may have use in the processes of isolation and purification.

A number of methods are useful for the preparation of the salts described above and are known to those skilled in the art. For example, reaction of the free acid or free base form of a compound of Formula I with one or more molar equivalents of the desired acid or base in a solvent or solvent mixture in which the salt is insoluble; or in a solvent like water after which the solvent is removed by evaporation, distillation or freeze drying. Altematively, the free acid or base form of the product may be passed over an ion exchange resin to form the desired salt or one salt form of the product may be converted to another using the same general process.

In the management of thromboembolic disorders the compounds of this invention may be utilized in compositions such as tablets, capsules or elixirs for oral administration; suppositories for rectal administration; sterile solutions or suspensions for injectable administration, and the like. Animals in need of treatment using compounds of this invention can be administered dosages that will provide optimal efficacy. The dose and method of administration will vary from animal to animal and be dependent upon such factors as weight, diet, concurrent medication and other factors which those skilled in the medical arts will recognize.

Dosage formulations of the nonpeptidyl inhibitors of the present invention are prepared for storage or administration by mixing the inhibitor having the desired degree of purity with physiologically acceptable carriers, excipients, or stabilizers. Such materials are non-toxic to the recipients at the dosages and concentrations employed, and include buffers such as phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as Tween, Pluronics or polyethyleneglycol.

Dosage formulations of the nonpeptidyl inhibitors of the present invention to be used for therapeutic administration must be sterile. Sterility is readily accomplished by filtration through sterile filtration membranes such as 0.2 micron membranes. Nonpeptidyl inhibitor formulations ordinarily will be stored in lyophilized form or as an aqueous solution. The pH of the cyclic inhibitor preparations typically will be between 3 and 11, more preferably from 5 to 9 and most preferably from 7 to 8. It will be understood that use of certain of the foregoing excipients, carriers, or stabilizers will result in the formation of cyclic polypeptide salts. While the preferred route of administration is by hypodermic injection needle, other methods of administration are also anticipated such as suppositories, aerosols, oral dosage formulations and topical formulations such as ointments, drops and dermal patches.

Therapeutic nonpeptidyl inhibitor formulations generally are paced into a container having a sterile access port, for example, an intravenous solution bag or vial having a stopper pierceable by hypodermic injection needle.

Therapeutically effective dosages may be determined by either in *vitro* or in *vivo* methods. For each particular nonpeptidyl inhibitor of the present invention, individual determinations may be made to determine the optimal dosage required. The range of therapeutically effective dosages will naturally be influenced by the route of administration. For injection by hypodermic needle it may be assumed the dosage is delivered into the body's fluids. For other routes of administration, the absorption efficiency must be individually determined for each inhibitor by methods well known in pharmacology.

The range of therapeutic dosages is from about 0.001 nM to 1.0 mM, more preferably from 0.1 nM to 100 mM, and most preferably from 1.0 nM to 50 mM.

Typical formulation of compounds of Formula I as pharmaceutical compositions are discussed below.

About 0.5 to 500 mg of a compound or mixture of compounds of Formula I, as the free acid or base form or as a pharmaceutically acceptable salt, is compounded with a physiologically acceptable vehicle, carrier, excipient, binder, preservative, stabilizer, flavor, etc., as called for by accepted pharmaceutical practice. The amount of active ingredient in these compositions is such that a suitable dosage in the range indicated is obtained.

Typical adjuvants which may be incorporated into tablets, capsules and the like are a binder such as acacia, corn starch or gelatin; an excipient such as microcrystalline cellulose; a disintegrating agent like corn starch or alginic acid; a lubricant such as magnesium stearate; a sweetening agent such as sucrose or lactose; a flavoring agent such as peppermint, wintergreen or cherry. When the dosage form is a capsule, in addition to the above materials it may also contain a liquid carrier such as a fatty oil. Other materials of various types may be used as coatings or as modifiers of the physical form of the dosage unit. A syrup or elixir may contain the active compound, a sweetener such as sucrose, preservatives like propyl paraben, a coloring agent and a flavoring agent such as cherry. Sterile compositions for injection can be formulated according to conventional pharmaceutical practice. For example, dissolution or suspension of the active compound in a vehicle such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice.

### F. Platelet Inhibition Assays

Evaluation of the Formula I inhibitors of the fibrinogen-platelet interaction is guided by *in vitro* receptor binding assays and *in vitro* platelet aggregation inhibition assays.

In-vitro biological activity of the compounds of Formula I are monitored using a modified fibrinogen-GPIIbIIIa ELISA based on the method of Nachman and Leung *J*. *Clin. Invest.* 69:263-269 (1982)) which measures the inhibition of fibrinogen binding to purified human platelet GPIIbIIIa receptor. Human fibrinogen is prepared by the method of Lipinska, *et al. (J. Lab. Clin. Med.* 84:509-516 (1974)). Platelet GPII_{b}IIIₐ is prepared by the method of Fitzgerald, *et al., Anal. Biochem.,*151:169-177 (1985).

Briefly, microtiter plates are coated with fibrinogen (10 mg/ml) and then blocked with TACTS buffer containing 0.5% bovine serum albumin (BSA). (TACTS buffer contains 20mM Tris.HCI, pH 7.5, 0.02% sodium azide, 2 mM calcium chloride, 0.05% Tween 20, 150 mM sodium chloride.) The plate is washed with phosphate buffered saline (PBS) containing 0.01% Tween 20 and the sample to be determined added, followed by addition of solubilized GP IIbIIIa receptor (40 mg/ml) in TACTS, 0.5% BSA. After incubation, the plate is wushed and 1 mg/ml of murine anti-platelet monoclonal antibody AP3 ( Newman *et al*., *Blood* 65:227-232 (1985)) is added. After another wash a goat anti-mouse IgG conjugated to horseradish peroxidase is added. A final wash is performed and developing reagent buffer (10 mg ophenylenediamine dihydrochloride, 0.0212% hydrogen peroxide, 0.22 mM citrate, 50 mM phosphate, pH 5.0) is added and then incubated until color develops. The reaction is stopped with 1N sulfuric acid and the absorbance at 492 nm is recorded.

In addition to the GPII_{b}IIIₐ ELISA assay, platelet aggregation assays may be performed, in human platelet rich plasma (PRP). Fifty milliliters of whole human blood (9 parts) is drawn on 3.6% sodium citrate (1 part) from a donor who has not taken aspirin or related medications for at least two weeks. The blood is centrifuged at 160 x g for 10 min at 22°C and then allowed to stand for 5 min after which the PRP is decanted. Platelet poor plasma (PPP) is isolated from the remaining blood after centrifugation at 2000 x g for 25 min. The platelet count of the PRP is adjusted to ca. 300,00 per microliter with PPP.

A 225 mL aliquot of PRP plus 25 mL of either a dilution of the test sample or a control (PBS) is incubated for 5 mins in a Chrono-log Whole Blood Aggregometer at 25°C. An aggregating agent (collagen, 1 mg/ml; U46619, 100 ng/ml; or ADP, 8 mM) is added and the platelet aggregation recorded.

### EXAMPLES

### Example 1

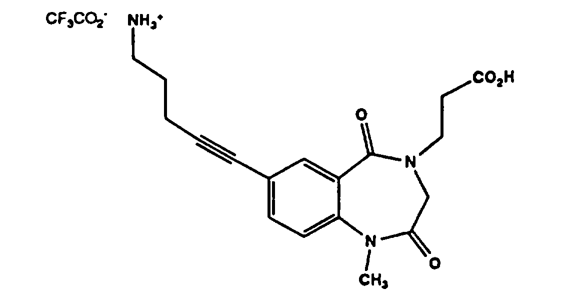

*1-methyl-4-(2-carboxyethyl)-7-(5-amino-1-pentynyl)-3,4-dihydro-1H-1,4-benzodiazepine- 2,5-dione trifluoracetate.*

a ) To a mechanically stirred solution of 26.3 grams of 5-iodo-2-amino benzoic acid (0.1 mol), 10.6 grams of sodium carbonate (0.1 mol), and 250 mL water, cooled to 0°C, was slowly added, *via* an addition funnel, 80mL of a 1.93M solution of phosgene in toluene. After 2 hours, the precipitated product was isolated by filtration. The solids were washed with 200mL water, 300mL of a 1:1 mixture ethanol and ether, 200mL of ether, and dried under vacuum to yield 24.3 grams (84%) of 5-iodoisatoic anhydride (264-268°C, decomposition).

b) A magnetically stirred solution of 5 grams of 5-iodoisatoic anhydride (0.0173 mol), 5.85 grams of b-alanine benzyl ester tosylate (0.0173 mol), 35mL pyridine, and 0.5 of dimethylaminopyridine (0.0041 mol) was heated to 80°C for 2 hrs. The reaction mixture was allowed to cool to room temperature and concentrated *in vacuo.* The resulting residue was dissolved in 100mL ethyl acetate and washed 2X50mL of 10% cupric sulfate, 1X50mL sat. sodium bicarbonate, 1X 50mL brine, dried over sodium sulfate, filtered and concentrated in *vacuo*. The product was further purified by column chromatography, using silica gel, eluting with a 1:1 mixture of ethyl acetate and hexane (TLC, SiO₂, 1:1 EtOAc/hexane, R_{f}=0.65, un positive) to yield 1.85 grams (25%) of N-(2-amino-5-iodobenzoyl)-b-alanine benzyl ester. ¹H NMR (CDCl₃,dTMS) 7.54 (1H, d,⁴J_{HH} = 2Hz, Ar-H *o*-CON), 7.42 (1H, dd, ³J_{HH}=9Hz, ⁴J_{HH}=2Hz, Ar-H *p*-CON), 7.38-7.32 (5H, s, ArH Ph), 6.65 (1H, t, ³J_{HH}=6Hz, CONH), 6.46 (1H, d, 3J_{HH}=9Hz, Ar-H *m*-CON), 5.16 (2H, s, OCH₂), 3.68 (2H, q, ³J_{HH}=6Hz, NCH₂), 2.69 (2H, t, ³J_{HH}=6Hz, CH₂CO₂) ¹³C NMR (CDCI₃,dTMS) 172.4, 167.8, 148.2, 140.6, 135.6, 135.5, 128.7, 128.4, 128.3, 119.3, 118.1, 76.3, 66.7, 35.2, 34.0.

Using the above procedure, but substituting the appropriate 3-aminoproprionate alkyl ester for b-alanine ethyl ester and N-substituted-5-iodo-isatoic anhydride for 5- iodoisatoic anhydride there may be prepared, for example, the following compounds:
ethyl N-(2-amino-5-iodobenzoyl)-3-amino-3-methylpropanoate,
ethyl N-(2-amino-5-iodobenzoyl)-3-amino-3-phenylpropanoate,
ethyl N-(2-amino-5-iodobenzoyl)-3-amino-3-(3-(methoxycarbonyl)phenyl)propanoate,
*t*-butyl N-[N-(2-amino-5-iodobenzoyl)-L-(b-benzyl)-aspartinate,
ethyl N-[N-(2-amino-5-iodobenzoyl)-L-aspartyl-(b-benzyl ester)]-glycinate,
ethyl N-[N-(2-amino-5-iodobenzoyl)-L-aspartyl-(b-benzyl ester)]-valinate,
ethyl N-[N-(2-amino-5-iodobenzoyl)-L-aspartyl-(b-benzyl ester)]-phenylalaninate,
ethyl N-(2-(N-methyl)-amino-5-iodobenzoyl)-3-amino-3-methylpropanoate,
ethyl N-(2-(N-methyl)-amino-5-iodobenzoyl)-3-amino-3-phenylpropanoate,
ethyl N-(2-(N-methyl)-amino-5-iodobenzoyl)-3-amino-3-(3-(methoxycarbonyl)phenyl)propanoate,
*t*-butyl N-[N-[2-(N-methyl)-amino-5-iodobenzoyl]-(±)-(b-benzyl)-aspartinate,
ethyl N-[N-(2-(N-methyl)-amino-5-indobenzoyl)-(±)-aspartyl-(b-benzyl ester)]-glycinate,
ethyl N-[N-(2-(N-methyl)-amino-5-indobenzoyl)-(±)-aspartyl-(b-benzyl ester)]-valinate,
ethyl N-[N-(2-(N-methyl)-amino-5-iodobenzoyl)-(±)-aspartyl-(b-benzyl ester)]-phenylalaninate,
ethyl N-(2-(N-phenyl)-amino-5-iodobenzoyl)-3-amino-3-methylpropanoate,
ethyl N-(2-(N-phenyl)-amino-5-iodobenzoyl)-3-amino-3-phenylpropanoate,
ethyl N-(2-(N-phenyl)-amino-5-iodobenzoyl)-3-amino-3-[3-(methoxycarbonyl)phenyl]propanoate,
*t*-butyl N-[N-[2-(N-phenyl)-amino-5-iodobenzoyl]-(±)-(b-benzyl)-aspartinate,
ethyl N-[N-(2-(N-phenyl)-amino-5-iodobenzoyl)-(±)-aspartyl-(b-benzyl ester)]-glycinate,
ethyl N-[N-(2-(N-phenyl)-amino-5-iodobenzoyl)-(±)-aspartyl-(b-benzyl ester)]-valinate,
ethyl N-[N-(2-(N-phenyl)-amino-5-iodobenzoyl)-(±)-aspartyl-(b-benzyl ester)]- phenylalaninate,
ethyl N-(2-(N-benzyl)-amino-5-iodobenzoyl)-3-amino-3-methylpropanoate,
ethyl N-(2-(N-benzyl)-amino-5-iodobenzoyl)-3-amino-3-phenylpropanoate,
ethyl N-(2-(N-benzyl)-amino-5-iodobenzoyl)-3-amino-3-(3- (methoxycarbonyl)phenyl)propanoate,
*t*-butyl N-[N-[2-(N-benzyl)-amino-5-iodobenzoyl]-(±)-(b-benzyl)-aspartinate,
ethyl N-[N-(2-(N-benzyl)-amino-5-iodobenzoyl)-(±)-aspartyl-(b-benzyl ester)]-glycinate,
ethyl N-[N-(2-(N-benzyl)-amino-5-iodobenzoyl)-(±)-aspartyl-(b-benzyl ester)]-valinate,
ethyl N-[N-(2-(N-benzyl)-amino-5-indobenzoyl)-(±)-aspartyl-(b-benzyl ester)]- phenylalaninate,
ethyl N-(2-(N-isopropyl)-amino-5-iodobenzoyl)-3-amino-3-methylpropanoate,
ethyl N-(2-(N-isopropyl)-amino-5-iodobenzoyl)-3-amino-3-phenylpropanoate,
ethyl N-(2-(N-isopropyl)-amino-5-iodobenzoyl)-3-amino-3-(3-(methoxycarbonyl)phenyl) propanoate,
*t*-butyl N-[N-[2-(N-isopropyl)-amino-5-iodobenzoyl]-(±)-(b-benzyl)-aspartinate,
ethyl N-[N-(2-(N-isopropyl)-amino-5-idobenzoyl)-(±)-aspartyl-(b-benzyl ester)]- glycinate,
ethyl N-[N-(2-(N-isopropyl)-amino-5-iodobenzoyl)-(±)-aspartyl-(b-benzyl ester)]- valinate,
ethyl N-[N-(2-(N-isopropyl)-amino-5-iodobenzoyl)-(±)-aspartyl-(b-benzyl ester)]- phenylalaninate,

c) A magnetically stirred solution of 0.848gram of N-(2-amino-5-iodobenzoyl)-b- alanine ethyl ester (2.0 mmol), 0.35mL 2,6-lutidine (3.0 mmol), 0.19mL methyl iodide (3.0 mmol), and 15mL dimethylformamide was heated to 50°C for 15 hours. The reaction mixture was allowed to cool to room temperature and concentrated *in vacuo*. The resulting residue was dissolved in 75mL ethyl acetate and washed 1X50mL 10% citric acid, 1X50mL sat. sodium bicarbonate, 1X50mL brine, dried over magnesium sulfate, filtered and concentrated *in vacuo.* The resulting oil was further purified by column chromatography, using silica gel, eluting with a solvent gradient of 35/65 ethyl acetate/hexane to 65/35 ethyl acetate/hexane (TLC, SiO₂, 1:1 EtOAc/hexane, R_{f}=0.84, un positive) to yield 305 mgs (35%) of N-(2-methylamino- 5-iodobenzoyl)-b-alanine benzyl ester . ¹H NMR (CDCI₃ , dTMS) 7.56 (2H, m, Ar-H *o,p-* CON), 7.43 (1H, bq, ³J_{HH}=5Hz, NHMe), 7.38-7.32 (5H, s, ArH Ph), 6.62 (1H, bt, ³JHH=6Hz, CONH), 6.42 (1H, d, ³J_{HH}=9Hz, Ar-H *m*-CON), 5.16 (2H, s, OCH₂), 3.64 (2H, q, ³J_{HH}=6Hz, NCH₂), 2.81 (3H, d, ³J_{HH}=5Hz, NCH₃), 2.64 (2H, t, ³J_{HH}=6Hz, CH₂CO₂). ¹³C NMR (CDCl₃, dTMS) 172.4, 168.4, 148.8, 141.0, 135.5, 128.7, 128.4, 128.3, 117.3, 113.4, 74.1, 66.7, 35.2, 34.0, 29.6.

d) To a magnetically stirred solution of 0.305 grms of N-(2-methylamino-5- iodobenzoyl)-b-alanine benzyl ester (0.69 mmol), 3 mL methylene chloride, and 0.144mL triethylamine (1.04 mmol), cooled to -30°C under an atmosphere of nitrogen was slowly added 0.09 mL of a-bromoacetylbromide (1.04 mmol) as a solution in 2mL methylene chloride. The reaction mixture was allowed to warm to room temperature and stir for 2 hours. The mixture was diluted with 40mL methylene chloride and washed with 1X50 10% citric acid, 1X50 sat. sodium bicarbonate, dried over sodium sulfate, filtered and concentrated *in vacuo.* The resulting residue was dissolved in 3mL dimethylformamide and added, *via* an addition funnel, to a slurry of 25 mgs sodium hydride (1.04 mmol) in 2mL dimethylformamide that was cooled to 0°C. After 2 hours, the mixture was poured over 50mL of an ice cooled solution of 10% citric acid and extracted with 3X40mL ethyl acetate. The combined organic layers were washed with 1X50mL sat. sodium bicarbonate, dried over magnesium sulfate, and concentrated *in vacuo.* The resulting residue was further purified by column chromatography, using silica gel, eluting with a solvent gradient of 40:60 ethyl acetate/hexane to 70:30 ethyl acetate/hexane to yield 0.16 gms (49%) of 1-methyl-4-(2-carboxyethyl)-7-iodo-3,4-dihydro- 1*H*-1,4-benzodiazepine-2,5-dione benzyl ester (TLC, SiO₂, 1:1 ethyl acetate/ hexane, R_{f}=0.48, un positive). ¹H NMR (CDCl₃, dTMS) 8.15 (1H, d, ⁴J_{HH}=2Hz, Ar-H *o*-CON), 7.78 (1H, dd, ⁴J_{HH}=2Hz, ³J_{HH}=9Hz, Ar-H p-CON), 6.90 (1H, d, ³J_{HH}=9Hz, Ar-H *m*-CON), 5.1 (2H, s, OCH₂), 3.92 (1H, d, J_{HH}=14Hz, COCHHN), 3.90 (2H, t, ³J_{HH}=8Hz, NCH₂),3.82 (1H, d, J_{HH}=14Hz, COCHHN) 3.27 (3H, s, NCH₃), 2.82 (1H, dt, J_{HH}=19Hz, ³J_{HH}=9Hz, CHHCO₂), 2.68 (1H, dt, J_{HH}=19Hz, ³J_{HH}=9Hz, CHHCO₂).

Using the above procedure, but substituting the appropriate N-(2-amino-5- iodobenzoyl)-b-alanine alkyl ester for N-(2-methylamino-5-iodobenzoyl)-b-alanine benzyl ester and a-substituted-a-halo acetyl halide for a-bromo acetyl bromide there may be prepared, for example, the following compounds:
1-methyl-4-(3-ethoxy-1-methyl-3-oxo-1-propyl)-7-iodo-3,4-dihydro-1H-1,4-benzodiazepine-2,5-dione,
1-methyl-4-(3-ethoxy-1-phenyl-3-oxo-1-propyl)-7-iodo-3,4-dihydro-1H-1,4-benzodiazepine-2,5-dione,
(±)-1-methyl-4-[2-(1-*t*-butoxy-4-benzyloxy)succinyl)]-7-iodo-3,4-dihydro-1H-1,4-benzodiazepine-2,5-dione,
(±)-1-methyl-4-[2-(1-benzyloxyglycinyl-4-benzyloxy)succinyl)]-7-iodo-3,4-dihydro-1H-1,4-benzodiazepine-2,5-dione,
(±)-1-methyl-4-[2-(1-benzyloxyvalinyl-4-benzyloxy)succinyl)-7-iodo-3,4-dihydro-1H-1,4-benzodiazepine-2,5-dione,
(±)-1-methyl-4-[2-(1-benzyloxyphenylalanyl-4-benzyloxy)succinyl)]-7-iodo-3,4-dihydro-1H-1,4-benzodiazepine-2,5-dione,
1-phenyl-4-(3-ethoxyl-1-methyl-3-oxo-1-propyl)-7-iodo-3,4-dihydro-1H-1,4-benzodiazepine-2,5-dione,
1-phenyl-4-(3-ethoxy-1-phenyl-3-oxo-1-propyl)-7-iodo-3,4-dihydro-1H-1,4-benzodiazepine-2,5-dione,
(±)-1-phenyl-4-[2-(1-*t*-butoxy-4-benzyloxy)succinyl)]-7-iodo-3,4-dihydro-1H-1,4-benzodiazepine-2,5-dione,
(±)-1-phenyl-4-[2-(1-benzyloxyglycinyl-4-benzyloxy)succinyl)]-7-idio-3,4-dihydro-1H-1,4-benzodiazepine-2,5-dione,
(±)-1-phenyl-4-[2-(1-benzyloxyvalinyl-4-benzyloxy)succinyl)]-7-iodo-3,4-dihydro-1H-1,4-benzodiazepine-2,5-dione,
(±)-1-phenyl-4-[2-(1-benzyloxyphenylalanyl-4-benzyloxy)succinyl)]-7-iodo-3,4-dihydro-1H-1,4-benzodiazepine-2,5-dione,
1-benzyl-4-(3-ethoxy-1-methyl-3-oxo-1-propyl)-7-iodo-3,4-dihydro-1H-1,4-benzodiazepine-2,5-dione,
1-benzyl-4-(3-ethoxy-1-phenyl-3-oxo-1-propyl)-7-iodo-3,4-dihydro-1H-1,4-benzodiazepine-2,5-dione,
(±)-1-benzyl-4-[2-(1-*t*-butoxy-4-benzyloxy)succinyl)]-7-iodo-3,4-dihydro-1H-1,4-benzodiazepine-2,5-dione,
(±)-1-benzyl-4-[2-(1-benzyloxyglycinyl-4-benzyloxy)succinyl)]-7-iodo-3,4-dihydro-1H-1,4-benzodiazepine-2,5-dione,
(±)-1-benzyl-4-[2-(1-benzyloxyvalinyl-4-benzyloxy)succinyl)]-7-iodo-3,4-dihydro-1H-1,4-benzodiazepine-2,5-dione,
(±)-1-benzyl-4-[2-(1-benzyloxyphenylalanyl-4-benzyloxy)succinyl)]-7-iodo-3,4-dihydro-1H-1,4-benzodiazepine-2,5-dione,
1-isopropyl-4-(3-ethoxy-1-methyl-3-oxo-1-propyl)-7-iodo-3,4-dihydro-1H-1,4-benzodiazepine-2,5-dione,
1-isopropyl-4-(3-ethoxy-1-phenyl-3-oxo-1-propyl)-7-iodo-3,4-dihydro-1H-1,4-benzodiazepine-2,5-dione,
(±)-1-isopropyl-4-[2-(1-*t*-butoxy-4-benzyloxy)succinyl)]-7-iodo-3,4-dihydro-1H-1,4-benzodiazepine-2,5-dione,
(±)-1-isopropyl-4-[2-(1-benzyloxyglycinyl-4-benzyloxy)succinyl)]-7-iodo-3,4-dihydro-1H-1,4-benzodiazepine-2,5-dione,
(±)-1-isopropyl-4-[2-(1-benzyloxyvalinyl-4-benzyloxy)succinyl)]-7-iodo-3,4-dihydro-1H-1,4-benzodiazepine-2,5-dione,
(±)-1-isopropyl-4-[2-(1-benzyloxyphenylalanyl-4-benzyloxy)succinyl)]-7-iodo-3,4-dihydro-1H-1,4-benzodiazepine-2,5-dione,
1-methyl-3-phenyl-4-(3-ethoxy-1-methyl-3-oxo-1-propyl)-7-iodo-3,4-dihydro-1H-1,4-benzodiazepine-2,5-dione,
1-methyl-3-phenyl-4-(3-ethoxy-1-phenyl-3-oxo-1-propyl)-7-iodo-3,4-dihydro-1H-1,4-benzodiazepine-2,5-dione,
(±)-1-methyl-3-phenyl-4-[2-(1-*t*-butoxy-4-benzyloxy)succinyl)]-7-iodo-3,4-dihydro-1H-1,4-benzodiazepine-2,5-dione,
(±)-1-methyl-3-phenyl-4-[2-(1-benzyloxyglycinyl-4-benzyloxy)succinyl)]-7-iodo-3,4-dihydro-1H-1,4-benzodiazepine-2,5-dione,
(±)-1-methyl-3-phenyl-4-[2-(1-benzyloxyvalinyl-4-benzyloxy)succinyl)]-7-iodo-3,4-dihydro-3-phenyl-1H-1,4-benzodiazepine-2,5-dione,
(±)-1-methyl-3-phenyl-4-[2-(1-benzyloxyphenylalanyl-4-benzyloxy)succinyl)]-7-iodo-3,4-dihydro-1H-1,4-benzodiazepine-2,5-dione,
1,3-diphenyl-4-(3-ethoxy-1-methyl-3-oxo-1-propyl)-7-iodo-3,4-dihydro-1H-1,4-benzodiazepine-2,5-dione,
1,3-diphenyl-4-(3-ethoxy-1-phenyl-3-oxo-1-propyl)-7-iodo-3,4-dihydro-1H-1,4-benzodiazepine-2,5-dione,
(±)-1,3-diphenyl-4-[2-(1-*t*-butoxy-4-benzyloxy)succinyl)]-7-iodo-3,4-dihydro-1H-1,4-benzodiazepine-2,5-dione,
(±)-1,3-diphenyl-4-[2-(1-benzyloxyglycinyl-4-benzyloxy)succinyl)]-7-iodo-3,4-dihydro-1H-1,4-benzodiazepine-2,5-dione,
(±)-1,3-diphenyl-4-[2-(1-benzyloxyvalinyl-4-benzyloxy)succinyl)]-7-iodo-3,4-dihydro-1H-1,4-benzodiazepine-2,5-dione,
(±)-1,3-diphenyl-4-[2-(1-benzyloxyphenylalanyl-4-benzyloxy)succinyl)]-7-iodo-3,4-dihydro-1H-1,4-benzodiazepine-2,5-dione,
1-benzyl-3-phenyl-4-(3-ethoxy-1-methyl-3-oxo-1propyl)-7-iodo-3,4-dihydro-1H-1,4-benzodiazepine-2,5-dione,
1-benzyl-3-phenyl-4-(3-ethoxy-1-phenyl-3-oxo-1-propyl)-7-iodo-3,4-dihydro-1H-1,4-benzodiazepine-2,5-dione,
(±)-1-benzyl-3-phenyl-4-[2-(1-*t*-butoxy-4-benzyloxy)succinyl)]-7-iodo-3,4-dihydro-1H-1,4-benzodiazepine-2,5-dione,
(±)-1-benzyl-3-phenyl-4-[2-(1-benzyloxyglycinyl-4-benzyloxy)succinyl)]-7-iodo-3,4-dihydro-1H-1,4-benzodiazepine-2,5-dione,
(±)-1-benzyl-3-phenyl-4-[2-(1-benzyloxyvalinyl-4-benzyloxy)succinyl)]-7-iodo-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-iodo-3,4-dihydro-1H-1,4-benzodiazepine-2,5-dione,
1-isopropyl-3-phenyl-4-(3-ethoxy-1-methyl-3-oxo-1-propyl)-7-iodo-3,4-dihydro-1H-1,4-benzodiazepine-2,5-dione,
1-isopropyl-3-phenyl-4-(3-ethoxy-1-phenyl-3-oxo-1-propyl)-7-iodo-3,4-dihydro-1H-1,4-benzodiazepine-2,5-dione,
(±)-1-isopropyl-3-phenyl-4-[2-(1-*t*-butoxy-4-benzyloxy)succinyl)]-7-iodo-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-iodo-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-iodo-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-iodo-3,4-dihydro-1H-1,4-benzodiazepine-2,5-dione,

e) To a magnetically stirred solution of 9.13 grams hex-5-ynoic acid (0.081 mol), 7.12mL triethylamine (0.051 mol), 30mL *tert*-butanol, was added 17.7mL diphenylphosphoryl azide (0.082 mol) and the mixture heated to reflux for 3 hours. The reaction mixture was allowed to cool to room temperature and poured over a biphasic mixture of 200mL water and 200mL ether. The layers were separated and the organic layer was washed 1X50mL 5% ethylenediaminetetraacetic acid disodium salt, 1X50mL 10% sodium bicarbonate, 1X50mL brine, dried over magnesium sulfate and activated carbon, filtered and concentrated *in vacuo*. The resulting residue was further purified by column chromatography, using silica gel, eluting with 1:1 ether/hexane (TLC, SiO₂, 1:3 ethyl acetate/hexane, R_{f}=0.18, ninhydrin char) to yield 3.8 grams (25%) N-boc-5-amino-1-pentyne. ¹H NMR (CDCl₃, dTMS) 4.65 (1H, bs, NH), 3.22 (2H, q, ³J_{HH}=6Hz, NCH₂), 2.23 (2H, dt, ³J_{HH}=6Hz, ⁴J_{HH}=3Hz, CH₂C∫C), 1.98 (1H, t, ⁴JHH=³Hz, C∫CH), 1.73 (2H, p, ³J_{HH}=6Hz, CH₂), 1.42 (9H, s, *t*-Bu)

f) To a magnetically stirred solution of 160mgs 1-methyl-2-(carboxyethyl)-7-iodo-3,4-dihydro-1H-1,4-benzodiazepine-2,5-dione benzyl ester (0.335mmol) in 5mL ethyl acetate, degassed of oxygen, under an atmosphere of nitrogen was added 123mgs N-boc-5-amino-1-pentyne (0.67mmol), 10mgs bis-triphenylphosphine palladium dichloride (0.014mmol)_{,} 5mgs cuprous iodide (0.026mmol) and 0.233mL triethylamine (1.675mmol). After 2 hours, the reaction mixture was diluted with 50mL ethyl acetate and washed 2X5OmL 10% citric acid, 2X50mL sat. sodium bicarbonate, dried over magnesium sulfate, filtered, and concentrated in *vacuo.* The resulting residue was further purified by column chromatography, using silica gel, eluting with a solvent gradient of 50:50 ethyl acetate/hexane to 75:25 ethyl acetate/hexane (TLC, SiO₂, 1:1 ethyl acetate/ hexane, R_{f}=0.39, un positive) to yield 90mgs (50%) 1-methyl-4-(2-carboxyethyl)-7-(N-boc-5-amino-1-pentynyl)-3,4-dihydro-1*H*-1,4-benzodiazepine-2,5-dione ethyl ester. ¹H NMR (CDCI₃,dTMS) 7.85 (1H,d,⁴JHH=2Hz, Ar-H *o*-CON), 7.48(1H, dd, ⁴JHH=2Hz, ³JHH=8Hz, Ar-H µ-CON), 7.32(5H,s,Ph), 7.06(1H,d, ³JHH=8Hz, Ar-H *m*-CON), 5.10 (2H, s, OCH₂), 4.67 (1H, bs, NH), 3.95 (1H, d, JHH=15Hz, NCHHCO), 3.91 (2H, t, ³J_{HH}=7Hz, NCH₂), 3.81 (1H, d, JHH=15Hz_{,} NCHHCO), 3.29 (3H, s, NCH3), 3.26 (2H, q, ³JHH=7Hz, BocNHCH2), 2.82 (1H, dt, JHH=16Hz, ³JHH=8Hz, CHHCO2), 2.69 (1H, dt, JHH=16Hz, ³JHH=8Hz, CHHCO₂), 2.46 (2H, t, ³JHH=7Hz, CjCCH₂), 1.78 (2H, p, ³JHH=7Hz, CH₂CH₂CH₂).

Using the above procedure, but substituting the appropriate 7-iodo-3,4-dihydro-1*H*-1,4-benzodiazepine-2,5-dione for 1-methyl-2-(carboxyethyl)-7-iodo-3,4-dihydro-1*H*-1,4-benzodiazepine-2,5-dione benzyl ester and alkyne for N-boc-5-amino-1-pentyne there may be prepared, for example, the following compounds:
1-methyl-4-(3-ethoxy-1-methyl-3-oxo-1-propyl)-7-[5-(N-Boc)-amino-1-pentyne]-3,4-dihydro-1H-1,4-benzodiazepine-2,5-dione,
1-methyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1-methyl-4-[2-(1-*t*-butoxy-4-benzyloxy)succinyl)]-7-(5-(N-Boc)-amino-1-pentyne]-3,4-dihydro-1H-1,4-benzodiazepine-2,5-dione,
(±)-1-methyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1-methyl-4-[2-(1-benzyloxyvalinyl-4-benzyloxy)succinyl)]-7-(5-(N-Boc)-amino-1-pentyne]-3,4-dihydro-1H-1,4-benzodiazepine-2,5-dione,
(±)-1-methyl-4-[2-(1-benzyloxyphenylalanyl-4-benzyloxy)succinyl)]-7-[5-(N-Bnc)-amino-1-pentyne]-3,4-dihydro-1H-1,4-benzodiazepine-2,5-dione,
1-phenyl-4-(3-ethoxy-1-methyl-3-oxo-1-propyl)-7-[5-(N-Boc)-amino-1-pentyne]-3,4-dihydro-1H-1,4-benzodiazepine-2,5-dione,
1-phenyl-4-(3-ethoxy-1-phenyl-3-oxo-1-propyl)-7-(5-(N-Boc)-amino-1-pentyne]-3,4-dihydro-1H-1,4-benzodiazepine-2,5-dione,
(±)-1-phenyl-4-[2-(1-*t*-butoxy-4-benzyloxy)succinyl)]-7-[5-(N-Boc)-amino-1-pentyne]-3,4-dihydro-1H-1,4-benzodiazepine-2,5-dione,
(±)-1-phenyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1-phenyl-4-[2-(1-benzyl**o**xyvalinyl-4-benzyloxy)succinyl)]-7-[5-(N-Boc)-amino-1-pentyne]-3,4-dihydro-1H-1,4-benzodiazepine-2,5-dione,
(±)-1-phenyl-4-[2-(1-benzyloxyphenylalanyl-4-benzyloxy)succinyl)]-7-[5-(N-Boc)-amino-1-pentyne]-3,4-dihydro-1H-1,4-benzodiazepine-2,5-dione,
1-benzyl-4-(3-ethoxy-1-methyl-3-oxo-1-propyl)-7-[5-(N-Boc)-amino-1-pentyne]-3,4-dihydro-1H-1,4-benzodiazepine-2,5-dione,
1-benzyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1-benzyl-4-[2-(1-*t*-butoxyl-4-benzyloxy)succinyl)]-7-[5-(N-Boc)-amino-1-pentyne]-3,4-dihydro-1H-1,4-benzodiazepine-2,5-dione,
(±)-1-benzyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1-benzyl-4-[2-(1-benzyloxyvalinyl-4-benzyloxy)succinyl)]-7-[5-(N-Boc)-amino-1-pentyne]-3,4-dihydro-1H-1,4-benzodiazepine-2,5-dione,
(±)-1-benzyl-4-[2-(1-benzyloxyphenylalanyl-4-benzyloxy)succinyl)]-7-[5-(N-Boc)-amino-1-pentyne]-3,4-dihydro-1H-1,4-benzodiazepine-2,5-dione,
1-isopropyl-4-(3-ethoxy-1-methyl-3-oxo-1-propyl)-7-[5-(N-Boc)-amino-1-pentyne]-3,4-dihydro-1H-1,4-benzodiazepine-2,5-dione,
1-isopropyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1-isopropyl-4-[2-(1-*t*-butoxy-4-benzyloxy)succinyl)]-7-[5-(N-Boc)-amino-1-pentyne]-3,4-dihydro-1H-1,4-benzodiazepine-2,5-dione,
(±)-1-isopropyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1-isopropyl-4-[2-(1-benzyloxyvalinyl-4-benzyloxy)succinyl)]-7-[5-(N-Boc)-amino-1-pentyne]-3,4-dihydro-1H-1,4-benzodiazepine-2,5-dione,
(±)-1-isopropyl-4-[2-(1-benzyloxyphenylalanyl-4-benzyloxy)succinyl)]-7-7-[5-(N-Boc)-amino-1-pentyne]-3,4-dihydro-1H-1,4-benzodiazepine-2,5-dione,
1-methyl-4-(3-ethoxy-1-methyl-3-oxo-1-propyl)-7-[5-cyano-1-pentyne]-3,4-dihydro-1H-1,4-benzodiazepine-2,5-dione,
1-methyl-4-(3-ethoxy-1-phenyl-3-oxo-1-propyl)-7-[5-cyano-1-pentyne]-3,4-dihydro-1H-1,4-benzodiazepine-2,5-dione,
(±)-1-methyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1-methyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1-methyl-4-[2-(1-benzyloxyvalinyl-4-benzyloxy)succinyl)]-7-[5-cyano-1-pentyne]**-**3,4**-** dihydro-1H-1,4-benzodiazepine-2,5-dione,
(±)-1-methyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
1-phenyl-4-(3-ethoxy-1-methyl-3-oxo-1-propyl)-7-[5-cyano-1-pentyne]-3,4-dihydro-1H-1,4-benzodiazepine-2,5-dione,
1-phenyl-4-(3-ethoxy-1-phenyl-3-oxo-1-propyl)-7-[5-cyano-1-pentyne]-3,4-dihydro-1H-1,4-benzodiazepine-2,5-dione,
(±)-1-phenyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1-phenyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1-phenyl-4-[2-(1-benzyloxyvalinyl-4-benzyloxy)succinyl)]-7-[5-cyano-1-pentyne]-3,4-dihydro-1H-1,4-benzodiazepine-2,5-dione,
(±)-1-phenyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
1-benzyl-4-(3-ethoxy-1-methyl-3-oxo-1-propyl)-7-[5-cyano-1-pentyne]-3,4-dihydro-1H-1,4-benzodiazepine-2,5-dione,
1-benzyl-4-(3-ethoxy-1-phenyl-3-oxo-1-propyl)-7-[5-cyano-1-pentyne]-3,4-dihydro-1H-1,4-benzodiazepine-2,5-dione,
(±)-1-benzyl-4-[2-(1-*t*-butoxy-4-benzyloxy)succinyl)]-7-[5-cyano-1-pentyne]-3,4-dihydro-1H**-** 1,4-benzodiazepine-2,5-dione,
(±)-1-benzyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1-benzyl-4-[2-(1-benzyloxyvalinyl-4-benzyloxy)succinyl)]-7-[5-cyano-1-pentyne]-3,4-dihydro-1H-1,4-benzodiazepine-2,5-dione,
(±)-1-benzyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
1-isopropyl-4-(3-ethoxy-1-methyl-3-oxo-1-propyl)-7-[5-cyano-1-pentyne]-3,4-dihydro-1H-1,4-benzodiazepine-2,5-dione,
1-isopropyl-4-(3-ethoxy-1-phenyl-3-oxo-1-propyl)-7-[5-cyano-1-pentyne]-3,4-dihydro-1H-1,4-benzodiazepine-2,5-dione,
(±)-1-isopropyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1-isopropyl-4-[2-(1-benzyloxyglycinyl-4-benzyloxy)succinyl)-7-[5-cyano-1-pentyne]-3,4-dihydro-1H-1,4-benzodiazepine-2,5-dione,
(±)-1-isopropyl-4-[2-(1-benzyloxyvalinyl-4-benzyloxy)succinyl)]-7-1[5-cyano-1-pentyne]-3,4-dihydro-1H-1,4-benzodiazepine-2,5-dione,
(±)-1-isopropyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
1-methyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
1-methyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-methyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-methyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-methyl-4-[2-(1-benzyloxyvalinyl-4-benzyloxy)succinyl)]-7-[2-(3-cyanophenyl)-1-ethyne]-3,4-dihydro-1H-3,4-benzodiazepine-2,5-dione,
(±)-1-methyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
1-phenyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
1-phenyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-phenyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-phenyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-phenyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1-phenyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
1-benzyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
1-benzyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-benzyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-benzyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-benzyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1-benzyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
1-isopropyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
1-isopropyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-isopropyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-isopropyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-isopropyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1-isopropyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
1-methyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
1-methyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-methyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-methyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-methyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-methyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
1-phenyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
1-phenyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-phenyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-phenyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-phenyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-phenyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
1-benzyl-4-(3-ethoxy-1-methyl-3-oxo-1-propyl)-7-[2-(4-nitropheny)-1-ethyne]-3,4-dihydro-1H-1,4-benzodiazepine-2,5-dione,
1-benzyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-benzyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-benzyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-benzyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-benzyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
1-isopropyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
1-isopropyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-isopropyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-isopropyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-isopropyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-isopropyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
1-methyl-4-(3-ethoxy-1-methyl-3-oxo-1-propyl)-7-[N-[2-(N-Boc)-aminoethane]-3-keto-1-propyn-3-amine]-3,4-dihydro-1H**-**1,4-benzodiazepine-2,5-dione,
1-methyl-4-(3-ethoxy-1-phenyl-3-oxo-propyl)-7-[N-[2-(N-Boc)-aminoethane]-3-keto-1-propyn-3-amine]-3,4-dihydro-1H-1,4-benzodiazepine-2,5-dione,
(±)-1-methyl-4-[2-(1-*t*-butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-4-[2-(1-benzyloxyvalinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
1-phenyl-4-(3-ethoxy-1-methyl-3-oxo-1-propyl)-7-[N-[2-(N-Boc)-aminoethane]-3-keto-1-propyn-3-amine]-3,4-dihydro-1H-1,4-benzodiazepine-2,5-dione,
1-phenyl-4-(3-ethoxy-1-phenyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-phenyl-4-[2-(1-*t-*butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-phenyl-4-[2-(1-benzyloxyglycinyl-4-benzyloxy)succinyl)]-7-[N-[2-(N-Boc)-aminoethane]-3-kelo-1-propyn-3-amine]-3,4-dihydro-1H-1,4-benzodiazepine-2,5-dione,
(±)-1-phenyl-4-[2-(1-benzyloxyvalinyl-4-benzyloxy)succinyl)]-7-[N-[2-(N-Boc)-aminoethane]-3-keto-1-propyn-3-amine]-3,4-dihydro-1-H-1,4-benzodiazepine-2,5-dione,
(±)-1-phenyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
1-benzyl-4-(3-ethoxy-1-methyl-3-oxo-1-propyl)-7-[N-[2-(N-Boc)-aminoethane]-3-keto-1-propyn-3-amine]-3,4-dihydro-1H-1,4-benzodiazepine-2,5-dione,
1-benzyl-4-(3-ethoxy-1-phenyl-3-oxo-1-propyl)-7-[N-[2-(N-Boc)-aminoethane]-3-keto-1-propyn-3-amine]-3,4-dihydro-1H**-**1,4-benzodiazepine-2,5-dione,
(±)-1-benzyl-4-[2-(1-*t*-butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-benzyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione
(±)-1-benzyl-4-[2-(1-benzyloxyvalinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-benzyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
1-isopropyl-1-(3-ethoxy-1-methyl-3-oxo-1-propyl)-7-[2-(N-Boc)-aminoethane]-3-keto-1-propyn-3-amine]-3,4-dihydro-1H-1,4-benzodiazepine-2,5-dione
1-isopropyl-4-(3-ethoxy-1-phenyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-isopropyl-4-[2-(1-*t-*butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-4-[2-(1-benzyloxyvalinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
1-methyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
1-methyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-methyl-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-methyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-methyl-4-[2-(1-benzyloxyvalinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-methyl-4-[2-(1-benzyloxyphenylalanyl-4-benzyloxy)succinyl)]-7-[3-keto-1-propyn-3-[4-(N-Boc)-1-piperizine]]-3,4-dihydro-1H-1,4-benzodiazepine-2,5-dione,
1-phenyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
1-phenyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-phenyl-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-phenyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-phenyl-4-[2-(1-benzyloxyvalinyl-4-benzyloxy)succinyl)]-7-(3-keto-1-propyn-3-[4-(N-Boc)-1-piperizine]]-3,4-dihydro-1H-1,4-benzodiazepine-2,5-dione,
(±)-1-phenyl-4-[2-(1-benzyloxyphenylalanyl-4-benzyloxy)succinyl)]-7-[3-keto-1-propyn-3-[4-(N-Boc)-1-piperizine]]-3,4-dihyaro-1H-1,4-benzodiazepine-2,5-dione,
1-benzyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
1-benzyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-benzyl-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-benzyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-benzyl-4-[2-(1-benzyloxyvalinyl-4-benzyloxy)succinyl)]-7-[3-keto-1-propyn-3-[4-(N-Boc)-1-piperizine]],3,4-dihydro-1H-1,4-benzodiazepine-2,5-dione,
(±)-1-benzyl-4-[2-(1-benzyloxyphenylalanyl-4-benzyloxy)succinyl)]-7-[3-keto-1-propyn-3-[4-(N-Boc)-1-piperizine]]-3,4-dihydro-1H-1,4-benzodiazepine-2,5-dione,
1-isopropyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
1-isopropyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-isopropy-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-4-[2-(1-benzyloxyvalinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-4-[2-(1-benzyloxyphenylalanyl-4-benzyloxy)succinyl)]-7-[3**-**keto-1-propyn-3-[4-(N-Boc)-1-piperizine]]-3,4-dihydro-1H-1,4-benzodiazepine-2,5-dione,
1-methyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
1-methyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1-methyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
1**-**phenyl-4-(3-ethoxy-1-methyl-3-oxo-1**-**propyl)-7-[2-[2-(N-Boc)aminoethanethiol]-1-ethyne]-3,4-dihydro-1H-1,4-benzodiazepine-2,5-dione,
1-phenyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1-phenyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-phenyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-phenyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-phenyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
1-benzyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
1-benzyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1-benzyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
1-isopropyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
1-isopropyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1-isopropyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-4-[2-(1-benzyloxyphenylalanyl-4-benzyloxy)succinyl)]-7-[2-[2-(N-Boc)aminoethanethiol]-1-ethyne]-3,4-dihydro,1H-1,4-benzodiazepine-2,5-dione,
1-methyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
1-methyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-methyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-methyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione,
(±)-1-methyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-methyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
1-phenyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
1-phenyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-phenyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-phenyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione,
(±)-1-phenyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-phenyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
1-benzyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
1-benzyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-benzyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-benzyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione
(±)-1-benzyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-benzyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
1-isopropyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
1-isopropyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-isopropyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-isopropyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione,
(±)-1-isopropyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-isopropyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
1-methyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
1-methyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
1-phenyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
1-phenyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-phenyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-phenyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-phenyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-phenyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
1-benzyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
1-benzyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
1-isopropyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
1-isopropyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
1-methyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
1-methyl-4-(3-ethoxy-1-phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-methyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-methyl-4-[2-(1-benzyloxyglycinyl-4-benzyloxy)succinyl)]-7-[5-[2-(N-Boc)aminopyridine-3-yl]-5-oxa-1-pentynel-3,4-dihydro-1H-1,4-benzodiazepine-2,5-dione,
(±)-1-methyl-4-[2-(1-benzyloxyvalinyl-4-benzyloxy)succ)nyl)]-7-[5-[2-(N-Boc)aminopyridine-3-yl]-5-oxa-1-pentyne)-3,4-dihydro-1H-1,4-benzodiazepine-2,5-dione,
(±)-1-methyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
1-phenyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
1-phenyl-4-(3-ethoxy-1**-**phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-phenyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-phenyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-phenyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-phenyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
1-benzyl-4-(3-ethoxy-1-methyl-3-oxo-1-propyl)-7-[5-[2-(N-Boc)aminopyridine-3yl]-5-oxa-1-pentyne]-3,4-dihydro-1H-1,4-benzodiazepine-2,5-dione,
1-benzyl-4-(3-ethoxy-1-phenyl-3-oxo-1-propyl)-7-[5-[2-(N-Boc)aminopyridine-3-yl]-5-oxa-1-pentyne)-3,4-dihydro-1H-1,4-benzodiazepine-2,5-dione,
(±)-1-benzyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
1-isopropyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
1-isopropyl-4-(3-ethoxy-1-phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-isopropyl-4-[2-(1-*t*-butoxy-4-benzyloxy)succinyl)]-7-[5-[2-(N-Boc)aminopyridine-3-yl]-5-oxa-1-pentyne]-3,4-dihydro-1H -1,4-benzodiazepine-2,5-dione,
(±)-1-isopropyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-isopropyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-isopropyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-methyl-3-phenyl-4-(3-ethoxy-1-methyl-3-oxo-1-propyl)-7-[5-(N-Boc)-amino-1-pentyne]-3,4-dihydro-1H-1,4-benzodiazepine-2,5-dione,
(±)-1-methyl-3-phenyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1-methyl-3-phenyl-4-[2-(1-*t*-butoxy-4-benzyloxy)succinyl)]-7-[5-(N-Boc)-amino-1-pentyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[5-(N-Boc)-amino-1-pentyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[5-(N-Boc)-amino-1-pentyne]-3,4-dihydro-1H-1,4-benzodiazepine-2,5-dione,
(±)-1,3-diphenyl-4-(3-ethoxy-1-methyl-3-oxo-1-propyl)-7-[5-(N-Boc)-amino-1-pentyne]-3,4-dihydro-1H-1,4-benzodiazepine-2,5-dione,
(±)-1,3-diphenyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1,3-diphenyl-4-[2-(1-*t*-butoxy-4-benzyloxy)succinyl)]-7-[5-(N-Boc)-amino-1-pentyne]-3,4-dihydro-1H-1,4-benzodiazepine-2,5-dione,
(±)-1,3-diphenyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1,3-diphenyl-4-[2-(1-benzyloxyvalinyl-4-benzyloxy)succinyl)]-7-[5-(N-Boc)-amino-1-pentyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[5-(N-Boc)-amino-1-pentyne]-3,4-dihydro-1H-1,4-benzodiazepine-2,5-dione,
(±)-1-benzyl-3-phenyl-4-(3-ethoxy-1-methyl-3-oxo-1-propyl)-7-[5-(N-Boc)-amino-1-pentyne]-3,4-dihydro-1H-1,4-benzodiazepine-2,5-dione,
(±)-1-benzyl-3-phenyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[5-(N-Boc)-amino-1-pentyne]-3,4-dihydro-1H-1,4-benzodiazepine-2,5-dione,
(±)-1-benzyl-3-phenyl-4-[2-(1-benzyloxyglycinyl-4-benzyloxy)succinyl)-7-[5-(N-Boc)-amino-1-pentyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[5-(N-Boc)-amino-1-pentyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[5-(N-Boc)-amino-1-pentyne]-3,4-dihydro-1H-1,4-benzodiazepine-2,5-dione,
(±)-1-isopropyl-3-phenyl-4-(3-ethoxy-1-methyl-3-oxo-1-propyl)-7-[5-(N-Boc)-amino-1-pentyne]-3,4-dihydro-1H-1,4-benzodiazepine-2,5-dione,
(±)-1-isopropyl-3-phenyl-4-(3-ethoxy-1-phenyl-3-oxo-1-propyl)-7-[5-(N-Boc)-amino-1-pentyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[5-(N-Boc)-amino-1-pentyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[5-(N-Boc)-amino-1-pentyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[5-(N-Boc)-amino-1-pentyne] -3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[5-(N-Boc)-amino-1-pentyne]-3,4-dihydro-1H-1,4-benzodiazepine-2,5-dione,
(±)-1-methyl-3-phenyl-4-(3-ethoxy-1-methyl-3-oxo-1-propyl)-7-[5-cyano-1-pentyne]-3,4-dihydro-1H-1,4-benzodiazepine-2,5-dione,
(±)-1-methyl-3-phenyl-4-(3-ethoxy-1-phenyl-3-oxo-1-propyl)-7-[5-cyano-1-pentyne]-3,4-dihydro-1H-1,4-benzodiazepine-2,5-dione,
(±)-1-methyl-3-phenyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[5-cyano-1-pentyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
(±)-1,3-diphenyl-4-(3-ethoxy-1-methyl-3-oxo-1-propyl)-7-[5-cyano-1-pentyne]-3,4-dihydro-1H-1,4-benzodiazepine-2,5-dione,
(±)-1,3-diphenyl-4-(3-ethoxy-1-phenyl-3-oxo-1-propyl-7-[5-cyano-1-pentyne]-3,4-dihydro-1H-1,4-benzodiazepine-2,5-dione,
(±)-1,3-diphenyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1,3-diphenyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1,3-diphenyl-4-[2-(1-benzyloxyvalinyl-4-benzyloxy)succinyl)]-7-[5-cyano-1-pentyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
(±)-1-benzyl-3-phenyl-4-(3-ethoxy-1-methyl-3-oxo-1-propyl)-7-[5-cyano-1-pentyne]-3,4-dihydro-1H-1,4-benzodiazepine-2,5-dione,
(±)-1-benzyl-3-phenyl-4-(3-ethoxy-1-phenyl-3-oxo-1-propyl)-7-[5-cyano-1-pentyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[5-cyano-1-pentyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
(±)-1-isopropyl-3-phenyl-4-(3-ethoxy-1-methyl-3-oxo-1-propyl)-7-[5-cyano-1-pentyne]-3,4-dihydro-1H-1,4-benzodiazepine-2,5-dione,
(±)-1-isopropyl-3-phenyl-4-(3-ethoxy-1-phenyl-3-oxo-1-propyl)-7-[5-cyano-1-pentyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[5-cyano-1-pentyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[5-cyano-1-pentyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[5-cyano-1-pentyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[5-cyano-1-pentyne]-3,4-dihydro-1H-1,4-benzodiazepine-2,5-dione,
(±)-1-methyl-3-phenyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
(±)-1-methyl-3-phenyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-methyl-3-phenyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1-methyl-3-phenyl 4-[2-(1-benzyloxyphenylalanyl-4-benzyloxy)succinyl)]-7-[2-(3-cyanophenyl)-1-ethyne]-3,4-dihydro-1H -1,4-benzodiazepine-2,5-dione,
(±)-1,3-diphenyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
(±)-1,3-diphenyl-4-(3-ethoxy-1**-**phenyl-3-oxo-1-propyl)-7-[2-(3-cyanophenyl)-1-ethyne]-3,4-dihydro-1H-1,4-benzodiazepine-2,5-dione,
(±)-1,3-diphenyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1,3-diphenyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1,3-diphenyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
(±)-1-benzyl-3-phenyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
(±)-1-benzyl-3-phenyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
(±)-1-isopropyl-3-phenyl-4-(3-ethoxy-1-methyl-3-oxo-1-propyl)-7-[2-(3-cyanophenyl)-1-ethyne]-3,4-dihydro-1H-1,4-benzodiazepine-2,5-dione,
(±)-1-isopropyl-3-phenyl-4-(3-ethoxy-1-phenyl-3-oxo-1-propyl)-7-[2-(3-cyanophenyl)-1-ethyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[2-(3-cyanophenyl)-1-ethyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-(3-cyanophenyl)-1-ethyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-(3-cyanophenyl)-1-ethyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-(3-cyanophenyl)-1-ethyne]-3,4-dihydro-1H-1,4-benzodiazepine-2,5-dione,
(±)-1-methyl-3-phenyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
(±)-1-methyl-3-phenyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-methyl-3-phenyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
(±)-1,3-diphenyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
(±)-1,3-diphenyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1,3-diphenyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1,3-diphenyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1,3-diphenyl-4-[2-(1-benzyloxyvalinyl-4-benzyloxy)succinyl)]-7-[2-(4-nitrophenyl-1-ethyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
(±)-1-benzyl-3-phenyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
(±)-1-benzyl-3-phenyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
(±)-1-isopropyl-3-phenyl-4-(3-ethoxy-1-methyl-3-oxo-1-propyl)-7-[2-(4-nitrophenyl)-1-ethyne]-3,4-dihydro-1H-1,4-benzodiazepine-2,5-dione,
(±)-1-isopropyl-3-phenyl-4-(3-ethoxy-1-phenyl-3-oxo-1-propyl)-7-[2-(4-nitrophenyl)-1-ethyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[2-(4-nitrophenyl)-1-ethyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-(4-nitrophenyl)-1-ethyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-(4-nitrophenyl)-1-ethyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-(4-nitrophenyl)-1-ethyne]-3,4-dihydro-1H-1,4-benzodiazepine-2,5-dione,
(±)-1-methyl-3-phenyl-4-(3-ethoxy-1-methyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-methyl-3-phenyl-4-(3-ethoxy-1-phenyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-methyl-3-phenyl-4-[2-(1-*t*-butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1,3-diphenyl-4-(3-ethoxy-1-methyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1,3-diphenyl-4-(3-ethoxy-1-phenyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1,3-diphenyl-4-[2-(1-*t*-butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1,3-diphenyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1,3-diphenyl-4-[2-(1-benzyloxyvalinyl-4-benzyloxy)succinyl)]-7-[N-[2-(N-Boc-aminoethane]-3-keto-1-propyn-3-amine]-3,4-dihydro-1H-1,4-benzodiazepine-2,5-dione,
(±)-1,3-diphenyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-benzyl-3-phenyl-4-(3-ethoxy-1-methyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-benzyl-3-phenyl-4-(3-ethoxy-1-phenyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-benzyl-3-phenyl-4-[2-(1-*t*-butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-3-phenyl-4-(3-ethoxy-1-methyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-isopropyl-3-phenyl-4-(3-ethoxy-1-phenyl-3-oxo-1-propyl)-7-[N-[2-(N-Boc)-aminoethane]-3-keto-1-propyn-3-amine]-3,4-dihydro-1H-1,4-benzodiazepine-2,5-dione,
(±)-1-isopropyl-3-phenyl-4-[2-(1-*t*-butoxy-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[N-[2-(N-Boc)-aminoethane]-3-keto-1-propyn-3-amine]-3,4-dihydro-1H-1,4-benzodiazepine-2,5-dione,
(±)-1-methyl-3-phenyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-methyl-3-phenyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-methyl-3-phenyl-4-[2-(1-*t*-butoxy-4-benzyloxy)succinyl)]-7-(3-keto-1-propyn-3-[4-(N-Boc)-1-piperizine]]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1,3-diphenyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
(±)-1,3-diphenyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1,3-diphenyl-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1,3-diphenyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1,3-diphenyl-4-[2-(1-benzyloxyvalinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1,3-diphenyl-4-[2-(1-benzyloxyphenylalanyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-benzyl-3-phenyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-benzyl-3-phenyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-benzyl-3-phenyl-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-3-phenyl-4-(3-ethoxy-1-methyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-isopropyl-3-phenyl-4-(3-ethoxy-1-phenyl-3-oxo-1-propyl)-7-[3-keto-1-propyn-3-[4-(N-Boc)-1-piperizine]]-3,4-dihydro-1H-1,4-benzodiazepine-2,5-dione,
(±)-1-isopropyl-3-phenyl-4-[2-(1-*t*-butoxy-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[3-keto-1-propyn-3-[4-(N-Boc)-1-piperizine]]-3,4-dihydro-1H-1,4-benzodiazepine-2,5-dione,
(±)-1-methyl-3-phenyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
(±)-1-methyl-3-phenyl-4-(3-ethoxy-1-phenyl-3-oxoi-1-propyl)-7-[2-[2-(N-Boc)aminoethanethiol]-1-ethyne]-3,4-dihydro-1H-1,4-benzodiazepine-2,5-dione,
(±)-1-methyl-3-phenyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
(±)-1,3-diphenyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
(±)-1,3-diphenyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1,3-diphenyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1,3-diphenyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1,3-diphenyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-3-phenyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
(±)-1-benzyl-3-phenyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-3-phenyl-4-(2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-3-phenyl-4-(3-ethoxy-1-methyl-3-oxo-1-propyl)-7-[2-[2-(N-Boc)aminoethanethiol]-1-ethyne]-3,4-dihydro-1H-1,4-benzodiazepine-2,5-dione,
(±)-1-isopropyl-3-phenyl-4-(3-ethoxy-1-phenyl-3-oxo-1-propyl)-7-[2-[2-(N-Boc)aminoethanethiol]-1-ethyne]-3,4-dihydro-1H-1,4-benzodiazepine-2,5-dione,
(±)-1-isopropyl-3-phenyl-1-[2-(1-*t*-butoxy-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-[2-(N-Boc)aminoethanethiol]-1-ethyne]-3,4-dihydro-1H-1,4-benzodiazepine-2,5-dione,
(±)-1-methyl-3-phenyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
(±)-1-methyl-3-phenyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-methyl-3-phenyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
(±)-1,3-diphenyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
(±)-1,3-diphenyl-4-(3-ethoxy-1-phenyl-3-oxo-1**-**propyl)-7-(2-(4-cyanothiophenol)-1-ethyne]-3,4-dihydro-1H-1,4-benzodiazepine-2,5-dione,
(±)-1,3-diphenyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1,3-diphenyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione,
(±)-1,3-diphenyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
(±)-1-benzyl-3-phenyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
(±)-1-benzyl-3-phenyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-benzyl-3-phenyl-4-[2-(1-benzyloxyglycinyl)-4-benzyloxy)succinyl)]-7-[2-(4-cyanothiophenol)-1-ethyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
(±)-1-isopropyl-3-phenyl-4-(3-ethoxy-1-methyl-3-oxo-1-propyl)-7-[2-(4-cyanothiophenol)-1-ethyne]-3,4-dihydro-1H-1,4-benzodiazepine-2,5-dione,
(±)-1-isopropyl-3-phenyl-4-(3-ethoxy-1-phenyl-3-oxo-1-propyl)-7-[2-(4-cyanothiophenol)-1-ethyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[2-(4-cyanothiophenol)-1-ethyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-(4-cyanothiophenol)-1-ethyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-(4-cyanothiophenol)-1-ethyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-(4-cyanothiophenol)-1-ethyne]-3,4-dihydro-1H-1,4-benzodiazepine-2,5-dione,
(±)-1-methyl-3-phenyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-3-phenyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-3-phenyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[4-(2-(N-Boc)amino-6-methylpyrimidine-4-yl]-4-oxa-1-butyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
1(±)-,3-diphenyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1,3-diphenyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1,3-diphenyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1,3-diphenyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1,3-diphenyl-4-[2-(1-benzyloxyvalinyl-4-benzyloxy)succinyl)]-7-[4-[2-(N-Boc)amino-6-methylpyrimidine-4-yl]4-oxa-1-butyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-3-phenyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-3-phenyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-3-phenyl-4-(3-ethoxy-1-methyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-3-phenyl-4-(3-ethoxy-1-phenyl-3-oxo-1-propyl)-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isoproryl-3-phenyl-4-[2-(1-benzyloxyvalinyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[4-[2-(N-Boc)amino-6-methylpyrimidine-4-yl]-4-oxa-1-butyne]-3,4-dihydro-1H-1,4-benzodiazepine-2,5-dione,
(±)-1-methyl-3-phenyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-methyl-3-phenyl-4-(3-ethoxy-1-phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-methyl-3-phenyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1,3-diphenyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1,3-diphenyl-4-(3-ethoxy-1-phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1,3-diphenyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1,3-diphenyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1,3-diphenyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1,3-diphenyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-3-phenyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-benzyl-3-phenyl-4-(3-ethoxy-1-phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-benzyl-3-phenyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-isopropyl-3-phenyl-4-(3-ethoxy-1-methyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-isopropyl-3-phenyl-4-(3-ethoxy-1-phenyl-3-oxo-1-propyl)-7-[5-[2-(N-Boc)aminopyridine-3-yl]-5-oxa-1-pentyne]-3,4-dihydro-1H-1,4-benzodiazepine-2,5-dione,
(±)-1-isopropyl-3-phenyl-4-[2-(1-*t*-butoxy-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[5-[2-(N-Boc)aminopyridine-3-yl]-5-oxa-1-pentyne]-3,4-dihydro-1H-1,4-benzodiazepine-2,5-dione,

g) To a solution of 22mgs 1-methyl-4-(2-carboxyethyl)-7-(N-boc-5-amino-1-pentynyl)-3,4-dihydro-1*H*-1,4-benzodiazepine-2,5-dione benzyl ester (0.0412mmol) in 0.5mL ethyl acetate was added 3mL sat. HCI in ethyl acetate. After 1 hour, the mixture was concentrated *in vacuo* and diluted with 2mL methanol. To the methanolic solution was added 0.5mL 2N sodium hydroxide. After 1 hour, the reaction was quenched with 3mL acetic acid, concentrated *in vacuo,* diluted with 3mL water and purified by high pressure liquid chromatography, using a 1/2" C-18 reverse-phase column, eluting with a solvent gradient of 10:90 methanol(0.1% trifluoracetic acid)/ water (0.1% trifluoracetic acid), time 0 to 10 min, to 50:50 methanol(0.1% trifluoracetic acid)/water (0.1% trifluoracetic acid), time 10 min to 40 min, flow = 10ml/min (Rₜ=33.1 min, un detection 254nm) to yield 13mgs (92%) 1-methyl-4-(2-carboxyethyl)-7-(5-amino-1-pentynyl)-3,4-dihydro-1*H*-1,4-benzodiazepine-2,5-dione trifluoracetate. HRMS (FAB) molecular ion m/z=344.1626 (cald. C₁₈H₂₂N₃O₄, 344.1610). 1_{H} NMR (D₂O) 7.63 (1H, d, ⁴J_{HH}, Ar-H *o*-CON), 7.54 (1H, dd, ⁴J_{HH}=2Hz, ³J_{HH}=8Hz, Ar-H *p*-CON), 7.20 (1H, d, ³J_{HH}=8Hz, Ar-H *m*-CON), 4.05 (1H, d, J_{HH}=14Hz, NCHHCO), 4.0 (1H, m, NCHHCH₂), 3,74 (1H, d, J_{HH}=14Hz, NCHHCO), 3.59 (1H, m, NCHHCH₂), 3.20 (3H, s, NCH₃), 3.03 (2H, t, ³J_{HH}=7Hz, NCH₂), 2.59 (2H, m, CH₂CO₂), 2.42 (2H, t, ³J_{HH}=7Hz, C∫CCH₂), 1.82 (2H, p, ³J_{HH}=7Hz, CH₂CH₂CH₂).

Using the above procedure, but substituting the appropriate 3,4-dihydro-1*H*-1,4-benzodiazepine-2,5-dione for 1-methyl-4-(2-carboxyethyl)-7-(N-boc-5-amino-1-pentynyl)-3,4-dihydro-1*H*-1,4-benzodiazepine-2,5-dione benzyl ester there may be prepared, for example, the following compounds:
1-methyl-4-(2-carboxy-1-methylethyl)-7-(5-amino-1-pentyne)-3,4-dihydro-1H-1,4-benzodiazepine-2,5-dione trifluoracetate,
1-methyl-4-(2-carboxy-1-phenylethyl)-7-(5-amino-1-pentyne)-3,4-dihydro-1H-1,4-benzodiazepine-2,5-dione trifluoracetate,
(±)-1-methyl-4-[succin-2-yl-(1-*t*-butoxy)]-7-(5-amino-1-pentyne)-3,4-dihydro-1H-1,4-benzodiazepine-2,5-dione trifluoracetate,
(±)-1-methyl-4-[succin-2-yl-(1-glycine)]-7-(5-amino-1-pentyne)-3,4-dihydro-1H-1,4-benzodiazepine-2,5-dione trifluoracetate,
(±)-1-methyl-4-[succin-2-yl-(1-valine)]-7-(5-amino-1-pentyne)-3,4-dihydro-1H-1,4-benzodiazepine-2,5-dione trifluoracetate,
(±)-1-methyl-4-[succin-2-yl-(1-phenylalanine)]-7-(5-amino-1-pentyne)-3,4-dihydro-1H-1,4-benzodiazepine-2,5-dione trifluoracetate,
1-phenyl-4-(2-carboxy-1-methylethyl)-7-(5-amino-1-pentyne)-3,4-dihydro-1H-1,4-benzodiazepine-2,5-dione trifluoracetate,
1-phenyl-4-(2-carboxy-1-phenylethyl)-7-(5-amino-1-pentyne)-3,4-dihydro-1H-1,4-benzodiazepine-2,5-dione trifluoracetate,
(±)-1-phenyl-4-[succin-2-yl-(1-*t*-butoxy)]-7-(5-amino-1-pentyne)-3,4-dihydro-1H-1,4-benzodiazepine-2,5-dione trifluoracetate,
(±)-1-phenyl-4-[succin-2-yl-(1-glycine)]-7-(5-amino-1-pentyne)-3,4-dihydro-1H-1,4-benzodiazepine-2,5-dione trifluoracetate,
(±)-1-phenyl-4-[succin-2-yl-(1-valine)]-7-(5-amino-1-pentyne)-3,4-dihydro-1H-1,4-benzodiazepine-2,5-dione trifluoracetate,
(±)-1-phenyl-4-[succin-2-yl-(1-phenylalanine)]-7-(5-amino-1-pentyne)-3,4-dihydro-1H-1,4-benzodiazepine-2,5-dione trifluoracetate,
1-benzyl-4-(2-carboxy-1-methylethyl)-7-(5-amino-1-pentyne)-3,4-dihydro-1H-1,4-benzodiazepine-2,5-dione trifluoracetate,
1-benzyl-4-(2-carboxy-1-phenylethyl)-7-(5-amino-1-pentyne)-3,4-dihydro-1H-1,4-benzodiazepine-2,5-dione trifluoracetate,
(±)-1-benzyl-4-[succin-2-yl-(1-*t*-butoxy)]-7-(5-amino-1-pentyne)-3,4-dihydro-1H-1,4-benzodiazepine-2,5-dione trifluoracetate,
(±)-1-benzyl-4-[succin-2-yl-(1-glycine)]-7-(5-amino-1-pentyne)-3,4-dihydro-1H-1,4-benzodiazepine-2,5-dione trifluoracetate,
(±)-1-benzyl-4-[succin-2-yl-(1-valine)]-7-(5-amino-1-pentyne)-3,4-dihydro-1H-1,4-benzodiazepine-2,5-dione trifluoracetate,
(±)-1-benzyl-4-[succin-2-yl-(1-phenylalanine)]-7-(5-amino-1-pentyne)-3,4-dihydro-1H-1,4-benzodiazepine-2,5-dione trifluoracetate,
1-isopropyl-4-(2-carboxy-1-methylethyl)-7-(5-amino-1-pentyne)-3,4-dihydro-1H-1,4-benzodiazepine-2,5-dione trifluoracetate,
1-isopropyl-4-(2-carboxy-1-phenylethyl)-7-(5-amino-1-pentyne)-3,4-dihydro-1H-1,4-benzodiazepine-2,5-dione trifluoracetate,
(±)-1-isopropyl-4-[succin-2-yl-(1-*t*-butoxy)]-7-(5-amino-1-pentyne)-3,4-dihydro-1H-1,4-benzodiazepine-2,5-dione trifluoracetate,
(±)-1-isopropyl-4-[succin-2-yl-(1-glycine)]-7-(5-amino-1-pentyne)-3,4-dihydro-1H-1,4-benzodiazepine-2,5-dione trifluoracetate,
(±)-1-isopropyl-4-[succin-2-yl-(1-valine)]-7-(5-amino-1-pentyne)-3,4-dihydro-1H-1,4-benzodiazepine-2,5-dione trifluoracetate,
(±)-1-isopropyl-4-[succin-2-yl-(1-phenylalanine)]-7-(5-amino-1-pentyne)-3,4-dihydro-1H-1,4-benzodiazepine-2,5-dione trifluoracetate,
1-methyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
1-methyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-phenyl-4-(2-carboxy-1-methylethyl)-7-(N-(2-aminoethane)-3-keto-1-propyn-3-amine]-3,4-dihydro-1H-1,4-benzodiazepine-2,5-dione trifluoracetate,
1-phenyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-phenyl-4-(succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(-phenylalanine)]-7-[N-(2-aminoethane)-3-keto-1-propyn-3-amine]-3,4-dihydro-1H-1,4-benzodiazepine-2,5-dione trifluoracetate,
1-benzyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
1-benzyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-isopropyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
1-isopropyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-methyl-4-(2-carboxy-1-methylethyl)-7-[3-keto-1-propyn-3-(1-piperizine)]-3,4-dihydro-1H-1,4-benzodiazepine-2,5-dione trifluoracetate,
1-methyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-phenyl-4-(2-carboxy-1-methylethyl)-7-[3-keto-1-propyn-3-(1-piperizine)]-3,4-dihydro-1H-1,4-benzodiazepine-2,5-dione trifluoracetate,
1-phenyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-*t-*butoxy)]-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-benzyl-4-(2-carboxy-1-methylethyl)-7-[3-keto-1-propyn-3-(1-piperizine)]-3,4-dihydro-1H-1,4-benzodiazepine-2,5-dione trifluoracetate,
1-benzyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)1-benzyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-isopropyl-4-(2-carboxy-1-methylethyl)-7-[3-keto-1-propyn-3-(1-piperizine)]-3,4-dihydro-1H-1,4-benzodiazepine-2,5-dione trifluoracetate,
1-isopropyl-4-(2-carboxy-1-phenylethyl)-7-[3-keto-1-propyn-3-(1-piperizine)]-3,4-dihydro-1lH-1,4-benzodiazepine-2,5-dione trifluoracetate,
(±)-1-isopropyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-isopropyl-4-[succin-2-yl-(1-glycine)]-7-[3-keto-1-propyn-3-(1-piperizine)]-3,4dihydro-1H-1,4-benzodiazepine-2,5-dione trifluoracetate,
(±)-1-isopropyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-isopropyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-methyl-4-(2-carboxy-1-methylethyl)-7-[2-(2-aminoethanethiol)-1-ethyne]-3,4-dihydro-1H-1,4-benzodiazepine-2,5-dione trifluoracetate,
1-methyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1-methyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-methyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-methyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-methyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
1-phenyl-4-(2-carboxy-1-methylethyl)-7-[2-(2-aminoethanethiol)-1-ethyne]-3,4-dihydro-1H-1,4-benzodiazepine-2,5-dione trifluoracetate,
1-phenyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1-phenyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-phenyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-phenyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-phenyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
1-benzyl-4-(2-carboxy-1-methylethyl)-7-[2-(2-aminoethanethiol)-1-ethyne]-3,4-dihydro-1H-1,4-benzodiazepine-2,5-dione trifluoracetate,
1-benzyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1-benzyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-benzyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-benzyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-benzyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
1-isopropyl-4-(2-carboxy-1-methylethyl)-7-[2-(2-aminoethanethiol)-1-ethyne]-3,4-dihydro-1H-1,4-benzodiazepine-2,5-dione trifluoracetate,
1-isopropyl-4-(2-carhoxy-1-phenylethyl)-7-[2-(2-aminoethanethiol)-1-ethyne]-3,4-dihydro-1H-1,4-benzodiazepine-2,5-dione trifluoracetate,
(±)-1-isopropyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-isopropyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-isopropyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-isopropyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
1-methyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
1-methyl-4-(2-carboxy-1-phenylethyl)-7-[1-(2-amino-6-methylpyrimidine-4-yl)-4-oxa-1-butyne]-3,4-dihydro-1H-1,4-benzodiazepine-2,5-dione trifluoracetate,
(±)-1-methyl-4-(succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-methyl-4-[succin-2-yl-(1-glycine)]-7-(4-(2-amino-6-methylpyrimidine-4-yl)-4-oxa-1-butyne]-3,4-dihydro-1H-1,4-benzodiazepine-2,5-dione trifluoracetate,
(±)-1-methyl-4-[succin-2-yl-(1-valine)]-7-[4-(2-amino-6-metylpyrimidine-4-yl)-4-oxa-1-butyne]-3,4-dihydro-1H-1,4-benzodiazepine-2,5-dione trifluoracetate,
(±)-1-methyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
1-phenyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
1-phenyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-phenyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-phenyl-4-[succin-2-yl-(1-glycine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-phenyl-4-[succin-2-yl-(1-valine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-phenyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
1-benzyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
1-benzyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-4-[succin-2-yl-(1-glycine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-4-[succin-2-yl-(1-valine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
1-isopropyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
1-isopropyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-4-[succin-2-yl-(1-glycine)]-7-[4-(2-amino-6-methylpyrimidine-4-yl)]-4-oxa-1-butyne]-3,4-dihydro-1H-1,4-benzodiazepine-2,5-dione trifluoracetate,
(±)-1-isoproryl-4-[succin-2-yl-(1-valine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
1-methyl-4-(2-carboxy-1-methylethyl)-7-[5-(2-aminopyridine-3-yl)-5-oxa-1-pentyne]-3,4-dihydro-1H-1,4-benzodiazepine-2,5-dione trifluoracetate,
1-methyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-methyl-4-[succin-2-yl-(1-*t*-butoxy)]-7-[5-(2-aminopyridine-3-yl)-5-oxa-1-pentyne]-3,4-dihydro-1H-1,4-benzodiazepine-2,5-dione trifluoracetate,
(±)-1-methyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-methyl-4-[succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1-methyl-4-[succin-2-yl-(1-phenylalanine)]-7-[5-(2-aminopyridine-3-yl)-5-oxa-1-pentyne]-3,4-dihydro-1H-1,4-benzodiazepine-2,5-dione trifluoracetate,
1-phenyl-4-(2-carboxy-1-methylethyl)-7-[5-(2-aminopyridine-3-yl)-5-oxa-1-pentyne]-3,4-dihydro-1H-1,4-benzodiazepine-2,5-dione trifluoracetate,
1-phenyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-phenyl-4-[succin-2-yl-(1-*t*-butoxy)]-7-[5-(2-aminopyridine-3-yl)-5-oxa-1-pentyne]-3,4-dihydro-1H-1,4-benzodiazepine-2,5-dione trifluoracetate,
(±)-1-phenyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-phenyl-4-[succin-2-yl-(1-valine)]-7-[5-(2-aminopyridine-3-yl)-5-oxa-1-pentyne]-3,4-dihydro-1H-],4-benzodiazepine-2,5-dione trifluoracetate,
(±)-1-phenyl-4-[succin-2-yl-(1-phenylalanine)]-7-[5-(2-aminopyridine-3-yl)-5-oxa-1-pentynel-3,4-dihydro-1H-1,4-benzodiazepine-2,5-dione trifluoracetate,
1-benzyl-4-(2-carboxy-1-methylethyl)-7-[5-(2-aminopyridine-3-yl)-5-oxa-1-pentyne]-3,4-dihydro-1H-1,4-benzodiazepine-2,5-dione,
1-benzyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-benzyl-4-[succin-2-yl-(1-*t*-butoxy)]-7-[5-(2-aminopyridine-3-yl)-5-oxa-1-pentyne]-3,4-dihydro-1H-1,4-benzodiazepine-2,5-dione trifluoracetate,
(±)-1-benzyl-4-(succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-benzyl-4-(succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1-benzyl-4-[succin-2-yl-(1-phenylalanine)]-7-[5-(2-aminopyridine-3-yl)-5-oxa-1-pentyne]-3,4-dihydro-1H-1,4-benzodiazepine-2,5-dione trifluoracetate,
1-isopropyl-4-(2-carboxy-1-methylethy)-7-[5-(2-aminopyridine-3-yl)-5-oxa-1-pentyne]-3,4-dihydro-1H-1,4-benzodiazepine-2,5-dione trifluoracetate,
1-isopropyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-isopropyl-4-[succin-2-yl-(1-*t*-butoxy)]-7-[5-(2-aminopyridine-3-yl)-5-oxa-1-pentyne]-3,4-dihydro-1H-1,4--benzodiazepine-2,5-dione trifluoracetate,
(±)-1-isopropyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-isopropyl-4-[succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1-isopropyl-4-[succin-2-yl-(1-phenylalanine)]-7-[5-(2-aminopyridine-3-yl)-5-oxa-1-pentyne]-3,4-dihydro-1H-1,4-benzodiazepine-2,5-dione trifluoracetate,
(±)-1-methyl-3-phenyl-4-(2-carboxy-1-methylethyl)-7-(5-amino-1-pentyne)-3,4-dihydro-1H-1,4-benzodiazepine-2,5-dione trifluoracetate,
(±)-1-methyl-3-phenyl-4-(2-carboxy-1-phenylethyl)-7-(5-amino-1-pentyne)-3,4-dihydro-1H-1,4-benzodiazepine-2,5-dione trifluoracetate,
(±)-1-methyl-3-phenyl-4-[succin-2-yl-(1-*t*-butoxy)]-7-(5-amino-1-pentyne)-3,4-dihydro-1H-1,4-benzodiazepine-2,5-dione trifluoracetate,
(±)-1-methyl-3-phenyl-4-[succin-2-yl-(1-glycine)]-7-(5-amino-1-pentyne)-3,4-dihydro-1H-1,4-benzodiazepine-2,5-dione trifluoracetate,
(±)-1-methyl-3-phenyl-4-[succin-2-yl-(1-valine)]-7-(5-amino-1-pentyne)-3,4-dihydro-1H-1,4-benzodiazepine-2,5-dione trifluoracetate,
(±)-1-methyl-3-phenyl-4-[succin-2-yl-(1-phenylalanine)]-7-(5-amino-1-pentyne)-3,4-dihydro-1H-1,4-benzodiazepine-2,5-dione trifluoracetate,
(±)-1,3-diphenyl-4-(2-carboxy-1-methylethyl)-7-(5-amino-1-pentyne)-3,4-dihydro-1H-1,4-benzodiazepine-2,5-dione trifluoracetate,
(±)-1,3-diphenyl-4-(2-carboxy-1-phenylethyl)-7-(5-amino-1-pentyne)-3,4-dihydro-1H-1,4-benzodiazepine-2,5-dione trifluoracetate,
(±)-1,3-diphenyl-4-[succin-2-yl-(1-*t*-butoxy)]-7-(5-amino-1-pentyne)-3,4-dihydro-1H-1,4-benzodiazepine-2,5-dione trifluoracetate,
(±)-1,3-diphenyl-4-[succin-2-yl-(1-glycine)]-7-(5-amino-1-pentyne)-3,4-dihydro-1H-1,4-benzodiazepine-2,5-dione trifluoracetate,
(±)-1,3-diphenyl-4-[succin-2-yl-(1-valine)]-7-(5-amino-1-pentyne)-3,4-dihydro-1H-1,4-benzodiazepine-2,5-dione trifluoracetate,
(±)-1,3-diphenyl-4-[succin-2-yl-(1-phenylalanine)]-7-(5-amino-1-pentyne)-3,4-dihydro-1H-1,4-benzodiazepine-2,5-dione trifluoracetate,
(±)-1-benzyl-3-phenyl-4-(2-carboxy-1-methylethyl)-7-(5-amino-1-pentyne)-3,4-dihydro-1H-1,4-benzodiazepine-2,5-dione trifluoracetate,
(±)-1-benzyl-3-phenyl-4-(2-carboxy-1-phenylethyl)-7-(5-amino-1-pentyne)-3,4-dihydro-1H-1,4-benzodiazepine-2,5-dione trifluoracetate,
(±)-1-benzyl-3-phenyl-4-[succin-2-yl-(1-*t*-butoxy)]-7-(5-amino-1-pentyne)-3,4-dihydro-1H-1,4-benzodiazepine-2,5-dione trifluoracetate,
(±)-1-benzyl-3-phenyl-4-[succin-2-yl-(1-glycine)]-7-(5-amino-1-pentyne)-3,4-dihydro-1H-1,4-benzodiazepine-2,5-dione trifluoracetate,
(±)-1-benzyl-3-phenyl-4-[succin-2-yl-(1-valine)]-7-(5-amino-1-pentyne)-3,4-dihydro-1H-1,4-benzodiazepine-2,5-dione trifluoracetate,
(±)-1-benzyl-3-phenyl-4-[succin-2-yl-(1-phenylalanine)]-7-(5-amino-1-pentyne)-3,4-dihydro-1H-1,4-benzodiazepine-2,5-dione trifluoracetate,
(±)-1-isopropyl-3-phenyl-4-(2-carboxy-1-methylethyl)-7-(5-amino-1-pentyne)-3,4-dihydro-1H-1,4-benzodiazepine-2,5-dione trifluoracetate,
(±)-1-isopropyl-3-phenyl-4-(2-carboxy-1-phenylethyl)-7-(5-amino-1-pentyne)-3,4-dihydro-1H-1,4-benzodiazepine-2,5-dione trifluoracetate,
(±)-1-isopropyl-3-phenyl-4-[succin-2-yl-(1-*t*-butoxy)]-7-(5-amino-1-pentyne)-3,4-dihydro-1H-1,4-benzodiazepine-2,5-dione trifluoracetate,
(±)-1-isopropyl-3-phenyl-4-[succin-2-yl-(1-glycine)]-7-(5-amino-1-pentyne)-3,4-dihydro-1H-1,4-benzodiazepine-2,5-dione trifluoracetate,
(±)-1-isopropyl-3-phenyl-4-[succin-2-yl-(1-valine)]-7-(5-amino-1-pentyne)-3,4-dihydro-1H-1,4-benzodiazepine-2,5-dione trifluoracetate,
(±)-1-isopropyl-3-phenyl-4-[succin-2-yl-(1-phenylalanine)]-7-(5-amino-1-pentyne)-3,4-dihydro-1lH-1,4-benzodiazepine-2,5-dione trifluoracetate,
(±)-1-methyl-3-phenyl-4-(2-carboxy-1-methylethyl)-7-(N-(2-aminoethane)-3-keto-1-propyn-3-amine]-3,4-dihydro-1H-1,4-benzodiazepine-2,5-dione trifluoracetate,
(±)-1-methyl-3-phenyl-1-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
(±)-1,3-diphenyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
(±)-1,3-diphenyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1,3-diphenyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1,3-diphenyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1,3-diphenyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1,3-diphenyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
(±)-1-benzyl-3-phenyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
(±)-1-benzyl-3-phenyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
(±)-1-isopropyl-3-phenyl-4-(2-carboxy-1-methylethyl)-7-[N(2-aminoethane)-3-keto-1-propyn-3-amine]-3,4-dihydro-1H-1,4-benzodiazepine-2,5-dione trifluoracetate,
(±)-1-isopropyl-3-phenyl-4-(2-carhoxy-1-phenylethyl)-7-[N-(2-aminoethane)-3-keto-1-propyn-3-amine]-3,4-dihydro-1H-1,4-benzodiazerine-2,5-dione trifluoracetate,
(±)-1-isopropyl-3-phenyl-4-[succin-2-yl-(1-*t*-butoxy)]-7-[N-(2-aminoethane)-3-keto-1-propyn-3-amine]-3,4-dihydro-lH-1,4-benzodiazepine-2,5-dione trifluoracetate,
(±)-1-isopropyl-3-phenyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-isopropyl-3-phenyl-4-[succin-2-yl-(1-valine)]-7-[N-(2-aminoethane)-3-keto-1-propyn-3-amine]-3,4-dihytdro-1H-1,4-benzodiazepine-2,5-dione trifluoracetate,
(±)-1-isopropyl-3-phenyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
(±)-1-methyl-3-phenyl-4-(2-carboxy-1-methylethyl)-7-[3-keto-1-propyn-3-(1-piperizine)]-3,4-dihydro-1H-1,4-benzodiazepine-2,5-dione trifluoracetate,
(±)-1-methyl-3-phenyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-methyl-3-phenyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-methyl-3-phenyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-methyl-3-phenyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-methyl-3-phenyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
(±)-1,3-diphenyl-4-(2-carboxy-1-methylethyl)-7-[3-keto-1-propyn-3-(1-piperizine)]-3,4-dihydro-1H-1,4-benzodiazepine-2,5-dione trifluoracetate,
(±)-1,3-diphenyl-4-(2-carboxy-1-phenylethyl)-7-[3-keto-1-propyn-3-(1-piperizine)]-3,4-dihydro-1H-1,4-benzodiazepine-2,5-dione triflullracetate,
(±)-1,3-diphenyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1,3-diphenyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1,3-diphenyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1,3-diphenyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
(±)-1-benzyl-3-phenyl-4-(2-carboxy-1-methylethyl)-7-[3-keto-1-propyn-3-(1-piperizine)]-3,4-dihydro-1H-1,4-benzodiazepine-2,5-dione trifluoracetate,
(±)-1-benzyl-3-phenyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-benzyl-3-phenyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-benzyl-3-phenyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-benzyl-3-phenyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-benzyl-3-phenyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
(±)-1-isopropyl-3-phenyl-4-(2-carboxy-1-methylethyl)-7-[3-keto-1-propyn-3-(1-piperizine)]-3,4-dihydro-1H-1,4-benzodiazepine-2,5-dione trifluoracetate,
(±)-1-isopropyl-3-phenyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-isopropyl-3-phenyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-isopropyl-3-phenyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-isopropyl-3-phenyl-4-[succin-2-yl-(1-valine)-7-[3-keto-1-propyn-3-(1-piperizine)]-3,4-dihydro-1H-1,4-benzodiazepine-2,5-dione trifluoracetate,
(±)-1-isopropyl-3-phenyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
(±)-1-methyl-3-phenyl-4-(2-carboxy-1-methylethyl)-7-[2-(2-aminoethanethiol)-1-ethyne]-3,4-dihydro-1H-1,4-benzodiazepine-2,5-dione trifluoracetate,
(±)-1-methyl-3-phenyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1-methyl-3-phenyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-methyl-3-phenyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-methyl-3-phenyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-methyl-3-phenyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
(±)-1,3-diphenyl-4-(2-carboxy-1-methylethyl)-7-[2-(2-aminoethanethiol)-1-ethyne]-3,4-dihydro-1H-1,4-benzodiazepine-2,5-dione trifluoracetate,
(±)-1,3-diphenyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1,3-diphenyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1,3-diphenyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1,3-diphenyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1,3-diphenyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
(±)-1-benzyl-3-phenyl-4-(2-carboxy-1-methylethyl)-7-[2-(2-aminoethanethiol)-1-ethyne]-3,4-dihydro-1H-1,4-benzodiazepine-2,5-dione trifluoracetate,
(±)-1-benzyl-3-phenyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-benzyl-3-phenyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-benzyl-3-phenyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-benzyl-3-phenyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
(±)-1-isopropyl-3-phenyl-4-(2-carboxy-1-methylethyl)-7-[2-(2-aminoethanethiol)-1-ethyne]-3,4-dihydro-1H-1,4-benzodiazepine-2,5-dione trifluoracetate,
(±)-1-isopropyl-3-phenyl-4-(2-carboxy-1-phenylethyl)-7-[2-(2-aminoethanethiol)-1-ethyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(2-aminoethanethiol)-1-ethyne]-3,4-dihydro-1H-1,4-benzodiazepine-2,5-dione trifluoracetate,
(±)-1-isopropyl-3-phenyl-4-[succin-2-yl-(1-glycine)]-7-[2-(2-aminoethanethiol)-1-ethyne]-3,4-dihydro-1H-1,4-benzodiazepine-2,5-dione trifluoracetate,
(±)-1-isopropyl-3-phenyl-4-[succin-2-yl-(1-valine)]-7-[2-(2-aminoethanethiol)-1-ethyne]-3,4-dihydro-1H-1,4-benzodiazepine-2,5-dione trifluoracetate,
(±)-1-isopropyl-3-phenyl-4-[succin-2-yl-(1-phenylalanine)]-7-[2-(2-aminoethanethiol)-1-ethyne]-3,4-dihydro-1H-1,4-benzodiazepine-2,5-dione trifluoracetate,
(±)-1-methyl-3-phenyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-methyl-3-phenyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-methyl-3-phenyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-methyl-3-phenyl-4-[succin-2-yl-(1-glycine)]-7-[4-(2-amino-6-methylpyrimidine-4-yl)-4-oxa-1-butyne] -3,4-dihydro-1H-1,4-benzodiazepine-2,5-dione trifluoracetate,
(±)-1-methyl-3-phenyl-4-[succin-2-yl-(1-valine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-methyl-3-phenyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
(±)-1,3-diphenyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1,3-diphenyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1,3-diphenyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1,3-diphenyl-4-[succin-2-yl-(1-glycine)]-7-[4-(2-amino-6-methylpyrimidine-4-yl)-4-oxa-1-butyne]-3,4-dihydro-1H-1,4-benzodiazepine-2,5-dione trifluoracetate,
(±)-1,3-diphenyl-4-[succin-2-yl-(1-valine)]-7-[4-(2-amino-6-methylpyrimidine-4-yl)-4-oxa-1-butyne]-3,4-dihydro-1H-1,4-benzodiazepine-2,5-dione trifluoracetate,
(±)-1,3-diphenyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-3-phenyl-4-(2-carboxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-3-phenyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-3-phenyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-3-phenyl-4-[succin-2-yl-(1-glycine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-3-phenyl-4-[succin-2-yl-(1-valine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-benzyl-3-phenyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-3-phenyl-4-(2-carhoxy-1-methylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-3-phenyl-4-(2-carboxy-1-phenylethyl)-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[4-(2-amino-6-methylpyrimidine-4-yl)-4-oxa-1-butyne]-3,4-dihydro-1H-1,4-benzodiazepine-2,5-dione trifluoracetate,
(±)-1-isopropyl-3-phenyl-4-[succin-2-yl-(1-glycine)]-7-[4-(2-amino-6-methylpyrimidine-4-yl)]-4-oxa-1-butyne]-3,4-dihydro-1H-1,4-benzodiazepine-2,5-dione trifluoracetate,
(±)-1-isopropyl-3-phenyl-4-[succin-2-yl-(1-valine)]-7-[4-(2-amino-6-methylpyrimidine-4-yl)4-oxa-1-butyne]-3,4-dihydro-1H-1,4-benzodiazepine-2,5-dione trifluoracetate,
(±)-1-isopropyl-3-phenyl-4-[succin-2-yl-(1-phenylalanine)]-7-[4-(2-amino-6-methylpyrimidine-4-yl)-4-oxa-1-butyne]-3,4-dihydro-1H-1,4-benzodiazepine-2,5-dione trifluoracetate,
(±)-1-methyl-3-phenyl-4-(2-carboxy-1-methylethyl)-7-[5-(2-aminopyridine-3-yl)-5-oxa-1-pentyne]-3,4-dihydro-1H-1,4-benzodiazepine-2,5-dione trifluoracetate,
(±)-1-methyl-3-phenyl-4-(2-carhoxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-methyl-3-phenyl-4-[succin-2-yl-(1-*t*-butoxy)]-7-[5-(2-aminopyridine-3-yl)-5-oxa-1-pentyne]-3,4-dihydro-1H-1,4-benzodiazepine-2,5-dione trifluoracetate,
(±)-1-methyl-3-phenyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-methyl-3-phenyl-4-[succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1-methyl-3-phenyl-4-[succin-2-yl-(1-phenylalanine)]-7-[5-(2-aminopyridine-3-yl)-5-oxa-1-pentyne]-3,4-dihydro-1H-1,4-benzodiazepine-2,5-dione trifluoracetate,
(±)-1,3-diphenyl-4-(2-carboxy-1-methylethyl)-7-[5-(2-aminopyridine-3-yl)-5-oxa-1-pentyne]-3,4-dihydro-1H-1,4-benzodiazepine-2,5-dione trifluoracetate,
(±)-1,3-diphenyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1,3-diphenyl-4-[succin-2-yl-(1-*t*-butoxy)]-7-[5-(2-aminopyridine-3-yl)-5-oxa-1-pentyne]-3,4-dihydro-1H-1,4-benzodiazepine-2,5-dione trifluoracetate,
(±)-1,3-diphenyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1,3-diphenyl-4-[succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1,3-diphenyl-4-[succin-2-yl-(1-phenylalanine)]-7-[5-(2-aminopyridine-3-yl)-5-oxa-1-pentyne]-3,4-dihydro-1H-1,4-benzodiazepine-2,5-dione trifluoracetate,
(±)-1-benzyl-3-phenyl-4-(2-carboxy-1-methylethyl)-7-[5-(2-aminopyridine-3-yl)-5-oxa-1-pentyne]-3,4-dihydro-1H-1,4-benzodiazepine-2,5-dione,
(±)-1-benzyl-3-phenyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-benzyl-3-phenyl-4-[succin-2-yl-(1-*t*-butoxy)]-7-[5-(2-aminopyridine-3-yl)-5-oxa-1-pentyne]-3,4-dihydro-1H-1,4-benzodiazepine-2,5-dione trifluoracetate,
(±)-1-benzyl-3-phenyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-benzyl-3-phenyl-4-[succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1-benzyl-3-phenyl-4-[succin-2-yl-(1-phenylalanine)]17-[5-(2-aminopyridine-3-yl)-5-oxa-1-pentyne]-3,4-dihydro-1H-1,4-benzodiazepine-2,5-dione trifluoracetate,
(±)-1-isopropyl-3-phenyl-4-(2-carboxy-1-methylethyl)-7-[5-(2-aminopyridine-3-yl)-5-oxa-1-pentyne]-3,4-dihydro-1H-1,4-benzodiazepine-2,5-dione trifluoracetate,
(±)-1-isopropyl-3-phenyl-4-(2-carboxy-1-phenylethyl)-7-[5-(2-aminopyridine-3-yl)-5-oxa-1-pentyne]-3,4-dihydro-1H-1,4-benzodiazepine-2,5-dione trifluoracetate,
(±)-1-isopropyl-3-phenyl-4-[sucdin-2-yl-(1*-t*-butoxy)]-7-[5-(2-aminopyridine-3-yl)-5-oxa-1-pentyne]-3,4-dihydro-1H-1,4-benzodiazepine-2,5-dione trifluoracetate,
(±)-1-isopropyl-3-phenyl-4-[succin-2-yl-(1-glycine)]-7-[5-(2-aminopyridine-3-yl)-5-oxa-1-pentyne]-3,4-dihydro-1H-1,4-benzodiazepine-2,5-dione trifluoracetate,
(±)-1-isopropyl-3-phenyl-4-[succin-2-yl-(1-valine)]-7-[5-(2-aminopyridine-3-yl)-5-oxa-1-pentyne]-3,4-dihydro-1H-1,4-benzodiazepine-2,5-dione trifluoracetate,
(±)-1-isopropyl-3-phenyl-4-[succin-2-yl-(1-phenylalanine)]-7-[5-(2-aminopyridine-3-yl)-5-oxa-1-pentyne]-3,4-dihydro-1H-1,4-benzodiazepine-2,5-dione trifluoracetate.

### Example 2

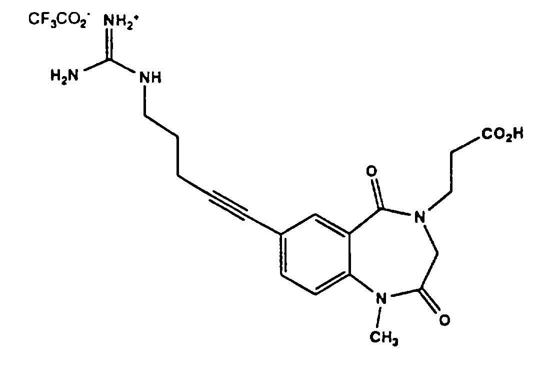

*1-methyl-4-(2-carboxyethyl)-7-(5-guanidino-1-pentynyl)-3,4-dihydro-1H-1,4-benzodiazepine-2,5-dione trifluoracetate.*

To a magnetically stirred solution of 6mgs of 1-methyl-4-(2-carboxyethyl)-7-(5-amino-1-pentynyl)-3,4-dihydro-1H-1,4-benzodiazepine-2,5-dione trifluoracetate (0.017mmol) in 1.0mL 10% potassium bicarbonate was added 31.0mgs of formamidine sulfonic acid (0.25 mmol). After 30 minutes, the reaction mixture was quenched with 0.5mL acetic acid and concentrated *in vacuo*. The resulting residue was diluted with 2mL water and purified by high pressure liquid chromatography, using a 1/2" C-18 reverse-phase column, eluting with a solvent gradient of 10:90 methanol(0.1% trifluoracetic acid)/ water (0.1% trifluoracetic acid), time 0 to 10 min, to 50:50 methanol(0.1% trifluoracetic acid)/water (0.1% trifluoracetic acid), time 10 min to 40 min, flow=10ml /min (Rₜ=36.8 min, un detection 254nm) to yield 4.5mgs (67%) 1-methyl-4-(2-carboxyethyl)-7-(5-guanidino-1-pentynyl)-3,4-dihydro-1*H*-1,4-benzodiazepine-2,5-dione trifluoracetate. HRMS (FAB) molecular ion m/z=386.1823 (cald. C₁₉H₂₄N₅O₄, 386.1829). ¹H NMR (D₂O) 7.60 (1H, d, ⁴J_{HH}, Ar-H *o*-CON), 7.51 (1H,dd, ⁴J_{HH}=2Hz, ³J_{HH}=8Hz, Ar-H *p*-CON), 7.20 (1H, d, ³J_{HH}=8Hz, Ar-H *m*-CON), 4.05 (1H, d, J_{HH}=14Hz, NCHHCO), 3.92 (1H, m, NCHHCH₂), 3.72 (1H, d, J_{HH}=14Hz, NCHHCO), 3.40 (1H, m, NCHHCH₂), 3.23-3,15 (5H, NCH_{3,} H₂NC(=NH₂)NHCH₂), 2.58 (2H, m, CH₂CO₂), 2.38 (2H, t, ³J_{HH}=7Hz, C∫CCH₂), 1.76 (2H, p, ³J_{HH}=7Hz, CH₂CH₂CH₂).

Using the above procedure, but substituting the appropriate amino acid for 1-methyl-4-(2-carboxyethyl)-7-(5-amino-1-pentynyl)-3,4-dihydro-1*H*-1,4-benzodiazepine-2,5-dione trifluoracetate there may be prepared, for example, the following compounds:
1-methyl-4-(2-carboxy-1-methylethyl)-7-(5-guanidino-1-pentyne)-3,4-dihydro-1H-1,4-benzodiazepine-2,5-dione trifluoracetate,
1-methyl-4-(2-carboxy-1-phenylethyl)-7-(5-guanidino-1-pentyne)-3,4-dihydro-1H-1,4-benzodiazepine-2,5-dione trifluoracetate,
(±)-1-methyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-methyl-4-[succin-2-yl-(1-glycine)]-7-(5-guanidino-1-pentyne)-3,4-dihydro-1H-1,4-benzodiazepine-2,5-dione trifluoracetate,
(±)-1-methyl-4-[succin-2-yl-(1-valine)]-7-(5-guanidino-1-pentyne)-3,4-dihydro-1H-1,4-benzodiazepine-2,5-dione trifluoracetate,
(±)-1-methyl-4-[succin-2-yl-(1-phenylalanine)]-7-(5-guanidino-1-pentyne)-3,4-dihydro-1H-1,4-benzodiazepine-2,5-dione trifluoracetate,
1-phenyl-4-(2-carboxy-1-methylethyl)-7-(5-guanidino-1-pentyne)-3,4-dihydro-1H-1,4-benzodiazepine-2,5-dione trifluoracetate,
1-phenyl-4-(2-carboxy-1-phenylethyl)-7-(5-guanidino-1-pentyne)-3,4-dihydro-1H-1,4-benzodiazepine-2,5-dione trifluoracetate,
(±)-1-phenyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-phenyl-4-[succin-2-yl-(1-glycine)]-7-(5-guanidino-1-pentyne)-3,4-dihydro-1H-1,4-benzodiazepine-2,5-dione trifluoracetate,
(±)-1-phenyl-4-[succin-2-yl-[1-valine)]-7-(5-guanidino-1-pentyne)-3,4-dihydro-1H-1,4-benzodiazepine-2,5-dione trifluoracetate,
(±)-1-phenyl-4-[succin-2-yl-(1-phenylalanine)]-7-(5-guanidino-1-pentyne)-3,4-dihydro-1H-1,4-benzodiazepine-2,5-dione trifluoracetate,
1-benzyl-4-(2-carboxy-1-methylethyl)-7-(5-guanidino-1-pentyne)-3,4-dihydro-1H-1,4-benzodiazepine-2,5-dione trifluoracetate,
1-benzyl-4-(2-carboxy-1-phenylethyl)-7-(5-guanidino-1-pentyne)-3,4-dihydro-1H-1,4-benzodiazepine-2,5-dione trifluoracetate,
(±)-1-benzyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-benzyl-4-[succin-2-yl-(1-glycine)]-7-(5-guanidino-1-pentyne)-3,4-dihydro-1H-1,4-benzodiazepine-2,5-dione trifluoracetate,
(±)-1-benzyl-4-[succin-2-yl-(1-valine)]-7-(5-guanidino-1-pentyne)-3,4-dihydro-1H-1,4-benzodiazepine-2,5-dione trifluoracetate,
(±)-1-benzyl-4-[succin-2-yl-(1-phenylalanine)]-7-(5-guanidino-1-pentyne)-3,4-dihydro-1H-1,4-benzodiazepine-2,5-dione trifluoracetate,
1-isopropyl-4-(2-carboxy-1-methylethyl)-7-(5-guanidino-1-pentyne)-3,4-dihydro-1H-1,4-benzodiazepine-2,5-dione trifluoracetate,
1-isopropyl-4-(2-carboxy-1-phenylethyl)-7-(5-guanidino-1-pentyne)-3,4-dihydro-1H-1,4-benzodiazepine-2,5-dione trifluoracetate,
(±)-1-isopropyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-isopropyl-4-[succin-2-yl-(1-glycine)]-7-(5-guanidino-1-pentyne)-3,4-dihydro-1H-1,4-benzodiazepine-2,5-dione trifluoracetate,
(±)-1-isopropyl-4-[succin-2-yl-(1-valine)]-7-(5-guanidino-1-pentyne)-3,4-dihydro-1H-1,4-benzodiazepine-2,5-dione trifluoracetate,
(±)-1-isopropyl-4-[succin-2-yl-(1-phenylalanine)]-7-(5-guanidino-1-pentyne)-3,4-dihydro-1H-1,4-benzodiazepine-2,5-dione trifluoracetate,
1-methyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
1-methyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1-methyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1-methyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1-methyl-4-[succin-2-yl-(1-valine)]-7-[N-(2-guanidinoethane)-3-keto-1-propyn-3-amine]-3,4-dihydro-1H-1,4-benzodiazepine-2,5-dione trifluoracetate,
(±)-1-methyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
1-phenyl-4-(2-carhoxy-1-methylethyl)-7-[N-(2-guanidinoethane)-3-keto-1-propyn-3-amine]-3,4-dihydro-1H-1,4-benzodiazepine-2,5-dione trifluoracetate,
1-phenyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1-phenyl-4-[succin-2-yl-(1-*t*-butoxy)]-7-[N-(2-guanidinoethane)-3-ket-1-propyn-3-amine]-3,4-dihydro-1H-1,4-benzodiazepine-2,5-dione trifluoracetate,
(±)-1-phenyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1-phenyl-4-[succin-2-yl-(1-valine)]-7-[N-(2-guanidinoethane)-3-keto-1-propyn-3-amine]-3,4-dihydro-1H-1,4-benzodiazepine-2,5-dione trifluoracetate,
(±)-1-phenyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
1-benzyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
1-benzyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1-benzyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1-benzyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1-benzyl-4-[succin-2-yl-(1-valine)]-7[N-(2-guanidinoethane)-3-keto-1-propyn-3-amine]-3,4-dihydro-1H-1,4-benzodiazepine-2,5-dione trifluoracetate,
(±)-1-benzyl-4-[succin-2-yl-(1-phenylalanine)]-7-[N-(2-guanidinoethane)-3-keto-propyn-3-amine]-3,4-dihydro-1H-1,4-benzodiazepine-2,5-dione trifluoracetate,
1-isopropyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
1-isopropyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1-isopropyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1-isopropyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1-isopropyl-4-[succin-2-yl-(1-valine)]-7-[N-(2-guanidinoethane)-3-keto-1-propyn-3-amine]-3,4-dihydro-1H-1,4-benzodiazepine-2,5-dione trifluoracetate,
(±)-1-isopropyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
1-methyl-4-(2-carboxy-1-methylethyl)-7-[3-keto-1-propyn-3-(4-amidino-1-piperizine)]-3,4-dihydro-1H-1,4-benzodiazepine-2,5-dione trifluoracetate,
1-methyl-4-(2-carboxy-1-phenylethyl)-7-[3-keto-1-propyn-3-(4-amidino-1-piperizine)]-3,4-dihydro-1H-1,4-benzodiazepine-2,5-dione trifluoracetate,
(±)-1-methyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1-methyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-methyl-4-[succin-2-yl-(1-valine)]-7-[3-keto-1-propyn-3-(4-amidino-1-piperizine)]-3,4-dihydro-1H-1,4-benzodiazepine-2,5-dione trifluoracetate,
(±)-1-methyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
1-phenyl-4-(2-carboxy-1-methylethyl)-7-[3-keto-1-propyn-3-(4-amidino-1-piperizine)]-3,4-dihydro-1H-1,4-benzodiazepine-2,5-dione trifluoracetate,
1-phenyl-4-(2-carboxy-1-phenylethyl)-7-[3-keto-1-propyn-3-(4-amidino-1-piperizine)]-3,4-dihydro-1H-1,4-benzodiazepine-2,5-dione trifluoracetate,
(±)-1-phenyl-4-[succin-2-yl-(1-*t*-butoxy)]-7-[3-keto-]-propyn-3-(4-amidino-1-piperizine)]-3,4-dihydro-1H-1,4-benzodiazepine-2,5-dione trifluoracetate,
(±)-1-phenyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-phenyl-4-[succin-2-yl-(1-valine)]-7-[3-keto-1-propyn-3-(4-amidino-1-piperizine)]-3,4-dihydro-1H-1,4-benzodiazepine-2,5-dione trifluoracetate,
(±)-1-phenyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
1-benzyl-4-(2-carboxy-1-methylethyl)-7-[3-keto-1-propyn-3-(4-amidino-1-piperizine)]-3,4-dihydro-1H-1,4-benzodiazepine-2,5-dione trifluoracetate,
1-benzyl-4-(2-carboxy-1-phenylethyl)-7-[3-keto-1-propyn-3-(4-amidino-1-piperizine)]-3,4-dihydro-1lH-1,4-benzodiazepine-2,5-dione trifluoracetate,
(±)-1-benzyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1-benzyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-benzyl-4-[succin-2-yl-(1-valine)]-7-[3-keto-1-propyn-3-(4-amidino-1-piperizine)]-3,4-dihydro-.1H-1,4-benzodiazepine-2,5-dione trifluoracetate,
(±)-1-benzyl-4-[succin-2-yl-(1-phenylalanine)]-7-(3-keto-1-propyn-3-(4-amidino-1-piperizine)]-3,4-dihydro-1H-1,4-benzodiazepine-2,5-dione trifluoracetate,
1-isopropyl-4-(2-carboxy-1-methylethyl)-7-[3-keto-1-propyn-3-(4-amidino-1-piperizine)]-3,4-dihydro-1H-1,4-benzodiazepine-2,5-dione trifluoracetate,
1-isopropyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-isopropyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1-isopropyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-isopropyl-4-[succin-2-yl-(1-valine)]-7-[3-keto-1-propyn-3-(4-amidino-1-pirerizine)]-3,4-dihydro-1H-1,4-benzodiazepine-2,5-dione trifluoraretate,
(±)-1-isopropyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
1-methyl-4-(2-carboxy-1-methylethyl)-7-[2-(2-guanidinoethanethiol)-1-ethyne]-3,4-dihydro-1H-1,4-benzodiazepine-2,5-dione trifluoracetate,
1-methyl-4-(2-carboxy-1-phenyletllyl)-7-[2-(2-guanidinoethanethiol)-1-ethyne]-3,4-dihydro-1H-1,4-benzodiazepine-2,5-dione trifluoracetate,
(±)-1-methyl-4-[succin-2-yl-(1-*t*-butoxy)]-7-[2-(7-guanidinoethanethiol)-1-ethyne]-3,4-dihydro-1H-1,4-benzodiazepine-2,5-dione trifluoracetate,
(±)-1-methyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-methyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-methyl-4-[succin-2-yl-(1-phenylalanine)]-7-[2-(2-guanidinoethanethiol)-1-ethyne]-3,4-dihydro-1H-1,4-benzodiazepine-2,5-dione trifluoracetate,
1-phenyl-4-(2-carboxy-1-methylethyl)-7-[2-(2-guanidinoethanethiol)-1-ethyne]-3,4-dihydro-1H-1,4-benzodiazepine-2,5-dione trifluoracetate,
1-phenyl-4-(2-carboxy-1-phenylethyl)-7-[2-(2-guanidinoethanethiol)-1-ethyne]-3,4-dihydro-1H-1,4-benzodiazepine-2,5-dione trifluoracetate,
(±)-1-phenyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1-phenyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-phenyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-phenyl-4-[succin-2-yl-(1-phenylalanine)]-7-(2-(2-guanidinoethanethiol)-1-ethyne]-3,4-dihydro-1H-1,4-benzodiazepine-2,5-dione trifluoracetate,
1-benzyl-4-(2-carboxy-1-methylethyl)-7-[-(2-guanidinoethanethiol)-1-ethyne]-3,4-dihydro-1H-1,4-benzodiazepine-2,5-dione trifluoracetate,
1-benzyl-4-(2-carboxy-1-phenylethyl)-7-[2-(2-guanidinoethanethiol)-1-ethyne]-3,4-dihydro-1H-1,4-benzodiazepine-2,5-dione trifluoracetate,
(±)-1-benzyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1-benzyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-benzyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-benzyl-4-[succin-2-yl-(1-phenylalanine)]-7-[2-(2-guanidinoethanethiol)-1-ethyne]-3,4-dihydro-1H-1,4-benziodiazepine-2,5-dione trifluoracetate,
1-isopropyl-4-(2-carboxy-1-methylethyl)-7-[2-(2-guanidinoethanethiol)-1-ethyne]-3,4-dihydro-1H-1,4-benzodiazepine-2,5-dione trifluoracetate,
1-isopropyl-4-(2-carboxy-1-phenylelhyl)-7-[2-(2-guanidinoethanethiol)-1-ethyne]-3,4-dihydro-1H-1,4-benzodiazepine-2,5-dione trifluoracetate,
(±)-1-isopropyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1-isopropyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-isopropyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-isopropyl-4-[succin-2-yl-(1-phenylalanine)]-7-[2-(2-guanidinoethanethiol)-1-ethyne]-3,4-dihydro-1H-1,4-benzodiazepine-2,5-dione trifluoracetate,
(±)-1-methyl-3-phenyl-4-(2-carboxy-1-methylethyl)-7-(5-guanidino-1-pentyne)-3,4-dihydro-1H-1,4-benzodiazepine-2,5-dione trifluoracetate,
(±)-1-methyl-3-phenyl-4-(2-carboxy-1-phenylethyl)-7-(5-guanidino-1-pentyne)-3,4-dihydro-1H-1,4-benzodiazepine-2,5-dione trifluoracetate,
(±)-1-methyl-3-phenyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-methyl-3-phenyl-4-[succin-2-yl-(1-glycine)]-7-(5-guanidino-1-pentyne)-3,4-dihydro-1H-1,4-benzodiazepine-2,5-dione trifluoracetate,
(±)-1-methyl-3-phenyl-4-[succin-2-yl-(1-valine)]-7-(5-guanidino-1-pentyne)-3,4-dihydro-1H-1,4-benzodiazepine-2,5-dione trifluoracetate,
(±)-1-methyl-3-phenyl-4-[succin-2-yl-(1-phenylalanine)]-7-(5-guanidino-1-pentyne)-3,4-dihydro-1H-1,4-benzodiazepine-2,5-dione trifluoracetate,
(±)-1,3-diphenyl-4-(2-carboxy-1-methylethyl)-7-(5-guanidino-1-pentyne)-3,4-dihydro-1H-1,4-benzodiazepine-2,5-dione trifluoracetate,
(±)-1,3-diphenyl-4-(2-carboxy-1-phenylethyl)-7-(5-guanidino-1-pentyne)-3,4-dihydro-1H-1,4-benzodiazepine-2,5-dione trifluoracetate,
(±)-1,3-diphenyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1,3-diphenyl-4-[succin-2-yl-(1-glycine)]-7-(5-guanidino-1-pentyne)-3,4-dihydro-1H-1,4-benzodiazepine-2,5-dione trifluoracetate,
(±)-1,3-diphenyl-4-[succin-2-yl-(1-valine)]-7-(5-guanidino-1-pentyne)-3,4-dihydro-1H-1,4-benzodiazepine-2,5-dione trifluoracetate,
(±)-1,3-diphenyl-4-[succin-2-yl-(1-phenylalanine)]-7-(5-guanidino-1-pentyne)-3,4-dihydro-1H-1,4-benzodiazepine-2,5-dione trifluoracetate,
(±)-1-benzyl-3-phenyl-4-(2-carboxy-1-methylethyl)-7-(5-guanidino-1-pentyne)-3,4-dihydro-1H-1,4-benzodiazepine-2,5-dione trifluoracetate,
(±)-1-benzyl-3-phenyl-4-(2-carboxy-1-phenylethyl)-7-(5-guanidino-1-pentyne)3,4-dihydro-1H-1,4-benzodiazepine-2,5-dione trifluoracetate,
(±)-1-benzyl-3-phenyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-benzyl-3-phenyl-4-[succin-2-yl-(1-glycine)]-7-(5-guanidino-1-pentyne)-3,4-dihydro-1H-1,4-benzodiazepine-2,5-dione trifluoracetate,
(±)-1-benzyl-3-phenyl-4-[succin-2-yl-(1-valine)]-7-(5-guanidino-1-pentyne)-3,4-dihydro-1H-1,4-benzodiazepine-2,5-dione trifluoracetate,
(±)-1-benzyl-3-phenyl-4-[succin-2-yl-(1-phenylalanine)]-7-(5-guanidino-1-pentyne)-3,4-dihydro-1H-1,4-benzodiazepine-2,5-dione trifluoracetate,
(±)-1-isopropyl-3-phenyl-4-(2-carboxy-1-methylethyl)-7-(5-guanidino-1-pentyne)-3,4-dihydro-1H-1,4-benzodiazepine-2,5-dione trifluoracetate,
(±)-1-isopropyl-3-phenyl-4-(2-carboxy-1-phenylethyl)-7-(5-guanidino-1-pentyne)-3,4-dihydro-1H-1,4-benzodiazepine-2,5-dione trifluoracetate,
(±)-1-isoproyryl-3-phenyl-4-[succin-2-yl-(1-*t*-butoxy)]-7-(5-guanidino-1-pentyne)-3,4-dihydro-1H-1,4-benzodiazepine-2,5-dione trifluoracetate,
(±)-1-isopropyl-3-phenyl-4-[succin-2-yl-(1-glycine)]-7-(5-guanidino-1-penlyne)-3,4-dihydro-1H-1,4-benzodiazepine-2,5-dione trifluoracetate,
(±)-1-isopropyl-3-phenyl-4-[succin-2-yl-(1-valine)]-7-(5-guanidino-1-pentyne)-3,4-dihydro-1H-1,4-benzodiazepine-2,5-dione trifluoracetate,
(±)-1-isopropyl-3-phenyl-4-[succin-2-yl-(1-phenylalanine)]-7-(5-guanidino-1-pentyne)-3,4-dihydro-1H-1,4-benzodiazepine-2,5-dione trifluoracetate,
(±)-1-methyl-3-phenyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
(±)-1-methyl-3-phenyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-valine)]-7-[N-(2-guanidinoethane)-3-keto-1-propyn-3-amine]-3,4-dihydro-1H-1,4-benzodiazepine-2,5-dione trifluoracetate,
(±)-1-methyl-3-phenyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
(±)-1,3-diphenyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
(±)-1,3-diphenyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1,3-diphenyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1,3-diphenyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1,3-diphenyl-4-[succin-2-yl-(1-valine)]-7-[N-(2-guanidinoethane)-3-keto-1-propyn-3-amine]-3,4-dihydro-1H-1,4-benzodiazepine-2,5-dione trifluoracetate,
(±)-1,3-diphenyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
(±)-1-benzyl-3-phenyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
(±)-1-benzyl-3-phenyl-4-(2-carboxy-1-phenylethyl)-7-(N-(2-guanidinoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-glycine)]-7-[N-(2-guanidinoethane)-3-keto-1-propyn-3-amine]-3,4-dihydro-1H-1,4-benzodiazepine-2,5-dione trifluoracetate,
(±)-1-benzyl-3-phenyl-4-[succin-2-yl-(1-valine)]-7-[N-(2-guanidinoethane)-3-keto-1-propyn-3-amine]-3,4-dihydro-1,4-benzodiazepine-2,5-dione trifluoracetate,
(±)-1-benzyl-3-phenyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
(±)-1-isopropyl-3-phenyl-4-(2-carboxy-1-methylethyl)-7-[N-(2-guanidinoethane)-3-keto-1-propyn-3-amine]-3,4-dihydro-1H-1,4-benzodiazepine-2,5-dione trifluoracetate,
(±)-1-isopropyl-3-phenyl-4-(2-carboxy-1-phenylethyl)-7-[N-(2-guanidinoethane)-3-keto-1-propyn-3-amine]-3,4-dihydro-1H-1,4-benzodiazepine-2,5-dione trifluoracetate,
(±)-1-isopropyl-3-phenyl-4-[succin-2-yl-(1-*t*-butoxy)]-7-[N-(2-guanidinoethane)-3-keto-1-propyn-3-amine]-3,4-dihydro-1H-1,4-benzodiazepine-2,5-dione trifluoracetate,
(±)-1-isopropyl-3-phenyl-4-[succin-2-yl-(1-glycine)]-7-[N-(2-guanidinoethane)-3-keto-]-propyn-3-amine]-3,4-dihydro-1H-1,4-benzodiazepine-2,5-dione trifluoracetate,
(±)-1-isopropyl-3-phenyl-4-[succin-2-yl-(1-valine)]-7-[N-(2-guanidinoethane)-3-keto-1-propyn-3-amine]-3,4-dihydro-1H-1,4-benzodiazepine-2,5-dione trifluoracetate,
(±)-1-isopropyl-3-phenyl-4-[succin-2-yl-(1-phenylalanine)]-7-[N-(2-guanidinoethane)-3-keto-1-propyn-3-amine]-3,4-dihydro-1H-1,4-benzodiazepine-2,5-dione trifluoracetate,
(±)-1-methyl-3-phenyl-4-(2-carboxy-1-methylethyl)-7-[3-keto-1-propyn-3-(4-amidino-1-piperizine)]-3,4-dihydro-1H-1,4-benzosiazepine-2,5-dione trifluoracetate,
(±)-1-methyl-3-phenyl-4-(2-carboxy-1-phenylethyl)-7-[3-keto-1-propyn-3-(4-amidino-1-piperizine)]-3,4-dihydro-1H-1,4-benzodiazepine-2,5-dione trifluoracetate,
(±)-1-methyl-3-phenyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1-methyl-3-phenyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-methyl-3-phenyl-4-[succin-2-yl-(1-valine)]-7-[3-keto-1-propyn-3-(4-amidino-1-piperizine)]-3,4-dihydro-1H-1,4-benzodiazepine-2,5-dione trifluoracetate,
(±)-1-methyl-3-phenyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
(±)-1,3-diphenyl-4-(2-carboxy-1-methylethyl)-7-[3-keto-1-propyn-3-(4-amidino-1-piperizine)]-3,4-dihydro-1H-1,4-benzodiazepine-2,5-dione trifluoracetate,
(±)-1,3-diphenyl-4-(2-carboxy-1-phenylethyl)-7-[3-keto-1-propyn-3-(4-amidino-1-piperizine)]-3,4-dihydro-1H-1,4-benzodiazepine-2,5-dione trifluoracetate,
(±)-1,3-diphenyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1,3-diphenyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1,3-diphenyl-1-[succin-2-yl-(1-valine)]-7-[3-keto-1-propyn-3-(4-amidino-1-piperizine)]-3,4-dihydro-1H-1,4-benzodiazepine-2,5-dione trifluoracetate,
(±)-1,3-diphenyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
(±)-1-benzyl-3-phenyl-4-(2-carboxy-1-melhylethyl)-7-[3-keto-1-propyn-3-(4-amidino-1-piperizine)]-3,4-dihydro-1H-1,4-benzodiazepine-2,5-dione trifluoracetate,
(±)-1-benzyl-3-phenyl-4-(2-carboxy-1-phenylethyl)-7-[3-keto-1-propyn-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-valine)]-7-[3-keto-1-propyn-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
(±)-1-isopropyl-3-phenyl-4-(2-carboxy-1-methylethyl)-7-[3-keto-1-propyn-3-(4-amidino-1-piperizine)]-3,4-dihydro-1H-1,4-benzodiazepine-2,5-dione trifluoracetate,
(±)-1-isopropyl-3-phenyl-4-(2-carboxy-1-phenylethyl)-7-[3-keto-1-propyn-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-*t*-butoxy)]-7-[3-keto-1-propyn-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-glycine)]-7-[3-keto-1-propyn-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-valine)]-7-[3-keto-1-propyn-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-phenylalanine)]-7-[3-keto-1-propyn-3-(4-amidino-1-piperizine)]-3,4-dihydro-1H-1,4-benzodiazepine-2,5-dione trifluoracetate,
(±)-1-methyl-3-phenyI-4-(2-carboxy-1-methylethyl)-7-[2-(2-guanidinoethanethiol)-1-ethyne]-3,4-dihydro-1H-1,4-benzodiazepine-2,5-dione trifluoracetate,
(±)-1-methyl-3-phenyl-4-(2-carboxy-1-phenylethyl)-7-[2-(2-guanidinoethanethiol)-1-ethyne]-3,4-dihydro-1H-1,4-benzadiazepine-2,5-dione trifluoracetate,
(±)-1-methyl-3-phenyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1-methyl-3-phenyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-methyl-3-phenyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-methyl-3-phenyl-4-[succin-2-yl-(1-phenylalanine)]-7-[2-(2-guanidinoethanethiol)-1-ethyne]-3,4-dihydro-1H-1,4-benzodiazepine-2,5-dione trifluoracetate,
(±)-1,3-diphenyl-4-(2-carboxy-1-methylethyl)-7-[2-(2-guanidinoethanethiol)-1-ethyne]-3,4-dihydro-1H-1,4-benzodiazepine-2,5-dione trifluoracetate,
(±)-1,3-diphenyl-4-(2-carboxy-1-phenylethyl)-7-[2-(2-guanidinoethanethiol)-1-ethyne]-3,4-dihydro-1H-1,4-benzodiazepine-2,5-dione trifluoracetate,
(±)-1,3-diphenyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1,3-diphenyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1,3-diphenyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1,3-diphenyl-4-[succin-2-yl-(1-phenylalanine)]-7-[2-(2-guanidinoethanethiol)-1-ethyne]-3,4-dihydro-1H-1,4-benzodiazepine-2,5-dione trifluoracetate,
(±)-1-benzyl-3-phenyl-4-(2-carboxy-1-methylethyl)-7-[2-(2-guanidinoethanethiol)-1-ethyne]-3,4-dihydro-1H-1,4-benzodiazepine-2,5-dione trifluoracetate,
(±)-1-benzyl-3-phenyl-4-(2-carboxy-1-phenylethyl)-7-[2-(2-guanidinoethanethiol)-1-ethyne]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1-benzyl-3-phenyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-benzyl-3-phenyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-benzyl-3-phenyl-4-[succin-2-yl-(1-phenylalanine)]-7-[2-(2-guanidinoethanethiol)-1-ethyne]-3,4-dihydro-1H-1,4-benzodiazepine-2,5-dione trifluoracetate,
(±)-1-isopropyl-3-phenyl-4-(2-carboxy-1-methylethyl)-7-[2-(2-guanidinoethanethiol)-1-ethyne]-3,4-dihydro-1H-1,4-benzodiazepine-2,5-dione trifluoracetate,
(±)-1-isopropyl-3-phenyl-4-(2-carboxy-1-phenylethyl)-7-[2-(2-guanidinoethanethiol)-1-ethyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(2-guanidinoethanethiol)-1-ethyne]-3,4-dihydro-1H-1,4-benzodiazepine-2,5-dione trifluoracetate,
(±)-1-isopropyl-3-phenyl-4-[succin-2-yl-(1-glycine)]-7-[2-(2-guanidinoethanethiol)-1-ethyne]-3,4-dihydro-1H-1,4-benzodiazepine-2,5-dione trifluoracetate,
(±)-1-isopropyl-3-phenyl-4-[succin-2-yl-(1-valine)]-7-[2-(2-guanidinoethanethiol)-1-ethyne]-3,4-dihydro-1H-1,4-benzodiazepine-2,5-dione trifluoracetate,
(±)-1-isopropyl-3-phenyl-4-[succin-2-yl-(1-phenylalanine)]-7-[2-(2-guanidinoethanethiol)-1-ethyne]-3,4-dihydro-1H-1,4-benzodiazepine-2,5-dione trifluoracetate.

### Example 3

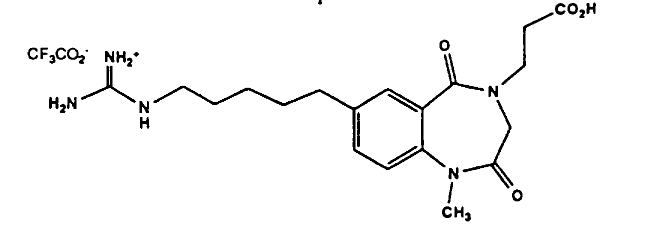

*1-methyl-4-(2-carboxyethyl)-7-(5-guanidino-1****-****pentyl)-3,4-dihydro-1H-1,4-benzodiazepine-2,5-dione trifluoracetate.*

a) A magnetically stirred slurry of 45mgs 1-methyl-4-(2-carboxyethyl)-7-(N-boc-5-amino-1-pentynyl)-3,4-dihydro-1*H*-1,4-benzodiazepine-2,5-dione benzyl ester (0.0924mmol) in 2.0mL ethyl acetate and 10mgs of 10% palladium on carbon was stirred under an atmosphere of hydrogen for 3 hours. The mixture was filtered, washing the solids with ethyl acetate, and concentrated *in vacuo* to yield 45mgs (99%) 1-methyl-4-(2-carboxyethyl)-7-(N-boc-5-amino-1-pentyl)-3,4-dihydro-1*H*-1,4-benzodiazepine-2,5-dione benzyl ester. ¹H NMR (CDCl₃, dTMS) 7.63 (1H, d, ⁴J_{HH}=2Hz, Ar-H *o*-CON)_{,} 7.35-7.27 (6H, Ph, *p*-CON), 7.06 (1H, d, ³J_{HH}=8Hz, *m*-CON), 5.11 (2H, s, OCH₂), 4.55 (1H, bs, BocNH), 3.98 (1H, d, J_{HH}=15Hz, NCHHCO), 3.94 (2H, q, ³J_{HH}=7Hz, NCH₂), 3.80 (1H, d, J_{HH}=15Hz, NCHHCO), 3.35 (3H, s, NCH₃), 3,08 (2H, q, ³J_{HH}=6.5Hz, BocNHCH₂), 2.88-2.67 (2H, m, CH₂CO₂), 2.62 (2H, t, ³J_{HH}=8Hz, ArCH₂), 1.65 (2H, p, ³J_{HH}=8Hz), 1.49 (2H, p, ³J_{HH}=8Hz), 1.42 (9H, s, *t*-Bu), 135 (2H, p, ³J_{HH}=8Hz). ¹³C NMR (CDCl₃,dTMS) 171.2, 168.9, 167.3, 140.1, 138.8, 135.6, 132.2, 130.2, 128.5, 128.4, 128.3, 120.9, 66.6, 52.3, 45.1, 40.4, 34.9, 34.8, 32.9, 30.7, 29.9, 28.4, 26.4.

Using the above procedure, but substituting the appropriate 7-alkynyl-3,4-dihydro-1*H*-1,4-benzodiazepine-2,5-dione for 1-methyl-4-(2-carboxyethyl)-7-(N-boc-5-amino-1-pentynyl)-3,4-dihydro-1H-1,4-benzodiazepine-2,5-dione benzyl ester there may be prepared, for example, the following compounds:
1-methyl-4-(3-ethoxy-1-methyl-3-oxo-1-propyl)-7-[5-(N-Boc)-amino)-1-pentyl]-3,4dihydro-1H-1,4-benzodiazepine-2,5-dione,
1-methyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1-methyl-4-[2-(1-*t*-butoxy-4-benzyloxy)succinyl)]-7-[5-(N-Boc)-amino-1-pentyl]-3,4-dihydro-1H-1,4-benzodiazepine-2,5-dione,
(±)-1-methyl-4-[2-(1-benzyloxyglycinyl-4-benzyloxy)succinyl)]-7-[5-(N.Boc)-amino-1-pentyl]-3,4-dihydro-1H-1,4-benzodiazepine-2,5-dione,
(±)-1-methyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-methyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
1-phenyl-4-(3-ethoxy-1-methyl-3-oxo-1-propyl)-7-[5-(N-Boc)-amino-1-pentyl]-3,4-dihydro-1H-1,4-benzodiazepine-2,5-dione,
1-phenyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1-phenyl-4-[2-(1-*t*- butoxy-4-benzyloxy)succiny 1)]-7-[5-(N-Boc)-amino-1-pentyl]-3,4-dihydro-1H-1,4-benzodiazepine-2,5-dione,
(±)-1-phenyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1-phenyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-phenyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
1-benzyl-4-(3-ethoxy-1-methyl-3-oxo-1-propyl)-7-[5-(N-Boc)-amino-1-pentyl]-3,4-dihydro-1H-1,4-benzodiazepine-2,5-dione,
1-benzyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1-benzyl-4-[2-(1-*t*-butoxy-4-benzyloxy)succinyl)]-7-[5-(N-Boc)-amino-1-pentyl]-3,4-dihyrdo-1H-1,4-benzodiazepine-2,5-dione,
(±)-1-benzyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1-benzyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-benzyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
1-isopropyl-4-(3-ethoxy-1-methyl-3-oxo-1-propyl)-7-[5-(N-Boc)-amino-1-pentyl]-3,4-dihydro-1H-1,4-benzodiazepine-2,5-dione,
1-isopropyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1-isopropyl-4-[2-(1-*t*-butoxy-4-benzyloxy)succinyl)]-7-[5-(N-Boc)-amino-1-pentyl]-3,4-dihydro-1H-1,4-benzodiazepine-2,5-dione,
(±)-1-isopropyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1-isopropyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-isopropyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
1-methyl-4-(3-ethoxy-1-methyl-3-oxo-1-propyl)-7-[5-cyano-1-pentyl]-3,4-dihydro-1H-1,4-benzodiazepine-2,5-dione,
1-methyl-4-(3-ethoxy-1-phenyl-3-oxo-1-propyl)-7-[5-cyano-1-pentyl)-3,4-dihydro-1H-1,4-benzodiazepine-2,5-dione,
(±)-1-methyl-4-[2-(1-*t*-butoxy-4-benzyloxy)succinyl)]-7-[5-cyano-1-pentyl]-3,4-dihydro-1H-1,4-benzodiazepine-2,5-dione,
(±)-1-methyl-4-[2-(1-benzyloxyglycinyl-4-benzyloxy)succinyl)]-7-[5-cyano-1-pentyl]-3,4-dihydro-1H-1,4-benzodiazepine-2,5-dione,
(±)-1-methyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1-methyl-4-[2-(1-benzyloxyphenylalanyl4-benzyloxy)succinyl)]-7-[5-cyano-1-pentyl]-3,4-dihydro-1H-1,4-benzodiazepine-2,5-dione,
1-phenyl-4-(3-ethoxy-1-methyl-3-oxo-1-propyl)-7-[5-cyano-1-pentyl]-3,4-dihydro-1H-1,4-benzodiazepine-2,5-dione,
1-phenyl-4-(3-ethoxy-1-phenyl-3-oxo-1-propyl)-7-[5-cyano)-1-pentyl]-3,4-dihydro-1H-1,4-benzodiazepine-2,5-dione,
(±)-1-phenyl-4-[2-(1-*t*-butoxy-4-benzyloxy)succinyl)]-7-[5-cyano-1-pentyl]-3,4-dihydro-1H-1,4-benzodiazepine-2,5-dione,
(±)-1-phenyl-4-[2-(1-benzyloxyglycinyl-4-benzyloxy)succinyl)]-7-[5-cyano-1-pentyl]-3,4-dihydro-1H-1,4-benzodiazepine-2,5-dione,
(±)-1-phenyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1-phenyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
1-benzyl-4-(3-ethoxy-1-methyl-3-oxo-1-propyl)-7-[5-cyano-1-pentyl]-3,4-dihydro-1H-1,4-benzodiazepine-2,5-dione,
1-benzyl-4-(3-ethoxy-1-phenyl-3-oxo-1-propyl)-7-[5-cyano-1-pentyl-3,4-dihydro-1H-1,4-benzodiazepine-2,5-dione,
(±)-1-benzyl-4-[2-(1*-t*-butoxy-4-benzyloxy)succinyl)]-7-[5-cyano-1-pentyl]-3,4-dihydro-1H-1,4-benzodiazepine-2,5-dione,
(±)-1-benzyl-4-[2-(1-benzyloxyglycinyl-4-benzyloxy)succinyl)]-7-[5-cyano-1-pentyl]-3,4-dihydro-1H-1,4-benzodiazepine-2,5-dione,
(±)-1-benzyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1-benzyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
1-isopropyl-4-(3-ethoxl-1-methyl-3-oxo-1-propyl)-7-[5-cyano-1-pentyl]-3,4-dihydro-1H-1,4-benzodiazepine-2,5-dione,
1-isopropyl-4-(3-ethoxy-1-phenyl-3-oxo-1-propyl)-7-[5-cyano-1-pentyl]-3,4-dihydro-1H-1,4-benzodiazepine-2,5-dione,
(±)-1-isopropyl-4-[2-(1-*t*-butoxy-4-benzyloxy)succinyl)]-7-[5-cyano-1-pentyl]-3,4-dihydro-1H-1,4-benzodiazepine-2,5-dione,
(±)-1-isopropyl-4-[2-(1-benzyloxyglycinyl-4-benzyloxy)succinyl)]-7-[5-cyano-1-pentyl]-3,4-dihydro-1H -1,4-benzodiazepine-2,5-dione,
(±)-1-isopropyl-4-[2-(1-benzyloxyvalinyl-4-benzyloxy)succinyl)]-7-[5-cyano -pentyl]-3,4-dihydro-1H-1,4-benzodiazepine-2,5-dione,
(±)-1-isopropyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
1-methyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
1-methyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1-methyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-methyl-4-(2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-methyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1-methyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
1-phenyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
1-phenyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1-phenyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-phenyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-phenyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1-phenyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
1-benzyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
1-benzyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1-benzyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-benzyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-benzyl-4-[2-(1-benzyloxyvalinyl-4-benzyloxy)succinyl)]-7-[2-(3-cyanophenyl)-1-elhyl]-3,4-dihydro-1H-1,4-benzodiazepine-2,5-dione,
(±)-1-benzyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
1-isopropyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
1-isopropyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1-isopropyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-isopropyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-isopropyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1-isopropyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
1-methyl-4-(3-ethoxy-1-methyl-3-oxo-1-propyl)-7-[N-(2-(N-Boc)-aminoethane]-3-keto-1-propyl-3-amine]-3,4-dihydro-1H-1,4-benzodiazepine-2,5-dione,
1-methyl-4-(3-ethoxy-1-phenyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-methyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-4-[2-(1-benzyloxyglycinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
1-phenyl-4-(3-ethoxy-1-methyl-3-oxo-1-propyl)-7-[N-[2-(N-Boc)-aminoethane]-3-keto-1-propyl-3-amine]-3,4-dihydro-1H-1,4-benzodiazepine-2,5-dione,
1-phenyl-4-(3-ethoxy-1-phenyl-3-oxo-1-propyl)-7-[N-[2-(N-Boc)-aminoethane]-3-keto-1-propyl-3-amine]-3,4-dihydro**-**1H-1,4-benzodiazepine-2,5-dione,
(±)-1-phenyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4--benzodiazepine-2,5-dione,
(±)-1-phenyl-4-[2-(1-benzyloxyglycinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-phenyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-phenyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
1-benzyl-4-(3-ethoxy-1-methyl-3-oxo-1-propyl)-7-[N-[2-(N-Boc)-aminoethane]-3-keto-1-propyl-3-amine]-3,4-dihydro-1H-1,4-benzodiazepine-2,5-dione,
1-benzyl-4-(3-ethoxy-1-phenyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-benzyl-4-[2-(1-*t*-butoxy-4-benzyloxy)succinyl)]-7-[N-[2-(N-Boc)-aminoethane]-3-keto1-propyl-3-amine]-3,4-dihydro-1H-1,4-benzodiazepine-2,5-dione,
(±)-1-benzyl-4-[2-(1-benzyloxyglycinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-benzyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-benzyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
1-isopropyl-4-(3-ethoxy-1-methyl-3-oxo-1-propyl)-7-[N-[2-(N-Boc)-aminoethane]-3-keto-1-propyl-3-amine]-3,4-dihydro-1H-1,4-benzodiazepine-2,5-dione,
1-isopropyl-4-(3-ethoxy-1-phenyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-isopropyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-4-[2-(1-benzyloxyglycinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
1-methyl-4-(3-ethoxy-1-methyl-3-oxo-1-propyl)-7-[3-keto-1-propyl-3-[4-(N-Boc)-1-piperizine]]-3,4-dihydro-1H-1,4-benzodiazepine-2,5-dione,
1-methyl-4-(3-ethoxy-1-phenyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-methyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-methyl-4-[2-(1-benzyloxyglycinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-methyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-methyl-4-[2-(1-benzyloxyphenylalanyl-4-benzyloxy)succinyl)]-7-[3-keto-1-propyl-3-[4-(N-Boc)-1-piperizine]]-3,4-dihydro-1H-1,4-benzodiazepine-2,5-dione,
1-phenyl-4-(3-ethoxy-1-methyl-3-oxo-1-propyl)-7-[3-keto-1-propyl-3-[4-(N-Boc)-1-piperizine]]-3,4-dihydro-1H-1,4-benzodiazepine-2,5-dione,
1-phenyl-4-(3-ethoxy-1-phenyl-3-oxo ]-propyl)-7-[3-keto-1-propyl-3-[4-(N-Boc)-1-piperizine]]-3,4-dihydro-1H-1,4-benzodiazepine-2,5-dione,
(±)-1-phenyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-phenyl-4-[2-(1-benzyloxyglycinyl-4-benzyloxy)succinyl)]-7-[3-keto-1-propyl-3-[4-(N-Boc)-1-piperizine]]-3,4.dihydro-1H-1,4-benzodiazepine-2,5-dione,
(±)-1-phenyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-phenyl-4-[2-(1-benzyloxyphenylalanyl-4-benzyloxy)succinyl)]-7-[3-keto-1-propyl-3-[4-(N-Boc)-1-piperizine]]-3,4-dihydro-1H-1,4-benzodiazepine-2,5-dione,
1-benzyl-4-(3-ethoxy-1-methyl-3-oxo-1-propyl)-7-[3-keto-1-propyl-3-[4-(N-Boc)-1-piperizine]]-3,4-dihydro-1H-1,4-benzodiazepine-2,5-dione,
1-benzyl-4-(3-ethoxy-1-phenyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-benzyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-benzyl-4-[2-(1-benzyloxyglycinyl-4-benzyloxy)succinyl)]-7-(3-keto-1-propyl-3-[4-(N-Boc)-1-piperizine]]-3,4-dihydro-1H-1,4-benzodiazepine-2,5-dione,
(±)-1-benzyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-benzyl-4-[2-(1-benzyloxyphenylalanyl-4-benzyloxy)succinyl)-7-[3-keto-1-propyl-3-[4-(N-Boc)-1-piperizine]]-3,4-dihydro-1H-1,4-benzodiazepine-2,5-dione,
1-isopropyl-4-(3-ethoxy-1-methyl-3-oxo-1-propyl)-7-[3-keto)-1-propyl-3-[4-(N-Boc)-1-piperizine]]-3,4-dihydro-1H-1,4-benzodiazepine-2,5-dione,
1-isopropyl-4-(3-ethoxy-1-phenyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-isopropyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-4-[2-(1-benzyloxyglycinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-4-[2-(1-benzyloxyphenylalanyl-4-benzyloxy)succinyl)]-7-[3-keto-1-propyl-3-(4-(N-Boc)-1-piperizine]]-3,4-dihydro-1H-1,4-benzodiazepine-2,5-dione,
1-methyl-4-(3-ethoxy-1-methyl-3-oxo-1-propryl)-7-[2-[2-(N-Boc)aminoethanethiol]-1-ethyl]-3,4-dihydro-1H-1,4-benzodiazepine-2,5-dione,
1-methyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1-methyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-methyl-4-[2-(1-benzyloxyglycinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-methyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-methyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
1-phenyl-4-(3-ethoxy-1-methyl-3-oxo-1-propyl)-7-[2-[2-(N-Boc)aminoethanethiol]-1-ethyl]-3,4-dihydro-1H-1,4-benzodiazepine-2,5-dione,
1-phenyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1-phenyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-phenyl-4-[2-(1-benzyloxyglycinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-phenyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-phenyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
1-benzyl-4-(3-ethoxy-1-methyl-3-oxo-1-propyl)-7-[2-[2-(N-Boc)aminoethanethiol]-1-ethyl]-3,4-dihydro-1H-1,4-benzodiazepine-2,5-dione,
1-benzyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1-benzyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-benzyl-4-[2-(1-benzyloxyglycinyl-4-benzyloxy)succinyl)]-7-[2-(2-(N-Boc)aminoethaiiethiol]-1-ethyl]-3,4-dihydro-1H-1,4-benzodiazepine-2,5-dione,
(±)-1-benzyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-benzyl-4-[2-(1-benzyloxyphenylalanyl-1-benzyloxy)succinyl)]-7-[2-[2-(N-Boc)aminoethanethiol]-1-ethyl]-3,4-dihydro-1H-1,4-benzodiazepine-2,5-dione,
1-isopropyl-4-(3-ethoxy-1-methyl-3-oxo-1-propyl)-7-[2-[2-(N-Boc)aminoethanethiol]-1-ethyl]-3,4-dihydro-1H-1,4-benzodiazepine-2,5-dione,
1-isopropyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1-isopropyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-4-[2-(1-benzyloxyglycinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
1-methyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
1-methyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-methyl-4-[2-(1-*t*-butoxy-4-benzyloxy)succinyl)]-7-[2-(4-cyanothiophenol)-1-ethyl]-3,4-dihydro-1H-1,4-benzodiazepine-2,5-dione,
(±)-1-methyl-4-(2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1-methyl-4-[2-(1-benzyloxyvalinyl-4-benzyloxy)succinyl)]-7-[2-(4-cyanothiophenol)-1ethyl]-3,4-dihydro-1H-1,4-benzodiazepine-2,5-dione,
(±)-1-methyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
1-phenyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
1-phenyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-phenyl-4-[2-(1-*t*-butoxy-4-benzyloxy)succinyl)]-7-[2-(4-cyanothiophenol)-1-ethyl]-3,4-dihydro-1H-1,4-benzodiazepine-2,5-dione,
(±)-1-phenyl-4-(2-(1-benzyloxyglycinyl-4-benzyloxy)succinyl)]-7-[2-(4-cyanothiophenol)-1-ethyl]-3,4-dihydro-1H-1,4-benzadiazepine-2,5-dione,
(±)-1-phenyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1-phenyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
1-benzyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
1-benzyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-benzyl-4-[2-(1-*t*-butoxy-4-benzyloxy)succinyl)]-7-[2-(4 cyanothiophenol)-1-ethyl]-3,4-dihydro-1H-1,4-benzodiazepine-2,5-dione,
(±)-1-benzyl-4-[2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1-benzyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1-benzyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
1-isopropyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
1-isopropyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-isopropyl-4-[2-(1-*t*-butoxy-4-benzyloxy)succinyl)]-7-[2-(4-cyanothiophenol)-1-ethyl]-3,4-dihydro-1H-1,4-benzodiazepine-2,5-dione,
(±)-1-isopropyl-4-[2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1-isopropyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1-isopropyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
1-methyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
1-methyl-4-(3-ethoxy-1-phenyl-3-oxo)-1-propyl)-7-[4-[2-(N-Boc)amino-6-methylpyrimidine-4-yl]-1-oxa-1-butyl]-3,4-dihydro-1H-1,4-benzodiazepine-2,5-dione,
(±)-1-methyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-4-[2-(1-benzyloxyglycinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-4-[2-(1-benzyloxyphenylylanyl-4-benzyloxy)succinyl)]-7-[4-[2-(N-Boc)amino-6-methylpyrimidine-4-yl]-4-oxa-1-butyl]-3,4-dihydro-11H-1,4-benzodiazepine-2,5-dione,
1-phenyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
1-phenyl-4-(3-ethoxy-1-phenyl-3-oxo)-1-propyl)-7-[4-[2-(N-Boc)amino-6-methylpyrimidine-4-yl]-4-oxa-1-butyl]-3,4-dihlydro-1H-1,4-benzodiazepine-2,5-dione,
(±)-1-phenyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-phenyl-4-[2-(1-benzyloxyglycinyl-4-benzyloxy)succinyl)]-7-[4-[2-(N-Boc)amino-6-methylpyrimidine-4-yl)-4-oxa-1-butyl]-3,4-dihydro-1H-1,4-benzodiazepine-2,5-dione,
(±)-1-phenyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-phenyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
1-benzyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
1-benzyl-4-(3-ethoxy-1-phenyl-3-oxo-1-propyl)-7-[4-(2-(N-Boc)amino-6-methylpyrimidine-4-yl]-4-oxa-1-butyl]-3,4-dihydro-1H-1,4-benzodiazepine-2,5-dione,
(±)-1-benzyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-4-[2-(1-benzyloxyglycinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
1-isopropyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
1-isopropyl-4-(3-ethoxy-1-phenyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-4-[2-(1-benzyloxyglycinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
1-methyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
1-methyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-methyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-methyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-methyl-4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-methyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
1-phenyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
1-phenyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-phenyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-phenyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-phenyl-4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-phenyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
1-benzyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
1-benzyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-benzyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-benzyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-benzyl-4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-benzyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
1-isopropyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
1-isopropyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-isopropyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-methyl-3-phenyl-4-(3-ethoxy-1-methyl-3-oxo-1-propyl)-7-[5-(N-Boc)-amino-1-pentyl]-3,4-dihydro-1H-1,4-benzodiazepine-2,5-dione,
(±)-1-methyl-3-phenyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1-methyl-3-pheny)-4-[2-(1-*t*-butoxy-4-benzyloxy)succinyl)]-7-[5-(N-Boc)-amino-1-pentyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
(±)-1,3-diphenyl-4-(3-ethoxy-1-methyl-3-oxo-1-propyl)-7-[5-(N-Boc)-amino-1-pentyl]-3,4-dihydro-1H-1,4-benzodiazepine-2,5-dione,
(±)-1,3-diphenyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1,3-diphenyl-4-[2-(1-*t*-butoxy-4-benzyloxy)succinyl)]-7-[5-(N-Boc)-amino-1-pentyl]-3,4-dihydro-1H-1,4-benzodiazepine-2,5-dione,
(±)-1,3-diphenyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1,3-diphenyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
(±)-1-benzyl-3-phenyl-4-(3-ethoxy-1-methyl-3-oxo-1-propyl)-7-[5-(N-Boc)-amino-1-pentyl]-3,4-dihydro-1H-1,4-benzodiazepine-2,5-dione,
(±)-1-benzyl-3-phenyl-4-(3-ethoxy-1-phenyl-3-oxo-1-propyl)-7-[5-(N-Boc)-amino-1-pentyl]-3,4-dihydro-1H-1,4-benzodiazepine-2,5-dione,
(±)-1-benzyl-3-phenyl-4-[2-(1-*t*-butoxy-4-benzyloxy)succinyl)]-7-[5-(N-Boc)-amino-1-pentyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
(±)-1-isopropyl-3-phenyl-4-(3-ethoxy-1-methyl-3-oxo-1-propyl)-7-(5-(N-Boc)amino-1-pentyl]-3,4-dihydro-1H-1,4-benzodiazepine-2,5-dione,
(±)-1-isopropyl-3-phenyl-4-(3-ethoxy-1-phenyl-3-oxo-1-propyl)-7-[5-(N-Boc)-amino-1-pentyl]-3,4-dihydro-1H-1,4-benzadiazepine-2,5-dione,
(±)-1-isopropyl-3-phenyl-4-[2-(1-*t*-butoxy-4-benzyloxy)succinyl)]-7-[5-(N-Boc)-amino-1-pentyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[5-(N-Boc)-amino-1-pentyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[5-(N-Boc)-amino-1-pentyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[5-(N-Boc)-amino-1-pentyl]-3,4-dihydro-1H-1,4-benzodiazepine-2,5-dione,
(±)-1-methyl-3-phenyl-4-(3-ethoxy-1-methyl-3-oxo-1-propyl)-7-[5-cyano-1-pentyl]-3,4-dihydro-1H-1,4-benzodiazepine-2,5-dione,
(±)-1-methyl-3-phenyl-4-(3-ethoxy-1-phenyl-3-oxo-1-propyl)-7-[5-cyano-1-pentyl]-3,4-dihydro-1H-1,4-benzodiazepine-2,5-dione,
(±)-1-methyl-3-phenyl-4-[2-(1-*t*-butoxy-4-benzyloxy)succinyl)]-7-[5-cyano-1-pentyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[5-cyano-1-pentyl]-3,4-dihydro-1H-1,4-benzadiazepine-2,5-dione,
(±)-1-methyl-3-phenyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
(±)-1,3-diphenyl-4-(3-ethoxy-1-methyl-3-oxo-1-propyl)-7-[5-cyano-1-pentyl]-3,4-dihydro-1H-1,4-benzodiazepine-2,5-dione,
(±)-1,3-diphenyl-4-(3-ethoxy-1-phenyl-3-oxo-1-propyl)-7-[5-cyano-1-pentyl]-3,4-dihydro-1H-1,4-benzodiazepine-2,5-dione,
(±)-1,3-diphenyl-4-[2-(1-*t*-butoxy-4-benzyloxy)succinyl)]-7-[5-cyano-1-pentyl]-3,4-dihydro-1H-1,4-benzodiazepine-2,5-dione,
(±)-1,3-diphenyl-4-[2-(1-benzyloxyglycinyl-4-benzyloxy)succinyl)]-7-[5-cyano-1-pentyl]-3,4-dihydro-1H-1,4-benzodiazepine-2,5-dione,
(±)-1,3-diphenyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
(±)-1-benzyl-3-phenyl-4-(3-ethoxy-1-methyl-3-oxo-1-propyl)-7-[5-cyano-1-pentyl]-3,4-dihydro-1H-1,4-benzodiazepine-2,5-dione,
(±)-1-benzyl-3-phenyl-4-(3-ethoxy-1-phenyl-3-oxo-1-propyl)-7-[5-cyano-1-pentyl-3,4-dihydro-1H-1,4-benzodiazepine-2,5-dione,
(±)-1-benzyl-3-phenyl-4-[2-(1-*t*-butoxy-4-benzyloxy)succinyl)]-7-[5-cyano-1-pentyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[5-cyano-1-pentyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
(±)-1-isopropyl-3-phenyl-4-(3-ethoxy-1-methyl-3-oxo-1-propyl)-7-[5-cyano-1-pentyl]-3,4-dihydro-1H-1,4-benzodiazepine-2,5-dione,
(±)-1-isopropyl-3-phenyl-4-(3-ethoxy-1-phenyl-3-oxo-1-propyl)-7-[5-cyano-1-pentyl]-3,4-dihydro-1H-1,4-benzodiazepine-2,5-dione,
(±)-1-isopropyl-3-phenyl-4-[2-(1-*t*-butoxy-4-benzyloxy)succinyl)]-7-(5-cyano-1-pentyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[5-cyano-1-pentyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[5-cyano-1-pentyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[5-cyano-1-pentyl]-3,4-dihydro-1H-1,4-benzodiazepine-2,5-dione,
(±)-1-methyl-3-phenyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
(±)-1-methyl-3-phenyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1-methyl-3-phenyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
(±)-1,3-diphenyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
(±)-1,3-diphenyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1,3-diphenyl-4-[2-(1-*t*-butoxy-4-benzyloxy)succinyl)]-7-[2-(3-cyanophenyl)-1-ethyl]-3,4-dihydro-1H-1,4-benzodiazepine-25-dione,
(±)-1,3-diphenyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1,3-diphenyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
(±)-1-benzyl-3-phenyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
(±)-1-benzyl-3-phenyl-4-(3-ethoxy-1-phenyl-3-oxo-1-propyl)-7-[2-(3-cyanophenyl)-1-ethyl]-3,4-dihydro-1h-1,4-benzodiazepine-2,5-dione,
(±)-1-benzyl-3-phenyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
(±)-1-isopropyl-3-phenyl-4-(3-ethoxy-1-methyl-3-oxo-1-propyl)-7-[2-(3-cyanophenyl)-1-ethyl]-3,4-dihydro-1H-1,4-benzodiazepine-2,5-dione,
(±)-1-isopropyl-3-phenyl-4-(3-ethoxy-1-phenyl-3-oxo-1-propyl)-7-[2-(3-cyanophenyl)-1-ethyl]-3,4-dihydro-1H-1,4-benzodiazepine-2,5-dione,
(±)-1-isopropyl-3-phenyl-4-[2-(1-*t*-butoxy-4-benzyloxy)succinyl)]-7-[2-(3-cyanophenyl)-1-ethyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-(3-cyanophenyl)-1-ethyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-(3-cyanophenyl)-1-ethyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-(3-cyanophenyl)-1-ethyl]-3,4-dihydro-1H-1,4-benzodiazepine-2,5-dione,
(±)-1-methyl-3-phenyl-4-(3-ethoxy-1-methyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-methyl-3-phenyl-4-(3-ethoxy-1-phenyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-methyl-3-phenyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-3-phenyl-4-[2-(1-benzyloxyglycine-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1,3-diphenyl-4-(3-ethoxy-1-methyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1,3-diphenyl-4-(3-ethoxy-1-phenyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1,3-diphenyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1,3-diphenyl-4-[2-(1-benzyloxyglycinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1,3-diphenyl-4-[2-(1-benzyloxyvalinyl-4-benzyloxy)succinyl)]-7-[N-[2-(N-Boc)-aminoethane]-3-keto-1- propyl-3-amine]-3,4-dihydro-1H-1,4-benzodiazepine-2,5-dione,
(±)-1,3-diphenyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-benzyl-3-phenyl-4-(3-ethoxy-1-methyl-3-oxo-1-propyl)-7-[N-[2-(N-Boc)-aminoethane]-3-keto-1-propyl-3-amine]-3,4-dihydro**-**1H-1,4-benzodiazepine-2,5-dione,
(±)-1-benzyl-3-phenyl-4-(3-ethoxy-1-phenyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-benzyl-3-phenyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-benzyl-3-phenyl-4-[2-(1-benzyloxyglycinyl-4-benzyloxy)succinyl)-7-[N-[2-(N-Boc)-aminoethane]-3-keto-1-propyl-3-amine]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-3-phenyl-4-(3-ethoxy-1-methyl-3-oxo-1-propyl)-7-[N-[2-(N-Boc)-aminoethane]-3-keto-1-propyl-3-amine]-3,4-dihydro-1H-1,4-benzodiazepine-2,5-dione,
(±)-1-isopropyl-3-phenyl-4-(3-ethoxy-1-phenyl-3-oxo-1-propyl)-7-[N-[2-(N-Boc)-aminoethane]-3-keto-1-propyl-3-amine]-3,4-dihydro-1H -1,4-benzodiazepine-2,5-dione,
(±)-1-isopropyl-3-phenyl-4-[2-(1-*t*-butoxy-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[N-[2-(N-Boc)-aminoethane]-3-keto-1-propyl-3-amine]-3,4-dihydro-1H-1,4-benzodiazepine-2,5-dione,
(±)-1-methyl-3-phenyl-4-(3-ethoxy-1-methyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-methyl-3-phenyl-4-(3-ethoxy-1-phenyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-methyl-3-phenyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1,3-diphenyl-4-(3-ethoxy-1-methyl-3-oxo-1-propyl)-7-[3-keto-1-propyl-3-[4-(N-Boc)-1-piperizine]]-3,4-dihydro-1H-1,4-benzodiazepine-2,5-dione,
(±)-1,3-diphenyl-4-(3-ethoxy-1-phenyl-3-oxo-1-propyl)-7-(3-keto-1-propyl-3-[4-(N-Boc)-1-piperizine]]-3,4-dihydro-1H-1,4-benzodiazepine-2,5-dione,
(±)-1,3-diphenyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1,3-diphenyl-4-[2-(1-benzyloxyglycinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1,3-diphenyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1,3-diphenyl-4-[2-(1-benzyloxyphenylalanyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-benzyl-3-phenyl-4-(3-ethoxy-1-methyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-benzyl-3-phenyl-4-(3-ethoxy-1-phenyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-benzyl-3-phenyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-3-phenyl-4-(3-ethoxy-1-methyl-3-oxo-1-propyl)-7-[3-keto-1-propyl-3-[4-(N-Boc)-1-piperizine])-3,4-dihydro-1H-1,4-benzodiazepine-2,5-dione,
(±)-1-isopropyl-3-phenyl-4-(3-ethoxy-1-phenyl-3-oxo-1-propyl)-7-[3-keto-1-propyl-3-[4-(N-Boc)-1-piperizine]]-3,4-dihydro-1H-1,4-benzodiazepine-2,5-dione,
(±)-1-isopropyl-3-phenyl-4-[2-(1-*t*-butoxy-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[3-keto-1-propyl-3-[4-(N-Boc)-1-piperizine]]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[3-keto-1-propyl-3-[4-(N-Boc)-1-pirerizine]]-3,4-dihydro-1H-1,4-benzodiazepine-2,5-dione,
(±)-1-methyl-3-phenyl-4-(3-ethoxy-1-methyl-3-oxo-1-propyl)-7-[2-[2-(N-Boc)aminoethanethiol]-1-ethyl]-3,4-dihydro-1H-1,4-benzodiazepine-2,5-dione,
(±)-1-methyl-3-phenyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1-methyl-3-phenyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-[2-(N-Boc)aminoethanethiol]-1-ethyl-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
(±)-1,3-diphenyl-4-(3-ethoxy-1-methyl-3-oxo-1-propyl)-7-[2-[2-(N-Boc)aminoethanethiol]-1-ethyl]-3,4-dihydro-1H-1,4-benzodiazepine-2,5-dione,
(±)-1,3-diphenyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1,3-diphenyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1,3-diphenyl-4-[2-(1-benzyloxyglycinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1,3-diphenyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-benzyl-3-phenyl-4-(3-ethoxy-1-methyl-3-oxo-1-propyl)-7-[2-[2-(NBoc)aminoethanethiol]-1-ethyl]-3,4-dihydro-1H-1,4-benzodiazepine-2,5-dione,
(±)-1-benzyl-3-phenyl-4-(3-ethoxy-1-phenyl-3-oxo-1-propyl)-7-[2-[2-(N-Boc)aminoethanethiol]-1-ethyl]-3,4-dhydro-1H-1,4-benzodiazepine-2,5-dione,
(±)-1-benzyl-3-phenyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-3-phenyl-4-(3-ethoxy-1-methyl-3-oxo-1-propyl)-7-[2-[2-(N-Boc)aminoethanethiol]-1-ethyl]-3,4-dihydro-1H-1,4-benzodiazepine-2,5-dione,
(±)-1-isopropyl-3-phenyl-4-(3-ethoxy-1-phenyl-3-oxo-1-propyl)-7-[2-[2-(N-Boc)aminoethanethiol]-1-ethyl]-3,4-dihydro-1H-1,4-benzodiazepine-2,5-dione,
(±)-1-isopropyl-3-phenyl-4-[2-(1-*t*-butoxy-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-[2-(N-Boc)aminoethanethiol]-1-ethyl]-3,4-dihydro-1H-1,4-benzodiazepine-2,5-dione,
(±)-1-methyl-3-phenyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
(±)-1-methyl-3-phenyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-methyl-3-phenyl-4-[2-(1-*t*-butoxy-4-benzyloxy)succinyl)]-7-[2-(4-cyanothiophenol)-1-ethyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
(±)-1,3-diphenyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
(±)-1,3-diphenyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1,3-diphenyl-4-[2-(1-*t*-butoxy-4-benzyloxy)succinyl)]-7-[2-(4-cyanothiophenol)-1-ethyl]3,4-dihydro-1H-1,4-benzodiazepine-2,5-dione,
(±)-1,3-diphenyl-4-[2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1,3-diphenyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
(±)-1-benzyl-3-phenyl-4-(3-ethoxy-1-methyl-3-oxo-1-propyl)-7-[2-(4-cyanothiophenol)-1-ethyl]-3,4-dihydro-1H -1,4-benzodiazepine-2,5-dione,
(±)-1-benzyl-3-phenyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-benzyl-3-phenyl-4-[2-(1-*t*-butoxy-4-benzyloxy)succinyl)]-7-[2-(4-cyanothiophenol)-1-ethyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
(±)-1-isopropyl-3-phenyl-4-(3-ethoxy-1-methyl-3-oxo-1-propyl)-7-[2-(4-cyanothiophenol)-1-ethyl]-3,4-dihydro-1H-1,4-benzodiazepine-2,5-dione,
(±)-1-isopropyl-3-phenyl-4-(3-ethoxy-1-phenyl-3-oxo-1-propyl)-7-[2-(4-cyanothiophenol)-1-ethyl]-3,4-dihydro-1H-1,4-benzodiazepine-2,5-dione,
(±)-1-isopropyl-3-phenyl-4-[2-(1-*t*-butoxy-4-benzyloxy)succinyl)]-7-[2-(4-cyanothiophenol)-1-ethyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[2-(4-cyanothiophenol)-1-ethyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[2-(4-cyanothiophenol)-1-ethyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[2-(4-cyanothiophenol)-1-ethyl]-3,4-dihydro-1H-1,4-benzodiazepine-2,5-dione,
(±)-1-methyl-3-phenyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-3-phenyl-4-(3-ethoxy-1-phenyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-3-phenyl-4-[2-(1-*t*-butoxy-4-benzyloxy)succinyl)]-7-[4-[2-(N-Boc)amino-6-methylprimidine-4-yl]-4-oxa-1-butyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1,3-diphenyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1,3-diphenyl-4-(3-ethoxy-1-phenyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1,3-diphenyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1,3-diphenyl-4-[2-(1-benzyloxyglycinyl-4-benzyloxy)succinyl)]-7-(4-[2-(N-Boc)amino-6-methylpyrimidine-4-yl]-4-oxa-1-butyl]-3,4-dihydro-1H-1,4-benzodiazepine-2,5-dione,
(±)-1,3-diphenyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-3-phenyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-3-phenyl-4-(3-ethoxy-1-phenyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-3-phenyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-3-phenyl-4-(3-ethoxy-1-methyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-3-phenyl-4-(3-ethoxy-1-phenyl-3-oxo-1-propyl)-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-3-phenyl-4-[2-(1-*t*-butoxy-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[4-[2-(N-Boc)amino-6-methylpyrimidine-4-yl]-4-oxa-1-butyl]-3,4-dihydro-1H-1,4-benzodiazepine-2,5-dione,
(±)-1-methyl-3-phenyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-methyl-3-phenyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-methyl-3-phenyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-methyl-3-phenyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-methyl-3-phenyl-4-[2-(1-benzyloxyvalinyl-4-benzyloxy)succinyl)]-7-[5-(2-(N-Boc)aminopyridine-3-yl]-5-oxa-1-pentyl]-3,4-dihydro-1H-1,4-benzodiazepine-2,5-dione,
(±)-1-methyl-3-phenyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1,3-diphenyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1,3-diphenyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1,3-diphenyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1,3-diphenyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1,3-diphenyl4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1,3-diphenyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-benzyl-3-phenyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-benzyl-3-phenyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-benzyl-3-phenyl-4-[2-(1-*t*-butoxy-4-benzyloxy)succinyl)]-7-[5-[2-(N-Boc)aminopyridine-3-yl)-5-oxa-1-pentyl]-3,4-dihydro-1H-1,4-benzodiazepine-2,5-dione,
(±)-1-benzyl-3-phenyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-benzyl-3-phenyl-4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-benzyl-3-phenyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-3-phenyl-4-(3-ethoxy-1-methyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-isopropyl-3-phenyl-4-(3-ethoxy-1-phenyl-3-oxo-1-propyl)-7-[5-[2-(N-Boc)aminopyridine-3-yl]-5-oxa-1-pentyl]-3,4-dihydro-1H-1,4-benzodiazepine-2,5-dione,
(±)-1-isopropyl-3-phenyl-4-[2-(1-*t*-butoxy-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-3-phenyl-4-[2-(1-benzyloxyglycinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-3-phenyl-4-[2-(1-benzyloxyvalinyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,
(±)-1-isopropyl-3-phenyl-4-[2-(1-benzyloxyphenylalanyl-4-benzyloxy)succinyl)]-7-[5-[2-(N-Boc)aminopyridine-3-yl]-5-oxa-1-pentyl]-3,4-dihydro-1H-1,4-benzodiazepine-2,5-dione,

b) 1-methyl-4-(2-carboxyethyl)-7-(5-amino-1-pentyl)-3,4-dihydro-1H-1,4-benzodiazepine-2,5-dione trifluoracetate was prepared using the method described in part (g) of example 1. Thus, 45mgs 1-methyl-4-(2-carboxyethyl)-7-(N-boc-5-amino-1-pentyl)-3,4-dihydro-1H-1,4-benzodiazepine-2,5-dione benzyl ester yielded 36mgs (93%) 1-methyl-4-(2-carboxyethyl)-7-(5-amino-1-pentyl)-3,4-dihydro-1*H*-1,4-benzodiazepine-2,5-dione trifluoracetate (1/2" C-18 reverse-phase column, eluting with a solvent gradient of 10:90 acetonitrite (0.1% trifluoracetic acid)/ water (0.1% trifluoracetic acid), time 0 to 10 min, to 50:50 acetonitrile (0.1% trifluoracetic acid)/water (0.1% trifluoracetic acid), time 10 min to 40 min, flow=10ml/min, Rₜ=30.3 min, un detection 254nm). HRMS (FAB) molecular ion m/z=348.1931 (cald. C₁₈H₂₅N₃O₄, 348.1923) ¹H NMR (CDCl₃, dTMS) 7.39 (1H, bs, Ar-H *o*-CON), 734 (1H, d, ³J_{HH}=8Hz, *p*-CON), 7.16 (1H, d, ³J_{HH}=8Hz, *m*-CON), 4.00-3.90 (2H, m, NCHHCO, NCHHCH₂), 3.67 (1H, d, J_{HH}=16Hz, NCHHCO), 3.56 (1H, dt, J_{HH}=14Hz, ³J_{HH}=6Hz, NCHHCH₂) 3.17 (3H, s, NCH₃), 2.82 (2H, t, ³J_{HH}=7Hz, H₃NCH₂), 2.67-2.47 (4H, m, CH₂CO_{2,} ArCH₂), 1.57-1.42 (4H, bs), 1.16 (2H, p, ³J_{HH}=7Hz).

Using the above procedure, but substituting the appropriate 3,4-dihydro-1*H*-1,4-benzodiazepine-2,5-dione for 1-methyl-4-(2-carboxyethyl)-7-(N-boc-5-amino-1-pentyl)-3,4-dihydro-1*H*-1,4-benzodiazepine-2,5-dione benzyl ester there could be prepared, for example, the following compounds:
1-methyl-4-(2-carboxy-1-methylethyl)-7-(5-amino-1-pentyl)-3,4-dihydro-1H-1,4-benzodiazepine-2,5-dione trifluoracetate,
1-methyl-4-(2-carboxy-1-phenylethyl)-7-(5-amino-1-pentyl)-3,4-dihydro-1H-1,4-benzodiazepine-2,5-dione trifluoracetate,
(±)-1-methyl-4-[succin-2-yl-(1-*t*-butoxy)]-7-(5-amino-1-pentyl)-3,4-dihydro-1H-1,4-benzodiazepine-2,5-dione trifluoracetate,
(±)-1-methyl-4-[succin-2-yl-(1-glycine)]-7-(5-amino-1-pentyl)-3,4-dihydro-1H-1,4-benzodiazepine-2,5-dione trifluoracetate,
(±)-1-methyl-4-[succin-2-yl-(1-valine)]-7-(5-amino-1-pentyl)-3,4-dihydro-1H-1,4-benzodiazepine-2,5-dione trifluoracetate,
(±)-1-methyl-4-[succin-2-yl-(1-phenylalanine)]-7-(5-amino-1-pentyl)-3,4-dihydro-1H-1,4-benzodiazepine-2,5-dione trifluoracetate,
1-phenyl-4-(2-carboxy-1-methylethyl)-7-(5-amino-1-pentyl)-3,4-dihydro-1H-1,4-benzodiazepine-2,5-dione trifluoracetate,
1-phenyl-4-(2-carboxy-1-phenylethyl)-7-(5-amino-1-pentyl)-3,4-dihydro-1H-1,4-benzodiazepine-2,5-dione trifluoracetate,
(±)-1-phenyl-4-[succin-2-yl-(1-*t*-butoxy)]-7-(5-amino-1-pentyl)-3,4-dihydro-1H-1,4-benzodiazepine-2,5-dione trifluoracetate,
(±)-1-phenyl-4-[succin-2-yl-(1-glycine)]-7-(5-amino-1-pentyl)-3,4-dihydro-1H-1,4-benzodiazepine-2,5-dione trifluoracetate,
(±)-1-phenyl-4-[succin-2-yl-(1-valine)]-7-(5-amino-1-pentyl)-3,4-dihydro-1H-1,4-benzodiazepine-2,5-dione trifluoracetate,
(±)-1-phenyl-4-[succin-2-yl-(1-phenylalanine)]-7-(5-amino-1-pentyl)-3,4-dihydro-1H-1,4-benzodiazepine-2,5-dione trifluoracetate,
1-benzyl-4-(2-carboxy-1-methylethyl)-7-(5-amino-1-pentyl)-3,4-dihydro-1H-1,4-benzodiazepine-25-dione trifluoracetate,
1-benzyl-4-(2-carboxy-1-phenylethyl)-7-(5-amino-1-pentyl)-3,4-dihydro-1H-1,4-benzodiazepine-2,5-dione trifluoracetate,
(±)-1-benzyl-4-[succin-2-yl-(1-*t*-butoxy)]-7-(5-amino)-1-pentyl)-3,4-dihydro-1H-1,4-benzodiazepine-2,5-dione trifluoracetate,
(±)-1-benzyl-4-[succin-2-yl-(1-glycine)]-7-(5-amino-1-pentyl)-3,4-dihydro-1H-1,4-benzodiazepine-2,5-dione trifluoracetate,
(±)-1-benzyl-4-[succin-2-yl-(1-valine)]-7-(5-amino-1-pentyl)-3,4-dihydro-1H-1,4-benzodiazepine-2,5-dione trifluoracetate,
(±)-1-benzyl-4-[succin-2-yl-(1-phenylalanine)]-7-(5-amino-1-pentyl)-3,4-dihydro-1H-1,4-benzodiazepine-2,5-dione trifluoracetate,
1-isopropyl-4-(2-carboxy-1-methylethyl)-7-(5-amino-1-pentyl)-3,4-dihydro-1H-1,4-benzodiazepine-2,5-dione trifluoracetate,
1-isopropyl-4-(2-carboxy-1-phenylethyl)-7-(5-amino-1-pentyl)-3,4-dihydro-1H-1,4-benzodiazepine-2,5-dione trifluoracetate,
(±)-1-isopropyl-4-[succin-2-yl-(1-*t*-butoxy)]-7-(5-amino-1-pentyl)-3,4-dihydro-1H-1,4-benzodiazepine-2,5-dione trifluoracetate,
(±)-1-isopropyl-4-[succin-2-yl-(1-glycine)]-7-(5-amino-1-pentyl)-3,4-dihydro-1H-1,4-benzodiazepine-2,5-dione trifluoracetate,
(±)-1-isopropyl-4-[succin-2-yl-(1-valine)]-7-(5-amino-1-pentyl)-3,4-dihydro-1H-1,4-benzodiazepine-2,5-dione trifluoracetate,
(±)-1-isopropyl-4-[succin-2-yl-(1-phenylalanine)]-7-(5-amino-1-pentyl)-3,4-dihydro-1H-1,4-benzodiazepine-2,5-dione trifluoracetate,
1-methyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
1-methyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-methyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-phenyl-4-(2-carboxy-1-methylethyl)-7-[N-(2-aminoethane)-3-keto-1-propyn-3-amine]-3,4-dihydro-1H-1,4-benzodiazerine-2,5-dione trifluoracetate,
1-phenyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-phenyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-benzyl-4-(2-carboxy-1-methylethyl)-7-[N-(2-aminoethane)-3-keto-propyn-3-amine]-3,4-dihydro-1H-1,4-benzodiazepine-2,5-dione trifluoracetate,
1-benzyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-benzyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-isopropyl-4-(2-carboxy-1-methylethyl)-7-[N-(2-aminoethane)-3-keto-1-propyn-3-amine]-3,4-dihydro-1H-1,4-benzodiazepine-2,5-dione trifluoracetate,
1-isopropyl-4-(2-carboxy-1-phenylethyl)-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-*t*-butoxy)]-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-glycine)]-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-valine)]-7-[N-(2-aminoethane)-3-keto-1-propyn-3-amine]-3,4-dihydro-1H-1,4-benzodiazepine-2,5-dione trifluoracetate,
(±)-1-isopropyl-4-[succin-2-yl-(1-phenylalanine)]-7-[N-(2-aminoethane)-3-keto-1-propyn-3-amine]-3,4-dihydro-1H-1,4-benzodiazepine-2,5-dione trifluoracetate,
1-methyl-4-(2-carboxy-1-methylethyl)-7-[3-keto-1-propyn-3-(1-piperizine)]-3,4-dihydro-1H-1,4-benzodiazepine-2,5-dione trifluoracetate,
1-methyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-methyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-phenyl-4-(2-carboxy-1-methylethyl)-7-[3-keto-1-propyn-3-(1-piperizine)]-3,4-dihydro-1H-1,4-benzodiazepine-2,5-dione trifluoracetate,
1-phenyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-phenyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-benzyl-4-(2-carboxy-1-methylethyl)-7-[3-keto-1-propyn-3-(1-piperizine)]-3,4-dihydro-1H-1,4-benzodiazepine-2,5-dione trifluoracetate,
1-benzyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-benzyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-isopropyl-4-(2-carboxy-1-methylethyl)-7-[3-keto-1-propyn-3-(1-piperizine)]-3,4-dihydro-1H-1,4-benzodiazepine-2,5-dione trifluoracetate,
1-isopropyl-4-(2-carboxy-1-phenylethyl)-7-[3-keto-1-propyn-3-(1-piperizine)]-3,4-dihydro-1H-1,4-benzodiazepine-2,5-dione trifluoracetate,
(±)-1-isopropyl-4-[succin-2-yl-(1-*t*-butoxy)]-7-[3-keto-1-propyn-3-(1-piperizine)]-3,4-dihydro-1H-1,4-benzodiazepine-2,5-dione trifluoracetate,
(±)-1-isopropyl-4-[succin-2-yl-(1-glycine)]-7-[3-keto-1-propyn-3-(1-piperizine)]-3,4-dihydro-1H-1,4-benzodiazepine-2,5-dione trifluoracetate,
(±)-1-isopropyl-4-[succin-2-yl-(1-valine)]-7-[3-keto-1-propyn-3-(1-piperizine)]-3,4-dihydro-1H-1,4-benzodiazepine-2,5-dione trifluoracetate,
(±)-1-isopropyl-4-[succin-2-yl-(1-phenylalanine)]-7-[3-keto-1-propyn-3-(1-piperizine)]-3,4-dihydro-1H-1,4-benzodiazepine-2,5-dione trifluoracetate,
1-methyl-4-(2-carboxy-1-methylethyl)-7-[2-(2-aminoethanethiol)-1-ethyl]-3,4-dihydro-1H-1,4-benzodiazepine-2,5-dione trifluoracetate,
1-methyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1-methyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-methyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1-methyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-methyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
1-phenyl-4-(2-carboxy-1-methylethyl)-7-[2-(2-aminoethanethiol)-1-ethyl]-3,4-dihydro-1H-1,4-benzodiazepine-2,5-dione trifluoracetate,
1-phenyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1-phenyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-phenyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1-phenyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-phenyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
1-benzyl-4-(2-carboxy-1-methylethyl)-7-[2-(2-aminoethanethiol)-1-ethyl]-3,4-dihydro-1H-1,4-benzodiazepine-2,5-dione trifluoracetate,
1-benzyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1-benzyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-benzyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1-benzyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-benzyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
1-isopropyl-4-(2-carboxy-1-methylethyl)-7-[2-(2-aminoethanethiol)-1-ethyl]-3,4-dihydro-1H-1,4-benzodiazepine-2,5-dione trifluoracetate,
1-isopropyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1-isopropyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-isopropyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1-isopropyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-isopropyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
1-methyl-4-(2-carboxy-1-methylethyl)-7-[4-(2-amino-6-methylpyrimidine-4-yl)-4-oxa-1-butyl)-3,4-dihydro-1H-1,4-benzodiazepine-2,5-dione trifluoracetate,
1-methyl-4-(2-carboxy-1-phenylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-4-[succin-2-yl-(1-glycine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
1-phenyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate,
1-phenyl-4-(2-carboxy-1-phenylethyl)-7-[4-(2-amino-6-methylpyrimidine-1-yl)-4-oxa-1-butyl]-3,4-dihydro-1H-1,4-benzodiazepine-2,5-dione trifluoracetate,
(±)-1-phenyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-phenyl-4-[succin-2-yl-(1-glycine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-phenyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-phenyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
1-benzyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate,
1-benzyl-4-(2-carboxy-1-phenylethyl)-7-[4-(2-amino-6-methylpyrimidine-4-yl]-4-oxa-1-butyl]-3,4-dihydro-1H-1,4-benzodiazepine-2,5-dione trifluoracetate,
(±)-1-benzyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-4-[succin-2-yl-(1-glycine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
1-isopropyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate,
1-isopropyl-4-(2-carboxy-1-phenylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-4-[succin-2-yl-(1-glycine)]-7-[4-(2-amino-6-methylpyrimidine-4-yl)]-4-oxa-1-butyl]-3,4-dihydro-1H-1,4-benzodiazepine-2,5-dione trifluoracetate,
(±)-1-isopropyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
1-methyl-4-(2-carboxy-1-methylethyl)-7-[5-(2-aminopyridine-3-yl)-5-oxa-1-pentyl]-3,4-dihydro-1H-1,4-benzodiazepine-2,5-dione trifluoracetate,
1-methyl-4-(2-carboxy-1-phenylethyl)-7-[5-(2-aminopyridine-3-yl)-5-oxa-1-pentyl]-3,4-dihydro-1H-1,4-benzodiazepine-2,5-dione trifluoracetate,
(±)-1-methyl-4-[succin-2-yl-(1-*t*-butoxy)]-7-[5-(2-aminopyridine-3-yl)-5-oxa-1-pentyl]-3,4-dihydro-1H-1,4-benzodiazepine-2,5-dione trifluoracetate,
(±)-1-methyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1-methyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-methyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
1-phenyl-4-(2-carboxy-1-methylethyl)-7-[5-(2-aminopyridine-3-yl)-5-oxa-1-pentyl]-3,4-dihydro-1H-1,4-benzodiazepine-2,5-dione trifluoracetate.
1-phenyl-4-(2-carboxy-1-phenylethyl)-7-(5-(2-aminopyridine-3-yl]-3,4-dihydro-1H-1,4-benzodiazepine-2,5-dione trifluoracetate,
(±)-1-phenyl-4-[succin-2-yl-(1-*t*-butoxy)]-7-[5-(2-aminopyridine-3-yl)-5-oxa-1-pentyl]-3,4-dihydro-1H-1,4-benzodiazepine-2,5-dione trifluoracetate,
(±)-1-phenyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1-phenyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-phenyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
1-benzyl-4-(2-carboxy-1-methylethyl)-7-[5-(2-aminopyridine-3-yl)-5-oxa-1-pentyl]-3,4-dihydro-1H-1,4-benzodiazepine-2,5-dione,
1-benzyl-4-(2-carboxy-1-phenylethyl)-7-[5-(2-aminopyridine-3-yl)-5-oxa-1-pentyl]-3,4-dihydro-1H-1,4-benzodiazepine-2,5-dione trifluoracetate,
(±)-1-benzyl-4-[succin-2-yl-(1-*t*-butoxy)]-7-[5-(2-aminopyridine-3-yl)-5-oxa-1-pentyl]-3,4-dihydro-1H-1,4-benzodiazepine-7,5-dione trifluoracetate,
(±)-1-benzyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1-benzyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-benzyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
1-isopropyl-4-(2-carboxy-1-methylethyl)-7-[5-(2-aminopyridine-3-yl)-5-oxa-1-pentyl]-3,4-dihydro-1H-1,4-benzodiazepine-2,5-dione trifluoracetate,
1-isopropyl-4-(2-carboxy-1-phenylethyl)-7-[5-(2-aminopyridine-3-yl)-5-oxa-1-pentyl]-3,4-dihydro-1H-1,4-benzodiazepine-2,5-dione trifluoracetate,
(±)-1-isopropyl-4-[succin-2-yl-(1-*t*-butoxy)]-7-[5-(2-aminopyridine-3-yl)-5-oxa-1-pentyl]-3,4-dihydro-1H-1,4-benzodiazepine-2,5-dione trifluoracetate,
(±)-1-isopropyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1-isopropyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-isopropyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
(±)-1-methyl-3-phenyl-4-(2-carboxy-1-methylethyl)-7-(5-amino-1-pentyl)-3,4-dihydro-1H-1,4-benzodiazepine-2,5-dione trifluoracetate,
(±)-1-methyl-3-phenyl-4-(2-carboxy-1-phenylethyl)-7-(5-amino-1-pentyl)-3,4-dihydro-1H-1,4-benzodiazepine-2,5-dione trifluoracetate,
(±)-1-methyl-3-phenyl-4-[succin-2-yl-(1-*t*-butoxy)]-7-(5-amino-1-pentyl)-3,4-dihydro-1H-1,4-benzodiazepine-2,5-dione trifluoracetate,
(±)-1-methyl-3-phenyl-4-[succin-2-yl-(1-glycine)]-7-(5-amino-1-pentyl)-3,4-dihydro-1H-1,4-benzodiazepine-2,5-dione trifluoracetate,
(±)-1-methyl-3-phenyl-4-[succin-2-yl-(1-valine)]-7-(5-amino-1-pentyl)-3,4-dihydro-1H-1,4-benzodiazepine-2,5-dione trifluoracetate,
(±)-1-methyl-3-phenyl-4-[succin-2-yl-(1-phenylalanine)]-7-(5-amino-1-pentyl)-3,4-dihydro-1H-1,4-benzodiazepine-2,5-dione trifluoracetate,
(±)-1,3 diphenyl-4-(2-carboxy-1-methylethyl)-7-(5-amino-1-pentyl)-3,4-dihydro-1H-1,4-benzodiazepine-2,5-dione trifluoracetate,
(±)-1,3-diphenyl-4-(2-carboxy-1-phenylethyl)-7-(5-amino-1-pentyl)-3,4-dihydro-1H-1,4-benzodiazepine-2,5-dione trifluoracetate,
(±)-1,3-diphenyl-4-[succin-2-yl-(1-*t*-butoxy)]-7-(5-amino-1-pentyl)-3,4-dihydro-1H-1,4-benzodiazepine-2,5-dione trifluoracetate,
(±)-1,3-diphenyl-4-[succin-2-yl-(1-glycine)]-7-(5-amino-1-pentyl)-3,4-dihydro-1H-1,4-benzodiazepine-2,5-dione trifluoracetate,
(±)-1,3-diphenyl-4-[succin-2-yl-(1-valine)]-7-(5-amino-1-pentyl)-3,4-dihydro-1H-1,4-benzadiazepine-2,5-dione trifluoracetate,
(±)-1,3-diphenyl-4-[succin-2-yl-(1-phenylalanine)]-7-(5-amino-1-pentyl)-3,4-dihydro-1H-1,4-benzodiazepine-2,5-dione trifluoracetate,
(±)-1-benzyl-3-phenyl-4-(2-carboxy-1-methylethyl)-7-(5-amino-1-pentyl)-3,4-dihydro-1H-1,4-benzodiazepine-2,5-dione trifluoracetate,
(±)-1-benzyl-3-phenyl-4-(2-carboxy-1-phenylethyl)-7-(5-amino-1-pentyl)-3,4-dihydro-1H-1,4-benzodiazepine-2,5-dione trifluoracetate,
(±)-1-benzyl-3-phenyl-4-[succin-2-yl-(1-*t*-butoxy)]-7-(5-amino-1-pentyl)-3,4-dihydro-1H-1,4-benzodiazepine-2,5-dione trifluoracetate,
(±)-1-benzyl-3-phenyl-4-[succin-2-yl-(1-glycine)]-7-(5-amino-1-pentyl)-3,4-dihydro-1H-1,4-benzodiazepine-2,5-dione trifluoracetate,
(±)-1-benzyl-3-phenyl-4-[succin-2-yl-(1-valine)]-7-(5-amino-1-pentyl)-3,4-dihydro-1H-1,4-benzodiazepine-2,5-dione trifluoracetate,
(±)-1-benzyl-3-phenyl-4-[succin-2-yl-(1-phenylalanine)]-7-(5-amino-1-pentyl)-3,4-dihydro-1H-1,4-benzodiazepine-2,5-dione trifluoracetate,
(±)-1-isopropyl-3-phenyl-4-(2-carboxy-1-methylethyl)-7-(5-amino-1-pentyl)-3,4-dihydro-1H-1,4-benzodiazepine-2,5-dione trifluoracetate,
(±)-1-isopropyl-3-phenyl-4-(2-carboxy-1-phenylethyl)-7-(5-amino-1-pentyl)-3,4-dihydro-1H-1,4-benzodiazepine-2,5-dione trifluoracetate,
(±)-1-isopropyl-3-phenyl-4-[succin-2-yl-(1-*t*-butoxy)]-7-(5-amino-1-pentyl)-3,4-dihydro-1H-1,4-benzodiazepine-2,5-dione trifluoracetate,
(±)-1-isopropyl-3-phenyl-4-[succin-2-yl-(1-glycine)]-7-(5-amino-1-pentyl)-3,4-dihydro-1H-1,4-benzodiazepine-2,5-dione trifluoracetate,
(±)-1-isopropyl-3-phenyl-4-[succin-2-yl-(1-valine)]-7-(5-amino-1-pentyl)-3,4-dihydro-1H-1,4-benzodiazepine-2,5-dione trifluoracetate,
(±)-1-isopropyl-3-phenyl-4-[succin-2-yl-(1-phenylalanine)]-7-(5-amino-1-pentyl)-3,4-dihydro-1H-1,4-benzodiazepine-2,5-dione trifluoracetate,
(±)-1-methyl-3-phenyl-4-(2-carboxy-1-methylethyl)-7-[N-(2-aminoethane)-3-keto-1-propyl-3-amine]-3,4-dihydro-1H-1,4-benzodiazepine-2,5-dione trifluoracetate,
(±)-1-methyl-3-phenyl-4-(2-carboxy-1-phenylethyl)-7-[N-(2-aminoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-*t*-butoxy)]-7-[N-(2-aminoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-glycine)]-7-[N-(2-aminoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-valine)]-7-[N-(2-aminoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-phenylalanine)]-7-[N-(2-aminoethane)-3-keto-1-propyl-3-amine]-3,4-dihydro-1H-1,4-benzodiazepine-2,5-dione trifluoracetate,
(±)-1,3-diphenyl-4-(2-carboxy-1-methylethyl)-7-[N-(2-aminoethane)-3-keto-1-propyl-3-amine]-3,4-dihydro-1H-1,4-benzodiazepine-2,5-dione trifluoracetate,
(±)-1,3-diphenyl-4-(2-carboxy-1-phenylethyl)-7-[N-(2-aminoethane)-3-keto-1-propyl-3-amine]-3,4-dihydro-1H-1,4-benzodiazepine-2,5-dione trifluoracetate,
(±)-1,3-diphenyl-4-[succin-2-yl-(1-*t*-butoxy)]-7-[N-(2-aminoethane)-3-keto-propyl-3-amine]-3,4-dihydro-1H-1,4-benzodiazepine-2,5-dione trifluoracetate,
(±)-1,3-diphenyl-4-[succin-2-yl-(1-glycine)]-7-[N-(2-aminoethane)-3-keto-1-propyl-3-amine]-3,4-dihydro-1H-1,4-benzodiazepine-2,5-dione trifluoracetate,
(±)-1,3-dipheny-4-[succin-2-yl-(1-valine)]-7-[N-(2-aminoethane)-3-keto-1-propyl-3-amine]-3,4-dihydro-1H-1,4-benzodiazepine-2,5-dione trifluoracetate,
(±)-1,3-diphenyl-4-[succin-2-yl-(1-phenylalanyne)]-7-[N-(2-aminoethane)-3-keto-1-propyl-3-amine]-3,4-dihydro-1H-1,4-benzodiazepine-2,5-dione trifluoracetate,
(±)-1-benzyl-3-phenyl-4-(2-carboxy-1-methylethyl)-7-[N-(2-aminoethane)-3-keto-1-propyl-3-amine]-3,4-dihydro-1H-1,4-benzodiazepine-2,5-dione trifluoracetate,
(±)-1-benzyl-3-phenyl-4-(2-carboxy-1-phenylethyl)-7-[N-(2-aminoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-*t*-butoxy)]-7-[N-(2-aminoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-glycine)]-7-[N-(2-aminoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-valine)]-7-[N-(2-aminoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-phenylalanine)]-7-[N-(2-aminoethane)-3-keto-1-propyl-3-amine]-3,4-dihydro-1H-l4-benzendiazepine-2,5-dione trifluoracetate,
(±)-1-isopropyl-3-phenyl-4-(2-carboxy-1-methylethyl)-7-[N-(2-aminoethane)-3-keto-1-propyl-3-amine]-3,4-dihydro-1H-1,4-benzodiazepine-2,5-dione trifluoracetate,
(±)-1-isopropyl-3-phenyl-4-(2-carboxy-1-phenylethyl)-7-[N-(2-aminoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-*t*-butoxy)]-7-[N-(2-aminoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-glycine)]-7-[N-(2-aminoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-valine)]-7-[N-(2-aminoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-phenylalanine)]-7-[N-(2-aminoethane)-3-keto-1-propyl-3-amine]-3,4-dihydro-1H-1,4-benzodiazeprine-2,5-dione trifluoracetate,
(±)-1-methyl-3-phenyl-4-(2-carboy-1-methylethyl)-7-[3-keto-1-propyl-3-(1-piperizine)]-3,4-dihydro-1H-1,4-benzodiazepine-2,5-dione trifluoracetate,
(±)-1-methyl-3-phenyl-4-(2-carboxy-1-phenylethyl)-7-[3-keto-1-propyl-3-(1-piperizine)]-3,4-dihydro-1H-1,4-benzodiazepine-2,5-dione trifluoracetate,
(±)-1-methyl-3-phenyl-4-[succin-2-yl-(1-*t*-butoxy)]-7-[3-keto-1-propyl-3-(1-piperizine)]-3,4-dihydro-1H-1,4-benzodiazepine-2,5-dione trifluoracetate,
(±)-1-methyl-3-phenyl-4-[succin-2-yl-(1-glycine)]-7-[3-keto-1-propyl-3-(1-piperizine)]-3,4-dihydro-1H-1,4-benzodiazepine-2,5-dione trifluoracetate,
(±)-1-methyl-3-phenyl-4-[succin-2-yl-(1-valine)]-7-[3-keto-1-propyl-3-(1-piperizine)]-3,4-dihydro-1H-1,4-benzodiazepine-2,5-dione trifluoracetate,
(±)-1-methyl-3-phenyl-4-[succin-2-yl-(1-phenylalanine)]-7-[3-keto-1-propyl-3-(1-piperizine)]-3,4-dihydro-1H-1,4-benzodiazepine-2,5-dione trifluoracetate,
(±)-1,3-diphenyl-4-(2-carboxy-1-methylethyl)-7-[3-keto-1-propyl-3-(1-piperizine)]-3,4-dihydro-1H-1,4-benzodiazepine-2,5-dione trifluoracetate,
(±)-1,3-diphenyl-4-(2-carboxy-1-phenylethyl)-7-[3-keto-1-propyl-3-(1-piperizine)]-3,4-dihydro-1H-1,4-benzodiazepine-2,5-dione trifluoracetate,
(±)-1,3-diphenyl-4-[succin-2-yl-(1-*t*-butoxy)]-7-[3-keto-1-propyl-3-(1-piperizine)]-3,4-dihydro-1H-1,4-benzodiazepine-2,5-dione trifluoracetate,
(±)-1,3-diphenyl-4-[succin-2-yl-(1-glycine)]-7-[3-keto-1-propyl-3-(1-piperizine)]-3,4-dihydJro-1H-1,4-benzodiazepine-2,5-dione trifluoracetate,
(±)-1,3-diphenyl-4-[succin-2-yl-(1-valine)]-7-[3-keto-1-propyl-3-(1-piperizine)]-3,4-dihydro-1H-1,4-benzodiazepine-2,5-dione trifluoracetate,
(±)-1,3-diphenyl-4-[succin-2-yl-(1-phenylalanine)]-7-[3-keto-1-propyl-3-(1-piperizine)]-3,4-dihydro-1H-1,4-benzodiazepine-2,5-dione trifluoracetate,
(±)-1-benzyl-3-phenyl-4-(2-carboxy-1-methylethyl)-7-[3-keto-1-propyl-3-(1-piperizine)]-3,4-dihydro-1H-1,4-benzodiazepine-2,5-dione trifluoracetate,
(±)-1-benzyl-3-phenyl-4-(2-carboxy-1-phenylethyl)-7-[3-keto-1-propyl-3-(1-piperizine)]-3,4-dihydro-1H-1,4-benzodiazepine-2,5-dione trifluoracetate,
(±)-1-benzyl-3-phenyl-4-[succin-2-yl-(1-*t*-butoxy)]-7-[3-keto-1-propyl-3-(1-piperizine)]-3,4-dihydro-1H-1,4-benzodiazepine-2,5-dione trifluoracetate,
(±)-1-benzyl-3-phenyl-4-[succin-2-yl-(1-glycine)]-7-[3-keto-1-propyl-3-(1-piperizine)]-3,4-dihydro-1H-1,4-benzodiazepine-2,5-dione trifluoracetate,
(±)-1-benzyl-3-phenyl-4-[succin-2-yl-(1-valine)]-7-[3-keto-1-propyl-3-(1-piperizine)]-3,4-dihydro-1H-1,4-benzodiazepine-2,5-dione trifluoracetate,
(±)-1-benzyl-3-phenyl-4-[succin-2-yl-(1-phenylalanine)]-7-[3-keto-1-propyl-3-(1-piperizine)]-3,4-dihydro-1H-1,4-benzodiazepine-2,5-dione trifluoracetate,
(±)-1-isopropyl-3-phenyl-4-(2-carboxy-1-methylethyl)-7-[3-keto-1-propyl-3-(1-piperizine)]-3,4-dihydro-1H-1,4-benzodiazepine-2,5-dione trifluoracetate,
(±)-1-isopropyl-3-phenyl-4-(2-carboxy-1-phenylethyl)-7-[3-keto-1-propyl-3-(1-piperizine)]-3,4-dihydro-1H-1,4-benzodiazepine-2,5-dione trifluoracetate,
(±)-1-isopropyl-3-phenyl-4-[succin-2-yl-(1-*t*-butoxy)]-7-[3-keto-1-propyl-3-(1-piperizine)]-3,4-dihydro-1H-1,4-benzodiazepine-2,5-dione trifluoracetate,
(±)-1-isopropyl-3-phenyl-4-[succin-2-yl-(1-glycine)]-7-[3-keto-1-propyl-3-(1-piperizine)]-3,4-dihydro-1H-1,4-benzodiazepine-2,5-dione trifluoracetate,
(±)-1-isopropyl-3-phenyl-4-[succin-2-yl-(1-valine)]-7-[3-keto-1-propyl-3-(1-piperizine)]-3,4-dihydro-1H-1,4-benzodiazepine-2,5-dione trifluoracetate,
(±)-1-isopropyl-3-phenyl-4-[succin-2-yl-(1-phenylalanine)]-7-[3-keto-1-propyl-3-(1-piperizine)]-3,4-dihydro-1H-1,4-benzodiazepine-2,5-dione trifluoracetate,
(±)-1-methyl-3-phenyl-1-(2-carboxy-1-methylethyl)-7-[2-(2-aminoethanethiol)-1-ethyl]-3,4-dihydro-1H-1,4-benzodiazepine-2,5-dione trifluoracetate,
(±)-1-methyl-3-phenyl-4-(2-carboxy-1-phenylethyl)-7-[2-(2-aminoethanethiol)-1-ethyl]-3,4-dihydro-1H-1,4-benzodiazepine-2,5-dione trifluoracetate
(±)-1-methyl-3-phenyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-methyl-3-phenyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1-methyl-3-phenyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-methyl-3-phenyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
(±)-1,3-diphenyl-4-(2-carboxy-1-methylethyl)-7-[2-(2-aminoethanethiol)-1-ethyl]-3,4-dihydro-1H-1,4-benzodiazepine-2,5-dione trifluoracetate,
(±)-1,3-diphenyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1,3-diphenyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1,3-diphenyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1,3-diphenyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1,3-diphenyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
(±)-1-benzyl-3-phenyl-4-(2-carboxy-1-methylethyl)-7-[2-(2-aminoethanethiol)-1-ethyl]-3,4-dihydro-1H-1,4-benzodiazepine-2,5-dione trifluoracetate,
(±)-1-benzyl-3-phenyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-benzyl-3-phenyl-4-[succin-2-yl-(1-glycine)]-7-[2-(2-aminoethanethiol)-1-ethyl]-3,4-dihydro-1H-1,4-benzodiazepine-2,5-dione trifluoracetate,
(±)-1-benzyl-3-phenyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-benzyl-3-phenyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
(±)-1-isopropyl-3-phenyl-4-(2-carboxy-1-methylethyl)-7-[2-(2-aminoethanethiol)-1-ethyl]-3,4-dihydro-1H-1,4-benzodiazepine-2,5-dione trifluoracetate,
(±)-1-isopropyl-3-phenyl-4-(2-carboxy-1-phenylethyl)-7-[2-(2-aminoethanethiol)-1-ethyl]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(2-aminoethanethiol)-1-ethyl]-3,4-dihydro-1H-1,4-benzodiazepine-2,5-dione trifluoracetate,
(±)-1-isopropyl-3-phenyl-4-[succin-2-yl-(1-glycine)]-7-[2-(2-aminoethanethiol)-1-ethyl]-3,4-dihydro-1H**-**1,4-benzodiazepine-2,5-dione trifluoracetate,
(±)-1-isopropyl-3-phenyl-4-[succin-2-yl-(1-valine)]-7-[2-(2-aminoethanethiol)-1-ethyl]-3,4-dihydro-1H-1,4-benzodiazepine-2,5-dione trifluoracetate,
(±)-1-isopropyl-3-phenyl-4-[succin-2-yl-(1-phenylalanine)]-7-[2-(2-aminoethanethiol)-1-ethyl]-3,4-dihydro-1H-1,4-benzodiazepine-2,5-dione trifluoracetate,
(±)-1-methyl-3-phenyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-3-phenyl-4-(2-carboxy-1-phenylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-3-phenyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-3-phenyl-4-[succin-2-yl-(1-glycine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-3-phenyl-4-[succin-2-yl-(1-valine)]-7-[4-(2-amino-6-methylpyrimidine-4-oxa-1-butyl]-3,4-dihydro-1H-1,4-benzodiazepine-2,5-dione trifluoracetate,
(±)-1-methyl-3-phenyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
(±)-1,3-diphenyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1,3-diphenyl-4-(2-carboxy-1-phenylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1,3-diphenyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1,3-diphenyl-4-[succin-2-yl-(1-glycine)]-7-[4-(2-amino-6-methylpyrimidine-4-yl)-4-oxa-1-butyl]-3,4-dihydro-1H-1,4-benzodiazepine-2,5-dione trifluoracetate,
(±)-1,3-diphenyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1,3-diphenyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-3-phenyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-3-phenyl-4-(2-carboxy-1-phenylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-3-phenyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-3-phenyl-4-[succin-2-yl-(1-glycine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-3-phenyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-benzyl-3-phenyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-3-phenyl-4-(2-carboxy-1-methylethyl)-7-[4-(2-amino-6-methylpyrimidine-4-yl)-4-oxa-1-butyl]-3,4-dihydro-1H-1,4-benzodiazepine-2,5-dione trifluoracetate.
(±)-1-isopropyl-3-phenyl-4-(2-carboxy-1-phenylethyl)-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-3-phenyl-4-[succin-2-yl-(1-*t*-butoxy)]-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-3-phenyl-4-[succin-2-yl-(1-glycine)]-7-[4-(2-amino-6-methylpyrimidine-4-yl)]-4-oxa-1-butyl]-3,4-dihydro-1H-1,4-benzodiazepine-2,5-dione trifluoracetate,
(±)-1-isopropyl-3-phenyl-4-[succin-2-yl-(1-valine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-isopropyl-3-phenyl-4-[succin-2-yl-(1-phenylalanine)]-7-[4-(2-amino-6-methylpyrimidine-4-yl)-4-oxa-1-butyl]-3,4-dihydro-1H-1,4-benzodiazepine-2,5-dione trifluoracetate,
(±)-1-methyl-3-phenyl-4-(2-carboxy-1-methylethyl)-7-[5-(2-aminopyridine-3-yl)-5-oxa-1-pentyl]-3,4-dihydro-1H-1,4-benzodiazepine-2,5-dione trifluoracetate,
(±)-1-methyI-3-phenyl-4-(2-carboxy-1-phenylethyl)-7-[5-(2-aminopyridine-3-yl)-5-oxa-1-pentyl]-3,4-dihydro-1H-1,4-benzodiazepine-2,5-dione trifluoracetate,
(±)-1-methyl-3-phenyl-4-[succin-2-yl-(1-*t*-butoxy]-7-[5-(2-aminopyridine-3-yl)-5-oxa-1-pentyl]-3,4-dihydro-1H-1,4-benzodiazepine-2,5-dione trifluoracetate,
(±)-1-methyl-3-phenyl-4-[succin-2-yl-(1-glycine)]-7-(5-(2-aminopyridine-3-yl)-5-oxa-1-pentyl]-3,4-dihydro-1H-1,4-benzodiazepine-2,5-dione trifluoracetate,
(±)-1-methyl-3-phenyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-methyl-3-phenyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
(±)-1,3-diphenyl-4-(2-carboxy-1-methylethyl)-7-[5-(2-aminopyridine-3-yl)-5-oxa-1-pentyl]-3,4-dihydro-1H-1,4-benzodiazepine-2,5-dione trifluoracetate,
(±)-1,3-diphenyl-4-(2-carboxy-1-phenylethyl)-7-[5-(2-aminopyridine-3-yl)-5-oxa-1-penty]]-3,4-dihydro-1H-1,4-benzodiazepine-2,5-dione trifluoracetate,
(±)-1,3-diphenyl-4-[succin-2-yl-(1-*t*-butoxy)]-7-[5-(2-aminopyridine-3-yl)-5-oxa-1-pentyl]-3,4-dihydro-1H-1,4-benzodiazepine-2,5-dione trifluoracetate,
(±)-1,3-diphenyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1,3-diphenyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1,3-diphenyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
(±)-1-benzyl-3-phenyl-4-(2-carboxy-1-methylethyl)-7-[5-(2-aminopyridine-3-yl)-5-oxa-1-pentyl]-3,4-dihydro-1H-1,4-benzodiazepine-2,5-dione,
(±)-1-benzyl-3-phenyl-4-(2-carboxy-1-phenylethyl)-7-[5-(2-aminopyridine-3-yl)-5-oxa-1-pentyl]-3,4-dihydro-1H-1,4-benzodiazepine-2,5-dione trifluoracetate,
(±)-1-benzyl-3-phenyl-4-[succin-2-yl-(1-*t*-butoxy)]-7-[5-(2-aminopyridine-3-yl)-5-oxa-1-pentyl]-3,4-dihydro-1H-1,4-benzodiazepine-2,5-dione trifluoracetate,
(±)-1-benzyl-3-phenyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1-benzyl-3-phenyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-benzyl-3-phenyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,
(±)-1-isopropyl-3-phenyl-4-(2-carboxy-1-methylethyl)-7-[5-(2-aminopyridine-3-yl)-5-oxa-1-pentyl]-3,4-dihydro-1H-1,4-benzodiazepine-2,5-dione trifluoracetate,
(±)-1-isopropyl-3-phenyl-4-(2-carboxy-1-phenylethyl)-7-[5-(2-aminopyridine-3-yl)-5-oxa-1-pentyl]-3,4-dihydro-1H-1,4-benzodiazepine-2,5-dione trifluoracetate,
(±)-1-isopropyl-3-phenyl-4-[succin-2-yl-(1-*t*-butoxy)]-7-[5-(2-aminopyridine-3-yl)-5-oxa-1-pentyl]-3,4-dihydro-1H-1,4-benzodiazepine-2,5-dione trifluoracetate,
(±)-1-isopropyl-3-phenyl-4-[succin-2-yl-(1-glycine)]-7-[5-(2-aminopyridine-3-yl)-5-oxa-1-pentyl]-3,4-dihydro-1H-1,4-benzodiazepine-2,5-dione trifluoracetate,
(±)-1-isopropyl-3-phenyl-4-[succin-2-yl-(1-valine)]-7-[5-(2-aminopyridine-3-yl)-5-oxa-1-pentyl]-3,4-dihydro-1H-1,4-benzodiazepine-2,5-dione trifluoracetate,
(±)-1-isopropyl-3-phenyl-4-[succin-2-yl-(1-phenylalanine)]-7-[5-(2-aminopyridine-3-yl)-5-oxa-1-pentyl]-3,4-dihydro-1H-1,4-benzodiazepine-2,5-dione trifluoracetate,

c) 1-methyl-4-(2-carboxyethyl)-7-(5-guanidino-1-pentyl)-3,4-dihydro-1*H*-1,4-benzodiazepine-2,5-dione was prepared by the method described in Example 2. Thus, 18mgs 1-methyl-4-(2-carboxyethyl)-7-(5-amino-1-pentyl)-3,4-dihydro-1*H*-1,4-benzodiazepine-2,5-dione yielded 17mgs (87%) 1-methyl-4-(2-carboxyethyl)-7-(5-guanidino-1-pentyl)-3,4-dihydro-1*H*-1,4-benzodiazepine-2,5-dione trifluoracetate (1/2" C-18 reverse-phase column, eluting with a solvent gradient of 10:90 methanol(0.1% trifluoracetic acid)/ water (0.1% trifluoracetic acid), time 0 to 10 min, to 50:50 methanol(0.1% trifluoracetic acid)/water (0.1% trifluoracetic acid), time 10 min to 40 min, flow=10ml/min, Rₜ=38.5 min, un detection 254nm). HRMS (FAB) molecular ion m/z=390.2132 (cald. C₁₉H₂₇N₅O₄, 390.2182)

Using the above procedure, but substituting the appropriate amino acid for 1-methyl-4-(2-carboxyethyl)-7-(5-amino-1-pentyl)-3,4-dihydro-1*H*-1,4-benzodiazepine-2,5-dione there may be prepared, for example, the following compounds:
1-methyl-4-(2-carboxy-1-methylethyl)-7-(5-guanidino-1-pentyl)-3,4-dihydro-1H-1,4-benzodiazepine-2,5-dione trifluoracetate,
1-methyl-4-(2-carboxy-1-phenylethyl)-7-(5-guanidino-1-pentyl)-3,4-dihydro-1H-1,4-benzodiazepine-2,5-dione trifluoracetate,
(±)-1-methyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-methyl-4-[succin-2-yl-(1-glycine)]-7-(5-guanidino-1-pentyl)-3,4-dihydro-1H-1,4-benzodiazepine-2,5-dione trifluoracetate,
(±)-1-methyl-4-[succin-2-yl-(1-valine)]-7-(5-guanidino-1-pentyl)-3,4-dihydro-1H-1,4-benzodiazepine-2,5-dione trifluoracetate,
(±)-1-methyl-4-[succin-2-yl-(1-phenylalanine)]-7-(5-guanidino-1-pentyl)-3,4-dihydro-1H-1,4-benzodiazepine-2,5-dione trifluoracetate,
1-phenyl-4-(2-carboxy-1-methylethyl)-7-(5-guanidino-1-pentyl)-3,4-dihydro-1H-1,4-benzodiazepine-2,5-dione trifluoracetate,
1-phenyl-4-(2-carboxy-1-phenylethyl)-7-(5-guanidino-1-pentyl)-3,4-dihydro-1H-1,4-benzodiazepine-2,5-dione trifluoracetate,
(±)-1-phenyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-phenyl-4-[succin-2-yl-(1-glycine)]-7-(5-guanidino-1-pentyl)-3,4-dihydro-1H-1,4-benzodiazepine-2,5-dione trifluoracetate,
(±)-1-phenyl-4-[lsuccin-2-yl-(1-valine)]-7-(5-guanidino-1-pentyl)-3,4-dihydro-1H-1,4-benzodiazepine-2,5-dione trifluoracetate,
(±)-1-phenyl-4-[succin-2-yl-(1-phenylalanine)]-7-(5-guanidino-1-pentyl)-3,4-dihydro-1H-1,4-benzodiazepine-2,5-dione trifluoracetate,
1-benzyl-4-(2-carboxy-1-methylethyl)-7-(5-guanidino-1-pentyl)-3,4-dihydro-1H-1,4-benzodiazepine-2,5-dione trifluoracetate,
1-benzyl-4-(2-carboxy-1-phenylethyl)-7-(5-guanidino-1-pentyl)-3,4-dihydro-1H-1,4-benzodiazepine-2,5-dione trifluoracetate,
(±)-1-benzyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-benzyl-4-[succin-2-yl-(1-glycine)]-7-(5-guanidino-1-pentyl)-3,4-dihydro-1H-1,4-benzodiazepine-2,5-dione trifluoracetate,
(±)-1-benzyl-4-[succin-2-yl-(1-valine)]-7-(5-guanidino-1-pentyl)-3,4-dihydro-1H-1,4-benzodiazepine-2,5-dione trifluoracetate,
(±)-1-benzyl-4-[succin-2-yl-(1-phenylalanine)]-7-(5-guanidino-1-pentyl)-3,4-dihydro-1H-1,4-benzodiazepine-2,5-dione trifluoracetate,
1-isopropyl-4-(2-carboxy-1-methylethyl)-7-(5-guanidino-1-pentyl)-3,4-dihydro-1H-1,4-benzodiazepine-2,5-dione trifluoracetate,
1-isopropyl-4-(2-carboxy-1-phenylethyl)-7-(5-guanidino-1-pentyl)-3,4-dihydro-1H-1,4-benzodiazepine-2,5-dione trifluoracetate,
(±)-1-isopropyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-isopropyl-4-[succin-2-yl-(1-glycine)]-7-(5-guanidino-1-pentyl)-3,4-dihydro-1H-1,4-benzodiazepine-2,5-dione trifluoracetate,
(±)-1-isopropyl-4-[succin-2-yl-(1-valine)]-7-(5-guanidino-1-pentyl)-3,4-dihydro-1H-1,4-benzodiazepine-2,5-dione trifluoracetate,
(±)-1-isopropyl-4-[succin-2-yl-(1-phenylalanine)]-7-(5-guanidino-1-pentyl)-3,4-dihydro-1H-1,4-benzodiazepine-2,5-dione trifluoracetate,
1-methyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
1-methyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-methyl-4-[succin-2-yl-(1-*t*-butoxy)-7-[N-(2-guanidinoethane)-3-keto-1-propyl-3-amine]-3,4-dihydro-1H-1,4-benzodiazepine-2,5-dione trifluoracetate,
(±)-1-methyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-methyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1-methyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
1-phenyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
1-phenyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-phenyl-4-[succin-2-yl-(1-*t*-butoxy)]-7-[N-(2-guanidinoethane)-3-keto-1-propyl-3-amine]-3,4-dihydro-1H-1,4-benzodiazepine-2,5-dione trifluoracetate,
(±)-1-phenyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-phenyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1-phenyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
1-benzyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
1-benzyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-benzyl-4-[succin-2-yl-(1-*t*-butoxy)]-7-[N-(2-guanidinoethane)-3-keto-1-propyl-3-amine]-3,4-dihydro-1H-1,4-benzodiazepine-2,5-dione trifluoracetate,
(±)-1-benzyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-benzyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1-benzyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
1-isopropyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
1-isopropyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-isopropyl-4-[succin-2-yl-(1-*t*-butoxy)]-7-[N-(2-guanidinoethane)-3-keto-1-propyl-3-amine]-3,4-dihydro-1H-1,4-benzodiazepine-2,5-dione trifluoracetate,
(±)-1-isopropyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-isopropyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1-isopropyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
1-methyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
1-methyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-methyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-methyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-methyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-methyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
1-phenyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
1-phenyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-phenyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-phenyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-phenyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-phenyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
1-benzyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
1-benzyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-benzyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-benzyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-benzyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-benzyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
1-isopropyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
1-isopropyl-4-(2-carboxy-1-phenylethyl)-7-[3-keko-1-propyl-3-(4-amidino-1-piperizine)]-3,4-dihydro-1H-1,4-benzodiazepine-2,5-dione trifluoracetate,
(±)-1-isopropyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-isopropyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-isopropyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-isopropyl-4-[succin-2-yl-(1-phenylalanine)]-7-[3-keto-1-propyl-3-(4-amidino-1-piperizine)]-3,4-dihydro-1H**-**1,4-benzodiazepine-2,5-dione trifluoracetate,
1-methyl-4-(2-carboxy-1-methylethyl)-7-[2-(2-guanidinoethanethiol)-1-ethyl]-3,4-dihydro-1H-1,4-benzodiazepine-2,5-dione trifluoracetate,
1-methyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-methyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-methyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-methyl-4-[succin-2-yl-(1-valine)]-7-[2-(2-guanidinoethanethiol)-1-ethyl]-3,4-dihydro-1H-1,4-benzodiazepine-2,5-dione trifluoracetate,
(±)-1-methyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
1-phenyl-4-(2-carboxy-1-methylethyl)-7-[2-(2-guanidinoethanethiol)-1-ethyl]-3,4-dihydro-1H-1,4-benzodiazepine-2,5-dione trifluoracetate,
1-phenyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-phenyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-phenyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-phenyl-4-[succin-2-yl-(1-valine)]-7-[2-(2-guanidinoethanethiol)-1-ethyl)-3,4-dihydro-1H-1,4-benzodiazepine-2,5-dione trifluoracetate,
(±)-1-phenyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
1-benzyl-4-(2-carboxy-1-methylethyl)-7-[2-(2-guanidinoethanethiol)-1-ethyl]-3,4-dihydro-1H-1,4-benzodiazepine-2,5-dione trifluoracetate,
1-benzyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-benzyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-benzyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-benzyl-4-[succin-2-yl-(1-valine)]-7-[2-(2-guanidinoethanethiol)-1-ethyl]-3,4-dihydro-1H-1,4-benzodiazepine-2,5-dione trifluoracetate,
(±)-1-benzyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
1-isopropyl-4-(2-carboxy-1-methylethyl)-7-[2-(2-guanidinoethanethiol)-1-ethyl]-3,4-dihydro-1H-1,4-benzodiazepine-2,5-dione trifluoracetate,
1-isopropyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-isopropyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-isopropyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-isopropyl-4-[succin-2-yl-(1-valine)]-7-[2-(2-guanidinoethanethiol)-1-ethyl]-3,4-dihydro-1H-1,4-benzodiazepine-2,5-dione trifluoracetate,
(±)-1-isopropyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
(±)-1-methyl-3-phenyl-4-(2-carboxy-1-methylethyl)-7-(5-guanidino-1-pentyl)-3,4-dihydro-1H-1,4-benzodiazepine-2,5-dione trifluoracetate,
(±)-1-methyl-3-phenyl-4-(2-carboxy-1-phenylethyl)-7-(5-guanidino-1-pentyl)-3,4-dihydro-1H-1,4-benzodiazepine-2,5-dione trifluoracetate,
(±)-1-methyl-3-phenyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-methyl-3-phenyl-4-[succin-2-yl-(1-glycine)]-7-(5-guanidino-1-pentyl)-3,4-dihydro-1H-1,4-benzodiazepine-2,5-dione trifluoracetate,
(±)-1-methyl-3-phenyl-4-[succin-2-yl-(1-valine)]-7-(5-guanidino-1-pentyl)-3,4-dihydro-1H-1,4-benzodiazepine-2,5-dione trifluoracetate,
(±)-1-methyl-3-phenyl-4-[succin-2-yl-(1-phenylalanine)]-7-(5-guanidino-1-pentyl)-3,4-dihydro-1H-1,4-benzodiazepine-2,5-dione trifluoracetate,
(±)-1,3-diphenyl-4-(2-carboxy-1-methylethyl)-7-(5-guanidino-1-pentyl)-3,4-dihydro-1H-1,4-benzodiazepine-2,5-dione trifluoracetate,
(±)-1,3-diphenyl-4-(2-carboxy-1-phenylethyl)-7-(5-guanidino-1-pentyl)-3,4-dihydro-1H-1,4-benzodiazepine-2,5-dione trifluoracetate,
(±)-1,3-diphenyl-4-[succin-2-yl-(1-*t-*butoxy)]-7-(5-guanidino-1-pentyl)-3,4-dihydro-1H-1,4-benzodiazepine-2,5-dione trifluoracetate,
(±)-1,3-diphenyl-4-[succin-2-yl-(1-glycine)]-7-(5-guanidino-1-pentyl)-3,4-dihydro-1H-1,4-benzodiazepine-2,5-dione trifluoracetate,
(±)-1,3-diphenyl-4-[succin-2-yl-(1-valine)]-7-(5-guanidino-1-pentyl)-3,4-dihydro-1H-1,4-benzodiazepine-2,5-dione trifluoracetate,
(±)-1,3-diphenyl-4-[succin-2-yl-(1-phenylalanine)]-7-(5-guanidino-1-pentyl)-3,4-dihydro-1H-1,4-benzodiazepine-2,5-dione trifluoracetate,
(±)-1-benzyl-3-phenyl-4-(2-carboxy-1-methylethyl)-7-(5-guanidino-1-pentyl)-3,4-dihydro-1H-1,4-benzodiazepine-2,5-dione trifluoracetate,
(±)-1-benzyl-3-phenyl-4-(2-carboxy-1-phenylethyl)-7-(5-guanidino-1-pentyl)-3,4-dihydro-1H-1,4-benzodiazepine-2,5-dione trifluoracetate,
(±)-1-benzyl-3-phenyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-benzyl-3-phenyl-4-[succin-2-yl-(1-glycine)]-7-(5-guanidino-1-pentyl)-3,4-dihydro-1H-1,4-benzodiazepine-2,5-dione trifluoracetate,
(±)-1-benzyl-3-phenyl-4-[succin-2-yl-(1-valine)]-7-(5-guanidino-1-pentyl)-3,4-dihydro-1H-1,4-benzodiazepine-2,5-dione trifluoracetate,
(±)-1-benzyl-3-phenyl-4-[succin-2-yl-(1-phenylalanine)]-7-(5-guanidino-1-pentyl)-3,4-dihydro-1H-1,4-benzodiazepine-2,5-dione trifluoracetate,
(±)-1-isopropyl-3-phenyl-4-(2-carboxy-1-methylethyl)-7-(5-guanidino-1-pentyl)-3,4-dihydro-1H-1,4-benzodiazepine-2,5-dione trifluoracetate,
(±)-1-isopropyl-3-phenyl-4-(2-carboxy-1-phenylethyl)-7-(5-guanidino-1-pentyl)-3,4-dihydro-1H-1,4-benzodiazepine-2,5-dione trifluoracetate,
(±)-1-isopropyl-3-phenyl-4-[succin-2-yl-(1-*t*-butoxy)]-7-(5-guanidino-1-pentyl)-3,4-dihydro-1H-1,4-benzodiazepine-2,5-dione trifluoracetate,
(±)-1-isopropyl-3-phenyl-4-[succin-2-yl-(1-glycine)]-7-(5-guanidino-1-pentyl)-3,4-dihydro-1H-1,4-benzodiazepine-2,5-dione trifluoracetate,
(±)-1-isopropyl-3-phenyl-4-[succin-2-yl-(1-valine)]-7-(5-guanidino-1-pentyl)-3,4-dihydro-1H-1,4-benzodiazepine-2,5-dione trifluoracetate,
(±)-1-isopropyl-3-phenyl-4-[succin-2-yl-(1-phenylalanine)]-7-(5-guanidino-1-pentyl)-3,4-dihydro-1H-1,4-benzodiazepine-2,5-dione trifluoracetate,
(±)-1-methyl-3-phenyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
(±)-1-methyl-3-phenyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-*t*-butoxy)]-7-[N-(2-guanidinoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1-methyl-3-phenyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
(±)-1,3-diphenyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
(±)-1,3-diphenyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1,3-diphenyl-4-[succin-2-yl-(1-*t*-butoxy)]-7-[N-(2-guanidinoethane)-3-keto-1-propyl-3-amine]-3,4-dihydro-1H-1,4-benzodiazepine-2,5-dione trifluoracetate,
(±)-1,3-diphenyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1,3-diphenyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1,3-diphenyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
(±)-1-benzyl-3-phenyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
(±)-1-benzyl-3-phenyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-*t*-butoxy)]-7-[N-(2-guanidinoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-benzyl-3-phenyl-4-[succin-2-yl-(1-valine)]-7-[N-(2-guanidinoethane)**-**3-keto-1-propyl-3-amine]-3,4-dihydro-1H-1,4-benzodiazepine-2,5-dione trifluoracetate,
(±)-1-benzyl-3-phenyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
(±)-1-isopropyl-3-phenyl-4-(2-carboxy-1-methylethyl)-7-[N-(2-guanidinoethane)-3-keto-1-propyl-3-amine]-3,4-dihydro-1H-1,4-benzodiazepine-2,5-dione trifluoracetate,
(±)-1-isopropyl-3-phenyl-4-(2-carboxy-1-phenylethyl)-7-[N-(2-guanidinoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-*t-*butoxy)]-7-[N-(2-guanidinoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-glycine)]-7-[N-(2-guanidinoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-valine)]-7-[N-(2-guanidinoethane)-3-keto-1-propyl-3-amine]-3,4-dihydro-1H-1,4-benzodiazepine-2,5-dione trifluoracetate,
(±)-1-isopropyl-3-phenyl-4-[succin-2-yl-(1-phenylalanine)]-7-[N-(2-guanidinoethane)-3-keto-1-propyl-3-amine]-3,4-dihydro-1H-1,4-benzodiazepine-2,5-dione trifluoracetate,
(±)-1-methyl-3-phenyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
(±)-1-methyl-3-phenyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-methyl-3-phenyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-methyl-3-phenyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-methyl-3-phenyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-methyl-3-phenyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
(±)-1,3-diphenyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
(±)-1,3-diphenyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1,3-diphenyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1,3-diphenyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1,3-diphenyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1,3-diphenyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
(±)-1-benzyl-3-phenyl-4-(2-carboxy-1-methylethyl)-7-[3-keto-1-propyl-3-(4-amidino-1-piperizine)]-3,4-dihydro-1H-1,4-benzodiazepine-2,5-dione trifluoracetate,
(±)-1-benzyl-3-phenyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-benzyl-3-phenyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
(±)-1-isopropyl-3-phenyl-4-(2-carboxy-1-methlylethyl)-7-[3-keto-1-propyl-3-(4-amidino-1-piperizine)]-3,4-dihydro-1H-1,4-benzodiazepine-2,5-dione trifluoracetate,
(±)-1-isopropyl-3-phenyl-4-(2-carboxy-1-phenylethyl)-7-[3-keto-1-propyl-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-*t*-butoxy)]-7-[3-keto-1-propyl-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-glycine)]-7-[3-keto-1-propyl-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-valine)]-7-[3-keto-1-propyl-3-(4-amidino-1-piperizine)]-3,4-dihydro-1H-1,4-benzodiazepine-2,5-dione trifluoracetate,
(±)-1-isopropyl-3-phenyl-4-[succin-2-yl-(1-phenylalanine)]-7-[3-keto-1-propyl-3-(4-amidino-1-piperizine)]-3,4-dihydro-1H-1,4-benzodiazepine-2,5-dione trifluoracetate,
(±)-1-methyl-3-phenyl-4-(2-carboxy-1-methylethyl)-7-[2-(2-guanidinoethanethiol)-1-ethyl]-3,4-dihydro-1H-1,4-benzodiazepine-2,5-dione trifluoracetate,
(±)-1-methyl-3-phenyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-methyl-3-phenyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-methyl-3-phenyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-methyl-3-phenyl-4-[succin-2-yl-(1-valine)]-7-[2-(2-guanidinoethanethiol)-1-ethyl]-3,4-dihydro-1H-1,4-benzodiazepine-2,5-dione trifluoracetate,
(±)-1-methyl-3-phenyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
(±)-1,3-diphenyl-4-(2-carboxy-1-methylethyl)-7-[2-(2-guanidinoethanethiol)-1-ethyl]-3,4-dihydro-1H-1,4-benzodiazepine-2,5-dione trifluoracetate,
(±)-1,3-diphenyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1,3-diphenyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1,3-diphenyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1,3-diphenyl-4-[succin-2-yl-(1-valine)]-7-[2-(2-guanidinoethanethiol)-1-ethyl]-3,4-dihydro-1H-1,4-benzodiazepine-2,5-dione trifluoracetate,
(±)-1,3-diphenyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
(±)-1-benzyl-3-phenyl-4-(2-carboxy-1-methylethyl)-7-[2-(2-guanidinoethanethiol)-1-ethyl]-3,4-dihydro-1H-1,4-benzodiazepine-2,5-dione trifluoracetate,
(±)-1-benzyl-3-phenyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-benzyl-3-phenyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-benzyl-3-phenyl-4-[succin-2-yl-(1-valine)]-7-[2-(2-guanidinoethanethiol)-1-ethyl]-3,4-dihydro-1H-1,4-benzodiazepine-2,5-dione trifluoracetate,
(±)-1-benzyl-3-phenyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate,
(±)-1-isopropyl-3-phenyl-4-(2-carboxy-1-methylethyl)-7-[2-(2-guanidinoethanethiol)-1-ethyl]-3,4-dihydro-1H-1,4-benzodiazepine-2,5-dione trifluoracetate,
(±)-1-isopropyl-3-phenyl-4-(2-carboxy-1-phenylethyl)-7-[2-(2-guanidinoethanethiol)-1-ethyl]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(2-guanidinoethanethiol)-1-ethyl]-3,4-dihydro-1H-1,4-benzodiazepine-2,5-dione trifluoracetate,
(±)-1-isopropyl-3-phenyl-4-[succin-2-yl-(1-glycine)]-7-[2-(2-guanidinoethanethiol)-1-ethyl]-3,4-dihydro-1H-1,4-benzodiazepine-2,5-dione trifluoracetate,
(±)-1-isopropyl-3-phenyl-4-[succin-2-yl-(1-valine)]-7-[2-(2-guanidinoethanethiol)-1-ethyl]-3,4-dihydro-1H-1,4-benzoadiazepine-2,5-dione trifluoracetate,
(±)-1-isopropyl-3-phenyl-4-[succin-2-yl-(1-phenylalanine)]-7-[2-(2-guanidinoethanethiol)-1-ethyl]-3,4-dihydro-1H-1,4-benzodiazepine-2,5-dione trifluoracetate.

### Example 4

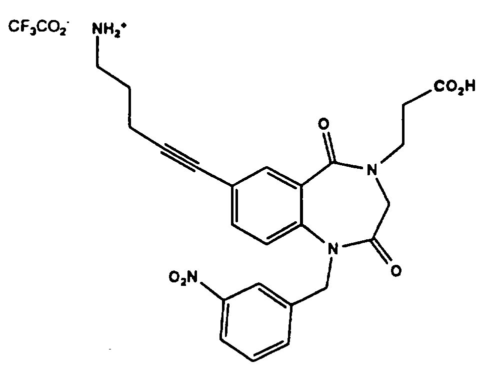

*1-(3-nitrobenzyl)-4-(2-carboxyethyl)-7-(5-amino-1-pentynyl)-3,4-dihydro-1H-1,4-benzodiazepine-2,5-dione trifluoracetate.*

a) N-(2-amino-5-iodobenzoyl)-b-alanine ethyl ester was prepared using the method described in part (b) of Example 1. Thus, from 21.0 grams of 6-iodoisatoïc anhydride (0.73 mol) and 11.3 grams of b-alanine ethyl ester hydrogenchloride (0.73 mol) was prepared 9.1 grams (35%) N-(2-amino-5-iodobenzoyl)-b-alanine ethyl ester (mp=83-85°C, TLC, SiO₂, 1:1 EtOAc/hexane, R_{f}=0.46, un positive). ¹H NMR (CDCl₃, dTMS) 7.57 (1H, d, ⁴J_{HH} = 2Hz, Ar-H *o*-CON), 7.42 (1H, dd, ³J_{HH}=9Hz, ⁴J_{HH}=2Hz, Ar-H *p*-CON), 6.63 (1H, bs, CONH), 6.44 (1H, d, ³J_{HH}=9Hz, Ar-H *m*-CON), 5.57 (2H, bs, NH₂), 4.10 (2H, q, ³J_{HH}=7Hz, OCH₂), 3.63 (2H, q, ³J_{HH}=6Hz, NCH₂), 2.63 (2H, t, ³J_{HH}=6Hz, CH₂CO₂), 1.14 (3H, t, ³J_{HH}=7Hz, CH₃).

b) A magnetically stirred solution of 1.2 grams N-(2-amino-5-iodobenzoyl)-b-alanine ethyl ester (3.35 mmol), 0.43mL 2,6-lutidine (3.7 mmol), 0.713 grams 3-nitrobenyl bromide (3.3 mmol), and 20mL dimethylformamide, under an atmosphere of nitrogen, was heated to 50°C for 24 hours. The reaction mixture was allowed to cool to room temperature, poured over 100mL 10% citric acid, and extracted 3X75mL ether. The combined organics were dried over magnesium sulfate, filtered and concentrated *in vacuo.* The resulting residue was purified by column chromatography, using silica gel, eluting with a solvent gradient of 1:4 ethyl acetate/hexane to 1:1 ethyl acetate/hexane isolating the yellow band (TLC, SiO₂, 1:1 ethyl acetate/hexane, R_{f}=0.66, un positive) characterized as N-[2-(3-nitrobenzyl)-5-iodobenzoyl]-b-alanine ethyl ester (1.1 grams, 66%,mp=104-105°C).

c) To a magnetically stirred solution of 1.0 grams N-[2-(3-nitrobenzyl)-5-iodobenzoyl]-b-alanine ethyl ester (2.0 mmol), 5mL methylene chloride, 0.56mL triethylamine (4.0 mmol) at -30°C under an atmosphere of nitrogen was slowly added, *via* an addition funnel, 0.26mL a-bromoacetylbromide (3.0 mmol) as a solution in 4mL methylene chloride. After 2 hours, the mixture was diluted with 35mL methylene chloride and washed 2X75mL 10% citric acid, 2X75mL sat. sodium bicarbonate, dried over magnesium sulfate, filtered and concentrated *in vacuo.* The resulting residue was dissolved in 5mL dimethylformamide and added, *via* an addition funnel, to a slurry of 72mgs sodium hydride (3.0 mmol) in 1.0mL dimethylformamide cooled to 0°C. The reaction was allowed to warm to room temperature, After 2 hours, the mixture was poured over 100mL of an ice cooled solution of 10% citric acid and extracted 3X75mL ethyl acetate. The combined organics were washed with 1X50mL sat. sodium bicarbonate, dried over magnesium sulfate, and concentrated *in vacuo.* The resulting residue was further purified by column chromatography, using silica gel, eluting with a solvent gradient of 40:60 ethyl acetate/hexane to 70:30 ethyl acetate/hexane to yield 0.45 grams (55%) 1-(3-nitrobenzyl)-4-(2-carboxyethyl)-7-iodo-3,4-dihydro-1*H*-1,4-benzodiazepine-2,5-dione ethyl ester (TLC, SiO₂, 1:1 ethyl acetate/hexane, R_{f}=0.39, un positive) and 0.18 grams of recovered N-[2-(3-nitrobenzyl)-5-iodobenzoyl]-b-alanine ethyl ester. ¹H NMR (CDCl₃, dTMS) 8.09 (1H, d, ⁴J_{HH}=2Hz, *o*-CON), 8.06 [1H, dt, ³J_{HH}=7Hz, ⁴J_{HH}=2Hz, NCH₂(C4-Ar-H)], 8.00 [1H, bs, NCH₂(C2-Ar-H)], 7.73 (1H, dd**,** ³J_{HH}=9Hz, ⁴J_{HH}=2Hz, Ar-H *p*-CON), 7.5-7.4 [2H, m, NCH₂(C5,C6-Ar-H)], 6.88 (1H, d, ³J_{HH}=9Hz, Ar-H *m*-CON), 5.20 (1H, d, J_{HH}=16Hz, NCHHAr), 5.03 (1H, d, J_{HH}=16Hz, NCHHAr), 4.15 (1H, d, J_{HH}=15Hz, NCHHCO), 4.10 (2H, q, ³J_{HH}=7Hz, OCH₂), 3.96 (1H, d, J_{HH}=15Hz, NCHHCO), 3.98-3.92 (2H, m, NCH₂CH₂), 2.71 (1H, dt, J_{HH}=16Hz, ³J_{HH}=8Hz, CHHCO2), 2.63 (1H, dt, J_{HH}=16Hz, ³J_{HH}=8Hz, CHHCO2), 1.22 (3H, t, ³J_{HH}=7Hz, CH₃). ¹³C NMR (CDCl₃, dTMS) 171.2, 168.3, 165.3, 148.4, 141.0, 139.7, 139.0, 138.5, 132.8, 131.1, 129.9, 122.9, 122.8, 121.9, 90.5, 60.8, 52.1, 50.3, 45.2, 32.7, 14.1.

d) 1-(3-nitrobenzyl)-4-(2-carboxyethyl)-7-(N-boc-5-amino-1-pentynyl)-3,4-dihydro-1*H*-1,4-benzodiazepine-2,5-dione ethyl ester was prepared using the method described in part (f) of Example 1. Thus, from 0.205 grams of 1-(3-nitrobenzyl)-4-(2-carboxyethyl)-7-iodo-3,4-dihydro-1*H*-1,4-benzodiazepine-2,5-dione ethyl ester was prepared 168.2mgs (57%) 1-(3-nitrobenzyl)-4-(2-carboxyethyl)-7-(N-boc-5-amino-1-pentynyl)-3,4-dihydro-1*H*-1,4-benzodiazepine-2,5-dione ethyl ester (TLC, SiO₂, 1:1 ethyl acetate/hexane, R_{f}=0.36, un positive). ¹H NMR (CDCl₃, dTMS) 8.09 [1H, dt, ³J_{HH}=7Hz, ⁴J_{HH}=2Hz, NCH₂(C4-Ar-H)], 8.02 [1H, bs, NCH₂(C2-Ar-H)], 7.81 (1H, d, ⁴J_{HH}=2Hz,*o*-CON), 7.50-7.35 [3H, m, NCH₂(C5,C6-Ar-H), Ar-H *p-*CON]*,* 7.04 (1H, d, ³J_{HH}=9Hz, Ar-H *m*-CON), 5.22 (1H, d, J_{HH}=16Hz, NCHHAr), 5.04 (1H, d, J_{HH}=16Hz, NCHHAr), 4.70 (1H, bs, BocNH), 4.40 (1H, d, J_{HH}=12Hz, NCHHCO), 4.12 (2H, q, ³J_{HH}=7Hz, OCH₂), 3.96 (1H, d, J_{HH}=12Hz, NCHHCO), 4.0-3.8 (2H, m, NCH₂CH₂), 3.23 (2H, q, ³J_{HH}=6Hz, BocNHCH₂), 2.72 (1H, dt, J_{HH}=17Hz, ³J_{HH}=8Hz, CHHCO₂), 2.62 (1H, dt, J_{HH}=17Hz, ³J_{HH}=8Hz, CHHCO2), 2.41 (2H, t, ³J_{HH}=7Hz, CJCCH₂), 1.74 (2H, p, ³J_{HH}=7Hz, CH₂CH₂CH₂), 1.40 (9H, s, *t*-Bu), 1.24 (3H, t, ³J_{HH}=7Hz, CH₃). ¹³C NMR (CDCl₃, dTMS) 171.2, 168.4, 166.2, 155.9, 148.4, 138.6, 138.2, 134.9, 134.2, 132.8, 129.9, 129.5, 122.7, 122.4, 122.0, 121.2, 91.4, 79.2, 60.8, 52.2, 50.4, 45.2, 39.8, 32.7, 28.7, 28.4, 16.8, 14.1.

e) 1-(3-nitrobenzyl)-4-(2-carboxyethyl)-7-(5-amino-1-pentynyl)-3,4-dihydro-1H-1,4-benzodiazepine-2,5-dione trifluoracetate was prepared using the method described in part (g) of example 1. Thus, 84 mgs 1-(3-nitrobenzyl)-4-(2-carboxyethyl)-7-(N-boc-5-amino-1-pentynyl)-3,4-dihydro-1*H*-1,4-benzodiazepine-2,5-dione ethyl ester (0.142 mmol) yielded 75mgs (91%) 1-(3-nitrobenzyl)-4-(2-carboxyethyl)-7-(5-amino-1-pentynyl)-3,4-dihydro-1H-1,4-benzodiazepine-2,5-dione trifluoracetate (1/2" C-18 reverse-phase column, eluting with a solvent gradient of 10:90 methanol (0.1% trifluoracetic acid)/ water (0.1% trifluoracetic acid), time 0 to 10 min, to 50:50 methanol(0.1% trifluoracetic acid)/water (0.1% trifluoracetic acid), time 10 min to 40 min, flow=10ml/min, Rₜ=45.1 min, un detection 254nm). HRMS (FAB) molecular ion m/z=465.1744 (cald. C₂₄H₂₅N₄O₆, 465.1774) ¹H NMR (CD₃ CN, dTMS) 8.07 [1H, dt, ⁴J_{HH}=2Hz, ³J_{HH}=7Hz, NCH₂(C4-Ar-H)], 8.02 [1H, bs, NCH₂(C2-Ar-H)], 7.73 (1H, d, ⁴J_{HH}=2Hz,*o*-CON), 7.60-7.50 [3H, m, NCH₂(C5,C6-Ar-H), Ar-H *p*-CON], 7.25 (1H, d, ³J_{HH}=9Hz, Ar-H *m*-CON), 5.40 (1H, d, J_{HH}=15Hz, NCHHAr), 5.00 (1H, d, J_{HH}=15Hz, NCHHAr), 4.13 (1H, d, J_{HH}=12Hz, NCHHCO), 4.0-3.6 (3H, m, NCHHCO, NCH₂CH₂), 3.1 (2H, bs, NH₃CH₂), 2.65 (2H, t, ³J_{HH}=7Hz, CH₂CO₂), 2.41 (2H, t, ³J_{HH}=7Hz, C∫CCH₂), 1.9 (2H, p, ³J_{HH}=7Hz, CH₂CH₂CH₂).

### Example 5

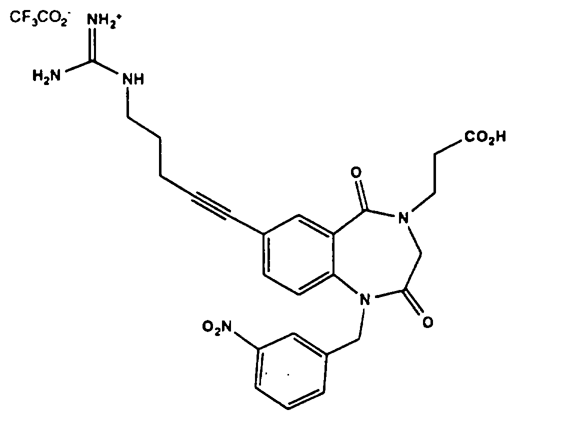

*1-(3-nitrobenzyl)-4-(2-carboxyethyl)-7-(5-guanidino-1-pentynyl)-3,4-dihydro-1H-1,4-benzodiazepine-2,5-dione trifluoracetate.*

1-(3-nitrobenzyl)-4-(2-carboxyethyl)-7-(5-guanidino-1-pentynyl)-3,4-dihydro-1*H*-1,4-benzodiazepine-2,5-dione trifluoracetate was prepared using the method described in Example 2. Thus, 30mgs of 1-(3-nitrobenzyl)-4-(2-carboxyethyl)-7-(5-amino-1-pentynyl)-3,4-dihydro-1H-1,4-benzodiazepine-2,5-dione trifluoracetate yielded 14mgs (44%) of 1-(3-nitrobenzyl)-4-(2-carboxyethyl)-7-(5-guanidino-1-pentynyl)-3,4-dihydro-1*H*-1,4-benzodiazepine-2,5-dione trifluoracetate (1/2" C-18 reverse-phase column, eluting with a solvent gradient of 20:80 methanol(0.1% trifluoracetic acid)/ water (0.1% trifluoracetic acid), time 0 to 10 min, to 70:30 methanol(0.1% trifluoracetic acid)/water (0.1% trifluoracetic acid), time 10 min to 40 min, flow=10ml/min, Rₜ=33.8 min, un detection 254nm). HRMS(FAB) molecular ion m/z=507.2007 (cald. C₂₅H₂₇N₆O₆, 507.1992) ¹H NMR (CD₃ CN/CD₃OD, dTMS) 8.04 [1H, d, ³J_{HH}=7Hz, NCH₂(C4-Ar-H)], 7.98 [1H, bs, NCH₂(C2-Ar-H)], 7.68 (1H, d, ⁴J_{HH}=2Hz,*o*-CON), 7.60-7.40 [3H, m, NCH₂(C5,C6-Ar-H), Ar-H *p*-CON], 7.25 (1H, d, ³J_{HH}=9Hz, Ar-H *m*-CON), 5.37(1H, d, J_{HH}=15Hz, NCHHAr), 5.00 (1H, d, J_{HH}=15Hz, NCHHAr), 4.13 (1H, d, J_{HH}=12Hz, NCHHCO), 4.0-3.6 (3H, m, NCHHCO, NCH₂CH₂), 3.24 (2H, bs, H₂N(H₂N=)NHCH₂), 2.65 (2H, t, CH₂CO₂), 2.41 (2H, t, C∫CCH₂), 1.9 (2H, p, ³J_{HH}=7Hz, CH₂CH₂CH₂).

### Example 6

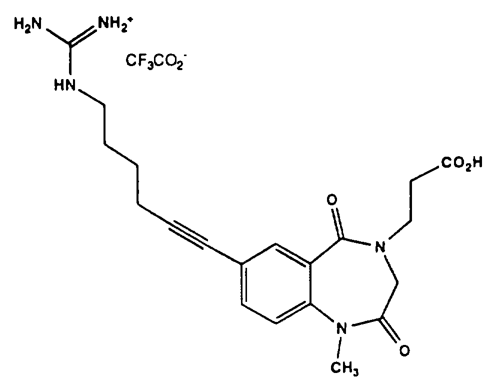

*1-(methyl)-4-(2-carboxyethyl)-7-(6-guanidino-1-hexynyl)-3,4-dihydro-1H-1,4-benzodiazepine-2,5-dione trifluoracetate.*

a) N-(2-methylamino-5-iodobenzoyl)-b-alanine ethyl ester was prepared using the method described in part (c) of Example 1. Thus, 0.5grams N-(2-amino-5-iodobenzoyl)-b-alanine ethyl ester yielded 204.5 mgs (39%) of N-(2-methylamino-5-iodobenzoyl)-b-alanine ethyl ester (TLC, SiO₂, 1:1 ethyl acetate/hexane R_{f}=0.67) and 253.2mgs of a 7:1 mixture of N-(2-amino-5-iodobenzoyl)-b-alanine ethyl ester and N-(2-dimethylamino-5-iodobenzoyl)-b-alanine ethyl ester (TLC, SiO₂, 1:1 ethyl acetate/hexane R_{f}=0.46, un positive). ¹H NMR (CDCl₃ , dTMS) 7.6-7.5 (2H, m, Ar-H *o*,*p*-CON), 7.43 (1H, bs, NHMe), 6.62 (1H, bt, CONH), 6.42 (1H, d, ³J_{HH}=9Hz, Ar-H *m-*CON)*,* 4.18 (2H, q, ³J_{HH}=7Hz, OCH₂), 3.63 (2H, q, ³J_{HH}=6Hz, NCH₂), 2.81 (3H, d, ³J_{HH}=5Hz, NCH₃), 2.61 (2H, t, ³J_{HH}=6Hz, CH₂CO₂), 1.28 (3H, t, ³J_{HH}=7Hz, CH₃).

b) 1-methyl-4-(2-carboxyethyl)-7-iodo-3,4-dihydro-1*H*-1,4-benzodiazepine-2,5-dione ethyl ester was prepared using the method described in part (d) of Example 1. Thus, 1.9 grams of N-(2-methylamino-5-iodobenzoyl)-b-alanine ethvl ester yielded 0.91 grams (43%) of 1-methyl-4-(2-carboxyethyl)-7-iodo-3,4-dihydro-1*H*-1,4-benzodiazepine-2,5-dione ethyl ester (TLC, SiO₂, 1:1 ethyl acetate/hexane R_{f}=0.36, un positive). ¹H NMR (CDCl₃, dTMS) 8.14 (1H, d, ⁴J_{HH}=2Hz, Ar-H *o*-CON), 7.79 (1H, dd, ⁴J_{HH}=2Hz, ³J_{HH}=9Hz, Ar-H *p*-CON), 6.92 (1H, d, ³J_{HH}=9Hz, Ar-H *m*-CON), 4.13 (2H, q, ³J_{HH}=7Hz, OCH₂), 4.01 (1H, d, J_{HH}=15Hz, COCHHN), 3.90 (2H, dt, ³J_{HH}=8Hz, J_{HH}=2Hz, NCH₂), 3.87 (1H, d, J_{HH}=15Hz, COCHHN), 3.34 (3H, s, NCH₃), 2.74 (1H, dt, J_{HH}=17Hz, ³J_{HH}=7Hz, CHHCO₂), 2.63 (1H, dt, J_{HH}=17Hz, ³J_{HH}=7Hz, CHHCO₂), 1.24 (3H, t, ³J_{HH}=7Hz, CH₃).

c) To a magnetically stirred slurry of 2.94 grams lithium aluminum hydride (0.077 mol) in 150mL ether cooled to 0°C was slowly added, *via* an addition funnel, 6 grams of 5-cyano-1-pentyne (0.065 mol) in 15mL ether. The reaction mixture was allowed to warm to room temperature. After 1 hour, the reaction was cooled to 0°C and quenched with 3.1 mL water, 233 mL 20% sodium hydroxide, 10.83mL water added in succession. After an additional 30 minutes, the slurry was filtered, washing the salts with 40mL ether. The ether was distilled at atmospheric pressure to leave an oil, which was further purified by Kugelrohr distillation (oven temp.*ca*. 145°C) to yield 3.99 grams 6-amino-1-hexyne (64%).

d) To a magnetically stirred solution of 3.99 grams of 6-amino-1-hexyne (0.0413 mol) in 50mL tetrhydrofuran was added 9.1 grams of di-*tert*-butyl dicarbonate (0.042 mol). After 2 hours, the reaction mixture was concentrated *in vacuo* and the resulting residue chromatographed, using silica gel, eluting with 1:1 ether/hexane to yield 3.4 grams (42%) N-boc-6-amino-1-hexyne (TLC, SiO₂, 1:1 ether/hexane, R_{f}=0.5, ninhydrin char). ¹H NMR (CDCl₃, dTMS) 4.55 (1H, bs, BocNH), 3.13 (2H, bq, ³J_{HH}=5Hz, BocNHCH₂), 2.21 (2H, dt, ⁴J_{HH}=³Hz, ³J_{HH}=7Hz, CJCCH₂), 1.95 (1H, t, ⁴J_{HH}=3Hz, CJCH), 1.6 (4H, m, CH₂CH₂CH₂CH₂), 1.42 (9H, s, *t*-Bu).

e) 1-(methyl)-4-(2-carboxyethyl)-7-(N-boc-6-amino-1-hexynyl)-3,4-dihydro-1*H*-1,4-benzodiazepine-2,5-dione ethyl ester was prepared using the method described in part (f) of Example 1. Thus, 0.126 grams of 1-methyl-4-(2-carboxyethyl)-7-iodo-3,4-dihydro-1H-1,4-benzodiazepine-2,5-dione ethyl ester and 0.119 grams of N-boc-6-amino-1-hexyne yielded 0.124 grams (84%) 1-(methyl)-4-(2-carboxyethyl)-7-(N-boc-6-amino-1-hexynyl)-3,4-dihydro-1*H*-1,4-benzodiazepine-2,5-dione ethyl ester (TLC, SiO₂, 1:1 ethyl acetate/hexane, R_{f}=0.36, un positive). ¹H NMR (CDCl₃, dTMS) 7.84 (1H, d, ⁴J_{HH}=2Hz, Ar-H *o*-CON), 7.47 (1H, dd, ⁴J_{HH}=2Hz, ³J_{HH}=9Hz, Ar-H *p*-CON), 7.07 (1H, d, ³J_{HH}=9Hz, Ar-H *m*-CON), 4.60 (1H, bs, NH), 4.12 (2H, q, ³J_{HH}=7Hz, OCH₂), 4.0 (1H, d, J_{HH}=15Hz, NCHHCO), 3.90 (2H, t, ³J_{HH}=7Hz, NCH₂), 3.84 (1H, d, J_{HH}=15Hz, NCHHCO), 3.26 (3H, s, NCH₃), 3.15 (2H, q, ³J_{HH}=7Hz, BocNHCH₂), 2.73 (1H, dt, J_{HH}=16Hz, ³J_{HH}=9Hz, CHHCO₂), 2.63 (1H, dt, J_{HH}=16Hz, ³J_{HH}=9Hz, CHHCO₂), 2.40 (2H, t, ³J_{HH}=6Hz, C∫CCH₂), 1.62 (4H, m, CH₂CH₂CH₂CH₂), 1.24 (3H, t, ³J_{HH}=7Hz, CH₃).

f) 1-(methyl)-4-(2-carboxyethyl)-7-(6-amino-1-hexynyl)-3,4-dihydro-1*H*-1,4-benzodiazepine-2,5-dione trifluoracetate was prepared using the method described in part (g) of Example 1. Thus, 62mgs of 1-(methyl)-4-(2-carboxyethyl)-7-(N-boc-6-amino-1-hexynyl)-3,4-dihydro-1*H*-1,4-benzodiazepine-2,5-dione ethyl ester yielded 50mgs (83%) 1-(methyl)-4-(2-carboxyethyl)-7-(6-amino-1-hexynyl)-3,4-dihydro-1*H*-1,4-benzodiazepine-2,5-dione trifluoracetate (1/2" C-18 reverse-phase column, eluting with a solvent gradient of 20:80 methanol(0.1% trifluoracetic acid)/ water (0.1% trifluoracetic acid), time 0 to 10 min, to 70:30 methanol(0.1% trifluoracetic acid)/water (0.1% trifluoracetic acid), time 10 min to 40 min, flow=10ml/min, Rₜ=26.2 min, un detection 254nm). HRMS (FAB) molecular ion m/z=358.1761 (cald. C₁₉H₂₄N₃O₄, 358.1767). ¹H NMR (D₂O,) 7.47 (1H, d, ⁴J_{HH}=2Hz, Ar-H *o*-CON), 7.37 (1H, dd, ⁴J_{HH}=2Hz, ³J_{HH}=9Hz, Ar-H *p*-CON), 7.12 (1H, d, ³J_{HH}=9Hz, Ar-H *m*-CON), 3.95-3.8 (2H, m, NCHHCO, NCHHCH₂), 3.63 (1H, d, J_{HH}=15Hz, NCHHCO), 3.52 (1H, dt, J_{HH}=15Hz, ³J_{HH}=5Hz, NCHHCO₂), 3.16 (3H, s, NCH₃), 2.85 (2H, t, ³J_{HH}=7Hz, NH₃CH₂), 2.6-2.4 (2H, m, CH₂CO₂), 2.28 (2H, t, ³J_{HH}=6Hz, C∫CCH₂), 1.62 & 1.47 (each 2H, p, ³J_{HH}=6Hz, CH₂CH₂CH₂CH₂).

g) 1-(methyl)-4-(2-carboxyethyl)-7-(6-guanidino-1-hexynyl)-3,4-dihydro-1*H*-1,4-benzodiazepine-2,5-dione trifluoracetate was prepared by the method described in Example 2. Thus, from 25mgs 1-(methyl)-4-(2-carboxyethyl)-7-(6-amino-1-hexynyl)-3,4-dihydro-1*H*-1,4-benzodiazepine-2,5-dione trifluoracetate was prepared 22mgs (71%) of 1-(methyl)-4-(2-carboxyethyl)-7-(6-guanidino-1-hexynyl)-3,4-dihydro-1*H*-1,4-benzodiazepine-2,5-dione trifluoracetate (1/2" C-18 reverse-phase column, eluting with a solvent gradient of 20:80 methanol(0.1% trifluoracetic acid)/ water (0.1% trifluoracetic acid), time 0 to 10 min, to 70:30 methanol(0.1% trifluoracetic acid)/water (0.1% trifluoracetic acid), time 10 min to 40 min, flow=10ml/min, Rₜ=28.6 min, un detection 254nm). HRMS (FAB) molecular ion m/z=400.1956 ( cald. C₂₀H₂₆N₅O₄, 400.1985). ¹H NMR (D₂O,) 7.58 (1H, d, ⁴J_{HH}=2Hz, Ar-H *o*-CON), 7.43 (1H, dd, ⁴J_{HH}=2Hz, ³J_{HH}=9Hz, Ar-H *p*-CON), 7.20 (1H, d, ³J_{HH}=9Hz, Ar-H *m*-CON), 4.05-3.95 (2H, m, NCHHCO₂, NCHHCH₂), 3.71 (1H, d, J_{HH}=15Hz, NCHHCO), 3.56 (1H, dt, J_{HH}=15Hz, ³J_{HH}=5Hz, NCHHCO₂), 3.19 (3H, s, NCH₃), 3.06 (2H, t, ³J_{HH}=7Hz, H₂N(H₂N=)CNHCH₂), 2.7-2.5 (2H, m, CH₂CO₂), 2.32 (2H, t, ³J_{HH}=6Hz, C∫CCH₂), 1.60 & 1.49 (each 2H, p, ³J_{HH}=6Hz, CH₂CH₂CH₂CH₂).

### Example 7

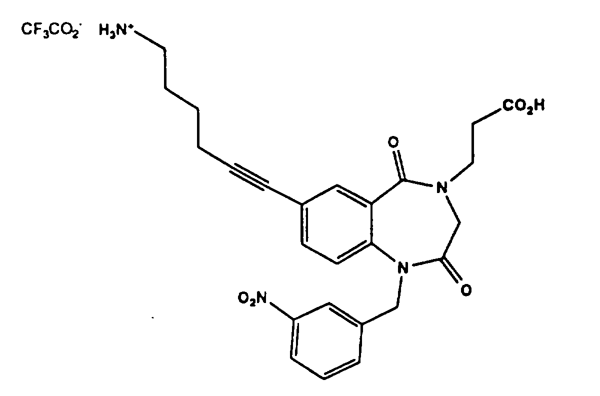

*1-(3-nitrobenzyl)-4-(2-carboxyethyl)-7-(6-amino-1-hexynyl)-3,4-dihydro-1H-1,4-benzodiazepine-2,5-dione trifuroacetate.*

a) 1-(3-nitrobenzyl)-4-(2-carboxyethyl)-7-(N-boc-6-amino-1-hexynyl)-3,4-dihydro-1*H*-1,4-benzodiazepine-2,5-dione ethyl ester was prepared using the method described in part (f) of Example 1. Thus, from 0.26 grams of 1-(3-nitrobenzyl)-4-(2-carboxyethyl)-7-iodo-3,4-dihydro-1*H*-1,4-benzodiazepine-2,5-dione ethyl ester and 0.19 grams of N-boc-6-amino-1-hexyne was prepared 226.3mgs (78%) 1-(3-nitrobenzyl)-4-(2-carboxyethyl)-7-(N-boc-6-amino-1-hexynyl)-3,4-dihydro-1*H*-1,4-benzodiazepine-2,5-dione ethyl ester (TLC, SiO₂, 1:1 ethyl acetate/hexane, R_{f}=0.34, un positive). ¹H NMR (CDCl₃, dTMS) 8.09 [1H, dt, ³J_{HH}=7Hz, ⁴J_{HH}=2Hz, NCH₂(C4-Ar-H)], 8.02 [1H, bs, NCH₂(C2-Ar-H)], 7.83 (1H, d, ⁴J_{HH}=2Hz,*o*-CON), 7.50-7.35 [3H, m, NCH₂(C5,C6-Ar-H), Ar-H *p*-CON], 7.05 (1H, d, ³J_{HH}=8Hz, Ar-H *m*-CON), 5.22 (1H, d, J_{HH}=16Hz, NCHHAr), 5.02 (1H, d, J_{HH}=16Hz, NCHHAr), 4.60 (1H, bs, BocNH), 4.20-4.08 (3H, m, NCHHCO, OCH₂), 4.0-3.8 (3H, m, NCH₂CH_{2,} NCHHCO ), 3.16 (2H, q, ³J_{HH}= 6Hz, BocNHCH₂), 2.72 (1H, dt, J_{HH}=16Hz, ³J_{HH}=8Hz, CHHCO₂), 2.62 (1H, dt, J_{HH}=16Hz, ³J_{HH}=8Hz, CHHCO2), 2.39 (2H, t, ³J_{HH}=6Hz, C∫CCH₂), 1.60 (4H, bs, CH₂CH₂CH₂CH₂), 1.41 (9H, s, *t*-Bu), 1.24 (3H, t, ³J_{HH}=7Hz, CH₃).

b) 1-(3-nitrobenzyl)-4-(2-carboxyethyl)-7-(6-amino-1-hexynyl)-3,4-dihydro-1*H*-1,4-benzodiazepine-3,5-dione trifluoracetate was prepared using the method described in part (g) of Example 1. Thus, from 0.11 grams of 1-(3-nitrobenzyl)-4-(2-carboxyethyl)-7-(N-boc-6-amino-1-hexynyl)-3,4-dihydro-1*H*-1,4-benzodiazepine-2,5-dione ethyl ester was prepared 90mgs (79%) of 1-(3-nitrobenzyl)-4-(2-carboxyethyl)-7-(6-amino-1-hexynyl)-3,4-dihydro-1*H*-1,4-benzodiazepine-2,5-dione trifluoracetate (1/2" C.18 reverse-phase column, eluting with a solvent gradient of 20:80 methanol(0.1% trifluoracetic acid)/ water (0.1% trifluoracetic acid), time 0 to 10 min, to 70:30 methanol(0.1% trifluoracetic acid)/water (0.1% trifluoracetic acid), time 10 min to 40 min, flow=10ml/min, Rₜ=39.2 min, un detection 254nm). HRMS (FAB) molecular ion m/z=479.1911 ( cald. C₂₅H₂₇N₄O₆, 479.1931). ¹H NMR (D₂O) 7.7 [1H, bd, ³J_{HH}=8Hz, NCH₂(C4-Ar-H)], 7.58 [1H, bs, NCH₂(C2-Ar-H)], 7.41 (1H, d, ⁴J_{HH}=2Hz,*o*-CON), 7.25-7.05 [3H, m, NCH₂(C5,C6-Ar-H), Ar-H *p*-CON], 7.01 (1H, d, ³J_{HH}=9Hz, Ar-H *m*-CON), 5.09 (1H, d, J_{HH}=16Hz, NCHHAr), 4.8 (1H, d, NCHHAr, overlapping with HOD), 4.00 (1H, d, J_{HH}=14Hz, NCHHCO), 3.9-3.65 (2H, m, NCHHCO, NCHHCH₂), 3.6-3.5 (1H, m, NCHHCH₂), 2.83 (2H, t, ³J_{HH}=7Hz, NH₃CH₂), 2.51 (2H, t, ³J_{HH}=7Hz, CH₂CO₂), 2.17 (2H, t, ³J_{HH}=7Hz, C∫CCH₂), 1.57 & 1.40 (each 2H, p, ³J_{HH}=7Hz, CH₂CH₂CH₂CH₂).

### Example 8

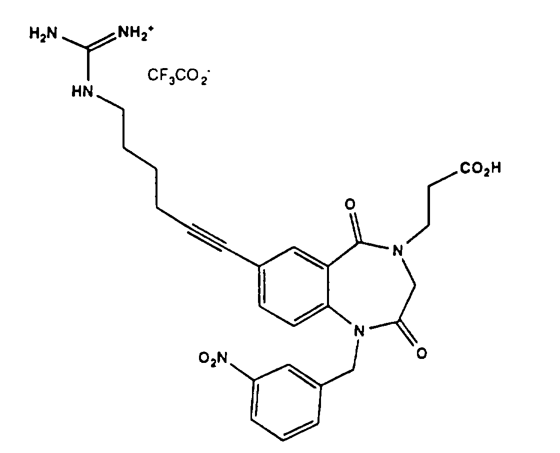

*1-(3-nitrobenzyl)-4-(2-carboxyethyl)-7-(6-guanidino-1-hexynyl)-3,4-dihydro-1H-1,4-benzodiazepine-2,5-dione trifluoracetate.*

1-(3-nitrobenzyl)-4-(2-carboxyethyl)-7-(6-guanidino-1-hexinyl)-3,4-dihydro-1*H*-1,4-benzodiazepine-2,5-dione trifluoracetate was prepared using the method described in Example 2. Thus, 45mgs of 1-(3-nitrobenzyl)-4-(2-carboxyethyl)-7-(6-amino-1-hexynyl)-3,4-dihydro-1*H*-1,4-benzodiazepine-2,5-dione trifluoracetate yielded 25mgs (52%) of 1-(3-nitrobenzyl)-4-(2-carboxyethyl)-7-(6-guanidino-1-hexynyl-3,4-dihydro-1*H*-1,4-benzodiazepine-2,5-dione trifluoracetate (1/2" C-18 reverse-phase column, eluting with a solvent gradient of 20:80 methanol(0.1% trifluoracetic acid)/ water (0.1% trifluoracetic acid), time 0 to 10 min, to 70:30 methanol(0.1% trifluoracetic acid)/water (0.1% trifluoracetic acid), time 10 min to 40 min, flow=10ml/min, Rₜ=41.3 min, un detection 254nm). HRMS (FAB) molecular ion m/z=521.2161 (cald. C₂₆H₂₈N₆O₆, 521.2148). ¹H NMR (D₂O) 7.86 [1H, dt, ⁴J_{HH}=3Hz, ³J_{HH}=6Hz, NCH₂(C4-Ar-H)], 7.64 [1H, bs, NCH₂(C2-Ar-H)], 7.46 (1H, d, ⁴J_{HH}=2Hz,*o*-CON), 7.30-7.23 [3H, m, NCH₂(C5,C6-Ar-H), Ar-H *p*-CON], 7.18 (1H, d, ³J_{HH}=8Hz, Ar-H *m*-CON), 5.30 (1H, d, J_{HH}=16Hz, NCHHAr), 4.7 (1H, NCHHAr, overlapping with HOD), 4.03 (1H, d, J_{HH}=14Hz, NCHHCO), 3.85 (1H, dt, J_{HH}=14Hz, ³J_{HH}=7Hz, NCHHCH₂), 3.74 (1H, d, J_{HH}=14Hz, NCHHCO), 3.55 (1H, dt, J_{HH}=14Hz, ³J_{HH}=7Hz, NCHHCH₂), 2.97 (2H, t, ³J_{HH}=7Hz, H₂N(H₂N=)NHCH₂), 2.49 (2H, t, ³J_{HH}=7Hz, CH₂CO₂), 2.21 (2H, t, ³J_{HH}=7Hz, C∫CCH₂), 1.48 & 1.38 (each 2H, p, ³J_{HH}=7Hz, CH₂CH₂CH₂CH₂).

### Example 9

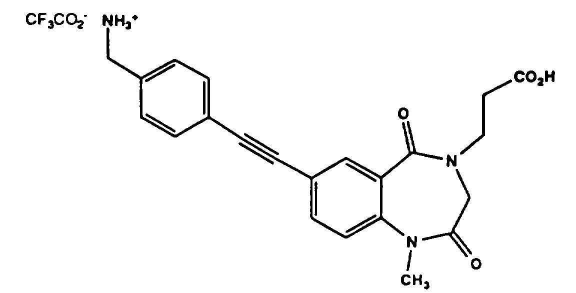

*1-(methyl)-4-(2-carboxyethyl)-7-[4-(aminoethyl)phenyl]ethynyl-3,4-dihydro-1H-1,4-benzodiazepine-2,5-dione trifluoracetate.*

a) 1-(methyl)-4-(2-carboxyethyl)-7-(4-cyanophenyl)ethynyl-3,4-dihydro-1*H*-1,4-benzodiazepine-2,5-dione ethyl ester was prepared using the method employed in part (f) of Example 1. Thus, from 0.41 grams of 1-methyl-2-(carboxyethyl)-7-iodo-3,4-dihydro-1*H*-1,4-benzodiazepine-2,5-dione ethyl ester and 0.127 grams of (4-cyanophenyl)ethyne (Gilbert, J.C.; Weerasooriya, *U.J.Org.Chem.*1979, 44, 4997-98) was prepared 0.205 grams of 1-(methyl)-4-(2-carboxyethyl)-7-(4-cyanophenyl)ethynyl-3,4-dihydro-1*H*-1,4-benzodiazepine-2,5-dione ethyl ester (mp=160-161°C, TLC, SiO2, 1:1 ethyl acetate/hexane, Rf=0.30, un positive). ¹H NMR (CDCl₃, dTMS) 7.98 (1H, d, ⁴J_{HH}=2Hz, Ar-H*o*-CON), 7.65-750 (5H, m, Ar-H *p*-CON, Ar-H *o,p*-CN), 7.17 (1H, d, ³J_{HH}= 9Hz, *m*-CON), 4.09 (2H, q, ³J_{HH}=7Hz, OCH₂), 4.02 (1H, d, J_{HH}=15Hz, NCHHCO), 3.89 (2H, t, ³J_{HH}=7Hz, NCH₂CH₂), 3.86 (1H, d, J_{HH}=15Hz, NCHHCO), 3.34 (3H, s, NCH₃), 2.72 (1H, dt, J_{HH}=16Hz, ³J_{HH}=7Hz, CHHCO₂), 2.62 (1H, dt, J_{HH}=16Hz, ³J_{HH}=7Hz, CHHCO₂), 1.20 (3H, t, 3J_{HH}=7Hz, CH₃). ¹³C NMR (CDCl₃, dTMS) 171.2, 168.6, 166.2, 141.0, 134.8, 134.4, 132.0, 128.8, 127.5, 121.1, 119.7, 118.3, 111.8, 91.7, 88.9, 60.3, 52.1, 45.1, 32.7, 31.5, 14.1.

b) A magnetically stirred solution of 3mL toluene, 6.8mgs dicobalt octacarbonyl (0.02 mmol) cooled to -20°C was saturated with triethylsilane *via* a gas bubbler. 0.1 grams of 1-(methyl)-4-(2-carboxyethyl)-7-(4-cyanophenyl)ethynyl-3,4-dihydro-1*H*-1,4-benzodiazepine-2,5-dione ethyl ester (0.24 mmol) was added as a solution in 0.5mL toluene and the reaction mixture heated to 60°C for 20 hours (Murai, T. *et al., Tetrahedron Lett.* 1985, 26, 5145-48). The mixture was allowed to cool to room temperature and concentrated *in vacuo.* The resulting residue was diluted with methanol and 2mL of 2N sodium hydroxide was added. After 30 minutes, the mixture was neutralized with 2mL acetic acid, concentrated in vacuo, diluted with 2mL of a 1:1 methanol/water solution and purified by high-pressure liquid chromatography (1/2" C-18 reverse-phase column, eluting with a solvent gradient of 20:80 methanol(0.1% trifluoracetic acid)/ water (0.1% trifluoracetic acid), time 0 to 10 min, to 70:30 methanol(0.1% trifluoracetic acid)/ water (0.1% trifluoracetic acid), time 10 min to 40 min, flow=10ml/min, Rₜ=36.8 min, un detection 254nm) to yield 30.0 mgs (25%) of 1-(methyl)-4-(2-carboxyethyl)-7-[4-(aminomethyl)phenyl]ethynyl-.3,4-dihydro-1*H*-1,4-benzodiazepine-2,5-dione trifluoracetate. ¹H NMR (CD₃OD) 7.68 (1H, d, ⁴J_{HH}=2Hz, Ar-H*o*-CON), 7.48 (1H, dd, ⁴J_{HH}=2Hz, ³J_{HH}=9Hz, Ar-H *p*-CON) 7.4-7.3 (4H, m, Ar-H *o,p*-CN), 7.17 (1H, d, *m*-CON), 4.02 (2H, s, ArCH₂N), 4.0-3.7 (4H, m, NCH₂CO, NCH₂CH₂), 3.0 (3H, s, NCH₃) 2.6 (2H, m, CH₂CO₂).

Using the above procedure, but substituting the appropriate 3,4-dihydro-1*H*-1,4-benzodiazepine-2,5-dione nitrile for 1-(methyl)-4-(2-carboxyethyl)-7-(4-cyanophenyl)ethynyl-3,4-dihydro-1*H*-1,4-benzodiazepine-2,5-dione ethyl ester there may be prepared, for example, the following compounds:
1-methyl-4-(2-carboxy-1-methylethyl)-7-[2-(3-aminomethylphenyl)-1-ethyne]-3,4-dihydro-1H-1,4-benzodiazepine-2,5-dione,
1-methyl-4-(2-carboxy-1-phenylethyl)-7-[2-(3-aminomethylphenyl)-1-ethyne]-3,4-dihydro-1H-1,4-benzodiazepine-2,5-dione trifluoracetate,
(±)-1-methyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-methyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1-methyl-4-[succin-2-yl-(1-valine)]-7-[2-(3-aminomethylphenyl)-1-ethyne]-3,4-dihydro-1H-1,4-benzodiazepine-7,5-dione trifluoracetate,
(±)-1-methyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
1-phenyl-4-(2-carboxy-1-methylethyl)-7-[2-(3-aminomethylphenyl)-1-ethyne]-3,4-dihydro-1H-1,4-benzodiazepine-2,5-dione trifluoracetate,
1-phenyl-4-(2-carboxy-1-phenylethy 1)-7-[2-(3-aminomethylphenyl)-1-ethyne]-3,4-dihydro-1H-1,4-benzodiazepine-2,5-dione trifluoracetate,
(±)-1-phenyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-phenyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1-phenyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1-phenyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
1-benzyl-4-(2-carboxy-1-methylethyl)-7-[2-(3-aminomethylphenyl)-1-ethyne]-3,4-dihydro-1H-1,4-benzodiazepine-2,5-dione trifluoracetate,
1-benzyl-4-(2-carboxy-1-phenylethyl)-7-[2-(3-aminomethylphenyl)-1-ethyne]-3,4-dihydro-1H-1,4-benzodiazepine-2,5-dione triuoracetate,
(±)-1-benzyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-benzyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1-benzyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1-benzyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
1-isopropyl-4-(2-carboxy-1I-methylethyl)-7-[2-(3-aminomethylphenyl)-1-ethyne]-3,4-dihydro-1H-1,4-benzodiazepine-2,5-dione trifluoracetate,
1-isopropyl-4-(2-carboxy-1-phenylethyl)-7-[2-(3-aminomethylphenyl)-1-ethyne]-3,4-dihydro-1H-1,4-benzodiazepine-2,5-dione trifluoracetate,
(±)-1-isopropyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-isopropyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1-isopropyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1-isopropyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
1-methyl-4-(2-carboxy-1-methylethyl)-7-[2-(4-aminomethylthiophenol)-1-ethyne]-3,4-dihydro-1H-1,4-benzodiazepine-2,5-dione trifluoracetate,
1-methyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-methyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1-methyl-4-[succin-2-yl-(1-glycine)]-7-[2-(4-aminomethylthiophenol)-1-ethyne]-3,4-dihydro-1H-1,4-benzodiazepine-2,5-dione trifluoracetnte,
(±)-1-methyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1-methyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
1-phenyl-4-(2-carboxy-1-methylethyl)-7-[2-(4-aminomethylthiophenol)-1-ethyne]-3,4-dihydro-1H-1,4-benzodiazepine-2,5-dione trifluoracetate,
1-phenyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-phenyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1-phenyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1-phenyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1-phenyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
1-benzyl-4-(2-carboxy-1-methylethyl)-7-[2-(4-aminomethylthiophenol)-1-ethyne]-3,4-dihydro-1H-1,4-benzodiazepine-2,5-dione trifluoracetate,
1-benzyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-benzyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1-benzyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1-benzyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1-benzyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
1-isopropyl-4-(2-carboxy-1-methylethyl)-7-[2-(4-aminomethylthiophenol)-1-ethyne]-3,4-dihydro-1H-1,4-benzodiazepine-2,5-dione trifluoracetate,
1-isopropyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-isopropyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1-isopropyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1-isopropyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1-isopropyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
(±)-1-methyl-3-phenyl-4-(2-carboxy-1-methylethyl)-7-[2-(3-aminomethylphenyl)-1-ethyne]-3,4-dihydro-1H-1,4-benzodiazepine-2,5-dione trifluoracetate,
(±)-1-methyl-3-phenyl-4-(2-carboxy-1-phenylethyl)-7-[2-(3-aminomethylphenyl)-1-ethyne]-3,4-dihydro-1H-1,4-benzodiazepine-2,5-dione trifluoracetate,
(±)-1-methyl-3-phenyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-methyl-3-phenyl-4-[succin-2-yl-(1-glycine)]-7-[2-(3-aminomethylphenyl)-1-ethyne]-3,4-dihydro-1 H-1,4-benzodiazepine-2,5-dione trifluoracetate,
(±)-1-methyl-3-phenyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1-methyl-3-phenyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
(±)-1,3-diphenyl-4-(2-carboxy-1-methylethyl)-7-[2-(3-aminomethylphenyl)-1-ethyne]-3,4-dihydro-1H-1,4-benzodiazepine-2,5-dione trifluoracetate,
(±)-1,3-diphenyl-4-(2-carboxy-1-phenylethyl)-7-[2-(3-aminomethylphenyl)-1-ethyne]-3,4-dihydro-1H-1,4-benzodiazepine-2,5-dione trifluoracetate,
(±)-1,3-diphenyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1,3-diphenyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1,3-diphenyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1,3-diphenyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
(±)-1-benzyl-3-phenyl-4-(2-carboxy-1-methylethyl)-7-[2-(3-aminomethylphenyl)-1-ethyne]-3,4-dihydro-1H-1,4-benzodiazepine-2,5-dione trifluoracetate,
(±)-1-benzyl-3-phenyl-4-(2-carboxy-1-phenylethyl)-7-[2-(3-aminomethylphenyl)-1-ethyne]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-benzyl-3-phenyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1-benzyl-3-phenyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1-benzyl-3-phenyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
(±)-1-isopropyl-3-phenyl-4-(2-carboxy-1-methylethyl)-7-[2-(3-aminomethylphenyl)-1-ethyne]-3,4-dihydro-1H-1,4-benzodiazepine-2,5-dione trifluoracetate,
(±)-1-isopropyl-3-phenyl-4-(2-carboxy-1-phenylethyl)-7-[2-(3-aminomethylphenyl)-1-ethyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(3-aminomethylphenyl)-1-ethyne]-3,4-dihydro-1H-1,4-benzodiazepine-2,5-dione trifluoracetate,
(±)-1-isopropyl-3-phenyl-4-[succin-2-yl-(1-glycine)]-7-[2-(3-aminomethylphenyl)-1-ethyne]-3,4-dihydro-1H-1,4-benzodiazepine-2,5-dione trifluoracetate,
(±)-1-isopropyl-3-phenyl-4-[succin-2-yl-(1-valine)]-7-[2-(3-aminomethylphenyl)-1-ethyne]-3,4-dihydro-1H-1,4-benzodiazepine-2,5-dione trifluoracetate,
(±)-1-isopropyl-3-phenyl-4-[succin-2-yl-(1-phenylalanine)]-7-[2-(3-aminomethylphenyl)-1-ethyne]-3,4-dihydro-1H-1,4-benzodiazepine-2,5-dione trifluoracetate,
(±)-1-methyl-3-phenyl-4-(2-carboxy-1-methylethyl)-7-[2-(4-aminomethylthiophenol)-1-ethyne]-3,4-dihydro-1H-1,4-benzodiazepine-2,5-dione trifluoracetate,
(±)-1-methyl-3-phenyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-methyl-3-phenyl-4-[succin-2-yl-(1-*t*-buoxy)]-7-[2-(4-aminomethylthiophenol)-1-ethyne]-3,4-dihydro-1H-1,4-benzodiazepine-2,5-dione trifluoracetate,
(±)-1-methyl-3-phenyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1-methyl-3-phenyl-4-[succin-2-yl-(1-valine)]-7-[2-(4-aminomethylthiophenol)-1ethyne]-3,4-dihydro-1H-1,4-benzodiazepine-2,5-dione trifluoracetate,
(±)-1-methyl-3-phenyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
(±)-1,3-diphenyl-4-(2-carboxy-1-methylethyl)-7-[2-(4-aminomethylthiophenol)-1-ethyne]-3,4-dihydro-1H-1,4-benzodiazepine-2,5-dione trifluoracetate,
(±)-1,3-diphenyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1,3-diphenyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1,3-diphenyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1,3-diphenyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1,3-diphenyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
(±)-1-benzyl-3-phenyl-4-(2-carboxy-1-methylethyl)-7-[2-(4-aminomethylthiophenol)-1-ethyne]-3,4-dihydro-1H-1,4-benzodiazepine-2,5-dione trifluoracetate,
(±)-1-benzyl-3-phenyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1-benzyl-3-phenyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1-benzyl-3-phenyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1-benzyl-3-phenyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate,
(±)-1-isopropyl-3-phenyl-4-(2-carboxy-1-methylethyl)-7-[2-(4-aminomethylthiophenol)-1-ethyne]-3,4-dihydro-1H-1,4-benzodiazepine-2,5-dione trifluoracetate,
(±)-1-isopropyl-3-phenyl-4-(2-carboxy-1-phenylethyl)-7-[2-(4-aminomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(4-aminomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-glycine)]-7-[2-(4-aminomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-valine)]-7-[2-(4-aminomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-phenylalanine)]-7-[2-(4-aminomethylthiophenol)-1-ethyne]-3,4-dihydro-1H-1,4-benzodiazepine-2,5-dione trifluoracetate.

### Example 10

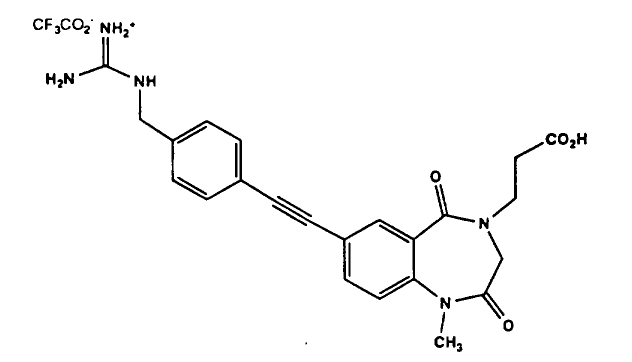

*1-(methyl)-4-(2-carboxyethyl)-7-(4-(guanidinomethyl)phenyl]ethynyl-3,4-dihydro-1H-1,4-benzodiazepine-2,5-dione trifluoracetate.*

1-(methyl)-4-(2-carboxyethyl)-7-[4-(guanidinomethyl)phenyl]ethynyl-3,4-dihydro-1*H*-1,4-benzodiazepine-2,5-dione trifluoracetate was prepared using the method described in example 2. Thus, from 15 mgs of 1-(methyl)-4-(2-carboxyethyl)-7-[4-(aminomethyl)phenyl]ethynyl-3,4-dihydro-1*H*-1,4-benzodiazepine-2,5-dione trifluoracetate was prepared 7mgs (37%) of 1-(methyl)-4-(2-carboxyethyl)-7-[4-(guanidinomethyl)phenyl]ethynyl-3,4-dihydro-1*H*-1,4-benzodiazepine-2,5-dione trifluoracetate (1/2" C-18 reverse-phase column, eluting with a solvent gradient of 20:80 methanol(0,1% trifluoracetic acid)/ water (0.1% trifluoracetic acid), time 0 to 10 min, to 70:30 methanol(0.1% trifluoracetic acid)/water (0.1% trifluoracetic acid), time 10 min to 40 min, flow=10ml /min, Rₜ=39.6 min, un detection 254nm). ¹H NMR (CD₃OD, D₂O) 7.50 (1H, d, ⁴J_{HH}= 2Hz, Ar-H*o*-CON), 7.38 (1H, bd, ³J_{HH}=9Hz, Ar-H *p*-CON) 7.20 (1H, d, ³J_{HH}=9Hz, *m*-CON), 7.15-7.0 (4H, m, Ar-H *o*,*p*-CN), 4.23 (2H, s, ArCH₂NHC(=NH₂)NH₂), 3.9-3.8 (2H, m, NCHHCO, NCHHCH₂), 3.72 (1H, d, 2J_{HH}=16Hz, NCHHCO), 358 (1H, m, NCHHCH₂), 3.12 (3H, s, NCH₃) 2.53 (2H, m, CH₂CO₂).

Using the above procedure, but substituting the appropriate amino acid for 1-(methyl)-4-(2-carboxyethyl)-7-[4-(aminomethyl)phenyl]ethynyl-3,4-dihydro-1*H*-1,4-benzodiazepine-2,5-dione trifluoracetate there may be prepared, for example, the following compounds:
1-methyl-4-(2-carboxy-1-methylethyl)-7-[2-(3-guanidinomethylphenyl)-1-ethyne]-3,4-dihydro-1H-1,4-benzodiazepine-2,5-dione,
1-methyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-methyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-methyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-methyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-methyl-4-[succin-2-yl-(1-phenylalanine)]-7-[2-(3-guanidinomethylphenyl)-1-ethyne]-,3,4-dihydro-1H-1,4-benzodiazepine-2,5-dione trifluoracetate,
1-phenyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
1-phenyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-phenyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-phenyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-phenyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-phenyl-4-[succin-2-yl-(1-phenylalanine)]-7-[2-(3-guanidinomethylphenyl)-1-ethyne]-3,4-dihydro-1H-1,4-benzodiazepine*-*2,5-dione trifluoracetate,
1-benzyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
1-benzyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-benzyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-benzyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-benzyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-benzyl-4-[succin-2-yl-(1-phenylalanine)]-7-[2-(3-guanidinomethylphenyl)-1-ethyne]-3,4-dihydro-1H-1,4-benzodiazepine-2,5-dione trifluoracetate,
1-isopropyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
1-isopropyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-isopropyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-isopropyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-isopropyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-isopropyl-4-[succin-2-yl-(1-phenylalanine)]-7-[2-(3-guanidinomethylphenyl)-1-ethynel-3,4-dihydro-1H-1,4-benzodiazepine-2,5-dione trifluoracetate,
1-methyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
1-methyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-methyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-methyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-methyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-methyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate,
1-phenyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
1-phenyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-phenyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-phenyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-phenyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-phenyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate,
1-benzyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
1-benzyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-benzyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-benzyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-benzyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-benzyl-4-[succin-2-yl-(1-phenylalanine)]-7-[2-(4-guanidinomethylthiophenol)-1-ethyne]-3,4-dihydro-1H-1,4-benzodiazepine-7,5-dione trifluoracetate,
1-isopropyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
1-isopropyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-isopropyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-isopropyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-isopropyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-isopropyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate,
(±)-1-methyl-3-phenyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
(±)-1-methyl-3-phenyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-methyl-3-phenyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-methyl-3-phenyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-methyl-3-phenyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-methyl-3-phenyl-4-[succin-2-yl-(1-phenylalanine)]-7-[2-(3-guanidinomethylphenyl)-1-ethyne]-3,4-dihydro-1H-1,4-benzodiazepine-2,5-dione trifluoracetate,
(±)-1,3-diphenyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
(±)-1,3-diphenyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1,3-diphenyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1,3-diphenyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1,3-diphenyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1,3-diphenyl-4-[succin-2-yl-(1-phenylalanine)]-7-[2-(3-guanidinomethylphenyl)-1-ethyne]-3,4-dihydro-1H-1,4-benzodiazepine-2,5-dione trifluoracetate,
(±)-1-benzyl-3-phenyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
(±)-1-benzyl-3-phenyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-benzyl-3-phenyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-benzyl-3-phenyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-benzyl-3-phenyl-4-[succin-2-yl-(1-phenylalanine)]-7-[2-(3-guanidinomethylphenyl)-1-ethyne]-3,4-dihydro-1H-1,4-benzodiazepine-2,5-dione trifluoracetate,
(±)-1-isopropyl-3-phenyl-4-(2-carboxy-1-methylethyl)-7-[2-(3-guanidinomethylphenyl)-1-ethyne]-3,4-dihydro-1H-1,4-benzodiazepine-2,5-dione trifluoracetate,
(±)-1-isopropyl-3-phenyl-4-(2-carboxy-1-phenylethyl)-7-[2-(3-guanidinomethylphenyl)-1-ethyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(3-guanidinomethylphenyl)-1-ethyne]-3,4-dihydro-1H-1,4-benzodiazepine-2,5-dione trifluoracetate,
(±)-1-isopropyl-3-phenyl-4-[succin-2-yl-(1-glycine)]-7-[2-(3-guanidinomethylphenyl)-1-ethyne]-3,4-dihydro-1H-1,4-benzodiazepine-2,5-dione trifluoracetate,
(±)-1-isopropyl-3-phenyl-4-[succin-2-yl-(1-valine)]-7-[2-(3-guanidinomethylphenyl)-1-ethyne]-3,4-dihydro-1H-1,4-benzodiazepine-2,5-dione trifluoracetate,
(±)-1-isopropyl-3-phenyl-4-[succin-2-yl-(1-phenylalanine)]-7-[2-(3-guanidinomethylphenyl)-1-ethyne]-3,4-dihydro-1H-1,4-benzodiazepine-2,5-dione trifluoracetate,
(±)-1-methyl-3-phenyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
(±)-1-methyl-3-phenyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-methyl-3-phenyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-methyl-3-phenyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-methyl-3-phenyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-methyl-3-phenyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate,
(±)-1,3-diphenyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
(±)-1,3-diphenyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1,3-diphenyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1,3-diphenyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1,3-diphenyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1,3-diphenyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate,
(±)-1-benzyl-3-phenyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
(±)-1-benzyl-3-phenyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-benzyl-3-phenyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-benzyl-3-phenyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-benzyl-3-phenyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-benzyl-3-phenyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate,
(±)-1-isopropyl-3-phenyl-4-(2-carboxy-1-methylethyl)-7-[2-(4-guanidinomethylthiophenol)-1-ethyne]-3,4-dihydro-1H-1,4-benzodiazepine-2,5-dione trifluoracetate,
(±)-1-isopropyl-3-phenyl-4-(2-carboxy-1-phenylethyl)-7-[2-(4-guanidinomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-*t*-butoxy)]-7-[2-(4-guanidinomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-glycine)]-7-[2-(4-guanidinomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-valine)]-7-[2-(4-guanidinomethylthiophenol)-1-ethyne]-3,4-dihydro-1H-1,4-benzodiazepine-2,5-dione trifluoracetate,
(±)-1-isopropyl-3-phenyl-4-[succin-2-yl-(1-phenylalanine)]-7-[2-(4-guanidinomethylthiophenol)-1-ethyne]-3,4-dihydro-1H-1,4-benzodiazepine-2,5-dione trifluoracetate.

### Example 11

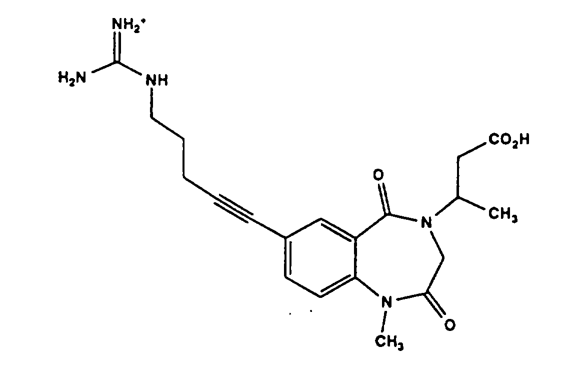

*1-methyl-4-(2-carboxy-1****-****methylethyl)****-****7-(5-guanidino****-****1-pentynyl)-3,4-dihydro-1H-1,4,benzodiazepine-2,5-dione trifluoracetate.*

a) Methyl N-(2-amino-5-iodobenzoyl)-3-amino-3-methylpropanate was prepared using the method described in part (b) of Example 1. Thus, 1.72 grams of 5-iodoisatoic anhydride and 1.73 grams of methyl 3-aminobutanoate p-tosylate yielded 0.65 grams (30%) methyl N-(2-amino-5-iodobenzoyl)-3-aminobutanoate (TLC, SiO₂, 1:1 EtOAc/hexane, R_{f}=0.63, un positive).

b) Methyl N-(2-methylamino-5-iodobenzoyl)-3-amino-3-methylypropanoate was prepared using the method described in part (c) of Example 1. Thus, 0.32 grams of methyl N-(2-amino-5-iodobenzoyl)-3-aminobutanoate yielded 0.14 grams (43%) of methyl N-(2-methylamino-5-iodobenzoyl)-3-aminobutanoate (TLC, SiO₂, 1:1 EtOAc/hexane, R_{f}=0.85, un positive).

c) 1-methyl-4-(3-methoxy-3-oxobutan-2-yl)-7-iodo-3,4-dihydro-1H-1,4-benzo-diazapine-2,5-dione was prepared using the method described in part (d) of Example 1. Thus, 0.25 grams of N-(2-methylamino-5-iodobenzoyl)-3-aminobutanoate yielded 0.08 grams (29%) of 1-methyl-4-(3-methoxy-3-oxobutan-2-yl)-7-iodo-3,4-dihydro-1H-1,4-benzo-diazapine-2,5-dione (TLC, SiO₂, 1:1 EtOAc/hexane, R_{f}=0.33, un positive).

d) 1-methyl-4-(3-methoxy-3-oxobutan-2-yl)-7-[5-(N-Boc)-amino-1-pentyne]-3,4-dihydro-1H-1,4-benzodiazepine-2,5-dione was prepared using the method described in part (f) of Example 1. Thus, 0.08 grams of 1-methyl-4-(3-methoxy-3-oxobutan-2-yl)-7-iodo-3,4-dihydro-1H-1,4-benzodiazapine-2,5-dione yielded 0.061 grams (68%) of 1-methyl-4-(3-methoxy-3-oxobutan-2-yl)-7-[5-(N-Boc)-amino-1-pentyne]-3,4-dihydro-1H-1,4-benzodiazepine-2,5-dione (TLC, SiO₂, 1:1 EtOAc/hexane, R_{f}=0.24, un positive).

e) 1-methyl-4-(2-carboxy-1-methylethyl)-7-(5-amino-1-pentyne)-3,4-dihydro-1H-1,4-benzodiazepine-2,5-dione trifluoracetate was prepared using the method described in part (g) of Example 1. Thus, 61mgs of 1-methyl-4-(3-methoxy-3-oxobutan-2-yl)-7-[5-(N-Boc)-amino-1-pentyne]-3,4-dihydro-1H-1,4-benzodiazepine-2,5-dione yielded 40 mgs (87%) of 1-methyl-4-(2-carboxy-1-methylethyl)-7-(5-amino-1-pentyne)-3,4-dihydro-1H-1,4-benzodiazepine-2,5-dione trifluoracetate (1/2" C-18 reverse-phase column, eluting with a solvent gradient of 10:90 methanol(0.1% trifluoracetic acid)/water(0.1% trifluoracetic acid), time 0 to 10 min, to 50:50 methanol(0.1% trifluoracetic acid)/water(0.1% trifluoracetic acid), time 10 min to 40 min, flow 10ml /min, Rₜ=31.2 min, un detection 254nM). HRMS (FAB) molecular ion m/z=358.1765 (cald. C₁₉H₂₄N₃O₄, 358.1767)

f) 1-methyl-4-(2-carboxy-1-methylethyl)-7-(5-guanidino-1-pentyne)-3,4-dihydro-1H-1,4-benzodiazepine-2,5-dione trifluoracetate was prepared using the method described in Example 2. Thus, 15 mgs of 1-methyl-4-(2-carboxy-1-methylethyl)-7-(5-amino-1-pentyne)-3,4-dihydro-1H-1,4-benzodiazepine-2,5-dione trifluoracetate yielded 10mgs (67%) of 1-methyl-4-(2-carboxy-1-methylethyl)-7-(5-guanidino-1-pentyne)-3,4-dihydro-1H-1,4-benzodiazepine-2,5-dione trifluoracetate (1/2" C-18 reverse-phase column, eluting with a solvent gradient of 10:90 methanol(0.1% trifluoracetic acid)/water(0.1% trifluoracetic acid), time 0 to 10 min, to 50:50 methanol(0.1%, trifluoracetic acid)/water(0.1% trifluoracetic acid), time 10 min to 40 min, flow 10ml/min,Rₜ=36.9 min, un detection 254nM). HRMS (FAB) molecular ion m/z=400.1999 (cald. C₂₀H₂₆N₅O₄, 400.1986).

### Example 12

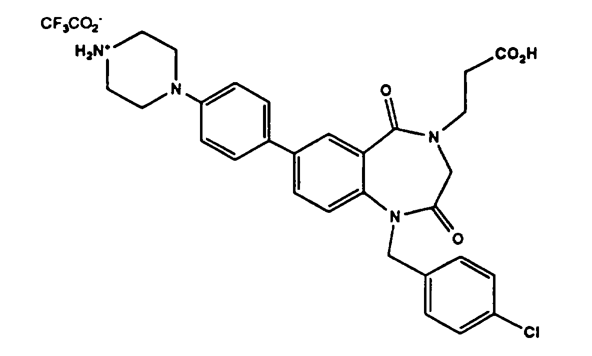

*1-(4-chlorophenyl)methyl-4-(2-carboxyethyl)-7-(4-(1-piperidin)-phenyl)-3,4-dihydro-1H-1,4-benzodiazapine-2,5-dione trifluoroacetate.*

a) Over a period of 15 minutes samples of iodine (8.62 grams, 0.034mol) and silver benzoate (7.78 grams, 0.034mol) were added in portions to a magnetically stirred solution of 5 grams of 1-phenylpiperazine in 80mL acetic acid. The mixture stirred for 1 hour. The solvent was evaporated and the residue partioned between 150mL diethyl ether and 150mL 2N sodium hydroxide. The organic layer was washed with 100mL water, 100mL brine, then dried over potassium carbonate and concentrated *in vacuo* to yield 1.0 gram (11%) of 1-(4-iodophenyl)-piperzine (110-115°C). The material obtained was used without further purification. ¹H NMR (CDCl₃, dTMS) 7.51 (2H, d, ³J_{HH}=9Hz, *m*-ArH), 6.69 (2H, d, ³J_{HH}=9Hz, *o*-ArH), 3.17 (4H, m), 3.04 (4H, m). ¹³C NMR (CDCl₃) 151.36, 137.73, 118.05, 81.35, 49.92, 45.95.

b) The crude material obtained from part (a) (1 gram, 3.47mmol) was dissolved in dry THF (10mL) and a catalytic amount of dimethylamino-pyridine (100mgs) and di-*tert*-butyl dicarbonate (0.9 grams, 4.1mmol, 1.2 molar excess) were added, monitoring the reaction by TLC (SiO₂, 1:1 EtOAc/hexane, R_{f}(product)=0.88). After 12 hours (overnight) the reaction was diluted with diethyl ether and extracted 1X75mL water, 1X75mL 1N HCl, 1X75mL sat. sodium bicarbonate, 1X75mL brine, dried over potassium carbonate, filtered and concentrated *in vacuo*. The material was further purified by filtering through a packet of SiO₂ using 1:1 diethyl ether/hexane as the eluting solvent to yield 1-(4-iodophenyl)-4-(N-Boc)-piperzine (1 grm, 77%, 142-143°C). ¹H NMR (CDCl₃, dTMS) 7.54 (2H, d, ³J_{HH}=8Hz, *m*-ArH), 6.69 (2H, d, ³J_{HH}=8Hz, *o*-ArH), 3.58 (4H, t, ³J_{HH}=5Hz), 3.04 (4H, t, ³J_{HH}=5Hz) 1.48 (9H, s, Bu^{t}).

c) A magnetically, stirred solution of N-(2-amino-5-iodobenzoyl)-3-aminoproprionate (8.8 grams, 23.4mmol), prepared using the procedure shown in part (b) of Example 1, dimethylformamide, 2,6-lutidine (1.5 molar equiv., 35.1mmol, 4.25mL) and 4-chlorobenzyl chloride (1.5 molar equiv., 35.1mmol, 5.96 grams) was heated to 100°C for 24 hours. The mixture was allowed to cool to room temperature and concentrated *in vacuo.* The resulting residue was dissolved in 150mL methylene chloride and washed with 2X100mL 10% citric acid, 1X100mL water, dried over sodium sulfate, filtered and concentrated *in vacuo.* The solid obtained was dissolved in a minimum volumn of methylene chloride and a 5 fold excess of hexanes added. The solution was allowed to stand overnight in the refrigerator (5-7°C) to yield 7.56 grams of N-(2-(*p*-chlorobenzyl)amino-5-iodobenzoyl)-3-aminoproprionate (153-155°C). Concentration of the mother liquor and repeating the steps for crystallization yielded a second crop (3.32 grams) for a total 92% isolated yield of the desired product. ¹H NMR (CDCl₃, dTMS) 8.10 (1H, bt, ³J_{HH}=5Hz, NH), 7.60 (1H, d, ⁴J_{HH}=2Hz, C6-H), 7.44 (1H, dd, ³J_{HH}=9Hz, ⁴J_{HH}=2Hz, C8-H), 7.29 (4H, m, *p*.ClC6H4), 6.72 (1H, bt, ³J_{HH}=6Hz, NH), 6.34 (1H, d, ³J_{HH}=9Hz, C9-H), 4.35 (2H, d, ³J_{HH}=9Hz, NCH₂Ar), 4.20 (2H, q, ³J_{HH}=7Hz, OCH₂), 3.68 (2H, q, J_{HH}=6Hz, NCH₂), 2.65 (2H, t, ³J_{HH}=6, CH₂CO₂), 1.31 (3H, t, ³J_{HH}=7Hz, CH₂CH₃). ¹³C NMR (CDCl₃) 172.6, 168.3, 148.6, 141.0, 137.0, 135.6, 128.8, 128.3, 117.6, 114.3, 75.1, 60.9, 46.3, 35.1, 33.9, 14.2

d) To a magnetically stirred biphasic solution of N-(2-*p*-chlorobenzyl)amino-5-iodobenzoyl)-3-aminoproprionate (10grams, 20.57mmol), 200mL methylene chloride, and 200mL water was added, at room temperature, a-bromoacetyl bromide (24.7mmol, 1.2 molar equiv., 2.15mL), monitoring the reaction by TLC (SiO₂, 1:1 EtOAc/hexane). The reaction was complete 6 hours. The layers were separated , washing the aqueous layer with 75mL methylene chloride. The combined organics were washed 1X100mL sat. sodium bicarbonate, 1X100mL Brine, dried over sodium sulfate, filtered and concentrated *in vacuo.* The resulting residue was dissolved in 50mL dimethylformamide and added, *via* an addition funnel, over a period of 30 minutes to a slurry of sodium hydride (1.2 molar equiv., 24.7mmol, 0.60 grams) in 10mL dimethylformamide cooled to 0°C under an atmosphere of nitrogen. The reaction was stirred for an additional 1 hour and then poured over an ice-cold 10% citric acid solution. The cloudy mixture was extracted 3X100mL EtOAc. The combined organics were dried over magnesium sulfate, filtered, and concentrated *in vacuo.* Purification was achieved by column chromatography (SiO₂ , 600mL, using 30%EtOAc/70% hexane to 60%EtOAc/40% hexane as the eluting solvent gradient) to yield 6.5 grams (63%) of 1-(p-chlorobenzyl)-7-iodo-4-(2-carboxyethyl)-3,4-dihydro-1*H*-1,4-benzodiazpine-2,4-dione ethyl ester. ¹H NMR (CDCl₃, dTMS) 8.11(1H, d, ³J_{HH}=2Hz, C6-H), 6.68 (1H, dd, ⁴J_{HH}=2Hz, ³J_{HH}=9Hz, C8-H), 7.23 (2H, d, ³J_{HH}=8Hz, ArH), 7.03 (2H, d, ³J_{HH}=8Hz, ArH), 6.87 (1H, d, ³J_{HH}=9Hz, C9-H), 5.05 (1H, d, J_{HH}=16Hz, NCHHAr), 4.95 (1H, d, 2J_{HH}=16Hz, NCHHAr), 4.1 (3H, m, OCH2, C3-H), 3.93 (1H, d, J_{HH}=15Hz, C3-H), 3.9 (2H, m, NCH₂CH₂), 2.65 (2H, m, CH₂CO₂), 1.25 (3H, t, ³J_{HH}=7Hz, OCH₂CH₃)

e) To a magnetically stirred solution of 1-(4-iodophenyl)-4-(N-Boc)-piperzine (0.5 grams, 1.29mmol) in 0.65mL THF at -78°C under an atmosphere of nitrogen was added n-BuLi as a 2.5M solution in hexane (1.42mmol, 1.1 molar equiv., 0.57mL) over a 5 minute period. The mixture was stirred for 15 minutes at -78°C and the treated with triisopropylborate (3 molar equiv., 3.9mmol, 0.9mL). The mixture was allowed to warm to room temperature and stirred overnight (ca. 12 hours). The mixture was cooled to 0°C and acidified to pH 6.5 with 5% aq. HCl and extracted with CH₂Cl₂ (50mL). The organic layer was washed with brine, dried over sodium sulfate, decanted and concentrated *in vacuo.* The resulting residue, in a minimum volume of ethanol, was added to a prestirred (10 mins) mixture of tetrakistriphenylphosphine palladium (0.03 molar equiv., 0.026mmol, 30mgs), and 0.447 grams of 1-(p-chlorobenzyl)-7-iodo-4-(2-carboxyethyl)-3,4-dihydro-1*H*-1,4-benzodiazepine-2,4-dione ethyl ester (0.85mmol) in 0.5mL dimethylformamide at room temperature. 1.3mL of a 2M solution of sodium carbonate (2 equiv.) was added and the reaction mixture heated to 80°C for 6 hours. The mixture was allowed to cool to room temperature and is quenched with 1N HCl (25mL) and extracted 3X50 mL ethyl acetate. The combined organics were washed 1X50mL sat NaHCO₃, brine, dried over Na₂SO₄, decanted and concentrated *in vacuo.* The resulting residue was chromatographed (SiO₂, 1:1 EtOAc/hexane to 9:1 EtOAc/hexane as the eluting solvent) to yield 1-(*p*-chlorobenzyl)-7-[4-(4-N-Boc-1-piperazine)phenyl]-4-(2-carboxyethyl)-3,4-dihydro-1*H*-1,4-benzodiazpine-2,4-dione ethyl ester (0.137 grams, 24%, R_{f} (SiO₂, 1:1 EtOAc/hexane)=0,15, mu and ninhydrin positive). ¹H NMR (CDCl₃, dTMS) 8.00 (1H, d, ⁴J_{HH}=2Hz, C6-H), 7.59 (1H, dd, ⁴J_{HH}=2Hz, ³J_{HH}=9Hz, C8-H), 7.50 (2H, d, ³J_{HH}=9Hz, C7-*o*-ArH), 7.23 (2H, d, ³J_{HH}=8Hz, CH₂-*m*-ArH), 7.16 (1H, d, ³J_{HH}=9Hz, C9-H), 7.10) (2H, d, ³J_{HH}=9Hz, CH₂-*o*-ArH), 6.95 (2H, d, ³J_{HH}=8Hz, C7-*m*-ArH), 5.08 (1H, d, J_{HH}=16Hz, NCHHAr), 4.99 (1H, d, J_{HH}=16Hz, NCHHAr), 4.18 (1H, d, J_{HH}=15Hz, C3-H), 4.13 (2H, q, ³J_{HH}=7Hz, OCH₂), 3.92 (2H, m, NCH₂CH₂), 3.92 (1H, d, J_{HH}=15Hz, C3-H), 3.58 (4H, t, ³J_{HH}=5Hz, NCH₂CH₂NH), 3.18 (4H, t, ³J_{HH}=5Hz, NCH₂NCH₂NH), 2.68 (2H, m, CH₂CO₂), 1.47 (9H, s, Bu^{t}), 1.25 (3H, t, ³J_{HH}=7Hz). 13C NMR 171.3, 168.5, 167.1, 154.7, 150.9, 138.6, 138.0, 135.2, 133.3, 132.1, 130.9, 129.9, 129.7, 128.9, 128.4, 1275, 121.9, 116.4, 79.9, 60.8, 52.4, 50.6, 48.8, 45.1, 32.8, 28.4, 14.2.

Using the above procedure, but substituting the appropriate 7-iodo-3,4-dihydro-1*H*-1,4-benzodiazpine-2,4-dione alkyl ester for 1-(p-chlorobenzyl)-7-iodo-4-(2-carboxyethyl)-3,4-dihydro-1*H*-1,4-benzodiazpine-2,4-dione ethyl ester and substituting the appropriate iodoarene for 1-(4-iodophenyl)-4-(N-Boc)-piperzine there may be prepared, for example, the following compounds:
1-methyl-7-[4-(4-N-Boc-1-piperazine)phenyl]-4-(2-carboxyethyl)-3,4-dihydro-1*H*-1,4-benzodiazpine-2,4-dione ethyl ester,
1-(diphenylmethyl)-7-[4-(4-N-Boc-1-piperazine)phenyl]-4-(2-carboxyethyl)-3,4-dihydro-1H-1,4-benzodiazpine-2,4-dione ethyl ester,
1-(2-napthyl)methyl-7-[4-(4-N-Boc-1-piperazine)phenyl]-4-(2-carboxyethyl)-3,4-dihydro-1H-1,4-benzodiazpine-2,4-dione ethyl ester,
(±)-1-methyl-7-[4-(4-N-Boc-1-piperazine)phenyl]-4-(3-phenyl-3-proprionate)-3,4-dihydro-1*H*-1,4-benzodiazpine-2,4-dione methyl ester,
(±)-1-(2-napthyl)methyl-7-[4-(4-N-Boc-1-piperazine)phenyl]-4-(3-phenyl-3-proprionate)-3,4-dihydro-1*H*-1,4-benzodiazpine-2,4-dione methyl ester,
1-(*p*-chlorobenzyl)-7-[4-(2-(N-Boc)-aminoethoxy)phenyl]-4-(2-carboxyethyl)-3,4-dihydro-1H-1,4-benzodiazpine-2,4-dione ethyl ester
1-methyl-7-[4-(2-(N-Boc)-aminoethoxy)phenyl]-4-(2-carboxyethyl)-3,4-dihydro-1*H*-1,4-benzodiazpine-2,4-dione ethyl ester,
1-(diphenylmethyl)-7-[4-(2-(N-Boc)-aminoethoxy)phenyl]-4-(2-carboxyethyl)-3,4-dihydro-1*H*-1,4-benzodiazpine-2,4-dione ethyl ester,
1-(2-napthyl)methyl-7-[4-(2-(N-Boc)-aminoethoxy)phenyl]-4-(2-carboxyethyl)-3,4-dihydro-1*H*-1,4-benzodiazpine-2,4-dione ethyl ester,
(±)-1-methyl-7-[4-(2-(N-Boc)-aminoethoxy)phenyl]-4-(3-phenyl-3-proprionate)-3,4-dihydro-1*H*-1,4-benzodiazpine-2,4-dione methyl ester,
(±)-1-(2-napthyl)methyl-7-[4-(2-(N-Boc)-aminoethoxy)phenyl]-4-(3-phenyl-3-proprionate)-3,4-dihydro-1*H*-1,4-benzodiazpine-2,4-dione methyl ester,
1-(*p*-chlorobenzyl)-7-[4-(N-Boc-aminomethyl)phenyl]-4-(2-carboxyethyl)-3,4-dihydro-1*H*-1,4-benzodiazpine-2,4-dione ethyl ester
1-methyl-7-[4-(N-Boc-aminomethyl)phenyl]-4-(2-carboxyethyl)-3,4-dihydro-1*H*-1,4-benzodiazpine-2,4-dione ethyl ester,
1-(diphenylmethyl)-7-4-(N-Boc-aminomethyl)phenyl]-4-(2-carboxyethyl)-3,4-dihydro-1H-1,4-benzodiazpine-2,4-dione ethyl ester,
1-(2-napthyl)methyl-7-[4-(N-Boc-aminomethyl)phenyl]-4-(2-carboxyethyl)-3,4-dihydro-1H-1,4-benzodiazpine-2,4-dione ethyl ester,
(±)-1-methyl-7-[4-(N-Boc-aminomethyl)phenyl]-4-(3-phenyl-3-proprionate)-3,4-dihydro-1H-1,4-benzodiazpine-2,4-dione methyl ester,
(±)-1-(2-napthyl)methyl-7-[4-(N-Boc-aminomethyl)phenyl]-4-(3-phenyl-3-proprionate)-3,4-dihydro-1*H*-1,4-benzodiazpine-2,4-dione methyl ester.

f) 1-(p-chlorobenzyl)-7-[4-(1-piperazine)phenyl]-4-(2-carboxyethyl)-3,4-dihydro-1*H*-1,4-benzodiazpine-2,4-dione trifluoracetate was prepared using the method described in part (c) of Example 1. Thus, 34 mgs (0.052mmol) 1-(p-chlorobenzyl)-7-[4-(4-N-Boc-1-piperazine)phenyl]-4-(2-carboxyethyl)-3,4-dihydro-1*H*-1,4-benzodiazpine-2,4-dione ethyl ester yielded 25 mgs (90%) of 1-(p-chlorobenzyl)-7-[4-(1-piperazine)phenyl]-4-(2-carboxyethyl)-3,4-dihydro-1*H*-1,4-benzodiazpine-2,4-dione trifluoroacetate (1/2" C-18 reverse-phase column, eluting with a solvent gradient of 1:9 acetonitrile(0.1%TFA)/water (0.1% TFA) time 0 to 10 minutes, to 1:1 acetonitrile(0.1%/TFA)/water (0.1% TFA) time 10 to 40 minutes, flow 10mL/min, Rₜ=34.9 min, mu detection 254nM). HRMS (FAB) molecular ion m/z=533,1950 (cald. C29H30N4O4Cl, 533.1956). ¹H NMR (D₂O,10%CD₃OD) 7.61 (1H, d, ⁴J_{HH}=2Hz, C6-H), 7.43 (1H, dd, ⁴J_{HH}=2Hz, ³J_{HH}=8Hz, C8-H), 7.28 (2H, d, C7-*o*-ArH), 7.10 (1H, d, ³J_{HH}=8Hz, C9-H), 6.98 (2H, d, ³J_{HH}=7Hz, CH₂-*m*-ArH), 6.87 (2H, d, ³J_{HH}=7Hz, CH₂-*o*-ArH), 6.80 (2H, d, ³J_{HH}=8Hz, C7-*m*-ArH), 5.20 (1H, d, J_{HH}=16Hz, NCHHAr), 3.95 (1H, d, J_{HH}=14Hz, C3-H), 3.84 (1H, dt, NCHHCH₂), 3.72 (1H, d, J_{HH}=14Hz, C3-H), 3.58 (1H, dt, C3-H), 3.23 (4H, bs, NCH₂CH₂NH), 3.18 (4H, bs, NCH₂CH₂NH), 2.58 (2H, t, 3J_{HH}=7Hz, CH₂CO₂).

Using the above procedure, but substituting the appropriate 7-aryl-3,4-dihydro-1H-1,4-benzodiazpine-2,4-dione N-Boc amino alkyl ester for 1-(p-chlorobenzyl)-7-[4-(4-N-Boc-1-piperazine)phenyl]-4-(2-carboxyethyl)-3,4-dihydro-1*H*-1,4-benzodiazpine-2,4-dione ethyl ester there may be prepared, for example, the following compounds:
1-methyl-7-[4-(1-piperazine)phenyl]-4-(2-carboxyethyl)-3,4-dihydro-1*H*-1,4-benzodiazpine-2,4-dione trifluoroacetate,
1-(diphenylmethyl)-7-[4-(1-piperazine)phenyl]-4-(2-carboxyethyl)-3,4-dihydro-1*H*-1,4-benzodiazpine-2,4-dione trifluoracetate,
1-(2-napthyl)methyl-7-[4-(1-piperazine)phenyl]-4-(2-carboxyethyl)-3,4-dihydro-1*H*-1,4-benzodiazpine-2,4-dione trifluoroacetate,
(±)-1-methyl-7-[4-(1-piperazine)phenyl]-4-(3-phenyl-3-proprionate)-3,4-dihydro-1*H*-1,4-benzodiazpine-2,4-dione trifluoroacetate,
(±)-1-(2-napthyl)methyl-7-[4-(1-piperazine)phenyl]-4-(3-phenyl-3-proprionate)-3,4-dihydro-1*H*-1,4-benzodiazpine-2,4-dione trifluoroacetate,
1-(*p*-chlorobenzyl)-7-[4-(2-aminoethoxy)phenyl]-4-(2-carboxyethyl)-3,4-dihydro-1*H*-1,4-benzodiazpine-2,4-dione trifluoracetate,
1-methyl-7-[4-(2-aminoethoxy)phenyl]-4-(2-carboxyethyl)-3,4-dihydro-1*H*-1,4-benzodiazpine-2,4-dione trifluoroacetate,
1-(diphenylmethyl)-7-[4-(2-aminoethoxy)phenyl]-4-(2-carboxyethyl)-3,4-dihydro-1*H*-1,4-benzodiazpine-2,4-dione trifluoroacetate,
1-(2-napthyl)methyl-7-[4-(2-aminoethoxy)phenyl]-4-(2-carboxyethyl)-3,4-dihydro-1*H*-1,4-benzodiazpine-2,4-dione trifluoroacetate,
(±)-1-methyl-7-[4-(2-aminoethoxy)phenyl]-4-(3-phenyl-3-proprionate)-3,4-dihydro-1*H*-1,4-benzodiazpine-2,4-dione trifluoroacetate,
(±)-1-(2-napthyl)methyl-7-[4-(2-aminoethoxy)phenyl]-4-(3-phenyl-3-proprionate)-3,4-dihydro-1*H*-1,4-benzodiazpine-2,4-dione trifluotoacetate,
1-(*p*-chlorobenzyl)-7-[4-(aminomethyl)phenyl]-4-(2-carboxyethyl)-3,4-dihydro-1*H*-1,4-benzodiazpine-2,4-dione trifluoroacetate,
1-methyl-7-[4-(aminomethyl)phenyl]-4-(2-carboxyethyl)-3,4-dihydro-1*H*-1,4-benzodiazpine-2,4-dione trifluoroacetate,
1-(diphenylmethyl)-7-4-(aminomethyl)phenyl]-4-(2-carboxyethyl)-3,4-dihydro-1*H*-1,4-benzodiazpine-2,4-dione trifluoroacetate,
1-(2-napthyl)methyl-7-[4-(aminomethyl)phenyl]-4-(2-carboxyethyl)-3,4-dihydro-1*H*-1,4-benzodiazpine-2,4-dione trifluoroacetate,
(±)-1-methyl-7-[4-(aminomethyl)phenyl]-4-(3-phenyl-3-proprionate)-3,4-dihydro-1*H*-1,4-benzodiazpine-2,4-dione trifluoroacetate,
(±)-1-(2-napthyl)methyl-7-[4-(aminomethyl)phenyl]-4-(3-phenyl-3-proprionate)-3,4-dihydro-1*H*-1,4-benzodiazpine-2,4-dione trifluoroacetate.

### Example 13

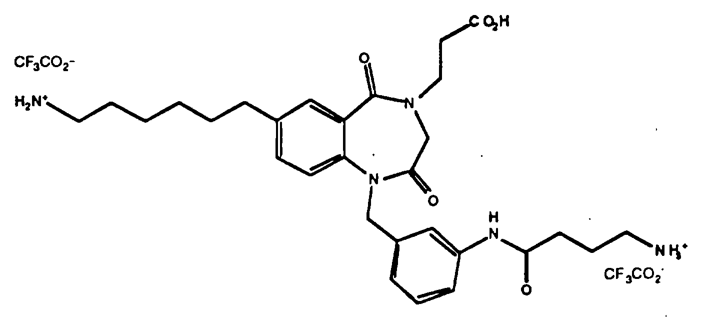

*1-[3-(N-(4-aminobuturyl)aminophenyl]methyl-4-(2-caboxyethyl)-7-(6-aminohexyl)-3,4-dihydro-1H-1,4-benzodiazapine-2,5-dione bistrifluoroacetate.*

a) 1-(3-nitrophenyl)methyl-4-(2-carboxyethyl)-7-(N-Boc-6-aminohexynyl)-3,4-dihydro-1H-1,4-benzodiazapine-2,5-dione ethyl ester (110mgs, 0.18mmol) was reduced under an atmosphere of nitrogen in 4mL ethyl acetate in the presence of a catalytic amount of 10% Pd on carbon (25mgs), monitoring the reaction by TLC (SiO₂, 1:1 ethyl acetate/hexane, R_{f}(starting material)=0.33, R_{f}(product)=0.15, mu and ninhydrin positive). After 3 hours, the reaction was filtered through Celite®, washing with ethyl acetate, and concentrated *in vacuo* to yield 97mgs (92%,) of 1-(3-aminophenyl)methyl-4-(2-carboxyethyl)-7-(N-Boc-6-aminohexyl)-3,4-dihydro-1H-1,4-benzodiazapine-2,5-dione ethyl ester. 1H NMR (CDCl₃, dTMS) 7.58 (1H, d, ⁴J_{HH}=2Hz, C6-H), 7.18 (1H, dd, ⁴J_{HH}=2Hz, ³J_{HH}=8Hz, C8-H), 7.09 (1H, d, ³J_{HH}=9Hz, CH₂ArH), 7.04 (1H, t, ³J_{HH}=8Hz, CH₂ArH), 6.54 (1H, bd, CH₂ArH), 6.48 (1H, bs, CH₂ArH), 4.99 (1H, d, J_{HH}=16Hz, CHHAr), 4.90 (1H, d, 2J_{HH}=16Hz, CHHAr), 4.53 (1H, vbs, NHBoc), 4.20-3.96 (4H, m, OCH₂, C3-H, NCHHCH₂), 3.92-3.80 (2H, m, C3-H, NCHHCH₂), 3.55 (2H, bs, NH₂), 3.06 (2H, bq, ³J_{HH}=6Hz, CH₂NHBoC), 2.72 (2H, m, CH₂CO₂), 2.56 (2H, t, ³J_{HH}=7Hz), 1.66-1.20 (2OH, m, (CH₂)₄, Bu^{t}, OCH₂CH₃). 13C NMR (CDCl3) 171.4, 168.5, 167.4, 146.3, 140.7, 138.1, 137.9, 132.2, 130.1, 129.6, 129.0, 121.6, 117.4, 114.5, 113.6, 79.0, 60.8, 52.4, 51.1, 45.1, 40.5, 34.9, 32.8, 30.8, 29.9, 28.8, 28.4, 26.5, 14.2.

Using the above procedure, but substituting the appropriate 7-alkyl or aryl-3,4-dihydro-1*H*-1,4-benzodiazpine-2,4-dione N-Boc amino alkyl ester for 1-(3-nitrophenyl)methyl-4-(2-carboxyethyl)-7-(N-Boc-6-aminohexynyl)-3,4-dihydro-1H-1,4-benzodiazapine-2,5-dione ethyl ester there may be prepared, for example, the following compounds:
1-(2-aminophenyl)methyl-4-(2-carboxyethyl)-7-(N-Boc-6-aminohexyl)-3,4-dihydro-1H-1,4-benzodiazapine-2,5-dione ethyl ester,
1-(4-aminophenyl)methyl-4-(2-carboxyethyl)-7-(N-Boc-6-aminohexyl)-3,4-dihydro-1H-1,4-benzodiazapine-2,5-dione ethyl ester,
1-(3-aminophenyl)methyl-4-(2-carboxyethyl)-7-[4-(4-N-Boc-1-piperazine)phenyl]-3,4-dihydro-1H-1,4-benzodiazapine-2,5-dione ethyl ester,
1-(3-aminophenyl)methyl-4-(3-phenyl-3-proprionate)-7-[4-(4-N-Boc-1-piperazine)phenyl]-3,4-dihydro-1H-1,4-benzodiazapine-2,5-dione ethyl ester,
1-(3-aminophenyl)methyl-4-(3-phenyl-3-proprionate)-7-(N-Boc-6-aminohexyl)-3,4-dihydro-1H-1,4-benzodiazapine-2,5-dione ethyl ester,

b) A solution of the aniline 1-(3-aminophenyl)methyl-4-(2-carboxyethyl)-7-(N-Boc-6-aminohexyl)-3,4-dihydro-1H-1,4-benzodiazapine-2,5-dione ethyl ester (50mgs, 0.09mmol) and 0.5mL dimethylacetamide was added to a prestirred solution of N-Boc-4-aminobutyric acid (0.16mmol, 32.5mgs), 0.5mL dimethylacetamide, 0.5mL pyridine, and benztriazol-1-yl-oxy-tris-(dimethylamino)phosphonium hexafluorophosphate (BOP Reagent) and a catalytic amount of dimethylaminopyridine (DMAP, 5mgs), monitoring by TLC (8:2 ethyl acetate/hexane). After 6 hours, the reaction was poured over 50mL 1N HCl and extracted 2X50mL ethyl acetate. The combined organics was dried over sodium sulfate, decanted, and concentrated in vacuo. The resulting residue was purified by column chromatography, SiO₂, using 7:3 ethyl acetate/hexane to 9:1 ethyl acetate/hexane, R_{f}(8:2 ethyl acetae/hexane, product)=0.15, to yield 59mgs (86%) of 1-[3-(4-N-Boc-aminobutyryl)aminophenyl]methyl-4-(2-carboxyethyl)-7-(N-Boc-6-aminohexyl)-3,4-dihydro-1H-1,4-benzodiazapine-2,5-dione ethyl ester. ¹H NMR (CDCl₃, dTMS) 8.78 (1H, bs, ArNHCO), 7.56 (1H, d, ⁴J_{HH}=2Hz, C6-H), 7.44 (1H, bd, ³J_{HH}=8Hz, C8-H), 7.36 (1H, bs, CH₂ArH), 7.17 (2H, m, CH₂ArH), 7.09 (1H, t, ³J_{HH}=8Hz, CH₂ArH), 6.81 (1H, bd, ³J_{HH}=8Hz, C9-H), 5.1-4.9 (3H, m, NHBoc, CH₂Ar), 458 (1H, bs, NHBoc), 4.20-4.05 (4H, m, OCH₂, C3-H, NCHHCH₂), 3.87 (1H, d, J_{HH}=15Hz, C3-H), 3.80 (1H, dt, NCHHCH₂), 3.16 (2H, bq, ³J_{HH}=6Hz, CH₂NHBoc), 3.04 (2H, bq, ³J_{HH}=6Hz, CH₂NHBoc), 2.71 (2H, m, CH₂CO₂), 2.54 (2H, t, ³J_{HH}=7Hz, NCOCH₂), 2.32 (2H, t, ³J_{HH}=7Hz, ArCH₂),1.82 (2H, p, ³J_{HH}=7Hz, NCOCH₂CH₂), 1.6-1.2 (20H, m, (CH₂)₄, Bu^{t}, OCH₂CH₃).

Using the above procedure, but substituting the appropriate 7-alkyl or aryl-3,4-dihydro-1*H*-1,4-benzodiazpine-2,4-dione N-Boc amino alkyl ester for 1-(3-aminophenyl)methyl-4-(2-carboxyethyl)-7-(N-Boc-6-aminohexyl)-3,4-dihydro-1H-1,4-benzodiazapine-2,5-dione ethyl ester there may be prepared, for example, the following compounds:
1-[2-(4-N-Boc-aminobutyryl)aminophenyl]methyl-4-(2-carboxyethyl)-7-(N-Boc-6-aminohexyl)-3,4-dihydro-1H-1,4-benzodiazapine-2,5-dione ethyl ester,
1-(4-(4-N-Boc-aminobutyryl)aminophenyl)methyl-4-(2-carboxyethyl)-7-(N-Boc-6-aminohexyl)-3,4-dihydro-1H-1,4-benzodiazapine-2,5-dione ethyl ester,
1-(3-(4-N-Boc-aminobutyryl)aminophenyl)methyl-4-(2-carboxyethyl)-7-[4-(4-N-Boc-1-piperazine)phenyl]-3,4-dihydro-1H-1,4-benzodiazapine-2,5-dione ethyl ester,
1-(3-(4-N-Boc-aminobutyryl)aminophenyl)methyl-4-(3-phenyl-3-proprionate)-7-[4-(4-N-Boc-1-piperazine)phenyl]-3,4-dihydro-1H-1,4-benzodiazapine-2,5-dione ethyl ester,
1-(3-(4-N-Boc-aminobutyryl)aminophenyl)methyl-4-(3-phenyl-3-proprionate)-7-(N-Boc-6-aminohexyl)-3,4-dihydro-1H-1,4-benzodiazapine-2,5-dione ethyl ester,
1-[3-(3-N-Boc-aminopropionyl)aminophenyl]methyl-4-(2-carboxyethyl)-7-(N-Boc-6-aminohexyl)-3,4-dihydro-1H-1,4-benzodiazapine-2,5-dione ethyl ester,
1-[2-(3-N-Boc-aminopropionyl)aminophenyl]methyl-4-(2-carboxyethyl)-7-(N-Boc-6-aminohexyl)-3,4-dihydro-1H-1,4-benzadiazapine-2,5-dione ethyl ester,
1-(4-(3-N-Boc-aminopropionyl)aminophenyl)methyl-4-(2-carboxyethyl)-7-(N-Boc-6-aminohexyl)-3,4-dihydro-1H-1,4-benzodiazapine-2,5-dione ethyl ester,
1-(3-(3-N-Boc-aminopropionyl)aminophenyl)methyl-4-(2-carboxyethyl)-7-[4-(4-N-Boc-1-piperazine)phenyl]-3,4-dihydro-1H-1,4-benzodiazapine-2,5-dione ethyl ester,
1-(3-(3-N-Boc-aminopropionyl)aminophenyl)methyl-4-(3-phenyl-3-proprionate)-7-[4-(4-N-Boc-1-piperazine)phenyl]-3,4-dihydro-1H-1,4-benzodiazapine-2,5-dione ethyl ester,
1-(3-(3-N-Boc-aminopropionyl)aminophenyl)methyl-4-(3-phenyl-3-proprionate)-7-(N-Boc-6-aminohexyl)-3,4-dihydro-1H-1,4-benzodiazapine-2,5-dione ethyl ester.

c) 1-[3-(N-(4-aminobuturyl))aminophenyl]methyl-4-(2-carboxyethyl)-7-(6-aminohexyl)-3,4-dihydro-1H-1,4-benzodiazapine-2,5-dione trifluoroacetate was prepared using the method described in part (c) of Example 1. Thus, 59 mgs (0.074mmol) 1-[3-(4-N-Boc-aminobutyryl)aminophenyl]methyl-4-(2-carboxyethyl)-7-(N-Boc-6-aminohexyl)-3,4-dihydro-1H-1,4-benzodiazapine-2,5-dione ethyl ester yielded 50 mgs (99%) of 1-[3-(N-(4-aminobuturyl))aminophenyl]methyl-4-(2-carboxyethyl)-7-(6-aminohexyl)-3,4-dihydro-1H-1,4-benzodiazapine-2,5-dione trifluoroacetate (1/2" C-18 reverse-phase column, eluting with a solvent gradient of 1:9 acetonitrile(0.1%TFA)/water (0.1% TFA) time 0 to 10 minutes, to 1:1 acetonitrile(0.1%TFA)/water (0.1% TFA) time 10 to 40 minutes, flow 10mL/min, Rₜ=30.5 min, mu detection 254nM). HRMS (FAB) molecular ion m/z=538.3032 (cald. C₂₉H₃₉N₅O₅, 538.3030). ¹H NMR (D₂O) 7.21 (1H, bs, C6-H), 7.05-6.85 (5H, m, CH₂ArH, C8-H), 6.52 (1H, bd, ³J_{HH}=8Hz, C9-H), 5.03 (1H, d, J_{HH}=16Hz, CHHAr), 4.47 (1H, d, J_{HH}=16Hz, CHHAr), 3.86 (1H, d, J_{HH}=15Hz, C3-H), 3.80 (1H, dt, ³J_{HH}=7Hz, J_{HH}=15Hz, NCHHCH₂), 3.63 (1H, d, J_{HH}=15Hz, C3-H), 3.51 (1H, dt, ³J_{HH}=7Hz, J_{HH}=15Hz, NCHHCH₂), 2.83 (2H, t, ³J_{HH}=8Hz, CH₂NH₂), 2.68 (2H, t, ³J_{HH}=8Hz, CH₂NH₂), 2.43 (2H, t, ³J_{HH}=7Hz), 2.3-2.2 (4H, m), 1.76 (2H, p, ³J_{HH}=7Hz, NCOCH₂CH₂), 135 (2H, p, ³J_{HH}=7Hz), 1.22 (2H, p, ³J_{HH}=7Hz), 1.1-0.9 (4H, m).

Using the above procedure, but substituting the appropriate 7-alkyl or aryl-3,4-dihydro-1H-1,4-benzodiazpine-2,4-dione bis-N-Boc diamino alkyl ester for 1-[3-(4-N-Boc-aminobutyryl)aminophenyl]methyl-4-(2-carboxyethyl)-7-(N-Boc-6-aminohexyl)-3,4-dihydro-1H-1,4-benzodiazapine-2,5-dione ethyl ester there may be prepared, for example, the following compounds:
1-[2-(4-aminobutyryl)aminophenyl]methyl-4-(2-carboxyethyl)-7-(6-aminohexyl)-3,4-dihydro-1H-1,4-benzodiazapine-2,5-dione bistrifluoroacetate,
1-(4-(4-aminobutyryl)aminophenyl)methyl-4-(2-carboxyethyl)-7-(6-aminohexyl)-3,4-dihydro-1H-1,4-benzodiazapine-2,5-dione bistrifluoroacetate,
1-(3-(4-aminobutyryl)aminophenyl)methyl-4-(2-carboxyethyl)-7-[4-(1-piperazine)phenyl]-3,4-dihydro-1H-1,4-benzodiazapine-2,5-dione bistrifluoroacetate,
1-(3-(4-aminobutyryl)aminophenyl)methyl-4-(3-phenyl-3-proprionate)-7-[4-(1-piperazine)phenyl]-3,1-dihydro-1H-1,4-benzodiazapine-2,5-dione bistrifluoroacetate,
1-(3-(4-aminobutyryl)aminophenyl)methyl-4-(3-phenyl-3-proprionate)-7-(6-aminohexyl)-3,4-dihydro-1H-1,4-benzodiazapine-2,5-dione bistrifluoroacetate,
1-[3-(3-aminopropionyl)aminophenyl]methyl-4-(2-carboxyethyl)-7-(6-aminohexyl)-3,4-dihydro-1H-1,4-benzodiazapine-2,5-dione bistrifluoroacetate,
1-[2-(3-aminopropionyl)aminophenyl]methyl-4-(2-carboxyethyl)-7-(6-aminohexyl)-3,4-dihydro-1H-1,4-benzodiazapine-2,5-dione bistrifluoroacetate,
1-(4-(3-aminopropionyl)aminophenyl)methyl-4-(2-carboxyethyl)-7-(6-aminohexyl)-3,4-dihydro-1H-1,4-benzodiazapine-2,5-dione bistrifluoroacetate,
1-(3-(3-aminopropionyl)aminophenyl)methyl-4-(2-carboxyethyl)-7-[4-(1-piperazine)phenyl]-3,4-dihydro-1H-1,4-benzodiazapine-2,5-dione bistrifluoroacetate,
1-(3-(3-aminopropionyl)aminophenyl)methyl-4-(3-phenyl-3-proprionate)-7-[4-(4-(1-piperazine)phenyl]-3,4-dihydro-1H-1,4-benzodiazapine-2,5-dione bistrifluoroacetate,
1-(3-(3-aminopropionyl)aminophenyl)methyl-4-(3-phenyl-3-proprionate)-7-(6-aminohexyl)-3,4-dihydro-1H-1,4-benzodiazapine-2,5-dione bistrifluoroacetate.

### Example 14

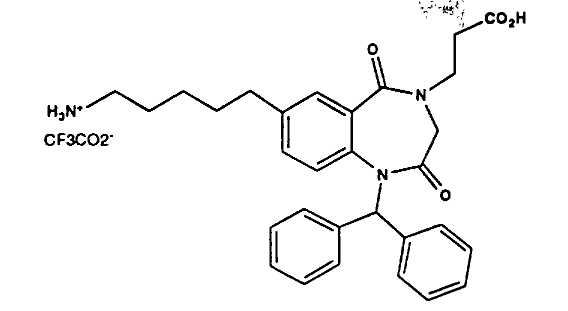

*1-(diphenylmethyl)-4-(2-carboxyethyl)-7-(5-aminopentyl)-3,4-dihydro-1H-1,4-benzodiazepine-2,5-dione trifluoroacetate*

a) A magnetically stirred solution of 1.1 gram of N-(2-amino-5-iodobenzoyl)-b-alanine ethyl ester (3.1mmol), prepared by the method shown in part (b) of Example 1, 0.48mL of 2,6-lutidine (4.1mmol), 1.2 grams of chlorodiphenylmethane (4.7mmol), and 10mL of dimethylformamide was heated to 50°C for 1 hour. The reaction mixture was allowed to cool to room temperature and concentrated *in vacuo.* The resulting oil was dissolved in 35mL of methylene chloride and washed with 2x50mL 10% citric acid, 1x50mL water, dried over sodium sulfate, decanted and concentrated *in vacuo.* The resulting oil was further purified by column chromatography, using silica gel, eluting with a solvent gradient of 10/90 ethyl acetate/hexane to 25/75 ethyl acetate/hexane (TLC, SiO₂, 1:1 EtOAc/hexane, R_{f}= 0.82, uv positive) to yield 0.92 gram (56%) of N-(2-diphenylmethylamino-5-iodobenzoyl)-b-alanine ethyl ester. ¹H NMR (CDCl₃, dTMS) 8.40 (1H, d, ³J_{HH}=5Hz, NHCHAr₂), 7.56 (1H, d, ³J_{HH}=2Hz, ArH *o*-CON), 7.18-7.36 (11H, m, ⁴J_{HH}=2Hz, ArH Ph, ArH p-CON), 6.73 (1H, t, ³J_{HH}=6Hz, NHCH₂), 6.29 (1H, d, ³J_{HH}=9Hz, ArH m-CON), 5.52 (1H, d, ³J_{HH}=5Hz, CHNH), 4.15 (2H, q, ³J_{HH}=7Hz, OCH₂), 3.61 (2H, q, ³J_{HH}=6Hz, CH₂NH), 2.58 (2H, t, ³J_{HH}=6Hz, CH₂CO), 1.25 (3H, t, ³J_{HH}=6Hz, CH₃CH₂O).

Using the above procedure, but substituting the appropriate alkyl or aryl halide there may be prepared, for example, the following compounds:
N-(2-(1-napthyl)methylamino-5-iodobenzoyl)-b-alanine ethyl ester,
N-(2-(2-napthyl)methylamino-5-iodobenzoyl)-b-alanine ethyl ester,
N-(2-(*p*-phenoxybenzyl)amino-5-iodobenzoyl)-b-alanine ethyl ester,
N-(2-(*m*-phenoxybenzyl)amino-5-iodobenzoyl)-b-alanine ethyl ester,
N-(2-(*p*-trifluoromethylbenzyl)amino-5-iodobenzoyl)-b-alanine ethyl ester,
N-(2-(ethyl-5-valeroyl)amino-5-iodobenzoyl)-b-alanine ethyl ester,
N-(2-(*p*-methoxybenzyl)amino-5-iodobenzoyl)-b-alanine ethyl ester,
Methyl 3(S)-N-(2-(2-methylnapthyl)amino-5-iodobenzoyl)-3-amino-3-methylpropionate,
Methyl 3(R)-N-(2-(2-napthyl)methylamino-5-iodobenzoyl)-3-amino-3-methylpropionate,
Methyl (+/-)-N-(2-(2-napthyl)methylamino-5-iodobenzoyl)-3-amino-3-methylpropionate,
Methyl 3(S)-N-(2-(2-napthyl)methylamino-5-iodobenzoyl)-3-amino-3-phenylpropionate,
Methyl 3(R)-N-(2-(2-napthyl)methylamino-5-iodobenzoyl)-3-amino-3-phenylpropionate,
Methyl (+/-)-N-(2-(2-napthyl)methylamino-5-iodobenzelyl)-3-amino-3-phenylpropionate,

b) To a magnetically stirred solution of 0.93 gram of N-(2-diphenyl-methylamino-5-iodobenzoyl)-b-alanine ethyl ester (1.8mmol) in 15mL of methylene chloride and 15mL water, was added 183mL a-bromoacetylbromide (2.1mmol). The reaction mixture was stirred overnight. The layers were separated, the aqueous fraction was washed 1x15mL methylene chloride, and the two methylene chloride fractions were combined and washed with 1x20mL of saturated sodium bicarbonate, dried over sodium sulfate, filtered and concentrated *in vacuo.* The resulting residue was dissolved in 7mL of dimethylformamide and added, *via* an addition funnel, to a slurry of 57 mgs of 95% sodium hydride (2.2mmol) in 5mL dimethylformamide that was cooled at 0°C. After 2 hours, the mixture was poured over 40mL of ice cooled 10% citric acid and extracted 3x40mL methylene chloride. The combined organic layers were washed with 2x50mL of 10% citric acid and 1x50mL water, dried over sodium sulfate, decanted, concentrated *in vacuo* and further purified by column chromatography, using silica gel, eluting with a 1:1 mixture of ethyl acetate and hexane (TLC, SiO₂, 1:1 EtOAc/hexane, R_{f}= 0.61, uv positive) to yield 474 mgs (48%) of 1-(diphenylmethyl)-4-(2-carboxyethyl)-8-iodo-3,4-dihydro-1H-1,4-benzodiazepine-2,5-dione ethyl ester. ¹H NMR (CDCl₃, dTMS) 8.03 (1H, d, ³J_{HH}=2Hz, ArH o-CON), 7.45 (1H, dd, ³J_{HH}=2Hz, ³J_{HH}=9Hz, ArH p-CON), 7.1-7.4 (1H, m, ⁴J_{HH}=2Hz, ArH Ph), 6.79 (1H, d, ³J_{HH}=9Hz, ArH m-CON), 6.70 (1H, s), 4.13 (2H, q, ³J_{HH}=7Hz, OCH₂), 3.98 (2H, dd, ³J_{HH}=100Hz, ³J_{HH}=15Hz, COCH₂N), 3.86 (2H, dm, ³J_{HH}=62Hz, ³J_{HH}=8Hz, CH₂N), 2.58 (2H, dm, ³J_{HH}=40Hz, ³J_{HH}=8Hz, CH₂CO), 1.27 (3H, t, ³J_{HH}=7Hz, CH₃CH₂O). ¹³C NMR (CDCl₃) 171.2, 168.0, 165.6, 139.9, 139.3, 138.8, 138.1, 137.5, 132.0, 129.1, 128.5, 128.4, 128.0, 128.0, 128.0, 127.9. 127.6, 125.4, 90.6, 67.2, 60.8, 53.1, 45.0, 32.6, 14.2.

Using the above procedure, but substituting the appropriate N-(2-amino-5-iodobenzoyl)-b-alanine ethyl ester for N-(2-diphenylmethylamino-5-iodobenzoyl)-b-alanine ethyl ester there may be prepared, for example, the following compounds:
1-(1-napthyl)methyl-4-(2-carboxyethyl)-7-iodo-3,4-dihydro-1H-1,4-benzodiazepine-2,5-dione ethyl ester,
1-(2-napthyl)methyl-4-(2-carboxyethyl)-7-iodo-3,4-dihydro-1H-1,4-benzodiazepine-2,5-dione ethyl ester,
1-(*m*-phenoxybenzyl)-4-(2-carboxyethyl)-7-iodo-3,4-dihydro-1H-1,4-benzodiazepine-2,5-dione ethyl ester,
1-(*p*-phenoxybenzyl)-4-(2-carboxyethyl)-7-iodo-3,4-dihydro-1H-1,4-benzodiazepine-2,5-dione ethyl ester,
1-(*p*-trifluoromethylbenzyl)-4-(2-carboxyethyl)-7-iodo-3,4-dihydro-1H-1,4-benzodiazepine-2,5-dione ethyl ester,
1-(ethyl-5-valeroyl)-4-(2-carboxyethyl)-7-iodo-3,4-dihydro-1H-1,4-benzodiazepine-2,5-dione ethyl ester,
1-(*p*-methoxybenzyl)-4-(2-carboxyethyl)-7-iodo-3,4-dihydro-1H-1,4-benzodiazepine-2,5-dione ethyl ester,
1-(*p*-methylmethylbenzoyl)-4-(2-carboxyethyl)-7-iodo-3,4-dihydro-1H-1,4-benzodiazepine-2,5-dione ethyl ester,
1-(2-napthyl)methyl-4-(3(S)-butanoate)-7-iodo-3,4-dihydro-1H-1,4-benzodiazepine-2,5-dione methyl ester,
1-(2-napthyl)methyl-4-(3(R)-butanoate)-7-iodo-3,4-dihydro-1H-1,4-benzodiazepine-2,5-dione methyl ester,
1-(2-napthyl)methyl-4-((+/-)3-butanoate)-7-iodo-3,4-dihydro-1H-1,4-benzodiazepine-2,5-dione methyl ester,
1-(2-napthyl)methyl-4-(3(S)-phenyl-3-proprionate)-7-iodo-3,4-dihydro-1H-1,4-benzodiazepine-2,5-dione methyl ester,
1-(2-napthyl)methyl-4-(3(R)-phenyl-3-proprionate)-7-iodo-3,4-dihydro-1H-1,4-benzodiazepine-2,5-dione methyl ester,
1-(2-napthyl)methyl-4-((+/-)3-phenyl-3-proprionate)-7-iodo-3,4-dihyro-1H-1,4-benzodiazepine-2,5-dione methyl ester.

c) To a magnetically stirred solution of 208 mgs of 1-(diphenylmethyl)-4-(2-carboxyethyl)-7-iodo-3,4-dihydro-1H-1,4-benzodiazepine-2,5-dione ethyl ester (0.37mmol) in 6mL ethyl acetate, degassed of oxygen, under an atmosphere of nitrogen, was added 134 mgs of N-boc-5-amino-1-pentyne (0.74mmol), prepared as described in part (e) of Example 1,10 mgs of bistriphenylphosphine palladium dichloride (0.014mmol), 5 mgs of cuprous iodide (0.026mmol) and 250uL of triethylamine (1.75mmol). After 1.5 hours, the reaction mixture was diluted with 35mL of ethyl acetate and washed with 1x15mL 5% EDTA˙Na₂, dried over sodium sulfate, decanted, concentrated *in vacuo,* and further purified by column chromatography, using silica gel, eluting with a 1:1 mixture of ethyl acetate and hexane (TLC, SiO₂,1:1 EtOAc/hexane, R_{f}= 0.46, uv positive) to yield 131 mgs (60%) of 1-(diphenylmethyl)-4-(2-carboxyethyl)-8-(N-boc-5-amino-1-pentynl)-3,4-dihydro-1H-1,4-benzodiazepine-2,5-dione ethyl ester. ¹H NMR (CDCl₃, TMS) 7.71 (1H, d, ⁴J_{HH}=2Hz, ArH o-CON), 7.10-7.36 (11H, m, ³J_{HH}=2Hz, ArH Ph, ArH p-CON), 6.94 (1H, d, ⁴J_{HH}=9Hz, ArH p-CON), 6.67 (1H, s, CHAr₂), 4.78 (1H, s, NHBoc), 4.10(2H, q, ³J_{HH}=7Hz, CH₂O) 3.95 (2H, dd, J_{HH}=106Hz, J_{HH}=15Hz, COCH₂CN), 3.82 (2H, dm, ʼ³J_{HH}=7Hz, ʼ³J_{HH}=59Hz, CH₂N), 3.19 (2H, q, ³J_{HH}=3Hz, CH₂NHBoc), 2.54 (2H, dm, ³J_{HH}=7Hz, ³J_{HH}=21Hz, CH₂CO), 2.37 (2H, t, ³J_{HH}=7Hz, CH₂CC), 1.71 (2H, m, ³J_{HH}=7Hz, CH₂CH₂CH₂), 1.38 (9H, s, Ch₃ Boc), 1.22 (3H, t, ³J_{HH}=7Hz, CH₃CH₂O).

Using the above procedure, but substituting the appropriate 7-iodo-3,4-dihydro-1H-1,4-benzodiazepine-2,5-dione for 1-(diphenylmethyl)-4-(2-carboxyethyl)-7-iodo-3,4-dihydro-1H-1,4-benzodiazepine-2,5-dione ethyl ester and alkyne for N-boc-5-amino-1-pentyne there may be prepared, for example, the following compounds:
1-(diphenylmethyl)-4-(2-carboxyethyl)-7-(N-boc-6-amino-1-hexynl)-3,4-dihydro-1H-1,4-benzodiazepine-2,5-dione ethyl ester,
1-(diphenylmethyl)-4-(2-carboxyethyl)-7-(N-boc-4-amino-1-butynl)-3,4-dihydro-1H-1,4-benzodiazepine-2,5-dione ethyl ester,
1-(1-napthyl)methyl-4-(2-carboxyethyl)-7-(N-boc-5-amino-1-pentynl)-3,4-dihydro-1H-1,4-benzodiazepine-2,5-dione ethyl ester,
1-(1-napthyl)methyl-4-(2-carboxyethyl)-7-(N-boc-6-amino-1-hexynl)-3,4-dihydro-1H-1,4-benzodiazepine-2,5-dione ethyl ester,
1-(2-napthyl)methyl-4-(2-carboxyethyl)-7-(N-boc-5-amino-1-pentynl)-3,4-dihydro-1H-1,4-benzodiazepine-2,5-dione ethyl ester,
1-(2-napthyl)methyl-4-(2-carboxyethyl)-7-(N-boc-6-amino-1-hexynl)-3,4-dihydro-1H-1,4-benzodiazepine-2,5-dione ethyl ester,
1-(*p*-phenoxybenzyl)-4-(2-carboxyethyl)-7-(N-boc-5-amino-1-pentynl)-3,4-dihydro-1H-1,4-benzodiazepine-2,5-dione ethyl ester,
1-(*p*-phenoxybenzyl)-4-(2-carboxyethyl)-7-(N-boc-6-amino-1-hexynl)-3,4-dihydro-1H-1,4-benzodiazepine-2,5-dione ethyl ester,
1-(*m*-phenoxybenzyl)-4-(2-carboxyethyl)-7-(N-boc-5-amino-1-pentynl)-3,4-dihydro-1H-1,4-benzodiazepine-2,5-dione ethyl ester,
1-(*m*-phenoxybenzyl)-4-(2-carboxyethyl)-7-(N-boc-6-amino-1-hexynl)-3,4-dihydro-1H-1,4-benzodiazepine-2,5-dione ethyl ester,
1-(*p*-trifluoromethylbenzyl)-4-(2-carboxyethyl)-7-(N-boc-5-amino-1-pentynl)-3,4-dihydro-1H-1,4-benzodiazepine-2,5-dione ethyl ester,
1-(*p*-trifluoromethylbenzyl)-4-(2-carboxyethyl)-7-(N-boc-6-amino-1-hexynl)-3,4-dihydro-1H-1,4-benzodiazepine-2,5-dione ethyl ester,
1-(ethyl-5-valeroyl)-4-(2-carboxyethyl)-7-(N-boc-5-amino-1-pentynl)-3,4-dihydro-1H-1,4-benzodiazepine-2,5-dione ethyl ester,
1-(ethyl-5-valeroyl)-4-(2-carboxyethyl)-7-(N-boc-6-amino-1-hexynl)-3,4-dihydro-1H-1,4-benzodiazepine-2,5-dione ethyl ester,
1-(*p*-methoxybenzyl)-4-(2-carboxyethyl)-7-(N-boc-5-amino-1-pentynl)-3,4-dihydro-1H-1,4-benzodiazepine-2,5-dione ethyl ester,
1-(p-methoxybenzyl)-4-(2-carboxyethyl)-7-(N-boc-6-amino-1-hexynl)-3,4-dihydro-1H-1,4-benzodiazepine-2,5-dione ethyl ester,
1-(*p*-methylmethylbenzoyl)-4-(2-carboxethyl)-7-(N-boc-5-amino-1-pentynl)-3,4-dihydro-1H-1,4-benzodiazepine-2,5-dione ethyl ester,
1-(*p*-methylmethylbenzoyl)-4-(2-carboxyethyl)-7-(N-boc-6-amino-1-hexynl)-3,4-dihydro-1H-1,4-benzodiazepine-2,5-dione ethyl ester,
1-(2-napthyl)methyl-4-(3(S)-butanoate)-7-(N-boc-5-amino-1-pentynl)-3,4-dihydro-1H-1,4-benzodiazepine-2,5-dione methyl ester,
1-(2-napthyl)methyl-4-(3(S)-butanoate)-7-(N-boc-6-amino-1-hexynl)-3,4-dihydro-1H-1,4-benzodiazepine-2,5-dione methyl ester,
1-(2-napthyl)methyl-4-(3(R)-butanoate)-7-(N-boc-5-amino-1-pentynl)-3,4-dihydro-1H-1,4-benzodiazepine-2,5-dione methyl ester,
1-(2-napthyl)methyl-4-(3(R)-butanoate)-7-(N-boc-6-amino-1-hexynl)-3,4-dihydro-1H-1,4-benzodiazepine-2,5-dione methyl ester,
1-(2-napthyl)methyl-4-((+/-)3-butanoate)-7-(N-boc-5-amino-1-pentynl)-3,4-dihydro-1H-1,4-benzodiazepine-2,5-dione methyl ester,
1-(2-napthyl)methyl-4-((+/-)3-butanoate)-7-(N-boc-6-amino-1-hexynl)-3,4-dihydro-1H-1,4-benzodiazepine-2,5-dione methyl ester,
1-(2-napthyl)methyl-4-(3(S)-phenyl-3-proprionate)-7-(N-boc-5-amino-1-pentynl)-3,4-dihydro-1H-1,4-benzodiazepine-2,5-dione methyl ester,
1-(2-napthyl)methyl-4-(3(S)-phenyl-3-proprionate)-7-( N-boc-6-amino-1-hexynl)-3,4-dihydro-1H-1,4-benzodiazepine-2,5-dione methyl ester,
1-(2-napthyl)methyl-4-(3(R)-phenyl-3-proprionate)-7-(N-boc-5-amino-1-pentynl)-3,4-dihydro-1H-1,4-benzodiazepine-2,5-dione methyl ester,
1-(2-napthyl)methyl-4-(3(R)-phenyl-3-proprionate)-7-(N-boc-6-amino-1-hexynl)-3,4-dihydro-1H-1,4-benzodiazepine-2,5-dione methyl ester,
1-(2-napthyl)methyl-4-((+/-)3-phenyl-3-proprionate)-7-(N-boc-5-amino-1-pentynl)-3,4-dihydro-1H-1,4-benzodiazepine-2,5-dione methyl ester,
1-(2-napthyl)methyl-4-((+/-)3-phenyl-3-proprionate)-7-(N-boc-6-amino-1-hexynl)-3,4-dihydro-1H-1,4-benzodiazepine-2,5-dione methyl ester.

d) To a magnetically stirred solution of 66 mgs of 1-(diphenylmethyl)-4-(2-carboxyethyl)-8-(N-boc-5-amino-1-pentynl)-3,4-dihydro-1H-1,4-benzodiazepine-2,5-dione ethyl ester (0.11mmol) in 2mL ethyl acetate, covered with an atmosphere of hydrogen, was added 23 mgs of palladium /carbon catalyst. The mixture was stirred for 1 hr. before filtering the mixture through Celite® and concentrating *in vacuo* to yield quantitative yield of 1-(diphenylmethyl)-4-(2-carboxyethyl)-8-(N-boc-5-aminopentyl)-3,4-dihydro-1H-1,4-benzodiazepine-2,5-dione ethyl ester. ¹H NMR (CDCl₃, dTMS) 7.54 (1H, d, ⁴J_{HH}=2Hz, Ar-H o-CON), 6.90-7.38 (12H, m, ³J_{HH}=2Hz, Ar-H Ph, ArH p-CON, ArH m-CON), 6.54 (1H, s, NCHAr₂), 4.64 (1H, s, NHBoc), 4.12 (2H, q, ³J_{HH}=7Hz, CH₂O) 3.97 (2H, dd, J_{HH}=129Hz, J_{HH}=15Hz, COCH₂CN), 3.82 (2H, dm, ʼ³J_{HH}=6Hz, ʼ³J_{HH}=75Hz, CH₂N), 3.07 (2H, q, ³J_{HH}=3Hz, CH₂NHBoc), 2.55 (4H, dm, t, ³J_{HH}=7Hz, ³J_{HH}=30Hz, ³J_{HH}=7Hz, CH₂CO, CH₂Ar), 1.3-1.6 (4H, m, m, ³J_{HH}=7Hz, ³J_{HH}=7Hz, CH₂CH₂Ar, CH₂CH₂NHBoc), 1.1-1.3 (14H, s, m, t, ³J_{HH}=7Hz, ³J_{HH}=7Hz, CH₃ Boc, CH₂CH₂CH₂NHBoC, CH₃CH₂).

Using the above procedure, but substituting the appropriate 4-(2-carboxyethyl)-7-(N-boc-amino-1-alkynl)-3,4-dihydro-1H-1,4-benzodiazepine-2,5-dione ethyl ester for 1-(diphenylmethyl)-4-(2-carboxyethyl)-7-(N-boc-5-amino-1-pentynl)-3,4-dihydro-1H-1,4-benzodiazepine-2,5-dione ethyl ester there may be prepared, for example, the following compounds:
1-(diphenylmethyl)-4-(2-carboxyethyl)-7-(N-boc-6-aminohexyl)-3,4-dihydro-1H-1,4-benzodiazepine-2,5-dione ethyl ester,
1-(diphenylmethyl)-4-(2-carboxyethyl)-7-(N-boc-4-aminobutyl)-3,4-dihydro-1H-1,4-benzodiazepine-2,5-dione ethyl ester,
1-(1-napthyl)methyl-4-(2-carboxyethyl)-7-(N-boc-5-aminopentyl)-3,4-dihydro-1H-1,4-benzodiazepine-2,5-dione ethyl ester,
1-(1-napthyl)methyl-4-(2-carboxyethyl)-7-(N-boc-6-aminohexyl)-3,4-dihydro-1H-1,4-benzodiazepine-2,5-dione ethyl ester,
1-(2-napthyl)methyl-4-(2-carboxyethyl)-7-(N-boc-5-aminopentyl)-3,4-dihydro-1H-1,4-benzodiazepine-2,5-dione ethyl ester,
1-(2-napthyl)methyl-4-(2-carboxyethyl)-7-(N-boc-6-aminohexyl)-3,4-dihydro-1H-1,4-benzodiazepine-2,5-dione ethyl ester,
1-(*p*-phenoxybenzyl)-4-(2-carboxyethyl)-7-(N-boc-5-aminopentyl)-3,4-dihydro-1H-1,4-benzodiazepine-2,5-dione ethyl ester,
1-(*p*-phenoxybenzyl)-4-(2-carboxyethyl)-7-(N-boc-6-aminohexyl)-3,4-dihydro-1H-1,4-benzodiazepine-2,5-dione ethyl ester,
1-(*m*-phenoxybenzyl)-4-(2-carboxyethyl)-7-(N-boc-5-aminopentyl)-3,4-dihydro-1H-1,4-benzodiazepine-2,5-dione ethyl ester,
1-(*m*-phenoxybenzyl)-4-(2-carboxyethyl)-7-(N-boc-6-aminohexyl)-3,4-dihydro-1H-1,4-benzodiazepine-2,5-dione ethyl ester,
1-(*p*-trifluoromethylbenzyl)-4-(2-carboxyethyl)-7-(N-boc-5-aminopentyl)-3,4-dihydro-1H-1,4-benzodiazepine-2,5-dione ethyl ester,
1-(*p*-trifluoromethylbenzyl)-4-(2-carboxyethyl)-7-(N-boc-6-amino hexyl)-3,4-dihydro-1H-1,4-benzodiazepine-2,5-dione ethyl ester,
1-(ethyl-5-valeroyl)-4-(2-carboxyethyl)-7-(N-boc-5-aminopentyl)-3,4-dihydro-1H-1,4-benzodiazepine-2,5-dione ethyl ester,
1-(ethyl-5-valeroyl)-4-(2-carboxyethyl)-7-(N-boc-6-aminohexyl)-3,4-dihydro-1H-1,4-benzodiazepine-2,5-dione ethyl ester,
1-(*p*-methoxybenzyl)-4-(2-carboxyethyl)-7-(N-boc-5-aminopentyl)-3,4-dihydro-1H-1,4-benzodiazepine-2,5-dione ethyl ester,
1-(*p*-methoxybenzyl)-4-(2-carboxyethyl)-7-(N-boc-6-aminohexyl)-3,4-dihydro-1H-1,4-benzodiazepine-2,5-dione ethyl ester,
1-(*p*-methylmethylbenzoyl)-4-(2-carboxyethyl)-7-(N-boc-5-amino pentyl)-3,4-dihydro-1H-1,4-benzodiazepine-2,5-dione ethyl ester,
1-(*p*-methylmethylbenzoyl)-4-(2-carboxyethyl)-7-(N-boc-6-amino hexyl)-3,4-dihydro-1H-1,4-benzodiazepine-2,5-dione ethyl ester,
1-(2-napthyl)methyl-4-(3(S)-butanoate)-7-(N-boc-5-amino-1-pentyl)-3,4-dihydro-1H-1,4-benzodiazepine-2,5-dione methyl ester,
1-(2-napthyl)methyl-4-(3(S)-butanoate)-7-(N-boc-6-amino-1-hexyl)-3,4-dihydro-1H-1,4-benzodiazepine-2,5-dione methyl ester,
1-(2-napthyl)methyl-4-(3(R)-butanoate)-7-(N-boc-5-amino-1-pentyl)-3,4-dihydro-1H-1,4-benzodiazepine-2,5-dione methyl ester,
1-(2-napthyl)methyl-4-(3(R)-butanoate)-7-(N-boc-6-amino-1-hexyl)-3,4-dihydro-1H-1,4-benzodiazepine-2,5-dione methyl ester,
1-(2-napthyl)methyl-4-((+/-)3-butanoate)-7-(N-boc-5-amino-1-pentyl)-3,4-dihydro-1H-1,4-benzodiazepine-2,5-dione methyl ester,
1-(2-napthyl)methyl-4-((+/-)3-butanoate)-7-(N-boc-6-amino-1-hexyl)-3,4-dihydro-1H-1,4-benzodiazepine-2,5-dione methyl ester,
1-(2-napthyl)methyl-4-(3(S)-phenyl-3-proprionate)-7-(N-boc-5-amino-1-pentyl)-3,4-dihydro-1H-1,4-benzodiazepine-2,5-dione methyl ester,
1-(2-napthyl)methyl-4-(3(S)-phenyl-3-proprionate)-7-(N-boc-6-amino-1-hexyl)-3,4-dihydro-1H-1,4-benzodiazepine-2,5-dione methyl ester,
1-(2-napthyl)methyl-4-(3(R)-phenyl-3-proprionate)-7-(N-boc-5-amino-1-pentyl)-3,4-dihydro-1H-1,4-benzodiazepine-2,5-dione methyl ester,
1-(2-napthyl)methyl-4-(3(R)-phenyl-3-proprionate)-7-(N-boc-6-amino-1-hexyl)-3,4-dihydro-1H-1,4-benzodiazepine-2,5-dione methyl ester,
1-(2-napthyl)methyl-4-((+/-)3-phenyl-3-proprionate)-7-(N-boc-5-amino-1-pentyl)-3,4-dihydro-1H-1,4-benzodiazepine-2,5-dione methyl ester,
1-(2-napthyl)methyl-4-((+/-)3-phenyl-3-proprionate)-7-(N-boc-6-amino-1-hexyl)-3,4-dihydro-1H-1,4-benzodiazepine-2,5-dione methyl ester.

e) To a solution of 33 mgs of 1-(diphenylmethyl)-4-(2-carboxyethyl)-7-(N-boc-5-aminopentyl)-3,4-dihydro-1H-1,4-benzodiazepine-2,5-dione ethyl ester (0.05mmol) in 2mL ethyl acetate was added 0.4mL triethylsilane (2.5mmol) and 6mL of saturated HCl/ethyl acetate at 5°C. After 1 hour at 5°C the mixture was concentrated *in vacuo* and diluted with 2mL of methanol. To the methanolic solution was added 2mL of 2N sodium hydroxide. After 1 hour, the reaction was quenched with 1/2mL of acetic acid, concentrated *in vacuo,* diluted with 5mL of methanol and water and purified by high pressure liquid chromatography, using a 1/2" C-18 reverse phase column, eluting with a solvent gradient of 30:70 methanol (0.1% trifluoroacetic acid)/water (0.1% trifluoroacetic acid), time 0 to 10 minutes, to 70:30 methanol (0.1% trifluoroacetic acid)/water (0.1% trifluoroacetic acid), time 10 minutes to 40 minutes, flow = 10mL/min. (Rₜ = 37.5 min., uv detection 254nm) to yield 14 mgs (46%) of 1-(diphenylmethyl)-4-(2-carboxyethyl)-7-(5-aminopentyl)-3,4-dihydro-1H-1,4-benxodiazepine-2,5-dione trifluoroacetate. HRMS (FAB) molecular ion m/z=500.2529 (calc. C₃₀N₃O₄H₃₃, 500.2549). ¹H NMR (CDCl₃, TMS) 6.60-7.20 (13H, m, ³J_{HH}=2Hz, ArH Ph, ArH p-CON, ArH *o*-CON, ArH *m*-CON), 6.33 (1H, s, NCH-Ar₂), 3.65 (2H, dd, J_{HH}=113Hz, J_{HH}=15Hz, COCH₂CN), 3.50 (2H, dm, ʼ³J_{HH}=7Hz, ʼ³J_{HH}=59Hz, CH₂N), 2.66 (2H, t, ³J_{HH}=8Hz, CH₂NH₂) 2.22-2.39 (4H, m, t, ³)_{HH}=6Hz, ³J_{HH}=8Hz, CH₂CO, CH₂Ar), 1.20-1.33(4H, m, m, ³J_{HH}=7Hz, CH₂CH₂Ar, CH₂CH₂NH₂), 1.00 (2H, m, ³J_{HH}=7Hz, CH₂CH₂CH₂NH₂).

Using the above procedure, but substituting the appropriate 3,4-dihydro-1H-1,4-benzodiazepine-2,5-dione for 1-(diphenylmethyl)-4-(2-carboxyethyl)-7-(N-boc-5-aminopentyl)-3,4-dihydro-1H-1,4-benzodiazepine-2,5-dione ethyl ester there may be prepared, for example, the following compounds:
1-(diphenylmethyl)-4-(2-carboxyethyl)-7-(6-aminohexyl)-3,4-dihydro-1H-1,4-benzodiazepine-2,5-dione trifluoroacetate,
1-(diphenylmethyl)-4-(2-carboxyethyl)-7-(4-aminobutyl)-3,4-dihydro-1H-1,4-benzodiazepine-2,5-dione trifluoroacetate,
1-(1-napthyl)methyl-4-(2-carboxyethyl)-7-(5-aminopentyl)-3,4-dihydro-1H-1,4-benzodiazepine-2,5-dione trifluoroacetate,
1-(1-napthyl)methyl-4-(2-carboxyethyl)-7-(6-aminohexyl)-3,4-dihydro-1H-1,4-benzodiazepine-2,5-dione trifluoroacetate,
1-(2-napthyl)methyl-4-(2-carboxyethyl)-7-(5-aminopentyl)-3,4-dihydro-1H-1,4-benzodiazepine-2,5-dione trifluoroacetate,
1-(2-napthyl)methyl-4-(2-carboxyethyl)-7-(6-aminohexyl)-3,4-dihydro-1H-1,4-benzodiazepine-2,5-dione trifluoroacetate,
1-(*p*-phenoxybenzyl)-4-(2-carboxyethyl)-7-(5-aminopentyl)-3,4-dihydro-1H-1,4-benzodiazepine-2,5-dione trifluoroacetate,
1-(*p*-phenoxybenzyl)-4-(2-carboxyethyl)-7-(6-aminohexyl)-3,4-dihydro-1H-1,4-benzodiazepine-2,5-dione trifluoroacetate,
1-(*m*-phenoxybenzyl)-4-(2-carboxyethyl)-7-(5-aminopentyl)-3,4-dihydro-1H-1,4-benzodiazepine-2,5-dione trifluoroacetate,
1-(*m*-phenoxybenzyl)-4-(2-carboxyethyl)-7-(6-aminohexyl)-3,4-dihydro-1H-1,4-benzodiazepine-2,5-dione trifluoroacetate,
1-(*p*-trifluoromethylbenzyl)-4-(2-carboxyethyl)-7-(5-aminopentyl)-3,4-dihydro-1H-1,4-benzodiazepine-2,5-dione trifluoroacetate,
1-(*p*-trifluoromethylbenzyl)-4-(2-carboxyethyl)-7-(6-aminohexyl)-3,4-dihydro-1H-1,4-benzodiazepine-2,5-dione trifluoroacetate,
1-(ethyl-5-valeroyl)-4-(2-carboxyethyl)-7-(5-aminopentyl)-3,4-dihydro-1H-1,4-benzodiazepine-2,5-dione trifluoroacetate,
1-(ethyl-5-valeroyl)-4-(2-carboxyethyl)-7-(6-aminohexy l)-3,4-dihydro-1H-1,4-benzodiazepine-2,5-dione trifluoroacetate,
1-(*p*-methoxybenzyl)-4-(2-carboxyethyl)-7-(5-aminopentyl)-3,4-dihydro-1H-1,4-benzodiazepine-2,5-dione trifluoroacetate,
1-(*p*-methoxybenzyl)-4-(2-carboxyethyl)-7-(6-aminohexyl)-3,4-dihydro-1H-1,4-benzodiazepine-2,5-dione trifluoroacetate,
1-(*p*-methylmethylbenzoyl)-4-(2-carboxyethyl)-7-(5-aminopentyl)-3,4-dihydro-1H-1,4-benzodiazepine-2,5-dione trifluoroacetate,
1-(*p*-methylmethylbenzoyl)-4-(2-carboxyethyl)-7-(6-aminohexyl)-3,4-dihydro-1H-1,4-benzodiazepine-2,5-dione trifluoroacetate,
1-(diphenylmethyl)-4-(2-carboxyethyl)-7-(6-amino-1-hexynl)-3,4-dihydro-1H-1,4-benzodiazepine-2,5-dione trifluoroacetate,
1-(diphenylmethyl)-4-(2-carboxyethyl)-7-(4-amino-1-butynl)-3,4-dihydro-1H-1,4-benzodiazepine-2,5-dione trifluoroacetate,
1-(1-napthyl)methyl-4-(2-carboxyethyl)-7-(5-amino-1-pentynl)-3,4-dihydro-1H-1,4-benzodiazepine-2,5-dione trifluoroacetate,
1-(1-napthyl)methyl-4-(2-carboxyethyl)-7-(6-amino-1-hexynl)-3,4-dihydro-1H-1,4-benzodiazepine-2,5-dione trifluoroacetate,
1-(2-napthyl)methyl-4-(2-carboxyethyl)-7-(5-amino-1-pentynl)-3,4-dihydro-1H-1,4-benzodiazepine-2,5-dione trifluoroacetate,
1-(2-napthyl)methyl-4-(2-carboxyethyl)-7-(6-amino-1-hexynl)-3,4-dihydro-1H-1,4-benzodiazepine-2,5-dione trifluoroacetate,
1-(*p*-phenoxybenzyl)-4-(2-carboxyethyl)-7-(5-amino-1-pentynl)-3,4-dihydro-1H-1,4-benzodiazepine-2,5-dione trifluoroacetate,
1-(*p*-phenoxybenzyl)-4-(2-carboxyethyl)-7-(6-amino-1-hexynl)-3,4-dihydro-1H-1,4-benzodiazepine-2,5-dione trifluoroacetate,
1-(*m*-phenoxybenzyl)-4-(2-carboxyethyl)-7-(5-amino-1-pentynl)-3,4-dihydro-1H-1,4-benzodiazepine-2,5-dione trifluoroacetate,
1-(*m*-phenoxybenzyl)-4-(2-carboxyethyl)-7-(6-amino-1-hexynl)-3,4-dihydro-1H-1,4-benzodiazepine-2,5-dione trifluoroacetate,
1-(*p*-trifluoromethylbenzyl)-4-(2-carboxyethyl)-7-(5-amino-1-pentynl)-3,4-dihydro-1H-1,4-benzodiazepine-2,5-dione trifluoroacetate,
1-(*p*-trifluoromethylbenzyl)-4-(2-carboxyethyl)-7-(6-amino-1-hexynl)-3,4-dihydro-1H-1,4-benzodiazepine-2,5-dione trifluoroacetate,
1-(ethyl-5-valeroyl)-4-(2-carboxyethyl)-7-(5-amino-1-pentynl)-3,4-dihydro-1H-1,4-benzodiazepine-2,5-dione trifluoroacetate,
1-(ethyl-5-valeroyl)-4-(2-carboxyethyl)-7-(6-amino-1-hexynl)-3,4-dihydro-1H-1,4-benzodiazepine-2,5-dione trifluoroacetate,
1-(*p*-methoxybenzyl)-4-(2-carboxyethyl)-7-(5-amino-1-pentynl)-3,4-dihydro-1H-1,4-benzodiazepine-2,5-dione trifluoroacetate,
1-(*p*-methoxybenzyl)-4-(2-carboxyethyl)-7-(6-amino-1-hexynl)-3,4-dihydro-1H-1,4-benzodiazepine-2,5-dione trifluoroacetate,
1-(*p*-methylmethylbenzoyl)-4-(2-carboxyethyl)-7-(5-amino-1-pentynl)-3,4-dihydro-1H-1,4-benzodiazepine-2,5-dione trifluoroacetate,
1-(*p*-methylmethylbenzoyl)-4-(2-carboxyethyl)-7-(6-amino-1-hexynl)-3,4-dihydro-1H-1,4-benzodiazepine-2,5-dione trifluoroacetate,
1-(2-napthyl)methyl-4-(3(S)-butanoate)-7-(5-amino-1-pentyl)-3,4-dihydro-1H-1,4-benzodiazepine-2,5-dione trifluoroacetate,
1-(2-napthyl)methyl-4-(3(S)-butanoate)-7-(6-amino-1-hexyl)-3,4-dihydro-1H-1,4-benzodiazepine-2,5-dionetrifluoroacetate,
1-(2-napthyl)methyl-4-(3(R)-butanoate)-7-(5-amino-1-pentyl)-3,4-dihydro-1H-1,4-benzodiazepine-2,5-dionetrifluoroacetate,
1-(2-napthyl)methyl-4-(3(R)-butanoate)-7-(6-amino-1-hexyl)-3,4-dihydro-1H-1,4-benzodiazepine-2,5-dione trifluoroacetate,
1-(2-napthyl)methyl-4-((+/-)3-butanoate)-7-(5-amino-1-pentyl)-3,4-dihydro-1H-1,4-benzodiazepine-2,5-dione trifluoroacetate,
1-(2-napthyl)methyl-4-((+/-)3-butanoate)-7-(6-amino-1-hexyl)-3,4-dihydro-1H-1,4-benzodiazepine-2,5-dione trifluoroacetate,
1-(2-napthyl)methyl-4-(3(S)-phenyl-3-proprionate)-7-(5-amino-1-pentyl)-3,4-dihydro-1H-1,4-benzodiazepine-2,5-dione trifluoroacetate,
1-(2-napthyl)methyl-4-(3(S)-phenyl-3-proprionate)-7-(6-amino-1-hexyl)-3,4-dihydro-1H-1,4-benzodiazepine-2,5-dione trifluoroacetate,
1-(2-napthyl)methyl-4-(3(R)-phenyl-3-proprionate)-7-(5-amino-1-pentyl)-3,4-dihydro-1H-1,4-benzodiazepine-2,5-dione trifluoroacetate,
1-(2-napthyl)methyl-4-(3(R)-phenyl-3-proprionate)-7-(6-amino-1-hexyl)-3,4-dihydro-1H-1,4-benzodiazepine-2,5-dione trifluoroacetate,
1-(2-napthyl)methyl-4-((+/-)3-phenyl-3-proprionate)-7-(5-amino-1-pentyl)-3,4-dihydro-1H-1,4-benzodiazepine-2,5-dione trifluoroacetate,
1-(2-napthyl)methyl-4-((+/-)3-phenyl-3-proprionate)-7-(6-amino-1-hexyl)-3,4-dihydro-1H-1,4-benzodiazepine-2,5-dione trifluoroacetate,
1-(2-napthyl)methyl-4-(3(S)-butanoate)-7-(5-amino-1-pentynl)-3,4-dihydro-1H-1,4-benzodiazepine-2,5-dione trifluoroacetate,
1-(2-napthyl)methyl-4-(3(S)-butanoate)-7-(6-amino-1-hexynl)-3,4-dihydro-1H-1,4-benzodiazepine-2,5-dione trifluoroacetate,
1-(2-napthyl)methyl-4-(3(R)-butanoate)-7-(5-amino-1-pentynl)-3,4-dihydro-1H-1,4-benzodiazepine-2,5-dione trifluoroacetate,
1-(2-napthyl)methyl-4-(3(R)-butanoate)-7-(6-amino-1-hexynl)-3,4-dihydro-1H-1,4-benzodiazepine-2,5-dione trifluoroacetate,
1-(2-napthyl)methyl-4-((+/-)3-butanoate)-7-(5-amino-1-pentynl)-3,4-dihydro-1H-1,4-benzodiazepine-2,5-dione trifluoroacetate,
1-(2-napthyl)methyl-4-((+/-)3-butanoate)-7-(6-amino-1-hexynl)-3,4-dihydro-1H-1,4-benzodiazepine-2,5-dione trifluoroacetate,
1-(2-napthyl)methyl-4-(3(S)-phenyl-3-proprionate)-7-(5-amino-1-pentynl)-3,4-dihydro-1H-1,4-benzodiazepine-2,5-dione trifluoroacetate,
1-(2-napthyl)methyl-4-(3(S)-phenyl-3-proprionate)-7-(6-amino-1-hexynl)-3,4-dihydro-1H-1,4-benzodiazepine-2,5-dione trifluoroacetate,
1-(2-napthyl)methyl-4-(3(R)-phenyl-3-proprionate)-7-(5-amino-1-pentynl)-3,4-dihydro-1H-1,4-benzodiazepine-2,5-dione trifluoroacetate,
1-(2-napthyl)methyl-4-(3(R)-phenyl-3-proprionate)-7-(6-amino-1-hexynl)-3,4-dihydro-1H-1,4-benzodiazepine-2,5-dione trifluoroacetate,
1-(2-napthyl)methyl-4-((+/-)3-phenyl-3-proprionate)-7-(5-amino-1-pentynl)-3,4-dihydro-1H-1,4-benzodiazepine-2,5-dione trifluoroacetate,
1-(2-napthyl)methyl-4-((+/-)3-phenyl-3-proprionate)-7-(6-amino-1-hexynl)-3,4-dihydro-1H-1,4-benzodiazepine-2,5-dione trifluoroacetate.

### Example 15

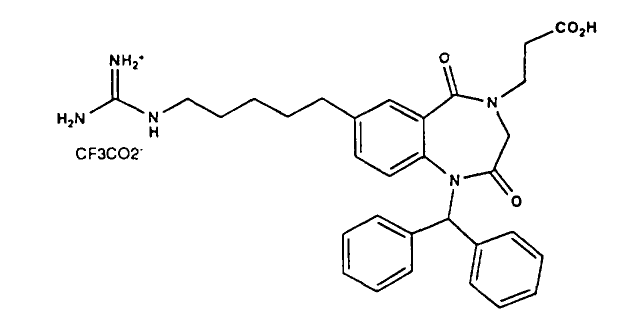

*1-(diphenylmethyl)-4-(2-carboxyethyl)-7-(5-guanidinopentyl)-3,4-dihydro-1H-1,4-benxodiazepine-2,5-dione trifluoroacetate*

a) To a solution of 11 mgs of 1-(diphenylmethyl)-4-(2-carboxyethyl)-7-(5-aminopentyl)-3,4-dihydro-1H-1,4-benxodiazepine-2,5-dione trifluoroacetate (0.02mmol) in 2mL of methanol was added 1mL of 5% potassium bicarbonate and 17 mgs of aminoiminomethanesulfonic acid (0.12mmol). After 30 minutes, the reaction mixture was quenched with 0.5mL of acetic acid and concentrated *in* v*acuo.* The resulting residue was diluted with 5mL of water and methanol and purified by high pressure liquid chromatography, using a 1/2" C-18 reverse phase column, eluting with a solvent gradient of 30:70 methanol (0.1% trifluoroacetic acid)/water (0.1% trifluoroacetic acid), time 0 to 10 minutes, to 70:30 methanol (0,1% trifluoroacetic acid)/water (0.1% trifluoroacetic acid), time 10 minutes to 40 minutes, flow = 10mL/min. (Rₜ = 40.5 min., uv detection 254nm) to yield 9.3 mg (71%) of 1-(diphenylmethyl)-4-(2-carboxyethyl)-7-(5-guanidinopentyl)-3,4-dihydro-1H-1,4-benzodiazepine-2,5-dione trifluoroacetate. HRMS (FAB) molecular ion m/z=542.2750 (calc. C₃₁N₅O₄H₃₅, 542.2767). ¹H NMR (CDCl₃, dTMS) 6.78-7.22 (13H, m, ³J_{HH}=2Hz, ArH Ph, ArH p-CON, ArH *o*-CON, ArH *m*-CON), 6.45 (1H, s, NCHAr₂), 3.83 (2H, dd, J_{HH}=113Hz, J_{HH}=15Hz, COCH₂CN), 3.65 (2H, dm, ʼ³J_{HH}=7Hz, ʼ³J_{HH}=59Hz, CH₂N), 2.85 (2H, t, ³J_{HH}=8Hz, CH₂NH₂) 2.25-2.45 (4H, t, t, CH₂CO, CH₂Ar), 1.31 (4H, m, m, ³J_{HH}=7Hz, CH₂CH₂Ar, CH₂CH₂NH₂), 0.98 (2H, m, ³J_{HH}=7Hz, CH₂CH₂CH₂NH₂).

Using the above procedure, but substituting the appropriate amino acid for 1-(diphenylmethyl)-4-(2-carboxyethyl)-7-(5-aminopentyl)-3,4-dihydro-1H-1,4-benxodiazepine-2,5-dione trifluoroacetate there may be prepared, for example, the following compounds:
1-(diphenylmethyl)-4-(2-carboxyethyl)-7-(6-guanidinohexyl)-3,4-dihydro-1H-1,4-benzodiazepine-2,5-dione trifluoroacetate,
1-(diphenylmethyl)-4-(2-carboxyethyl)-7-(4-guanidinobutyl)-3,4-dihydro-1H-1,4-benzodiazepine-2,5-dione trifluoroacetate,
1-(1-napthyl)methyl-4-(2-carboxyethyl)-7-(5-guanidinopentyl)-3,4-dihydro-1H-1,4-benzodiazepine-2,5-dione trifluoroacetate,
1-(1-napthyl)methyl-4-(2-carboxyethyl)-7-(6-guanidinohexyl)-3,4-dihydro-1H-1,4-benzodiazepine-2,5-dione trifluoroacetate,
1-(2-napthyl)methyl-4-(2-carboxyethyl)-7-(5-guanidinopentyl)-3,4-dihydro-1H-1,4-benzodiazepine-2,5-dione trifluoroacetate,
1-(2-napthyl)methyl-4-(2-carboxyethyl)-7-(6-guanidinohexyl)-3,4-dihydro-1H-1,4-benzodiazepine-2,5-dione trifluoroacetate,
1-(*p*-phenoxybenzyl)-4-(2-carboxyethyl)-7-(5-guanidinopentyl)-3,4-dihydro-1H-1,4-benzodiazepine-2,5-dione trifluoroacetate,
1-(*p*-phenoxybenzyl)-4-(2-carboxyethyl)-7-(6-guanidinohexyl)-3,4-dihydro-1H-1,4-benzodiazepine-2,5-dione trifluoroacetate,
1-(*m*-phenoxybenzyl)-4-(2-carboxyethyl)-7-(5-guanidinopentyl)-3,4-dihydro-1H-1,4-benzodiazepine-2,5-dione trifluoroacetate,
1-(*m*-phenoxybenzyl)-4-(2-carboxyethyl)-7-(6-guanidinohexyl)-3,4-dihydro-1H-1,4-benzodiazepine-2,5-dione trifluoroacetate,
1-(*p*-trifluoromethylbenzyl)-4-(2-carboxyethyl)-7-(5-guanidinopentyl)-3,4-dihydro-1H-1,4-benzodiazepine-2,5-dione trifluoroacetate,
1-(*p*-trifluoromethylbenzyl)-4-(2-carboxyethyl)-7-(6-guanidinohexyl)-3,4-dihydro-1H-1,4-benzodiazepine-2,5-dione trifluoroacetate,
1-(ethyl-5-valeroyl)-4-(2-carboxyethyl)-7-(5-guanidinopentyl)-3,4-dihydro-1H-1,4-benzodiazepine-2,5-dione trifluoroacetate,
1-(ethyl-5-valeroyl)-4-(2-carboxyethyl)-7-(6-guanidinohexy l)-3,4-dihydro-1H-1,4-benzodiazepine-2,5-dione trifluoroacetate,
1-(*p*-methoxybenzyl)-4-(2-carboxyethyl)-7-(5-guanidinopentyl)-3,4-dihydro-1H-1,4-benzodiazepine-2,5-dione trifluoroacetate,
1-(*p*-methoxybenzyl)-4-(2-carboxyethyl)-7-(6-guanidinohexyl)-3,4-dihydro-1H-1,4-benzodiazepine-2,5-dione trifluoroacetate,
1-(*p*-methylmethylbenzoyl)-4-(2-carboxyethyl)-7-(5-guanidinopentyl)-3,4-dihydro-1H-1,4-benzodiazepine-2,5-dione trifluoroacetate,
1-(*p*-methylmethylbenzoyl)-4-(2-carboxyethyl)-7-(6-guanidinohexyl)-3,4-dihydro-1H-1,4-benzodiazepine-2,5-dione trifluoroacetate,
1-(diphenylmethyl)-4-(2-carboxyethyl)-7-(6-guanidino-1-hexynl)-3,4-dihydro-1H-1,4-benzodiazepine-2,5-dione trifluoroacetate,
1-(diphenylmethyl)-4-(2-carboxyethyl)-7-(4-guanidino-1-butynl)-3,4-dihydro-1H-1,4-benzodiazepine-2,5-dione trifluoroacetate,
1-(1-napthyl)methyl-4-(2-carboxyethyl)-7-(5-guanidino-1-pentynl)-3,4-dihydro-1H-1,4-benzodiazepine-2,5-dione trifluoroacetate,
1-(1-napthyl)methyl-4-(2-carboxyethyl)-7-(6-guanidino-1-hexynl)-3,4-dihydro-1H-1,4-benzodiazepine-2,5-dione trifluoroacetate,
1-(2-napthyl)methyl-4-(2-carboxyethyl)-7-(5-guanidino-1-pentynl)-3,4-dihydro-1H-1,4-benzodiazepine-2,5-dione trifluoroacetate,
1-(2-napthyl)methyl-4-(2-carboxyethyl)-7-(6-guanidino-1-hexynl)-3,4-dihydro-1H-1,4-benzodiazepine-2,5-dione trifluoroacetate,
1-(*p*-phenoxybenzyl)-4-(2-carboxyethyl)-7-(5-guanidino-1-pentynl)-3,4-dihydro-1H-1,4-benzodiazepine-2,5-dione trifluoroacetate,
1-(*p*-phenoxybenzyl)-4-(2-carboxyethyl)-7-(6-guanidino-1-hexynl)-3,4-dihydro-1H-1,4-benzodiazepine-2,5-dione trifluoroacetate,
1-(*m*-phenoxybenzyl)-4-(2-carboxyethyl)-7-(5-guanidino-1-pentynl)-3,4-dihydro-1H-1,4-benzodiazepine-2,5-dione trifluoroacetate,
1-(*m*-phenoxybenzyl)-4-(2-carboxyethyl)-7-(6-guanidino-1-hexynl)-3,4-dihydro-1H-1,4-benzodiazepine-2,5-dione trifluoroacetate,
1-(*p*-trifluoromethylbenzyl)-4-(2-carboxyethyl)-7-(5-guanidino-1-pentynl)-3,4-dihydro-1H-1,4-benzodiazepine-2,5-dione trifluoroacetate,
1-(*p*-trifluoromethylbenzyl)-4-(2-carboxyethyl)-7-(6-guanidino-1-hexynl)-3,4-dihydro-1H-1,4-benzodiazepine-2,5-dione trifluoroacetate,
1-(ethyl-5-valeroyl)-4-(2-carboxyethyl)-7-(5-guanidino-1-pentynl)-3,4-dihydro-1H-1,4-benzodiazepine-2,5-dione trifluoroacetate,
1-(ethyl-5-valeroyl)-4-(2-carboxyethyl)-7-(6-guanidino-1-hexynl)-3,4-dihydro-1H-1,4-benzodiazepine-2,5-dione trifluoroacetate,
1-(*p*-methoxybenzyl)-4-(2-carboxyethyl)-7-(5-guanidino-1-pentynl)-3,4-dihydro-1H-1,4-benzodiazepine-2,5-dione trifluoroacetate,
1-(*p*-methoxybenzyl)-4-(2-carboxyethyl)-7-(6-guanidino-1-hexynl)-3,4-dihydro-1H-1,4-benzodiazepine-2,5-dione trifluoroacetate,
1-(*p*-methylmethylbenzoyl)-4-(2-carboxyethyl)-7-(5-guanidino-1-pentynl)-3,4-dihydro-1H-1,4-benzodiazepine-2,5-dione trifluoroacetate,
1-(*p*-methylmethylbenzoyl)-4-(2-carboxyethyl)-7-(6-guanidino-1-hexynl)-3,4-dihydro-1H-1,4-benzodiazepine-2,5-dione trifluoroacetate,
1-(2-napthyl)methyl-4-(3(S)-butanoate)-7-(5-guanidino-1-pentyl)-3,4-dihydro-1H-1,4-benzodiazepine-2,5-dione trifluoroacetate,
1-(2-napthyl)methyl-4-(3(S)-butanoate)-7-(6-guanidino-1-hexyl)-3,4-dihydro-1H-1,4-benzodiazepine-2,5-dione trifluoroacetate,
1-(2-napthyl)methyl-4-(3(R)-butanoate)-7-(5-guanidino-1-pentyl)-3,4-dihydro-1H-1,4-benzodiazepine-2,5-dione trifluoroacetate,
1-(2-napthyl)methyl-4-(3(R)-butanoate)-7-(6-guanidino-1-hexyl)-3,4-dihydro-1H-1,4-benzodiazepine-2,5-dione trifluoroacetate,
1-(2-napthyl)methyl-4-((+/-)3-butanoate)-7-(5-guanidino-1-pentyl)-3,4-dihydro-1H-1,4-benzodiazepine-2,5-dione trifluoroacetate,
1-(2-napthyl)methyl-4-((+/-)3-butanoate)-7-(6-guanidino-1-hexyl)-3,4-dihydro-1H-1,4-benzodiazepine-2,5-dione trifluoroacetate,
1-(2-napthyl)methyl-4-(3(S)-phenyl-3-proprionate)-7-(5-guanidino-1-pentyl)-3,4-dihydro-1H-1,4-benzodiazepine-2,5-dione trifluoroacetate,
1-(2-napthyl)methyl-4-(3(S)-phenyl-3-proprionate)-7-(6-guanidino-1-hexyl)-3,4-dihydro-1H-1,4-benzodiazepine-2,5-dione trifluoroacetate,
1-(2-napthyl)methyl-4-(3(R)-phenyl-3-proprionate)-7-(5-guanidino-1-pentyl)-3,4-dihydro-1H-1,4-benzodiazepine-2,5-dione trifluoroacetate,
1-(2-napthyl)methyl-4-(3(R)-phenyl-3-proprionate)-7-(6-guanidino-1-hexyl)-3,4-dihydro-1H-1,4-benzodiazepine-2,5-dione trifluoroacetate,
1-(2-napthyl)methyl-4-((+/-)3-phenyl-3-proprionate)-7-(5-guanidino-1-pentyl)-3,4-dihydro-1H-1,4-benzodiazepine-2,5-dione trifluoroacetate,
1-(2-napthyl)methyl-4-((+/-)3-phenyl-3-proprionate)-7-(6-guanidino-1-hexyl)-3,4-dihydro-1H-1,4-benzodiazepine-2,5-dione trifluoroacetate,
1-(2-napthyl)methyl-4-(3(S)-butanoate)-7-(5-guanidino-1-pentynl)-3,4-dihydro-1H-1,4-benzodiazepine-2,5-dione trifluoroacetate,
1-(2-napthyl)methyl-4-(3(S)-butanoate)-7-(6-guanidino-1-hexynl)-3,4-dihydro-1H-1,4-benzodiazepine-2,5-dione trifluoroacetate,
1-(2-napthyl)methyl-4-(3(R)-butanoate)-7-(5-guanidino-1-pentynl)-3,4-dihydro-1H-1,4-benzodiazepine-2,5-dione trifluoroacetate,
1-(2-napthyl)methyl-4-(3(R)-butanoate)-7-(6-guanidino-1-hexynl)-3,4-dihydro-1H-1,4-benzodiazepine-2,5-dione trifluoroacetate,
1-(2-napthyl)methyl-4-((+/-)3-butanoate)-7-(5-guanidino-1-pentynl)-3,4-dihydro-1H-1,4-benzodiazepine-2,5-dione trifluoroacetate,
1-(2-napthyl)methyl-4-((+/-)3-butanoate)-7-(6-guanidino-1-hexynl)-3,4-dihydro-1H-1,4-benzodiazepine-2,5-dione trifluoroacetate,
1-(2-napthyl)methyl-4-(3(S)-phenyl-3-proprionate)-7-(5-guanidino-1-pentynl)-3,4-dihydro-1H-1,4-benzodiazepine-2,5-dione trifluoroacetate,
1-(2-napthyl)methyl-4-(3(S)-phenyl-3-proprionate)-7-(6-guanidino-1-hexynl)-3,4-dihydro-1H-1,4-benzodiazepine-2,5-dione trifluoroacetate,
1-(2-napthyl)methyl-4-(3(R)-phenyl-3-proprionate)-7-(5-guanidino-1-pentynl)-3,4-dihydro-1H-1,4-benzodiazepine-2,5-dione trifluoroacetate,
1-(2-napthyl)methyl-4-(3(R)-phenyl-3-proprionate)-7-(6-guanidino-1-hexynl)-3,4-dihydro-1H-1,4-benzodiazepine-2,5-dione trifluoroacetate,
1-(2-napthyl)methyl-4-((+/-)3-phenyl-3-proprionate)-7-(5-guanidino-1-pentynl)-3,4-dihydro-1H-1,4-benzodiazepine-2,5-dione trifluoroacetate,
1-(2-napthyl)methyl-4-((+/-)3-phenyl-3-proprionate)-7-(6-guanidino-1-hexynl)-3,4-dihydro-1H-1,4-benzodiazepine-2,5-dione trifluoroacetate.

### Example 16

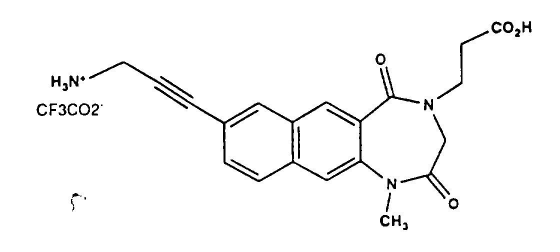

*1-methyl-4-(2-carboxyethyl)-8-(3-amino-1-propynyl)-3,4-dihydro 1H-1,4-napthodiazepine-2,4-dione trifluoracetate*

a) [4,5]-Benzoisatoic anhydride was prepared according to the method described in part (a) of Example 1. Thus, 35g of 3-amino-2-naphthoic acid (187mMol) was dissolved in 500mL of water containing 20g of potassium carbonate. A solution of phosgene in toluene (1.93M, 290mL) was added slowly with vigorous stirring. A precipitate appeared along with evolution of gas. Stirring was continued for 30 min after complete addition of the phosgene. The precipitate was collected by suction filtration. The solid material was washed thoroughly with water and hexane and air dried yielding 37.7g (95%) of [4,5]-benzoisatoic anhydride. M.p. >400°C. ¹H NMR (CDCL₃): 12.0, s, 1H; 8.95, s, 1H; 8.3, d, J=7Hz, 1H; 8.15, d, J=7Hz, 1H; 7.85, t, J=7Hz, 1H; 7.7, t, J=7Hz, 1H; 7,7, s, 1H.

b) 10.3g (48.3mMol) of [4,5]-benzoisatoic anhydride was dissolved in 50mL of anhydrous N,N-dimethylformamide (DMF) and added dropwise to an ice-cooled suspension of sodium hydride (97%, 1.25g) in 50mL of DMF with gas evolution. After stirring an additional 30min, 3.16mL (105mol%) of methyl iodide was added to the solution. After one hour, the reaction mixture was poured over ice and the precipitated product was collected by suction filtration and air dried, yielding 9.3g (85%) of N-methyl-[4,5]-benzoisatoic anhydride, a tan solid. M.p. 219-222°C. ¹H NMR (d⁶-DMSO): 8.8, s, 1H; 8.15, d, J=7Hz, 1H; 8.0, d, J=7Hz, 1H; 7.85, s, 1H; 7.7, t, J=7Hz, 1H; 7.55, t, J=7Hz, 1H; 3.35, s, 3H.

Using the above procedure, but substituting the appropriate alkyl halide for methyl iodide there may be prepared, for example, the following compounds:
N-benzyl-[4,5]-benzoisatoic anhydride, N-(2,4-diflurobenzyl)-[4,5]-benzoisatoic anhydride, N-(b-napthyl)methyl-[4,5]-benzoisatoic anhydride.

c) A solution of 20.0g (89mMol) of N-methyl-[4,5]-benzoisatoic anhydride, 20.5g (133.5mMol) of the hydrochloride salt of ethyl 3-aminopropionate, and 18.6mL (133.5mMol) of triethylamine in 100mL of DMF was stirred at 80°C for four hr. The reaction mixture was then poured over ice containing citric acid and the precipitated product was collected by suction filtration. The crude product was recrystallized from benzene/hexane, yielding 24g (60%) of 3-(N-methylamino)-2-[N-(2-carboxyethyl)]-naphthocarboxamide ethyl ester, a yellow crystalline solid. MS (FAB) 300.1. ¹H NMR (CDCL₃): 7.8, s, 1H; 7.65, d, J=7Hz, 1H; 7.6, d, J=7Hz, 1H; 7.4, t, J=7Hz, 1H; 7.2, t, J=7Hz, 1H; 6.9, br s, 1H; 6.8, s, 1H; 4.2, q, J=7Hz, 2H; 3.75, q, J=5Hz, 2H; 2.95, s, 3H; 2.65, t, J=5Hz, 2H; 1.3, t, J=7Hz, 3H.

Using the above procedure, but substituting the appropriate 3-aminopropionate for ethyl 3-aminopropionate there may be prepared, for example, the following compounds:
(±)-3-(N-methyl)amino-2-[N-(3-butanoate)]-naphthocarboxamide ethyl ester,
(±)-3-(N-methyl)amino-2-[N-(3-phenyl-3-proprionate)]-naphthocarboxamide ethyl ester,
(±)-3-(N-benzyl)amino-2-[N-(3-butanoate)]-naphthocarboxamide ethyl ester,
(±)-3-(N-benzyl)amino-2-[N-(3-phenyl-3-proprionate)]-naphthocarboxamide ethyl ester,
(±)-3-(N-2,4-difluorobenzyl)amino-2-[N-(3-butanoate)]-naphthocarboxamide ethyl ester,
(±)-3-(N-2,4-difluorobenzyl)amino-2-[N-(3-phenyl-3-proprionate)]-naphthocarboxamide ethyl ester,
(±)-3-(N-(b-napthyl)methyl)amino-2-[N-(3-butanoate)]-naphthocarboxamide ethyl ester,
(±)-3-(N-(b-napthyl)methyl)amino-2-[N-(3-phenyl-3-proprionate)]-naphthocarboxamide ethyl ester.

d) To an ice-cooled solution of 5.0g (17.5mMol) of 3-(N-methyl)amino-2-[N-(2-carboxyethyl)]-naphthocarboxamide ethyl ester and 2.68mL (19mMol) of triethylamine in 50mL of methylene chloride was added dropwise a solution of 1.59mL 19(mMol) of bromoacetyl bromide. After warming to room temperature, the solution was washed with aqueous citric acid and aqueous sodium bicarbonate solution, dried over sodium sulfate and evaporated. This material was dissolved in DMF (20mL) and added dropwise to a suspension of 97% sodium hydride (462mg, 19mMol) at -15°C. After warming to room temperature the solution was poured over ice/citric acid and extracted into ether. 3,1g (52%) of 1-methyl-4-(carboxyethyl)-3,4-dihydro-1H-1,4-naphthodiazepine-2,5-dione ethyl ester, a yellow oil, was obtained after column chromatography on silica gel. MS (FAB) 341. ¹H NMR (CDCL₃): 8.4, s, 1H; 7.9, d, J=8.5, 1H; 7.8, d, J=8.5, 1H; 7.6, s, 1H; 7.45-7.6, m, 2H; 4.15, q, J=7Hz, 2H; 4.1, d, J=14Hz, 1H; 3.95, t, J=7Hz, 2H; 3.85, d, J=14Hz, 1H; 3.5, s, 3H; 2.75, m, 2H; 1.25, t, J=7Hz, 3H.

Using the above procedure, but substituting the appropriate naphthocarboxamide for 3-(N-methyl)amino-2-[N-(2-carboxyethyl)]-naphthocarboxamide ethyl ester there may be prepared, for example, the following compounds:
1-benzyl-4-(carboxyethyl)-3,4-dihydro-1H-1,4-naphthodiazepine-2,5-dione ethyl ester
1-(2,4-diflurobenzyl)-4-(carboxyethyl)-3,4-dihydro-1H-1,4-naphthodiazepine-2,5-dione ethyl ester
1-(b-napthyl)methyl-4 -(carboxyethyl)-3,4-dihydro-1H-1,4-naphthodiazepine-2,5-dione ethyl ester
(±)-1-methyl-4-(3-butanoate)-3,4-dihydro-1H-1,4-naphthodiazepine-2,5-dione ethyl ester,
(±)-1-methyl-4-(3-phenyl-3-proprionate)-3,4-dihydro-1H-1,4-naphthodiazepine-2,5-dione ethyl ester,
(±)-1-benzyl-2-(3-butanoate)-3,4-dihydro-1H-1,4-naphthodiazepine-2,5-dione ethyl ester,
(±)-1-benzyl-2-(3-phenyl-3-proprionate)-3,4-dihydro-1H-1,4-naphthodiazepine-2,5-dione ethyl ester,
(±)-1-(2,4-diflurobenzyl)-2-(3-butanoate)-3,4-dihydro-1H-1,4-naphthodiazepine-2,5-dione ethyl ester,
(±)-1-(2,4-diflurobenzyl)-2-(3-phenyl-3-proprionate)-3,4-dihydro-1H-1,4-naphthodiazepine-2,5-dione ethyl ester,
(±)-1-(b-napthyl)methyl-2-(3-butanoate)-3,4-dihydro-1H-1,4-naphthodiazepine-2,5-dione ethyl ester,
(±)-1-(b-napthyl)methyl-4-(3-phenyl-3-proprionate)-3,4-dihydro-1H-1,4-naphthodiazepine-2,5-dione ethyl ester.

e) 1-Methyl-4-(ethoxycarbonylethyl)-3,4-dihydro-1H-1,4-naphthodiazepine-2,5-dione (3.1g, 9.1mMol) was placed in an ice bath and 4mL of fuming nitric acid was added. After stirring for 1hr at 0°C the reaction was allowed to warm to room temperature and stirred for an additional 2hr. The reaction mixture was poured into ice water and extracted with ethyl acetate, dried (sodium sulfate) and evaporated to yield a yellow solid. Thin layer chromatography (TLC) and ¹H NMR analysis indicated a mixture of nitrated products. The crude mixture was purified by column chromatography (SiO₂) to yield 600mgs (17%) of 8-Nitro-1-methyl-4-(ethoxycarbonylethyl)-3,4-dihydro-1H-1,4-naphthodiazepine-2,5-dione. ¹H NMR (CDCl₃, dTMS) 8.8 (1H, d, ⁴J_{HH}=2Hz), 8.6 (1H, s), 8.3 (1H, dd, J_{HH}=9Hz, ⁴J_{HH}=2Hz), 7.95 (1H, d, ³J_{HH}=9Hz), 7.7 (1H, s), 4.15 (2H, q, ³J_{HH}=8Hz), 4.1 (1H, d, J_{HH}=14Hz), 4.0 (2H, t, ³J_{HH}=7Hz), 3.95 (1H, J_{HH}=14Hz), 3.5 (3H, s), 2.75 (2H, m), 1.25 (3H, t, ³J_{HH}=8Hz).

Using the above procedure, but substituting the appropriate naphthocarboxamide for 3-(N-methyl)amino-2-[N-(2-carboxyethyl)]-naphthocarboxamide ethyl ester there may be prepared, for example, the following compounds:
1-(2,4-diflurobenzyl)-4-(carboxyethyl)-8-nitro-3,4-dihydro-1H-1,4-naphthodiazepine-2,5-dione ethyl ester,
(±)-1-methyl-4-(3-butanoate)-8-nitro-3,4-dihydro-1H-1,4-naphthodiazepine-2,5-dione ethyl ester,
(±)-1-(2,4-diflurobenzyl)-2-(3-butanoate)-8-nitro-3,4-dihydro-1H-1,4-naphthodiazepine-2,5-dione ethyl ester.

f) 1-Methyl-4-(carboxylethyl)-8-nitro-3,4-dihydro-1H-1,4-naphthodiazepine-2,5-dione ethyl ester (600mg, 1.56mMol) was hydrogenated in ethanol with 200mg of 10% palladium on carbon catalyst at 50psi hydrogen for 24hr after which time tlc indicated that reduction was complete. The catalyst was removed by filtration through Celite®, and the filtrate was evaporated yielding ca. 500mg of 1-methyl-4-(carboxylethyl)-8-amino-3,4-dihydro-1H-1,4-naphthodiazepine-2,5-dione ethyl ester. ¹H NMR (CDCL₃): 8.1, s, 1H; 7.6, d, J=9.5Hz, 1H; 7.45, s, 1H; 7.15, s, 1H; 7.1, d, J=9.5Hz, 1H; 4.15, q, J=8Hz, 2H; 4.1, d, J=14Hz, 1H; 3.95, t, J=7Hz, 2H; 3.8, d, J=14Hz, 1H; 3.4, s, 3H; 2.7, m, 2H; 1.2, t, J=8Hz. This material was dissolved in 4mL of water containing 117mL of concentrated sulfuric acid and cooled to 0°C with an ice bath. Sodium nitrite (97mg, 1.5mMol) in 2mL of water was added to the solution dropwise, and the temperature of the solution was allowed to rise to room temperature over one hour. 2.6g (14mMol) of potassium iodide was added to the solution and stirring was continued for 24hr. The reaction mixture was diluted with water and extracted with ethyl acetate, and the ethyl acetate was dried (sodium sulfate) and evaporated. Chromatography on silica gel yielded ca. 240 mg of 8-Iodo-1-methyl-4-(ethoxycarbonylethyl)-3,4-dihydro-1H-1,4-naphthodiazepine-2,5-dione as an amber oil. MS (FAB) 466.9. ¹H NMR (CDCL₃): 8.3, s, 2H; 7.8, dd, J=8.5Hz, J'=1.5Hz, 1H; 7.55, s, 1H; 7.55, d, J=8.5Hz, 1H; 4.15, q, J=7Hz, 2H; 4.1, d, J=14Hz, 1H; 3.95, t, J=7Hz, 2H; 3.9, d, J=14Hz, 1H; 3.5, s, 3H; 2.75, m, 2H; 1.25, t, J=7Hz.

Using the above procedure, but substituting the appropriate 8-nitro-3,4-dihydro-1H-1,4-naphthodiazepine-2,5-dione for 1-methyl-4-(2-carboxylethyl)-8-nitro-3,4-dihydro-1H-1,4-naphthodiazepine-2,5-dione ethyl ester there may be prepared, for example, the following compounds:
1-(2,4-diflurobenzyl)-4-(carboxyethyl)-8-iodo-3,4-dihydro-1H-1,4-naphthodiazepine-2,5-dione ethyl ester,
(±)-1-methyl-4-(3-butanoate)-8-iodo-3,4-dihydro-1H-1,4-naphthodiazepine-2,5-dione ethyl ester,
(±)-1-(2,4-diflurobenzyl)-2-(3-butanoate)-8-iodo-3,4-dihydro-1H-1,4-naphthodiazepine-2,5-dione ethyl ester.

g) 1-Methyl-4-(ethoxycarbonylethyl)-8-Iodo-3,4-dihydro-1H-1,4-naphthodiazepine-2,5-dione (100mg, 215mM) was combined in 1mL of ethyl acetate with 166mg (500m%) of N-t-butoxycarbonylpropargyl amine, 15mg (100mol%) of bistriphenylphosphine palladium dichloride, and 299mL (10X excess) of triethylamine. The apparatus was degassed and an atmosphere of nitrogen was introduced. 8.2mg (20mol%) of cuprous iodide was added and the solution was again degassed and nitrogen atmosphere was replaced. After 24hr the reaction mixture was diluted with ethyl acetate and washed succesively with aqueous citric acid and aqueous sodium bicarbonate, dried (sodium sulfate) and evaporated. The residue was chromatographed on silica gel, yielding 110mg of 1-methyl-4-(crboxylethyl)-8-(3-t-butyloxycarbonylamino-1-propynyl)-3,4-dihydro-1H-1,4-naphthodiazepine-2,5-dione. MS (FAB) 494.1. ¹H NMR (CDCL₃): 8.35, s, 1H; 7.95, s, 1H; 7.75, d, J=8.5Hz, 1H; 7.55, s, 1H; 7.5, d, J=8.5Hz, 1H; 4.9, br s, 1H; 4.2, br s, 2H; 4.2, q, J=7Hz, 2H; 4.1, d, J=14Hz, 1H; 4.0, t, J=7Hz, 2H; 3.9, d, J=7Hz, 1H; 3.5, s, 3H; 2.75, m, 2H; 1.5, s, 9H; 1.25, t, J=7Hz, 3H.

Using the above procedure, but substituting the appropriate 8-iodo-3,4-dihydro-1H-1,4-naphthodiazepine-2,5-dione for 1-methyl-4-(2-carboxylethyl)-8-iodo-3,4-dihydro-1H-1,4-naphthodiazepine-2,5-dione ethyl ester there may be prepared, for example, the following compounds :
1-(2,4-diflurobenzyl)-4-(carboxyethyl)-8-(3-t-butyloxycarbonylamino-1-propynyl)-3,4-dihydro-1H-1,4-naphthodiazepine-2,5-dione ethyl ester,
(±)-1-methyl-4-(3-butanoate)-8-(3-t-butyloxycarbonylamino-1-propynyl)-3,4-dihydro-1H-1,4-naphthodiazepine-2,5-dione ethyl ester,
(±)-1-(2,4-diflurobenzyl)-2-(3-butanoate)-8-(3-t-butyloxycarbonylamino-1-propynyl)-3,4-dihydro-1H-1,4-naphthodiazepine-2,5-dione ethyl ester,
1-(2,4-diflurobenzyl)-4-(carboxyethyl)-8-(4-t-butyloxycarbonylamino-1-butynyl)-3,4-dihydro-1H-1,4-naphthodiazepine-2,5-dione ethyl ester,
(±)-1-methyl-4-(3-butanoate)-8-(4-t-butyloxycarbonylamino-1-butynyl)-3,4-dihydro-1H-1,4-naphthodiazepine-2,5-dione ethyl ester,
(±)-1-(2,4-diflurobenzyl)-2-(3-butanoate)-8-(4-t-butyloxycarbonylamino-1-butynyl)-3,4-dihydro-1H-1,4-naphthodiazepine-2,5-dione ethyl ester.

h) To a stirred solution of 1-methyl-4-(carboxyethyl)-8-(3-t-butyloxycarbonylamino-1-propynyl)-3,4-dihydro-1H-1,4-naphthodiazepine-2,5-dione ethyl ester (110mg, 0.22mMol) in 8mL of methanol was added 1mL of 1N sodium hydroxide solution. After 4hr no more starting material was detected by tlc. The solvent was removed *in vacuo* and the residue was treated with 10mL of 4N HCl in dioxane. After 2hr the solvent was removed *in vacuo* and the residue was purified by reverse phase HPLC, yielding 25mg of 1-methyl-4-(carboxyethyl)-8-(3-amino-1-propynyl)-3,4-dihydro-1H-1,4-naphthodiazepine-2,5-dione. HRMS (FAB) molecular ion m/z=366.1454 (cald. C₂₀H₂₀N₃O₄, 366.4004). ¹H NMR (CD₃OD): 7.9, s, 1H; 7.7, s, 1H; 7.55, d, J=8Hz, 1H; 7.45, s, 1H; 7.2, d, J=8Hz, 1H; 3.75, d, J=14Hz, 1H; 3.65, s, 2H; 3.55, d, J=14Hz, 1H; 3.5, m, 2H; 3.05, s, 3H; 2.2, q, J=7Hz, 2H.

Using the above procedure, but substituting the appropriate 8-(t-butyloxycarbonylamino-1-alkynyl)-3,4-dihydro-1H-1,4-naphthodiazepine-2,5-dione for 1-methyl-4-(carboxyethyl)-8-(3-t-butyloxycarbonylamino-1-propynyl)-3,4-dihydro-1H-1,4-naphthodiazepine-2,5-dione ethyl ester there may be prepared, for example, the following compounds:
1-(2,4-diflurobenzyl)-4-(carboxyethyl)-8-(3-amino-1-propynyl)-3,4-dihydro-1H-1,4-naphthodiazepine-2,5-dione,
(±)-1-methyl-4-(3-butanoate)-8-(3-amino-1-propynyl)-3,4-dihydro-1H-1,4-naphthodiazepine-2,5-dione,
(±)-1-(2,4-diflurobenzyl)-4-(3-butanoate)-8-(3-amino-1-propynyl)-3,4-dihydro-1H-1,4-naphthodiazepine-2,5-dione,
1-(2,4-diflurobenzyl)-4-(carboxyethyl)-8-(4-amino-1-butynyl)-3,4-dihydro-1H-1,4-naphthodiazepine-2,5-dione,
(±)-1-methyl-4-(3-butanoate)-8-(4-amino-1-butynyl)-3,4-dihydro-1H-1,4-naphthodiazepine-2,5-dione,
(±)-1-(2,4-diflurobenzyl)-4-(3-butanoate)-8-(4-amino-1-butynyl)-3,4-dihydro-1H-1,4-naphthodiazepine-2,5-dione.

### Example 17

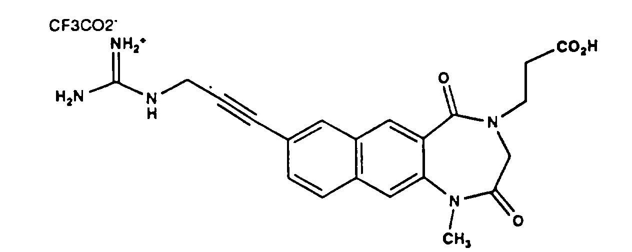

*1-Methyl-4-(carboxyethyl)-8-(3-guanidino-1-propynyl)-3,4-dihydro-1H-1,4-naphthodiazepine-2,5-dione*

The title compound was prepared from 1-methyl-4-(carboxyethyl)-8-(3-amino-1-propynyl)-3,4-dihydro-1H-1,4-naphthodiazepine-2,5-dione (50mg) according to the procedure described in Example 2. HRMS (FAB) molecular ion m/z=408.1672 (cald. C₂₁H₂₂N₅O₄, 408.1672). ¹H NMR (CD₃OD): 7.9, s, 1H; 7.7, s, 1H; 7.55, d, J=8Hz, 1H; 7.45, s, 1H; 7.2, d, J=8Hz, 1H; 3.75, d, J=14Hz, 1H; 3.65, s, 2H; 3.55, d, J=14Hz, 1H; 3.5, m, 2H; 3.05, s, 3H; 2.2, q, J=7Hz, 2H.

Using the above procedure, but substituting the appropriate 8-(amino-1-alkynyl)-3,4-dihydro-1H-1,4-naphthodiazepine-2,5-dione for 1-methyl-4-(carboxyethyl)-8-(3-amino-1-propynyl)-3,4-dihydro-1H-1,4-naphthodiazepine-2,5-dione there may be prepared, for example, the following coumpounds:
1-(2,4-diflurobenzyl)-4-(carboxyethyl)-8-(3-guanidino-1-propynyl)-3,4-dihydro-1H-1,4-naphthodiazepine-2,5-dione,
(±)-1-methyl-4-(3-butanoate)-8-(3-guanidino-1-propynyl)-3,4-dihydro-1H-1,4-naphthodiazepine-2,5-dione,
(±)-1-(2,4-diflurobenzyl)-2-(3-butanoate)-8-(3-guanidino-1-propynyl)-3,4-dihydro-1H-1,4-naphthodiazepine-2,5-dione,
1-(2,4-diflurobenzyl)-4-(carboxyethyl)-8-(4-guanidino)-1-butynyl)-3,4-dihydro-1H-1,4-naphthodiazepine-2,5-dione,
(±)-1-methyl-4-(3-butanoate)-8-(4-guanidino-1-butynyl)-3,4-dihydro-1H-1,4-naphthodiazepine-2,5-dione,
(±)-1-(2,4-diflurobenzyl)-2-(3-butanoate)-8-(4-guanidino-1-butynyl)-3,4-dihydro-1H-1,4-naphthodiazepine-2,5-dione.

### Example 18

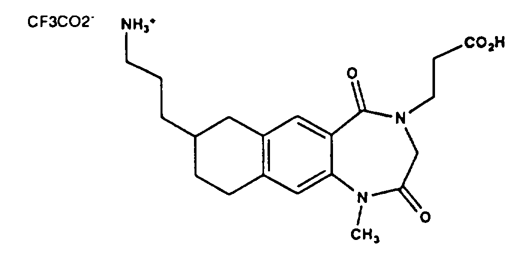

*1-Methyl-4-(carboxyethyl)-8-(3-amino-1*-*propyl)-3*,*4*,*7*,*8*,*9*,*10-hexahydrohydro-1H-1,4-naphthodiazepine-2,5-dione*

1-Methyl-4-(carboxyethyl)-8-(3-amino-1-propynyl)-3,4-dihydro-1H-1,4-naphthodiazepine-2,5-dione (50mg) was reduced with 10mg of 10% Pd/C in 10mL of 5:1 water ethanol at 45psi of hydrogen in a Parr hydrogenation apparatus for 24hr yielding, after filtration through Celite® and purification by reverse phase HPLC, the title compound. MS (FAB) 374.1. ¹H NMR (CD₃OD): 7.4, s, 1H; 7.0, s, 1H; 3.95, d, J=14Hz, 1H; 3.8, d, J=14Hz, 1H; 3.6, m, 2H; 3.2, s, 3H; 2.85, t, J=7Hz, 2H; 2.4, t, J=7Hz, 2H; 1.9, m, 1H; 1.65, m, 4H; 1.4, m, 4H.

Using the above procedure, but substituting the appropriate 8-(amino-1-alkynyl)-3,4-dihydro-1H-1,4-naphthodiazepine-2,5-dione for 1-methyl-4-(carboxyethyl)-8-(3-amino-1-propynyl)-3,4,7,8,9,10-hexahydrohydro-1H-1,4-naphthodiazepine-2,5-dione there may be prepared, for example, the following compounds:
1-(2,4-diflurobenzyl)-4-(carboxyethyl)-8-(3-guanidino-1-propynyl)-3,4,7,8,9,10-hexahydrohydro-1H-1,4-naphthodiazepine-2,5-dione,
(±)-1-methyl-4-(3-butanoate)-8-(3-guanidino-1-propynyl)-3,4,7,8,9,10-hexahydrohydro-1H-1,4-naphthodiazepine-2,5-dione,
(±)-1-(2,4-diflurobenzyl)-2-(3-butanoate)-8-(3-guanidino-1-propynyl)-3,4,7,8,9,10-hexahydrohydro-1H-1,4-naphthodiazepine-2,5-dione,

### Example 19

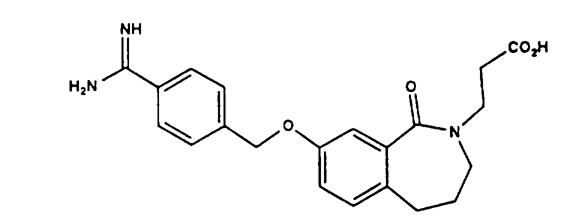

*2-(2-carboxyethyl)*-*8-(4-amidinobenzyloxy)-2,3,4,5-tetrahydro-1H-2-benzazepin-1-one trifluoroacetate.*

a) A solution of 7-methoxytetralone (Fluka Chemical, 100.0 g, 0.567 mol) in dry pyridine (600 mL) was treated with hydroxylamine hydrochloride (Fluka, 43.0 g, 0.618 mol) and the resulting dark yellow solution was stirred at room temperature for 1 hour. The solution was then concentrated *in vacuo,* and azeotroped with several volumes of toluene, then partitioned between ethyl acetate and brine. The combined organic layers were dried (K₂CO₃), filtered, and concentrated *in vacuo.* The residue was crystallized from ethyl acetate/hexanes to yield 88 g (81%) of the oxime as a waxy solid.

b) Following a procedure of Tomita, et. al., *J*. *Chem. Soc. C* 1969, 183, 7*-*methoxytetralone oxime (45.0 g, 0.235 mol) was mixed with solid trichloroacetic acid (150 g), and the resulting suspension heated to ca. 80 C, at which time a violent exotherm ensued, yielding a black solid mixture. The residue was cooled, partitioned between ethyl acetate and saturated aqueous sodium bicarbonate solution, and the combined organic layers were concentrated *in vacuo.* The residue was purified by chromatography over silica gel to yield 10.0 g (22%) of 8-methoxy-2,3,4,5-tetrahydro-1H-2-benzazepin-1-one. An analytical sample was obtained by recrystallization from CH₂Cl₂ /hexanes to yield colorless crystals: mp. 99-101°C (lit. (see above) 100-101°C); ¹H NMR(CDCl₃) d7.35(brs, 1H), 7.30(d, J=3Hz, 1H), 7.10(d, J=9Hz, 1H), 6.95(dd, J=3, 9Hz, 1H), 3.82(s, 3H), 3.10(q, J=6Hz, 2H), 2.80(t, J=6Hz, 2H), 1.98(m, 2H).

c) A solution of 8-methoxy-2,3,4,5-tetrahydro-1H-2-benzazepin-1-one (10.0 g, 0.052 mol) in N,N-dimethylformamide (30 mL) and allylbromide (23 mL, 0.260 mL) was treated with NaH (60% by wt. dispersion in oil, 2.50 g, 0.063 mol) and the resulting grey suspension stirred at room temperature under an atmosphere of argon. Gas evolution was noted. After 15 minutes, the solution was diluted with brine and partitioned between ethyl acetate and brine. The combined organic layers were concentrated *in vacuo* and purified by chromatography over silica gel eluting with a gradient of hexane to 1:1 ethyl acetate/hexane to yield 8 g (83%) of 2-allyl-8-methoxy-2,3,4,5-tetrahydro-1H-2-benzazepin-1-one as a colorless oil: ¹H NMR(CDCl₃) d7.23(m, 1H), 7.03(d, J=9Hz, 1H), 6.90(dd, J=3, 9Hz, 1H), 5.91(m, 1H), 5.23(m, 2H), 4.20(d, J=6Hz, 2H), 3.82(s, 3H), 3.18(t, J=6Hz, 2H), 2.72(t, J=6Hz, 2H), 1.96(pentet, J=6Hz, 2H); ¹³C NMR(CDCl₃) d170.59(CO), 158.42(NCO), 136.88, 133.81, 129.43, 129.35, 129.33, 117.56, 117.54, 112.98, 55.26, 49.43, 45.37, 29.57, 29.22; MS(FAB) *m/z=* 232.1(MH⁺).

d) A solution of 2-allyl-8-methoxy-2,3,4,5-tetrahydro-1H-2-benzazepin-1-one (8.0 g, 0.043 mol) in dry tetrahydrofuran (40 mL) was treated with 9-borabicyclononane (1.0 M in THF) and the resulting mixture was stirred for 18 hours at room temperature. The solution was then diluted with EtOH (50 mL), cooled to 0°C (ice bath), and treated with 15% aqueous NaOH (15 mL) followed by 30% aqueous H₂O₂ (10 mL). The resulting mixture was stirred for 1 hour at 0°C, then treated cautiously with saturated aqueous sodium thiosulfate (50 mL). The mixture was partitioned between ethyl acetate and brine, and the combined organic layers were concentrated *in vacuo,* and the residue was purified by chromatography over silica gel eluting with ethyl acetate to yield 6.5 g (60%) of 2-(3-hydroxypropyl)-8-methoxy-2,3,4,5-tetrahydro-1H-2-benzazepin-1-one as colorless oil; ¹H NMR(CDCl₃) d7.19(s, 1H), 7.06(d, J=9Hz, 1H, 6.92(dd, J=3, 9Hz, 1H), 4.30(brs, 1H), 3.82(s, 3H), 3.71(t, J=6Hz, 2H), 3.61(brs, 2H), 3.18(t, J=6Hz, 2H), 2.72(t, J=6Hz, 2H), 2.02(pentet, J=6Hz, 2H), 1.79(pentet, J=6Hz, 2H); ¹³C NMR(CDCl₃) d172.25(CO), 158.51(NCO), 136.34, 129.53, 117.46, 112.94, 58.19, 55.42, 46.16, 43.30, 30.91, 29.45, 29.12; MS (FAB) m/z=250.1(MH⁺); HRMS (FAB, MH⁺) m/z=249.1364 (cald C₁₄H₁₉NO₃: 249.1364).

e) A solution of 2-(3-hydroxypropyl)-8-methoxy-2,3,4,5-tetrahydro-1H-2-benzazepin-1-one (6.50g, 0.026 mol) in CH₂Cl₂ was added, at -78°C (CO₂/acetone), to a solution of (COCl)₂ (4.50 mL, 0.052 mol) and DMSO (7.4 mL, 0.104 mol) in CH₂Cl₂ (100 mL) at -78°C. After stirring for 15 minutes at -78°C, the mixture was treated with NEt₃ (30 mL, 0.208 mol), and the bath removed. The CH₂Cl₂ solution was washed with brine, dried (MgSO₄) and concentrated in vacuo. The residue was immediately dissolved in N,N-dimethylformamide (30 mL) and MeOH (30mL), and treated, in portions, with pyridinium dichromate (30 g total). After stirring for 24 hours, the dark brown suspension was diluted with ethyl acetate and filtered, and the filtrate was partitioned between ethyl acetate and brine. The combined organic layers were washed with water, brine, dried (MgSO₄) and concentrated in vacuo. The residue was purified by chromatography over silica gel eluting with a gradient of 10% ethyl acetate/hexane to 100% ethyl acetate to yield 2.5 g (35%) of 2-(2-carboxyethyl)-8-methoxy-2,3,4,5-tetrahydro-1H-2-benzazepin-1-one methyl ester as a colorless oil; ¹H NMR(CDCl₃) d7.18(d, J=3Hz, 1H), 7.02(d, J=9Hz, 1H), 6.88(dd, J=3, 9Hz, 1H), 3.83(t, J=6Hz, 2H), 3.80(s, 3H), 3.69(s, 3H), 3.23(t, J=6Hz, 2H), 2.78(t, J=6Hz, 2H), 2.67(t, J=6Hz, 2H), 1.96jm, 2H); ¹³C NMR(CDCl₃) d172.41(CO), 171.15(CO), 158.50, 136.80, 129.58, 129.40, 117.34, 112.86, 55.42, 51.78, 47.37, 44.13, 33.64, 29.85, 29.14; MS(FAB) *m/z=*278.1 (MH⁺); HRMS(FAB, MH⁺) m/z= 278.1392 (cald for C₁₅H₁₉NO₄: 277.1313).

f) A solution of 2-(2-carboxyethyl)-8-methoxy-2,3,4,5-tetrahydro-1H-2-benzazepin-1-one methyl ester (3.20 g, 0.011 mol) in CH₂Cl₂ was added to a suspension of AlCl₃ (4.70g, 0.034 mol) and *n*-propanethiol (5.3 mL, 0.057 mol) at 0°C (ice bath), and the resulting mixture stirred for 10 minutes at room temperature. The mixture was diluted with saturated aqueous sodium bicarbonate (50 mL), and the resulting white suspension stirred for 1 hour. The gelatinous mixture was filtered, and the filtrate was dried (MgSO₄) and concentrated *in vacuo* to yield 2.0 g (85%) of 2-(2-carboxyethyl)-8-hydroxy-2,3,4,5-tetrahydro-1H-2-benzazepin-1-one methyl ester as a colorless oil; ¹H NMR(CD₃OD) d7.08(d, J=9Hz, 1H, 7.02(d, J=3Hz, 1H), 6.87(dd, J=3, 9Hz, 1H), 3.05(t, J=6Hz, 2H), 2.72(t, J=6Hz, 2H), 1.97(t, J=6Hz, 2H).

g) A suspension of 2-(2-carboxyethyl)-8-hydroxy-2,3,4,5-tetrahydro-1H-2-benzazepin-1-one methyl ester (250 mg, 0.950 mmol), *p*-cyanobenzylbromide (186 mg, 0.950 mmol) and K₂CO₃ (500 mg) in N,N-dimethylformamide (20 mL) was stirred vigorously at room temperature under an atmosphere of argon. After 1 hour, the suspension was diluted with ethyl acetate (100 mL), washed with water and brine, dried (Na₂SO₄) and concentrated *in vacuo.* The residue was purified by chromatography over silica gel to yield 150 mg (40%) of 2-(2-carboxyethyl)-8-(4-cyanobenzyloxy)-2,3,4,5-tetrahydro-1H-2-benzazepin-1-one methyl ester as a colorless oil; ¹H NMR(CDCl₃) d7.66(d, J=6Hz, 1H), 7.54(d, J=6Hz, 1H), 7.00(dd, J=3, 9Hz, 1H), 5.13(s, 2H), 3.84(t, J=6Hz, 2H), 3.70(s, 3H), 3.24(t, J=6Hz, 2H), 2.72(m, 4H), 1.98(pentet, J=6Hz, 2H); ¹³C NMR(CDCl₃) d172.27(CO), 170.82(CO), 157.05, 142.32, 137.01, 132.39, 132.32, 130.45, 129.63, 127.52, 118.66, 117.83, 113.98, 111.53, 68.90, 51.76, 47.31, 44.14, 33.58, 29.76, 29.13; MS(FAB) *m/z*= 379.1(MH⁺).

h) A solution of 2-(2-carboxyethyl)-8-(4-cyanobenzyloxy)-2,3,4,5-tetrahydro-1H-2-benzazepin-1-one methyl ester (150 mg, 0.400 mmol) in 50 mL of 1:1 pyridine-NEt₃ was saturated with H₂S, and heated at 50°C under argon. After 1 hour, the solvents were removed *in vacuo.* The residue was dissolved in CH₂Cl₂ (20 mL) and treated with iodomethane (4 mL), and the resulting mixture heated at reflux for 1 hour. The solvents were removed *in vacuo,* and the residue was dissolved in MeOH and treated with an excess of NH₄OAc (1 g). The resulting suspension was stirred for 15 minutes at 60°C. The MeOH was then removed, and the residue was treated with 50% NaOH (3 mL) in tetrahydrofuran (20 mL), and stirred for 15 minutes at room temperature. The mixture was then diluted with trifluoroacetic acid, and the volatiles were removed *in vacuo.* The residue was purified by HPLC (1/2" C-18 reverse-phase column, eluting with a solvent gradient of 1:9 acetonitrile(0.1%TFA)/water (0.1% TFA) time 0 to 10 minutes, to 1:1 acetonitrile(0.1%TFA)/water (0.1% TFA) time 10 to 40 minutes, flow 10mL/min, Rₜ=30.5 min, mu detection 254nM), to give 18.5 mg (9%) of 2-(2-carboxyethyl)-8-(4-amidinobenzyloxy)-2,3,4,5-tetrahydro-1H-2-benzazepin-1-one trifluoroacetate as a yellowish-brown solid after lyophylization; ¹H NMR(CD₃OD) d7.66(d, J=6Hz, 1H), 7.54(d, J=6Hz, 1H), 7.00(dd, J=3, 9Hz, 1H), 5.13(s, 2H), 3.84(t, J=6Hz, 2H), 3.70(s, 3H), 3.24(t, J=6Hz, 2H), 2.72(m, 4H), 1.98(m, J=6Hz, 2H); MS(FAB) *m/z*= 381.1688 (MH⁺).

### Example 20

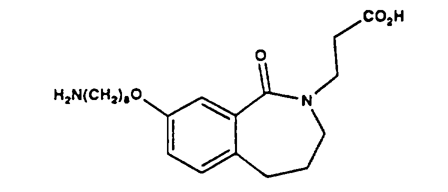

*2-(2-carboxyethyl)-8-(8-aminooctyloxy)-2,3,4,5-tetrahydro-1H-2-benzazepin-1-one trifluoroacetate.*
a) A solution of 2-allyl-8-methoxy-2,3,4,5-tetrahydro-1H-2-benzazepin-1-one (1.10 g, 0.0047 mol) in CH₂Cl₂ (20 mL) was treated at 0°C with BBr₃ (1M in CH₂Cl₂, 9.50 mL), and the resulting mixture stirred at 0°C for 30 minutes. The solution was poured over ice, NaHCO₃ was added, and the solution was extracted with CH₂Cl₂. The combined organic layers were dried (MgSO₄), filtered, and concentrated *in vacuo* to yield ca. 1.0 g (100%) of 2-allyl-8-hydroxy-2,3,4,5-tetrahydro-1H-2-benzazepin-1-one as a colorless oil; ¹H NMR(CDCl₃) d8.2(brs, 1H), 7.47(s,1H), 6.95(d, J=6Hz, 1H), 6.84(dd, J=3, 6Hz, 1H), 5.89(m, 1H), 5.22(m, complex, 2H), 4.20(d, J=6Hz, 2H), 3,18(t, J=6Hz, 2H), 2.66(t, J=6Hz, 2H), 2.15(s, 1H), 1.96(m, 2H);¹³C NMR(CDCl₃) d171.65(CO), 156.01, 135.68, 133.27, 129.55, 128.46, 118.51, 118.05, 115.91, 53.46, 49.81, 45.83, 30.87, 29.76, 29.12.
b) A suspension of 2-allyl-8-hydroxy-2,3,4,5-tetrahydro-1H-2-benzazepin-1-one (1.0 g, 0.0049 mol), 8-azido-1-(toluenesulfonyloxy)octane (1.50 g, 0.0049 mol) and K₂CO₃ in N,N-dimethylformamide (20 mL) was stirred vigorously under argon, and heated at 65°C for 18 hours. The solution was then partitioned between ethyl acetate and brine, and the combined organic layers were washed with water, dried (K₂CO₃), filtered, and concentrated in vacuo. The residue was purified by chromatography over silica gel to yield 920 mg (51%) of 2-allyl-8-(8-azidooctyloxy)-2,3,4,5-tetrahydro-1H-2-benzazepin-1-one as a colorless oil; ¹H NMR(CDCl₃)d7.47(s, 1H), 6.95(d, J=6Hz,1H), 6.84(dd, J=3, 6Hz, 1H), 5.89(m, 1H), 5.22(m, complex, 2H), 4.20(d, J=6Hz, 2H), 3.95(t, J=6Hz, 2H), 3.22(t, J=6Hz, 2H), 3.18(t, J=6Hz, 2H), 2.66(t, J=6Hz, 2H), 1.90(t, J=6Hz, 2H), 1.79(t, J=6Hz, 2H), 1.60(m, 4H), 1.35(m, 6H).
c) A solution of 2-allyl-8-(8-azidooctyloxy)-2,3,4,5-tetrahydro-1H-2-benzazepin-1-one (920 mg, 2.48 mmol) in tetrahydrofuran (20 mL) was treated with 9-borabicyclononane (0.5 M/THF, 10 mL, 5.00 mmol), and the mixture was stirred under argon at room temperature for 18 hours. The mixture was then cooled in an ice bath, and EtOH (5 mL) followed by 15% aqueous NaOH (10 mL) and 30% H₂O₂ (15 mL) were added. The mixture was stirred for 1 hour, then saturated aqueous sodium thiosulfate was added (20 mL). The solution was partitioned between ethyl acetate and brine, and the combined ethyl acetate layers were dried (MgSO₄), filtered, and concentrated *in vacuo*. The residue was purified by chromatography over silica gel to yield 400 mg (42%) of 2-(3-hydroxypropyl)-8-(8-azidooctyloxy)-2,3,4,5-tetrahydro-1H-2-benzazepin-1-one as a colorless oil; ¹H NMR(CDCl₃) d7.20(d, J=3Hz, 1H), 7.04(d, J=6Hz, 1H), 6.91(dd, J=3, 6Hz, 1H), 3.96(t, J=6Hz, 2H), 3.72(t, J=6Hz, 2H), 3.61(t, J=6Hz, 2H), 3.26(t, J=6Hz, 2H), 3.18(t, J=6Hz, 2H), 2.73(t, J=6Hz, 2H), 2.02(t, J=6Hz, 2H), 1.77(m, 4H), 1.60(m, 2H), 1.36(m, 8H).
d) 2-(3-hydroxypropyl)-8-(8-azidooctyloxy)-2,3,4,5-tetrahydro-1H-2-benzazepin-1-one was subjected to standard Swern conditions followed by PDC oxidation as described in part (e) of Example 14 to yield 140 mg (45%) of 2-(2-carboxyethyl)-8-(8-azidooctyloxy)-2,3,4,5-tetrahydro-1H-2-benzazepin-1-one methyl ester as a colorless oil; ¹H NMR(CDCl₃) d7.18(d, J=3Hz, 1H), 7.00(d, J=9Hz, 1H), 6.89(dd, J=3, 9Hz, 1H), 3.96(t, J=6Hz, 2H), 3.84(t, J=6Hz, 2H), 3.70(s, 3H), 3.26(m, 4H), 2.74(m, 4H), 1.97(t, J=6Hz, 2H), 1.77(t, J=6Hz, 2H), 1.60(t, J=6Hz, 2H), 1.35(m, 6H); ¹³C NMR(CDCl₃) d172.38(CO), 171.21(CO), 158.04, 136.72, 129.40, 129.34, 117.78, 113.57, 68.05, 51.76, 51.41, 47.37, 44.13, 33.64, 29.86, 29.13, 19.02, 28.77, 26.59, 25.87; MS(FAB) 417.2(MH⁺).
e) A solution of 2-(2-carboxyethyl)-8-(8-azidooctyloxy)-2,3,4,5-tetrahydro-1H-2-benzazepin-1-one methyl ester (70 mg, 0.168 mmol) in MeOH (1 mL) was treated with 10% Pd/C (20 mg) and the resulting suspension was reduced under an atmosphere of hydrogen (30psi) for 2 hours. The solution was then filtered through Celite®, and the filtrate was concentrated *in vacuo*. The residue was dissolved in tetrahydrofuran (1 mL) and treated with 50% NaOH (0.5 mL), and the resulting mixture was stirred at room temperature. After 1 hour, the solution was treated with trifluoroacetic acid (1 mL), and concentrated in vacuo. The white paste remaining was purified by HPLC (1/2' C-18 reverse-phase column) eluting with a gradient of 3:7 methanol (0.1% trifluoroacetate)/water (0.1% trifluoroaceate), time 0 to 10 min, to 7:3 methanol(0.1% trifluoroacetate)/water(0.1% trifluoroaceate), time 10 to 40 min, to yield 30 mg (36%) of 2-(2-carboxyethyl)-8-(8-aminooctyloxy)-2,3,4,5-tetrahydro-1H-2-benzazepin-1-one as a colorless oil; ¹H NMR(CD₃OD) d7.095(m, 2H), 6.96(dd, J=3, 9Hz, 1H), 3.98(t, J=6Hz, 2H), 3.83(t, J=6Hz, 2H), 3.27(m, 4H), 2.90(t, J=6Hz, 2H), 2.69(m, 4H), 2.03(t, J=6Hz, 2H), 1.78(t, J=6Hz, 2H), 1.64(t, J=6Hz, 2H), 1.40(m, 6H); MS(FAB) *m/z*= 377.1(MH⁺-TFA).

### Example 21

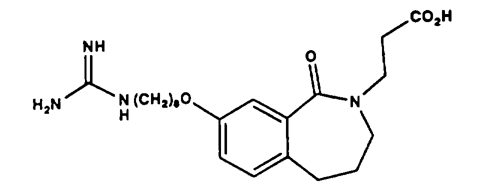

*2-(2-carboxyethyl)-8-(8-guanadinooctyloxy)-2,3,4,5-tetrahydro-1H-2-benzazepin-1-one* t*rifluoroacetate.*

a) A solution of 2-(2-carboxyethyl)-8-(8-aminooctyloxy)-2,3,4,5-tetrahydro-1H-2-benzazepin-1-one trifluoroacetate (30 mg,0.061 mmol) in saturated aqueous KHCO₃ (2 mL) was treated with formamidine sulfonic acid (85 mg, 1.08 mmol), and the resulting mixture was stirred at room temperature for 20 minutes. The mixture was neutralized with acetic acid, and the residue was purified by HPLC (1/2' C-18 reverse-phase column) eluting with a gradient of 3:7 methanol (0.1% trifluoroaceate)/water (0.1% trifluoroaceate), time 0 to 10 min, to 7:3 methanol(0.1%, trifluoroaceate)/water(0.1% trifluoroaceate), time 10 to 40 min, to yield 7.3 mg (25%) of 2-(2-carboxyethyl)-8-(8-guanadinooctyloxy)-2,3,4,5-tetrahydro-1H-2-benzazepin-1-one trifluoroacetate as a light brown solid; ¹H NMR(CD₃OD) d7.09(m, 2H), 6.96(dd, J=3, 9Hz, 1H), 3.98(t, J=6Hz, 2H), 3.83(t, J=6Hz, 2H), 3.33(m, 4H), 2.91(t, J=6Hz, 2H), 2.69(t, J=6Hz, 4H), 2.30(m, 2H), 1.75(m, 2H), 1.65(m, 2H), 1.40(m, 6H); ¹³C NMR(CD₃OD) d175.9(CO), 173.8(CO), 160.0, 137.0, 131.16, 130.79, 118.66, 114.81, 69.17, 45.33, 40.78, 34.32, 31.15, 30.27, 30.18, 30.15, 30.04, 28.59, 27.39, 27.06; MS(FAB) *m/z*= 419.2(MH⁺).

### Example 22

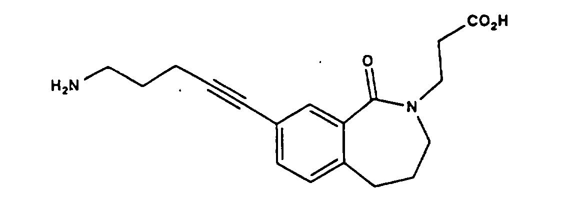

*2-(2-carboxyethyl)-8-(5-aminopentynyl)-2,3,4,5-tetrahydro-1H-2-benzazepin-1-one trifluoroacetate.*

a) *A* solution of 2-(2-carboxyethyl)-8-hydroxy-2,3,4,5-tetrahydro-1H-2-benzazepin-1-one methyl ester (2.20g, 0.00836 mol) and NEt₃ (1.40 mL, 0.0100 mol) in CH₂Cl₂ (20 mL) was cooled to 0°C (ice bath) and treated with trifluoromethanesulfonic anhydride (1.70 mL, 0.0100 mol), and the resulting mixture stirred to 30 minutes. The mixture was then partitioned between ethyl acetate and saturated aqueous NaHCO₃. The combined organic layers were dried (MgSO₄), filtered, and concentrated *in vacuo* to an oil, used immediately without purification. A solution of the triflate (400 mg, 1.00 mmol) and 5-((*teri*butyloxy)carbonyl)amino-2-pentyne (366 mg, 2.00 mmol) in NEt₃ (10 mL) was treated with *bis*-triphenylphosphine palladium dichloride (20 mg) and CuI (10 mg), and the resulting mixture was de-gassed thoroughly with argon, and heated at reflux for 12 hours. The solution was then cooled, and volatiles removed *in vacuo,* and the residue was purified by chromatography over silica gel eluting with 1:1 ethyl acetate/hexanes to yield 400 mg (93%) of 2-(2-carboxyethyl)-8-(5-N-Boc-aminopentynyl)-2,3,4,5-tetrahydro-1H-2-benzazepin-1-one methyl ester as a colorless oil; ¹H NMR(CDCl₃) d7.68(s, 1H), 7.37(d, J=6Hz, 1H), 7.50(d, ]=6Hz, 1H), 4.7(brs, 1H), 3.84(t, J=6Hz, 2H), 3.71(s, 3H), 3.25(q, J=6Hz, 4H), 2.73(t, J=6Hz, 4H), 2.46(t, J=6Hz, 2H), 2.00(m, 2H), 1.78(m, 2H); ¹³C NMR(CDCl₃) d171.38(CO), 170.49(CO), 155.92, 136.83, 136.06, 133.61, 131.68, 128.25, 122.55, 89.55, 80.43, 51.78, 47.18, 44.11, 39.75, 33.58, 29.92, 29.57, 28.72, 28.37, 16.88; MS(FAB) *m/z*= 429.1(MH⁺).

b) A solution of 2-(2-carboxyethyl)-8-(5-N-Boc-aminopentynyl)-2,3,4,5-tetrahydro-1H-2-benzazepin-1-one methyl ester (66 mg, 0.154 mmol) in EtOH (1 mL) was treated with trifluoroacetic acid (0.5 mL), and the resulting mixture stirred for 18 hours. The volatiles were then removed, and the residue was dissolved in tetrahydrofuran (1 mL), and treated with 50% NaOH (0.5 mL), and the mixture was stirred for 20 minutes at room temperature. Trifluoroacetic acid was then added (3 mL), and the solution was concentrated *in vacuo*. The residue was purified by HPLC (1/2' C-18 reverse-phase column) eluting with a gradient of 3:7 methanol (0.1% trifluoroaceate)/water (0.1% trifluoroaceate), time 0 to 10 min, to 7:3 methanol(0.1% trifluoroaceate)/water(0.1% trifluoroaceate), time 10 to 40 min, to yield 46 mg (95%) of 2-(2-carboxyethyl)-8-(5-aminopentynyl)-2,3,4,5-tetrahydro-1H-2-benzazepin-1-one trifluoroacetate as a light brown solid; ¹H NMR(D₂O) d7.32(s, 1H), 7.25(d, J=6Hz, 1H), 7.00(d, J=6Hz, 1H), 3.63(t, J=6Hz, 2H), 2.95(m, 4H), 2.58(t, J=6Hz, 2H), 2.50(t, J=6Hz, 2H), 2.38(t, J=6Hz, 2H), 1.80(m, 4H); MS(FAB) *m/z*= 315(MH⁺).

### Example 23

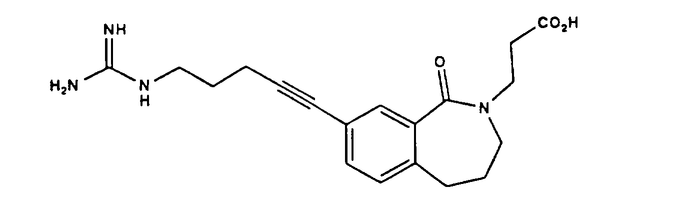

*2-(2-carboxyethyl)-8-(5-guanidinopentynyl)-2,3,4,5-tetrahydro-1H-2-benzazepin-1-one trifluoroacetate.*

a) A solution of 2-(2-carboxyethyl)-8-(5-aminopentynyl)-2,3,4,5-tetrahydro-1H-2-benzazepin-1-one trifluoroacetate (20 mg, 0.063 mmol) in saturated aqueous KHCO₃ (1 mL) was treated with formamidine sulfonic acid (80 mg, 1.0 mmol), and the resulting mixture stirred for 2 hours. The mixture was then treated with acetic acid, and the volatiles were removed. The residue was purified by HPLC (1/2' C-18 reverse-phase column) eluting with a gradient of 3:7 methanol (0.1% trifluoroaceate)/water (0.1% trifluoroaceate), time 0 to 10 min, to 7:3 methanol(0.1% trifluoroaceate)/water(0.1% trifluoroaceate), time 10 to 40 min, to yield 4.5 mg (15%) of2-(2-carboxyethyl)-8-(5-guanidinopentynyl)-2,3,4,5-tetrahydro-1H-2-benzazepin-1-one trifluoroacetate as a light brown solid; ¹H NMR(D₂O) d7.40(s, 1H), 7.25(dd, J=3, 6Hz, 1H), 7.15(d, J=6Hz, 1H), 3.80(t, J=6Hz, 2H), 3.30(m, 4H), 3.15(t, J=6Hz, 2H), 2.00(t, J=6Hz, 2H), 1.80(m, 2H); MS(FAB) *m/z*=357.1(MH⁺).

### Example 24

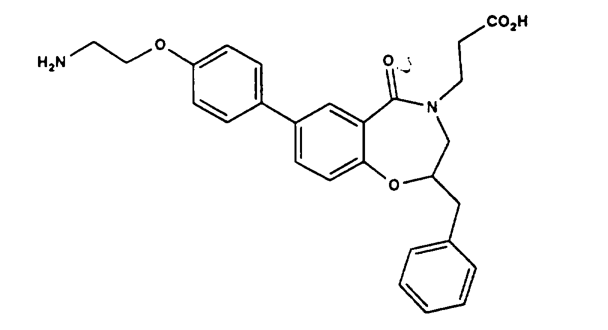

*2-(2-carboxyethyl)-8-(4-(2-aminoethoxy)phenyl)-2,3,4,5-tetrahydro-1H-5-oxa-2-benzazepin-1-one trifluoroacetate.*

a) A solution of 1-aminopropylene (10.6 grams, 0.186mol) in methanol was added to a methanolic solution of 3-phenylpropyleneoxide (5.07 grams, 0.037mol) and the mixture heated to reflux for 30 minutes. The mixture was allowed to warm to room temperature and concentrated *in vacuo* to yield 7.2 grams (99%) of N-allyl-3-phenyl-2-hydroxypropylamine. ¹H NMR (CDCl₃, dTMS) 7.34-7.18 (5H, m, Ar-H), 5.84 (1H, ddt, ³J_{HH}=17,1Hz, ³J_{HH}=10.2Hz, ³J_{HH}=6Hz, CH₂CH), 5.2-5.0 (2H, m, CH=CH₂), 3.86 (1H, m, CHOH), 3.19 (2H, m, NCH₂CH=CH₂), 2.76-2.66 (3H, m, NCH₂CH(OH)CHHPh), 2.50 (1H, dd J_{HH}=12Hz, ³J_{HH}=9Hz). ¹³C NMR (CDCl₃) 138.4, 136.5, 129.3, 128.4, 126.3, 116.0, 70.6, 54.0, 51.9, 41.7.

Using the above procedure, but substituting the appropriate alkenyloxide or allyl amine for 3-phenylpropyleneoxide or 1-aminopropylene, respectively, there may be prepared, for example, the following compounds:
N-allyl-2-hydroxypropylamine, N-(3-but-1-ene)-3-phenyl-2-hydroxypropylamine, N-(3-but-1-ene)-2-hydroxypropylamine.

b) To a magnetically stirred suspension of 5-iodosalisylic acid (Aldrich, 8.5 grams, 0.032mol), was added oxalyl chloride (8.6 grams, 0.064 mol) and one drop of dimethylformamide. The mixture was stirred until effervescence had ceased and concentrated *in vacuo.* The resulting residue was diluted with diethyl ether and added to a solution of N-allyl-3-phenyl-2-hydroxypropyl-amine (6.4 grams, 0.034 mol) in diethyl ether over a period of 30 minutes. The reaction was stirred for 1 hour, filtered, and concentrated *in vacuo.* the resulting residue was dissolved in methylene chloride and washed with 1N HCl, sat. sodium bicarbonate, dried over sodium sulfate, filtered and concentrated in vacuo to yield 12.26 grams (87%) of N-allyl-N-(2-hydroxy-5-iodobenzoyl)-2-hydroxy-3-phenylpropylamine. The material was used without further purification.

Using the above procedure, but substituting the appropriate hydroxypropylamine for N-allyl-3-phenyl-2-hydroxypropylamine there may be prepared, for example, the following compounds:
N-allyl-N-(2-hydroxy-5-iodobenzoyl)-2-hydroxypropylamine,
N-(3-but-1-ene)-N-(2-hydroxy-5-iodobenzoyl)-2-hydroxy-3-phenylpropylamine,
N-(3-but-1-ene)-N-(2-hydroxy-5-iodobenzoyl)-2-hydroxypropylamine.

c) To a magnetically stirred solution of N-allyl-N-(2-hydroxy-5-iodobenzoyl)-2-hydroxy-3-phenylpropylamine (12.05 grams, 0.027 mol) in 100mL THF was added triphenylphosphine (7.24 grams, 0.027 mol), and diethyl azodicarboxylate (4.7 grams, 0.027 mol). The mixture was heated to reflux overnight, allowed to cool to room temperature, and concentrated *in vacuo.* the resulting residue was dissolved in ethyl acetate (10mL) and diluted with diethyl ether. The solids that form were filtered and the mother liquor was concentrated *in vacuo.* The yellow oil was purified by column chromatography (SiO₂, using 15% diethyl ether/85% hexane to 1:1 diethyl ether/hexane as the eluting solvent gradient, Rf(product, 1:1 Et2O/heaxane)=0.64) to yield 4.31 grams (38%) of 2-allyl-4-benzyl-8-iodo-2,3,4,5-tetrahydro-1H-5-oxa-2-benzazepin-1-one. ¹H NMR (CDCl₃, dTMS) 8.10(1H, d, ⁴J_{HH}=2Hz, C9-H), 7.69 (1H, dd, ⁴J_{HH}=2Hz, ³J_{HH}=9Hz, C7-H), 7.4-7.2 (5H, m, C₆H₅), 6.76 (1H, d, ³J_{HH}=9Hz, C6-H), 5.79 (1H, ddt, ³J_{HH}=17Hz, ³J_{HH}=11Hz, ³J_{HH}=6Hz, CH₂CH=CH₂), 5.2-5.0 (2H, m, CH=CH₂), 5.71 (1H, m, CHOH), 4.31 (1H, ddt, J_{HH}=15Hz, ³J_{HH}=6Hz, ⁴J_{HH}=1Hz, NCHHCH=CH₂), 4.00 (1H, ddt, J_{HH}=15Hz, ³J_{HH}=6Hz, ⁴J_{HH}=1Hz, NCHHCH=CH₂), 3.34 (1H, dd, J_{HH}=16Hz, ³J_{HH}=5.1Hz, C3.H), 3.25 (1H, dd, J_{HH}=16Hz, ³J_{HH}=4.2Hz, C3-H), 3.09 (1H, dd, J_{HH}=14Hz, ³J_{HH}=7Hz, CHHPh), 2.73 (1H, dd, J_{HH}=14Hz, ³J_{HH}=7Hz, CHHPh). ¹³C NMR (CDCl₃) 166.9, 152.7, 141.4, 139.6, 136.5, 132.6, 129.8, 129.1, 128.8, 127.0, 124.6, 118.4, 86.7, 84.7, 50.4, 48.9, 38.9. IR (NaCl, cm⁻¹) 1641, 1589, 1463, 1430, 1264, 1217, 699.

Using the above procedure, but substituting the appropriate hydroxypropylamine for N-allyl-N-(2-hydroxy-5-iodobenzoyl)-2-hydroxy-3-phenylpropylamine there may be prepared, for example, the following compounds:
2-allyl-4-methyl-8-iodo-2,3,4,5-tetrahydro-1H-5-oxa-2-benzazepin-1-one,
2-(3-but-1-ene)-4-benzyl-8-iodo-2,3,4,5-tetrahydro-1H-5-oxa-2-benzazepin-1-one,
2-(3-but-1-ene)-4-methyl-8-iodo-2,3,4,5-tetrahydro-1H-5-oxa-2-benzazepin-1-one,

d) To a magnetically stirred solution of 2-allyl-4-benzyl-8-iodo-2,3,4,5-tetrahydro-1H-5-oxa-2-benzazepin-1-one (0.9234 grams, 2.2mmol) in 4mL THF at room temperature under an atmosphere of nitrogen was added 6.0mL of a 0.5M solution of 9-borabicylo[3.3.1]nonane in THF (3 mmol) and the mixture stirred for 1 hour. The reaction was quenched with 6mL of a solution of 2 grams of sodium hydroxide and 22mL of a 1:1 mixture of ethanol and water. To the resulting solution was added 7mL of a 30% solution hydrogen peroxide in water. After 30 minutes the mixture was partitioned between diethyl ether and water. The organic layer was washed with 10% citric acid and brine, dried over sodium sulfate, concentrated *in vacuo,* and purified by column chromatography (SiO2, using 1:5 ethyl acetate/methylene chloride as the eluting solvent, R_{f}(product)=0.22) to yield 0.74 grams (77%) of 2-(3-hydroxypropyl)-4-benzyl-8-iodo-2,3,4,5-tetrahydro-1H-5-oxa-2-benzazepin-1-one. 1H NMR (CDCl₃, dTMS) 8.08(1H, d, ⁴J_{HH}=2Hz, C9-H), 7.71 (1H, dd, ⁴J_{HH}=2Hz, ³J_{HH}=8Hz, C7-H), 7.4-7.2 (5H, m, C₆H₅), 6.78 (1H, d, ³J_{HH}=8Hz, C6-H), 4.64 (1H, m, ArOCH), 3.70 (2H, m, CH₂OH), 3.58 (2H, bt, ³J_{HH}=5Hz, NCH₂CH₂), 3.39 (1H, dd, J_{HH}=15Hz, ³J_{HH}=8Hz, C3-H), 3.24 (1H, dd, J_{HH}=15Hz, ³J_{HH}=3Hz, C3-H), 3.1(1H. dd, J_{HH}=14Hz, ³J_{HH}=7Hz, CHHPh), 2.76 (1H, dd, J_{HH}=14Hz, ³J_{HH}=7Hz, CHHPh), 1.68 (2H, m, CH₂CH₂OH), ¹³C NMR (CDCl₃) 168.3, 152.6, 141.6, 139.4, 136.2, 132.1, 131.9, 129.2, 129.0, 128.8, 128.4, 127.0, 124.7, 86.8, 85.7, 58.0, 49.9, 44.5, 38.8, 30.3. IR (NaCl, cm⁻¹) 3422 (b), 1636, 1463, 1436, 1217, 1064.

Using the above procedure, but substituting the appropriate 5-oxa-2-benzazepin-1-one for 2-allyl-4-benzyl-8-iodo-2,3,4,5-tetrahydro-1H-5-oxa-2-benzazepin-1-one there may be prepared, for example, the following compounds:
2-(3-hydroxypropyl)-4-methyl-8-iodo-2,3,4,5-tetrahydro-1H-5-oxa-2-benzazepin-1-one,
2-(4-hydroxybut-2-yl)-4-benzyl-8-iodo-2,3,4,5-tetrahydro-1H-5-oxa-2-benzazepin-1-one,
2-(4-hydroxybut-2-yl)-4-methyl-8-iodo-2,3,4,5-tetrahydro-1H-5-oxa-2-benzazepin-1-one.

e) To magnetically stirred solution of 2-(3-hydroxypropyl)-4-benzyl-8-iodo-2,3,4,5-tetrahydro-1H-5-oxa-2-benzazepin-1-one (0.75 grams, 1.7mmol) in 5mL dimethylformamide at room temperature was added a solution of pyridinium dichromate (2.58 grams, 6.86mmol) in 5mL dimethylformamde. The reaction mixture was stirred overnight, diluted with 75mL water, washed 3X75mL diethyl ether, made acidic to a pH of 2 with conc. HCl and extracted 3X75mL diethyl ether. The combined organics were extracted 3X40mL 1N sodium hydroxide. The combined aqueous layers were acidified to a pH of 2 with canc HCl and extracted 3X50mL diethyl ether. These ether layers were combined and dried over magnesium sulfate, filtered, and concentrated *in vacuo* to yield 0.557 grams (73%) of 2-(2-carboxyethyl)-4-benzyl-8-iodo-2,3,4,5-tetrahydro-1H-5-oxa-2-benzazepin-1-one. ¹H NMR (CDCl₃, dTMS) 8.07 (1H, d, ⁴J_{HH}=2Hz, C9-H), 7.69 (1H, dd, ⁴J_{HH}=2Hz, ³J_{HH}=8Hz, C7-H), 7.4-7.2 (5H, m, C₆H₅), 6.75 (1H, d, ³J_{HH}=8Hz, C6-H), 4.75 (1H, m, ArOCH), 3.89 (1H, dt, J_{HH}=14Hz, ³J_{HH}=6Hz, NCHHCH₂CO₂), 3.69 (1H, dt, J_{HH}=14Hz, ³J_{HH}=6Hz, NCHHCH₂CO₂), 3.44 (2H, d, ³J_{HH}=3Hz, CH₂Ph), 3.07 (1H, dd, J_{HH}=14Hz, ³J_{HH}=7Hz, C3-H), 2.8-2.7 (3H, m, C3-H, CH₂CO₂). ¹³C NMR (CDCl3) 176.1, 167.6, 164.4, 152.8, 141.7, 139.5, 136.4, 129.4,129.1,128.9, 128.8, 127.0, 124.7, 86.7, 84.7, 51.4, 45.0, 38.8, 33.0.

Using the above procedure, but substituting the appropriate 5-oxa-2-benzazepin-1-one for 2-(3-hydroxypropyl)-4-benzyl-8-iodo-2,3,4,5-tetrahydro-1H-5-oxa-2-benzazepin-1-one there may be prepared, for example, the following compounds:
2-(2-carboxyethyl)-4-methyl-8-iodo-2,3,4,5-tetrahydro-1H-5-oxa-2-benzazepin-1-one,
2-(3-carboxy-2-propyl)-4-benzyl-8-iodo-2,3,4,5-tetrahydro-1H-5-oxa-2-benzazepin-1-one,
2-(3-carboxy-2-propyl)-4-methyl-8-iodo-2,3,4,5-tetrahydro-1H-5-oxa-2-benzazepin-1-one.

f) A solution of diazomethane in ether, prepared by allowing a slurry of 2.98 grams of N-nitroso-N-methylurea and 10mL ether to react with a solution of 20 grams of KOH in 20mL water, was carefully added *via* a pipet to solution of 2-(2-carboxyethyl)-8-iodo-2,3,4,5-tetrahydro-1H-5-oxa-2-benzazepin-1-one (0.42 grams, 0.93mmol) in 20mL diethyl ether until yellow color persists. The mixture was allowed to stand overnight and concentrated *in vacuo*. The resulting residue as purified by column chromatography (SiO2, 1:9 ethyl acetate/hexane, R_{f}(product)=0.2) to yield 0.275 grams (64%) of 2-(2-carboxyethyl)-8-iodo-2,3,4,5-tetrahydro-1H-5-oxa-2-benzazepin-1-one methyl ester. ¹H NMR (CDCl₃, dTMS) 8.06 (1H, d, ⁴J_{HH}=2Hz, C9-H), 7.68 (1H, dd, ⁴J_{HH}=2Hz, ³J_{HH}=8Hz, C7-H), 7.4-7.2 (5H, m, C₆H₅), 6.75 (1H, d, ³J_{HH}=8Hz, C6-H), 4.76 (1H, m, ArOCH), 3.88 (1H, dt, J_{HH}=14Hz, ³J_{HH}=6Hz, NCHHCH₂CO₂), 3.69 (4H, m, NCHHCH₂CO₂CH₃), 3.44 (2H, m, C3-H), 3.06 (1H, dd, J_{HH}=14Hz, ³J_{HH}=7Hz, C3-H), 2.76 (1H, dd, J_{HH}=14Hz, ³J_{HH}=7Hz, CHHPh), 2.69 (2H, m, CHHPh, CH₂CO₂). ¹³C NMR (CDCl3) 172.4, 167.2, 164.4, 152.6, 141.4, 139.4, 136.5, 129.6, 129.1, 121.0, 128.8, 127.0, 124.6, 86.6, 84.7, 51.8, 51.3, 45.1, 38.7, 33.0.

Using the above procedure, but substituting the appropriate 5-oxa-2-benzazepin-1-one for 2-(2-carboxyethyl)-8-iodo-2,3,4,5-tetrahydro-1H-05-oxa-2-benzazepin-1-one there may be prepared, for example, the following compounds:
2-(2-carboxyethyl)-4-methyl-8-iodo-2,3,4,5-tetrahydro-1H-5-oxa-2-benzazepin-1-one methyl ester,
2-(3-carboxy-2-propyl)-4-benzyl-8-iodo-2,3,4,5-tetrahydro-1H-5-oxa-2-benzazepin-1-one methyl ester,
2-(3-carboxy-2-propyl)-4-methyl-8-iodo-2,3,4,5-tetrahydro-1H-5-oxa-2-benzazepin-1-one methyl ester.

g) To a magnetically stirred solution of 4-iodophenol (15 grams,0.068mol) in 100mL THF was sequentially added triphenylphosphine (19.67 grams, (0.75mol), diethyl azodicarboxylate (13.06 grams, 0.073mol), and 2-azidoethanol (5.93 grams, 0.068mol). The mixture was stirred overnight, concentrated in vacuo, diluted with 90mL ethyl acetate and diluted with hexane (400mL), and the resulting solids filtered away. The filtrate was concentrated in vacuo, diluted with diethyl ether, and extracted with 1N NaOH and water. Pentane was added to induce precipitation of a white solid that was again filtered away. The filtrate was concentrated *in vacuo*, and dissolved in methanol (60mL). Triethylamine (27 grams, 0.267mol) and propanedithiol (23 grams, 0.215mol) were added and the mixture stirred at room temperature overnight. The solution was diluted with 100mL water and partitioned between methylene chloride and 1N NaOH. The organic layer was extracted with 2X200mL 1N HCl and the aqueous layer washed with 100mL methylene chloride, made basic with 2N NaOH and extracted 3X75mL methylene chloride. The combined organics were dried over sodium carbonate, filtered and concentrate *in vacuo* to yield 12.97 grams of 2-(4-iodophenoxy)ethyl amine (72%). ¹H NMR (CDCl₃, dTMS) 7.56 (2H, d, ³J_{HH}= 9Hz, Ar-H), 6.63 (2H, d, ³J_{HH}= 9Hz, Ar-H), 4.03 (2H, t, ³J_{HH}=5Hz, CH₂N₃), 3.57 (2H, t, ³J_{HH}=5Hz, OCH₂). ¹³C NMR (CDCl₃) 158.0, 138.2, 116.9, 83.4, 66.9, 49.9. IR (NACl, cm⁻¹) 2930, 2107 (s), 1589, 1483, 1284, 1237, 1058, 819.

h) 2-(4-iodophenoxy)ethylamine (10 grams, 0.038 mol) dissolved in THF (50mL) was allowed to react with di-*t*-butyl dicarbonate (9.13 grams, 0.041mol) at room temperature for 1 hour. The reaction mixture was concentrated in vacuo to yield 17.7 grams (99%) of N-Boc-2-(4-iodophenoxy)ethylamine.

i) A slurry of 2-(2-carboxyethyl)-4-benzyl-8-iodo-2,3,4,5-tetrahydro-1H-5-oxa-2-benzazepin-1-one methyl ester (0.155 grams, 0.334mmol), N-Boc-2-(4-iodophenoxy)ethylamine (0.246 grams, 0.664mol), degassed triethylamine (2mL), bis(triphenylphosphine)palladium dichloride (11mgs, 10 mol %), and coprous iodide (6mgs, 20 mol %) was heated to reflux overnight. The reaction was concentrated *in vacuo,* triturating the resulting residue with diethyl ether (30mL). The ethereal solution was concentrated in vacuo and the residue purified by column chromatography (SiO₂, using 1:1 ethyl acetate/hexane to ethyl acetate as the eluting solvent gradient, R_{f}(product)=0.08) to yield 19.1mgs (10%) of 2-(2-carboxyethyl)-4-benzyl-8-(4-(2-N-Boc-amino)ethoxyphenyl)-2,3,4,5-tetrahydro-1H-5-oxa-2-benzazepin-1-one methyl ester. 1H NMR (CDCl₃, dTMS) 7.94 (1H, d, ⁴J_{HH}=2Hz, C9-H), 7.59 (1H, dd, ⁴J_{HH}=2Hz, ³J_{HH}=8Hz, C7-H), 7.52 (2H, d, ³J_{HH}=9Hz, C8-*o*-Ar-H), 7.40-7.25 (5H, m, C₆H₅), 7.05 (1H, d, ³J_{HH}=8Hz, C6-H), 6.96 (2H, d, ³J_{HH}=9Hz, C8-*in*-Ar-H), 5.03 (1H, bs, NH), 4.76 (1H, m, OCH), 4.06 (2H, t, ³J_{HH}=5Hz, OCH₂CH₂N), 3.92 (1H, dt, J_{HH}=14Hz, ³J_{HH}=7Hz, NCHHCH₂CO₂), 3.73 (1H, dt, J_{HH}=14Hz, ³J_{HH}=7Hz, NCHHCH₂CO₂), 3.67 (3H, s, CH₃), 3.56 (2H, bq, ³J_{HH}=6Hz, CH₂NH), 3.51 (1H, dd, J_{HH}=16Hz, ³J_{HH}=8Hz, C3-H), 3.43 (1H, dd, J_{HH}=16Hz, ³J_{HH}=5Hz, C3-H), 3.12 (1H, dd, J_{HH}=14Hz, ³J_{HH}=7Hz, CHHPh), 2.78 (1H, dd, 2J_{HH}=14Hz, ³J_{HH}=7Hz, CHHPh), 2.72 (2H, m, CH₂CO₂), 1.48 (9H, s, Bu^{t}),¹³C NMR (CDCl3) 172.6, 168.9, 136.8, 136.5, 130.8, 129.1, 128.8, 128.7, 128.3. 126.9, 123.0, 114.8, 84.6, 60.4, 51.8, 51.5, 45.0, 38.8, 33.2, 28.3.

Using the above procedure, but substituting the appropriate 5-oxa-2-benzazepin-1-one for 2-(2-carboxyethyl)-8-iodo-2,3,4,5-tetrahydro-1H-5-oxa-2-benzazepin-1-one there may be prepared, for example, the following compounds:
2-(2-carboxyethyl)-4-methyl-8-(4-(2-N-Boc-amino)ethoxyphenyl)-2,3,4,5-tetrahydro-1H-5-oxa-2-benzazepin-1-one methyl ester,
2-(3-carboxy-2-propyl)-4-benzyl-8-(4-(2-N-Boc-amino)ethoxyphenyl)-2,3,4,5-tetrahydro-1H-5-oxa-2-benzazepin-1-one methyl ester,
2-(3-carboxy-2-propyl)-4-methyl-8-(4-(2-N-Boc-amino)ethoxyphenyl)-2,3,4,5-tetrahydro-1H-5-oxa-2-benzazepin-1-one methyl ester,
2-(2-carboxyethyl)-4-benzyl-8-[4-(4-N-Boc-piperazine)phenyl]-2,3,4,5-tetrahydro-1H-5-oxa-2-benzazepin-1-one methyl ester,
2-(2-carboxyethyl)-4-methyl-8-[4-(4-N-Boc-piperazine)phenyl]-2,3,4,5-tetrahydro-1H-5-oxa-2-benzazepin-1-one methyl ester,
2-(3-carboxy-2-propyl)-4-benzyl-8-[4-(4-N-Boc-piperazine)phenyl]-2,3,4,5-tetrahydro-1H-5-oxa-2-benzazepin-1-one methyl ester,
2-(3-carboxy-2-propyl)-4-methyl-8-[4-(4-N-Boc-piperazine)phenyl]-2,3,4,5-tetrahydro-1H-5-oxa-2-benzazepin-1-one methyl ester,
2-(2-carboxyethyl)-4-benzyl-8-[4-(4.N-Boc-aminomethyl)phenyl]-2,3,4,5-tetrahydro-1H-5-oxa-2-benzazepin-1-one methyl ester,
2-(2-carboxyethyl)-4-methyl-8-[4-(4-N-Boc-aminomethyl)phenyl]-2,3,4,5-tetrahydro-1H-5-oxa-2-benzazepin-1-one methyl ester,
2-(3-carboxy-2-propyl)-4-benzyl-8-[4-(4-N-Boc-aminomethyl)phenyl]-2,3,4,5-tetrahydro-1H-5-oxa-2-benzazepin-1-one methyl ester,
2-(3-carboxy-2-propyl)-4-methyl-8-[4-(4-N-Boc-aminomethyl)phenyl]-2,3,4,5-tetrahydro-1H-5-oxa-2-benzazepin-1-one methyl ester.

j) To a solution of 2-(2-carboxyethyl)-4-benzyl-8-(4-(2-N-Boc-amino)ethoxyphenyl)-2,3,4,5-tetrahydro-1H-5-oxa-2-benzazepin-1-one methyl ester (19.1mgs, 0.03mmol) in 0.5mL acetonitrile and 0.5mL water was added a concentrated solution of HCl in ethyl acetate (1mL) and the reaction allowed to stand for 15 minutes, concentrated *in vacuo,* dissolved in methanol (1mL0 and allowed to react with 2N NaOH (1mL). The reaction was quenched with acetic acid (0.5mL) and concentrated *in vacuo,* dissolved in a minimum of water and purified by high-pressure column chromatography (HPLC, 1/2" reverse-phase C-18 column, 1:4 acetonitrile(0.1%TFA)/water (0.1%TFA) to 3:2 acetonitrile(0.1%TFA)/water (0.1%TFA) as the eluting solvent gradient) to yield 4.2mgs (30%) of 2-(2-carboxyethyl)-8-(4-(2-aminoethoxy)phenyl)-2,3,4,5-tetrahydro-1H-5-oxa-2-benzazepin-1-one. ¹H NMR (CD₃OD) 7.80(1H, d, ⁴J_{HH}=2Hz, C9-H), 7.67 (1H, dd, ⁴J_{HH}=2Hz, ³J_{HH}=8Hz, C7-H), 7.58 (2H, d, ³J_{HH}=9Hz, C8-*o*-Ar-H), 7.40-7.35 (5H, m, C₆H₅), 7.10 (2H, d, ³J_{HH}=9Hz, C8-*m*-Ar-H), 7.03 (1H, d, ³J_{HH}=8Hz, C6-H), 4.28 (2H, t, 3J_{HH}=4Hz, OCH₂CH₂), 3.81 (2H, m, NCH₂CH₂CO₂), 3.61(1H, dd, J_{HH}=14Hz, ³J_{HH}=1Hz, C3-H), 3.44 (1H, dd, J_{HH}=14Hz, ³J_{HH}=4Hz, C3-H), 3.41 (2H, t, ³J_{HH}=5Hz, CH₂Ph), 2.87 (2H, t, ³J_{HH}=7Hz, CH₂NH₂), 2.50 (2H, t, ³J_{HH}=7Hz, CH₂CO₂). HRMS (FAB, MH⁺)m/z= 461.2109 (cald for C₂₇H₂₉N₂O₅: 461.2076).

Using the above procedure, but substituting the appropriate 5-oxa-2-benzazepin-1-one for 2-(2-carboxyethyl)-4-benzyl-8-(4-(2-N-Boc-amino)ethoxyphenyl)-2,3,4,5-tetrahydro-1H-5-oxa-2-benzazepin-1-one methyl ester there may be prepared, for example, the following compounds:
2-(2-carboxyethyl)-4-methyl-8-(4-(2-amino)ethoxyphenyl)-2,3,4,5-tetrahydro-1H-5-oxa-2-benzazepin-1-one,
2-(3-carboxy-2-propyl)-4-benzyl-8-(4-(2-amino)ethoxyphenyl)-2,3,4,5-tetrahydro-1H-5-oxa-2-benzazepin-1-one,
2-(3-carboxy-2-propyl)-4-methyl-8-(4-(2-amino)ethoxyphenyl)-2,3,4,5-tetrahydro-1H-5-oxa-2-benzazepin-1-one,
2-(2-carboxyethyl)-4-benzyl-8-[4-(1-piperazine)phenyl]-2,3,4,5-tetrahydro-1H-5-oxa-2-benzazepin-1-one,
2-(2-carboxyethyl)-4-methyl-8-[4-(1-piperazine)phenyl]-2,3,4,5-tetrahydro-1H-5-oxa-2-benzazepin-1-one,
2-(3-carboxy-2-propyl)-4-benzyl-8-[4-(1-piperazine)phenyl]-2,3,4,5-tetrahydro-1H-5-oxa-2-benzazepin-1-one,
2-(3-carboxy-2-propyl)-4-methyl-8-[4-(1-piperazine)phenyl]-2,3,4,5-tetrahydro-1H-5-oxa-2-benzazepin-1-one,
2-(2-carboxyethyl)-4-benzyl-8-[4-(4-aminomethyl)phenyl)-2,3,4,5-tetrahydro-1H-5-oxa-2-benzazepin-1-one,
2-(2-carboxyethyl)-4-methyl-8-[4-(4-aminomethyl)phenyl]-2,3,4,5-tetrahydro-1H-5-oxa-2-benzazepin-1-one,
2-(3-carboxy-2-propyl)-4-benzyl-8-[4-(4-aminomethyl)phenyl]-2,3,4,5-tetrahydro-1H-5-oxa-2-benzazepin-1-one,
2-(3-carboxy-2-propyl)-4-methyl-8-[4-(4-aminomethyl)phenyl]-2,3,4,5-tetrahydro-1H-5-oxa-2-benzazepin-1-one.

### Example 25

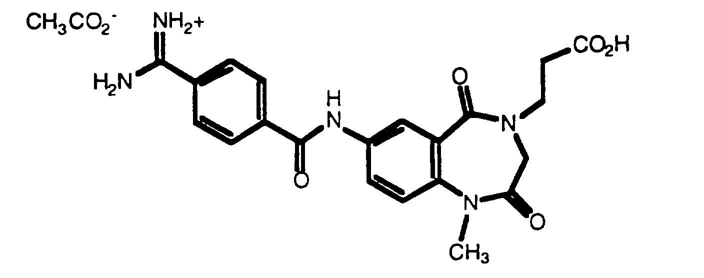

*1-Methyl-4-(2-carboxyethyl)-7-(4-amidino)benzamido-3,4-dihydro-1H-1,4-benzodiazepine-2,5-dione acetate*

a) A solution of N-methylisatoic anhydride (64.0 g, 0.325 mol) and b-alanine ethyl ester hydrochloride (50.0 g, 0.325 mol) in N,N-dimethylformamide (325 mL) was treated with N,N-dimethylaminopyridine (2.0 g), the resulting mixture cooled to 0°C (ice bath), and triethylamine (46.0 mL, 0.325 mol) was added in one batch. Vigorous CO₂ evolution was noted. The mixture was stirred for 18 h at room temperature, then diluted with 400 mL ethyl acetate. The solution was washed exhaustively with water and brine to remove the N,N-dimethylformamide, then dried over MgSO₄, filtered and concentrated in vacuo. The residue was adsorbed on silica gel, and elution with a gradient of hexane to 1:1 ethyl acetate-hexane yielded 80 g (98%) of an oil. ¹H NMR (CDCl₃, dTMS) 7.28 (3H, m), 6.75 (1H, s, NHCH₃), 6.62 (1H, d, J=9Hz, ArH), 6.55(1H, t, J=9Hz, ArH), 4.14 (q, J=9Hz, 2H, CH₂O), 3.63 (q, J=6Hz, NCH₂CH₂), 2.83 (s, 3H, NCH₃), 2.59(2H, t, J=6Hz, CH₂CO₂Et), 1.24 (3H, t, J=9Hz, CH₂CH₃); ¹³C NMR(CDCl₃, dTMS) 172.88, 169.89, 150.71, 132.96, 127.42, 115.01, 114.60, 111.16, 60.87, 35.11, 34.19, 29.74, 14.28.

b) A solution of N-(N-methyl-2-aminobenzoyl)-b-alanine ethyl ester (80.0 g, 0.319 mol) in CH₂Cl₂ (400 mL) and H₂O (200 mL) was cooled to 0°C (ice bath) and treated rapidly dropwise with bromoacetylbromide (42 mL, 0.478 mol). After the addition was complete, the phases were separated, and the aqueous phase was washed twice with CH₂Cl₂, and the combined organic phases were dried (MgSO₄), filtered and concentrated *in vacuo* to yield 105 g (89%) of an oil. This material was dissolved in N,N-dimethylformamide (200 mL), and with rapid stirring, was treated in one portion with Cs₂CO₃ (93.0 g, 0.283 mol). The mixture was stirred for 1/2 h at room temperature, then diluted with ethyl acetate and washed exhaustively with brine and water to remove N,N-dimethylformamide. The solution was then dried (MgSO₄), filtered and concentrated in vacuo. The residue was dissolved in minimal ethyl acetate and diluted with hexane until cloudy. A small amount of ethyl acetate was then added, and the mixture stored at -20°C. The crystals that had formed were collected and washed with hexane, then dried *in vacuo* to yield 43 g (52%) of colorless crystals, mp. 119-121°C. ¹H NMR(CDCl₃, dTMS) 7.83 (1H, dd, J=1.5, 8Hz, ArH), 7.51 (1H, td, J=1.5, 6Hz, ArH), 728 (1H, t, J=6Hz, ArH), 7.18 (1H, d, J=9Hz, ArH), 4.13(2H, q, J=9Hz, CH₂O), 4.03 (1H, B part, ABq, J=15Hz, CH₂CO), 3.92 (2H, t, J=6Hz, NCH₂), 3.84 (1H, A part, ABq, J=15Hz, CH₂CO), 3.37 (s, 3H, NCH₃), 2.71 (2H, m, complex, CH₂CO₂Et), 1.24 (3H, t, J=6Hz, CH₂CH₃),171.43, 169.16, 167.26, 141.08, 132.25, 130.92, 128.74, 125.75, 121.07, 60.89, 52.31, 45.18, 35.03, 32.97, 14.29; MS(FAB) *m/z* 291(MH⁺).

c) Fuming nitric acid (200 mL) was cooled to 0°C and treated portionwise with 1-methyl-4-(2-carboxyethyl)-3,4-dihydro-1*H*-1,4,benzodiazepine-2,5-dione ethyl ester (43.0 g, 0.148 mol), and the resulting dark yellow mixture was stirred for 24 h at room temperature. The solution was then poured over ice onto solid NaHCO₃ and extracted exhaustively with ethyl acetate. The combined organic layers were dried (MgSO₄), filtered and concentrated *in vacuo* to yield 36 g (73%) af an orange oil; ¹H NMR(CDCl₃, dTMS) 8.70(1H, d, J=3Hz, ArH), 8.34 (1H, dd, J=3, 9Hz, ArH), 7.38 (1H, d, J=9Hz, ArH), 4.14 (2H, q, J=6Hz, CH₂O), 4.04 (2H, d, J=6Hz, CH₂CO), 3.93 (2H, t, J=6Hz, NCH₂), 3.44 (3H, s, NCH₃), 2.74 (2H, m, complex, CH₂CO₂Et), 1.26 (3H, t, J=6Hz, CH₂CH₃); ¹³C NMR(CDCl₃) d194.51, 191.54, 188.52, 168.96, 167.69, 152.58, 150.31, 150.05, 145.12, 84.22, 75.21, 68.61, 58.39, 55.97, 37.44; MS(FAB) *m/z* 336.1(MH⁺).

Using the same above method, but substituting the appropriate 1-alkyl-4-(2-carboxyethyl)-3,4-dihydro-1*H*-1,4-benzodiazepine-2,5-dione ethyl ester for 1-methyl-4-(2-carboxyethyl)-3,4-dihydro-1*H*-1,4-benzodiazepine-2,5-dione ethyl ester there may be prepared, for example, the following compounds:
1-ethyl-4-(2-carboxyethyl)-7-nitro-3,4-dihydro-1*H*-1,4-benzodiazepine-2,5-dione ethyl ester,
1-isopropyl-4-(2-carboxyethyl)-7-nitro-3,4-dihydro-1*H*-1,4-benzodiazepine-2,5-dione ethyl ester,
1-isobytyl-4-(2-carboxyethyl)-7-nitro-3,4-dihydro-1*H*-1,4-benzodiazepine-2,5-dione ethyl ester,
1-cyclohexyl-4-(2-carboxyethyl)-7-nitro-3,4-dihydro-1*H*-1,4-benzodiazepine-2,5-dione ethyl ester.

d) A solution of 1-methyl-4-(2-carboxyethyl)-7-nitro-3,4-dihydro-1H-1,4-benzodiazepine-2,5-dione ethyl ester (36.0 g, .107 mol) in ethyl acetate (200 mL) and absolute ethanol (150 mL) was hydrogenated over Pd on carbon (4 g) at 40 PSI for 24 h. The black suspension was then filtered and concentrated *in vacuo* to give 32.3 g (100%) of a colorless foam. This foam was dissolved in CH₂Cl₂, cooled to 0°C (ice bath) and treated with NEt₃ (17.5 mL, 0.126 mol) followed by 4-cyanobenzoyl chloride (20.86 g, 0.126 mol), and the resulting mixture was stirred for 24 h at room temperature. The mixture was then washed with water and brine, dried over MgSO₄, filtered and concentrated *in vacuo* to yield a light brown solid. Recrystallization of the solid from CH₂Cl₂/hexanes yielded 33 g (76%) of a white granular solid: mp. 158-160°C; ¹H NMR(CDCl₃, dTMS) 8.76 (1H, s, NH), 8.00 (1H, dd, J=3, 9Hz, ArH), 7.72 (1H, d, J=9Hz, ArH), 7.58 (1H, d, J=3Hz, ArH), 7.47 (1H, d, J=9Hz, ArH), 6.94 (1H, d, J=9Hz, ArH), 3.83 (2H, q, J=6Hz, OCH₂CH₃), 3.77 (1H, B part, ABq, J=15Hz, CH₂CO), 35.7 (1H, A part, ABq, J=15Hz, CH₂CO), 3.53 (2H, t, J=6Hz, NCH₂), 3.09 (3H, s, NCH₃), 2.34 (2H, m, complex, CH₂CO₂Et), 0.95 (3H, t, J=6Hz, OCH₂CH₃); ¹³C NMR(CDCl₃) d171.04, 168.57, 166.90), 164.31, 138.24, 137.34, 135.51, 132.47, 128.60, 128.05, 124.64, 121.85, 117.83, 115.51, 60.94, 52.09, 45.01, 34.92, 32.60, 14.13; MS(FAB) *m/z* 435(MH⁺).

Using the same above procedure, but substituting the appropriate cyano acid chloride or amino protected amino acid chloride for 4-cyanobenzoyl chloride there may be prepared, for example, the following compounds:
1-methyl-4-(2-carboxyethyl)-7-(3-cyano)benzamido-3,4-dihydro-1*H*-1,4-benzodiazepine-2,5-dione ethyl ester,
1-methyl-4-(2-carboxyethyl)-7-(4-cyano)butanamido-3,4-dihydro-1*H*-1,4-benzodiazepine-2,5-dione ethyl ester,
1-methyl-4-(2-carboxyethyl)-7-(5-cyano)pentamido-3,4-dihydro-1*H*-1,4-benzodiazepine-2,5-dione ethyl ester,
1-methyl-4-(2-carboxyethyl)-7-4-(N-Boc)-aminobutanamido-3,4-dihydro-1*H*-1,4-benzodiazepine-2,5-dione ethyl ester,
1-methyl-4-(2-carboxyethyl)-7-5-(N-Boc)-aminopentamido-3,4-dihydro-1*H*-1,4-benzodiazepine-2,5-dione ethyl ester. Furthermore, using the same procedure but substituting the appropriate 1-alkyl-4-(2-carboxyethyl)-7-nitro-3,4-dihydro-1H-1,4-benzodiazepine-2,5-dione ethyl ester for 1-methyl-4-(2-carboxyethyl)-7-nitro-3,4-dihydro-1H-1,4-benzodiazepine-2,5-dione ethyl ester there may be prepared, for example, the following cnmpounds:
1-ethyl-4-(2-carboxyethyl)-(4-cyano)benzamido-3,4-dihydro-1*H*-1,4-benzodiazepine-2,5-dione ethyl ester,
1-isopropyl-4-(2-carboxyethyl)-(4-cyano)benzamido-3,4-dihydro-1*H*-1,4-benzodiazepine-2,5-dione ethyl ester,
1-isobytyl-4-(2-carboxyethyl)-(4-cyano)benzamido-3,4-dihydro-1*H*-1,4-benzodiazepine-2,5-dione ethyl ester,
1-cyclohexyl-4-(2-carboxyethyl)-(4-cyano)benzamido-3,4-dihydro-1*H*-1,4-benzodiazepine-2,5-dione ethyl ester.

e) A solution of 1-methyl-4-(2-carboxyethyl)-7-(4-cyano)benzamido-3,4-dihydro-1*H*-1,4-benzodiazepine-2,5-dione ethyl ester (10.0 g, 0.023 mol) in NEt₃ (50 mL) and pyridine (70 mL) was saturated with H₂S, then heated at 70°C for 24 h. The solvents were removed in vacuo, and the residue remaining was suspended in CH₂Cl₂ (200 mL) and treated with iodomethane (10 mL). The resulting solution was heated at reflux for 24 h. The volatiles were then removed *in vacuo,* and the resulting residue was dissolved in EtOH (100 mL) and treated with NH₄OAc (3.2 g), and the resulting suspension heated to 50°C with stirring for 24 h. The mixture was concentrated *in vacuo*, and the product was dissolved in 0.5% aqueous HOAc, and isolated by HPLC to yield a white solid; ¹H NMR (D₂O, TFA salt) d7.72 (2H, d, J=12Hz, ArH), 7.65 (2H, d, J=12Hz, ArH), 7.63 (1H, d, J=3Hz, ArH), 7.53 (1H, dd, J=3, 9Hz, ArH), 7.12 (1H, d, J=12Hz, ArH), 4.40 (2H, B part, ABq, J=15Hz, C3-H and m, CHHCH₂CO₂), 3.91(2H, q, J=6Hz, OCH₂), 3.68 (1H, A part, ABq, C3-H), 3.48 (1H, m, CHHCH₂CO₂), 3.18 (3H, s, NCH₃), 2.54 (2H, m, CH₂CO₂), 0.98 (3H, t, J=6Hz, OCH₂CH₃); MS (FAB) *m/z* 452(MH⁺). Using the above procedure, but substituting the appropriate 1-alkyl-4-(2-carboxyethyl)-7-(4-cyano)alkyl/benzamido-3,4-dihydro-1*H*-1,4-benzodiazepine-2,5-dione ethyl ester for 1-methyl-4-(2-carboxyethyl)-7-(4-cyano)benzamido-3,4-dihydro-1*H*-1,4-benzodiazepine-2,5-dione ethyl ester, there may be prepared, for example, the following compounds.
1-methyl-4-(2-carboxyethyl)-7-(3-amidino)benzamido-3,4-dihydro-1*H*-1,4-benzodiazepine-2,5-dione ethyl ester,
1-methyl-4-(2-carboxythyl)-7-(4-amidino)butanamido-3,4-dihydro-1*H*-1,4-benzodiazepine-2,5-dione ethyl ester,
1-methyl-4-(2-carboxyethyl)-7-(5-amidino)pentamido-3,4-dihydro-1*H*-1,4-benzodiazepine-2,5-dione ethyl ester,
1-ethyl-4-(2-carboxyethyl)-(4-amidino)benzamido)-3,4-dihydro-1*H*-1,4-benzodiazepine-2,5-dione ethyl ester,
1-isopropyl-4-(2-carboxyethyl)-(4-amidino)benzamido-3,4-dihydro-*H*-1,4-benzodiazepine-2,5-dione ethyl ester,
1-isobytyl-4-(2-carboxyethyl)-(4-amidino)benzamido-3,4-dihydro-1*H*-1,4-benzodiazepine-2,5-dione ethyl ester,
1-cyclohexyl-4-(2-carboxyethyl)-(4-amidino)benzamido-3,4-dihydro-1*H*-1,4-benzodiazepine-2,5-dione ethyl ester.

f) A solution of 1-methyl-4-(2-carboxyethyl)-7-(4-amidino)benzamido-3,4-dihydro-1*H-*1,4-benzodiazepine-2,5-dione ethyl ester acetate (0.2 g, 0.456 mmol) in THF (20 mL) and 50% aqueous NaOH (3 mL) was stirred for 24 h at room temperature. Acetic acid was then added, and the solution was concentrated *i*n *vacuo.* The residue remaining was purified by HPLC to yield 122 mg (57%) of a white foam (isolated by lyophylization); ¹H NMR(D₂O) d7.73 (2H, d, J=6Hz, ArH), 7.63 (2H, d, J=6Hz, ArH), 7.62 (1H, m, ArH), 7.54 (1H, dd, J=3, 9Hz, ArH), 7.12 (1H, d, J=9Hz, ArH), 3.87 (1H, B part, ABq, J=15Hz, CH₂CO) overlapping 3.84 (1H, m, complex), 3.20 (1H, A part, ABq, J=15Hz, CH₂CO), 3.48 (1H, m), 3.03 (3H, s, NCH₃), 2.50 (2H, m, complex, CH₂CO); MS(FAB) *m/z* 424.3(MH⁺); High Resolution MS(FAB) calculated for C₂₁H₂₂N₅O₅ 424.1620, found 424.1647.

Using the above method, but substituting the appropriate 1-alkyl-4-(2-carboxyethyl)-7-(4-amidino)alkyl/benzamido-3,4-dihydro-1*H*-1,4-benzadiazepine-2,5-dione ethyl ester for 1-methyl-4-(2-carboxyethyl)-7-(4-amidino)benzamido-3,4-dihydro-1*H*-1,4-benzodiazepine-2,5-dione ethyl ester, there may be prepared, for example, the following compounds:
1-methyl-4-(2-carboxyethyl)-7-(3-amidino)benzamido-3,4-dihydro-1*H*-1,4-benzodiazepine-2,5-dione acetate,
1-methyl-4-(2-carboxyethyl)-7-(4-amidino)butanamido-3,4-dihydro-1*H*-1,4-benzodiazepine-2,5-dione acetate,
1-methyl-4-(2-carboxyethyl)-7-(5-amidino)pentamido-3,4-dihydro-1*H*-1,4-benzodiazepine-2,5-dione acetate,
1-ethyl-4-(2-carboxyethyl)-(4-amidino)benzamido-3,4-dihydro-1*H*-1,4-benzodiazepine-2,5-dione acetate,
1-isopropyl-4-(2-carboxyethyl)-(4-amidino)benzamido-3,4-dihydro-1*H*-1,4-benzodiazepine-2,5-dione acetate,
1-isobytyl-4-(2-carboxythyl)-(4-amidino)benzamido-3,4-dihydro-1*H*-1,4-benzodiazepine-2,5-dione acetate,
1-cyclohexyl-4-(2-carboxyethyl)-(4-amidino)benzamido-3,4-dihydro-1*H*-1,4-benzodiazepine-2,5-dione acetate.

### Example 26

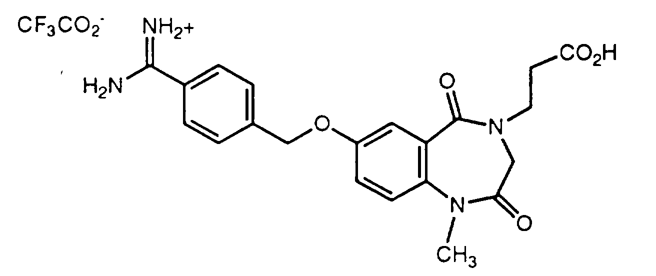

*1-methyl-4-(2-carboxyethyl)-7-(4-amidinobenzyloxy)-3,4-dihydro-1H-1,4-benzodiazepine-2,5-dione trifluoroacetate.*

a) To a stirred solution of 643 mg (2.11 mmol) of 1-methyl-4-(2-carboxyethyl)-7-amino-3,4-dihydro-1*H*-1,4-benzodiazepine-2,5-dione ethyl ester (prepared from 1-methyl-4-(2-carboxyethyl)-7-nitro-3,4-dihydro-1*H*-1,4-benzodiazepine-2,5-dione ethyl ester as a colorless foam as described in part (d) of example 26) in 60 mL of 0.1 N sulfuric acid at 0°C was added a solution of 153 mg (2.21 mmol) of sodium nitrite in 5 ml of water. After 10 min., a solution of 49.0 g (211 mmol) of copper (II) nitrate hemipentahydrate in 90 mL of water was added followed by 287 mg (2.00 mmol) of copper (II) oxide. The mixture was warmed to ambient temperature and extracted with three portions of methylene chloride. The combined extracts were washed with water, brine, dried over anhydrous magnesium sulfate, and concentrated *in vacuo.* Chromatography (silica gel 60, gradient elution from 1:2 ethyl acetate/hexane to ethyl acetate, R_{f} = 0.2 in 2:1 ethyl acetate/hexane on silica) gave 353 mg (55%) of 1-methyl-4-(2-carboxyethyl)-7-hydroxy-3,4-dihydro-1*H*-1,4-benzodiazepine-2,5-dione ethyl ester as a colorless stable foam. ¹H NMR (300 MHz, CDCl₃)d 8.60 (1H, bs, Ar-OH), 7.52 (1H, bs, Ar-H), 7.1-7.0 (2H, m, Ar-H), 4.12 (2H, q, J=7.3 Hz, OCH₂CH₃), 4.07 (1H, d, J=14.7 Hz, NCHHCON), 3.90 (2H, m, NCH₂CH₂), 3.86 (1H, d, J=14.7 Hz, NCHHCON), 3.33 (3H, s, NCH₃), 2.70 (2H, m, NCH₂CH₂), 1.23 (3H, t, J=7.3 Hz, OCH₂CH₃)); Exact mass (FAB, M+H⁺) calcd for C₁₅H₁₉ N₂O₅: 307.1294; Found: 307.1289.

Using the above procedure, but substituting the appropriate 1-alkyl-4-(2-carboxyethyl)-7-nitro-3,4-dihydro-1*H*-1,4-benzodiazepine-2,5-dione ethyl ester for 1-methyl-4-(2-carboxyethyl)-7-nitro-3,4-dihydro-1*H*-1,4-benzodiazepine-2,5-dione ethyl ester, there can be prepared, for example, the following compounds :
1-ethyl-4-(2-carboxyethyl)-7-hydroxy-3,4-dihydro-1*H*-1,4-benzodiazepine-2,5-dione ethyl ester,
1-isopropyl-4-(2-carboxyethyl)-7-hydroxy-3,4-dihydro-1*H*-1,4-benzodiazepine-2,5-dione ethyl ester,
1-isobytyl-4-(2-carboxyethyl)-7-hydroxy-3,4-dihydro-1*H*-1,4-benzodiazepine-2,5-dione ethyl ester,
1-cyclohexyl-4-(2-carboxyethyl)-7-hydroxy-3,4-dihydro-1*H*-1,4-benzodiazepine-2,5-dione ethyl ester.

b) To a stirred solution of 121 mg (0.395 mmol) of 1-methyl-4-(2-carboxyethyl)-7-hydroxy-3,4-dihydro-1*H*-1,4-benzodiazepine-2,5-dione ethyl ester and 93.0 mg (0.474 mmol) of a-bromo-p-tolylnitrile in 2 ml of dry dimethylformamide was added 60.0 mg (0.434 mmol) of potassium carbonate. The mixture was stirred overnight at ambient temperature then partitioned between ethyl acetate and water. The organic phase was separated and washed with water, brine, dried over anhydrous magnesium sulfate, and concentrated *in vacuo*. Chromatogrpahy (silica gel 60, gradient elution from 2:1 ethyl acetate/hexane to ethyl acetate, R_{f} = 0.35 in 2:1 ethyl acetate/hexane on silica) gave 125 mg (75%) of 1-methyl-4-(2-carboxyethyl)-7-(4-cyanobenzyloxy)-3,4-dihydro-1*H*-1,4-bennzodiazepine-2,5-dione ethyl ester as a colorless foam. ¹H NMR (300 MHz, CDCl₃) d 7.68 (2H, d, J=8.8 Hz, NC-Ar-H₂), 7.54 (2H, d, J=8.8 Hz, NC-Ar-H₂),
7.52 (1H, bs, O-Ar-H), 7.13 (2H, bs, O-Ar-H), 5.16 (2H, ABq, J=12.7 Hz, ArCH₂O), 4.13 (2H, q, J=7.3 Hz, OCH₂CH₃), 4.03 (1H, d, J=14.7 Hz, NCHHCON), 3.92 (2H, m, NCH₂CH₂), 3.84 (1H, d, J=14.7 Hz, NCHHCON), 3.34 (3H, s, NCH₃), 2.70 (2H, m, NCH₂CH₂), 1,24 (3H, t, J=7.3 Hz, OCH₂CH₃); Exact mass (FAB, M+H⁺) calcd for C₂₃H₂₄ N₃O₅: 422.1716; Found: 422.1744.

Using the above procedure, but substituting the appropriate 1-alkyl-4-(2-carboxyethyl)-7-hydroxy-3,4-dihydro-1*H*-1,4-benzodiazepine-2,5-dione ethyl ester for 1-methyl-4-(2-carboxyethyl)-7-hydroxy-3,4-d**i**hydro-1*H*-1,4-benzodiazepine-2,5-dione ethyl ester there may be prepared, for example, the following compounds:
1-ethyl-4-(2-carboxyethyl)-7-(4-cyanobenzyl)oxy-3,4-dihydro-1*H*-1,4-benzodiazepine-2,5-dione ethyl ester,
1-isopropyl-4-(2-carboxyethyl)-7-(4-cyanobenzyl)oxy-3,4-dihydro-1*H*-1,4-benzodiazepine-2,5-dione ethyl ester,
1-isobytyl-4-(2-carboxyethyl)-7-(4-cyanobenzyl)oxy-3,4-dihydro-1*H*-1,4-benzodiazepine-2,5 dione ethyl ester,
1-cyclohexyl-4-(2-carboxyethyl)-7-(4-cyanobenzyl)oxy-3,4-dihydro-1*H*-1,4-benzodiazepine-2,5-dione ethyl ester,
1-ethyl-4-(2-carboxyethyl)-7-(3-cyanobenzyl)oxy-3,4-dihydro-1*H*-1,4-benzodiazepine-2,5-dione ethyl ester,
1-isopropyl-4-(2-carboxyetyl)-7-(3-cyanobenzyl)oxy-3,4-dihydro-1*H*-1,4-benztldiazepine-2,5-dione ethyl ester,
1-isobutyl-4-(2-carboxyethyl)-7-(3-cyanobenzyl)oxy-3,4-dihydro-1*H*-1,4-benzodiazepine-2,5-dione ethyl ester,
1-cyclohexyl-4-(2-carboxyethyl)-7-(3-cyanobenzyl)oxy-3,4-dihydro-1*H*-1,4-benzodiazepine-2,5-dione ethyl ester,
1-ethyl-4-(2-carboxyethyl)-7-(4-cyanobutyl)oxy-3,4-dihydro-1*H*-1,4-benzodiazepine-2,5-dione ethyl ester,
I-isopropyl-4-(2-carboxyethyl)-7-(4-cyanobutyl)oxy-3,4-dihydro-1*H*-1,4-benzodiazepine-2,5-dione ethyl ester,
1-isobytyl-4-(2-caroxyethyl)-7-(4-cyanobutyl)oxy-3,4-dihydro-1*H*-1,4-benzodiazepine-2,5-dione ethyl ester,
1-cyclohexyl-4-(2-carboxyethyl)-7-(4-cyanobutyl)oxy-3,4-dihydro-1*H*-1,4-benzodiazepine-2,5-dione ethyl ester.

c) Hydrogen sulfide gas was bubbled into a solution of 83.6 mg (0.198 mmol) of 1-methyl-4-(2-carboxyethyl)-7-(4-cyanobenzyloxy)-3,4-dihydro-1*H*-1,4-benzodiazepine-2,5-dione ethyl ester in 3 mL of 1:1 pyridine/triethylamine for 15 min and the mixture was warmed in a 50 °C oil bath for 4 h. After evaporation of the solvent, the residue was taken up in 6 mL of 5:1 methylene chloride/methyl iodide and warmed at 50 °C for 1 h. The solution was again concentrated and the residue redissolved in 3 mL of methanol containing 0.5 g of ammonium acetate. The mixture was heated at 50°C for 12 h, concentrated to a volume of 1 mL, and purified by reverse phase HPLC (Rainin Microsorb 1/2" C-18). The product was eluted with a solvent gradient of 0:100 acetonitrile (0.1% trifluoroacetic acid) /water (0.1% trifluoroacetic acid), time 0 to 10 min, to 50:50 acetonitrile (0.1% trifluoroacetic acid)/water (0.1% trifluoroacetic acid), time 10 to 40 min, flow=12 ml/min, Rₜ=35.2 min, uv detection at 254 nM. Concentration of the clean fractions gave 70.0 mg (65%) of 1-methyl-4-(2-carboxyethyl)-7-(4-amidinobenzyloxy)-3,4-dihydro-1*H*-1,4-benzodiazepine-2,5-dione ethyl ester trifluoroacetate as a colorless amorphous solid. ¹H NMR (300 MHz, 1:1 D₂O/d⁶-acetone) d 7.85 (2H, d, J=7.5 Hz, HNC-Ar-H₂), 7.68 (2H, d, J=7.5 Hz, HNC-Ar-H₂), 7.40-7.25 (3H, m, O-Ar-H₃), 5.24 (2H, s, ArCH₂0), 4.07 (1H, d, J=15.1 Hz, NCHHCON), 4.04 (1H, m, NCHHCH₂), 4.02 (2H, q, J=7.3 Hz, OCH₂CH₃), 3.82 (1H, d, J=15.1 Hz, NCHHCON), 3.71 (1H, ddd, J=14.2, 5.9, 5.9 Hz, NCHHCH₂), 3.29 (3H, s, NCH₃), 2.65 (2H, m, NCH₂CH₂), 1.11 (3H, t, J=73 Hz, OCH₂CH₃); Exact mass (FAB, M+H⁺) calcd for C₂₃H₂₇N₄O₅: 439.1981; Found: 439.1977.

Using the above procedure, but substituting the appropriate 1-alkyl-4-(2-carboxyethyl)-7-(cyanobenzyl/alkyl)oxy-3,4-dihydro-1*H*-1,4-benzodiazepine-2,5-dione ethyl ester for 1-methyl-4-(2-carboxyethyl)-7-(4-cyanobenzyl)oxy-3,4-dihydro-1*H*-1,4-benzodiazepine-2,5-dione ethyl ester there may be prepared, for example, the following compounds:
1-ethyl-4-(2-carboxyethyl)-7-(4-amidinobenzyl)oxy-3,4-dihydro-1*H*-1,4-benzodiazepine-2,5-dione ethyl ester,
1-isopropyl-4-(2-carboxyethyl)-7-(4-amidinobenzyl)oxy-3,4-dihydro-1*H*-1,4-benzodiazepine-2,5-dione ethyl ester,
1-isobytyl-4-(2-caboxyethyl)-7-(4-amidinobenzyl)oxy-3,4-dihydro-1*H*-1,4-benzodiazepine-2,5-dione ethyl ester,
1-cyclohexyl-4-(2-carboxyethyl)-7-(4-amidinobenzyl)oxy-3,4-dihydro-1*H*-1,4-benzodiazepine-2,5-dione ethyl ester,
1-ethyl-4-(2-carboxyethyl)-7-(3-amidinobenzyl)oxy-3,4-dihydro-1*H*-1,4-benzodiazepine-2,5-dione ethyl ester,
1-isopropyl-4-(2-carboxyethyl)-7-(3-amidinobenzyl)oxy-3,4-dihydro-1*H*-1,4-benzodiazepine-2,5-dione ethyl ester,
1-isobytyl-4-(2-carboxyethyl)-7-(3-amidinobenzyl)oxy-3,4-dihydro-1*H*-1,4-benzodiazepine-2,5-dione ethyl ester,
1-cyclohexyl-4-(2-carboxyethyl)-7-(3-amidinobenzyl)oxy-3,4-dihydro-1*H*-1,4-benzodiazepine-2,5-dione ethyl ester,
1-ethyl-4-(2-carboxyethyl)-7-(4-amidinobutyl)oxy-3,4-dihydro-1*H*-1,4-benzodiazepine-2,5-dione ethyl ester,
1-isopropyl-4-(2-carboxyethyl)-7-(4-amidinobutyl)oxy-3,4-dihydro-1*H*-1,4-benzodiazepine-2,5-dione ethyl ester,
1-isobytyl-4-(2-caboxyethyl)-7-(4-amidinobutyl)oxy-3,4-dihydro-1*H*-1,4-benzodiazepine-2,5-dione ethyl ester,
1-cyclohexyl-4-(2-carboxyethyl)-7-(4-amidinobutyl)oxy-3,4-(dihydro-1*H*-1,4-benzodiazepine-2,5-dione ethyl ester.

d) To a stirred, 0°C solution of 37.3 mg (0.0690 mmol) of 1-methyl-4-(2-carboxyethyl)-7-(4-amidinobenzyloxy)-3,4-dihydro-1*H*-1,4-benzodiazepine-2,5-dione ethyl ester trifluoroacetate in 1 mL of tetrahydrofuran was added 0.28 mL of 1 N aqueous sodium hydroxide and the mixture warmed to ambient temperature over 1 h. The reaction was acidified with 50 mL of acetic acid, concentrated in vacuo, and purified by reverse phase HPLC (Rainin Microsorb 1/2" C-18). The product was eluted with a solvent gradient of 0:100 acetonitrile (0.1% trifluoroacetic acid) /water (0.1% trifluoroacetic acid), time 0 to 10 min, to 50:50 acetonitrile (0.1% trifluoroacetic acid)/water (0.1% trifluoroacetic acid), time 10 to 40 min,flow=12 ml/min, Rₜ=32.4 min, uv detection at 254 nM. Concentration of the clean fractions gave 26.6 mg (75%) of 1-methyl-4-(2-carboxyethyl)-7-(4-amidinobenzyloxy)-3,4-dihydro-1*H*-1,4-benzodiazepine-2,5-dione trifluoroacetate as a colorless amorphous solid. ¹H NMR (300 MHz, 1:1 D₂O/d⁶-acetone) d 7.75 (2H, d, J=8.3 Hz, HNC-Ar-H₂), 7.60 (2H, d, J=8.3 Hz, HNC-Ar-H₂), 7.32-7.18 (3H, m, O-Ar-H₃), 5.16 (2H, s, ArCH₂O), 4.01 (1H, d, J=15.1 Hz, NCHHCON), 4.04 (1H, ddd, J=14.2, 7.3, 7.3 Hz, NCHHCH₂), 3.77 (1H, d, J=15.1 Hz, NCHHCON), 3.67 (1H, ddd, J=14.2, 5.8, 5.8 Hz, NCHHCH₂), 3.20 (3H, s, NCH₃), 2.59 (2H, m, NCH₂CH₂); Exact mass (FAB, M+H⁺) calcd for C₂₁H₂₃ N₄O₅: 411.1668; Found: 411.1701.

Using the above procedure, but substituting the appropriate 1-alkyl-4-(2-carboxyethyl)-7-(amidinobenzyl/alkyl)oxy-3,4-dihydro-1*H*-1,4-benzodiazepine-2,5-dione ethyl ester for 1-methyl-4-(2-carboxyethyl)-7-(4-amidinobenzyl)oxy-3,4-dihydro-1*H*-1,4-benzodiazepine-2,5-dione ethyl ester there may be prepared, for example, the following compounds:
1-ethyl-4-(2-carboxyethyl)-7-(4-amidinobenzyl)oxy-3,4-dihydro-1*H*-1,4-benzodiazepine-2,5-dione trifluoracetate,
1-isopropyl-4-(2-carboxyethyl)-7-(4-amidinobenzyl)oxy-3,4-dihydro-1*H*-1,4-benzodiazepine-2,5-dione trifluoracetate,
1-isobytyl-4-(2-carboxyethyl)-7-(4-amidinobenzyl)oxy-3,4-dihydro-1*H*-1,4-benzodiazepine-2,5-dione trifluoracetate,
1-cyclohexyl-4-(2-carboxyethyl)-7-(4-amidinobenzyl)oxy-3,4-dihydro-1*H*-1,4-benzodiazepine-2,5-dione trifluoracetate,
1-ethyl-4-(2-carboxyethyl)-7-(3-amidonobenzyl)oxy-3,4-dihydro-1*H*-1,4-benzodiazepine-2,5-dione trifluoracetate,
1-isopropyl-4-(2-carboxyethyl)-7-(3-amidinobenzyl)oxy-3,4-dihydro-1*H*-1,4-benzodiazepine-2,5-dione trifluoracetate,
1-isobytyl-4-(2-carboxyethyl)-7-(3-amidinobenzyl)oxy-3,4-dihydro-1*H*-1,4-benzodiazepine-2,5-dione trifluoracetate,
1-cyclohexyl-4-(2-carboxyethyl)-7-(3-amidinobenzyl)oxy-3,4-dihydro-1*H*-1,4-benzodiazepine-2,5-dione trifluoracetate,
1-ethyl-4-(2-carboxyethyl)-7-(4-amidinobutyl)oxy-3,4-dihydro-1*H*-1,4-benzodiazepine-2,5-dione trifluoracetate,
1-isopropyl-4-(2-carboxyethyl)-7-(4-amidinobutyl)oxy-3,4-dihydro-1*H*-1,4-benzodiazepine-2,5-dione trifluoracetate,
1-isobytyl-4-(2-carboxyethyl)-7-(4-amidinobutyl)oxy-3,4-dihydro-1*H*-1,4-benzodiazepine-2,5-dione trifluoracetate,
1-cyclohexyl-4-(2-carboxyethyl)-7-(4-amidinobutyl)oxy-3,4-dihydro-1*H*-1,4-benzodiazepine-2,5-dione trifluoracetate.

### Example 27

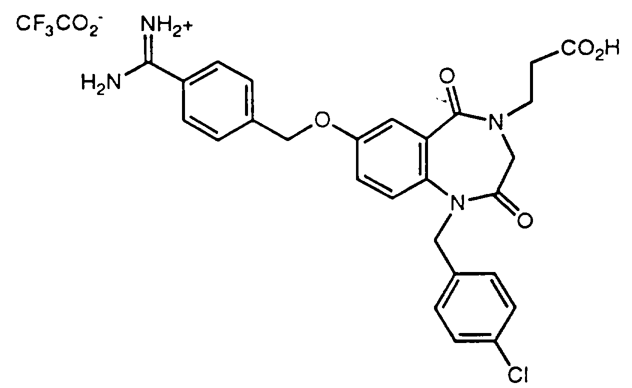

*1-(4-chlorobenzyl)-4-(2-carboxyethyl)7-(4-amidinobenzyloxy)-3,4-dihydro-1H-1,4-benzodiazepine-2,5-dione trifluoroacetate.*

a) To a stirred solution of 10.0g (59.8 mmol) of 5-hydroxy-2-nitrobenzaldehyde and 12.3 g (62.8 mmol) of a-bromo-p-tolynitrile in 50 ml of dry dimethyl-formamide was added 9.10 g (65.8 g) of anhydrous potassium carbonate. The mixture was warmed to 50°C for 2 h and partitioned between water and ethyl acetate. The organic phase was washed with 1 N sodium bicarbonate, water, brine, and dried over anhydrous magnesium sulfate. Concentration gave 13.8 g (82%) of 5-(4-cyanobenzyloxy)-2-nitrobenzaldehyde. ¹H NMR (300 MHz, d⁶-acetone) d 10.39 (1H, s, Ar-CHO), 8.23 (1H, d, J=9.0 Hz, O-Ar-H), 7.85 (2H, d, J=8.3 Hz, NC-Ar-H₂), 7.77 (2H, d, J=8.3 Hz, NC-Ar-H₂), 7.48 (1H, dd, J=9.0, 2.9 Hz, O-Ar-H), 7.43 (1H, d, J=2.9 Hz, O-Ar-H), 5.50 (2H, s, ArCH₂O).

b) Potassium permanganate (6,18 g, 39.1 mmol) was added in portions to a stirred solution of 13.8 g (48.9 mmol) of 5-(4-cyanobenzyloxy)-2-nitrobenzaldehyde and 1.58 g (4.89 mmol) of tetrabutylammonium bromide in 70 mL of pyridine. After 1 h, the solvent was removed in vacuo and the residue was partitioned between ethyl acetate and water. Sodium hydrogen sulfite was added to destroy excess permanganate and the aqueous phase was separated and extracted with ethyl acetate. The combined organics were washed with 1 N sodium hydrogen sulfate, brine, dried over anhydrous magnesium sulfate, and concentrated to give 14.6 g of 5-(4-cyanobenzyloxy)-2-nitrobenzoic acid as a powder, used without further purification. ¹H NMR (300 MHz, d⁶-acetone) d 8.07 (1H, d, J=8.8 Hz, O-Ar-H), 7.86 (2H, d, J=8.3 Hz, NC-Ar-H₂), 7.78 (2H, d, J=8.3 Hz, NC-Ar-H₂), 7.39 (1H, d, J=2.9 Hz, O-Ar-H), 7.36 (1H, dd, J=8.8, 2.9 Hz, O-Ar-H), 5.48 (2H, s, ArCH₂O).

c) Oxalyl chloride (5.70 mL, 65.4 mmol) was added slowly to a rapidly stirred suspension of 14.6 g (48.9 mmol) of 5-(4-cyanobenzyloxy)-2-nitrobenzoic acid and 2 mL of dimethylformamide in 100 mL of benzene. When gas evolution ceased, the solution was heated briefly to 60 °C, concentrated in vacuo, and redissolved in 75 mL of tetrahydrofuran. This solution was added dropwise to a rapidly stirred suspension of 8.50 g (55.3 mmol) of b-alanine ethyl ester hydrochloride and 21.1 g (251 mmol) of sodium bicarbonate in 325 mL of 2:1 tetrahydrofuran/water at 0°C. The mixture was warmed to ambient temperature for 12 h and the tetrahydrofuran was removed in vacuo. The residue was partitioned between ethyl acetate and 1 N sodium bicarbonate and the aqueous phase was separated and extracted with ethyl acetate. The combined organics were washed with 1 N sodium hydrogen sulfate, water, brine, dried over anhydrous magnesium sulfate, and concentrated to give 17.7 g (91%) of N-(5-(4-cyanobenzyloxy)-2-nitrobenzoyl)-b-alanine ethyl ester as a tan powder. ¹H NMR (300 MHz, CDCl₃) d 8.11 (1H, d, J=8.3 Hz, O-Ar-H), 7.71 (2H, d, J=8.3 Hz, NC-Ar-H₂), 7.52 (2H, d, J=8.3 Hz, NC-Ar-H₂), 7.01 (2H, m, O-Ar-H₂), 6.50 (1H, bt, J=5.9 Hz, N-H), 5.21 (2H, s, ArCH₂O), 4.14 (2H, q, J=7.3 Hz, OCH₂CH₃), 3.69 (2H, bq, J=5.8 Hz, NCH2CH2), 2.70 (2H, t, J=5.8 Hz, NCH2CH2), 1.26 (3H, t, J=7.3 Hz, OCH₂CH₃); Exact mass (FAB, M+H⁺) calcd for C₂₀H₂₀ N₃O₆:398.1352; Found : 398.1343,

d) Tin(II)chloride(12.2 g, 64.4 mmol) was added to a solution of 5.12 g (12.9 mmol) of N-(5-(4-cyanobenzyloxy)-2-nitrobenzoyl)-b-alanine ethyl ester in 40 mL of 1:1 ethyl acetate/ethanol and the mixture heated to 70°C for 15 min, cooled, and poured into a mixture of ice water, 1 N sodium bicarbonate, and ethyl acetate. 1 N sodium hydroxide was added carefully until a clear solution resulted and the aqueous phase was separated and extracted with ethyl acetate. The combined organics were washed with water, brine, dried over anhydrous magnesium sulfate, and concentrated to give a dark solid that was purified by chromatography (silica gel-60, 1:1 ethyl acetate/hexane) to give 2.51 g (53%) of N-(5-(4-cyanobenzyloxy)-2-aminobenzoyl)-b-alanine ethyl ester as a light yellow solid. ¹H NMR (300 MHz, CDCl₃) d 7.67 (2H, d, J=8.3 Hz, NC-Ar-H₂), 7.52 (2H, d, J=8.3 Hz, NC-Ar-H₂), 6.94 (1H, d, J=2.9 Hz, O-Ar-H), 6.89 (1H, dd, J=2.9, 8.8 Hz, O-Ar-H), 6.78 (1H, b, N-H), 6.66 (1H, d, J=8.8 Hz, O-Ar-H), 5.04 (2H, s, ArCH₂O), 4.16 (2H,q, J=7.3 Hz, OCH₂CH₃), 3.66 (2H, bq, J=5.8 Hz, NHCH₂CH₂), 2.62 (2H, t, J=5.8 Hz, NHCH₂CH₂), 1.27 (3H, t, J=73 Hz, OCH₂CH₃); Exact mass (FAB, M+H⁺) calcd for C₂₀H₂₂N₃O₄: 368.1610; Found: 368.1603.

e) A solution of 1.03 g (2.80 mmol) of N-(5-(4-cyanobenzyloxy)-2-aminobenzoyl)-b-alanine ethyl ester, 394 mg (2.80 mmol) of p-chlorobenzaldehyde, and 20 mg of p-toluenesulfonic acid in 30 mL of toluene was heated at reflux through a Dean-Stark trap for 45 min. The solvent was removed in vacuo and the residue was dissolved in 15 mL of trifluoroacetic acid. Triethylsilane (1.34 mL, 8.40 mmol) was added and the mixture was stirred at room temperature for 12 h, concentrated in vacuo, and partitioned between 1 N aqueous sodium bicarbonate and ethyl acetate. The organic phase was washed with water, brine, dried over anhydrous magnesium sulfate, and concentrated. Chromatography (silica gel-60, 2:3 ethyl acetate/hexane) gave 820 mg (59%) of N-(5-(4-cyanobenzyloxy)-2-(4-chlorobenzylamino)benzoyl)-b-alanine ethyl ester as a colorless solid. ¹H NMR (300 MHz, CDCl₃) d 7.66 (2H, d, J=7.8 Hz, NC-Ar-H₂), 7.51 (2H, d, J=7.8 Hz, NC-Ar-H₂), 7.27 (4H, s, ClArH₄), 6.98 (1H, d, J=2.0 Hz, O-Ar-H), 6.89 (1H, dd, J=2.0, 9.3 Hz, O-Ar-H), 6.78 (1H, b, N-H), 6.66 (1H, d, J=9.3 Hz, O-Ar-H), 5.03 (2H, s, ArCH₂O), 4.32 (2H, s, ClArCH₂), 4.17 (2H, q, J=7.3 Hz, OCH₂CH₃), 3.65 (2H, bq, J=5.7 Hz, NHCH₂CH₂), 2.62 (2H, t, J=5.7 Hz, NHCH₂CH₂), 1.28 (3H, t, J=7.3 Hz, OCH₂CH₃); Exact mass (FAB, M+H⁺) calcd for C₂₇H₂₇ClN₃O₄: 492.1690; Found: 492.1681.

f) To a rapidly stirred mixture 820 mg (1.67 mmol) of N-(5-(4-cyanobenzyloxy) -2-(4-chlorobenzylamino)benzoyl)-b-alanine ethyl ester in 20 mL of 3:1 methytene chloride/water at ambient temperature was added 174 mL (2.00mmol) of bromoacetyl bromide via syringe. After 6 h, an additional 1(X) mL (1.15 mmol) was added and the mixture stirred for 12 h. The aqueous phase was discarded and the organic layer washed with 1 N aqueous sodium bicarbonate, brine, dried over anhydrous magnesium sulfate, and concentrated. The residue was dissolved in 5 mL of dimethylformamide, 1.08g (3.33 mmol) of cesium carbonate was added, and the mixture warmed briefly to 60°C and allowed to stir at ambient temperature for 12 h. The resulting suspension was partitioned between ethyl acetate and water and the organic phase was separated and washed with water, brine, dried over anhydrous magnesium sulfate, and concentrated. Chromatography (silica gel-60, 1:1 ethyl acetate/hexane) gave 574 mg (65%) of 1-(4-chlorobenzyl)-4-(2-carboxyethyl)-7-(4-cyanobenzyloxy)-3,4-dihydro-1*H*-1,4-benzodiazepine-2,5-dione ethyl ester as a colorless foam. ¹H NMR (300 MHz, CDCl₃) d 7.68 (2H, d, J=8.3 Hz, NC-Ar-H₂), 7.51 (2H, d, J=8.3 Hz, NC-Ar-H₂), 7.35 (1H, d, J=3.0 Hz, O-Ar-H), 7.24 (2H, d, J=9.7 Hz, ClArH₂), 7.10 (1H, d, J=8.8 Hz, O-Ar-H), 7.07 (2H, d, J=9.7 Hz, ClArH₂), 7.01 (1H, dd, J=3.0, 8.8 Hz, O-Ar-H), 5.15 (1H, d, J=13.2 Hz, ArCHHO), 5.10 (1H, d, J=13.2 Hz, ArCHHO), 5.08 (1H, d, J=15.6 Hz, ClArCH₂), 5.91 (1H, d, J=15.6 Hz, ClArCH₂), 4,14 (2H, q, J=7.3 Hz, OCH₂CH₃), 4.13 (1H, d, J=14.7 Hz, NCHHCON), 4.00 (1H, m, NCHHCH₂), 3,89 (1H, d, J=14,7 Hz, NCHHCON), 3.88 (NCHHCH₂), 2.68 (2H, m, NHCH₂CH₂), 1.26 (3H, t, J=73 Hz, OCH₂CH₃); Exact mass (FAB, M+H⁺) calcd for C₂₉H₂₇ClN₃O₅: 532.1639; Found: 532.1628.

g) Hydrogen sulfide gas was bubbled into a solution of 295 mg (0.555 mmol) of 1-(4-chlorobenzyl)-4-(2-carboxyethyl)-7-(4-cyanobenzyloxy)-3,4-dihydro-1*H*-1,4-benzodiazepine-2,5-dione ethyl ester in 4 mL of 1:1 pyridine/triethylamine for 15 min and the mixture was warmed in a 50 °C oil bath for 12 h. After evaporation of the solvent, the residue was taken up in 4 mL of 3:1 methylene chloride/methyl iodide and heated at reflux for 1 h. The solution was again concentrated and the residue redissolved in 3 mL of methanol containing 0.5 g of ammonium acetate. The mixture was heated at 50°C for 4 h, concentrated to a volume of 1 mL, and purified by reverse phase HPLC (Rainin Microsorb 1/2" C-18). The product was eluted with a solvent gradient of 0.100 acetonitrile (0.1% trifluoroacetic acid) /water (0.1% trifluoroacetic acid) to 50:50 acetonitrile (0.1% trifluoroacetic acid)/water (0.1% trifluoroacetic acid), time 0 to 30 min, flow=12 ml/min, Rₜ=39,9 min, uv detection at 230 nM. Concentration of the clean fractions gave 215 mg (60%) of 1-(4-chlorobenzyl)-4-(2-carboxyethyl)-7-(4-amidinobenzyloxy)-3,4-dihydro-1*H*-1,4-benzodiazepine-2,5-dione ethyl ester trifluoroacetate as a colorless amorphous solid. ¹H NMR (300 MHz, d⁶-acetone) d 8.60 (1H, b, NH), 7.95 (2H, d, J=8.3 Hz, HNC-Ar-H₂), 7.73 (2H, d, J=8.3 Hz, HNC-Ar-H₂), 7.40 (1H, d, J=9.3 Hz, O-Ar-H), 7.32-7.16 (6H, m, O-Ar-H, ArH₄Cl), 5.34 (1H, d, J=16.1 Hz, NCHHAr), 5.27 (2H, s, ArCH₂O), 4.94 (1H, d, J=16.1 Hz, NCHHAr), 4.24 (1H, d, J=14.7 Hz, NCHHCON), 4.11 (2H, q, J=7.3 Hz, OCH₂CH₃), 4.14 (1H, m, NCHHCH₂), 3.94 (1H, d, J=14.7 Hz, NCHHCON), 3.80 (1H, m, NCHHCH₂), 2.68 (2H, bt, J=6.8 Hz, NCH₂CH₂), 1.22 (3H, t, J=7.3 Hz, OCH₂CH₃); Exact mass (FAB, M+H⁺) calcd for C₂₉H₃₀ClN₄O₅: 549.1905; Found: 549.1912.

h) To a stirred, 0 °C solution of 104 mg (0.160 mmol) of 1-(4-chlorobenzyl)-4-(2-carboxyethyl)-7-(4-amidinobenzyloxy)-3,4-dihydro-1*H*-1,4-benzodiazepine-2,5-dione ethyl ester trifluoroacetate in 1,5 mL of tetrahydrofuran was added 0.48 mL of 1 N aqueous sodium hydroxide and the mixture warmed to ambient temperature over 2 h. The reaction was acidified with 0.10 mL of acetic acid, concentrated in vacuo, and purified by reverse phase HPLC (Rainin Microsorb 1/2'' C-18). The product was eluted with a solvent gradient of 0:100 acetonitrile (0.1% trifluoroacetic acid) /water (0.1% trifluoroacetic acid) to 50:50 acetonitrile (0.1% trifluoroacetic acid)/water (0.1% trifluoroacetic acid), time 0 to 30 min, flow=12 ml/min, Rₜ=29.1 min, uv detection at 230 nM. Lyophilization of the clean fractions gave 90.0 mg (90%) of 1-(4-chlorobenzyl)-4-(2-carboxyethyl)-7-(4-amidinobenzyl)-3,4-dihydro-1*H*-1,4-benzodiazepine-2,5-dione trifluoroacetate as a colorless powder. ¹H NMR (300 MHz, d⁶-acetone) d 10.80 (1H,b, NH), 8,70 (1H, b, NH), 7,94 (2H, d, J=8.8 Hz, HNC-Ar-H₂), 7.71 (2H, d, J=8.8 Hz, HNC-Ar-H₂), 7.41 (1H, d, J=8.8 Hz, O-Ar-H), 7.32-7.16 (6H, m, O-Ar-H, ArH₄Cl), 5.36 (1H, d, l=15.6 Hz, NCHHAr), 5.24 (2H, s, ArCH₂O), 4.92 (1H, d, J=15.6 Hz, NCHHAr), 4.23 (1H, d, J=14.7 Hz, NCHHCON), 4.03 (1H, m, NCHHCH₂), 3.98 (1H, d, J=14.7 Hz, NCHHCON), 3.81 (1H, m, NCHHCH₂), 2.71 (2H, bt, J=6.8 Hz, NCH₂CH₂); Exact mass (FAB, M+H⁺) calcd for C₂₇H₂₆Cl N₄O₅: 521,1592; Found: 521.1581.

### Example 28

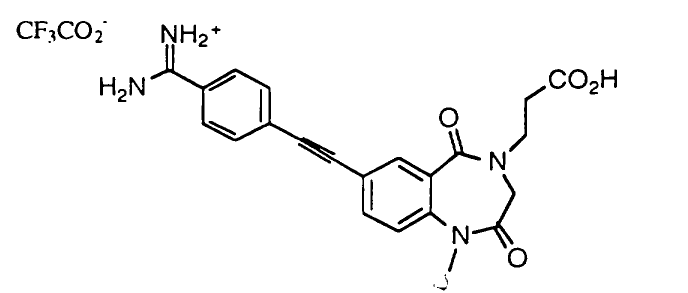

*1-(methyl)-4-(2-carboxyethyl)-7-(4-amidinophenyl)ethynyl-3,4-dihydro-1H-1,4-benzodiazepine-2,5-dione trifluoracetate.*

a) 1-methyl-4-(2-carboxyethyl)-7-(4-amidinophenyl)ethynyl-3,4-dihydro-1*H*-1,4-benzodiazepine-2,5-dione ethyl ester was prepared using the method employed in part (h) of example 19. Thus, from 0.242 grams of 1-methyl-4-(2-carboxyethyl)-7-(4-cyanophenyl)ethynyl-3,4-dihydro-1*H*-1,4-benzodiazepine-2,5,dione ethyl ester was prepared 0.145 grams (56%) of 1-methyl-4-(2-carboxyethyl)-7-(4-amidinophenyl)ethynyl-3,4-dihydro-1*H*-1,4-benzodiazepine-2,5-dione ethyl ester. The product was purified by reverse phase HPLC (Column: Dynamax 60A C18, Method: 30:70 acetonitrile (0.1% trifluoroacetic acid) /water (0.1% trifluoroacetic acid), time 0 to 10 min, 30:70 acetonitrile (0.1% trifluoroacetic acid) /water (0.1% trifluoroacetic acid) to 70:30 acetonitrile (0.1% trifluoroacetic acid)/water (0.1% trifluoroacetic acid), time 10-40 min, 70:30 acetonitrile (0.1% trifluoroacetic acid)/ water (0.1% trifluoroacetic acid), time 40-55min, flow=10 ml/min, Rₜ=28.9 min, uv detection at 254 nM). ¹H NMR (300 MHz, D₂O) d 7.89 (1H, d, 3J_{HH}=2Hz, C6-H), 7.80 (5H, m, C8-H, o,m-ArH), 7.46 (1H, d, 3J_{HH}=9 Hz, C9-H), 4.17 (1H, d, J=15Hz, C3-H), 4.06 (1H, dt, 2J_{HH}=14Hz, 3J_{HH}=7Hz, NCHHCH₂CO₂), 3.92 (1H, d, J=15 Hz, C3-H), 3.77 (1H, dt, 2J_{HH}=14Hz, 3J_{HH}=7Hz, NCHHCH₂CO₂), 3.37 (3H, s, NCH₃), 2.71 (2H, m, NCH₂CH₂CO₂); Mass spectrum (FAB, M+H⁺) calcd for C₂₄H₂₅N₄O₄:433.2; Found: 431.2.

Using the above procedure, but substituting the appropriate 4-(2-carboxyethyl)-3,4-dihydro-1*H*-1,4-benzodiazepine-2,5-dione ethyl ester for 1-methyl-4-(2-carboxyethyl)-7-(4-cyanophenyl)ethynyl-3,4-dihydro-1*H*-1,4-benzodiazepine-2,5-dione ethyl ester there was prepared, for example, the following compounds:
1-methyl-4-(2-carboxyethyl)-7-(3-amidinophenyl)ethynyl-3,4-dihydro-1*H*-1,4-benzodiazepine-2,5,dione ethyl ester
1-ethyl-4-(2-carboxyethyl)-7-(4-amidinophenyl)ethynyl-3,4-dihydro-1*H*-1,4-benzodiazepine-2,5,dione ethyl ester
1-isopropyl-4-(2-carboxyethyl)-7-(4-amidinophenyl)ethynyl-3,4-dihydro-1*H*-1,4-benzodiazepine-2,5,dione ethyl ester
1-isobutyl-4-(2-carboxyethyl)-7-(4-amidinophenyl)ethynyl-3,4-dihydro-1*H*-1,4-benzodiazepine-2,5,dione ethyl ester
1-phenyl-4-(2-carboxyethyl)-7-(4-amidinophenyl)ethynyl-3,4-dihydro-1*H*-1,4-benzodiazepine-2,5,dione ethyl ester

b) A magnetically stirred solution of 1-methyl-4-(2-carboxyethyl)-7-(4-amidinophenyl)ethynyl-3,4-dihydro-1*H*-1,4-benzodiazepine-2,5-dione ethyl acetate (0.045 grams, 0.104 mmol) in 0.5mL methanol at room temperature was added 0.5mL 2N sodium hydroxide. After 30 mins, the reaction was quenched with 1mL acetic acid, concentrated *in vacuo,* diluted in a minimum of a 1:3 methanol:water mixture and purified by HPLC (Column: Dynamax 60A C18, Method: 10-50% MeCN/H2O 10ml/min. Detector: 254nm. Time(%MeCN): 0:00(10), 00:15(10,inj.,detect on), 10:00(10), 40:00(50), 55:00(50), 59:58(10, detect off), 60:00(off), R_{f}=32.3min) to yield 27mgs of 1-(methyl)-4-(2-carboxyethyl)-7-(4-amidinophenyl)ethynyl-3,4-dihydro-1H-1,4-benzodiazepinedione-2,5,dione trifluoracetate. Exact mass (FAB, M+H⁺) calcd for C₂₂H₂₂N₄O₄: 405.1563, found: 405.1562

Using the above procedure, but substituting the appropriate 4-(2-carboxyethyl)-3,4-dihydro-1*H*-1,4-benzodiazepine-2,5-dione ethyl ester for 1-methyl-4-(2-carboxyethyl)-7-(4-amidinophenyl)ethynyl-3,4-dihydro-1*H*-1,4-benzodiazepine-2,5,dione ethyl ester there was prepared, for example, the following compounds:
1-methyl-4-(2-carboxyethyl)-7-(3-amidinophenyl)ethynyl-3,4-dihydro-1*H*-1,4-benzodiazepine-2,5,dione trifluoracetate
1-ethyl-4-(2-carboxyethyl)-7-(4-amidinophenyl)ethynyl-3,4-dihydro-1*-H*-1,4-benzodiazepine-2,5,dione trifluoracetate
1-isopropyl-4-(2-carboxyethyl)-7-(4-amidinophenyl)ethynyl-3,4-dihydro-1*H*-1,4-benzodiazepine-2,5,dione trifluoracetate
1-isobutyl-4-(2-carboxyethyl)-7-(4-amidinophenyl)ethynyl-3,4-dihydro-1*H*-1,4-benzodiazepine-2,5,dione trifluoracetate
1-phenyl-4-(2-carboxjyethyl)-7-(4-amidinophenyl)ethynyl-3,4-dihydro-1*H*-1,4-benzodiazepine-2,5,dione trifluoracetate

### Example 29

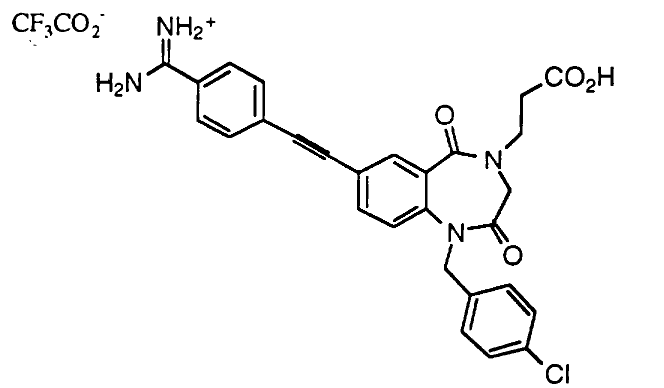

*1-(4-chlorobenzyl)-4-(2-carboxyethyl)-7-(4-amidinophenyl)ethynyl-3,4-dihydro-1H-1,4-benzodiazepine-2,5,dione trifluoracetat*e.

a) 1-(4-chlorobenzyl)-4-(2-carboxyethyl)-7-(4-cyanophenyl)ethynyl-3,4-dihydro-1*H*-1,4-benzodiazepine-2,5-dione ethyl ester was prepared using the method described in part (f) of example 1. Thus, from 0.265 grams (0.46mmol) of 1-(4-chlorobenzyl)-4-(2-carboxyethyl)-7-iodo-3,4-dihydro-1H-1,4-benzodiazepinedione ethyl ester and 0.127 grams (4-cyanophenyl)ethyne was prepared 0.296 grams of 1-(4-chlorobenzyl)-4-(2-carboxyethyl)-7-(4-cyanophenyl)etynyl-3,4-dihydro-1*H*-1,4-benzodiazepine-2,5-dione ethyl ester (99%, TLC, SiO₂, 1:1 ethyl acetate/hexane, R_{f}=0.38, uv positive). ¹H NMR (CDCl₃, dTMS) 7.99 (1H, d, ⁴J_{HH}=2Hz, C6-H), 7.63 (2H, d, ³J_{HH}=8Hz, ArH), 7.55 (2H, d, ³J_{HH}=8Hz, ArH), 7.52 (1H, dd, ⁴J_{HH}=2Hz, ³J_{HH}=8Hz, C8-H), 7.24 (2H, d, ³J_{HH}=8Hz, *o*-ArH NBn), 7.14 (1H, d, ³J_{HH}=8Hz, C9-H);7.02 (2H, d, ³J_{HH}=8Hz, *m*-ArH NBn) 5.09 (1H, d, J_{HH}=15Hz, NCHHAr), 4.98 (1H, d, J_{HH}=15Hz, NCHHAr), 4.12 (4H, m, OCH2, C3-H, NCHHCH2CO2), 3.95 (1H, d, J_{HH}=15Hz, C3-H), 3.92 (1H, m, NCHHCH2CO2), 2.68 (2H, m, CH2CO2), 1.24 (3H, t, ³J_{HH}=7Hz, CH3)

b) 1-(4-chlorobenzyl)-4-(2-carboxyethyl)-7-(4-amidinophenyl)etynyl-3,4-dihydro-1*H*-1,4-benzodiazepine-2,5-dione ethyl ester was prepared using the method described in part (h) of example 19. Thus, from 0.15 grams (0.286mmol) of 1-(4-chlorobenzyl)-4-(2-carboxyethyl)-7-(4-cyanophenyl)etynyl-3,4-dihydro-1*H*-1,4-benzodiazepinedione ethyl ester was prepared 0.03 grams (20%) of 1-(4-chlorobenzyl)-4-(2-carboxyethyl)-7-(4-amidinophenyl)etynyl-3,4-dihydro-1*H*-1,4-benzodiazepine-2,5-dione ethyl ester purified by HPLC (30-70% MeOH/H2O 10ml/min. Detector: 254nm. Time(%MeOH):0:00(30), 00:15(30,inj.,detect on), 10:00(30), 40:00(70), 55:00(70), 59:58(30, detect off), 60:00(off), R_{f}=38.0min), ¹H NMR (CDCl₃, dTMS) 7.99 (1H, d, ⁴J_{HH}=2Hz, C6-H), 7.74 (2H, d, ³J_{HH}=8Hz, ArH), 7.63 (2H, d, ³J_{HH}=8Hz, ArH), 7.54 (1H, dd, ⁴J_{HH}=2Hz, ³ J_{HH}=8Hz, C8-H), 7.26 (2H, d, ³J_{HH}=8Hz, *o*-ArH NBn), 7.16 (1H, d, ³J_{HH}=8Hz, C9-H), 7.07 (2H, d, ³J_{HH}=8Hz, *m*-ArH NBn) 5.10 (1H, d, J_{HH}=16Hz, NCHHAr), 4.98 (1H, d, J_{HH}=16Hz, NCHHAr), 4.13 (4H, m, OCH₂, C3-H, NCHHCH₂CO₂), 3.95 (2H, m, C3-H, NCHHCH₂CO₂), 2.68 (2H, m, CH₂CO₂), 1.25 (3H, t, ³J_{HH}=7Hz, CH3)

c) 1-(4-chlorobenzyl)-4-(2-carboxyethyl)-7-(4-amidinophenyl)etynyl-3,4-dihydro-1*H*-1,4-benzodiazepine-2,5-dione trifluoracetate was prepared using the method described in part (b) of example 27. Thus, 30mgs of 1-(4-chlorobenzyl)-4-(2-carboxyethyl)-7-(4-amidinophenyl)etynyl-3,4-dihydro-1*H*-1,4-benzodiazepinedione ethyl ester was saponified to yield 20mgs of 1-(4-chlorobenzyl)-4-(2-carboxyethyl)-7-(4-amidinophenyl)etynyl-3,4-dihydro-1*H*-1,4-benzodiazepine-2,5-dione trifluoracetate purified by HPLC (Column: Dynamax 60A C18, Method: 10-50% MeCN/H2O 10ml/min. Detector: 254nm. Time(%MeCN): 0:00(10), 00:15(10,inj.,detect on), 10:00(10), 40:00(50), 55:00(50), 59:58(10, detect off), 60:00(off), R_{f}=38.3min). ¹HNMR (D₂O) 7.25 (6H, m, C6-H, ArH, C8-H), 6.98 (1H, d, ³J_{HH}=8Hz, C9-H), 6,72 (2H, d, ³J_{HH}=8Hz, *o*-ArH NBn), 6.57 (2H, d, ³J_{HH}=8Hz, *m*-ArH NBn) 4.92 (1H, d, J_{HH}=16Hz, NCHHAr), NCHHAr overlaps with HOD, 3.70 (1H, d, J_{HH}=15Hz, C3-H), 3.54 (1H, m, NCHHCH₂CO₂), 3.43 (1H, d, J_{HH}=15Hz, C3-H), 3.30 (1H, m, NCHHCH₂CO₂), 2.59 (2H, m, CH₂CO₂). Exact mass (FAB, M+H⁺) calcd for C₂₈H₂₄N₄O_{4Cl}:515.1514, found: 515.1486

### Example 30

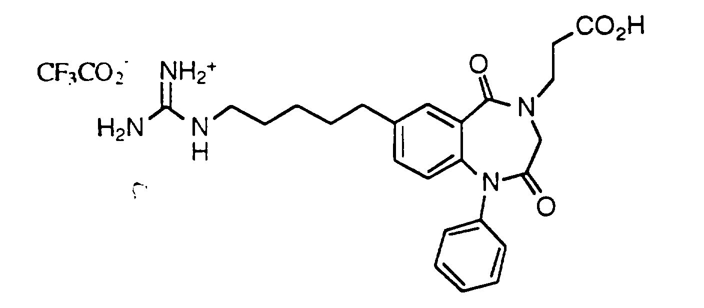

*1-phenyl-4-(2-carboxyethyl)-7-(5-guanidinophenyl)-3,4-dihydro-1H-1,4-benzodiazepine-2,5-dione trifluoracetate.*

a) A solution of 2-chloro-5-nitrobenzoic acid (5,24 grams, 0.026mol), aniline (0.166mol, 155 grams), potassium carbonate (4.1 grams) and 0.1 grams copper oxide is heated refluxed for 2 hrs. The reaction mixture was allowed to cool to room temperature and diluted with water (100mL), and extracted with 1X50mL ether. The aqueous layer was made acidic by the addition of cone. HCl and the mixture extracted 3X50mL ethyl acetate. The combined organics were dried over magnesium sulphate, filtered and concentrated *in vacuo.* The resulting residue, N-phenyl-5-nitroanthranilic acid (0.8 grams, 12%), was used without further purification. ¹H NMR (D₂O, CD₃OD) 8.84 (1H, d, ⁴J_{HH}=2Hz, C6-H), 8.15 (1H, dd, J_{HH}=2Hz, ³J_{HH}=8Hz, C4-H), 7.42 (2H, d, *m*-NPh-H), 7.31 (3H, m,*o,p*-NPh-H), 7.15 (1H, d, ³J_{HH}=8Hz, C3-H).

Using the above method, but substituting the appropriate substituted aniline for aniline there may be prepared, for example, the following compounds: N-(4-methoxy)phenyl-5-nitroanthranilic acid, N-(4-chloro)phenyl-5-nitroanthranilic acid, N-(3-trifluromethyl)phenyl-5-nitroanthranilic acid.

b) To a solution of N-phenyl-5-nitroanthranilic acid (0.8 grams, 3.1mmol), in 10mL methylene chloride and two drops dimethylformamide was added oxalyl chloride (0.3mL, 3.4mmol), and the reaction stirred for 30 mins. The mixture was concentrated in vacuo, diluted with 15mL methylene chloride cooled to 0°C and allowed to react with b-alanine ethyl ester (3.4mmol, 0.522grams) and triethylamine (3.5mmol, 0.49mL). After 1 hour, the reaction was concentrated in vacuo and the resulting residue purified by column chromatography on SiO2 using 25% ethyl acetate in hexane to 40% ethyl acetate in hexane (TLC, 1:2 ethyl acetate/hexane R_{f}=0.31, uv positive) to yield 1 gram (90%) of N-(N-phenyl-5-nitroanthranoyl)-b-alanine ethyl ester. ¹H NMR (CDCl₃, dTMS) 10.38 (1H, bs, CO₂H), 8.40 (1H, d, ⁴J_{HH}=2Hz, C6-H anthranoyl), 8.08 (1H, dd, J_{HH}=2Hz, ³J_{HH}=9Hz, C4-H anthranoyl), 7.41 (2H, t, ³J_{HH}=8Hz, *m*-NPh-H), 7.24 (3H, m, *o,p*-NPh-H), 7.15 (1H, d, ³J_{HH}=9Hz, C3-H anthranoyl), 7.10 (1H, bt, ³J_{HH}=5Hz, C(O)NH), 4.21 (2H, q, ³J_{HH}=7Hz, OCH₂), 3.75 (2H, q, ³J_{HH}=6Hz, NCH₂CH₂CO₂), 2.71 (2H, t, ³J_{HH}=6Hz, CH₂CO₂), 1.31 (3H, t, ³J_{HH}=7Hz, CH3); ¹³C NMR (CDCl₃, dTMS) 172.5, 167.9, 151.9, 138.7, 137.1, 129.7, 128.1, 125.5, 124.6, 123,6, 114.8, 113,2, 61,1, 35.5, 33.7, 14,2.

Using the above method, but substituting the appropriate substituted N-aryl-5-nitroanthranilic acid for N-phenyl-5-nitroanthranilic acid there may be prepared, for example, the following compounds:
N-(N-(4-methoxy)phenyl-5-nitroanthranoyl)-b-alanine ethyl ester, N-(N-(4-chloro)phenyl-5-nitroanthranoyl)-b-alanine ethyl ester, N-(N-(4-trifluromethyl)phenyl-5-nitroanthranoyl)-b-alanine ethyl ester.

c) A solution of 0.8 grams (2.3mmol) of N-(N-phenyl-5-nitroanthranoyl)-b-alanine ethyl ester in 5mL ethanol, 2.5mL 1N HCl, and 0.1 grams 10% palladium on carbon was placed under an atmosphere of hydrogen and stirred for 2 hrs. The mixture was filtered through Celite® washing with ethanol water mixture. The filtrate was concentrated *in vacuo* and the resulting residue diluted with 10mL water. The mixture was cooled to 0°C and allowed to react with sodium nitrite (158mgs, 2.3mmol) dissolved in 1mL water. After 2 hrs potassium iodide (23 grams) in 3mL water was added and the reaction warmed to 50°C for 0.5 hrs. The cooled mixture was diluted with sat sodium bicarbonate and extracted 3X50mL ethyl acetate. The combined organics were dried over magnesium suphate, filtered, concentrated *in vacuo.* and chromatographed on SiO2, using 20% ethyl acetate in hexane to 60% ethyl acetate and hexane (TLC, 25%ethyl acetate/hexane, R_{f}=0.52 uv positive) to give 0.18 grams (18%) of N-(N-phenyl-5-iodoanthranoyl)-b-alanine ethyl ester. ¹H NMR (CDCl₃, dTMS) 9.31 (1H, bs, NH), 7.66 (1H, d, ⁴J_{HH}=2Hz, C6-H anthranoyl), 7.48 (1H, dd, J_{HH}=2Hz, ³J_{HH}=8Hz, C4-H anthranoyl), 7.32 (2H, m, NPh-H), 7.17 (2H, m, NPh-H), 7.10 (1H, d, ³J_{HH}=9Hz, C3-H anthranoyl), 7.04 (1H, m, NPh-H), 6.85 (1H, bt, ³J_{HH}=5Hz, C(O)NH), 4.20 (2H, q, ³J_{HH}=7Hz, OCH₂), 3.69 (2H, q, ³J_{HH}=6Hz, NCH₂CH₂CO₂), 2.65 (2H, t, ³J_{HH}=6Hz, CH₂CO₂), 1.29 (3H, t, ³J_{HH}=7Hz, CH3); ¹³C NMR (CDCl₃, dTMS) 172.7, 168.0, 151.2, 140.7, 140.5, 135.9, 129.4, 123.0, 121.2, 120.2, 117.2, 77.9, 60.9, 35.3, 33.9, 14.2.

Using the above method, but substituting the appropriate substituted N-(N-aryl-5-nitroanthranoyl)-b-alanine ethyl ester for N-(N-phenyl-5-nitroanthranoyl)-b-alanine ethyl ester there may be prepared, for example, the following compounds: N-(N-(4-methoxy)phenyl-5-iodoanthranoyl)-b-alanine ethyl ester, N-(N-(4-chloro)phenyl-5-iodoanthranoyl)-b-alanine ethyl ester, N-(N-(4-trifluromethyl)phenyl-5-iodoanthranoyl)-b-alanine ethyl ester.

d) To a magnetically stirred biphasic mixture of N-(N-phenyl-5-iodoanthranoyl)-b-alanine ethyl ester (180mgs, 0.41mmol), 10mL methylene chloride, 10mL water was added bromoacetyl bromide (0.054mL, 0.45mmol). After 2 hrs, the layers were separated. The organic layer was dried over sodium sulphate, decanted, and concentrated in vacuo. The resulting residue was dissolved in dimethylformamide (3mL) and cesium carbonate (2.5 molar excess, 1.0mmol) added. After 30 mins, the mixture was diluted with 75mL water and extracted with 3X50mL ethyl acetate. The combined organics were dried over sodium sulphate, decanted, and concentrated in vacuo. The resulting residue was further purified by column chromatography on SiO2, using 35% ethyl acetate in hexane to 65% ethyl acetate and hexane to yield 0.142 grams (72%) of 1-phenyl-4-(2-carboxyethyl)-7-iodo-3,4-dihydro-1*H*-1,4-benzodiazepine-2,5-dione ethyl ester (TLC, 1:1 ethyl acetate/hexane, R_{f}=0.29, uv positive). ¹H NMR (CDCl₃, dTMS) 8.20(1H, d, ⁴J_{HH}=2Hz, C6-H), 7.60(1H, dd, J_{HH}=2Hz, ³J_{HH}=8Hz, C8-H), 7.38 (3H, m, NPh-H), 7.20 (2H, m, NPH-H), 6.53 (1H, d, ³J_{HH}=9Hz, C9-H), 4.21 (1H, d, 2J_{HH}=15Hz, C3-H), 4.11 (2H, q, ³J_{HH}=7Hz, OCH₂), 4.01(1H, d, 2J_{HH}=15Hz, C3-H), 3.95 (2H, t, ³J_{HH}=7Hz, NCH₂CH₂CO₂), 2.72 (2H, m, CH₂CO₂), 1.24 (3H, t, ³J_{HH}=7Hz, CH3); ¹³C NMR (CDCl₃, dTMS) 171.2, 167.2, 165.6, 140.7, 140.5, 139.8, 139.3, 130.7, 129.5, 128.1, 128.0, 126.1, 89.9, 60.8, 52.6, 45.2, 32.6, 14.1.

Using the above method, but substituting the appropriate substituted N-aryl-5-iodoanthranilic acid for N-(N-phenyl-5-iodoanthranoyl)-b-alanine ethyl ester there may be prepared, for example, the following compounds :
1-(4-methoxy)phenyl-4-(2-carboxyethyl)-7-iodo-3,4-dihydro-1*H*-1,4-benzodiazepine-2,5-dione ethyl ester, 1-(4-chloro)phenyl-4-(2-carboxyethyl)-7-iodo-3,4-dihydro-1*H*-1,4-benzodiazepine-2,5-dione ethyl ester, 1-(4-trifluromethyl)phenyl-4-(2-carboxyethyl)-7-iodo-3,4-dihydro-1*H*-1,4-benzodiazepine-2,5-dione ethyl ester.

e) 1-phenyl-4-(2-carboxyethyl)-7-(N-Boc-5-amino-1-pentynyl)-3,4-dihydro-1*H*-1,4-benzodiazepine-2,5-dione ethyl ester was prepared using the method described in part (f) of example 1. Thus, 71mgs of 1-phenyl-4-(2-carboxyethyl)-7-iodo-3,4-dihydro-1H-1,4-benzodiazepinedione-2,5,dione ethyl ester and 30mgs of N-Boc-5-amino-1-pentyne yielded 61mgs (76%) of 1-phenyl-4-(2-carboxyethyl)-7-(N-Boc-5-amino-1-pentynyl)-3,4-dihydro-1*H*-1,4-benzodiazepine-2,5-dione ethyl ester. ¹H NMR (CDCl₃, dTMS) 7.90 (1H, d, ⁴J_{HH}=2Hz, C6-H), 7.37 (6H, m, C8-H, NPh-H), 6.69 (1H, d, ³J_{HH}=9Hz, C9-H), 4.72 (1H, bs, NH), 4.21 (1H, d, 2J_{HH}=15Hz, C3-H), 4.11 (2H, q, ³J_{HH}=7Hz, OCH₂), 4-00 (1H, d, J_{HH}=15Hz, C3-H), 3.96 (2H, t, ³J_{HH}=7 Hz, NCH₂CH₂CO₂), 3.25 (2H, bq, ³J_{HH}=7Hz, CH₂NH), 2.72 (2H, m, CH₂CO₂), 2.44 (2H, t, ³J_{HH}=7Hz, CCCH₂), 1.76 (2H, p, ³J_{HH}=7Hz, CH₂CH₂CH₂), 1.43 (9H, s, C(CH₃)₃), 1.23 (3H, t, ³J_{HH}=7Hz, CH3); ¹³C NMR (CDCl₃, dTMS) 171.3, 167.3, 166.4, 155.9, 140.0, 139.7, 134.5, 133.8, 129.4, 129.1, 128.1, 127.9, 124.3, 121.7, 90.0, 79.5, 60.8, 52.6, 45.1, 39.7, 32.7, 28.7, 28.4, 16.9, 14.1.

Using the above procedure, but substituting the appropriate 1-aryl-4-(2-carboxyethyl)-7-iodo-3,4-dihydro-1H-1,4-benzodiazepinedione-2,5,dione ethyl ester for 1-phenyl-4-(2-carboxyethyl)-7-iodo-3,4-dihydro-1H-1,4-benzodiazepinedione-2,5,dione ethyl inter and amino or cyano alkyne for N-Boc-5-amino-1-pentyne there may be prepared, for example, the following compounds:
1-(4-methoxy)phenyl-4-(2-carboxyethyl)-7-(N-Boc-5-amino-1-pentynyl)-3,4-dihydro-1*H*-1,4-benzodiazepine-2,5-dione ethyl ester,
1-(4-chloro)phenyl-4-(2-carboxyethyl)-7-(N-Boc-5-amino-1-pentynyl)-3,4-dihydro-1*H*-1,4-benzodiazepine-2,5-dione ethyl ester,
1-(3-triflurophenyl)phenyl-4-(2-carboxyethyl)-7-(N-Boc-5-amino-1-pentynyl)-3,4-dihydro-1*H*-1,4-benzodiazepine-2,5-dione ethyl ester
1-(4-methoxy)phenyl-4-(2-carboxyethyl)-7-(N-Boc-6-amino-1-hexynyl)-3,4-dihydro-1*H*-1,4-benzodiazepine-2,5-dione ethyl ester,
1-(4-chloro)phenyl-4-(2-carboxyethyl)-7-(N-Boc-6-amino-1-hexynyl)-3,4-dihydro-1*H*-1,4-benzodiazepine-2,5-dione ethyl ester ,
1-(3-triflurophenyl)phenyl-4-(2-carboxyethyl)-7-(N-Boc-6-amino-1-hexynyl)-3,4-dihydro-1*H*-1,4-benzodiazepine-2,5-dione ethyl ester
1-(4-methoxy)phenyl-4-(2-carboxyethyl)-7-(4-cyanophenyl)ethynyl-3,4-dihydro-1*H*-1,4-benzodiazepine-2,5-dione ethyl ester,
1-(4-chloro)phenyl-4-(2-carboxyethyl)-7-(4-cyanophenyl)ethynyl-3,4-dihydro-1*H*-1,4-benzodiazepine-2,5-dione ethyl ester,
1-(3-triflurophenyl)phenyl-4-(2-carboxyethyl)-7-(4-cyanophenyl)ethynyl-3.4-dihydro-1*H*-1,4-benzodiazepine-2,5-dione ethyl ester

f) Using the method described in part (a) of example 3 1-phenyl-4-(2-carboxyethyl)-7-(N-Boc-5-aminopentyl)-3,4-dihydro-1*H*-1,4-benzodiazepinedione-2,5,dione ethyl ester was prepared from 1-phenyl-4-(2-carboxyethyl)-7-(N-Boc-5-amino-1-pentynyl)-3,4-dihydro-1*H*-1,4-benzodiazepine-2,5-dione ethyl ester in quantitative yield. ¹H NMR (CDCl₃, dTMS) 7.67 (1H, d, ⁴J_{HH}=2Hz, C6-H), 7.40(5H, m, NPh-H), 7.13 (1H, dd, ⁴J_{HH}=2Hz, ³J_{HH}=8Hz, C8-H), 6.70 (1H, d, ³J_{HH}=8Hz, C9-H), 4.55 (1H, bs, NH), 4.24 (1H, d, 2J_{HH}=15Hz, C3-H), 4,11 (2H, q, ³J_{HH}=7Hz, OCH₂), 3.97 (2H, m, C3-H, NCH₂CH₂CO₂), 3.10 (2H, bq, ³J_{HH}=7Hz, CH₂NH), 2.72 (2H, m, CH₂CO₂), 2.60 (2H, t, ³J_{HH}=7Hz, ArCh₂), 1.62 (2H, p, 3J_{HH}=7Hz), 1.47 (2H, p, ³J_{HH}=7Hz), 1.41 (9H, s, C(CH₃)₃), 1.34 (2H, m), 1.23 (3H, t, ³J_{HH}=7Hz, CH3); ¹³C NMR (CDCl₃, dTMS) 171.3, 167.6, 167.2, 155.9, 140.4, 140.3, 138.6, 132.0, 130.1, 129.3, 128.9, 128.1, 127.6, 124.3, 60.7, 52.7, 45.1, 40.4, 34.9, 32.7, 30.6, 29.9, 28.4, 26.4, 14.1.

Using the above procedure, but substituting the appropriate 1-aryl-4-(2-carboxyethyl)-7-alkynyl-3,4-dihydro-1*H*-1,4-benzodiazepinedione-2,5,dione ethyl ester for 1-phenyl-4-(2-carboxyethyl)-7-(N-Boc-5-amino-1-pentynyl)-3,4-dihydro-1*H*-1,4-benzodiazepine-2,5-dione ethyl ester there may be prepared, for example, the following compounds:
1-(4-methoxy)phenyl-4-(2-carboxyethyl)-7-(N-Boc-5-amino-1-pentyl)-3,4-dihydro-1*H*-1,4-benzodiazepine-2,5-dione ethyl ester,
1-(4-chloro)phenyl-4-(2-carboxyethyl)-7-(N-Boc-5-amino-1-pentyl)-3,4-dihydro-1*H*-1,4-benzodiazepine-2,5-dione ethyl ester
1-(3-triflurophenyl)phenyl-4-(2-carboxyethyl)-7-(N-Boc-5-amino-1-pentyl)-3,4-dihydro-1*H*-1,4-benzodiazepine-2,5-dione ethyl ester
1-(4-methoxy)phenyl-4-(2-carboxyethyl)-7-(N-Boc-6-amino-1-hexyl)-3,4-dihydro-1*H*-1,4-benzodiazepine-2,5-dione ethyl ester,
1-(4-chloro)phenyl-4-(2-carboxyethyl)-7-(N-Boc-6-amino-1-hexyl)-3,4-dihydro-1*H*-1,4-benzodiazepine-2,5-dione ethyl ester ,
1-(3-triflurophenyl)phenyl-4-(2-carboxyethyl)-7-(N-Boc-6-amino-1-hexyl)-3,4-dihydro-1*H*-1,4-benzodiazepine-2,5-dione ethyl ester

g) To a solution of 13.3mgs (0.025mmol) 1-phenyl-4-(2-carboxyethyl)-7-(N-Boc-5-aminopentyl)-3,4-dihydro-1H-1,4-benzodiazepine-2,5-dione ethyl ester in 1mL ethyl acetate and 0.5mL triethylsilane was added 2mL sat. solution of HCl in ethyl acetate. After 30 mins, the reaction mixture was concentrated *in vacuo,* diluted in a minimum of THF and 18mL of a 30% by weight hydrogen peroxide. An equivalent of lithium hydroxide was added and the reaction stirred for 1 hrs. The reaction was quenched with sodium sufite and concentrated to yield a residue, which was diluted in a methanol water mixture and purified by HPLC (10-50% MeCN/H2O 10ml/min. Detector: 254nm. Time(%MeCN): 0:00(10), 00:15(10,inj.,detect on), 10:00(10), 40:00(50), 55:00(50), 59:58(10, detect off), 60:00(off), R_{f}=33.5min) to yield 1-phenyl-4-(2-carboxyethyl)-7-(5-amino-1-pentyl)-3,4-dihydro-1H-1,4-benzodiazepine-2.5-dione (6mgs). ¹H NMR (CDCl3, dTMS) 7.12 (1H, bs, C6-H), 6.97 (3H, m, *m,p*-NPh-H), 6.79 (1H, bd, ³J_{HH}=8Hz, C8-H), 6.74 (2H, d, ³J_{HH}=8Hz, o-NPh-H), 6.36 (1H, d, ³J_{HH}=8Hz, C9-H), 3.89 (1H, d, J_{HH}=15Hz, C3-H), 3.74 (1H, dt, J_{HH}=14Hz, ³J_{HH}=7Hz, NCHHCH2CO2), 3.47 (1H, d, J_{HH}=15Hz, C3-H), 3.21 (1H, dt, J_{HH}=14Hz, ³J_{HH}=7Hz, NCHHCH₂CO₂), 2.43 (2H, t, ³J_{HH}=7Hz, CH2NH2), 2.25 (2H, m, CH₂CO₂), 2.15 (2H, t, ³J_{HH}=7Hz, ArCH₂), 1.12 (4H, m,), 0.83 (2H, m)

Using the above procedure but substituting the appropriate 1-aryl-4-(2-carboxyethyl)-7-alkynyl-3,4-dihydro-1H-1,4-benzodiazepinedione-2,5,dione ethyl ester for 1-phenyl-4-(2-carboxyethyl)-7-(N-Boc-5-amino-1-pentynyl)-3,4-dihydro-1*H*-1,4-benzodiazepine-2,5-dione ethyl ester there may be prepared, for example, the following compounds:
1-(4-methoxy)phenyl-4-(2-carboxyethyI)-7-(N-Boc-5-amino-1-pentyl)-3,4-dihydro-1*H*-1,4-benzodiazepine-2,5-dione ethyl ester,
1-(4-chloro)phenyl-4-(2-carboxyethyl)-7-(N-Boc-5-amino-1-pentyl)-3,4-dihydro-1*H*-1,4-benzodiazepine-2,5-dione ethyl ester ,
1-(3-trifluropenyl)phenyl-4-(2-carboxyethyl)-7-(N-Boc-5-amino-1-pentyl)-3,4-dihydro-1*H*-1,4-benzodiazepine-2,5-dione ethyl ester
1-(4-methoxy)phenyl-4-(2-carboxyethyl)-7-(N-Boc-6-amino-1-hexyl)-3,4-dihydro-1*H*-1,4-benzodiazepine-2,5-dione ethyl ester,
1-(4-chloro)phenyl-4-(2-carboxyethyl)-7-(N-Boc-6-amino-1-hexyl)-3,4-dihydro-1*H*-1,4-benzodiazepine-2,5-dione ethyl ester,
1-(3-triflurophenyl)phenyl-4-(2-carboxyethyl)-7-(N-Boc-6-amino-1-hexyl)-3,4-dihydro-1*H*-1,4-benzodiazepine-2,5 ethyl ester

h) 6mgs of 1-phenyl-4-(2-carboxyethyl)-7-(5-amino-1-pentyl)-3,4-dihydro-1*H*-1,4-benzodiazepine-2,5-dione was dissolved in sat 5% potassium carbonate and an excess of formamidine sulphonic acid (15mgs). After 30 mins, the reaction was quenched with acetic acid , concentrated *in vacuo,* diluted in a minimum of a water/methanol mixture and purified by HPLC (Column: Dynamax 60A C18, Method:10-50% MeCN/H2O 10ml/min. Detector: 254nm. Time(%MeCN): 0;00(10), 00:15(10, inj. detect on), 10:00(10), 40:00(50), 55:00(50), 59:58(10, detect off), 60:00(off), R_{f}=37.1min). ¹H NMR (D₂O) 7.08 (1H, bs, ArH C6-H), 6.93 (3H, m), 6.63 (1H, bd, 3J_{HH}=4Hz), 6.21 (2H, d, ³J_{HH}=8Hz), 3,79 (1H, d, J_{HH}=15Hz, C3-H), 3.74 (1H, dt, J_{HH}=14Hz, ³J_{HH}=7Hz, NCHHCH₂CO₂), 3.42 (1H, d, J_{HH}=15Hz, C3-H), 3.19 (1H, dt, J_{HH}=14Hz, ³J_{HH}=7Hz, NCHHCH2CO2), 2.41 (2H, t, ³J_{HH}=7Hz, CH₂NH), 223 (2H, m, CH₂CO₂), 2.12 (2H, bt, ³J_{HH}=7Hz), 1.02(4Hz, m), 0.79 (2H, m) Exact mass (FAB, M+H⁺) calculated for C₂₄ H₃₀N₅O₄; 452.2298, found 452.2270.

Using the above procedure, but substituting the appropriate 1-aryl-4-(2-carboxyethyl)-7-alkyl-3,4-dihydro-1H-1,4-benzodiazepinedione-2,5,dione ethyl ester for 1-phenyl-4-(2-carboxyethyl)-7-(N-Boc-5-amino-1-pentyl)-3,4-dihydro-1*H*-1,4-benzodiazepine-2,5-dione ethyl ester there may be prepared, for example, the following compounds:
1-(4-methoxy)phenyl-4-(2-carboxyethyl)-7-(5-amino-1-pentyl)-3,4-dihydro-1*H*-1,4-benzodiazepine-2,5-dione trifluoracetate,
1-(4-chloro)phenyl-4-(2-carboxyethyl)-7-(5-amino-1-pentyl)-3,4-dihydro-1*H*-1,4-benzodiazepine-2,5-dione trifluoracetate,
1-(3-triflurophenyl)phenyl-4-(2-carboxyethyl)-7-(5-amino-1-pentyl)-3,4-dihydro-1*H*-1,4-benzodiazepine-2,5-dione trifluoracetate
1-(4-methoxy)phenyl-4-(2-carboxyethyl)-7-(6-amino)-1-hexyl)-3,4-dihydro-1*H*-1,4-benzodiazepine-2,5-dione trifluoracetate,
1-(4-chloro)phenyl-4-(2-carboxyethyl)-7-(6-amino-1-hexyl)-3,4-dihydro-1*H-*1,4-benzodiazepine-2,5-dione trifluoracetate,
1-(3-triflurophenyl)phenyl-4-(2-carboxyethyl)-7-(6-amino-1-hexyl)-3,4-dihydro-1*H*-1,4-benzodiazepine-2,5-dione trifluoracetate

### Example 31

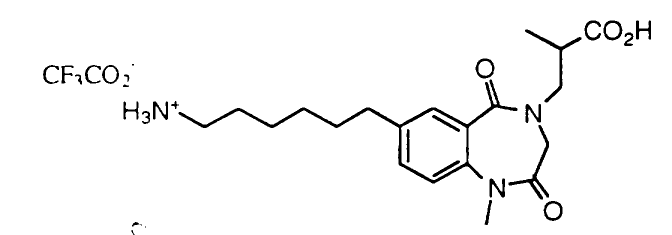

*(+)-1-methyl-4-(2-carboxy-2-methylethyl)-7-(6-amino-1-hexyl)-3,4-dihydro-1H-1,4-benzodiazepine-2,5-dione trifluoracetate*.

a) In a 1L beaker was placed 40 grams (0.267mmol) of N-methylanthranilic acid dissolved in 300mL water and 26.7mL conc. HCl cooled to 20°C. A solution of ICL, prepared by dissolving 44grams of ICl to a cooled (0°C) solution of 167mL water and 45ml conc. HCl, was rapidly added to the stirred N-methylanthranile acid solution. The reaction was allowed to stir for 2hrs, filtered on a medium glass frit funnel washing the solids with water. The green powder isolated was dried under vacuum to yield 70 grams (96%) of 5-iodo-N-methylanthranilic acid. ¹H NMR (DMSO-d₆)7.61 (1H, bs, C6-H), 7.21 (1H, bd,³J_{HH}=8Hz, C4-H), 6.19 (1H, d,³J_{HH}=8Hz,C3-H), 2.43(3H, s, NCH3); ¹³C NMR (DMSO-d₆) 169.0, 151.4, 142.7, 139.6, 114.2, 112.7, 74.2, 29.6.

b) To a mechanically stirred solution of 5-iodo-N-methylanthranilic acid (40 grams 0.144 mol), potassium carbonate (19.95 grams, 0.144mol), and 500ml water at 0°C was added phosgene (1.93M solution in toluene, 0.2mol, 104mL) over a period of 1hr. the resulting slurry was stirred for an additional 3 hrs and the solids isolated by filtration, washing with water, and a small amount (70mL) of ether. The solids were dried overnight *in vacuo* to yield 34 grams (78%) of 5-iodo-N-methylisatoic anhydride. ¹H NMR (DMSO-d₆) 8.18 (1H, bs, C6-H), 8.08 (1H, bd,³J_{HH}=8Hz, C4-H), 7.12 (1H, d,³J_{HH}=8Hz, C3-H), 3.42 (3H, s, NCH₃).

c) A solution of (±)-3-amino-2-methylpropionic acid (1.9 grams, 18.77mmol), *p*-toluenesulphonic acid, in 50mL methanol was heated to reflux for 62hrs. the reaction was allowed to cool to room temperature, concentrated *in vacuo* to yield an oil. 1/2 by volume of this oil was dissolved in 10 mL dimethylformamide and allowed to react with 5-iodo-N-methylisatoic anhydride (2.5 grams, 8.4mmol) and 7.0mL triethylamine (5-fold excess) and a catalytic amount of dimethylaminopyridine (50mgs), the reaction was heated to 50°C for 1hr. The reaction was allowed to cool to room temperature, diluted with 100mL water and extracted 3X50mL ethyl acetate. The combined organics were dried over sodium sulphate, decanted, and concentrated *in vacuo*. The resulting residue was further purified by column chromatography (SiO₂, 25%ethyl acetate in hexane to 50% ethyl acetate and hexane) to yield 1.5 grams (47%) of (±)-N-(N-methyl-2-amino-5-iodobenzoyl)-3-amino-2-methylproprionate methyl ester. ¹H NMR (CDCl₃, dTMS)7.50(1H, d, ⁴J_{HH}=2Hz, C6-H), 7.43 (1H, dd, ⁴J_{HH}=2Hz,³J_{HH}=9Hz, C4-H), 6.79 (1H, bt, NHCH2) 6.35 (1H, d, ³J_{HH}=9Hz, C3-H), 3.64 (3H, s, OCH₃),3.51 (1H, ddd, J_{HH}=14Hz, ³J_{HH}=6Hz, ³J_{HH}=5Hz, NHCHHCH), 3.40 (1H, ddd, J_{HH}=14Hz, ³J_{HH}=8Hz, ³J_{HH}=6Hz, NHCHHCH), 2.75 (4H, m, CHCO₂, NCH₃), 1.15 (3H, d, ³J_{HH}=7Hz, CHCH₃); ¹³C NMR 175.9, 168.7, 149.9, 141.0, 135.7, 117.6, 113.4, 74.2, 52.1, 42.1, 39.6, 29.7, 15.1

d) To a biphasic mixture of (±)-N-(N-methyl-2-amino-5-iodobenzoyl)-3-amino-2-methylpropionate methyl ester (2.0mmol, 0.752grams), 8mL methylene chloride, and 8mL water was added bromoacetylbromide (0.174mL, 1.5 molar equivalents) and the reaction stirred for 2hrs. The layers were separated and the organics were dried over sodium sulphate, decanted, and concentrated *in vacuo*. The resulting residue was diluted in 10mL dimethylformamide and a 3-fold excess of cesium carbonate (6.0mmol, 1.95 grams). After 30 mins the mixture was diluted with 75mL water and extracted 2X50mL ethyl acetate. The combined organics were dried over magnesium sulphate, filtered, and concentrated in vacuo. The resulting residue was further purified by column chromatography (SiO2, 50% ethyl acetate/hexane to 70% ethyl acetate/hexane, TLC, R_{f}=0.21, uv positive) to yield (±)-1-methyl-4-(2-carboxy-2-methylethyl)-7-iodo-3,4-dihydro-1*H*-1,4-benzodiazepine-2,5-dione methyl ester (0.5 grams, 61%, mp=138-140°C). ¹H NMR (CDCl₃, dTMS) 8.13 (1H, d, ⁴J_{HH}=2Hz, C6-H), 7.79 (1H, dd, ⁴J_{HH}=2Hz, ³J_{HH}=9Hz, C8-H), 6.92 (1H, d, ³J_{HH}=9Hz, C9-H), 3.80 (7H, s, NCH₂CHCO₂, C3-H, OCH₃), 3.34 (3H, two s, NCH₃, diastereotopic resonances are observed), 1.19 (1.5H, d, ³J_{HH}=7Hz, CHCH₃), 1.16 (1.5H, d, ³J_{HH}=7Hz, CHCH₃).

e) (±)-1-methyl-4-(2-carboxy-2-methylethyl)-7-(N-Boc-5-amino-1-hexynyl)-3,4-dihydro-1*H*-1,4-benzodiazepine-2,5-dione methyl ester was prepared using the method described in part (f) of example 1. Thus, 0.15 grams (0.36mmol) of (±)-1-methyl-4-(2-carboxy-2-methylethyl)-7-iodo-3,4-dihydro-1*H*-1,4-benzodiazepinedione-2,5,dione methyl ester and 0.11 grams (0.54mmol) of N-Boc-6-amino-1-hexyne yielded 0.164 grams (94%) of (±)-1-methyl-4-(2-carboxy-2-methylethyl)-7-(N-Boc-5-amino-1-hexynyl)-3,4-dihydro-1*H*-1,4-benzodiazepine-2,5-dione methyl ester (TLC, 1:1 ethyl acetate/hexane, R_{f}=0.23). ¹H NMR (CDCl₃, dTMS) 7.79 (1H, d, ⁴J_{HH}=2Hz, C6-H), 7.43 (1H, dd, ⁴J_{HH}=2Hz, ³J_{HH}=8Hz, C8-H), 7.06 (1H, d, ³J_{HH}=8Hz, C9-H), 4.67 (1H, bs, NHBoc), 4.04 (0.5H, d, J_{HH}=15Hz, C3-H), 3.94 (0.5H, d, J_{HH}=15Hz, C3-H), 3.93 (0.5H, d, J_{HH}=15Hz, C3-H), 3.73 (2.5H, m, NCH₂CH, C3-H), 3.66 (1.5H, s, OCH₃), 3.59 (1.5H, s, OCH₃), 3.30 (3H, bs, NCH₃), 3.10 (2H, bq, CH₂NH), 2.93 (1H, m, CHCO₂), 2.38 (2H, bt, ³J_{HH}=7Hz, CCCH₂), 1.58 (4H, m), 1.38 (9H, s, C(CH₃)₃), 1.14 (1.5H, d, ³J_{HH}=7Hz, CHCH₃), 1.11 (1.5H, d, ³J_{HH}=7Hz, CHCH₃); ¹³C NMR 175.1, 174.6, 168.7, 166.9, 156.1, 139.9, 134.9, 134.0, 128.7, 121.8, 120.9, 91.5, 79.3, 79.1, 52.5, 52.1, 52.0, 51.5, 40.2, 38.5, 38.4, 34.9, 34.8, 29.4, 28.5, 25.8, 19.2, 16.1, 14.6.

f) (±)-1-methyl-4-(2-carboxy-2-methylethyl)-7-(N-Boc-5-amino-1-hexyl)-3,4-dihydro-1*H*-1,4-benzodiazepine-2,5-dione methyl ester was prepared using the method described in part (a) of example 3. Thus, 0.085 grams (0.17mmol) of (±)-1-methyl-4-(2-carboxy-2-methylethyl)-7-iodo-3,4-dihydro-1*H*-1,4-benzodiazepinedione-2,5,dione methyl ester yielded 0.081 grams (95%) of (±)-1-methyl-4-(2-carboxy-2-methylethyl)-7-(N-Boc-5-amino-1-hexyl)-3,4-dihydro-1H-1,4-benzodiazepine-2,5-dione methyl ester (TLC, 3:1 ethyl acetate/hexane, R_{f}=0.62). ¹H NMR (CDCl₃, dTMS) 7.58 (1H, d, ⁴J_{HH}=2Hz, C6-H), 7.27 (1H, dd, ⁴J_{HH}=2Hz, ³J_{HH}=8Hz, C8-H), 7.06 (1H, d, ³J_{HH}=8Hz, C9-H), 4.60 (1H, bs, NHBoc), 4.07 (0.5H, d, J_{HH}=15Hz, C3-H), 3.98 (0.5H, d, J_{HH}=15Hz, C3-H), 3.97 (0.5H, d, J_{HH}=15Hz, C3-H), 3.75 (2.5H, m, NCH₂CH, C3-H), 3.67 (1.5H, s, OCH₃), 3.60 (1.5H, s, OCH₃), 3.33 (3H, two s, NCH₃), 3.06 (2H, bq, CH₂NH), 2.95 (1H, m, CHCO₂), 2.58 (2H, bt, ³J_{HH}=7Hz, ArCH2), 1.59 (2H, m), 1.40 (11H, m, C(CH₃)₃), 1.30 (4H, m), 1.15 (1.5H, d, ³J_{HH}=7Hz, CHCH₃), 1.13 (1.5H, d, ³J_{HH}=7Hz, CHCH₃);¹³C NMR 174.9, 174.5, 168.8, 167.4, 155.9, 140.2, 138.6, 132.1, 130.1, 128.2, 120.7, 78.8, 52.4, 52.0, 51.9, 51.2, 40.3, 38.4, 34.8, 34.7, 30.8, 29.8, 28.7, 28.3, 26.4, 14.9, 14.4.

g) (±)-1-methyl-4-(2-carboxy-2-methylethyl)-7-(6-amino-1-hexyl)-3,4-dihydro-1*H*-1,4-benzodiazepine-2,5-dione trifluoracetate was prepared using the method described in part (g) of example 1. Thus 0.04grams of (±)-1-methyl-4-(2-carboxy-2-methylethyl)-7-(N-Boc-5-amino-1-hexyl)-3,4-dihydro-1*H*-1,4-benzodiazepine-2,5-dione methyl ester yielded 27mgs (88%) of the desired product, purified by HPLC (Column: Dynamax 60A C18, Method: 10-50% MeCN/H2O 10ml/min. Detector: 254nm. Time(%MeCN): 0:00(10), 00:15(10,inj.,detect on), 10:00(10), 40:00(50), 55:00(50), 59:58(10, detect off), 60:00(off), R_{f}=32.1min). ¹H NMR (D2O) 7.30 (1H, d, ⁴J_{HH}=2Hz, ArH C6-H), 7.24 (1H, dd, ⁴J_{HH}=2Hz, ³J_{HH}=8Hz, ArH C8-H), 7.08 (1H, d, ³J_{HH}=8Hz, ArH C9-H), 3.85 (1.6H, m, C3-H, NCHHCH(Me)CO₂), 3.57 (1.8H, m, C3-H, NCHHCH(Me)CO₂), 3.25 (0.6H, dd, J_{HH}=14Hz, ³J_{HH}=5Hz), 3.10 (3H, s, NCH₃), 2.74 (3H, m, CH(Me)CO₂, CH₂NH₂), 2.40 (2H, t, ³J_{HH}=7Hz, ArCH₂), 1.38 (4H, m), 1.13 (4H, m),0.95 (1.8H, d, ³J_{HH}=7Hz, CHCH₃), 0.88 (1.2H, d, ³J_{HH}=7Hz, CHCH₃). Exact mass (FAB, M+H⁺)calculated for C₂₀H₃₀N₃O₄ 376.2236, found 376.2228.

### Example 32

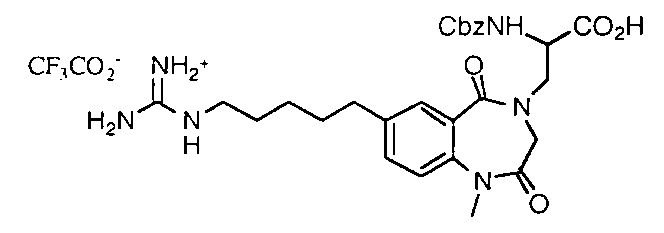

*(±)-1-methyl-4-(N-Cbz-2-carboxy-2-aminolethyl)-7-(6-amino-1-hexyl)-3,4-dihydro-1H-1,4-benzodiazepine-2,5-dione trifluoracetate.*

a) A solution of (±)-2-(N-Cbz-amino)-3-aminopropionic acid (1gram, 4.22mmol), 10mL methanol, 1.6mL of a 4N HCl in dioxane was stirred at room temperature for 24 hrs. The reaction mixture was concentrated *in vacuo*, triturated with ether (50mL). The resulting residue was dissolved in dimethylformamide (6mL) and allowed to react with N-methyl-6-iodoisatoic anhydride (1.34grams, 4.5mmol) and triethylamine (1.9mL, 14mmol). The reaction was heated to 50°C for 1.5hrs, cooled to room temperature, diluted with 100mL water and extracted with 2×75mL ethyl acetate. The combined organics were dried over sodium sulphate, decanted, and concentrated *in vacuo*. The resulting residue was purified by column chromatography (SiO2, 35%ethyl acetata/hexane to 65%ethyl acetate/hexane) to yield (±)-N-(N³-methyl-2-amino-5-iodobenzoyl)-3-amino-2-(N¹-Cbz)-aminopropionate methyl ester (1.65 grams, 77%).

b) (±)-1-methyl-4-(2-carboxy-2-(N-Cbz)-aminoethyl)-7-iodo-3,4-dihydro-1*H*-1,4-benzodiazepine-2,5-dione methyl ester was prepared using the method described in part (d) of example 31. Thus, 0.825 grams (1,6mmol) of (±)-N-(N³-methyl-2-amino-5-iodobenzoyl)-3-amino-2-(N¹-Cbz)-aminopropionate methyl ester yielded 0.756 grams (86%) of (±)-1-methyl-4-(2-carboxy-2-(N-Cbz)-aminoethyl-7-iodo-3,4-dihydro-1*H*-1,4-benzodiazepine-2,5-dione methyl ester (TLC, SiO2, 1:1 ethyl acetate/hexane, R_{f}=0.21, uv positive). ¹H NMR (CDCl₃, dTMS) 8.09 (0.5H, d, ⁴J_{HH}=2Hz, C6-H), 8.04 (0.5H, d, ⁴J_{HH}=2Hz, C6-H), 7.76 (1H, dd, ⁴J_{HH}=2Hz, ³J_{HH}=8Hz, C8-H), 7.25 (5H, m, Ph), 6.92 (0.5H, d, ³J_{HH}=8Hz, C9-H), 6.87 (0.5H, d, ³J_{HH}=8Hz, C9-H), 6.22 (0.5H, d, ³J_{HH}=8Hz, CbzNH), 5.90 (0.5H, d, ³J_{HH}=8Hz, CbzNH), 4.67 (1H, bs, NHBoc), 5.05 (2H, OCH2), 4.63 (1H, m, CHCO2), 4.2-3.6 (7H, m, C3-H, NCH₂CH, C3-H, OCH₃), 3.29 (1.5H, bs, NCH₃), 3.24 (1.5H, bs, NCH₃); ¹³C NMR 170.5, 170.4, 168.4, 168.3, 166.8, 166.7, 156.1, 141.3, 140.7, 139.6, 139.4, 136.5, 136.2, 129.8, 128.7, 128.6, 128.5, 128.4, 128.3, 128.1, 123.1, 122.9, 89.5, 76.9, 67.0, 52.6, 52.4, 35.0.

c) (±)-1-methyl-4-(2-carboxy-2-(N-Cbz)aminoethyl)-7-(N-Boc-5-amino-1-pentynyl)-3,4-dihydro-1*H*-1,4-benzodiazepine-2,5-dione methyl ester was prepared using the method described in part (f) of example 1. Thus, 0.2 grams (0.36mmol) of (±)-1-methyl-4-(2-carboxy-2-(N-Cbz)-aminoethyl)-7-iodo-3,4-dihydro-1-*H*-1,4-benzodiazepine-2,5-dione methyl ester yielded 0.187 grams (85%) of the desired product (TLC, 1:1 ethyl acetate/hexane R_{f}=0.17 uv and ninhydrin positive). ¹H NMR (CDCl₃, dTMS) 7.83 (0.5H, d, ⁴J_{HH}=2Hz, C6-H), 7.79 (0.5H, d, ⁴J_{HH}=2Hz, C6-H), 7.50 (1H, bd, ³J_{HH}=8Hz, C8-H), 7.30 (5H, m, Ph), 7.10 (0.5H, d, ³J_{HH}=8Hz, C9-H), 7.07 (0.5H, d, ³J_{HH}=8Hz, C9-H), 6.24 (0.5H, d, ³J_{HH}=8Hz, CbzNH), 5.84 (0.5H, d, ³J_{HH}=8Hz, CbzNH), 5.09 (2H, OCH2), 4.66 (2H, m, NHBoc, CHCO₂), 4.2-3.6 (7H, m, C3-H, NCH₂CH, C3-H, OCH₃), 3.34 (1.5H, bs, NCH₃), 3.29 (1.5H, bs, NCH₃) 3.24 (2H, m, CH₂NHBoc), 2.43 (2H, m CCCH₂), 1.78 (2H, m, CH₂CH₂CH₂), 1.44 (9H, s, C(CH₃)₃); ¹³C NMR 170.3, 170.2, 168.2, 168.1, 167.5, 167.4, 155.9, 139.7, 135.1, 135.0, 133.9, 133.8, 128.4, 123.3, 128.0, 127.9, 127.8, 121.4, 120.9, 90.9, 79.1, 79.0, 66.9, 66.8, 53.7, 52.9, 52.8, 52.7, 51.9, 49.6, 34.8, 28.7, 28.3, 16.8.

d) (±)-1-methyl-4-(2-carboxy-2-(N-Cbz)aminoethyl)-7-(N-Boc-5-amino-1-pentyl)-3,4-dihydro-1*H*-1,4-benzodiazepine-2,5-dione methyl ester was prepared using the method described in part (a) of example 3. Thus, 93mgs of (±)-1-methyl-4-(2-carboxy-2-(N-Cbz)aminoethyl)-7-(N-Boc-5-amino-1-pentyl)-3,4-dihydro-1*H*-1,4-benzodiazepine-2,5-dione methyl ester yielded 93mgs (99%) of the desired product. ¹H NMR (CDCl₃, dTMS) 7.60 (0.6H, d, ⁴J_{HH}=2Hz, C6-H), 7.55 (0.4H, d, ⁴J_{HH}=2Hz, C6-H), 7.30 (6H, m, Ph, C8-H), 7.07 (0.6H, d, ³J_{HH}=8Hz, C9-H), 7.06 (0.4H, d, ³J_{HH}=8Hz, C9-H), 6.34 (0.6H, d, ³J_{HH}=8Hz, CbzNH), 5.89 (0.4H, d, ³J_{HH}=8Hz, CbzNH), 5.09 (2H, m, OCH2), 4.60 (2H, bs, CHCO₂, NHBoc), 4.2-3.6 (7H, m, C3-H, NCH₂CH, C3-H, OCH₃), 3.33 (1.5H, bs, NCH₃), 3.30 (1.5H, bs, NCH₃) 3.07 (2H, m, CH₂NHBoc), 2.60 (2H, m, ArCH₂), 1.60 (2H, m), 1.45 (2H, m), 1.40 (9H, s, C(CH₃)₃), 1.33 (2H, m); ¹³C NMR 170.4, 168.5, 168.4, 155.9, 140.3, 138.7, 132.4, 130.4, 128.4, 128.3, 128,0, 121.0, 66.9, 60.3, 53.9, 53.0, 52.8, 52.7, 52.0, 50.5, 49.7, 40.4, 34.8, 30.7, 29.9, 28.3, 26.3, 20.9, 14.1.

e) (±)-1-methyl-4-(2-carboxy-2-(N-Cbz)aminoethyl)-7-(5-amino-1-pentyl)-3,4-dihydro-1*H*-1,4-benzodiazepine-2,5-dione trifluoracetate was prepared using the method described in part (g) of example 1. Thus 46mgs of (±)-1-methyl-4-(2-carboxy-2-(N-Cbz)aminoethyl)-7-(N-boc-5-amino-1-pentyl)-3,4-dihydro-1*H*-1,4-benzodiazepine-2,5-dione methyl ester yielded 16mgs (40%) of the desired product, purified by HPLC (Column: Dynamax 60A C18, Method: 10-50% MeCN/H2O 10ml/min. Detector: 254nm. Time(%MeCN): 0:00(10), 00:15(10,inj.,detect on), 10:00(10), 40:00(50), 55:00(50), 59:58(10, detect off), 60:00(off) R_{f}=38.3min). ¹H NMR (D₂O) 7.38 (2H, bs), 7.18 (3H, m), 6.95 (2H, m), 6.78 (1H, m), 4.94 (2H, m, OCH₂Ph), 4.0-3.3 (4H, m), 3.11 (1H, s, NCH₃), 2.86 (2H, s, NCH₃), 2.81 (2H, m, CH₂NH₂), 2.58(1.3H, t, ³J_{HH}=7Hz, ArCH₂), 2.47 (0.7H, t, ³J_{HH}=7Hz, ArCH₂), 1.57 (4H, m), 1.23 (2H, m).

f) (±)-1-methyl-4-(2-carboxy-2-(N-Cbz)aminoethyl)-7-(5-guanidino-1-pentyl)-3,4-dihydro-1*H*-1,4-benzodiazepine-2,5-dione trifluoracetate was prepared using the method described in example 2. Thus 8mgs of (±)-1-methyl-4-(2-carboxy-2-(N-Cbz)aminoethyl)-7-(5-amino-1-pentyl)-3,4-dihydro-1*H*-1,4-benzodiazepine-2,5-dione trifluoracetate yielded 6mgs (69%) of (±)-1-methyl-4-(2-carboxy-2-(N-Cbz)aminoethyl)-7-(5-guanidino-1-pentyl)-3,4-dihydro-1*H*-1,4-benzodiazepine-2,5-dione trifluoracetate (HPLC, Column: Dynamax 60A C18, Method: 10-50% MeCN/H2O 10ml/min. Detector: 254nm. Time(%MeCN): 0:00(10), 00:15(10,inj., detect on), 10:00(10), 40:00(50), 55:00(50), 59:58(10), detect off), 60:00(off), R_{f}=45.4min). ¹H NMR (D₂O) 7.40 (2H, bs), 7.20 (3H, m), 6.96 (2H, m), 6.78 (1H,t, ³J_{HH}=8Hz), 4.98 (2H, m, OCH₂Ph), 4.0-3.3 (4H, m), 3.13 (1H, s, NCH₃), 2.94 (2H, m, CH₂NH), 2.83 (2H, s, NCH₃), 2.59 (1.3H, t, ³J_{HH}=7Hz, ArCH₂), 2.48 (0.7H, t, ³J_{HH}=7Hz, ArCH₂), 1.42 (4H, m), 1.20 (2H, m). Exact mass calculated for C₂₇H₃₅N₆O₆ 539.2618, found 539.2618.

### Example 33

### Fibrinogen-GP II_{b}IIIₐ Receptor ELISA Binding Assay

The method used is essentially that described in Nachman and Leung (J. Clin. Invest., 69: 263-269 [1982]). The GP II_{b}IIIₐ is essentially purified as described in Fitzgerald *et al.,* (Anal. Biochem. 151: 169-177 [1985]).

### A. GP II_{b}IIIₐPurification

Outdated human platelets are washed 3 times with 10 mM tris-HCl, 150 mM NaCl (TBS), 1 mM EDTA, pH 7.5, and centrifuged at 2000 × g to pellet cells. Cells are lysed in 5 pellet volumes of TBS, 1% Triton X-100, 1 mM Ca₂Cl₂, and followed by centrifugation at 30,000 x g. The supernatant fraction is collected and the supernatant is loaded onto a concanavalin-A column, previously equilibrated in TBS, 1 mM Ca₂Cl₂, 0.1% Triton, 0.05% NaN₃ and eluted with 0.2 M a-methylmannoside. Fractions are pooled and loaded onto a heparin-agarose column. The flowthrough is collected and concentrated on an Amicon YM 30 filter to a volume of approximately 5-10 ml. The concentrate is then applied to an S-300 column (500 ml) and 6 ml fractions are collected. The GP II_{b}IIIₐ containing fractions are collected, pooled, and stored at -80° C.

### B. Purification of Low Solubility Fraction of Fibrinogen

The purification of fibrinogen is conducted essentially as described by Lipinska *et al.,* (*J. Lab*. *Clin. Med.* 507, [1974]). Briefly, a 0.3 % w/v solution of human fibrinogen (Kabi #5302) is dissolved in 150 mM NaCl. Saturated (NH₄)₂SO₄ is added dropwise with stirring to the fibrinogen solution to obtain about 16 % saturation. The precipitate is spun down in appropriate size bottles at 2000 x g. The supernatant is decanted and the precipitate resuspended in 150 mM NaCl (approximately 50% of the original volume). NH₄SO₄ is again added dropwise to obtain 16 % saturation. The suspension is spun down and the precipitate is resuspended in Tris-saline in a minimal volume (approximately 5% of the original volume). Any remaining insoluble material is spun down at 2000 rpm in a Sorval type centrifuge and the fibrinogen supernatant is decanted and dialyzed overnight at 4°C against Tris-saline. Characterization of the fibrinogen is by the Bradford protein assay, SDS-PAGE, and/or Western blotting using well known standard procedures.

### C. ELISA Assay

Briefly, 96 well plates are coated (Type Nunc 1 Maxisorp™) with 10 mg/ml purified fibrinogen (100 ml/well), and allowed to stand overnight at 4°C. The plates are washed three times with PBS Tween (0.137 M NaCl, 0.003 M KCl, 0.008 M Na₂HPO₄, 0.001 M KH₂PO_{4,} pH 7.4 at room temperature, 0.05% Tween-20) and blocked for 1 to 2 hours at room temperature with 200 ml/well TNCNT (which is 0.5% BSA, 20mM Tris, pH 7.5 at room temperature, 120mM NaCl, 0.2% NaN_{3,} 2mM CaCl₂, 0.05% Tween 20, 0.5% BSA [Calbiochem RIA grade or better]) on a plate shaker. The plates are again washed three times with PBS/Tween and then 50 ml of sample in TNCNT is added. The mixture is incubated for 15 minutes at room temperature on a plate shaker. The stock solution of purified GP II_{b}IIIₐ receptor from human platelets, (0.4 - 1.0 mg/ml GP II_{b}IIIₐ in 0.1% Triton X-100, 1mM CaCl₂, 20 mM Tris, 150 mM NaCl, 0.05% NaN₃ in 0.3 M N-acetyl glucosamine pH 7.5, stored at -70°C), is reconstituted to about 40 mg/ml in TNCNT. Fifty ml of this diluted GP II_{b}IIIₐ is then added to each well and incubated on a plate shaker at room temperature. After one hour, the plates are washed four times with PBS/Tween and 100 ml of a polyclonal or monoclonal antibody specific for GP IIIa such as AP3 (1mg/ml) (See e.g. Newman *et al., Blood,* 65: 227-232 [1985]) in ELISA buffer (PBS, 0.5% BSA, 0.05% Tween 20, 0.01% Thimerasol) is added. After a one hour incubation at room temperature on a plate shaker, the samples are washed 4 times with PBS/Tween. One hundred ml of GAM-HRP (horse radish peroxidase conjugate of goat anti-mouse IgG [Pel-Freeze Cat. 715305-1] dissolved in ELISA buffer) previously diluted to 1:10,000 is then added and the samples are incubated 1 hour at room temperature on a plate shaker. The samples are then washed 4 times with PBS/Tween and 100 ml OPD/H₂O₂ substrate is added (OPD/H₂O₂ substrate: 10 mg *o*-phenylenediamine in 15 ml phosphate/citrate buffer, at room temperature and covered with foil; just before use, 6.25 ml of 30% H₂O₂ is added to give a final solution of 0.67 mg OPD/ml in 0.0125% H₂O₂ ). (The phosphate/citrate buffer consists of 16 mM Citric Acid, 50 mM Na₂ HPO₄, pH 5.0). The color develops within about 3 to 20 minutes and the reaction is stopped with 100 ml 1 M H₂SO₄. The optical density at 492 nm vs 405 nm is recorded and IC₅₀ values are determined.

### Example 34

### Human Vitronectin-Vitronectin Receptor (aᵥb₃) ELISA Assay

### A. Human Vitronectin Purification

Human vitronectin (Vn) is isolated from human plasma and purified by affinity chromatography by the method of Yatohgo et. al., (*Cell Structure and Function* 13: 281-292 [1988]).

### B. Human Vitronectin receptor (aᵥb₃) Purification

Human vitronection receptor (VnR) is purified from human placenta by the method of Pytela *et al., (Methods Enzymol*., 144: 475 [1987]). Alternatively the aᵥb₃ receptor can be purified from some cell lines (e.g., human embryonic kidney 293 cells) transfected with DNA sequences for both the aᵥ and b₃ subunits. The subunits are purified by employing octylglucoside extraction followed by Con-A, Heparin-Sepharose, and S-300 Chromatography.

### C. Monoclonal Antibodies

Anti-GP II_{b}IIIₐ monoclonal antibodies specific for human GPIIIₐ are prepared by the method of Newman *et al. (Blood,* 65: 227-232 [1985]), or a similar procedure. This mouse Mab is specific for the b₃ subunit of the vitronectin receptor.

Rabbit Fab 2 anti-mouse Fc fragment horse radish peroxidase conjugate (anti-MuFc HRP) is obtained from PelFreeze (cat. no. 715305-1).

### D. ELISA Assay

Maxisorp microtiter plates are coated with 2 mg/ml human vitronectin dissolved in PBS (50 ml/well) and stored overnight at 4°C. The plates are washed two times with PBS-0.05% Tween-20 (wash buffer) and blocked by incubating with about 150 ml/well of assay buffer (1%, BSA [RIA grade or better] in 50 mM Tris-HCl, 100 mM NaCl, 1mM MgCl₂, CaCl₂, MnCl₂ pH 7.4) for 60 minutes. Dilutions of standards are prepared and putative inhibitors (Table 3) are dissolved in assay buffer. The blocked plates are emptied and 25 ml/well of inhibitor or standard solution is added to each well. Twenty-five ml of a 30 mg/ml solution of purified aᵥb₃ in assay buffer is pipetted into the coated plate. The final concentration of receptor in the assay well is about 15 mg/ml. The plate is incubated on a shaker for 60 minutes. Meanwhile , for each microtite plate, 6 ml buffer solution containing 1.5 mg/ml of mouse monoclonal antibody specific for b₃ is prepared. To this solution is added ml of the secondary antibody, which is anti-mouse-Fc-HRP antibody conjugate. For example, for one plate, prepare 6 ml of a 1.5 mg/ml mouse Mab solution to which is added 1 ml of anti-mouse-Fc-HRP antibody stock, (this represents a 1:6000 dilution of the antibody - HRP conjugate). This mixture is allowed to incubate during the receptor-inhibitor incubation. The assay plates are washed 4 times with PBS-Tween and 50 ml/well of the antibody mixture is then pipetted into the plate for a 60 minute incubation. The plate is washed 4 times and the color reaction is developed with 50 ml/well of 0.67 mg/ml *o*-phenyldiamine in PBS containing 0.012% H₂O₂. Alternatively, 16 mM citric acid , 50 mM Na₂PO₄ at pH 5.0 can be used as a substrate buffer. The reaction is stopped with 50 ml/well 1 M H₂SO₄. The plates are read at 492-405 nm and the data analyzed by four-parameter fit.

### Example 35

### GP II_{b}IIIₐ-von Willebrand factor (vWF) ELISA Assay

### A. ELISA Assay

Microtiter plates are coated with 1.0 mg/ml GP II_{b}III_{a,} prepared by the method of Fitzgerald *et al., (Anal. Biochem. 151:* 169-177 [1985]) and allowed to incubate overnight in coat buffer. The plates are then washed three times in wash buffer (0.05% Tween 20 in PBS) and 150 ml of assay buffer is added and allowed to incubate for 1-2 hours at room temperature on plate shaker. The plates are washed three times and 50 ml of 2x inhibitor in assay buffer (Assay buffer: 0.5% BSA/50mM Tris, 100mM NaCl, 1.0mM CaCl₂, 1.0mM MgCl₂, 1.0mM MnCl₂; coat buffer is the same but without BSA) is added. Fifty ml of 4.0 mg/ml vWF (prepared as described by Ledford *et al., Thrombosis and Haemostasis, 64(4):* 569-575 [1990]) in assay buffer is then added and allowed to incubate for one hour at room temperature on plate-shaker. The plates are washed three times and the antibody mixture is added (1:5000 of mouse anti-vWF and 1:5000 of rabbit-anti-mouse-Fc-HRP, both commercially available) in assay buffer and incubated for 1 hour at room temperature on plate-shaker. Plates are again washed three times and 100 ml of substrate solution (10 mg OPD, 6.5 ml H₂ O₂, 15 ml phosphate citrate buffer) is added and incubated at room temperature. The color change of OPD/H₂O₂ reagent is read at 492 nm with a 405 nm reference wavelength on the filter photometer.

### Example 36

### In Vitro Human Platelet Aggregation Assay

Platelet aggregation assays are performed in human platelet rich plasma (PRP). Fifty milliliters of whole human blood (9 parts) is drawn on 3.6% sodium citrate (1 part) from a donor who has not taken aspirin or related medications for at least two weeks. The blood is centrifuged at 160 x g for 10 minutes at 22°C and allowed to stand for 5 minutes after which the PRP is decanted. Platelet poor plasma (PPP) is isolated from the remaining blood after centrifugation at 2000 x g for 25 minutes. The platelet count of the PRP is diluted to about 300,000 platelets per microliter with PPP.

A 225 ml aliquot of PRP plus 25 ml of either a dilution of the test inhibitor sample or a control (PBS) is incubated for 5 minutes in a Chrono-log Whole Blood Aggregometer at 25°C. An aggregating agent (collagen, 1 mg/ml; U46619, 100 ng/ml; or ADP, 17 mM) is added and the transmission is recorded.

### Example 37

### Representative 1, 3, 4, and 7 substituted Benzodiazepinediones

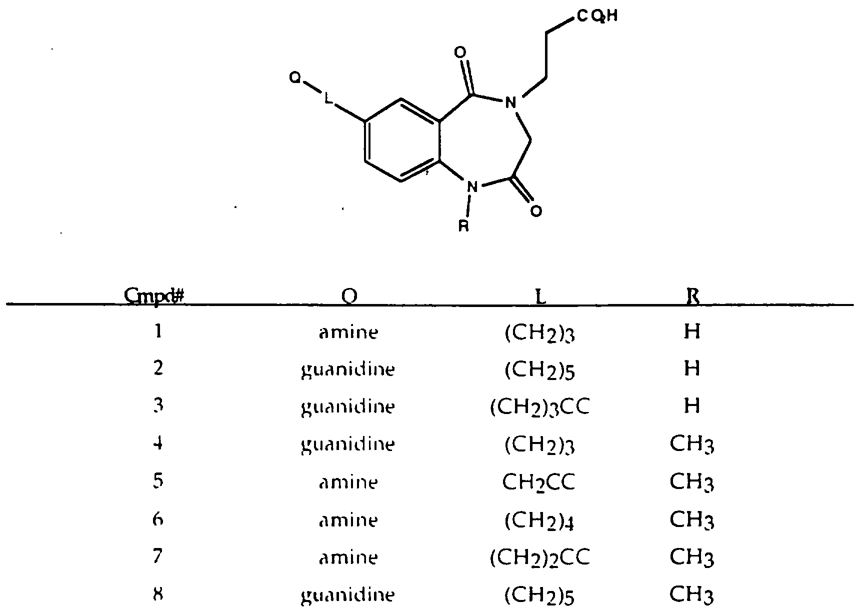

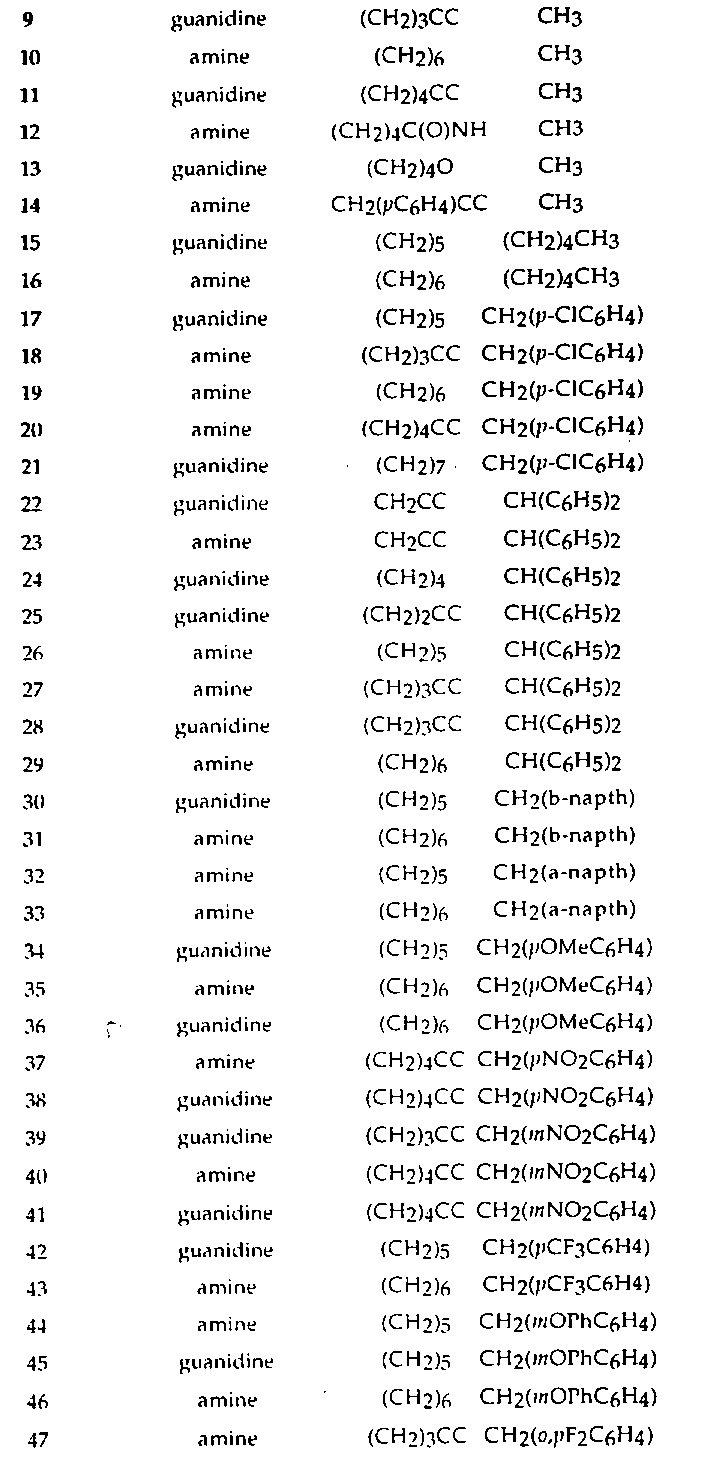

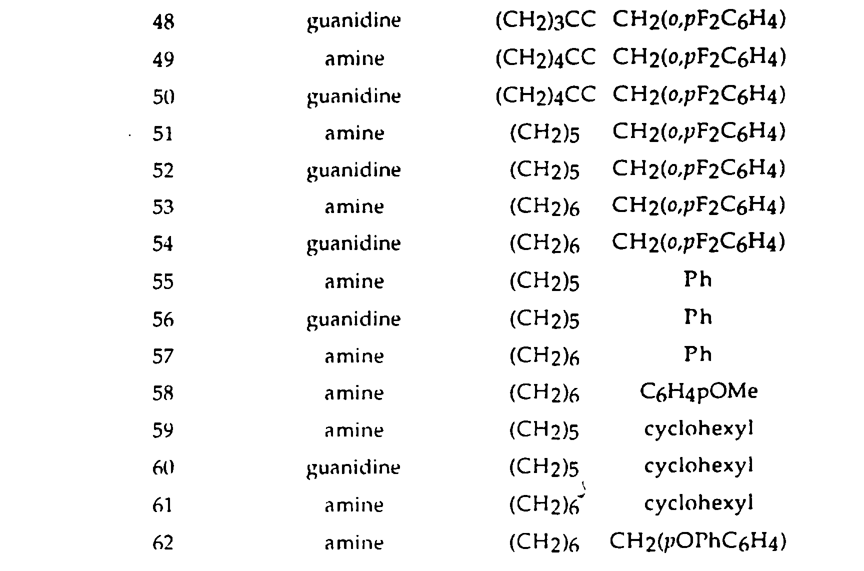

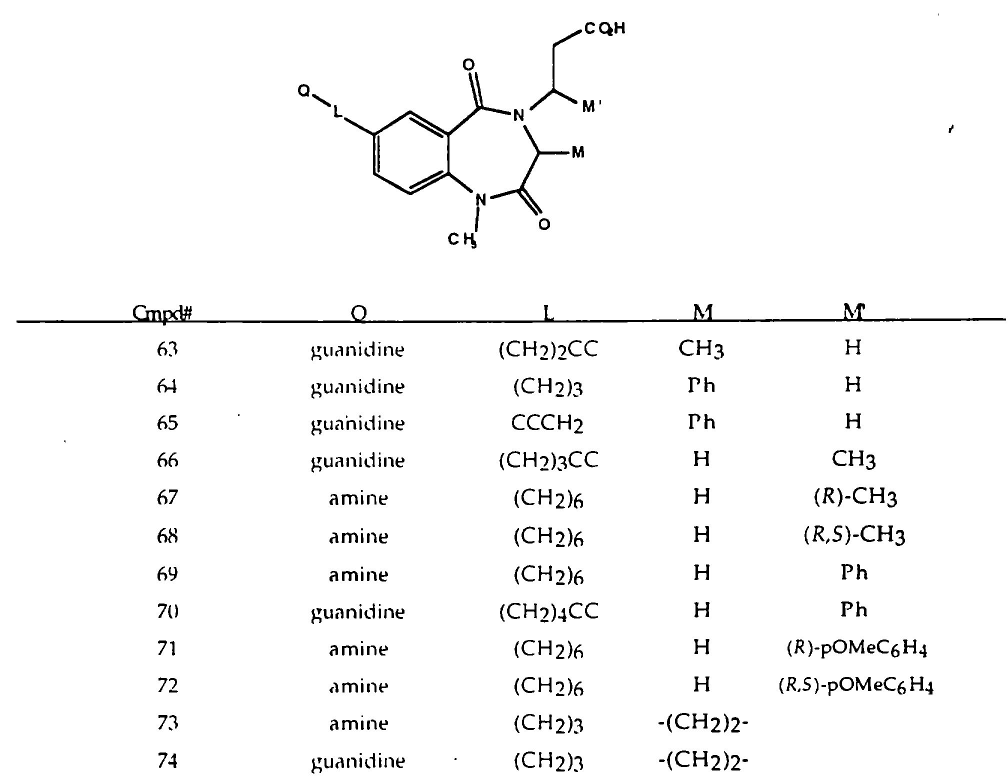

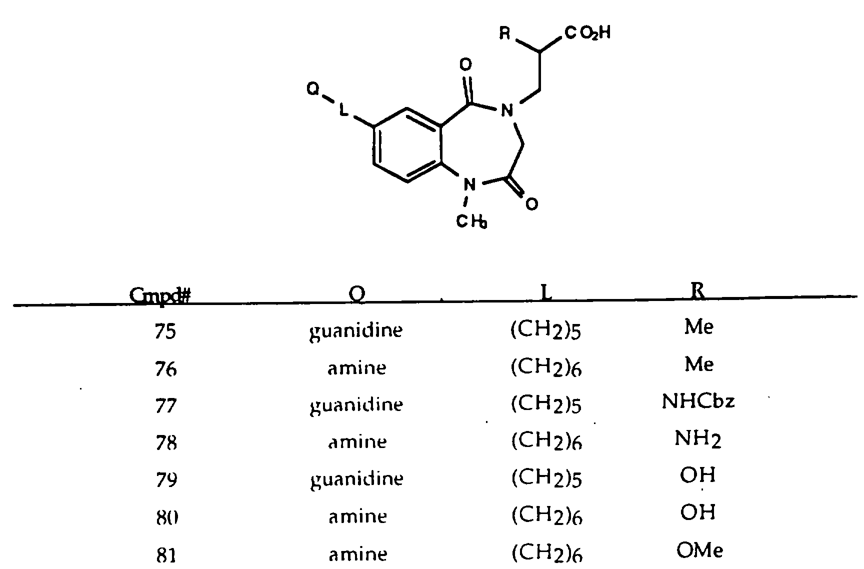

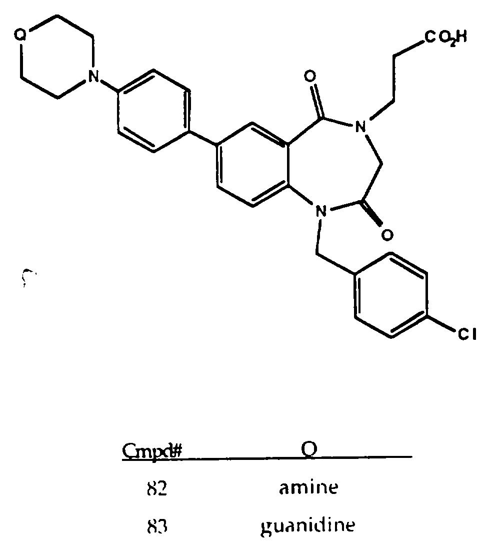

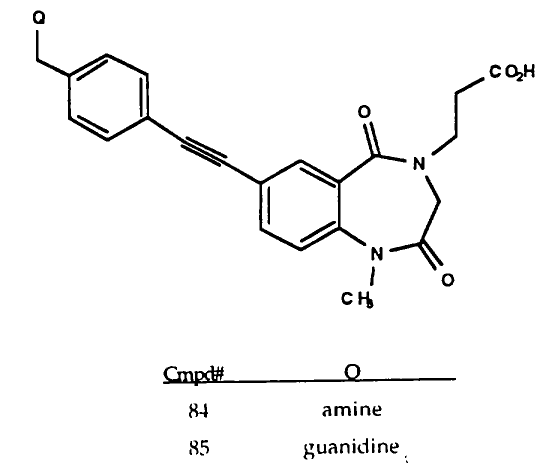

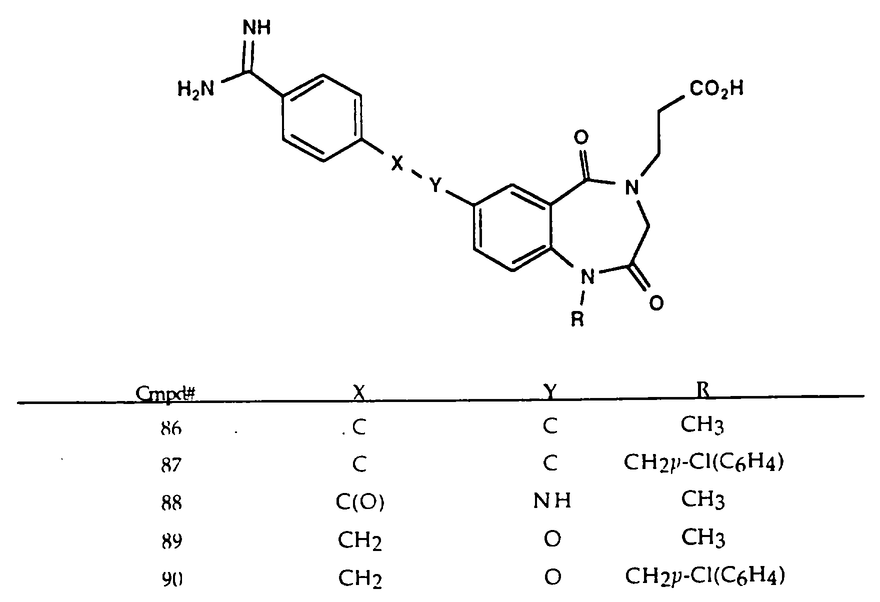

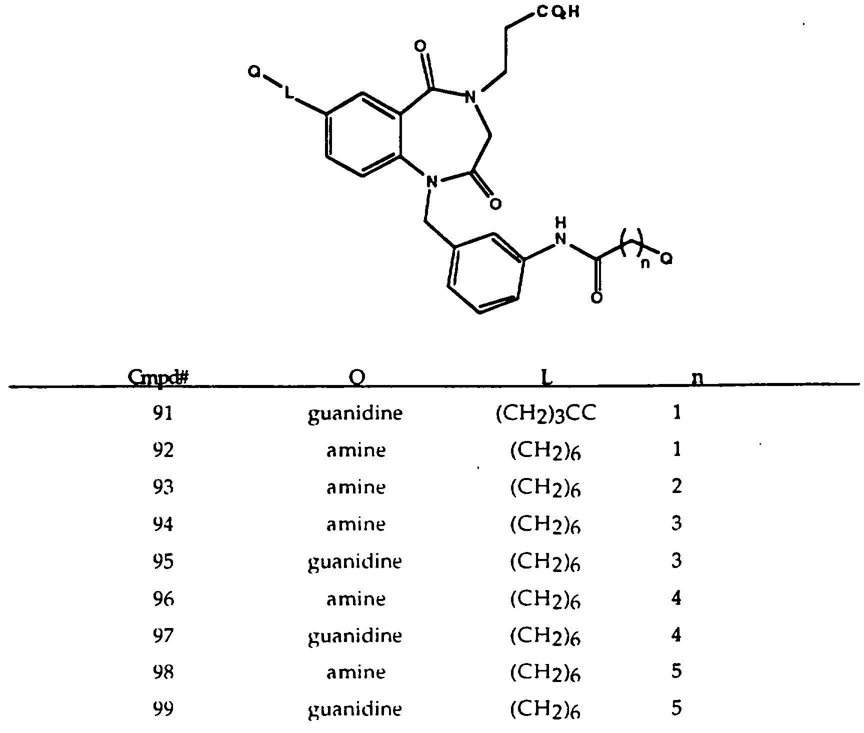

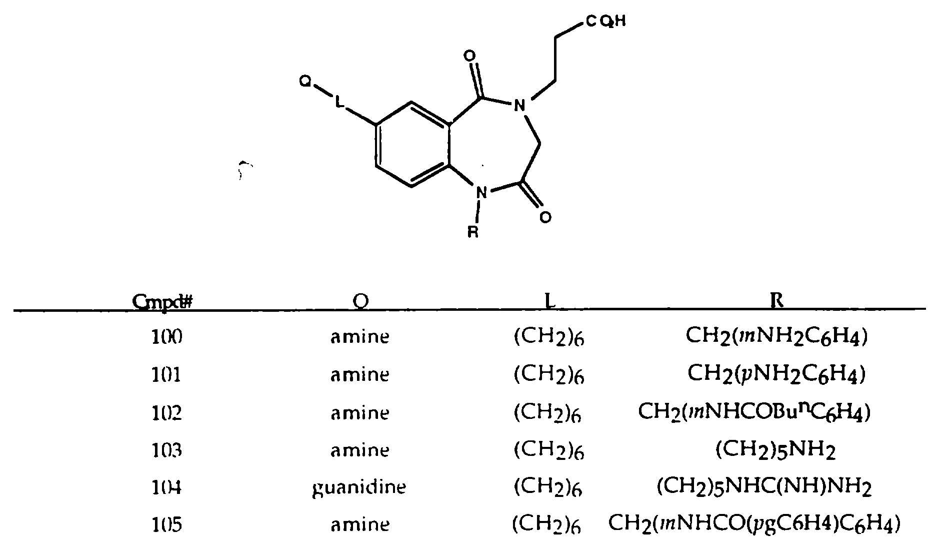

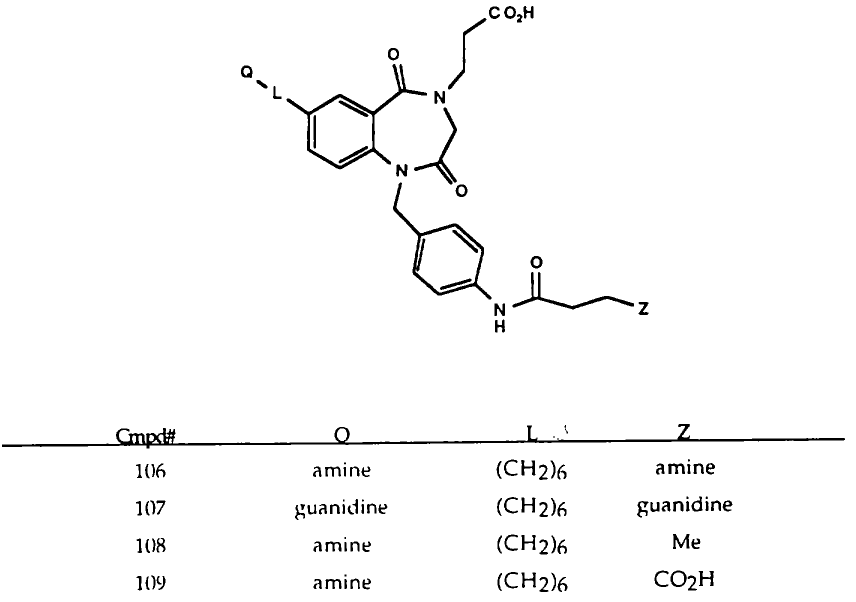

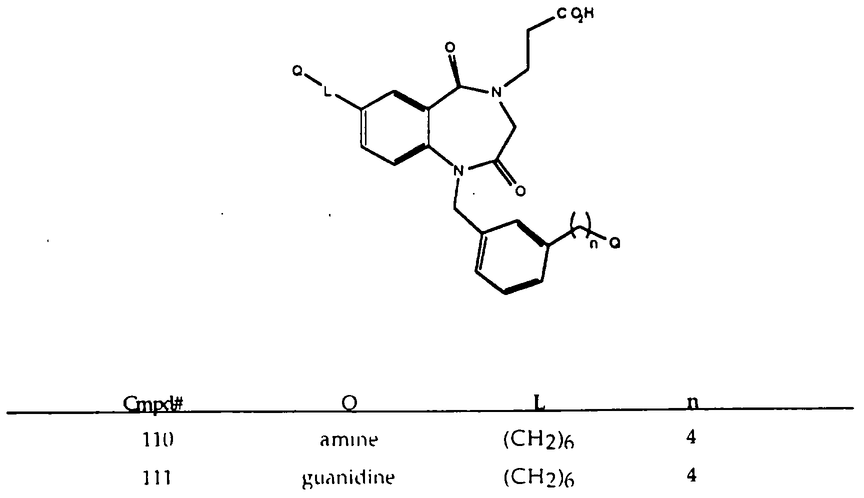

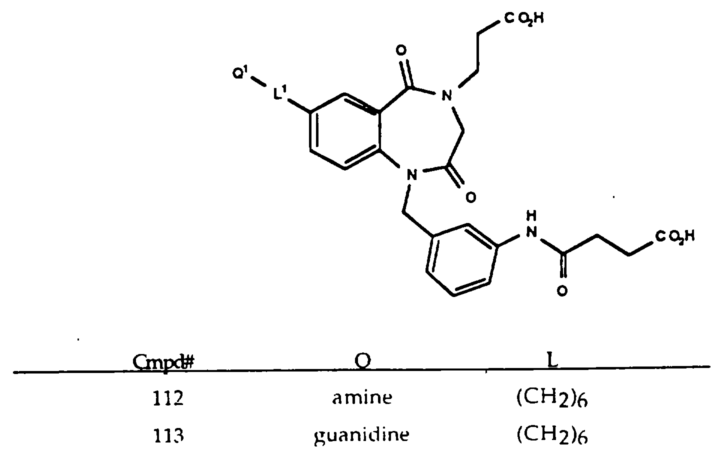

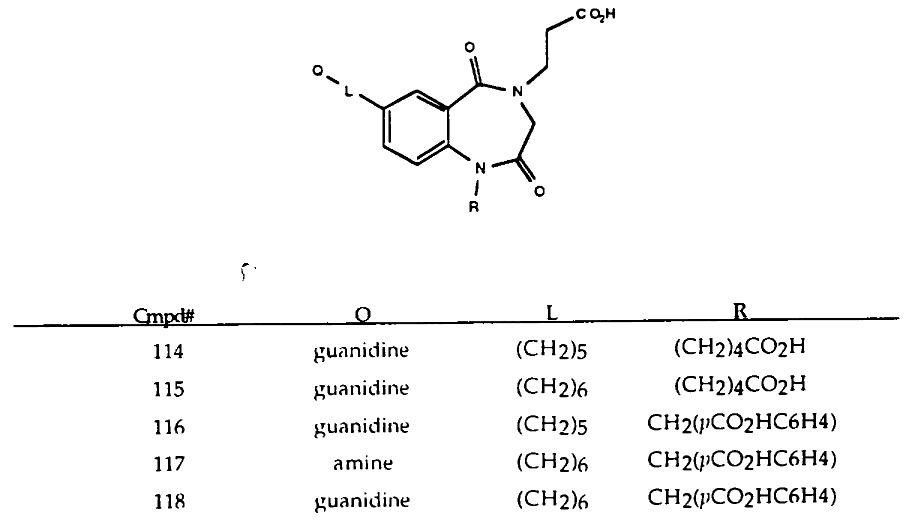

### Example 38

### Results of ELISA and Platelet Aggregation (PA) Assays for 1, 3, 4, and 7 substituted Benzodiazepinediones

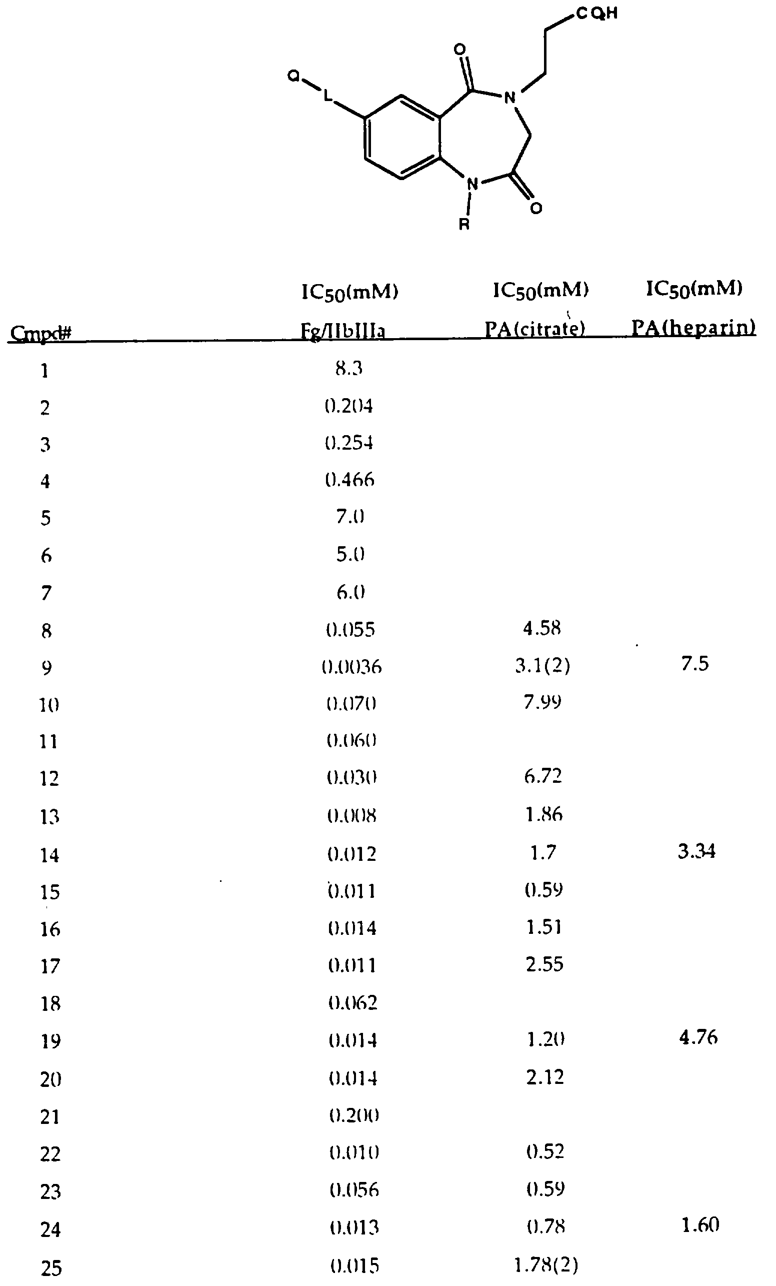

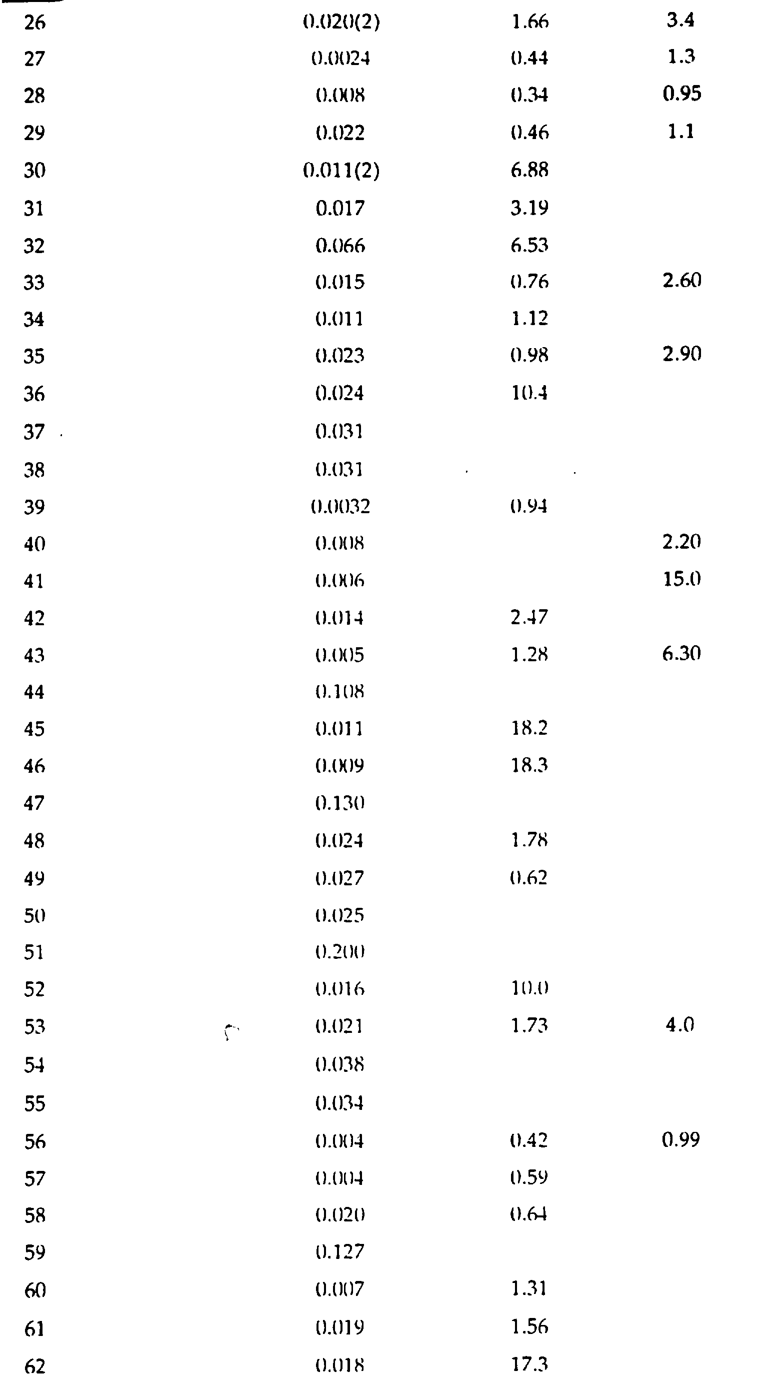

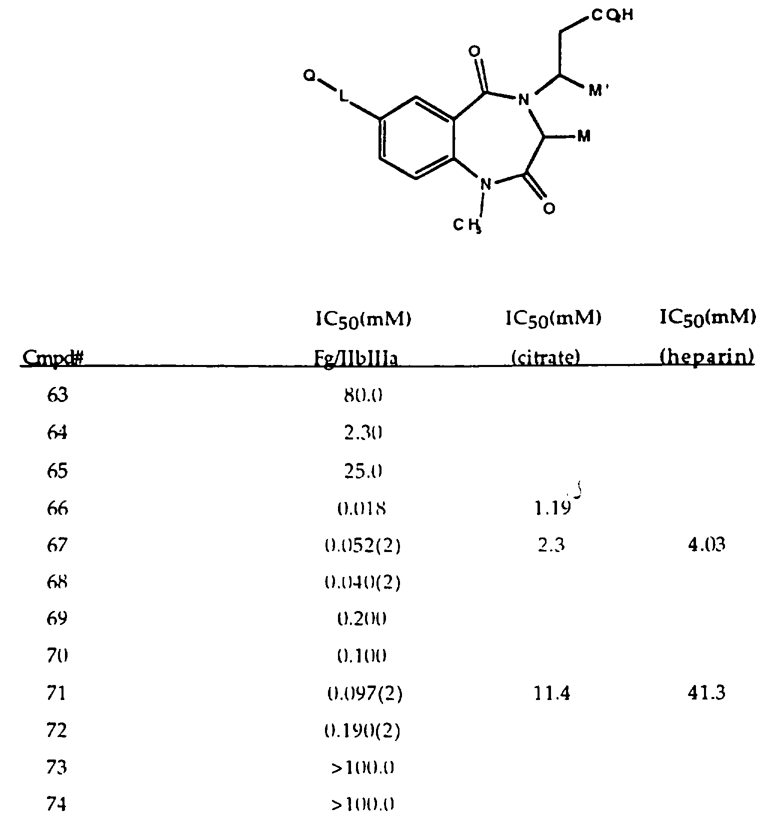

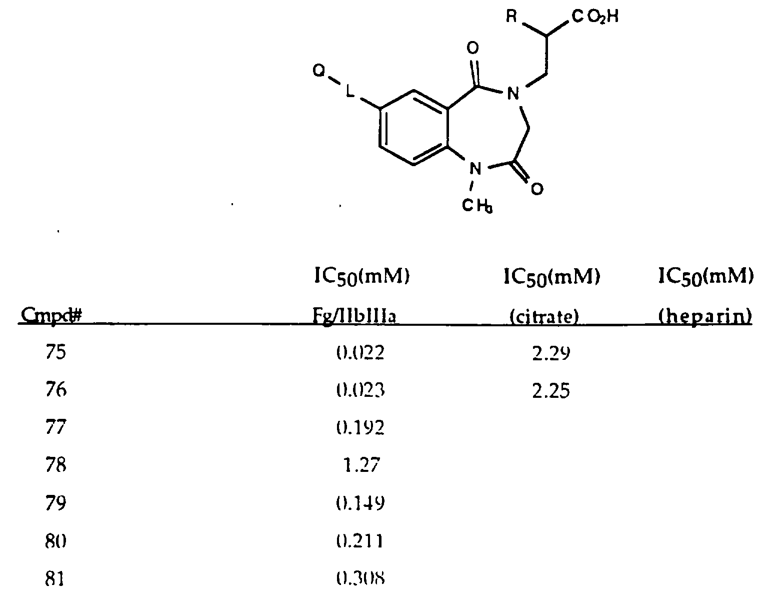

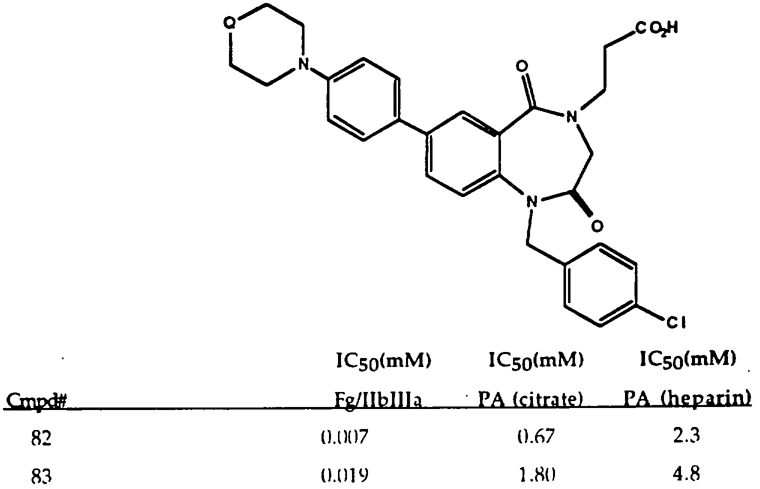

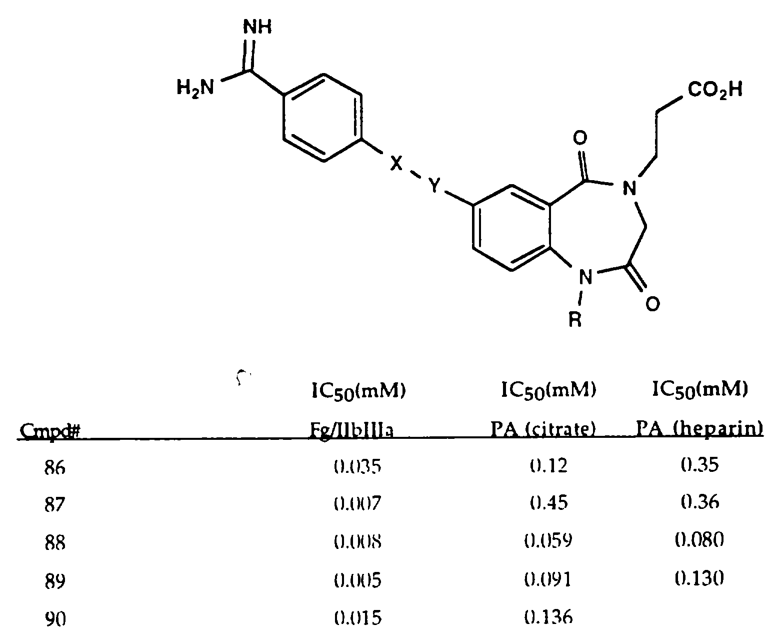

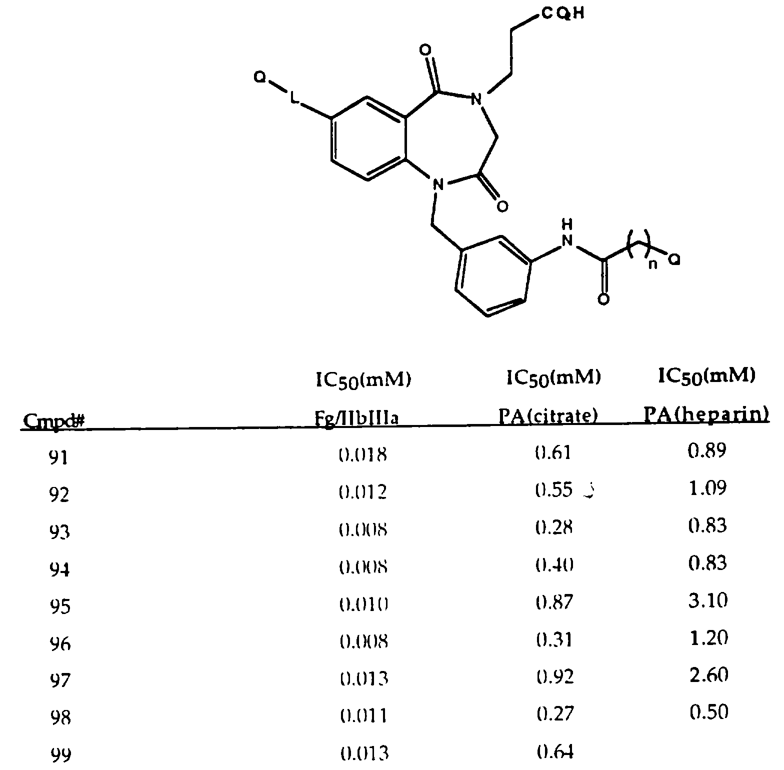

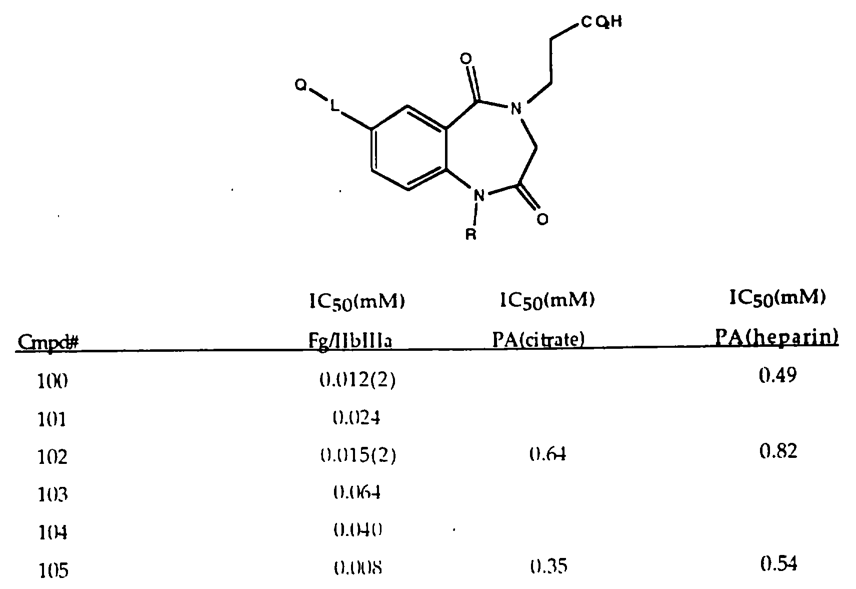

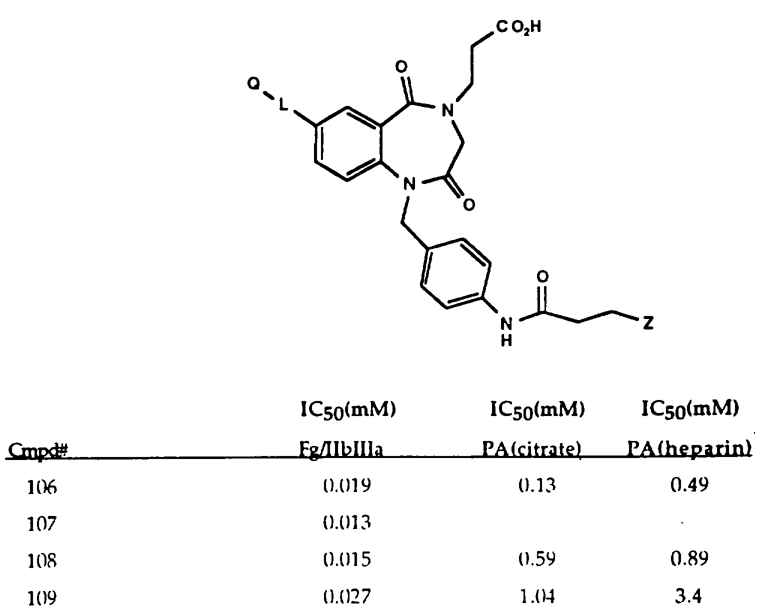

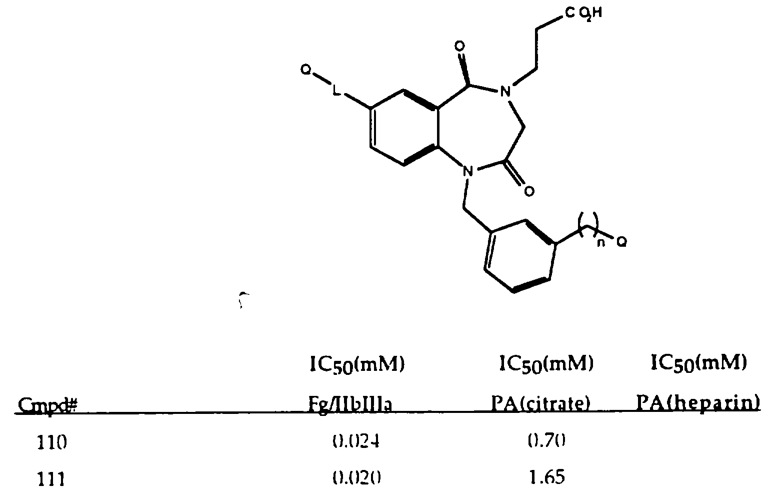

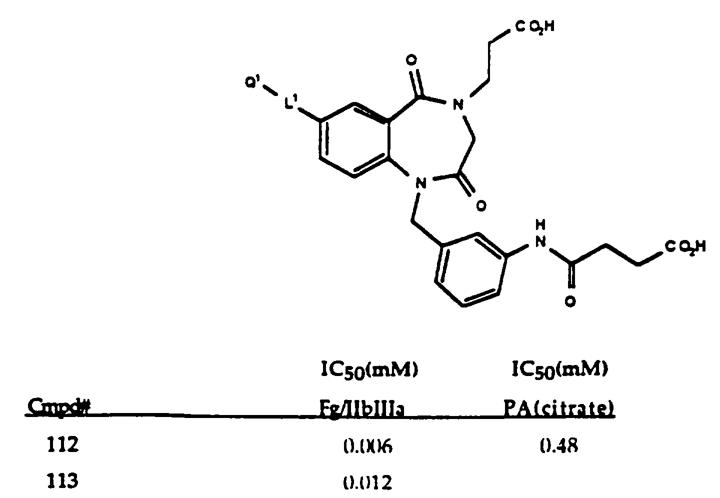

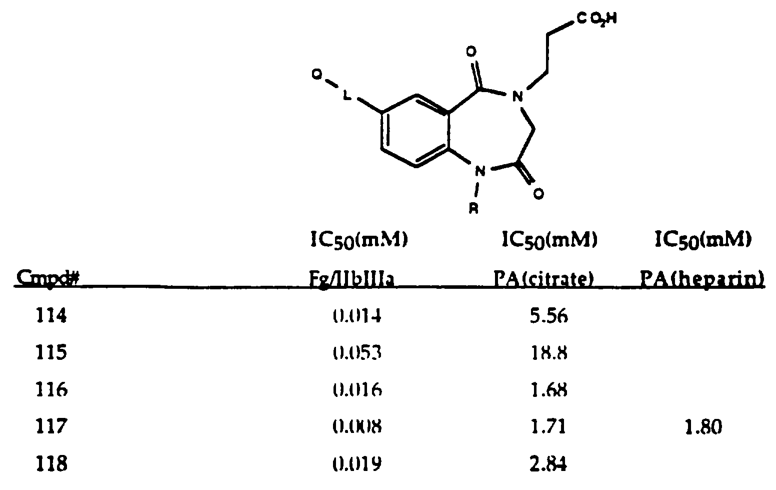

## Claims

1. A compound represented by structural formula (I): 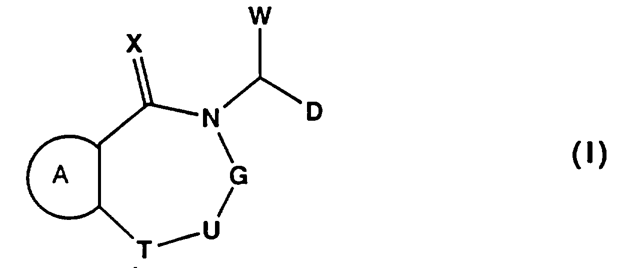
where the partial structure 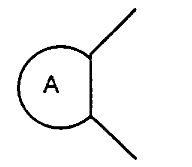
represents 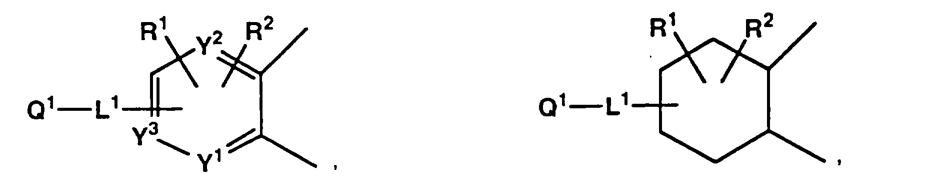 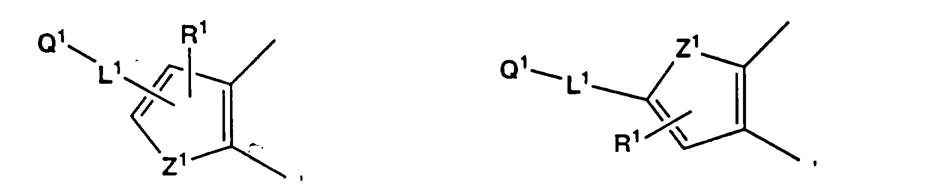 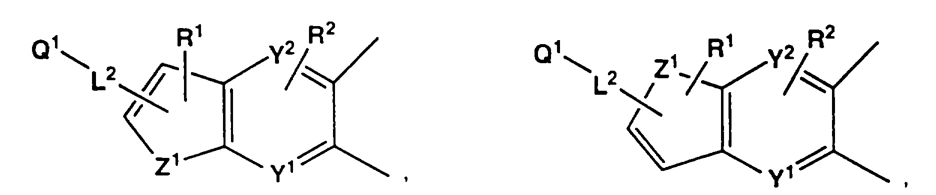 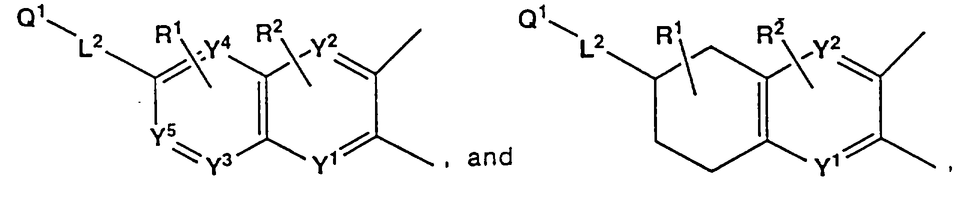
where
R¹ and R are independently selected from hydrogen, halo(F, Cl, Br, I), cyano, carboxamido, carbamoyloxy, formyloxy, formyl, azido, nitro, ureido, thioureido, thiocyanato, hydroxy, mercapto, sulfonamido, and an optionally substituted radical selected from C₁-C₁₂ alkyl, C₂-C₁₂ alkenyl, C₃-C₁₂ alkynyl, C₃-C₁₂ cycloalkyl, C₆-C₁₄ aryl, C₆-C₁₀ aryl-C₁-C₈-alkyl, C₁-C₁₂ alkyloxy, C₆-C₁₄ aryloxy, C₁-C₁₂ acylamino, N,N-di(C₁-C₁₂)acylamino, N-(C₁-C₁₂)alkyl-N-(C₁-sulfonylamino, C₁-C₁₂ alkylthiocarbonyl, C₁-C₁₂ alkylthio, C₁-C₁₂ alkylsulfinyl, C₁-C₁₂ alkylsulfonyl, C₁-C₁₂ alkylsulfonato, N-(C₁-C₁₂)alkylsulfonamido, N,N-di-(C₁-C₁₂)alkyl sulfonamido, N-(C₁-C₁₂) alkyl-N-thioformylamino, C₁-C₁₂ thioacylamino, N-(C₁-C₁₂)alkyl-N-(C₁-C₁₂) thioacylamino, C₁-C₁₂ alkylsulfinamido, N-(C₁-C₁₂)alkyl-N-(C₁-C₁₂)alkylsulfinylamino, C₁-C₁₂ carbalkoxy, C₁-C₁₂ alkylcarbonyl, C₁-C₁₂ alkanoyloxy, N-(C₁-C₁₂)alkylcarboxamido, N,N-di-(C₁-C₁₂)carboxamido, N-(C₁-C₁₂) alkylcarbamoyloxy, N,N-di-(C₁-C₁₂)carbamoyloxy, and heterocycloalkyl or heteroaryl having from 1 to 3 rings, each ring having from 5 to 7 atoms with from 0-3 heteroatoms selected from N, O, and S, provided that at least one ring contains a heteroatom, where the substituents are selected from halo (F, Cl, Br, I), cyano, azido, nitro, hydroxy, mercapto, sulfonamido, ureido, thioureido, carboxamido, carbamoyloxy, formyloxy, formyl, C₁-C₄alkyl, C₁-C₄alkoxy, phenyl, and phenoxy, optionally, R¹ and R when bonded to adjacent carbon atoms may join to form an unsubstituted or substituted aryl ring, where the substituents are selected from halo (F, Cl, Br, I), cyano, azido, nitro, hydroxy, mercapto, sulfonamido, ureido, thioureido, carboxamido, carbamoyloxy, formyloxy, formyl, C₁-C₄alkyl, C₁-C₄alkoxy, phenyl, and phenoxy;
Y¹, Y, Y³, Y⁴ and Y⁵ are independently selected from CH, CR¹, CR, and N;
X is selected from O, S, =NCN, and =NO(C₁-C₃)alkyl;
Z¹ is selected from CH₂, NH, S, and O;
Q¹ is selected from the group consisting of
(A) an amino group selected from
-NH₂,
-NR³H,
-NR³R⁴, and
-NR³R⁴R⁵,
where R³, R⁴, and R⁵ are independently selected from
(i) Cyano,
(ii) an optionally substituted radical selected from
(a) -NR⁶R⁷,
(b) -C(=NR⁸)-NR⁶R⁷,
(c) -N=CR⁹-NR⁶R⁷,
(d) -NR¹⁰-CR⁹=NR⁸, and
(e) -NR¹⁰-C(=NR⁸)-NR⁶R⁷,
where each R⁶, R⁷, R⁸, R⁹, and R¹⁰ is independently selected from
hydrogen,
C₁ -C₄ alkoxy,
C₁-C₄ alkyl, and
halo(F, Cl, Br, I)-C₁-C₄-alkyl,
(iii) optionally substituted C₁-C₁₂ alkyl,
(iv) optionally substituted C₃-C₁₂ cycloalkyl,
(v) optionally substituted C₆-C₁₄ aryl,
(vi optionally substituted C₁-C₆ alkyl-C₆-C₁₄ aryl,
(vii) optionally substituted C₁-C₈ alkoxy,
(viii) optionally substituted C₆-C₁₄ aryloxy, and
(xi) optionally substituted C₁-C₈ alkoxycarbonyl,
where the substituents are one to three R¹¹, each R¹¹ independently selected from
(a) optionally substituted C₆-C₁₂ aryloxy,
(b) optionally substituted C6-C12 arylamino,
(c) optionally substituted C₆-C₁₂ aroyl,
(d) optionally substituted C₁-C₈ alkoxy,
(e) optionally substituted C₁-C₈ alkoxyalkyl,
(f) optionally substituted C₁-C₈ alkoxyalkyloxy,
(g) optionally substituted C₁-C₈ alkoxycarbonyl,
(h) optionally substituted C₁C₈ alkylcarbonyl,
(i) optionally substituted C₁-C₈ aralkylcarbonyl,
(j) optionally substituted C₁-C₈ alkylthiocarbonyl,
(k) optionally substituted C₆-C₁₂ aralkylthiocarbonyl,
(I) optionally substituted C₂-C₈ alkoxythiocarbonyl,
(m) optionally substituted C₆-C₁₂ aryl,
(n) optionally substituted C₁-C₄ alkanoylamino,
(o) optionally substituted C₁-C₆ alkoxycarbonyl-C₀-C₆alkylamino,
(p) optionally substituted C₁-C₈ alkylsulfonylamino,
(q) optionally substituted C₆-C₁₀ aralkylsulfonylamino,
(r) optionally substituted C₆-C₁₀ aralkyl,
(s) optionally substituted C₆-C₁₀ alkaryl,
(t) optionally substituted C₁-C₈ alkylthio,
(u) optionally substituted C₆-C₁₀ aralkylthio,
(v) optionally substituted C₁-C₈ alkylsulfinyl,
(w) optionally substituted C₆-C₁₀ aralkylsulfinyl,
(x) optionally substituted C₁-C₈ alkylsulfonyl,
(y) optionally substituted C₆-C₁₀ aralkylsulfonyl,
(z) optionally substituted C₁-C₈ alkylaminosulfonyl,
(aa) optionally substituted C₆-C₁₀ aralkylsulfonylamino,
(ab) optionally substituted 5- or 6-member heterocycle containing 1-4 hetero atoms selected from N, O, and S, and
(ac) optionally substituted C₂-C₈ alkenyl,
where the substituents are one to three R¹, each R¹ independently selected from
nitro,
amino,
C₁-C₈ alkylamino,
di-(C₁-C₈) alkylamino,
amidino,
aminomethyleneimino,
imino,
imino-C₁-C₄ alkyl,
iminomethyleneamino,
guanidino,
C₆-C₁₀ arylamino,
C₁-C₈ acylamino,
C₁-C₄ alkylsulfonamino,
azido,
cyano,
hydroxy,
hydroxy-C₁-C₈-alkyl,
C₁-C₈-alkoxy,
phenyloxy,
C₁-C₈ alkanoyloxy,
C₁-C₈ alkanoyl,
C₆-C₁₂ aroyl,
benzamido,
phenyl,
halo(F, Cl, Br, I),
halo-C₁-C₈-alkyl, and
C₁-C₈-alkyl,
(ad) aminosulfonyl,
(ae) oxo,
(af) thio,
(ag) thiocarbonyl,
(ah) hydroxy,
(ai) mercapto,
(aj) formyl,
(ak) formyloxy,
(al) carboxy,
(am) amino,
(an) ureido,
(ao) amidino,
(ap) guanidino,
(aq) aminomethyleneimino,
(ar) imino,
(as) glycyl,
(at) phthalimido,
(au) succinimido,
(av) morpholino
(aw) C₃-C₇ cycloalkyl, and
(ax) halo(F, Cl, Br, I),
optionally R³ and R⁴ taken together may form optionally substituted
tetramethylene,
pentamethylene,
3-oxopentamethylene, and
3-azapentamethylene,
where the substituents are selected from one to three R¹,
(B) an amidino group selected from
-C(=NH)-NH₂,
-C(=NH)-NHR³,
-C(=NR⁴)-NHR³,
-C(=NH)-NR³R⁴, and
-C(=NR⁵)-NR³R⁴,
where R³, R⁴, and R⁵ are defined above,
(C) an aminoalkyleneimino group selected from
-N=CH-NH₂,
-N=CH-NHR³,
-N=CH-NR³R⁴, and
-N=CR⁵-NR³R⁴,
where R³, R⁴, and R⁵ are defined above,
(D) an iminoalkyleneamino group selected from
-NH-CH=NH,
-NH-CH=NR³,
-NH-CR⁴=NR³, and
-NR⁵-CR⁴=NR³,
where R³, R⁴, and R⁵ are defined above,
(E) a guanidino group selected from
-NH-C(=NH)-NH₂,
-NH-C(=NH)-NR³H,
-NH-C(=NH)-NR³R⁴,
-NH-C(=NR⁵)-NR³R⁴,
-NR³-C(=NR³)-NR³R⁴,
-NR³-C(=NH)-NR³R⁴,
-NR³-C(=NR³)-NH₂,
-NR³-C(=NH)-NH₂,
-NR³-C(=NR³)-NHR⁴, and
-NR³-C(=NH)-NHR⁴,
where R³, R⁴, and R⁵ are defined above;
(F) an optionally substituted saturated heterocyclic group selected from 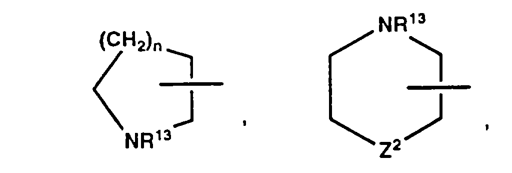 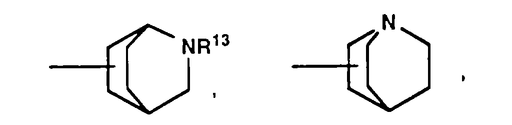 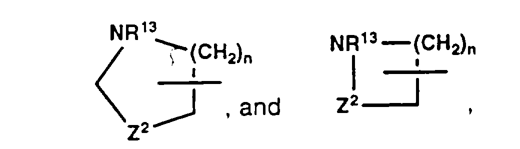
where (1) n is 0, 1, 2, or 3, (2) R¹³ is selected from R⁶, -CR⁹=NR⁸, -CR⁹(=NR⁸)-NR⁶R⁷,-C(=NR⁸)-NR⁶R⁷, -N=CR⁹-NR⁶R⁷, -NR¹⁰-CR⁹=NR⁸, and -NR¹⁰-(C=NR⁸)-NR⁶R⁷ where R⁶-R¹⁰ are defined above, (3) Z is O, S, or NR¹³, and (4) the substituents are independently one to three R¹;
(G) an optionally substituted unsaturated (nonaromatic) heterocyclyl selected from 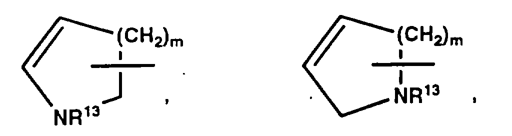 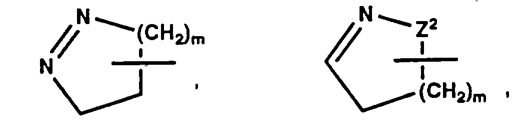 and 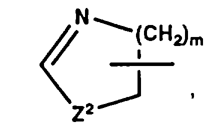
where (1) m is 1, 2, or 3, (2) Z and R¹³ are defined above, and (3) the substituents are independently one to three R¹;
(H) an optionally substituted unsaturated (aromatic) heterocyclyl selected from 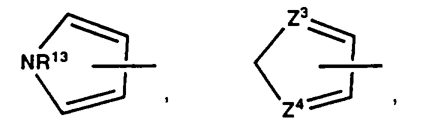 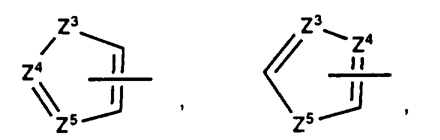 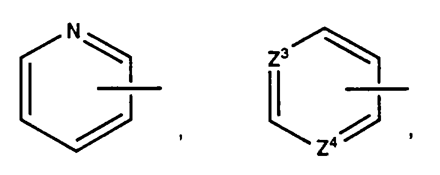 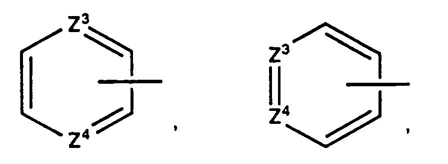 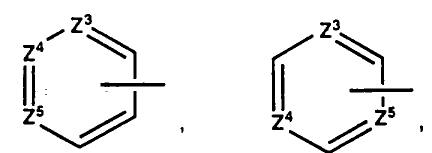 and 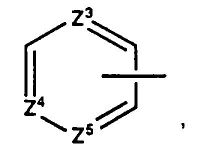
where (1) Z³, Z⁴, and Z⁵ are selected from O, S, N, and NH, provided that at least one Z³, Z⁴, or Z⁵ is N or NH, (2) R¹³ is defined above, and the substituents are independently one to three R¹,
(1) an optionally substituted bicycloheterocyclic group selected from; 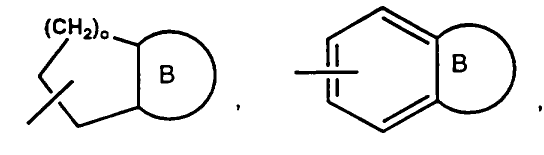 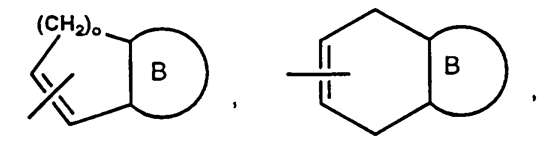 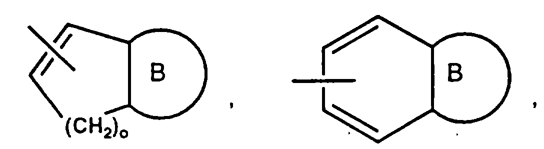 and 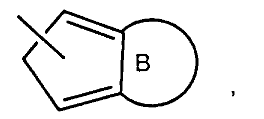 where the partial structure 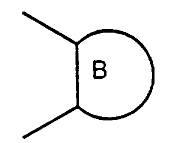 represents 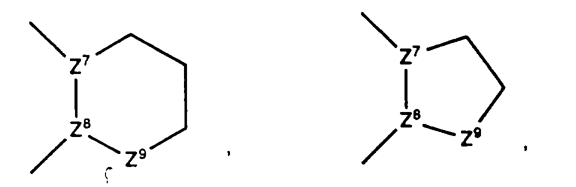 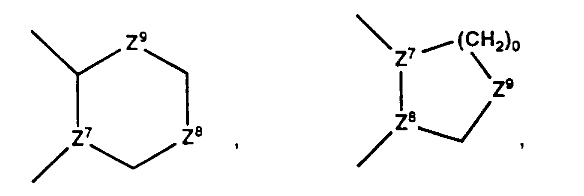 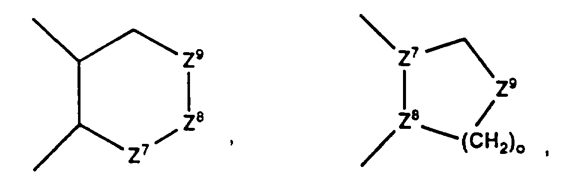 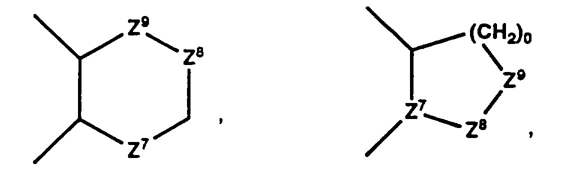 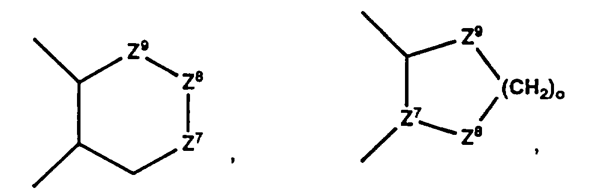 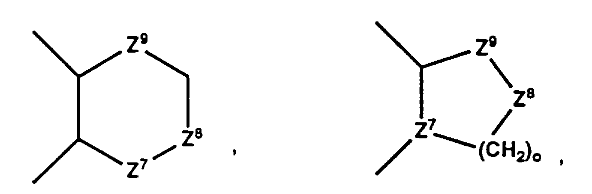 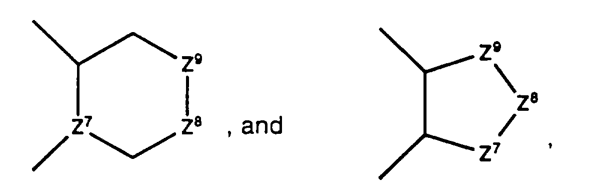 where Z⁷, Z⁸ and Z9 are independently selected from 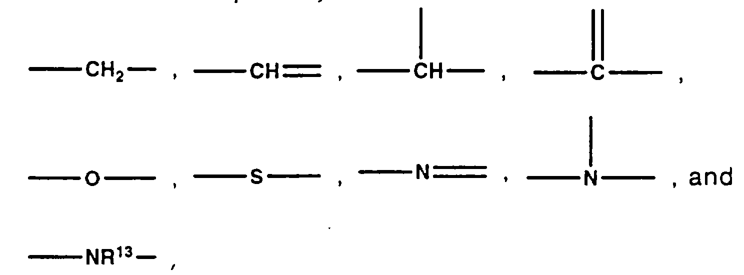 provided that at least one Z⁷, Z⁸, or Z⁹ is 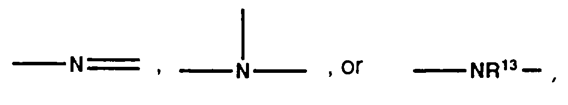 where (1) o is 0, 1, or 2, (2) R¹³ is defined above, and (3) the substituents are independently one to three R¹;
L¹ is an optionally substituted bivalent radical selected from the group consisting of
(A) C₃-C₇-alkylene,
(B) C₃-C₇-cycloalkylene,
(C) C₃-C₇-alkenylene,
(D) C₃-C₇-alkadienylene,
(E) C₃-C₇-alkynylene,
(F) C₄-C₇-alkadiynylene,
(G) C₄-C₇-alkenynylene,
(H) C₆-C₁₄-arylene,
(I) C₆-C₁₄-aryl-C₂-C₄-alkynylene,
(J) C₁-C₃-alkyl-C₆-C₁₄-aryl-C₂-C₄-alkynylene,
(K) C₆-C₁₄-aryl-C₂-C₄-alkenylene,
(L) C₁-C₃-alkyl-C₆-C₁₄-arylene,
(M) C₁-C₃-alkyl-C₆-C₁₄-aryl-C₂-C₄-alkenylene,
(N) C₆-C₁₄-aryl-C₁-C₃-alkylene,
(O) C₆-C₁₄-aryl-C₁-C₃-alkyloxyene,
(P) C₁-C₂-alkyl-C₆-C₁₄-aryl-C₁-C₂-alkylene,
(Q) C₁-C₃-alkyloxy-C₆-C₁₄-arylene,
(R) C₂-C₆-alkyloxyene,
(S) C₁-C₅-alkyloxy-C₁-C₅-alkylene,
(T) C₆-C₁₀-aryloxyene,
(U) C₆-C₁₀-aryloxy-C₁-C₅-alkylene,
(V) C₂-C₆-alkylthioene,
(W) C₁-C₅-alkylthio-C₁-C₅-alkylene,
(X) C₆-C₁₀-arylthioene,
(Y) C₆-C₁₀-arylthio-C₁-C₅-alkylene,
(Z) C₁-C₅-alkylsulfoxide-C₁-C₅-alkylene,
(AA) C₁-C₅-alkylsulfone-C₁-C₅-alkylene,
(AB) 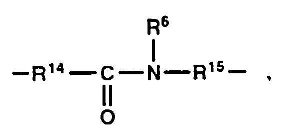
(AC) 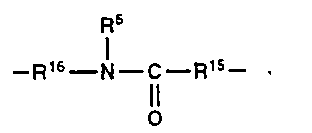
(AD) 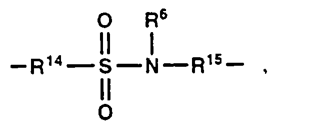
(AE) 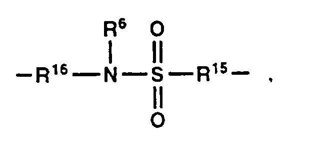
(AF) 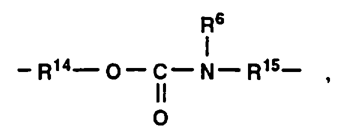
(AG) 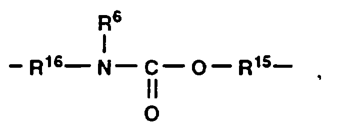
(AH) 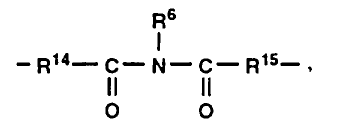
(AI) 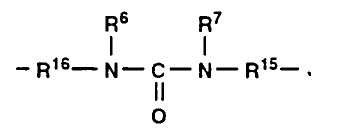
(AJ) 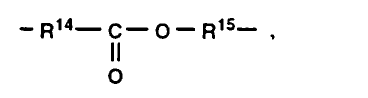
(AK) 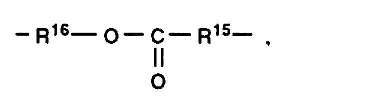
(AL) 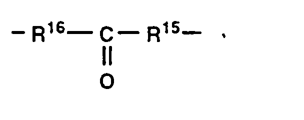
(AM) 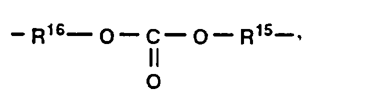
(AN) 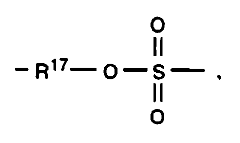
(AO) 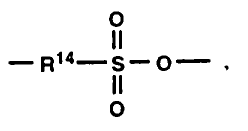
(AP) 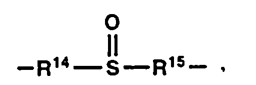
(AQ) 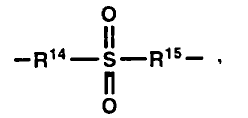
(AR) 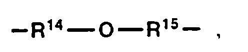
(AS) 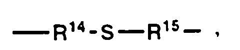
(AT) 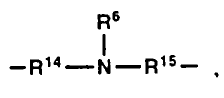 and
(AU) 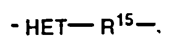
where
R¹⁴ is selected from
a chemical bond,
C₁-C₈-alkyl,
C₃-C₇-cycloalkyl
C₂-C₅-alkenyl,
C₃-C₅-alkynyl,
C₆-C₁₀-aryl,
C₁-C₃-alkyl-C₆-C₁₂-aryl,
C₁-C₂-alkyl-C₆-C₁₀-aryl-C₁-C₂-alkyl,
C₆-C₁₀-aryl-C₁C₂-alkyl, and
C₆-C₁₀-aryloxy-C₁-C₂-alkyl,
R¹⁵ is selected from
a chemical bond,
C₁-C₄-alkyl,
C₂-C₄-alkenyl,
C₂-C₄-alkynyl,
C₆-C₁₀-aryl, and
C₁-C₃-alkyl-C₆-C₁₂-aryl,
R¹⁶ is selected from
a chemical bond,
C₁-C₅-alkyl,
C₃-C₇-cycloalkyl
C₃-C₅-alkenyl,
C₃-C₅-alkynyl,
C₆-C₁₀-aryl,
C₁-C₃-alkyl-C₆-C₁₂-aryl, and
C₆-C₁₀-aryl-C₁-C₂-alkyl,
R¹⁷ is selected from
C₃-C₄-alkenyl,
C₃-C₄-alkynyl,
C₆-C₁₀-aryl, and
benzyl, and
HET is a heterocycle having from 5-14 atoms in from 1-3 cycle(s), each cycle having from 1-3 heteroatoms selected from N, O, and S,
where the substituents are selected from one to three R¹;
L is an optionally substituted bivalent radical selected from the group consisting of 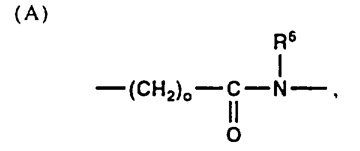 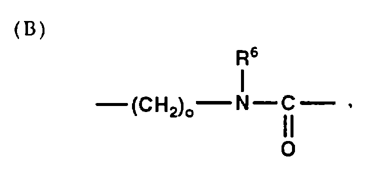 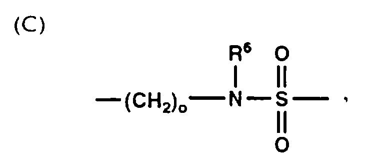 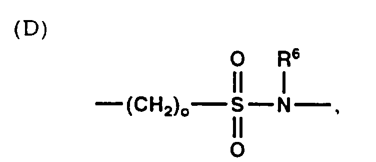 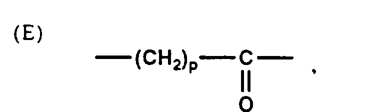 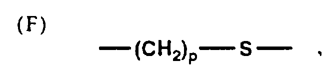 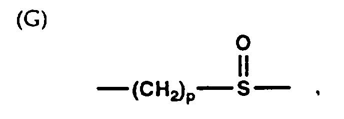 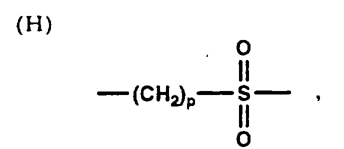 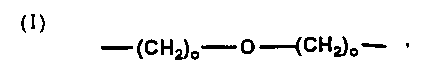 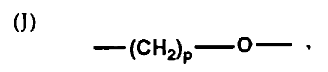 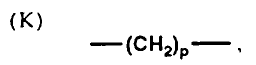 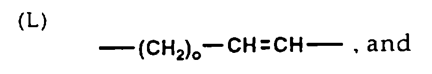 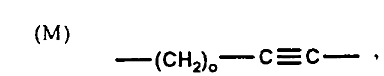
where
o is 1 or 2,
p is 1, 2, 3 or 4,
R⁶ is defined above, and
where the substituents are selected from one to three R¹;
T-U-G is selected from the group consisting of 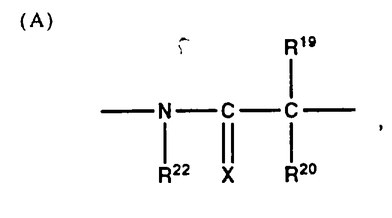 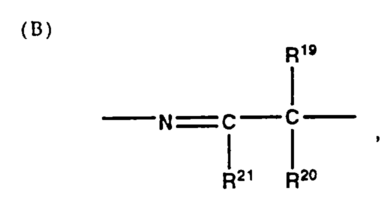 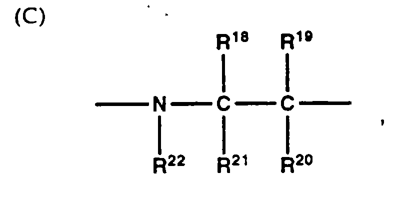 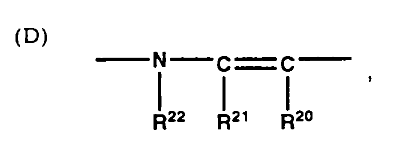 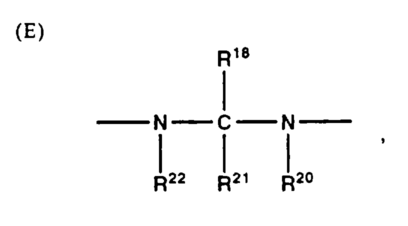 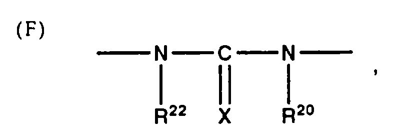 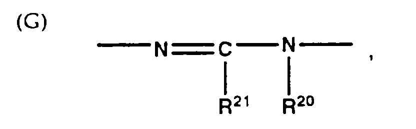 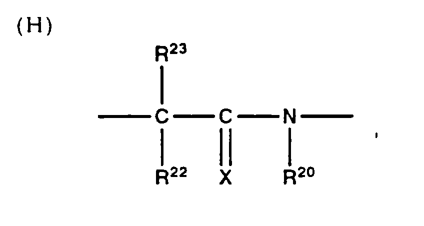 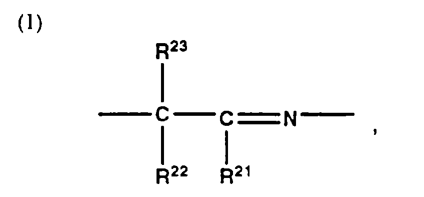 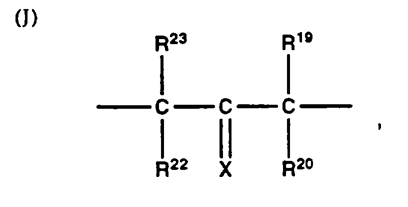 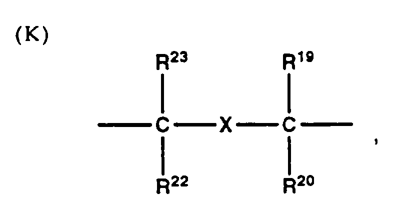 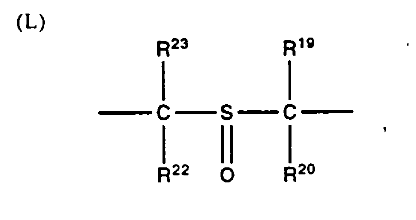 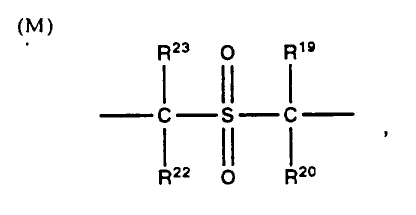 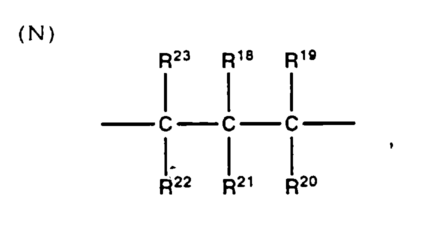 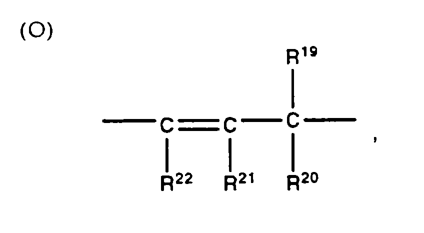 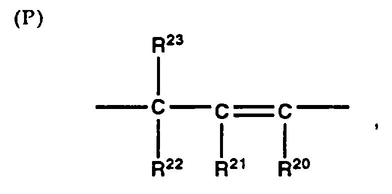 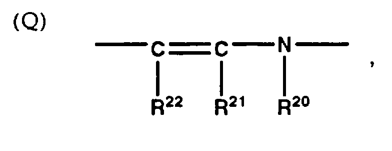 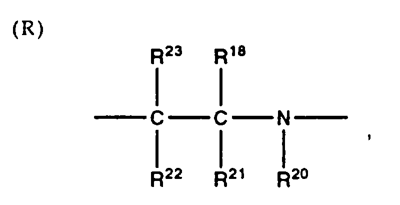 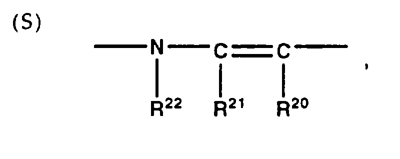 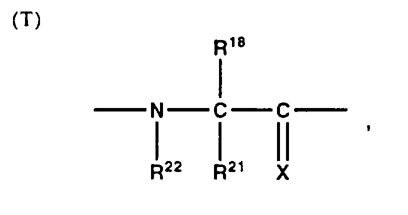 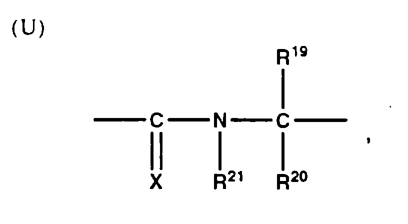 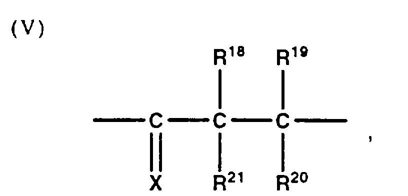 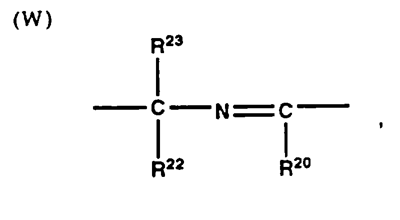 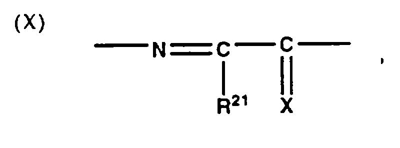 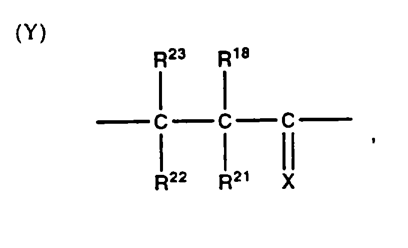 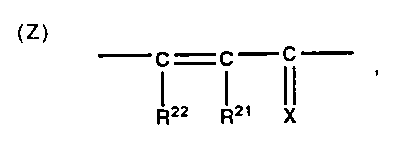 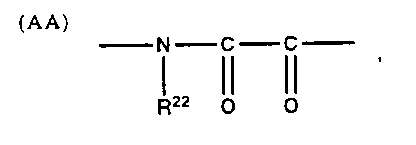 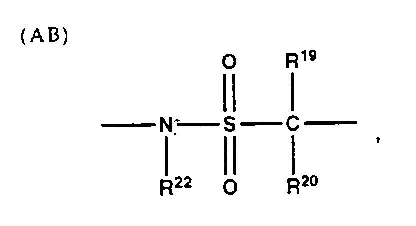 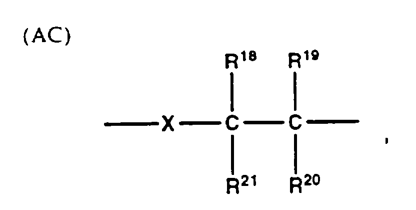 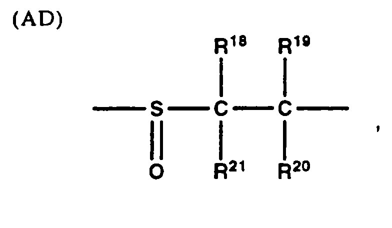 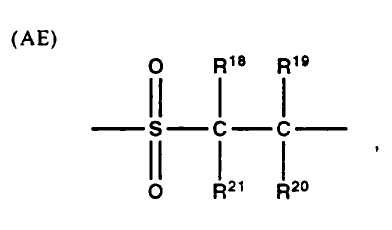 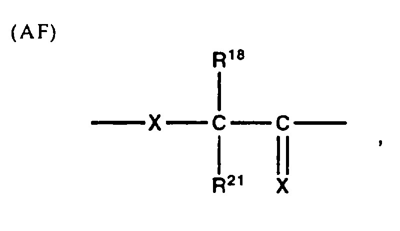 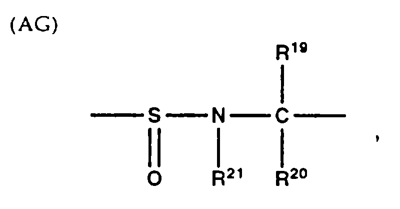 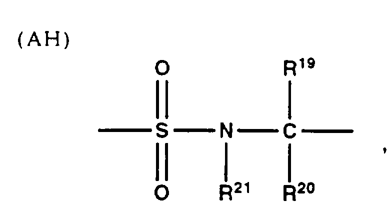 and 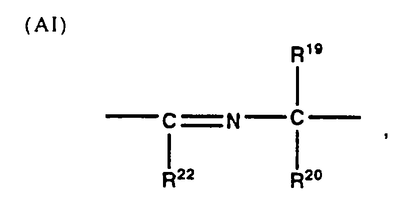
where
X is O or S;
R¹⁸ and R³ are independently selected from the group consisting of
(i) hydrogen,
(ii) optionally substituted C₁-C₁₂ alkyl,
(iii) optionally substituted C₃-C₁₂ alkenyl,
(iv) optionally substituted C₃-C₁₄ cycloalkyl,
(v) optionally substituted C₁-C₁₂ alkyl-C₆-C₁₄-aryl,
(vi) optionally substituted C₆-C₁₄ aryl,
(vii) optionally substituted C₁-C₄ alkylphenyl, and
(viii) optionally substituted C₁-C₁₂ alkoxy,
R¹⁹ and R⁰ are independently selected from
(i) hydrogen,
(ii) halo(F, Cl, Br, I),
(iii) C₁-C₄ alkoxy,
(iv) C₁-C₄ alkyl,
(v) phenyl,
(vi) benzyl, and
(vii) halo(F, Cl, Br, I)-C₁-C₄-alkyl,
R¹ and R are independently selected from the group consisting of
(i) hydrogen,
(ii) optionally substituted C₁-C₁₂-alkyl,
(iii) optionally substituted C₆-C₁₄-aryl,
(iv) optionally substituted C₃-C₁₄-cycloalkyl,
(v) optionally substituted C₁-C₁₂-alkyl-C₆-C₁₄-aryl, and
(vi) optionally substituted C₁-C₁₂-alkyl-C₃-C₁₄-cycloalkyl,
where the substituents are selected from
(a) halo (F, Cl, Br, I),
(b) nitro,
(c) hydroxy,
(d) carboxy,
(e) tetrazole,
(f) hydroxamate,
(g) sulfonamide,
(h) trifluoroimide,
(i) phosphonate,
(j) C₁-C₆-alkyl,
(k) C₆-C₁₄-aryl,
(I) benzyl,
(m) C₃-C₁₄-cycloalkyl,
(n) COR⁴
where R⁴ is selected from the group
C₁-C₈ alkoxy,
C₃-C₁₂ alkenoxy,
C₆-C₁₂ aryloxy,
C₁-C₆-alkyl-C₆-C₁₂-aryloxy,
di-C₁-C₈-alkylamino-C₁-C₈-alkoxy,
acylamino-C₁-C₈-alkoxy selected from the group
acetylaminoethoxy,
nicotinoylaminoethoxy, and
succinamidoethoxy,
C₁-C₈-alkoyloxy-C₁-C₈-alkoxy, and
C₆-C₁₂ aryl-C₁-C₈-alkoxy where the aryl group is unsubstituted or substituted with one to three of the groups
nitro,
halo (F, Cl, Br, I),
C₁-C₄-alkoxy,
amino,
hydroxy,
hydroxy-C₂-C₈-alkoxy, and
dihydroxy-C₃-C₈-alkoxy, and
(o) CONR⁵R⁶
where R⁵ and R⁶ are independently selected from hydrogen,
C₁-C₁₀-alkyl,
C₃-C₁₀-alkenyl,
C₆-C₁₄-aryl,
C₁-C₆-alkyl-C₆-C₁₀-aryl,
optionally R⁵ and R⁶ taken together may form
trimethylene,
tetramethylene,
pentamethylene, and
3-oxopentamethylene, and
(vii) Q-L³-
where Q is selected from
hydrogen, and
Q¹, and
L³ is selected from
a chemical bond,
L¹, and
L;
D is selected from the group consisting of
R, and
-(C=O)-Xaa, where Xaa is one to three D or L α-amino acid residues;
W is -R⁷-w where
R⁷ is selected from
(a) a covalent bond,
(b) substituted or unsubstituted methylene, and
(c) substituted or unsubstituted ethylene,
where the substituents are selected from
(i) nitro,
(ii) halo(F, Cl, Br, I),
(iii) C₁-C₆ alkyl,
(iv) halo(F, Cl, Br, I)-C₁,C₆ alkyl, and
(v) substituted or unsubstituted phenyl
where the phenyl substituents are selected from
(1) C₁-C₆ alkyl,
(2) C₁-C₆ alkoxy,
(3) halo(F, Cl, Br, I), and
(4) CF₃;
w is selected from
(a) -COR⁸,
(b) -SO₃R³¹,
(c) -NHSO₂R³,
(d) -PO(OR³¹)₂,
(e) -SO₂NHR³,
(f) -CONHOR³¹,
(g) -C(OH)R³³PO(OR³³)₂,
(h) -CN,
(i) -SO₂ NH-heterocycle where the heterocycle is a 5- or 6-member aromatic ring containing 1 to 3 heteroatoms selected from O, N, and S and where the heterocycle is unsubstituted or substituted with one or two substituents selected from the group
(i) -OH,
(ii) -SH,
(iii) -(C₁-C₄alkyl),
(iv) -C₁-C₄alkoxyl,
(v) CF₃,
(vi) halo(F, Cl, Br, I),
(vii) NO₂,
(viii) -COOH,
(ix) -COO-(C₁-C₄alkyl),
(x) -NH₂,
(xi) -NH(C₁-C₄alkyl), and
(xii) -N(C₁-C₄alkyl)₂,
(j) -CH₂SO₂NH-heterocycle,
(k) -SO₂NHCOR³³,
(l) -CH₂SO₂NHCOR³ʼ
(m) -CONHSO₂R³³,
(n) -CH₂CONHSO₂R³³,
(o) -NHCONHSO₂R³³,
(p) -NHSO₂NHCOR³³,
(q) -CONHNHSO₂CF₃,
(r) -CON(OH)R³¹,
(s) -CONHCOCF₃,
(t) -CONHSO₂R⁸,
(u) -CONHSO₂R⁹,
(v) -CONHSO₂R³⁰, 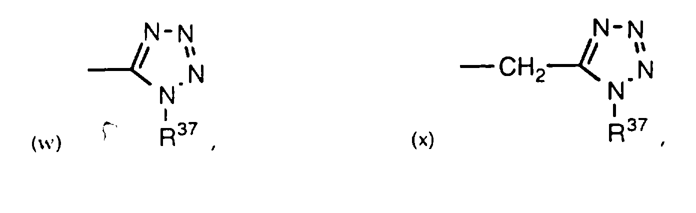 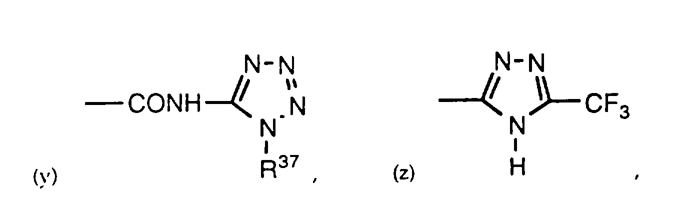 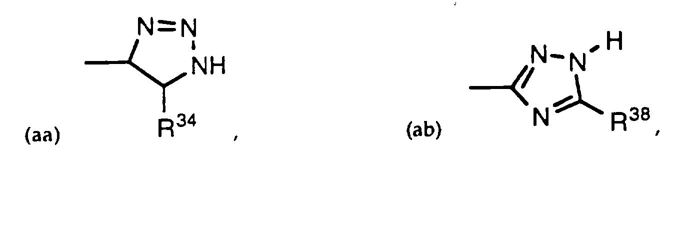 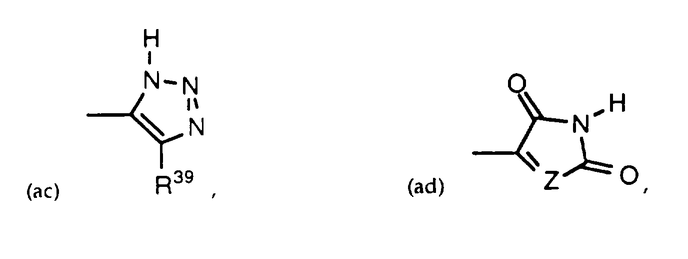 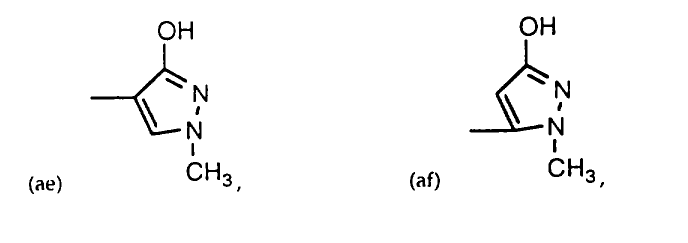 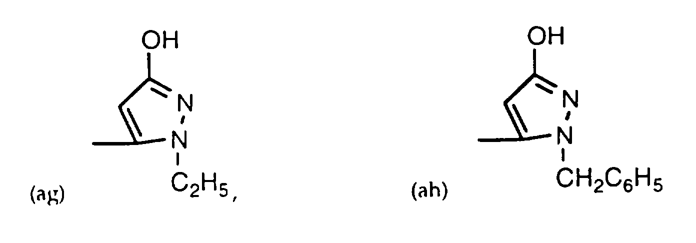 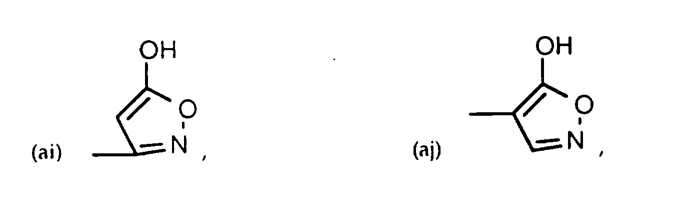 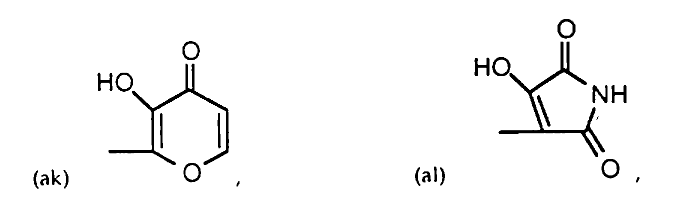 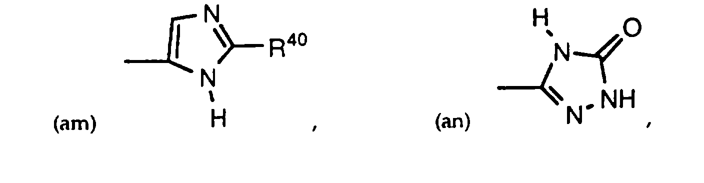 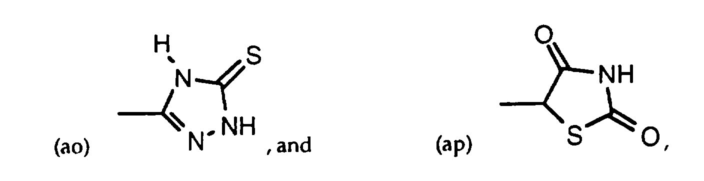
R⁸ is selected from the group consisting of
(a) hydroxy,
(b) C₁-C₈-alkoxy,
(c) C₃-C₁₂-alkenoxy,
(d) C₆-C₁₂-aryloxy,
(e) C₁-C₆-alkyl-C₆-C₁₂-aryloxy,
(f) di-C₁-C₈-alkylamino-C₁-C₈-alkoxy,
(g) acylamino-C₁-C₈-alkoxy selected from the group
(i) acetylaminoethoxy,
(ii) nicotinoylaminoethoxy, and
(iii) succinamidoethoxy,
(h) C₁-C₈-alkoyloxy-C₁-C₈-alkoxy,
(i) C₆-C₁₂-aryl-C₁-C₈-alkoxy,
where any aryl groups are unsubstituted or substituted with one to three of the groups
(i) nitro,
(ii) halo (F, Cl, Br, I),
(iii) C₁-C₄-alkoxy, and
(iv) amino,
(j) hydroxy-C₂-C₈-alkoxy,
(k) dihydroxy-C₃-C₈-alkoxy, and
(l) NR⁹R³⁰;
R⁹ and R³⁰ are independently selected from the group
(a) hydrogen
(b) C₁-C₈-alkyl,
(c) C₃-C₈-alkenyl,
(d) C₆-C₁₂-aryl,
(e) C₆-C₁₂-aryl-C₁-C₈-alkyl,
where any aryl groups are unsubstituted or substituted with one to three of the groups
(i) nitro,
(ii) halo (F, Cl, Br, I),
(iii)C₁-C₄-alkoxy, and
(iv) amino;
R³¹ is selected from the group consisting of
(a) hydrogen,
(b) C₁-C₆ alkyl,
(c) halo(F, Cl, Br, I)-C₁-C₆ alkyl,
(d) phenyl,
(e) benzyl, and
(f) -CH₂-O-COCH₃;
R³ is selected from the group consisting of
(a) hydrogen,
(b) benzyl and
(c) -CH(R³⁵)-O-C(O)R³⁵;
R³³ is selected from the group consisting of
(a) aryl,
(b) heteroaryl,
(c) (C₃-C₇)-cycloalkyl,
(d) (C₁-C₄)-alkyl, unsubstituted or substituted with a substituent selected from the group consisting of
(i) aryl,
(ii) heteroaryl,
(iii) -OH,
(iv) -SH,
(v) (C₁-C₄)-alkyl,
(vi) (C₁-C₄)-alkoxy,
(vii) (C₁-C₄)-alkylthio,
(viii) -CF_{3,}
(ix) halo (F, Cl, Br, I),
(x) -NO₂,
(xi) -CO₂H,
(xii) CO₂-(C₁-C₄)-alkyl,
(xiii) -NH₂,
(xiv) -N[(C₁-C₄)-alkyl]₂,
(xv) -NH[(C₁-C₄)-alkyl],
(xvi) -PO₃H, and
(xvii) PO(OH)(C₁-C₄)-alkoxy, and
(e) (C₁-C₄)-perfluoroalkyl;
R³⁴ is selected from the group consisting of
(a) -CN,
(b) -NO₂,
(c) -COOR³¹,
(d) C₁-C₆-perfluoroalkyl, and
(e) CF₃;
R³⁵ is independently selected from the group consisting of
(a) hydrogen,
(b) optionally substituted (C₁-C₆)-alkyl,
(c) optionally substituted (C₂-C₆)-alkenyl,
(d) optionally substituted (C₂-C₆)-alkynyl, and
(e) optionally substituted(C₃-C₈)-cycloalkyl,
where the substituents are selected from the group
(i) OH,
(ii) (C₁-C₄)-alkoxy,
(iii) CO₂R³³,
(iv) OCOR³³,
(v) CONHR³³,
(vi) CON(R³³)₂,
(vii) N(R³³)C(O)R³³,
(viii) NH₂,
(ix) (C₁-C₄)-alkylamino,
(x) di[(C₁-C₄)-alkyl]amino,
(xi) aryl, and
(xii) heteroaryl,
(f) -C(O)-aryl,
(g) -NO,
(h) halo(Cl, Br, I, F),
(i) -OH,
(j) -OR³⁶,
(k) -(C₁-C₄)-perfluoroalkyl,
(I) -SH,
(m) -S(O)₁₋₂ (C₁-C₄)-alkyl,
(n) -CO₂R³³,
(o) -SO₃H,
(p) -NR³³R³⁶,
(q) -NR³³C(O)R³⁶,
(r) -NR³³COOR³,
(s) -SO₂NHR³,
(t) -SO₂NR³³R³³,
(u) -NHSO₂R³,
(v) -C(O)NHSO₂R³,
(w) aryl,
(x) heterocycle,
(y) morpholin-4-yl,
(z) CONH₂, and
(aa) 1H-tetrazol-5-yl;
R³⁶ is selected from the group
(a) hydrogen, and
(b) (C₁-C₄)-alkyl unsubstituted or substituted with
(i) NH₂,
(ii) NH[(C₁-C₄)-alkyl],
(iii) N[(C₁-C₄)-alkyl]₂,
(iv) CO₂H,
(v) CO₂(C₁-C₄)-alkyl,
(vi) OH,
(vii) SO₃H, and
(viii) SO₂NH₂;
R³⁷ is selected from the group consisting of
(a) hydrogen,
(b) (C₁-C₆)-alkyl,
(c) (C₂-C₆)-alkenyl,
(d) (C₁-C₆)-alkoxyalkyl,
(e) -CH₂-O-COCH_{3,} and
(f) -CH₂-phenyl, where the phenyl is unsubstituted or substituted with a substituent selected from
(i) -NO₂,
(ii) -NH₂,
(iii) -OH, and
(iv) -OCH₃;
R³⁸, R³⁹, and R⁴⁰ are each independently selected from
(a) hydrogen,
(b) Cl,
(c) CN,
(d) NO₂,
(e) CF₃,
(f) C₂F₅,
(g) C₃F₇,
(h) CHF₂,
(i) CH₂F,
(j) CO₂CH₃,
(k) CO₂C₂H₅,
(l) SO₂CH₃,
(m) SO₂CF₃, and
(n) SO₂C₆F₅,
wherein Z is selected from O, S, NR⁴¹ and CH₂;
R⁴¹ is selected from hydrogen, CH₃, and CH₂C₆H₅; and
pharmaceutically acceptable salts thereof.

2. The compound of Claim 1 represented by formula II: 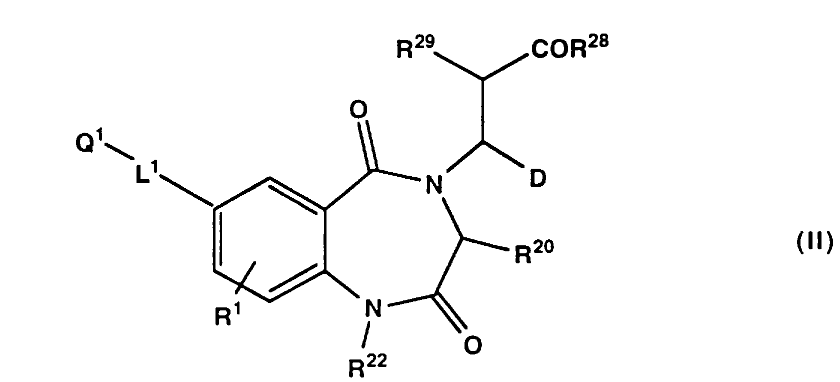
where
R¹ is one to three groups independently selected from hydrogen, halo(F, Cl, Br, I), cyano, carboxamido, carbamoyloy, formyloxy, formyl, azido, nitro,ureido, thioureido, hydroxy, mercapto, sulfonamido, and an optionally substituted radical selected from C₁-C₁₂ alkyl, C₂-C₁₂ alkenyl, C₃-C₁₂ alkynyl, C₃-C₁₂ cycloalkyl, C₆-C₁₄ aryl, C₆-C₁₀ aryl-C₁-C₈ alkyl, C₁-C₁₂ alkyloxy, C₆-C₁₄ aryloxy, and C₁-C₁₂ acylamino, where the substituents are selected from halo(F, Cl, Br, I), cyano, azido, nitro, hydroxy, mercapto, sulfonamido, ureido, thioureido, carboxamido, carbamoyloxy, formyloxy, formyl, C₁-C₄ alkyl, C₁-C₄ alkoxy, phenyl, and phenoxy;
Q¹ is selected from the group consisting of
(A) an amino group selected from
-NH₂,
-NR³H,
-NR³R⁴, and
-NR³R⁴R⁵,
where R³, R⁴, and R⁵ are independently selected from (i) an optionally substituted radical selected from (a) -NR⁶R⁷, (b) -C(=NR⁸)-NR⁶R⁷, (c) - N=CR⁹-NR⁶R⁷, (d) -NR¹⁰-CR⁹=NR⁸, and (e) -NR¹⁰-C(=NR⁸)-NR⁶R⁷ where each R⁶, R⁷, R⁸, R⁹, and R¹⁰ is independently selected from hydrogen, C₁-C₄ alkoxy, C₁-C₄ alkyl, and halo(F, Cl, Br, I)-C₁-C₄-alkyl, (ii) optionally substituted C₁-C₁₂ alkyl, (iii) optionally substituted C₃-C₁₂ cycloalkyl, (iv) optionally substituted C₆-C₁₄ aryl, (v) optionally substituted C₁-C₆ alkyl-C₆-C₁₄-aryl, (vi) optionally substituted C₁-C₈ alkoxy, and (vii) optionally substituted C₆-C₁₄ aryloxy, where the substituents are one to three R¹¹, each R¹¹ independently selected from (a) optionally substituted C₆-C₁₂ aryloxy, (b) optionally substituted C₆-C₁₂ arylamino, (c) optionally substituted C₆-C₁₂ aroyl, where the substituents are one to three R¹, each R¹ independently selected from nitro, amino, C₁-C₈ alkylamino, di-(C₁-C₈) alkylamino, amidino, aminomethyleneimino, imino, imino-C₁-C₄ alkyl, iminomethyleneamino, guanidino, C₆-C₁₀ arylamino, C₁-C₈ acylamino, C₁-C₄ alkylsulfonamino, azido, cyano, hydroxy, hydroxy-C₁-C₈-alkyl, C₁-C₈-alkoxy, phenyloxy, C₁-C₈ alkanoyloxy, C₁-C₈ alkanoyl, C₆-C₁₂ aroyl, benzamido, phenyl, halo(F, Cl, Br, I), halo-C₁-C₈-alkyl, and C₁-C₈-alkyl, (d) halo(F, Cl, Br, I), (e) hydroxy, (f) mercapto, (g) formyl, (h) formyloxy, (i) carboxy, (j) amino, (k) ureido, (I) amidino, (m) guanidino, (n) aminomethylimino, (o) imino, (p) glycyl, (q) phthalimido, (r) succinimido, (s) morpholino, and (t) C₃-C₇ cycloalkyl, optionally R³ and R⁴ taken together may form optionally substituted tetramethylene, pentamethylene, 3-oxopentamethylene, and 3-azapentamethylene, where the substituents are selected from one to three R¹,
(B) an amidino group selected from
-C(=NH)-NH₂,
-C(=NH)-NHR³,
-C(=NR⁴)-NHR³,
-C(=NH)-NR³R⁴, and
-C(=NR⁵)-NR³R⁴,
where R³, R⁴, and R⁵ are defined above,
(C) an aminoalkyleneamino group selected from
-N=CH-NH₂,
-N=CH-NHR³,
-N=CH-NR³R⁴, and
-N=CR⁵-NR³R⁴,
where R³, R⁴, and R⁵ are defined above,
(D) an iminoalkyleneamino group selected from
-NH-CH=NH,
-NH-CH=NR³,
-NH-CR⁴=NR³, and
-NR⁵-CR⁴=NR³,
where R³, R⁴, and R⁵ are defined above,
(E) guanidino group selected from
-NH-C(=NH)-NH₂,
-NH-C(=NH)-NR³H,
-NH-C(=NH)-NR³R⁴,
-NH-C(=NR⁵)-NR³R⁴,
-NR³-C(=NR³)-NR³R⁴,
-NR³-C(=NH)-NR³R⁴,
-NR³-C(=NR³)-NH₂,
-NR³-C(=NH)-NH_{2,}
-NR³-C(=NR³)-NHR⁴, and
-NR³-C(=NH)-NHR⁴,
where R³, R⁴, and R⁵ are defined above, and
(F) an optionally substituted saturated heterocyclic group selected from 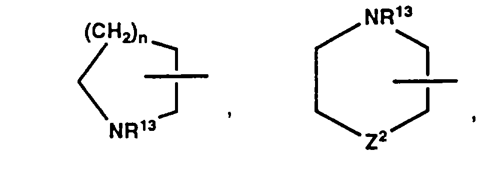 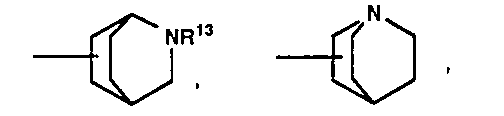 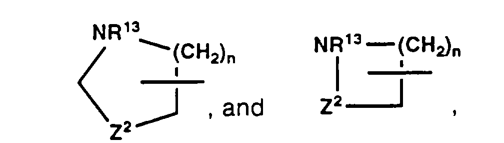
where (1) n is 0, 1, 2, or 3, (2) R¹³ is selected from; R⁶, -CR⁹=NR⁸,-CR⁹(=NR⁸)-NR⁶R⁷, -C(=NR⁸)-NR⁶R⁷, -N=CR⁹-NR⁶R⁷, -NR¹⁰-CR⁹=NR⁸, and -NR¹⁰-(C=NR⁸)-NR⁶R⁷ where R⁶-R¹⁰ are defined above, (3) Z is O, S, or NR¹³, and (4) the substituents are independently one to three R¹;
L¹ is an optionally substituted bivalent radical selected from the group consisting of C₃-C₇-alkylene, C₃-C₇-cycloalkylene, C₃-C₇-alkenylene, C₄-C₇-cycloalkenylene, C₅-C₈-cycloalkadienylene, C₃-C₇-alkadienylene, C₃-C₇-alkynylene, C₄-C₇-alkenynylene, C₆-C₁₄-arylene, C₆-C₁₄-aryl-C₂-C₄-alkynylene, C₁-C₃-alkyl-C₆-C₁₄-aryl-C₂-C₄-alkynylene, C₆-C₁₄-aryl-C₂-C₄-alkenylene, C₁-C₃-alkyl-C₆-C₁₄-arylene, C₁-C₃-alkyl-C₆-C₁₄-aryl-C₂-C₄-alkenylene, C₆-C₁₄-aryl-C₁-C₃-alkylene, C₆-C₁₄-aryl-C₁-C₃-alkyloxyene, C₁-C₂-alkyl-C₆-C₁₄-aryl-C₁-C₂-alkylene, C₁-C₃-alkyloxy-C₆-C₁₄-arylene, C₂-C₈-alkyloxyene, C₁-C₅-alkyloxy-C₁-C₅-alkylene, C₆-C₁₀-aryloxyene, C₆-C₁₀-aryloxy-C₁-C₅-alkylene, C₆-C₁₀-arylthio-C₁-C₅-alkylene, 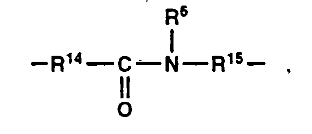 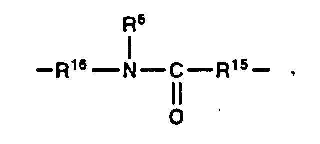 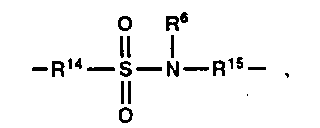 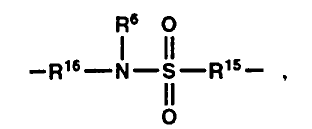 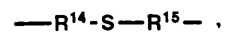 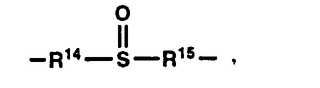 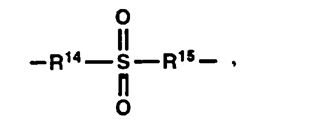 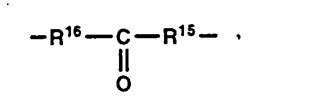 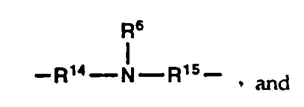 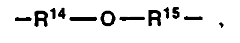
where R¹⁴ is selected from a chemical bond, C₁-C₈-alkyl, C₃-C₇-cycloalkyl, C₂-C₅-alkenyl, C₃-C₅-alkynyl, C₆-C₁₀-aryl, C₁-C₃-alkyl-C₆-C₁₂-aryl, C₁-C₂-alkyl-C₆-C₁₀-aryl-C₁-C₂-alkyl, and C₆-C₁₀-aryl-C₁-C₂-alkyl, R¹⁵ is selected from a chemical bond, C₁-C₄-alkyl, C₂-C₄-alkenyl, C₂-C₄-alkynyl, and C₆-C₁₀-aryl, R¹⁶ is selected from a chemical bond, C₁-C₅-alkyl, C₃-C₇-cycloalkyl, C₃-C₅-alkenyl, C₃-C₅-alkynyl, C₆-C₁₀-aryl, C₁-C₃-alkyl-C₆-C₁₂-aryl, C₁-C₃-alkyl-C₆-C₁₂-aryl, and C₆-C₁₀-aryl-C₁-C₂-alkyl, where the substituents are selected from one to three R¹;
R⁰ is selected from the group hydrogen, halo(F, Cl, Br, I), C₁-C₄ alkoxy, C₁-C₄ -alkyl, phenyl, benzyl, and halo(F, Cl, Br, I)-C₁-C₄-alkyl;
R is selected from the group consisting of (i) hydrogen, (ii) optionally substituted C₁-C₁₂-alkyl, (iii) optionally substituted C₆-C₁₄-aryl, (iv) optionally substituted C₃-C₁₄-cycloalkyl, (v) optionally substituted C₁-C₁₂-alkyl-C₆-C₁₄-aryl, (vi) optionally substituted C₁-C₁₂-alkyl-C₃-C₁₄-cycloalkyl, where the substituents are selected from (a) halo (F, Cl, Br, I), (b) nitro, (c) hydroxy, (d) carboxy, (e) tetrazole, (f) hydroxamate, (g) sulfonamide, (h) trifluoroimide, (i) phosphonate, (j) C₁-C₆-alkyl, (k) C₆-C₁₄-aryl, (l) benzyl, (m) C₃-C₁₄-cycloalkyl, (n) COR⁴ where R⁴ is selected from the group C₁-C₈-alkoxy, C₃-C₁₂-alkenoxy, C₆-C₁₂-aryloxy, di-C₁-C₈-alkylamino-C₁-C₈-alkoxy, acylamino-C₁-C₈-alkoxy selected from the group acetylaminoethoxy, nicotinoylaminoethoxy, succinamidoethoxy, and pivaloyloxyethoxy, and C₆-C₁₂-aryl-C₁-C₈-alkoxy where the aryl group is unsubstituted or substituted with one to three of the groups nitro, halo (F, Cl, Br, I), C₁-C₄-alkoxy, amino, hydroxy, hydroxy-C₂-C₈-alkoxy, and dihydroxy-C₃-C₈-alkoxy, (o) CONR⁵R⁶ where R⁵ and R⁶ are independently selected from hydrogen, C₁-C₁₀-alkyl, C₃-C₁₀-alkenyl, C₆-C₁₄-aryl, C₁-C₆-alkyl-C₆-C₁₀-aryl, optionally R⁵ and R⁶ taken together may form trimethylene, tetramethylene, pentamethylene, and 3-oxopentamethylene, and (vii) Q-L³- where Q is selected from hydrogen, and Q¹, and L³ is selected from a chemical bond, L¹, and L, where L is an optionally substituted bivalent radical selected from the group consisting of 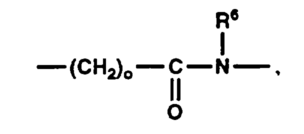 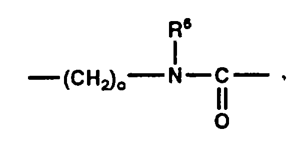 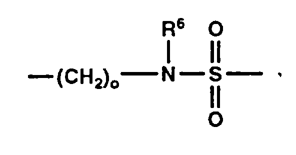 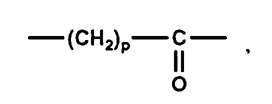 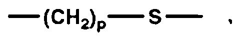 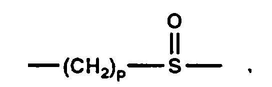 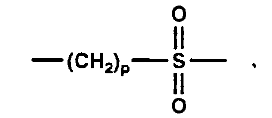 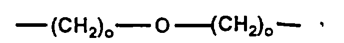 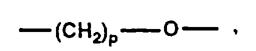 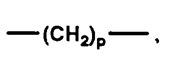 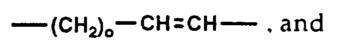 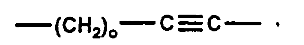
where o is 1 or 2, p is 1, 2, 3 or 4, R⁶ is defined above, and where the substituents are selected from one to three R¹;
D is selected from the group consisting of R, and -(C=O)-Xaa, where Xaa is one to three D or L α-amino acid;
R⁸ is selected from the group consisting of (i) hydroxy, (ii) C₁-C₈-alkoxy, (iii) C₃-C₁₂-alkenoxy, (iv) C₆-C₁₂-aryloxy, (v) C₁-C₆-alkyl-C₆-C₁₂-aryloxy, (vi) di-C₁-C₈-alkylamino-C₁-C₈-alkoxy, (vii) acylamino-C₁-C₈-alkoxy selected from the group (a) acetylaminoethoxy, (b) nicotinoylaminoethoxy, and (c) succinamidoethoxy, (viii) C₁-C₈-alkoyloxy-C₁-C₈-alkoxy, (ix) C₆-C₁₂-aryl-C₁-C₈-alkoxy where the aryl group is unsubstituted or substituted with one to three of the groups (a) nitro, (b) halo (F, Cl, Br, I), (c) C₁-C₄-alkoxy, and (d) amino, (x) hydroxy-C₂-C₈-alkoxy, (xi) dihydroxy-C₃-C₈-alkoxy, and (xii) NR⁹R³⁰ where R⁹ and R³⁰ are independently selected from the group (a) hydrogen, (b) C₁-C₈-alkyl, (c) C₃-C₈-alkenyl, (d) C₆-C₁₂-aryl where the aryl group is unsubstituted or substituted with one to three of the groups nitro, halo (F, Cl, Br, I), C₁-C₄-alkoxy, and amino, and (e) C₆-C₁₂-aryl-C₁-C₈-alkyl where the aryl group is unsubstituted or substituted with one to three of the groups nitro, halo (F, Cl, Br, I), and C₁-C₄-alkoxy;
R⁹ is one or two groups independently selected from the group (a) hydrogen, (b) C₁-C₈-alkyl, (c) C₃-C₈-alkenyl, (d) C₆-C₁₂-aryl, and (e) C₆-C₁₂-aryl-C₁-C₈-alkyl, where any aryl groups are unsubstituted or substituted with one to three of the groups (i) nitro, (ii) halo (F, Cl, Br, I), (iii)C₁-C₄-alkoxy, and (iv) amino; and
pharmaceutically acceptable salts thereof.

3. The compound of Claim 2 where -L¹- is selected from the group consisting of 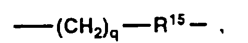 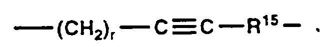 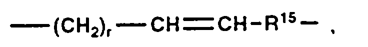 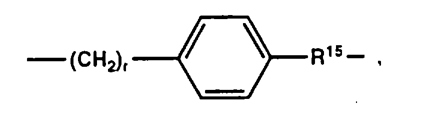 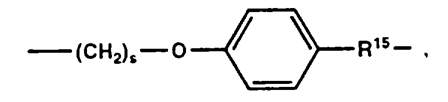 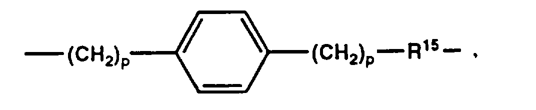 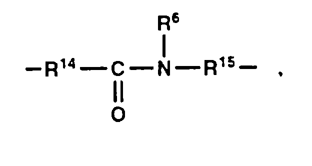 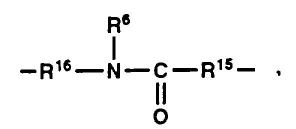 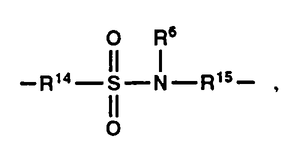 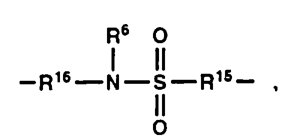 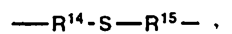 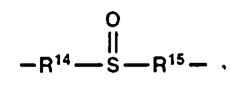 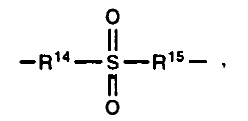 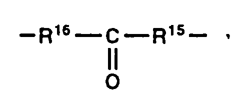 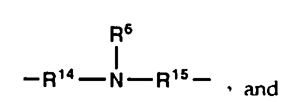 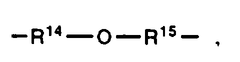
where
p is 1, 2, 3, or 4;
q is 3, 4, 5, 6 or 7;
r is 1, 2, 3, 4, or 5;
s is 2 or 3;
R¹⁴ is selected from
C₂-C₅-alkyl,
C₃-C₇-cycloalkyl,
C₂-C₅-alkenyl,
C₃-C₅-alkynyl,
C₆-C₁₀-aryl,
C₁-C₂-alkyl-C₆-C₁₂-aryl,
C₁-C₂-alkyl-C₆-C₁₀-aryl-C₁-C₂-alkyl,
C₆-C₁₀-aryl-C₁-C₂-alkyl, and
C₆-C₁₀-aryloxy-C₁-C₂-alkyl;
R¹⁵ is a chemical bond connecting L¹ to position 7 of the benzodiazepinedione;
R¹⁶ is selected from
C₂-C₅-alkyl,
C₃-C₇-cycloalkyl,
C₃-C₅-alkenyl,
C₃-C₅-alkynyl,
C₆-C₁₀-aryl,
C₁-C₃-alkyl-C₆-C₁₂-aryl, and
C₆-C₁₀-aryl-C₁-C₂-alkyl,
wherein R¹⁴ and R¹⁶ bond L¹ to Q¹.

4. The compound of Claim 3 where R is selected from the group consisting of
hydrogen,
optionally substituted C₁-C₆-alkyl,
where the substituents are selected from
amino,
hydroxy,
halo(F, Cl, Br, I),
carboxy, and
C₁-C₄-alkoxycarbonyl,
optionally substituted C₆-C₁₀-aryl, and optionally substituted C₆-C₁₂-aryl-C₁-C₄-alkyl, where the substituents on any aryl group are selected from
amino,
nitro,
halo(F, Cl, Br, I),
halo(F, Cl, Br, I)-C₁-C₆-alkyl,
C₁-C₆-alkoxy,
C₆-C₁₀-aryloxy,
amino-C₁-C₆-acylamino,
amino-C₁-C₆-acyl, and
guanidino-C₆-C₁₀-aroylamino.

5. The compound of Claim 3 where Q¹-L¹- is selected from the group consisting of 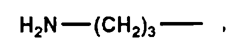 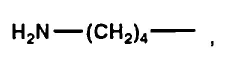 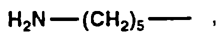 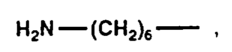 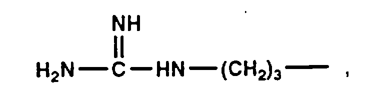 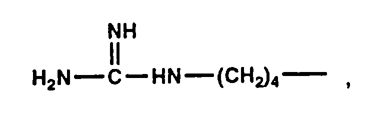 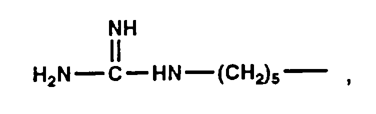 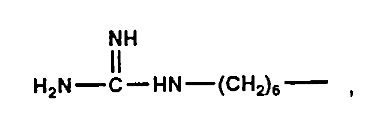 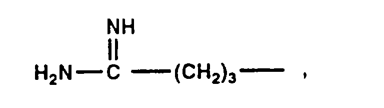 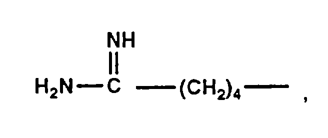 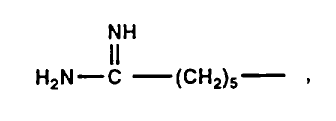 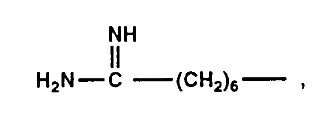 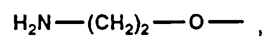 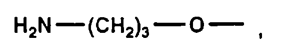 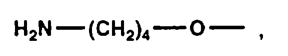 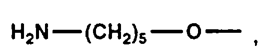 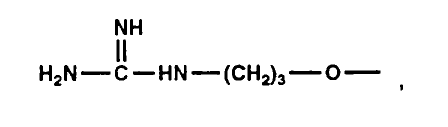 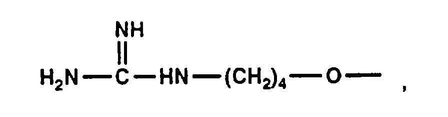 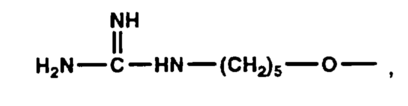 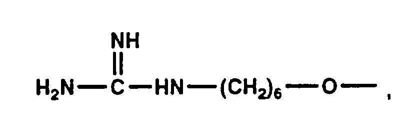 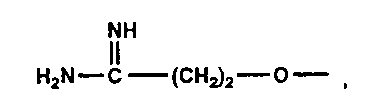 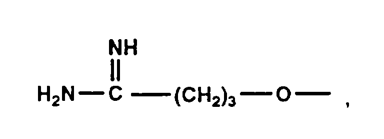 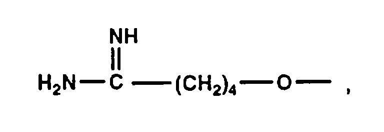 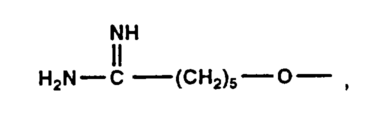 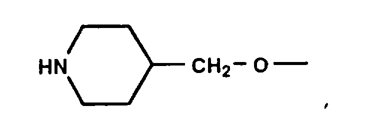 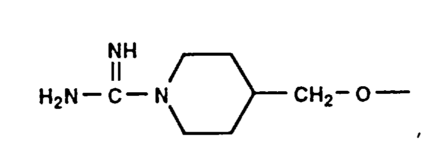 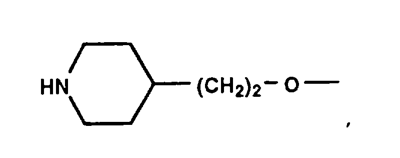 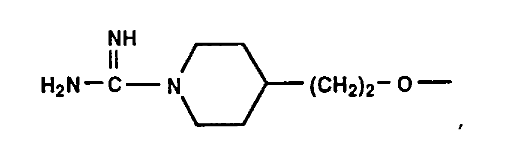 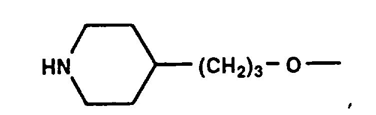 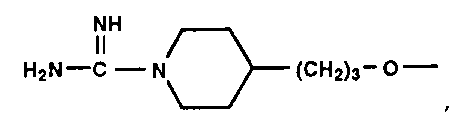 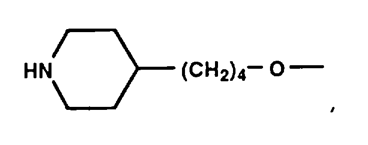 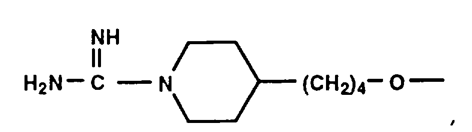 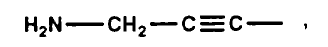 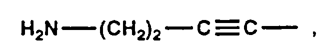 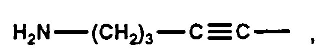 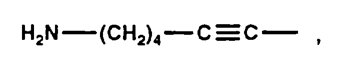 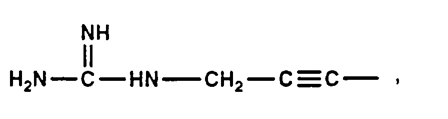 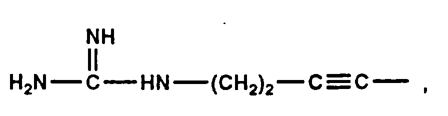 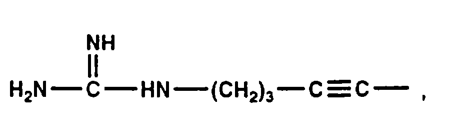 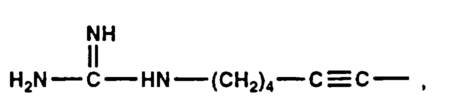 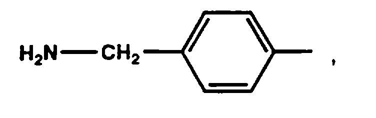 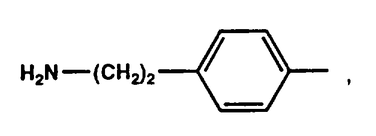 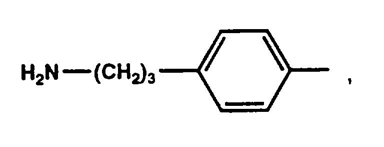 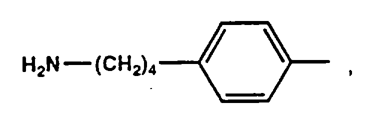 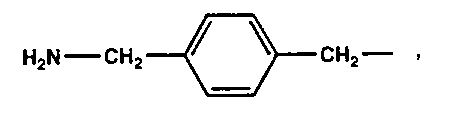 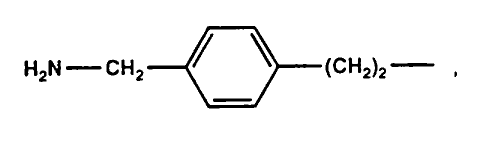 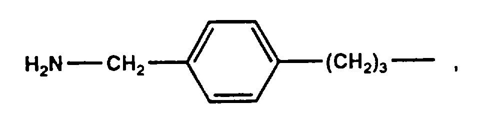 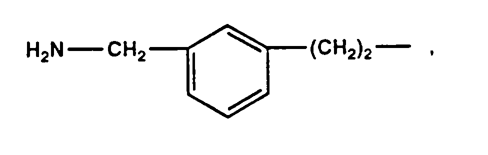 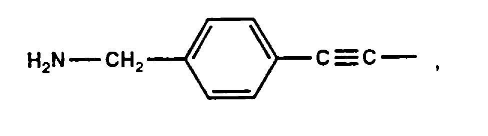 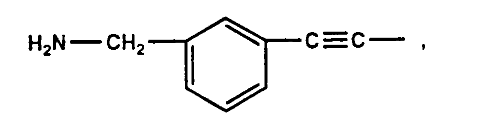 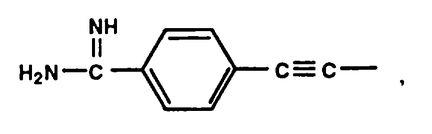 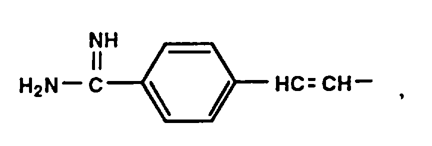 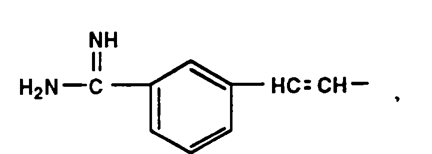 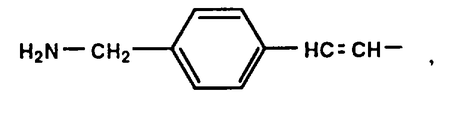 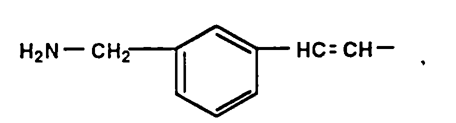 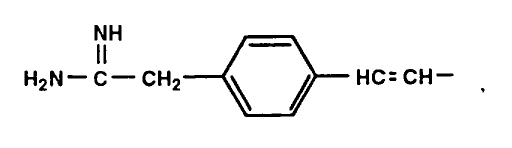 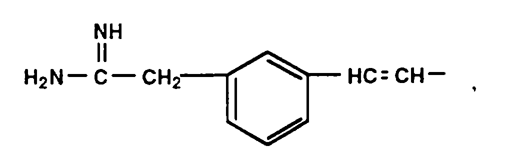 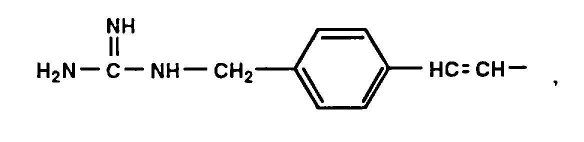 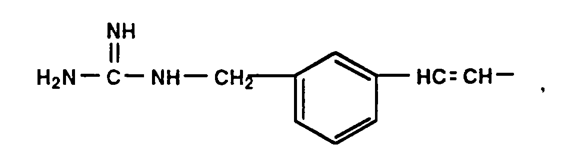 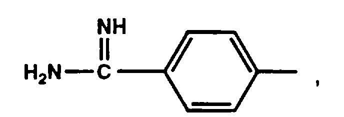 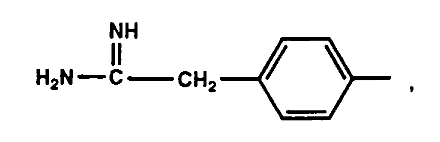 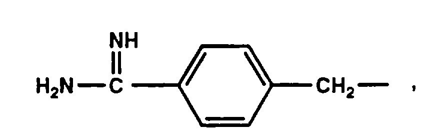 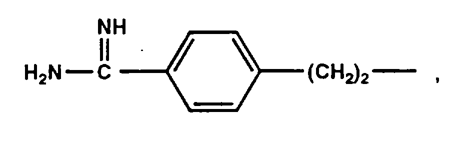 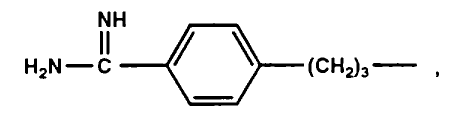 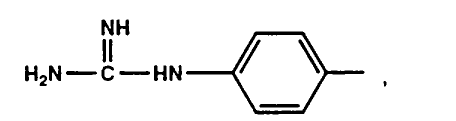 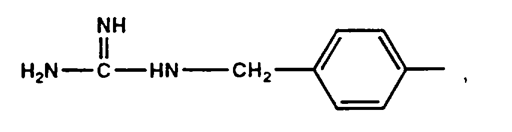 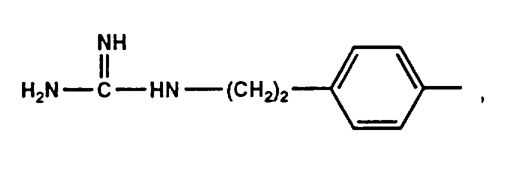 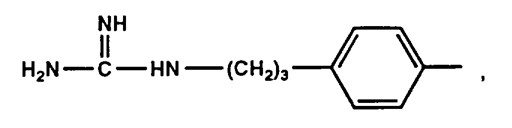 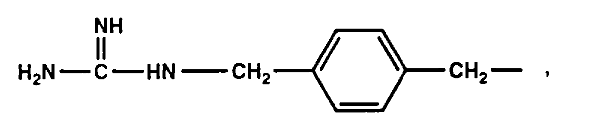 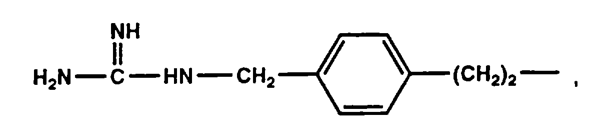 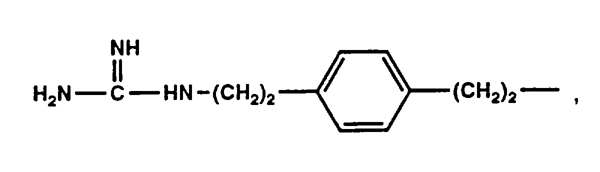 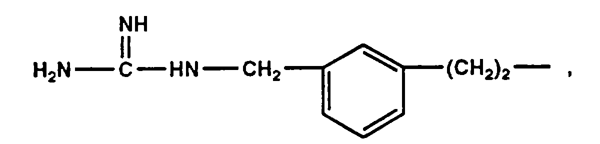 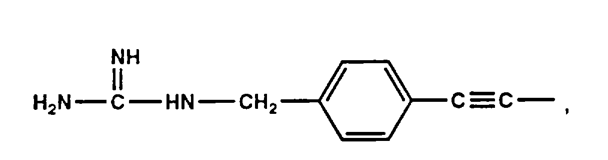 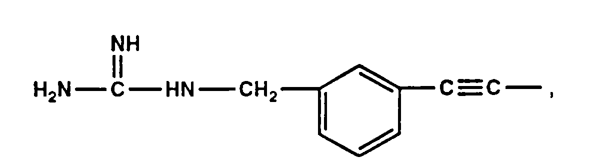 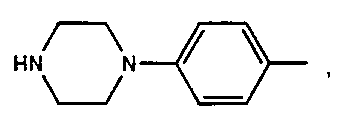 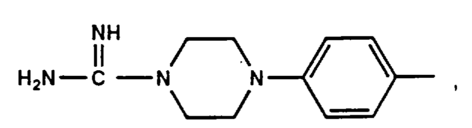 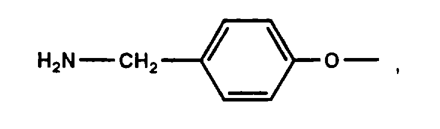 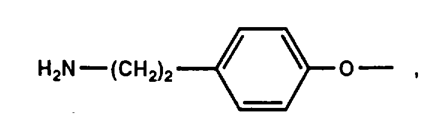 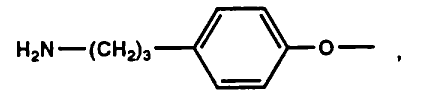 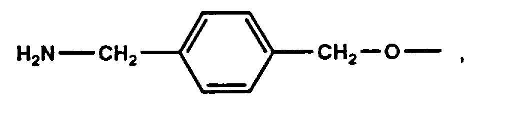 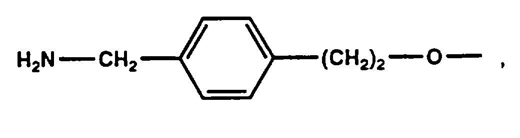 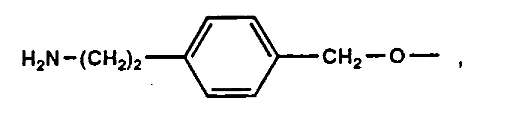 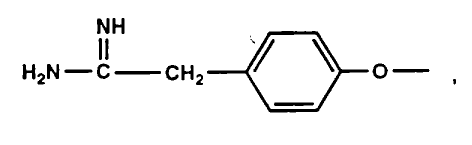 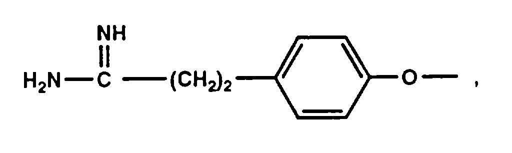 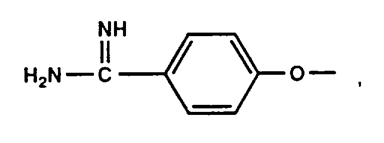 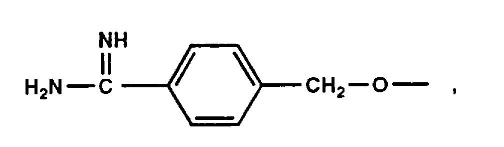 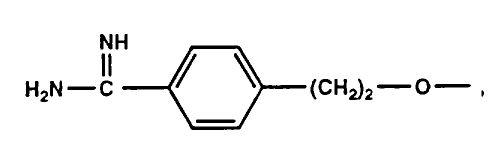 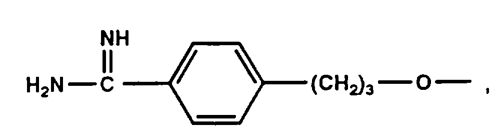 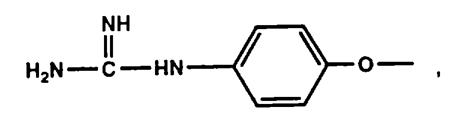 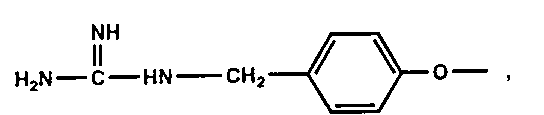 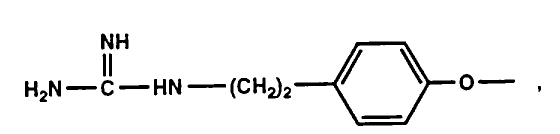 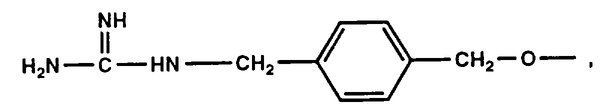 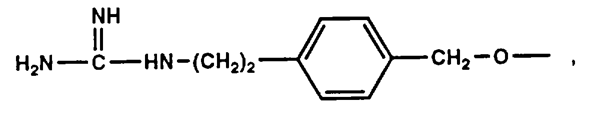 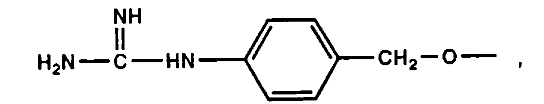 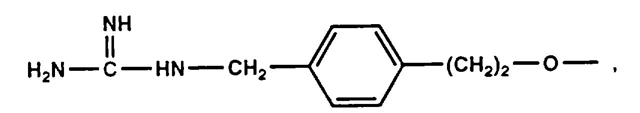 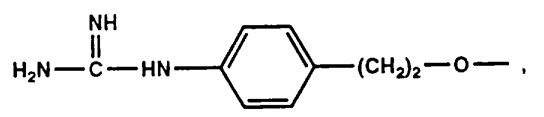 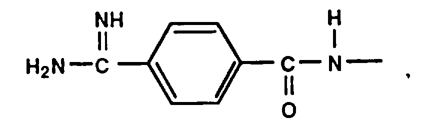 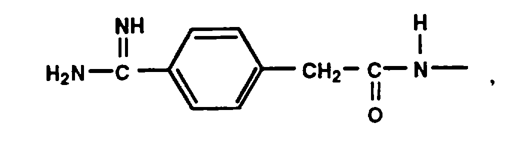 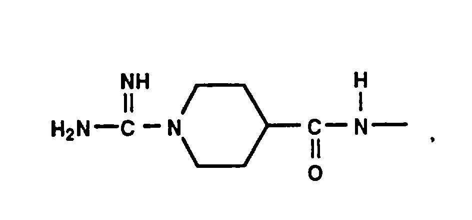 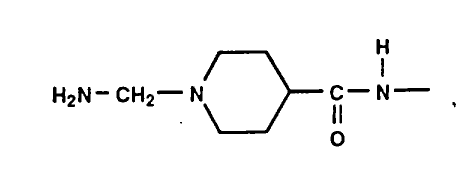 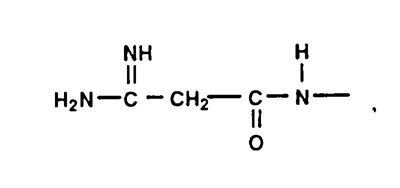 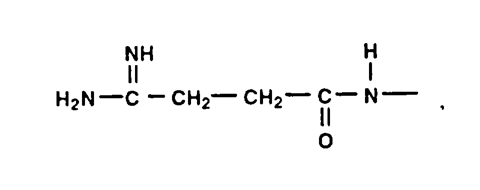 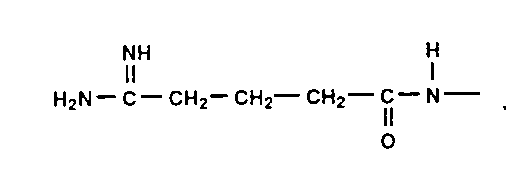 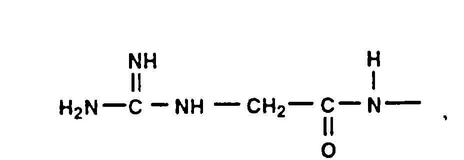 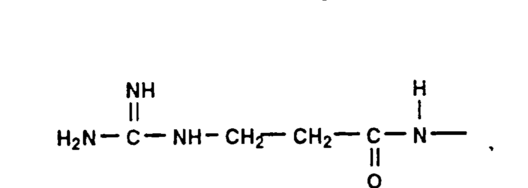 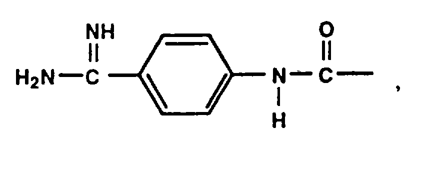 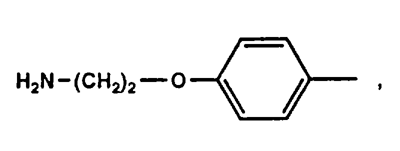 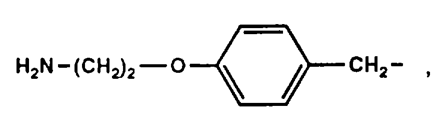 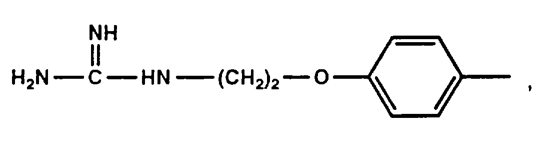 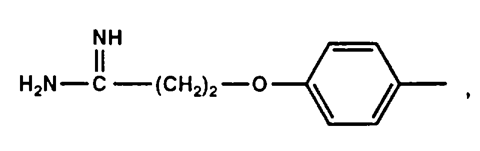 and

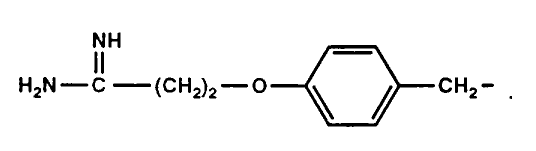

6. The compound of Claim 2 selected from the group consisting of;
1-(4-chlorophenyl)methyl-4-(2-carboxyethyl)-7-(4-(1-piperizin)-phenyl)-3,4-dihydro-1H-1,4-benzodiazapine-2,5-dione,
1-(diphenyl)methyl-4-(2-carboxyethyl)-7-(4-(1-piperizin)-phenyl)-3,4-dihydro-1H-1,4-benzodiazapine-2,5-dione,
1-(*m*-trifluromethyl)phenyl-4-(2-carboxyethyl)-7-(4-(1-piperizin)-phenyl)-3,4-dihydro-1H-1,4-benzodiazapine-2,5-dione,
1-methyl-4-(2-carboxyethyl)-7-(4-(1-piperizin)-phenyl)-3,4-dihydro-1H-1,4-benzodiazapine-2,5-dione,
1-(1-methyl)ethyl-4-(2-carboxyethyl)-7-(4-(1-piperizin)-phenyl)-3,4-dihydro-1H-1,4-benzodiazapine-2,5-dione,
1-(2-methyl)propyl-4-(2-carboxyethyl)-7-(4-(1-piperizin)-phenyl)-3,4-dihydro-1H-1,4-benzodiazapine-2,5-dione,
1-(4-chlorophenyl)methyl-4-(2-carboxyethyl)-7-(4-(2-aminoethoxy)-phenyl)-3,4-dihydro-1H-1,4-benzodiazapine-2,5-dione,
1-(diphenyl)methyl-4-(2-carboxyethyl)-7-(4-(2-aminoethoxy)-phenyl)-3,4-dihydro-1H-1,4-benzodiazapine-2,5-dione,
1-(*m*-trifluromethyl)phenyl-4-(2-carboxyethyl)-7-(4-(2-aminoethoxy)-phenyl)-3,4-dihydro-1H-1,4-benzodiazapine-2,5-dione,
1-methyl-4-(2-carboxyethyl)-7-(4-(2-aminoethoxy)-phenyl)-3,4-dihydro-1H-1,4-benzodiazapine-2,5-dione,
1-(1-methyl)ethyl-4-(2-carboxyethyl)-7-(4-(2-aminoethoxy)-phenyl)-3,4-dihydro-1H-1,4-benzodiazapine-2,5-dione,
1-(2-methyl)propyl-4-(2-carboxyethyl)-7-(4-(2-aminoethoxy)-phenyl)-3,4-dihydro-1H-1,4-benzodiazapine-2,5-dione,
1-(diphenyl)methyl-4-(2-carboxyethyl)-7-(6-aminohexyl)-3,4-dihydro-1H-1,4-benzodiazapine-2,5-dione,
1-(diphenyl)methyl-4-(2-carboxyethyl)-7-(6-aminohex-1-ynyl)-3,4-dihydro-1H-1,4-benzodiazapine-2,5-dione,
1-(diphenyl)methyl-4-(2-carboxyethyl)-7-(5-aminopentoxy)-3,4-dihydro-1H-1,4-benzodiazapine-2,5-dione,
1-(diphenyl)methyl-4-(2-carboxyethyl)-7-(5-guanidinopentyl)-3,4-dihydro-1H-1,4-benzodiazapine-2,5-dione,
1-(diphenyl)methyl-4-(2-carboxyethyl)-7-(4-guanidinobutoxy)-3,4-dihydro-1H-1,4-benzodiazapine-2,5-dione,
1-(diphenyl)methyl-4-(2-carboxyethyl)-7-(5-aminopent-1-ynyl)-3,4-dihydro-1H-1,4-benzodiazapine-2,5-dione,
1-(diphenyl)methyl-4-(2-carboxyethyl)-7-(5-guanidinopent-1-ynyl)-3,4-dihydro-1H-1,4-benzodiazapine-2,5-dione,
1-(diphenyl)methyl-4-(2-carboxyethyl)-7-(4-amidino)benzamido-3,4-dihydro-1H-1,4-benzodiazapine-2,5-dione,
I-(diphenyl)methyl-4-(2-carboxyethyl)-7-(4-amidino)benzyloxy-3,4-dihydro-1H-1,4-benzodiazapine-2,5-dione,
1-(diphenyl)methyl-4-(2-carboxyethyl)-7-[2-(4-amidinophenyl)]ethenyl-3,4-dihydro-1H-1,4-benzodiazapine-2,5-dione,
1-(diphenyl)methyl-4-(2-carboxyethyl)-7-[2-(4-amidinophenyl)]ethynyl-3,4-dihydro-1H-1,4-benzodiazapine-2,5-dione,
1-(4-chlorophenyl)methyl-4(2-carboxyethyl)-7-(6-aminohexyl)-3,4-dihydro-1H-1,4-benzodiazapine-2,5-dione,
1-(4-chlorophenyl)methyl-4-(2-carboxyethyl)-7-(5-aminopentoxy)-3,4-dihydro-1H-1,4-benzodiazapine-2,5-dione,
1-(4-chlorophenyl)methyl-4-(2-carboxyethyl)-7-(4-amidino)benzamido-3,4-dihydro-1H-1,4-benzodiazapine-2,5-dione,
1-(4-chlorophenyl)methyl-4-(2-carboxyethyl)-7-(4-amidino)benzyloxy-3,4-dihydro-1H-1,4-benzodiazapine-2,5-dione,
1-(4-chlorophenyl)methyl-4-(2-carboxyethyl)-7-[2-(4-amidinophenyl)]ethenyl-3,4-dihydro-1H-1,4-benzodiazapine-2,5 dione,
1-(4-chlorophenyl)methyl-4-(2-carboxyethyl)-7-[2-(4-amidinophenyl)]ethynyl-3,4-dihydro-1H-1,4-benzodiazapine-2,5-dione,
1-methyl-4-(2-carboxyethyl)-7-(4-amidino)benzamido-3,4-dihydro-1H-1,4-benzodiazapine-2,5-dione,
1-methyl-4-(2-carboxyethyl)-7-(4-amidino)benzyloxy-3,4-dihydro-1H-1,4-benzodiazapine-2,5-dione,
1-methyl-4-(2-carboxyethyl)-7-[2-(4-amidinophenyl)]ethenyl-3,4-dihydro-1H-1,4-benzodiazapine-2,5-dione,
1-methyl-4-(2-carboxyethyl)-7-[2-(4-amidinophenyl)]ethynyl-3,4-dihydro-1-1,4-benzodiazapine-2,5-dione,
I-(l-methyl)ethyl-4-(2-carboxyethyl)-7-(4-amidino)benzamido-3,4-dihydro-1H-1,4-benzodiazapine-2,5-dione,
1-(1-methyl)ethyl-4-(2-carboxyethyl)-7-(4-amidino)benzyloxy-3,4-dihydro-1H-1,4-benzodiazapine-2,5-dione,
1-(1-methyl)ethyl-4-(2-carboxyethyl)-7-[2-(4-amidinophenyl)]ethenyl-3,4-dihydro-1H-1,4-benzodiazapine-2,5-dione,
1-(2-methyl)ethyl-4-(2-carboxyethyl)-7-[2-(4-amidinophenyl)]ethynyl-3,4-dihydro-1H-1,4-benzodiazapine-2,5-dione,
1-(2-methyl)propyl-4-(2-carboxyethyl)-7-(4-amidino)benzamido-3,4-dihydro-1H-1,4-benzodiazapine-2,5-dione,
1-(2-methyl)propyl-4-(2-carboxyethyl)-7-(4-amidino)benzyloxy-3,4-dihydro-1H-1,4-benzodiazapine-2,5-dione,
1-(2-methyl)propyl-4-(2-carboxyethyl)-7-[2-(4-amidinophenyl)]ethenyl-3,4-dihydro-1H-1,4-benzodiazapine-2,5-dione,
1-(2-methyl)propyl-4-(2-carboxyethyl)-7-[2-(4-amidinophenyl)]ethynyl-3,4-dihydro-1H-1,4-benzodiazapine-2,5-dione,
1-(2,4-diflurophenyl)methyl-4-(2-carboxyethyl)-7-(4-(1-piperizin)-phenyl)-3,4-dihydro-1H-1,4-benzodiazapine-2,5-dione,
1-(2,4-diflurophenyl)methyl-4-(2-carboxyethyl)-7-(4-(2-aminoethoxy)-phenyl)-3,4-dihydro-1H-1,4-benzodiazapine-2,5-dione,
1-(2,4-diflurophenyl)methyl-4-(2-carboxyethyl)-7-(6-aminohexyl)-3,4-dihydro-1H-1,4-benzodiazapine-2,5-dione,
1-(2,4-diflurophenyl)methyl-4-(2-carboxyethyl)-7-(5-aminopentoxy)-3,4-dihydro-1H-1,4-benzodiazapine-2,5-dione,
1-(2,4-diflurophenyl)methyl-4-(2-carboxyethyl)-7-(4-amidino)benzamido-3,4-dihydro-1H-1,4-benzodiazapine-2,5-dione,
1-(2,4-diflurophenyl)methyl-4-(2-carboxyethyl)-7-(4-amidino)benzyloxy-3,4-dihydro-1H-1,4-benzodiazapine-2,5-dione,
1-(2,4-diflurophenyl)methyl-4-(2-carboxyethyl)-7-[2-(4-amidinophenyl)]ethenyl-3,4-dihydro-1H-1,4-benzodiazapine-2,5-dione,
1-(2,4-diflurophenyl)methyl-4-(2-carboxyethyl)-7-[2-(4-amidinophenyl)]ethynyl-3,4-dihydro-1H-1,4-benzodiazapine-2,5-dione,
1-(4-chlorophenyl)methyl-4-(2-carboxyethyl)-7-[2-(4-piperidinyl)ethyloxy]-3,4-dihydro-1H-1,4-benzodiazapine-2,5-dione,
1-(diphenyl)methyl-4-(2-carboxyethyl)-7-[2-(4-piperidinyl)ethyloxy]3,4-dihydro-1H-1,4-benzodiazapine-2,5-dione,
1-(*m*-trifluromethyl)phenyl-4-(2-carboxyethyl)-7-[2-(4-piperidinyl)ethyloxy]-3,4-dihydro-1H-1,4-benzodiazapine-2,5-dione,
1-methyl-4-(2-carboxyethyl)-7-[2-(4-piperidinyl)ethyloxy]-3,4-dihydro-1H-1,4-benzodiazapine-2,5-dione,
1-(1-methyl)ethyl-4-(2-carboxyethyl)-7-[2-(4-piperidinyl)ethyloxy]-3,4-dihydro-1H-1,4-benzodiazapine-2,5-dione,
1-(2-methyl)propyl-4-(2-carboxyethyl)-7-[2-(4-piperidinyl)ethyloxy]-3,4-dihydro-1H-1,4-benzodiazapine-2,5-dione,
1-(2,4-diflurophenyl)methyl-4-(2-carboxyethyl)-7-[2-(4-piperidinyl)ethyloxy]-3,4-dihydro-1H-1,4-benzodiazapine-2,5-dione,
1-(4-chlorophenyl)methyl-4-(3-butanoyl)-7-(4-(1-piperizin)-phenyl)-3,4-dihydro-1H-1,4-benzodiazapine-2,5-dione,
1-(diphenyl)methyl-4-(3-butanoyl)-7-(4-(1-piperizin)-phenyl)-3,4-dihydro-1H-1,4-benzodiazapine-2,5-dione,
1-(*m*-trifluromethyl)phenyl-4-(3-butanoyl)-7-(4-(1-piperizin)-phenyl)-3,4-dihydro-1H-1,4-benzodiazapine-2,5-dione,
1-methyl-4-(3-butanoyl)-7-(4-(1-piperizin)-phenyl)-3,4-dihydro-1H-1,4-benzodiazapine-2,5-dione,
1-(1-methyl)ethyl-4-(3-butanoyl)-7-(4-(1-piperizin)-phenyl)-3,4-dihydro-1H-1,4-benzodiazapine-2,5-dione,
1-(2-methyl)propyl-4-(3-butanoyl)-7-(4-(1-piperizin)-phenyl)-3,4-dihydro-1H-1,4-benzodiazapine-2,5-dione,
1-(4-chlorophenyl)methyl-4-(3-butanoyl)-7-(4-(2-aminoethoxy)-phenyl)-3,4-dihydro-1H-1,4-benzodiazapine-2,5-dione,
1-(diphenyl)methyl-4-(3-butanoyl)-7-(4-(2-aminoethoxy)-phenyl)-3,4-dihydro-1H-1,4-benzodiazapine-2,5-dione,
1-(*m*-trifluromethyl)phenyl-4-(3-butanoyl)-7-(4-(2-aminoethoxy)-phenyl)-3,4-dihydro-1H-1,4-benzodiazapine-2,5-dione,
1-methyl-4-(3-butanoyl)-7-(4-(2-aminoethoxy)-phenyl)-3,4-dihydro-1H-1,4-benzodiazapine-2,5-dione,
1-(1-methyl)ethyl-4-(3-butanoyl)-7-(4-(2-aminoethoxy)-phenyl)-3,4-dihydro-1H-1,4-benzodiazapine-2,5-dione,
1-(2-methyl)propyl-4-(3-butanoyl)-7-(4-(2-aminoethoxy)-phenyl)-3,4-dihydro-1H-1,4-benzodiazapine-2,5-dione,
1-(diphenyl)methyl-4-(3-butanoyl)-7-(6-aminohexyl)-3,4-dihydro-1H-1,4-benzodiazapine-2,5-dione,
1-(diphenyl)methyl-4-(3-butanoyl)-7-(6-aminohex-1-ynyl)-3,4-dihydro-1H-1,4-benzodiazapine-2,5-dione,
1-(diphenyl)methyl-4-(3-butanoyl)-7-(5-aminopentoxy)-3,4-dihydro-1H-1,4-benzodiazapine-2,5-dione,
1-(diphenyl)methyl-4-(3-butanoyl)-7-(5-guanidinopentyl)-3,4-dihydro-1H-1,4-benzodiazapine-2,5-dione,
1-(diphenyl)methyl-4-(3-butanoyl)-7-(4-guanidinobutoxy)-3,4-dihydro-1H-1,4-benzodiazapine-2,5-dione,
1-(diphenyl)methyl-4-(3-butanoyl)-7-(5-aminopent-1-ynyl)-3,4-dihydro-1H-1,4-benzodiazapine-2,5-dione,
1-(diphenyl)methyl-4-(3-butanoyl)-7-(5-guanidinopent-1-ynyl)-3,4-dihydro-1H-1,4-benzodiazapine-2,5-dione,
1-(diphenyl)methyl-4-(3-butanoyl)-7-(4-amidino)benzamido-3,4-dihydro-1H-1,4-benzodiazapine-2,5-dione,
1-(diphenyl)methyl-4-(3-butanoyl)-7-(4-amidino)benzyloxy-3,4-dihydro-1H-1,4-benzodiazapine-2,5-dione,
I-(diphenyl)methyl-4-(3-butanoyl)-7-[2-(4-amidinophenyl)]ethenyl-3,4-dihydro-1H-1,4-benzodiazapine-2,5-dione,
1-(diphenyl)methyl-4-(3-butanoyl)-7-[2-(4-amidinophenyl)]ethynyl-3,4-dihydro-1H-1,4-benzodiazapine-2,5-dione,
1-(4-chlorophenyl)methyl-4-(3-butanoyl)-7-(6-aminohexyl)-3,4-dihydro-1H-1,4-benzodiazapine-2,5-dione,
1-(4-chlorophenyl)methyl-4-(3-butanoyl)-7-(5-aminopentoxy)-3,4-dihydro-1H-1,4-benzodiazapine-2,5-dione,
1-(4-chlorophenyl)methyl-4-(3-butanoyl)-7-(4-amidino)benzamido-3,4-dihydro-1H-1,4-benzodiazapine-2,5-dione,
1-(4-chlorophenyl)methyl-4-(3-butanoyl)-7-(4-amidino)benzyloxy-3,4-dihydro-1H-1,4-benzodiazapine-2,5-dione,
1-(4-chlorophenyl)methyl-4-(3-butanoyl)-7-[2-(4-amidinophenyl)]ethenyl-3,4-dihydro-1H-1,4-benzodiazapine-2,5-dione,
1-(4-chlorophenyl)methyl-4-(3-butanoyl)-7-[2-(4-amidinophenyl)]ethynyl-3,4-dihydro-1H-1,4-benzodiazapine-2,5-dione,
1-methyl-4-(3-butanoyl)-7-(4-amidino)benzamido-3,4-dihydro-1H-1,4-benzodiazapine-2,5-dione,
1-methyl-4-(3-butanoyl)-7-(4-amidino)benzyloxy-3,4-dihydro-1H-1,4-benzodiazapine-2,5-dione,
1-methyl-4-(3-butanoyl)-7-[2-(4-amidinophenyl)]ethenyl-3,4-dihydro-1H-1,4-benzodiazapine-2,5-dione,
1-methyl-4-(3-butanoyl)-7-[2-(4-amidinophenyl)]ethynyl-3,4-dihydro-1H-1,4-benzodiazapine-2,5-dione,
1-(1-methyl)ethyl-4-(3-butanoyl)-7-(4-amidino)benzamido-3,4-dihydro-1H-1,4-benzodiazapine-2,5-dione,
1-(1-methyl)ethyl-4-(3-butanoyl)-7-(4-amidino)benzyloxy-3,4-dihydro-1H-1,4-benzodiazapine-2,5-dione,
1-(1-methyl)ethyl-4-(3-butanoyl)-7-[2-(4-amidinophenyl)]ethenyl-3,4-dihydro-1H-1,4-benzodiazapine-2,5-dione,
1-(2-methyl)ethyl-4-(3-butanoyl)-7-[2-(4-amidinophenyl)]ethynyl-3,4-dihydro-1H-1,4-benzodiazapine-2,5-dione,
1-(2-methyl)propyl-4-(3-butanoyl)-7-(4-amidino)benzamido-3,4-dihydro-1H-1,4-benzodiazapine-2,5-dione,
1-(2-methyl)propyl-4-(3-butanoyl)-7-(4-amidino)benzyloxy-3,4-dihydro-1H-1,4-benzodiazapine-2,5-dione,
1-(2-methyl)propyl-4-(3-butanoyl)-7-[2-(4-amidinophenyl)]ethenyl-3,4-dihydro-1H-1,4-benzodiazapine-2,5-dione,
1-(2-methyl)propyl-4-(3-butanoyl)-7-[2-(4-amidinophenyl)]ethynyl-3,4-dihydro-1H-1,4-benzodiazapine-2,5-dione,
1-(2,4-diflurophenyl)methyl-4-(3-butanoyl)-7-(4-(1-piperizin)-phenyl)-3,4-dihydro-1H-1,4-benzodiazapine-2,5-dione,
1-(2,4-diflurophenyl)methyl-4-(3-butanoyl)-7-(4-(2-aminoethoxy)-phenyl)-3,4-dihydro-1H-1,4-benzodiazapine-2,5-dione,
1-(2,4-diflurophenyl)methyl-4-(3-butanoyl)-7-(6-aminohexyl)-3,4-dihydro-1H-1,4-benzodiazapine-2,5-dione,
1-(2,4-diflurophenyl)methyl-4-(3-butanoyl)-7-(5-aminopentoxy)-3,4-dihydro-1H-1,4-benzodiazapine-2,5-dione,
1-(2,4-diflurophenyl)methyl-4-(3-butanoyl)-7-(4-amidino)benzamido-3,4-dihydro-1H-1,4-benzodiazapine-2,5-dione,
1-(2,4-diflurophenyl)methyl-4-(3-butanoyl)-7-(4-amidino)benzyloxy-3,4-dihydro-1H-1,4-benzodiazapine-2,5-dione,
1-(2,4-diflurophenyl)methyl-4-(3-butanoyl)-7-[2-(4-amidinophenyl)]ethenyl-3,4-dihydro-1H-1,4-benzodiazapine-2,5-dione,
1-(2,4-diflurophenyl)methyl-4-(3-butanoyl)-7-[2-(4-amidinophenyl)]ethynyl-3,4-dihydro-1H-1,4-benzodiazapine-2,5-dione,
1-(4-chlorophenyl)methyl-4-(3-butanoyl)-7-[2-(4-piperidinyl)ethyloxy]-3,4-dihydro-1H-1,4-benzodiazapine-2,5-dione,
1-(diphenyl)methyl-4-(3-butanoyl)-7-[2-(4-piperidinyl)ethyloxy]3,4-dihydro-1H-1,4-benzodiazapine-2,5-dione,
1-(*m*-trifluromethyl)phenyl-4-(3-butanoyl)-7-[2-(4-piperidinyl)ethyloxy]-3,4-dihydro-1H-1,4-benzodiazapine-2,5-dione,
1-methyl-4-(3-butanoyl)-7-[2-(4-piperidinyl)ethyloxy]-3,4-dihydro-1H-1,4-benzodiazapine-2,5-dione,
1-(1-methyl)ethyl-4-(3-butanoyl)-7-[2-(4-piperidinyl)ethyloxy]-3,4-dihydro-1H-1,4-benzodiazapine-2,5-dione,
1-(2-methyl)propyl-4-(3-butanoyl)-7-[2-(4-piperidinyl)ethyloxy]-3,4-dihydro-1H-1,4-benzodiazapine-2,5-dione,
1-(2,4-diflurophenyl)methyl-4-(3-butanoyl)-7-[2-(4-piperidinyl)ethyloxy]-3,4-dihydro-1H-1,4-benzodiazapine-2,5-dione,
1-(4-chlorophenyl)methyl-4-[(2-methyl)carboxyethyl]-7-(4-(1-piperizin)-phenyl)-3,4-dihydro-1H-1,4-benzodiazapine-2,5-dione,
1-(diphenyl)methyl-4-[(2-methyl)carboxyethyl]-7-(4-(1-piperizin)-phenyl)-3,4-dihydro-1H-1,4-benzodiazapine-2,5-dione,
1-(*m*-trifluromethyl)phenyl-4-[(2-methyl)carboxyethyl]-7-(4-(1-piperizin)-phenyl)-3,4-dihydro-1H-1,4-benzodiazapine-2,5-dione,
1-methyl-4-[(2-methyl)carboxyethyl]-7-(4-(1-piperizin)-phenyl)-3,4-dihydro-1H-1,4-benzodiazapine-2,5-dione,
1-(1-methyl)ethyl-4-[(2-methyl)carboxyethyl]-7-(4-(1-piperizin)-phenyl)-3,4-dihydro-1H-1,4-benzodiazapine-2,5-dione,
1-(2-methyl)propyl-4-[(2-methyl)carboxyethyl]-7-(4-(1-piperizin)-phenyl)-3,4-dihydro-1H-1,4-benzodiazapine-2,5-dione,
1-(4-chlorophenyl)methyl-4-[(2-methyl)carboxyethyl]-7-(4-(2-aminoethoxy)-phenyl)-3,4-dihydro-1H-1,4-benzodiazapine-2,5-dione,
1-(diphenyl)methyl-4-[(2-methyl)carboxyethyl]-7-(4-(2-aminoethoxy)-phenyl)-3,4-dihydro-1H-1,4-benzodiazapine-2,5-dione,
1-(*m*-trifluromethyl)phenyl-4-[(2-methyl)carboxyethyl]-7-(4-(2-aminoethoxy)-phenyl)-3,4-dihydro-1H-1,4-benzodiazapine-2,5-dione,
1-methyl-4-[(2-methyl)carboxyethyl] -7-(4-(2-aminoethoxy)-phenyl)-3,4-dihydro-1H-1,4-benzodiazapine-2,5-dione,
1-(1-methyl)ethyl-4-[(2-methyl)carboxyethyl]-7-(4-(2-aminoethoxy)-phenyl)-3,4-dihydro-1H-1,4-benzodiazapine-2,5-dione,
1-(2-methyl)propyl-4-[(2-methyl)carboxyethyl]-7-(4-(2-aminoethoxy)-phenyl)-3,4-dihydro-1H-1,4-benzodiazapine-2,5-dione,
1-(diphenyl)methyl-4-[(2-methyl)carboxyethyl]-7-(6-aminohexyl)-3,4-dihydro-1H-1,4-benzodiazapine-2,5-dione,
1-(diphenyl)methyl-4-[(2-methyl)carboxyethyl]-7-(6-aminohex-1-ynyl)-3,4-dihydro-1H-1,4-benzodiazapine-2,5-dione,
1-(diphenyl)methyl-4-[(2-methyl)carboxyethyl]-7-(5-aminopentoxy)-3,4-dihydro-1H-1,4-benzodiazapine-2,5-dione,
I-(diphenyl)methyl-4-[(2-methyl)carboxyethyl]-7-(5-guanidinopentyl)-3,4-dihydro-1H-1,4-benzodiazapine-2,5-dione,
1-(diphenyl)methyl-4-[(2-methyl)carboxyethyl]-7-(4-guanidinobutoxy)-3,4-dihydro-1H-1,4-benzodiazapine-2,5-dione,
1-(diphenyl)methyl-4-[(2-methyl)carboxyethyl]-7-(5-aminopent-1-ynyl)-3,4-dihydro-1H-1,4-benzodiazapine-2,5-dione,
1-(diphenyl)methyl-4-[(2-methyl)carboxyethyl]-7-(5-guanidinopent-1-ynyl)-3,4-dihydro-1H-1,4-benzodiazapine-2,5-dione,
1-(diphenyl)methyl-4-[(2-methyl)carboxyethyl]-7-(4-amidino)benzamido-3,4-dihydro-1H-1,4-benzodiazapine-2,5-dione,
1-(diphenyl)methyl-4-[(2-methyl)carboxyethyl]-7-(4-amidino)benzyloxy-3,4-dihydro-1H-1,4-benzodiazapine-2,5-dione,
1-(diphenyl)methyl-4-[(2-methyl)carboxyethyl]-7-[2-(4-amidinophenyl)]ethenyl-3,4-dihydro-1H-1,4-benzodiazapine-2,5-dione,
1-(diphenyl)methyl-4-[(2-methyl)carboxyethyl]-7-[2-(4-amidinophenyl)]ethynyl-3,4-dihydro-1H-1,4-benzodiazapine-2,5-dione,
1-(4-chlorophenyl)methyl-4-[(2-methyl)carboxyethyl]-7-(6-aminohexyl)-3,4-dihydro-1H-1,4-benzodiazapine-2,5-dione,
1-(4-chlorophenyl)methyl-4-[(2-methyl)carboxyethyl]-7-(5-aminopentoxy)-3,4-dihydro-1H-1,4-benzodiazapine-2,5-dione,
1-(4-chlorophenyl)methyl-4-[(2-methyl)carboxyethyl]-7-(4-amidino)benzamido-3,4-dihydro-1H-1,4-benzodiazapine-2,5-dione,
1-(4-chlorophenyl)methyl-4-[(2-methyl)carboxyethyl]-7-(4-amidino)benzyloxy-3,4-dihydro-1H-1,4-benzodiazapine-2,5-dione,
1-(4-chlorophenyl)methyl-4-[(2-methyl)carboxyethyl]-7-[2-(4-amidinophenyl)]ethenyl-3,4-dihydro-1H-1,4-benzodiazapine-2,5-dione,
1-(4-chlorophenyl)methyl-4-[(2-methyl)carboxyethyl]-7-[2-(4-amidinophenyl)]ethynyl-3,4-dihydro-1H-1,4-benzodiazapine-2,5-dione,
1-methyl-4-[(2-methyl)carboxyethyl]-7-(4-amidino)benzamido-3,4-dihydro-1H-1,4-benzodiazapine-2,5-dione,
1-methyl-4-[(2-methyl)carboxyethyl]-7-(4-amidino)benzyloxy-3,4-dihydro-1H-1,4-benzodiazapine-2,5-dione,
1-methyl-4-[(2-methyl)carboxyethyl]-7-[2-(4-amidinophenyl)]ethenyl-3,4-dihydro-1H-1,4-benzodiazapine-2,5-dione,
1-methyl-4-[(2-methyl)carboxyethyl]-7-[2-(4-amidinophenyl)]ethynyl-3,4-dihydro-1H-1,4-benzodiazapine-2,5-dione,
1-(1-methyl)ethyl-4-[(2-methyl)carboxyethyl]-7-(4-amidino)benzamido-3,4-dihydro-1H-1,4-benzodiazapine-2,5-dione,
1-(1-methyl)ethyl-4-[(2-methyl)carboxyethyl]-7-(4-amidino)benzyloxy-3,4-dihydro-1H-1,4-benzodiazapine-2,5-dione,
1-(1-methyl)ethyl-4-[(2-methyl)carboxyethyl]-7-[2-(4-amidinophenyl)]ethenyl-3,4-dihydro-1H-1,4-benzodiazapine-2,5-dione,
1-(2-methyl)ethyl-4-[(2-methyl)carboxyethyl]-7-[2-(4-amidinophenyl)]ethynyl-3,4-dihydro-1H-1,4-benzodiazapine-2,5-dione,
1-(2-methyl)propyl-4-[(2-methyl)carboxyethyl]-7-(4-amidino)benzamido-3,4-dihydro-1H-1,4-benzodiazapine-2,5-dione,
1-(2-methyl)propyl-4-[(2-methyl)carboxyethyl]-7-(4-amidino)benzyloxy-3,4-dihydro-1H-1,4-benzodiazapine-2,5-dione,
1-(2-methyl)propyl-4-[(2-methyl)carboxyethyl]-7-[2-(4-amidinophenyl)]ethenyl-3,4-dihydro-1H-1,4-benzodiazapine-2,5-dione,
1-(2-methyl)propyl-4-[(2-methyl)carboxyethyl]-7-[2-(4-amidinophenyl)]ethynyl-3,4-dihydro-1H-1,4-benzodiazapine-2,5-dione,
1-(2,4-diflurophenyl)methyl-4-[(2-methyl)carboxyethyl]-7-(4-(1-piperizin)-phenyl)-3,4-dihydro-1H-1,4-benzodiazapine-2,5-dione,
1-(2,4-diflurophenyl)methyl-4-[(2-methyl)carboxyethyl]-7-(4-[(2-aminoethoxy)-phenyl)-3,4-dihydro-1H-1,4-benzodiazapine-2,5-dione,
1-(2,4-diflurophenyl)methyl-4-[(2-methyl)carboxyethyl]-7-(6-aminohexyl)-3,4-dihydro-1H-1,4-benzodiazapine-2,5-dione,
1-(2,4-diflurophenyl)methyl-4-[(2-methyl)carboxyethyl]-7-(5-aminopentoxy)-3,4-dihydro-1H-1,4-benzodiazapine-2,5-dione,
1-(2,4-diflurophenyl)methyl-4-[(2-methyl)carboxyethyl]-7-(4-amidino)benzamido-3,4-dihydro-1H-1,4-benzodiazapine-2,5-dione,
1-(2,4-diflurophenyl)methyl-4-[(2-methyl)carboxyethyl]-7-(4-amidino)benzyloxy-3,4-dihydro-1H-1,4-benzodiazapine-2,5-dione,
1-(2,4-diflurophenyl)methyl-4-[(2-methyl)carboxyethyl]-7-[2-(4-amidinophenyl)]ethenyl-3,4-dihydro-1H-1,4-benzodiazapine-2,5-dione,
1-(2,4-diflurophenyl)methyl-4-[(2-methyl)carboxyethyl]-7-[2-(4-amidinophenyl)]ethynyl-3,4-dihydro-1H-1,4-benzodiazapine-2,5-dione,
1-(4-chlorophenyl)methyl-4-[(2-methyl)carboxyethyl]-7-[2-(4-piperidinyl)ethyloxy]-3,4-dihydro-1H-1,4-benzodiazapine-2,5-dione,
1-(diphenyl)methyl-4-[(2-methyl)carboxyethyl]-7-[2-(4-piperidinyl)ethyloxy]3,4-dihydro-1H-1,4-benzodiazapine-2,5-dione,
1-(*m*-trifluromethyl)phenyl-4-[(2-methyl)carboxyethyl]-7-[2-(4-piperidinyl)ethyloxy]-3,4-dihydro-1H-1,4-benzodiazapine-2,5-dione,
1-methyl-4-[(2-methyl)carboxyethyl]-7-[2-(4-piperidinyl)ethyloxy]-3,4-dihydro-1H-1,4-benzodiazapine-2,5-dione,
1-(1-methyl)ethyl-4-[(2-methyl)carboxyethyl]-7-[2-(4-piperidinyl)ethyloxy]-3,4-dihydro-1H-1,4-benzodiazapine-2,5-dione,
1-(2-methyl)propyl-4-[(2-methyl)carboxyethyl]-7-[2-(4-piperidinyl)ethyloxy]-3,4-dihydro-1H-1,4-benzodiazapine-2,5-dione,
1-(2,4-diflurophenyl)methyl-4-[(2-methyl)carboxyethyl]-7-[2-(4-piperidinyl)ethyloxy]-3,4-dihydro-1H-1,4-benzodiazapine-2,5-dione,
1-(3-(butyryl)aminophenyl)methyl-4-(2-carboxyethyl)-7-(6-aminohexyl)-3,4-dihydro-1H-1,4-benzodiazapine-2,5-dione,
1-(3-(butyryl)aminophenyl)methyl-4-(2-carboxyethyl)-7-(5-guanidinopentyl)-3,4-dihydro-1H-1,4-benzodiazapine-2,5-dione,
1-(3-(4-aminobutyryl)aminophenyl)methyl-4-(2-carboxyethyl)-7-(6-aminohexyl)-3,4-dihydro-1H-1,4-benzodiazapine-2,5-dione,
1-(3-(4-guanidinobutyryl)aminophenyl)methyl-4-(2-carboxyethyl)-7-(5-guanidinopentyl)-3,4-dihydro-1H-1,4-benzodiazapine-2,5-dione,
1-(3-(6-aminohexoyl)aminophenyl)methyl-4-(2-carboxyethyl)-7-(6-aminohexyl)-3,4-dihydro-1H-1,4-benzodiazapine-2,5-dione,
1-(3-(6-guanidinohexoyl)aminophenyl)methyl-4-(2-carboxyethyl)-7-(5-guanidinopentyl)-3,4-dihydro-1H-1,4-benzodiazapine-2,5-dione,
1-(3-(6-aminopentoyl)aminophenyl)methyl-4-(2-carboxyethyl)-7-(6-aminohexyl)-3,4-dihydro-1H-1,4-benzodiazapine-2,5-dione,
1-(3-(6-guanidinopentoyl)aminophenyl)methyl-4-(2-carboxyethyl)-7-(5-guanidinopentyl)-3,4-dihydro-1H-1,4-benzodiazapine-2,5-dione,
1-(3-(6-aminopropionyl)aminophenyl)methyl-4-(2-carboxyethyl)-7-(6-aminohexyl)-3,4-dihydro-1H-1,4-benzodiazapine-2,5-dione,
1-(3-(6-guanidinopropionyl)aminophenyl)methyl-4-(2-carboxyethyl)-7-(5-guanidinopentyl)-3,4-dihydro-1H-1,4-benzodiazapine-2,5-dione,
1-(3-(6-aminoacetyl)aminophenyl)methyl-4-(2-carboxyethyl)-7-(6-aminohexyl)-3,4-dihydro-1H-1,4-benzodiazapine-2,5-dione,
1-(3-(6-guanidinoacetyl)aminophenyl)methyl-4-(2-carboxyethyl)-7-(5-guanidinopentyl)-3,4-dihydro-1H-1,4-benzodiazapine-2,5-dione,
1-(3-(4-guanidinobenzoyl)aminophenyl)methyl-4-(2-carboxyethyl)-7-(6-aminohexyl)-3,4-dihydro-1H-1,4-benzodiazapine-2,5-dione,
1-(4-(6-aminopropionyl)aminophenyl)methyl-4-(2-carboxyethyl)-7-(6-aminohexyl)-3,4-dihydro-1H-1,4-benzodiazapine-2,5-dione,
1-(3-(4-aminobutyl)phenyl)methyl-4-(2-carboxyethyl)-7-(6-aminohexyl)-3,4-dihydro-1H-1,4-benzodiazapine-2,5-dione, and
1-(4-carboxyphenyl)methyl-4-(2-carboxyethyl)-7-(6-aminohexyl)-3,4-dihydro-1H-1,4-benzodiazapine-2,5-dione.

7. The compound of Claim 1 represented by formula III and IV: 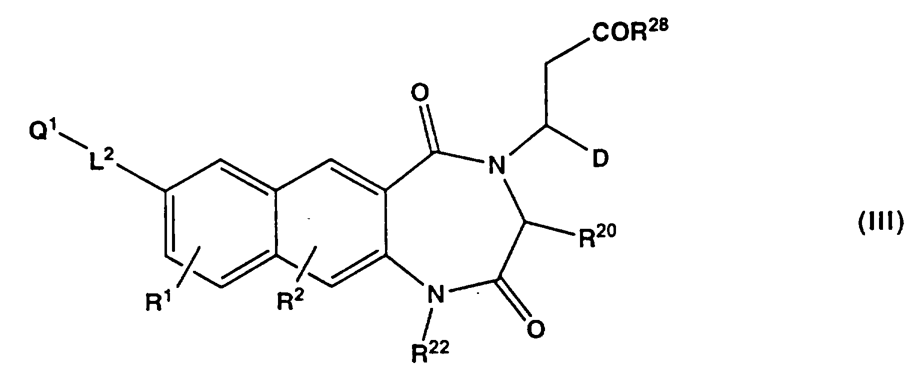 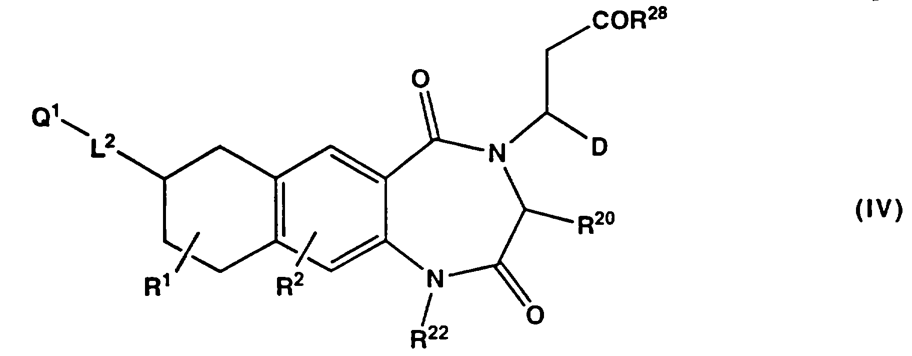
where
R¹ and R are independently selected from hydrogen and halo(F, Cl, Br, I);
Q¹ is selected from the group consisting of
(A) an amino group,
(B) an amidino group,
(C) an aminoalkyleneimino group,
(D) an iminoalkyleneamino group, and
(E) a guanidino group;
R⁰ is hydrogen;
R is selected from the group consisting of (i) hydrogen, (ii) optionally substituted C₁-C₁₂-alkyl, (iii) optionally substituted C₆-C₁₄-aryl, (iv) optionally substituted C₃-C₁₄-cycloalkyl, (v) optionally substituted C₁-C₁₂-alkyl-C₆-C₁₄-aryl, (vi) optionally substituted C₁-C₁₂-alkyl-C₃-C₁₄-cycloalkyl, where the substituents are selected from (a) halo (F, Cl, Br, I), (b) nitro, (c) hydroxy, (d) carboxy, (e) tetrazole, (f) hydroxamate, (g) sulfonamide, (h) trifluoroimide, (i) phosphonate, (j) C₁-C₆-alkyl, (k) C₆-C₁₄-aryl, (l) benzyl, (m) C₃-C₁₄-cycloalkyl, (n) COR⁴ where R⁴ is selected from the group C₁-C₈-alkoxy, C₃-C₁₂-alkenoxy, C₆-C₁₂-aryloxy, di-C₁-C₈-alkylamino-C₁-C₈-alkoxy, acylamino-C₁-C₈-alkoxy selected from the group acetylaminoethoxy, nicotinoylaminoethoxy, succinamidoethoxy, and pivaloyloxyethoxy, and C₆-C₁₂-aryl-C₁-C₈-alkoxy where the aryl group is unsubstituted or substituted with one to three of the groups nitro, halo (F, Cl, Br, I), C₁-C₄-alkoxy, amino, hydroxy, hydroxy-C₂-C₈-alkoxy, and dihydroxy-C₃-C₈-alkoxy, (o) CONR⁵R⁶ where R⁵ and R⁶ are independently selected from hydrogen, C₁-C₁₀-alkyl, C₃-C₁₀-alkenyl, C₆-C₁₄-aryl, C₁-C₆-alkyl-C₆-C₁₀-aryl, optionally R⁵ and R⁶ taken together may form trimethylene, tetramethylene, pentamethylene, and 3-oxopentamethylene, and (vii) Q-L³- where Q is selected from hydrogen, and Q¹, and L³ is selected from a chemical bond, and L¹, where L¹ is an optionally substituted bivalent radical selected from the group consisting of C₃-C₇-alkylene, C₃-C₇-cycloalkylene, C₃-C₇-alkenylene, C₄-C₇-cycloalkenylene, C₅-C₈-cycloalkadienylene, C₃-C₇-alkadienylene, C₃-C₇-alkynylene, C₄-C₇-alkenynylene, C₆-C₁₄-arylene, C₆-C₁₄-aryl-C₂-C₄-alkynylene, C₁-C₃-alkyl-C₆-C₁₄-aryl-C₂-C₄-alkynylene, C₆-C₁₄-aryl-C₂-C₄-alkenylene, C₁-C₃-alkyl-C₆-C₁₄-arylene, C₁-C₃-alkyl-C₆-C₁₄-aryl-C₂-C₄-alkenylene, C₆-C₁₄-aryl-C₁-C₃-alkylene, C₆-C₁₄-aryl-C₁-C₃-alkyloxyene, C₁-C₂-alkyl-C₆-C₁₄-aryl-C₁-C₂-alkylene, C₁-C₃-alkyloxy-C₆-C₁₄-arylene, C₂-C₈-alkyloxyene, C₁-C₅-alkyloxy-C₁-C₅-alkylene, C₆-C₁₀-aryloxyene, C₆-C₁₀-aryloxy-C₁-C₅-alkylene, C₆-C₁₀-arylthio-C₁-C₅-alkylene, 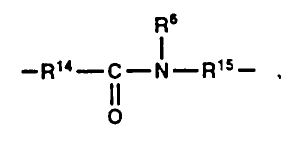 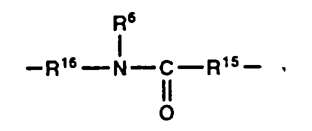 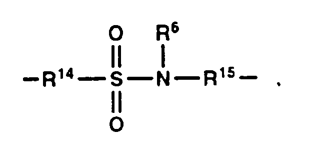 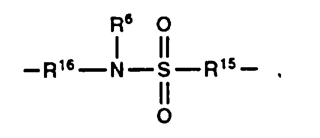 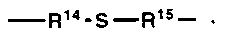 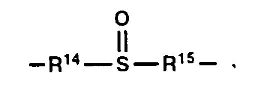 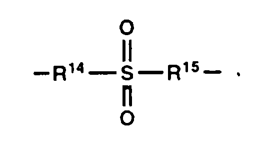 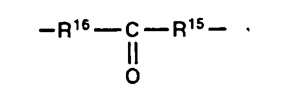 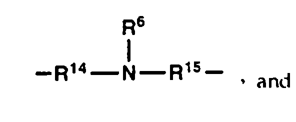 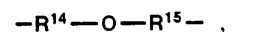
where R¹⁴ is selected from a chemical bond, C₁-C₈-alkyl, C₃-C₇-cycloalkyl, C₂-C₅-alkenyl, C₃-C₅-alkynyl, C₆-C₁₀-aryl, C₁-C₃,alkyl-C₆-C₁₂-aryl, C₁-C₂-alkyl-C₆-C₁₀-aryl-C₁-C₂-alkyl, and C₆-C₁₀-aryl-C₁-C₂-alkyl, R¹⁵ is selected from a chemical bond, C₁-C₄-alkyl, C₂-C₄-alkenyl, C₂-C₄-alkynyl, and C₆-C₁₀-aryl, R¹⁶ is selected from a chemical bond, C₂-C₅-alkyl, C₃-C₇-cycloalkyl, C₃-C₅-alkenyl, C₃-C₅-alkynyl, C₆-C₁₀-aryl, C₁-C₃-alkyl-C₆-C₁₂-aryl, C₁-C₃-alkyl-C₆-C₁₂-aryl, and C₆-C₁₀-aryl-C₁-C₂-alkyl, where R¹⁴ or R¹⁶ is bonded to N and R¹⁵ is bonded to Q and where the substituents are selected from one to three R¹;
L is an optionally substituted bivalent radical selected from the group consisting of 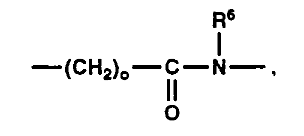 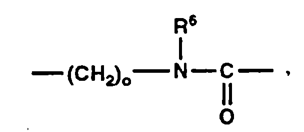 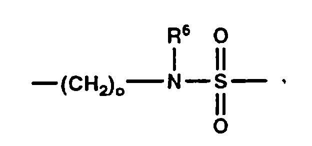 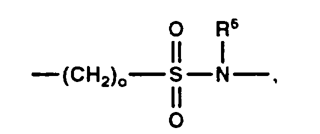 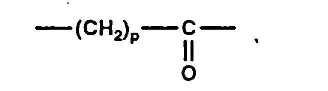
-(CH₂)ₚ-S- , 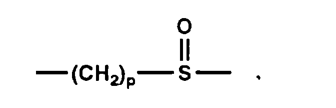 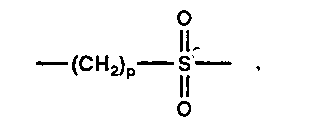 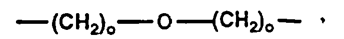 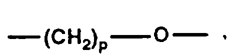 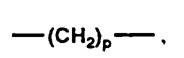 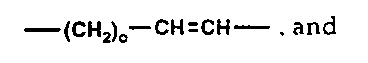 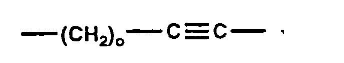
where o is 1 or 2, p is 1, 2, 3 or 4, R⁶ is defined above, and where the substituents are selected from one to three R¹;
D is selected from the group consisting of R, and -(C=O)-Xaa, where Xaa is one to three D or L α-amino acid;
R⁸ is selected from the group consisting of (i) hydroxy, (ii) C₁-C₈-alkoxy, (iii) C₃-C₁₂-alkenoxy, (iv) C₆-C₁₂-aryloxy, (v) C₁-C₆-alkyl-C₆-C₁₂-aryloxy, (vi) di-C₁-C₈-alkylamino-C₁-C₈-alkoxy, (vii) acylamino-C₁-C₈-alkoxy selected from the group (a) acetylaminoethoxy, (b) nicotinoylaminoethoxy, and (c) succinamidoethoxy, (viii) C₁-C₈-alkoyloxy-C₁-C₈-alkoxy, (ix) C₆-C₁₂-aryl-C₁-C₈-alkoxy where the aryl group is unsubstituted or substituted with one to three of the groups (a) nitro, (b) halo (F, Cl, Br, I), (c) C₁-C₄-alkoxy, and (d) amino, (x) hydroxy-C₂-C₈-alkoxy, (xi) dihydroxy-C₃-C₈-alkoxy, and (xii) NR⁹R³⁰ where R⁹ and R³⁰ are independently selected from the group (a) hydrogen, (b) C₁-C₈-alkyl, (c) C₃-C₈-alkenyl, (d) C₆-C₁₂-aryl where the aryl group is unsubstituted or substituted with one to three of the groups nitro, halo (F, Cl, Br, I), C₁-C₄-alkoxy, and amino, and (e) C₆-C₁₂-aryl-C₁-C₈-alkyl where the aryl group is unsubstituted or substituted with one to three of the groups nitro, halo (F, Cl, Br, I), and C₁-C₄-alkoxy; and
pharmaceutically acceptable salts thereof.

8. The compound of Claim 1 represented by formula V: 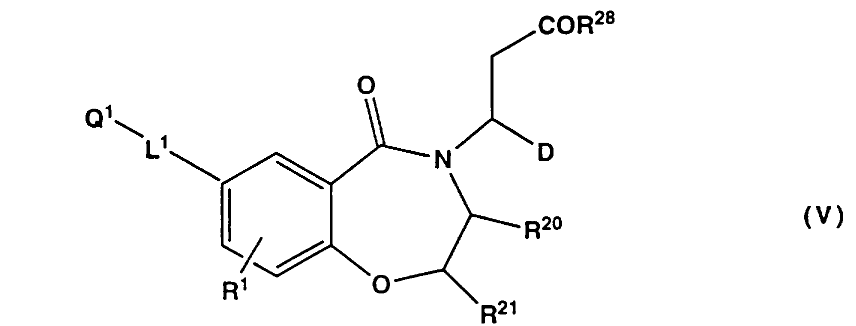
where
R¹ is one to three groups independently selected from hydrogen and halogen(F, Cl, Br, I);
Q¹ is selected from the group consisting of
(A) an amino group,
(B) an amidino group,
(C) an aminoalkyleneimino group,
(D) an iminoalkyleneamino group, and
(E) a guanidino group;
L¹ is an optionally substituted bivalent radical selected from the group consisting of 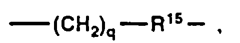 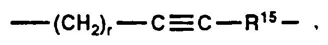 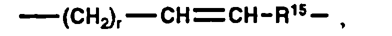 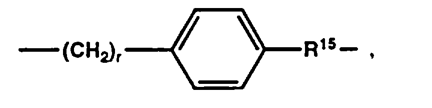 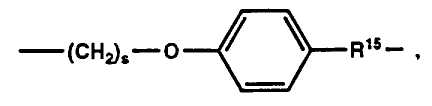 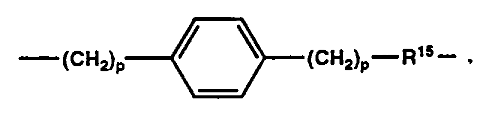 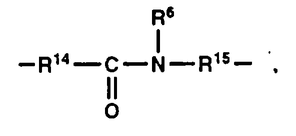 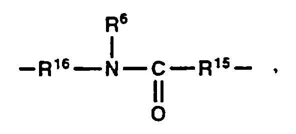 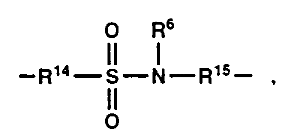 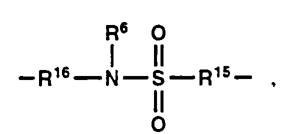 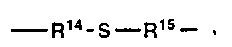 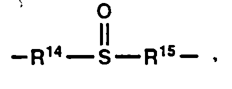 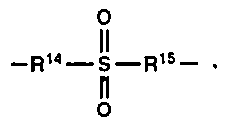 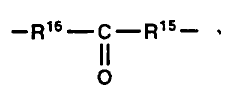 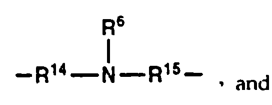 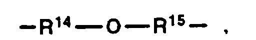
where
p is 1, 2, 3, or 4;
q is 3, 4, 5, 6 or 7;
r is 1, 2, 3, 4,or 5;
s is 2 or 3;
R¹⁴ is selected from
C₂-C₅-alkyl,
C₃-C₇-cycloalkyl,
C₂-C₅-alkenyl,
C₃-C₅-alkynyl,
C₆-C₁₀-aryl,
C₁-C₂-alkyl-C₆-C₁₂-aryl,
C₁-C₂-alkyl-C₆-C₁₀-aryl-C₁-C₂-alkyl,
C₆-C₁₀-aryl-C₁-C₂-alkyl, and
C₆-C₁₀-aryloxy-C₁-C₂-alkyl;
R¹⁵ is a chemical bond connecting L¹ to position 8 of the benzazepine-one;
R¹⁶ is selected from
C₂-C₅-alkyl,
C₃-C₇-cycloalkyl,
C₃-C₅-alkenyl,
C₃-C₅-alkynyl,
C₆-C₁₀-aryl,
C₁-C₃-alkyl-C₆-C₁₂-aryl, and
C₆-C₁₀-aryl-C₁-C₂-alkyl,
wherein R¹⁴ and R¹⁶ bond L¹ to Q¹, and
where the substituents are selected from the group hydrogen, halo(F, Cl, Br, I), C₁-C₄ alkoxy, C₁-C₄ alkyl, phenyl, benzyl, and halo(F, Cl, Br, I)-C₁-C₄ alkyl;
R⁰ is hydrogen;
R¹ is selected from the group consisting of (i) hydrogen, (ii) optionally substituted C₁-C₁₂-alkyl, (iii) optionally substituted C₆-C₁₄-aryl, (iv) optionally substituted C₃-C₁₄-cycloalkyl, (v) optionally substituted C₁-C₁₂-alkyl-C₆-C₁₄-aryl, (vi) optionally substituted C₁-C₁₂-alkyl-C₃-C₁₄-cycloalkyl, where the substituents are selected from (a) halo (F, Cl, Br, I), (b) nitro, (c) hydroxy, (d) carboxy, (e) tetrazole, (f) hydroxamate, (g) sulfonamide, (h) trifluoroimide, (i) phosphonate, (j) C₁-C₆-alkyl, (k) C₆-C₁₄-aryl, (l) benzyl, (m) C₃-C₁₄-cycloalkyl, (n) COR⁴ where R⁴ is selected from the group C₁-C₈-alkoxy, C₃-C₁₂-alkenoxy, C₆-C₁₂-aryloxy, di-C₁-C₈-alkylamino-C₁-C₈-alkoxy, acylamino-C₁-C₈-alkoxy selected from the group acetylaminoethoxy, nicotinoylaminoethoxy, succinamidoethoxy, and pivaloyloxyethoxy, and C₆-C₁₂-aryl-C₁-C₈-alkoxy where the aryl group is unsubstituted or substituted with one to three of the groups nitro, halo (F, Cl, Br, I), C₁-C₄-alkoxy, amino, hydroxy, hydroxy-C₂-C₈-alkoxy, and dihydroxy-C₃-C₈-alkoxy, (o) CONR⁵R⁶ where R⁵ and R⁶ are independently selected from hydrogen, C₁-C₁₀-alkyl, C₃-C₁₀-alkenyl, C₆-C₁₄-aryl, C₁-C₆-alkyl-C₆-C₁₀-aryl, optionally R⁵ and R⁶ taken together may form trimethylene, tetramethylene, pentamethylene, and 3-oxopentamethylene, and (vii) Q-L³- where Q is selected from hydrogen and Q¹, and L³ is selected from a chemical bond and L¹ where R¹⁵ bonds L¹ to position 4 of the benzazepine-one;
D is selected from the group consisting of R¹, and -(C=O)-Xaa, where Xaa is one to three D or L α-amino acid;
R⁸ is selected from the group consisting of (i) hydroxy, (ii) C₁-C₈-alkoxy, (iii) C₃-C₁₂-alkenoxy, (iv) C₆-C₁₂-aryloxy, (v) C₁-C₆-alkyl-C₆-C₁₂-aryloxy, (vi) di-C₁-C₈-alkylamino-C₁-C₈-alkoxy, (vii) acylamino-C₁-C₈-alkoxy selected from the group (a) acetylaminoethoxy, (b) nicotinoylaminoethoxy, and (c) succinamidoethoxy, (viii) C₁-C₈-alkoyloxy-C₁-C₈-alkoxy, (ix) C₆-C₁₂-aryl-C₁-C₈-alkoxy where the aryl group is unsubstituted or substituted with one to three of the groups (a) nitro, (b) halo (F, Cl, Br, I), (c) C₁-C₄-alkoxy, and (d) amino, (x) hydroxy-C₂-C₈-alkoxy, (xi) dihydroxy-C₃-C₈-alkoxy, and (xii) NR⁹R³⁰ where R⁹ and R³⁰ are independently selected from the group (a) hydrogen, (b) C₁-C₈-alkyl, (c) C₃-C₈-alkenyl, (d) C₆-C₁₂-aryl where the aryl group is unsubstituted or substituted with one to three of the groups nitro, halo (F, Cl, Br, I), C₁-C₄-alkoxy, and amino, and (e) C₆-C₁₂-aryl-C₁-C₈-alkyl where the aryl group is unsubstituted or substituted with one to three of the groups nitro, halo (F, Cl, Br, I), and C₁-C₄-alkoxy; and
pharmaceutically acceptable salts thereof.

9. The compound of Claim 1 represented by formula VI: 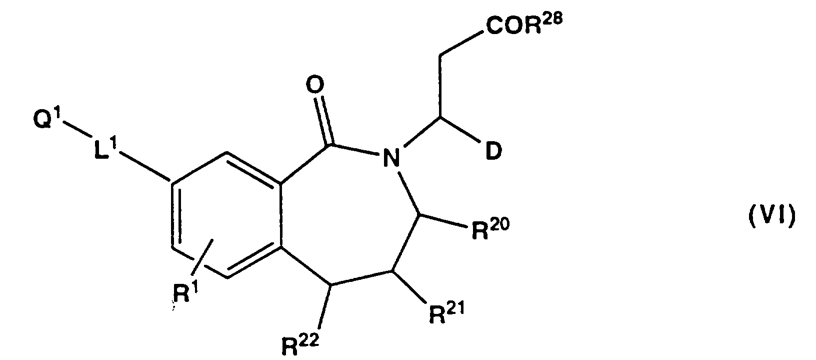
where
R¹ is one to three groups independently selected from hydrogen and halogen(F, Cl, Br, I);
Q¹ is selected from the group consisting of
(A) an amino group,
(B) an amidino group,
(C) an aminoalkyleneimino group,
(D) an iminoalkyleneamino group, and
(E) an guanidino group;
L¹ is an optionally substituted bivalent radical selected from the group consisting of 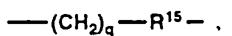 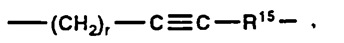 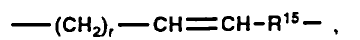 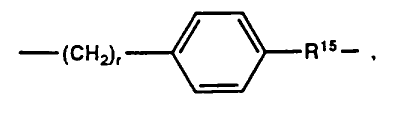 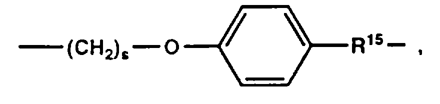 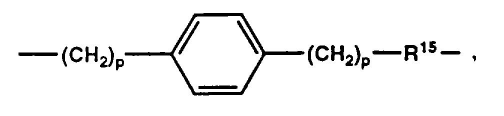 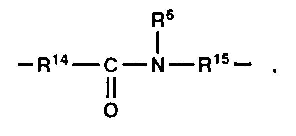 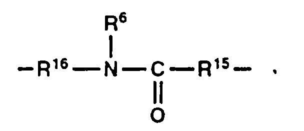 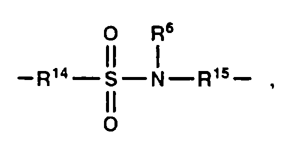 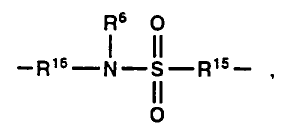 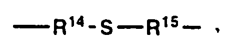 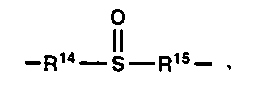 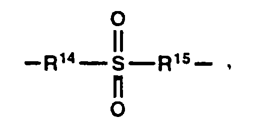 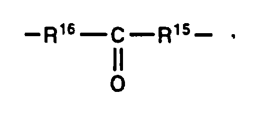 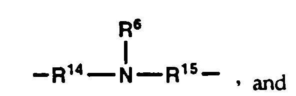 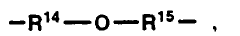
where
p is 1, 2, 3, or 4;
q is 3, 4, 5, 6 or 7;
r is 1, 2, 3, 4, or 5;
s is 2 or 3;
R¹⁴ is selected from
C₂-C₅-alkyl,
C₃-C₇-cycloalkyl,
C₂-C₅-alkenyl,
C₃-C₅-alkynyl,
C₆-C₁₀-aryl,
C₁-C₂-alkyl-C₆-C₁₂-aryl,
C₁-C₂-alkyl-C₆-C₁₀-aryl-C₁-C₂-alkyl,
C₆-C₁₀-aryl-C₁-C₂-alkyl, and
C₆-C₁₀-aryloxy-C₁-C₂-alkyl;
R¹⁵ is a chemical bond connecting L¹ to position 8 of the benzazepine-one;
R¹⁶ is selected from
C₂-C₅-alkyl,
C₃-C₇-cycloalkyl,
C₃-C₅-alkenyl,
C₃-C₅-alkynyl,
C₆-C₁₀-aryl,
C₁-C₃-alkyl-C₆-C₁₂-aryl, and
C₆-C₁₀-aryl-C₁-C₂-alkyl,
wherein R¹⁴ and R¹⁶ bond L¹ to Q¹, and
where the substituents are selected from the group hydrogen, halo(F, Cl, Br, I), C₁-C₄ alkoxy, C₁-C₄ -alkyl, phenyl, benzyl, and halo(F, Cl, Br, I)-C₁-C₄-alkyl;
R⁰ is hydrogen;
R¹ and R are selected from the group consisting of (i) hydrogen, (ii) optionally substituted C₁-C₁₂-alkyl, (iii) optionally substituted C₆-C₁₄-aryl, (iv) optionally substituted C₃-C₁₄-cycloalkyl, (v) optionally substituted C₁-C₁₂-alkyl-C₆-C₁₄-aryl, (vi) optionally substituted C₁-C₁₂-alkyl-C₃-C₁₄-cycloalkyl, where the substituents are selected from (a) halo (F, Cl, Br, I), (b) nitro, (c) hydroxy, (d) carboxy, (e) tetrazole, (f) hydroxamate, (g) sulfonamide, (h) trifluoroimide, (i) phosphonate, (j) C₁-C₆-alkyl, (k) C₆-C₁₄-aryl, (l) benzyl, (m) C₃-C₁₄-cycloalkyl, (n) COR⁴ where R⁴ is selected from the group C₁-C₈-alkoxy, C₃-C₁₂-alkenoxy, C₆-C₁₂-aryloxy, di-C₁-C₈-alkylamino-C₁-C₈-alkoxy, acylamino-C₁-C₈-alkoxy selected from the group acetylaminoethoxy, nicotinoylaminoethoxy, succinamidoethoxy, and pivaloyloxyethoxy, and C₆-C₁₂-aryl-C₁-C₈-alkoxy where the aryl group is unsubstituted or substituted with one three of the groups nitro, halo (F, Cl, Br, I), C₁-C₄-alkoxy, amino, hydroxy, hydroxy-C₂-C₈-alkoxy, and dihydroxy-C₃-C₈-alkoxy, (o) CONR⁵R⁶ where R⁵ and R⁶ are independently selected from hydrogen, C₁-C₁₀-alkyl, C₃-C₁₀-alkenyl, C₆-C₁₄-aryl, C₁-₆-alkyl-C₆-C₁₀-aryl, optionally R⁵ and R⁶ taken together may form trimethylene, tetramethylene, pentamethylene, and 3-oxopentamethylene, and (vii) Q-L³-where Q is selected from hydrogen and Q¹, and L³ is selected from a chemical bond and L¹ where R¹⁵ bonds L¹ to position 4 or 5 of the benzazepine-one;
D is selected from the group consisting of R¹, and -(C=O)-Xaa, where Xaa is one to three D or L α-amino acid;
R⁸ is selected from the group consisting of (i) hydroxy, (ii) C₁-C₈-alkoxy, (iii) C₃-C₁₂-alkenoxy, (iv) C₆-C₁₂-aryloxy, (v) C₁-C₆-alkyl-C₆-C₁₂-aryloxy, (vi) di-C₁-C₈-alkylamino-C₁-C₈-alkoxy, (vii) acylamino-C₁-C₈-alkoxy selected from the group (a) acetylaminoethoxy, (b) nicotinoylaminoethoxy, and (c) succinamidoethoxy, (viii) C₁-C₈-alkoyloxy-C₁-C₈-alkoxy, (ix) C₆-C₁₂-aryl-C₁-C₈-alkoxy where the aryl group is unsubstituted or substituted with one to three of the groups (a) nitro, (b) halo (F, Cl, Br, I), (c) C₁-C₄-alkoxy, and (d) amino, (x) hydroxy-C₂-C₈-alkoxy, (xi) dihydroxy-C₃-C₈-alkoxy, and (xii) NR⁹R³⁰ where R⁹ and R³⁰ are independently selected from the group (a) hydrogen, (b) C₁-C₈-alkyl, (c) C₃-C₈-alkenyl, (d) C₆-C₁₂-aryl when the aryl group is unsubstituted or substituted with one to three of the groups nitro, halo (F, Cl, Br, I), C₁-C₄-alkoxy, and amino, and (e) C₆-C₁₂-aryl-C₁-C₈-alkyl where the aryl group is unsubstituted or substituted with one to three of the groups nitro, halo (F, Cl, Br, I), and C₁-C₄-alkoxy; and
pharmaceutically acceptable salts thereof.

10. A pharmaceutical composition comprising a pharmaceutically acceptable excipient and the compound of Claims 1 to 9.

11. The compound of any one of claims 1 to 9 for use in a method of treatment.

12. Use of a compound of any one of claims 1 to 9 in the preparation of a medicament to inhibit platelet aggregation.

13. Use of a compound of anyone of claims 1 to 9 in the preparation of a medicament to reduce platelet aggregation.

14. Use of a compound of any one of claims 1 to 9 in the preparation of a medicament to treat a mammal having an increased propensity for thrombus formation.

15. The use of claim 14 wherein the medicament is administered in combination with a thrombolytic agent.

16. The use of claim 14 wherein the medicament is administered in combination with an anticoagulant.

## Patentansprüche

1. Verbindung gemäß Strukturformel (I): 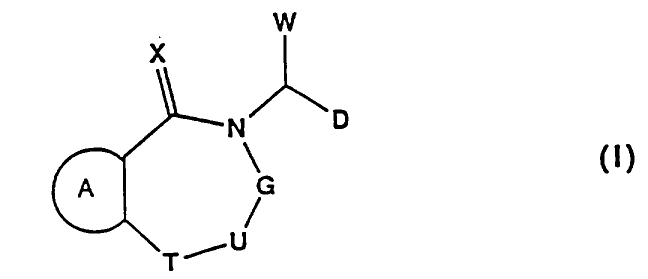
worin die Teilstruktur 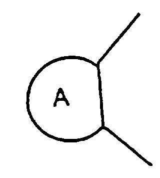
folgendes darstellt: 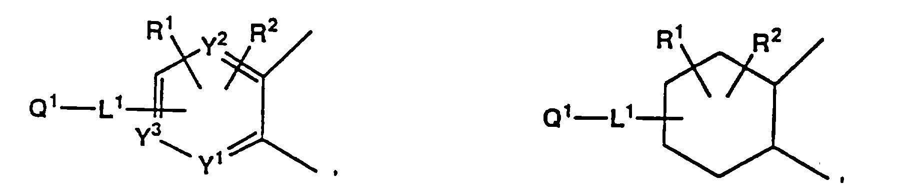 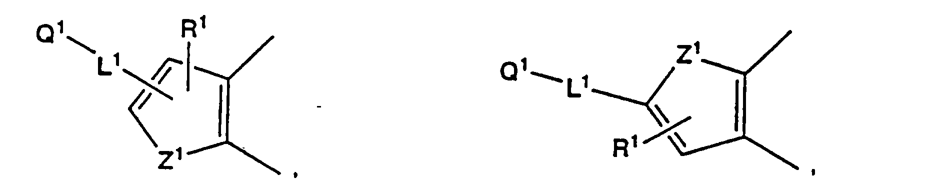 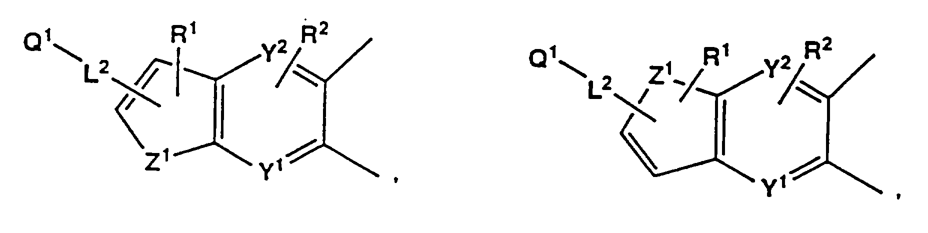 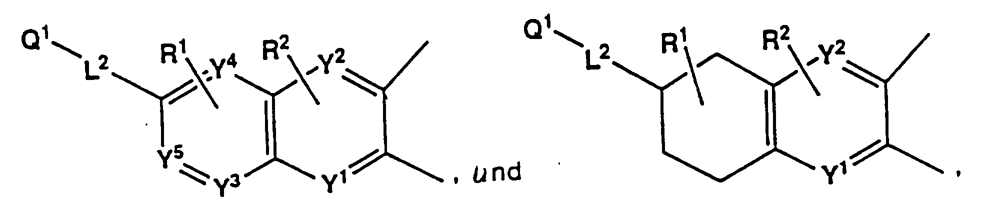
worin:
R¹ und R unabhängig voneinander ausgewählt sind aus: Wasserstoff, Halogen (F, Cl, Br, I), Cyano, Carboxamido, Carbamoyloxy, Formyloxy, Formyl, Azido, Nitro, Ureido, Thioureido, Thiocyanato, Hydroxy, Mercapto, Sulfonamido und einem gegebenenfalls substituierten Rest, der ausgewählt ist aus: C₁-C₁₂-Alkyl, C₂-C₁₂-Alkenyl, C₃-C₁₂-Alkinyl, C₃-C₁₂-Cycloalkyl, C₆-C₁₄-Aryl, C₆-C₁₀-Aryl-C₁-C₈-alkyl, C₁-C₁₂-Alkyloxy, C₆-C₁₄-Aryloxy, C₁-C₁₂-Acylamino, N,N-Di-(C₁-C₁₂)-acylamino, N-(C₁-C₁₂)-Alkyl-N-(C₁-sulfonylamino), C₁-C₁₂-Alkylthiocarbonyl, C₁-C₁₂-Alkylthio, C₁-C₁₂-Alkylsulfinyl, C₁-C₁₂-Alkylsulfonyl, C₁-C₁₂-Alkylsulfonato, N-(C₁-C₁₂)-Alkylsulfonamido, N,N-Di-(C₁-C₁₂)-Alkylsulfonamido, N-(C₁-C₁₂)-Alkyl-N-thioformylamino, C₁-C₁₂-Thioacylamino, N-(C₁-C₁₂)-Alkyl-N-(C₁-C₁₂)-thioacylamino, C₁-C₁₂-Alkylsulfinamido, N-(C₁-C₁₂)-Alkyl-N-(C₁-C₁₂)-alkylsulfinylamino, C₁-C₁₂-Carbalkoxy, C₁-C₁₂-Alkylcarbonyl, C₁-C₁₂-Alkanoyloxy, N-(C₁-C₁₂)-Alkylcarboxamido, N,N-Di-(C₁-C₁₂)-carboxamido, N-(C₁-C₁₂)-Alkylcarbamoyloxy, N,N-Di-(C₁-C₁₂)carbamoyloxy, und Heterocycloalkyl oder Heteroaryl mit 1 bis 3 Ringen, wobei jeder Ring 5 bis 7 Atome mit 0 - 3 Heteroatomen, ausgewählt aus N, O und S, aufweist, vorausgesetzt, daß zumindest ein Ring ein Heteroatom enthält, wobei die Substituenten ausgewählt sind aus Halogen (F, Cl, Br, 1), Cyano, Azido, Nitro, Hydroxy, Mercapto, Sulfonamido, Ureido, Thioureido, Carboxamido, Carbamoyloxy, Formyloxy, Formyl, C₁-C₄-Alkyl, C₁-C₄-Alkoxy, Phenyl und Phenoxy, wobei sich gegebenenfalls R¹ und R, falls sie an benachbarte Kohlenstoffatome gebunden sind, zu einem unsubstituierten oder substituierten Arylring verbinden können, wobei die Substituenten ausgewählt sind aus: Halogen (F, Cl, Br, I), Cyano, Azido, Nitro, Hydroxy, Mercapto, Sulfonamido, Ureido, Thioureido, Carboxamido, Carbamoyloxy, Formyloxy, Formyl, C₁-C₄-Alkyl, C₁-C₄-Alkoxy, Phenyl und Phenoxy;
Y¹, Y, Y³, Y⁴ und Y⁵ unabhängig voneinander ausgewählt sind aus: CH, CR¹, CR und N;
X ausgewählt ist aus: O, S, = NCN und = NO-(C₁-C₃)-Alkyl;
Z¹ ausgewählt ist aus: CH₂, NH, S und O;
Q¹ aus der Gruppe ausgewählt ist, die besteht aus:
(A) einer Aminogruppe, ausgewählt aus:
-NH₂,
-NR³H,
-NR³R⁴ und
-NR³R⁴R⁵,
worin R³, R⁴ und R⁵ unabhängig voneinander ausgewählt sind aus:
(i) Cyano,
(ii) einem gegebenenfalls substituierten Rest ausgewählt aus:
(a) -NR⁶R⁷,
(b) -C(=NR⁸)-NR⁶R⁷,
(c) -N=CR⁹-NR⁶R⁷,
(d) -NR¹⁰-CR⁹=NR⁸ und
(e) -NR¹⁰-C(=NR⁸)-NR⁶R⁷,
worin R⁶, R⁷, R⁸, R⁹ und R¹⁰ jeweils unabhängig voneinander ausgewählt sind aus:
Wasserstoff,
C₁-C₄-Alkoxy,
C₁-C₄-Alkyl und
(mit F, Cl, Br, I) halogeniertem C₁-C₄-Alkyl,
(iii) gegebenenfalls substituiertem C₁-C₁₂-Alkyl,
(iv) gegebenenfalls substituiertem C₃-C₁₂-Cycloalkyl,
(v) gegebenenfalls substituiertem C₆-C₁₄-Aryl,
(vi) gegebenenfalls substituiertem C₁-C₆-Alkyl-C₆-C₁₄-aryl,
(vii) gegebenenfalls substituiertem C₁-C₈-Alkoxy,
(viii) gegebenenfalls substituiertem C₆-C₁₄-Aryloxy und
(xi) gegebenenfalls substituiertem C₁-C₈-Alkoxycarbonyl,
worin die Substituenten ein bis drei Reste R¹¹ sind, die jeweils
unabhängig voneinander ausgewählt sind aus:
(a) gegebenenfalls substituiertem C₆-C₁₂-Aryloxy,
(b) gegebenenfalls substituiertem C₆-C₁₂-Arylamino,
(c) gegebenenfalls substituiertem C₆-C₁₂-Aroyl,
(d) gegebenenfalls substituiertem C₁-C₈-Alkoxy,
(e) gegebenenfalls substituiertem C₁-C₈-Alkoxyalkyl,
(f) gegebenenfalls substituiertem C₁-C₈-Alkoxyalkyloxy,
(g) gegebenenfalls substituiertem C₁-C₈-Alkoxycarbonyl,
(h) gegebenenfalls substituiertem C₁-C₈-Alkylcarbonyl,
(i) gegebenenfalls substituiertem C₁-C₈-Aralkylcarbonyl,
(j) gegebenenfalls substituiertem C₁-C₈-Alkylthiocarbonyl,
(k) gegebenenfalls substituiertem C₆-C₁₂-Aralkylthiocarbonyl,
(l) gegebenenfalls substituiertem C₂-C₈-Alkoxythiocarbonyl,
(m) gegebenenfalls substituiertem C₆-C₁₂-Aryl,
(n) gegebenenfalls substituiertem C₁-C₄-Alkanoylamino,
(o) gegebenenfalls substituiertem C₁-C₆-Alkoxycarbonyl-C₀-C₆-alkylamino,
(p) gegebenenfalls substituiertem C₁-C₈-Alkylsulfonylamino,
(q) gegebenenfalls substituiertem C₆-C₁₀-Aralkylsulfonylamino,
(r) gegebenenfalls substituiertem C₆-C₁₀-Aralkyl,
(s) gegebenenfalls substituiertem C₆-C₁₀-Alkaryl,
(t) gegebenenfalls substituiertem C₁-C₈-Alkylthio,
(u) gegebenenfalls substituiertem C₆-C₁₀-Aralkylthio,
(v) gegebenenfalls substituiertem C₁-C₈-Alkylsulfinyl,
(w) gegebenenfalls substituiertem C₆-C₁₀-Aralkylsulfinyl,
(x) gegebenenfalls substituiertem C₁-C₈-Alkylsulfonyl,
(y) gegebenenfalls substituiertem C₆-C₁₀-Aralkylsulfonyl,
(z) gegebenenfalls substituiertem C₁-C₈-Alkylaminosulfonyl,
(aa) gegebenenfalls substituiertem C₆-C₁₀-Aralkylsulfonylamino,
(ab) gegebenenfalls substituierten 5- oder 6-gliedrigen
Heterocyclen mit 1 - 4 Heteroatomen, ausgewählt aus N, O und S, und
(ac) gegebenenfalls substituiertem C₂-C₈-Alkenyl,
worin die Substituenten ein bis drei Reste R¹ sind, die jeweils
unabhängig voneinander ausgewählt sind aus:
Nitro,
Amino,
C₁-C₈-Alkylamino,
Di-(C₁-C₈)-alkylamino,
Amidino,
Aminomethylenimino,
Imino,
Imino-C₁-C₄-Alkyl,
Iminomethylenamino,
Guanidino,
C₆-C₁₀-Arylamino,
C₁-C₈-Acylamino,
C₁-C₄-Alkylsulfonamino,
Azido,
Cyano,
Hydroxy,
Hydroxy-C₁-C₈-alkyl,
C₁-C₈-Alkoxy,
Phenyloxy,
C₁-C₈-Alkanoyloxy,
C₁-C₈-Alkanoyl,
C₆-C₁₂-Aroyl,
Benzamido,
Phenyl,
Halogen (F, Cl, Br, I),
halogeniertem C₁-C₈-Alkyl und
C₁-C₈-Alkyl,
(ad) Aminosulfonyl,
(ae) Oxo,
(af) Thio,
(ag) Thiocarbonyl,
(ah) Hydroxy,
(ai) Mercapto,
(aj) Formyl,
(ak) Formyloxy,
(al) Carboxy,
(am) Amino,
(an) Ureido,
(ao) Amidino,
(ap) Guanidino,
(aq) Aminomethylenimino,
(ar) Imino,
(as) Glycyl,
(at) Phthalimido,
(au) Succinimido,
(av) Morpholino,
(aw) C₃-C₇-Cycloalkyl, und
(ax) Halogen (F, Cl, Br, 1),
wobei R³ und R⁴ gegebenenfalls zusammen ein gegebenenfalls substituiertes Tetramethylen,
Pentamethylen,
3-Oxopentamethylen und
3-Azapentamethylen,
bilden können
worin die Substituenten ausgewählt sind aus ein bis drei Resten R¹,
(B) einer Amidino-Gruppe, ausgewählt aus
-C(=NH)-NH₂,
-C(=NH)-NHR³,
-C(=N R⁴)-NHR³,
-C(=NH)-NR³R⁴ und
-C(=NR⁵)-NR³R⁴,
worin R³, R⁴ und R⁵ wie zuvor definiert sind,
(C) einer Aminoalkylenimino-Gruppe, ausgewählt aus:
-N=CH-NH₂,
-N=CH-NHR³,
-N=CH-NR³R⁴ und
-N=CR⁵-NR³R⁴,
worin R³, R⁴ und R⁵ wie zuvor definiert sind,
(D) einer Iminoalkylenamino-Gruppe, ausgewählt aus:
-NH-CH=NH,
-NH-CH=NR³,
-NH-CR⁴=NR³ und
-NR⁵-CR⁴=NR³,
worin R³, R⁴ und R⁵ wie zuvor definiert sind,
(E) einer Guanidino-Gruppe, ausgewählt aus:
-NH-C(=NH)-NH₂,
-NH-C(=NH)-NR³H,
-NH-C(=NH)-NR³R⁴,
-NH-C(=NR⁵)-NR³R⁴,
-NR³-C(=NR³)-NR³R⁴,
-NR³-C(=NH)-NR³R⁴,
-NR³-C(=NR³)-NH₂,
-NR³-C(=NH)-NH₂,
-NR³-C(=NR³)-NHR⁴ und
-NR³-C(=NH)-NHR⁴,
worin R³, R⁴ und R⁵ wie zuvor definiert sind;
(F) einer gegebenenfalls substituierten, gesättigten heterozyklischen Gruppe, ausgewählt aus: 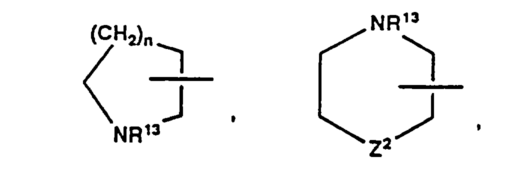 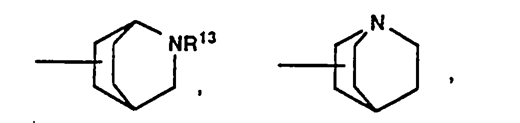 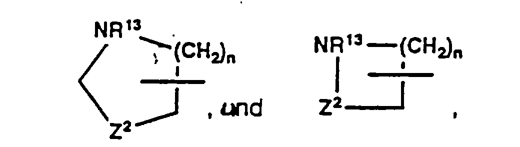
worin (1) n = 0, 1, 2 oder 3 ist, (2) R¹³ ausgewählt ist aus: R⁶, -CR⁹=NR⁸, -CR⁹(=NR⁸)-NR⁶R⁷, -C(=NR⁸)-NR⁶R⁷, -N=CR⁹-NR⁶R⁷, -NR¹⁰-CR⁹=NR⁸ und -NR¹⁰-(C=NR⁸)-NR⁶R⁷, worin R⁶ bis R¹⁰ wie zuvor definiert sind, (3) Z = O, S oder NR¹³ ist und (4) die Substituenten ein bis drei voneinander unabhängige Reste R¹ sind;
(G) einem gegebenenfalls substituierten, ungesättigten, (nichtaromatischen) heterozyklischen Rest, ausgewählt aus 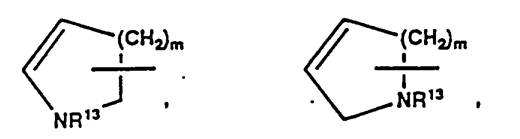 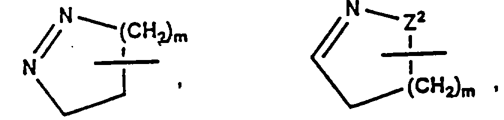 und 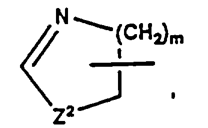
worin (1) m = 1, 2 oder 3 ist, (2) Z und R¹³ wie zuvor definiert sind und (3) die Substituenten ein bis drei voneinander unabhängige Reste R¹ sind;
(H) einem gegebenenfalls substituierten, ungesättigten, (aromatischen) heterozyklischen Rest, ausgewählt aus: 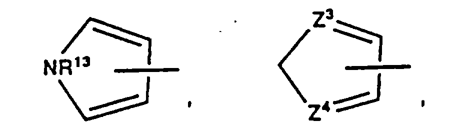 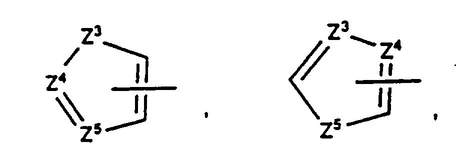 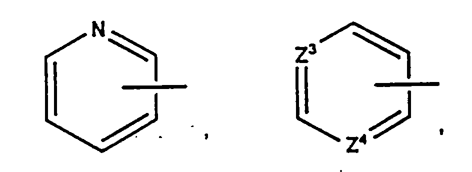 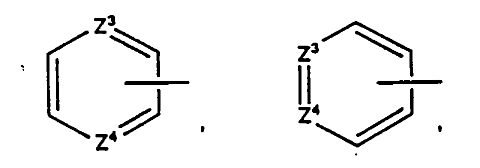 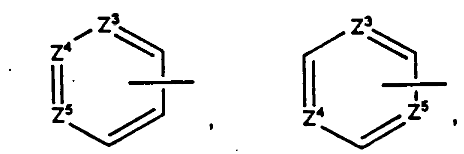 und 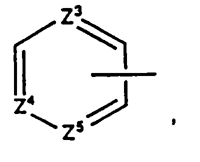
worin (1) Z³, Z⁴ und Z⁵ ausgewählt sind aus: O, S, N und NH, vorausgesetzt, daß zumindest einer von Z³, Z⁴ oder Z⁵ N oder NH ist, (2) R¹³ wie zuvor definiert ist und die Substituenten ein bis drei voneinander unabhängige Reste R¹ sind,
(I) einer gegebenenfalls substituierten, bicyclo-heterozyklischen Gruppe, ausgewählt aus: 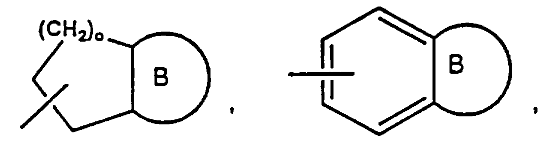 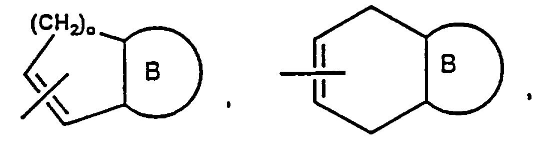 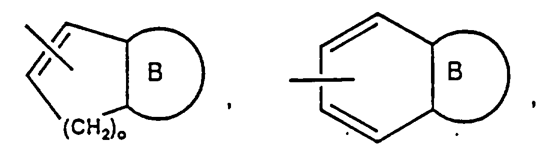 und 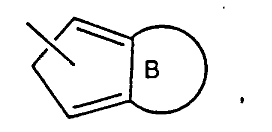
worin die Teilstruktur 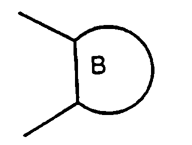
folgendes darstellt: 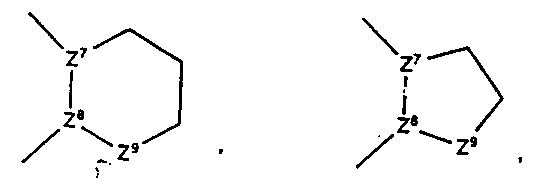 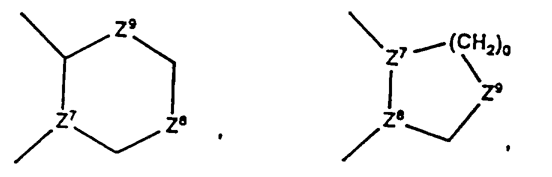 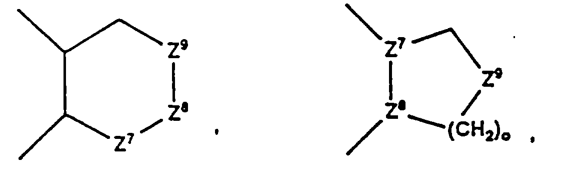 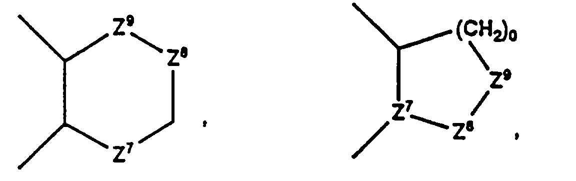 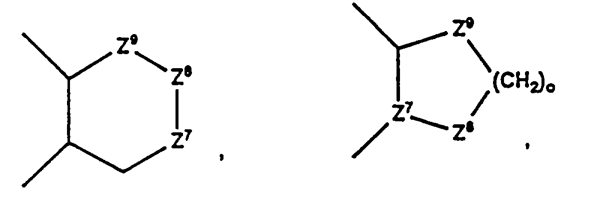 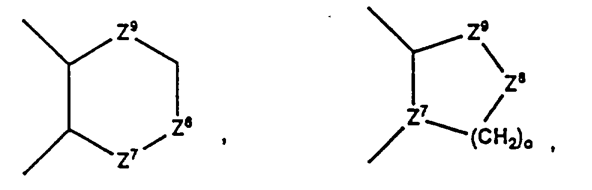 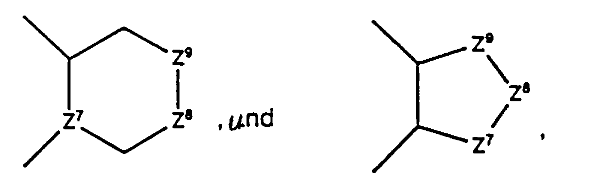
worin Z⁷, Z⁸ und Z9 unabhängig voneinander ausgewählt sind aus: 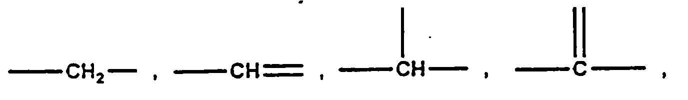 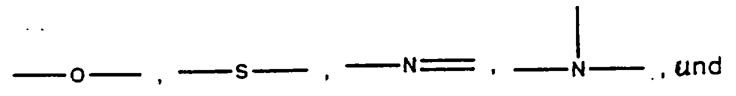 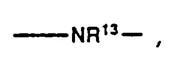
vorausgesetzt, daß zumindest einer von Z⁷, Z⁸ oder Z⁹ 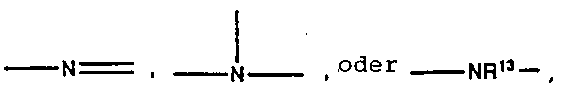 ist,
worin (1) o = 0, 1 oder 2 ist, (2) R¹³ wie zuvor definiert ist und (3) die Substituenten ein bis drei voneinander unabhängige Reste R¹ sind;
L¹ ein gegebenenfalls substituierter, zweiwertiger Rest ist, ausgewählt aus der Gruppe, bestehend aus:
(A) C₃-C₇-Alkylen,
(B) C₃-C₇-Cycloalkylen,
(C) C₃-C₇-Alkenylen,
(D) C₃-C₇-Alkadienylen,
(E) C₃-C₇-Alkinylen,
(F) C₄-C₇-Alkadiinylen,
(G) C₄-C₇-Alkeninylen,
(H) C₆-C₁₄-Arylen,
(I) C₅-C₁₄-Aryl-C₂-C₄-alkinylen,
(J) C₁-C₃-Alkyl-C₆-C₁₄-aryl-C₂-C₄-alkinylen,
(K) C₆-C₁₄-Aryl-C₂-C₄-alkenylen,
(L) C₁-C₃-Alkyl-C₆-C₁₄-arylen,
(M) C₁-C₃-Alkyl-C₆-C₁₄-aryl-C₂-C₄-alkenylen,
(N) C₆-C₁₄-Aryl-C₁-C₃-alkylen,
(O) C₆-C₁₄-Aryl-C₁-C₃-alkyloxyen,
(P) C₁-C₂-Alkyl-C₆-C₁₄-aryl-C₁-C₂-alkylen,
(Q) C₁-C₃-Alkyloxy-C₆-C₁₄-arylen,
(R) C₂-C₆-Alkyloxyen,
(S) C₁-C₅-Alkyloxy-C₁-C₅-alkylen,
(T) C₆-C₁₀-Aryloxyen,
(U) C₆-C₁₀-Aryloxy-C₁-C₅-alkylen,
(V) C₂-C₆-Alkylthioen,
(W) C₁-C₅-Alkylthio-C₁-C₅-alkylen,
(X) C₆-C₁₀-Arylthioen,
(Y) C₆-C₁₀-Arylthio-C₁-C₅-alkylen,
(Z) C₁-C₅-Alkylsulfoxid-C₁-C₅-alkylen,
(AA) C₁-C₅-Alkylsulfon-C₁-C₅-alkylen, 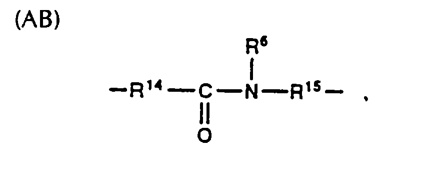 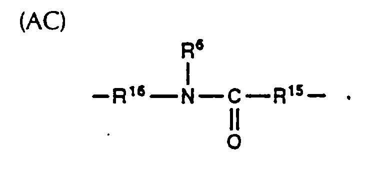 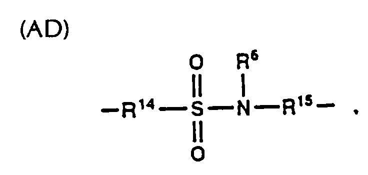 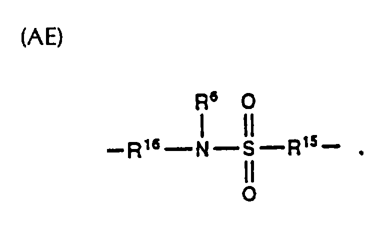 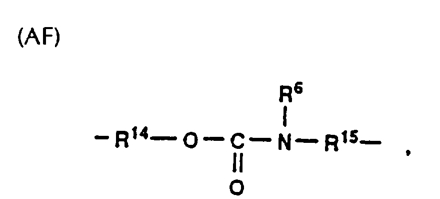 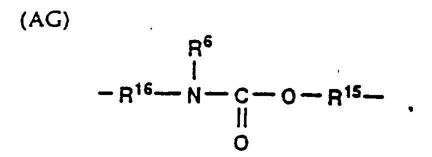 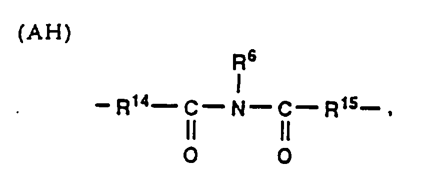 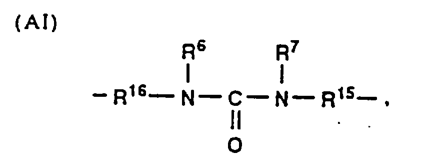 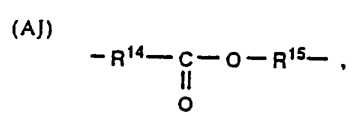 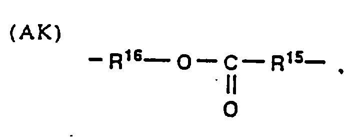 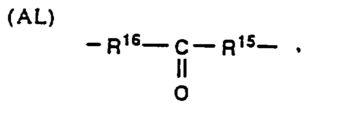 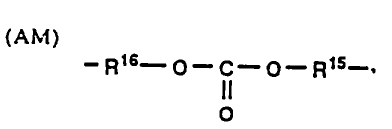 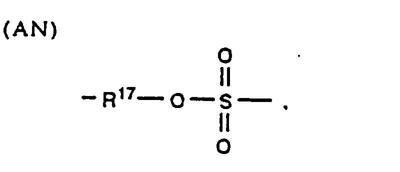 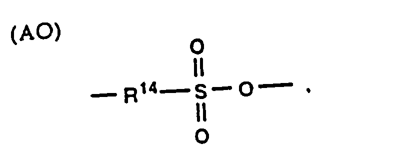 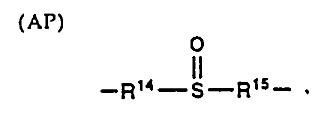 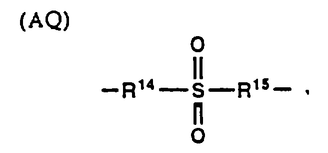 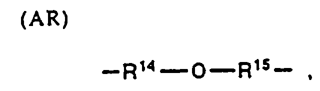 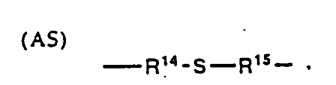 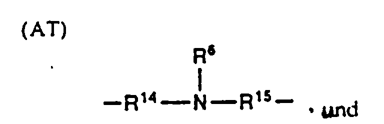 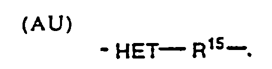
worin:
R¹⁴ ausgewählt ist aus:
einer chemischen Bindung,
C₁-C₈-Alkyl,
C₃-C₇-Cycloalkyl
C₂-C₅-Alkenyl,
C₃-c₅-Alkinyl,
C₆-C₁₀-Aryl,
C₁-C₃-Alkyl-C₆-C₁₂-aryl,
C₁-C₂-Alkyl-C₆-C₁₀-aryl-C₁-C₂-alkyl,
C₆-C₁₀-Aryl-C₁-C₂-alkyl und
C₆-C₁₀-Aryloxy-C₁-C₂-alkyl,
R¹⁵ ausgewählt ist aus:
einer chemischen Bindung,
C₁-C₄-Alkyl,
C₂-C₄-Alkenyl,
C₂-C₄-Alkinyl,
C₆-C₁₀-Aryl und
C₁-C₃-Alkyl-C₆-C₁₂-aryl,
R¹⁶ ausgewählt ist aus
einer chemischen Bindung,
C₁-C₅-Alkyl,
C₃-C₇-Cycloalkyl
C₃-C₅-Alkenyl,
C₃-C₅-Alkinyl,
C₆-C₁₀-Aryl,
C₁-C₃-Alkyl-C₆-C₁₂-aryl und
C₆-C₁₀-Aryl-C₁-C₂-alkyl,
R¹⁷ ausgewählt ist aus:
C₃-C₄-Alkenyl,
C₃-C₄-Alkinyl,
C₆-C₁₀-Aryl und
Benzyl; und
HET ein Heterozyklus mit 5 - 14 Atomen in 1 - 3 Ringen ist, wobei jeder Ring 1 - 3 Heteroatome aufweist, ausgewählt aus N, O und S,
worin die Substituenten aus ein bis drei Resten R¹ ausgewählt sind;
L ein gegebenenfalls substituierter, zweiwertiger Rest ist, ausgewählt aus der Gruppe, bestehend aus: 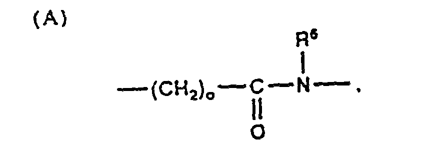 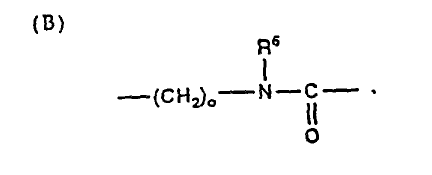 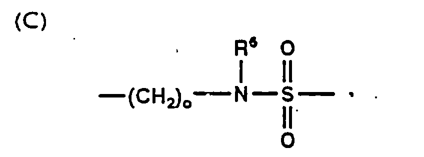 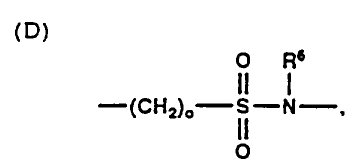 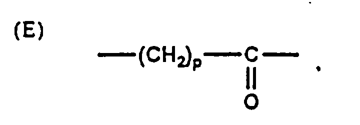 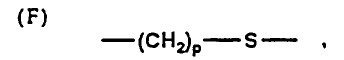 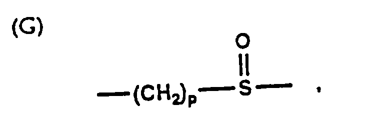 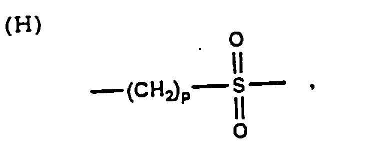 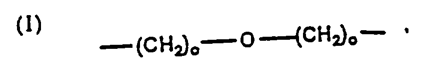 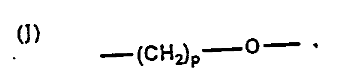 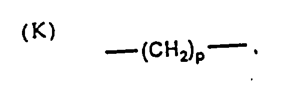 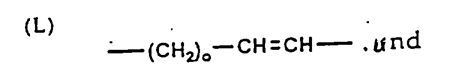 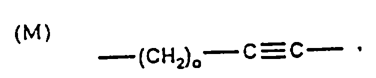
worin:
o - 1 oder 2 ist,
p - 1, 2, 3 oder 4 ist,
R⁶ wie zuvor definiert ist, und
worin die Substituenten aus ein bis drei Resten R¹ ausgewählt sind;
T-U-G aus der Gruppe ausgewählt ist, bestehend aus: 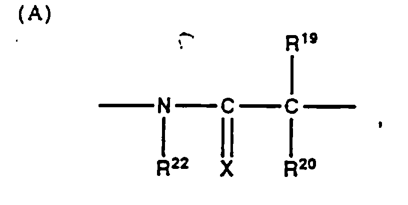 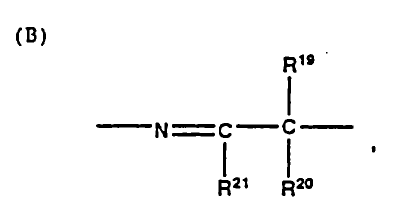 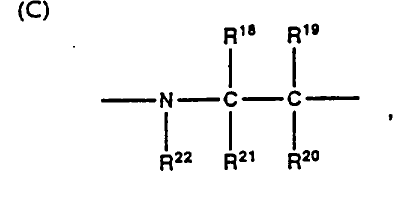 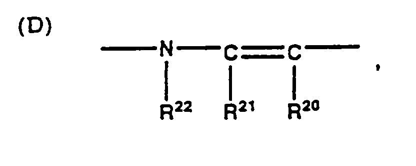 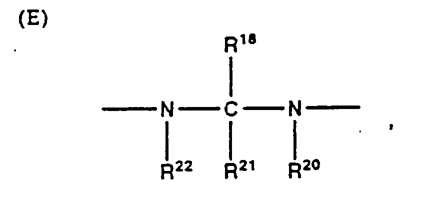 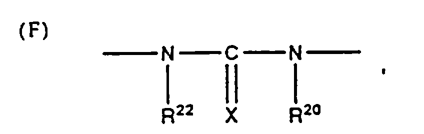 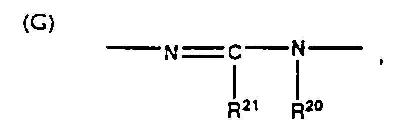 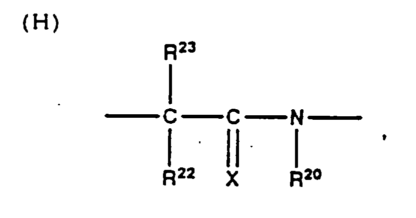 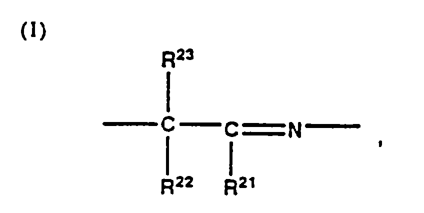 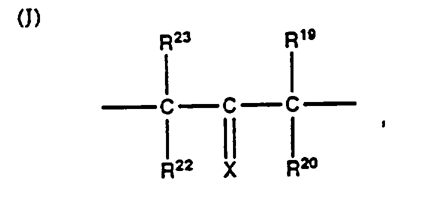 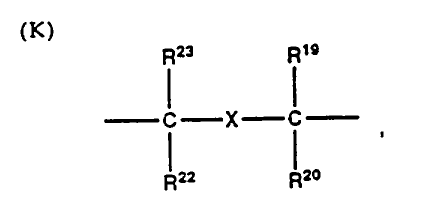 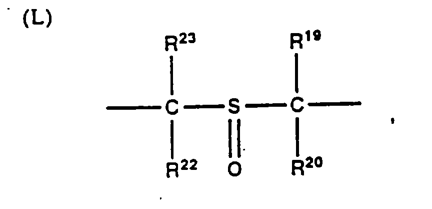 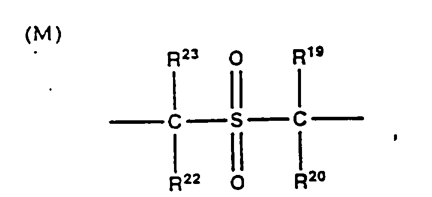 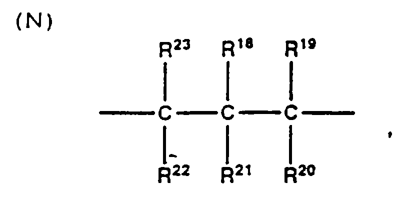 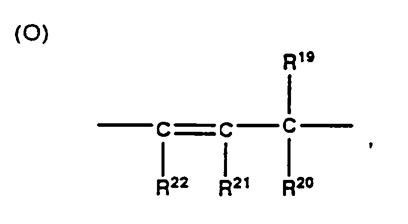 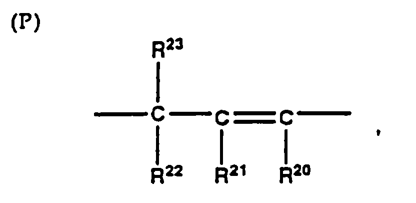 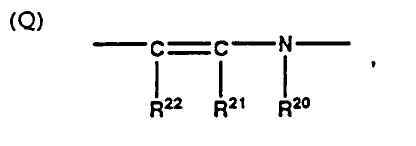 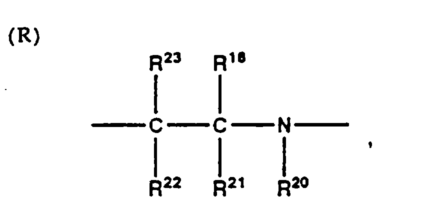 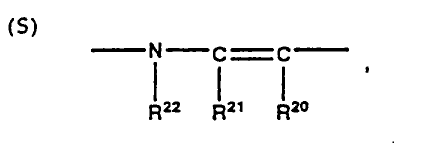 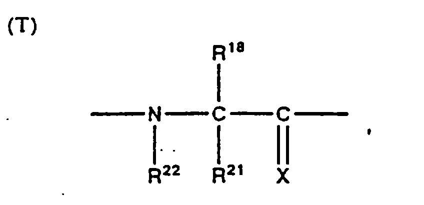 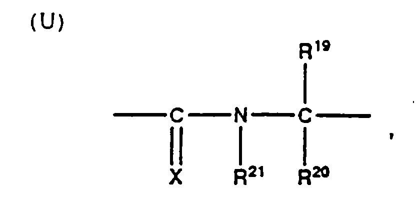 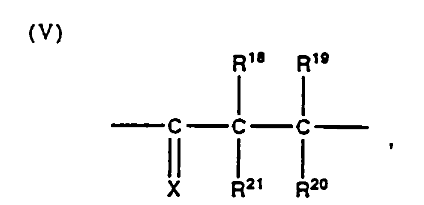 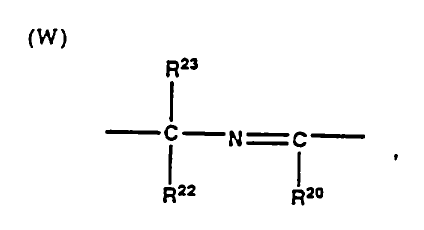 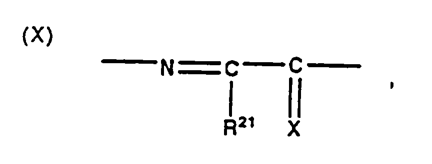 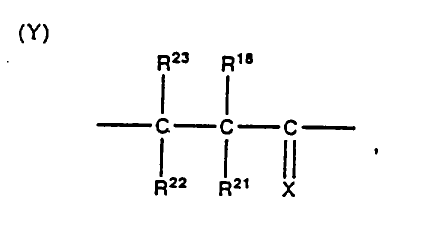 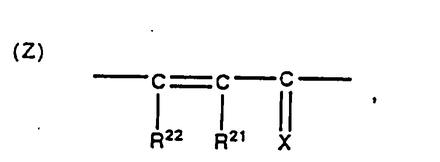 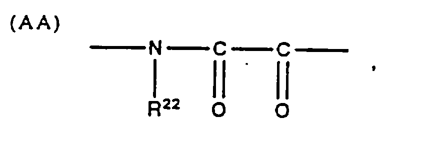 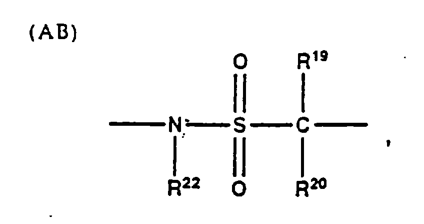 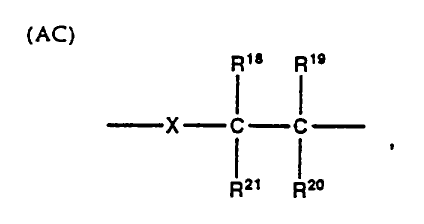 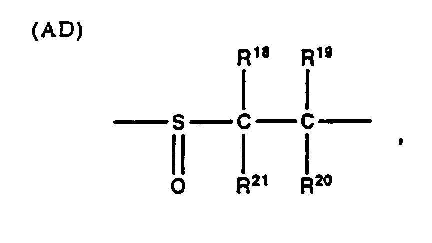 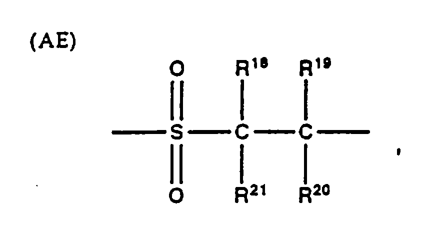 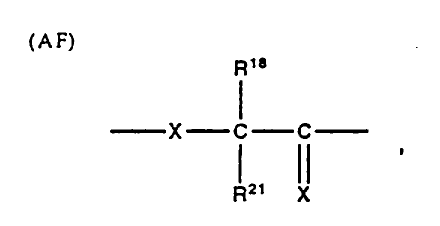 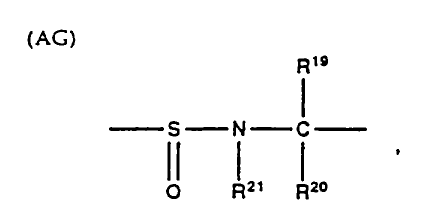 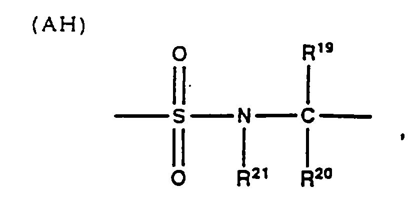 und 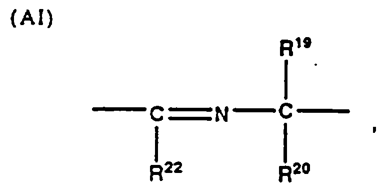
worin:
X = O oder S ist;
R¹⁸ und R³ unabhängig voneinander ausgewählt sind aus der Gruppe, bestehend aus:
(i) Wasserstoff,
(ii) gegebenenfalls substituiertem C₁-C₁₂-Alkyl,
(iii) gegebenenfalls substituiertem C₃-C₁₂-Alkenyl,
(iv) gegebenenfalls substituiertem C₃-C₁₄-Cycloalkyl,
(v) gegebenenfalls substituiertem C₁-C₁₂-Alkyl-C₆-C₁₄-aryl,
(vi) gegebenenfalls substituiertem C₆-C₁₄-Aryl,
(vii) gegebenenfalls substituiertem C₁-C₄-Alkylphenyl und
(viii) gegebenenfalls substituiertem C₁-C₁₂-Alkoxy,
R¹⁹ und R⁰ unabhängig voneinander ausgewählt sind aus:
(i) Wasserstoff,
(ii) Halogen (F, Cl, Br, I),
(iii) C₁-C₄-Alkoxy,
(iv) C₁-C₁₄-Alkyl,
(v) Phenyl,
(vi) Benzyl und
(vii) (mit F, Cl, Br, I) halogeniertem C₁-C₄-Alkyl,
R¹ und R unabhängig voneinander ausgewählt sind aus der Gruppe, bestehend aus:
(i) Wasserstoff,
(ii) gegebenenfalls substituiertem C₁-C₁₂-Alkyl,
(iii) gegebenenfalls substituiertem C₆-C₁₄-Aryl,
(iv) gegebenenfalls substituiertem C₃-C₁₄-Cycloalkyl,
(v) gegebenenfalls substituiertem C₁-C₁₂-Alkyl-C₆-C₁₄-aryl und
(vi) gegebenenfalls substituiertem C₁-C₁₂-Alkyl-C₃-C₁₄-cycloalkyl,
worin die Substituenten ausgewählt sind aus:
(a) Halogen (F, Cl, Br, 1),
(b) Nitro,
(c) Hydroxy,
(d) Carboxy,
(e) Tetrazol,
(f) Hydroxamat,
(g) Sulfonamid,
(h) Trifluorimid,
(i) Phosphonat,
(j) C₁-C₆-Alkyl,
(k) C₆-C₁₄-Aryl,
(l) Benzyl,
(m) C₃-C₁₄-Cycloalkyl,
(n) COR⁴
worin R⁴ ausgewählt ist aus der Gruppe, bestehend aus:
C₁-C₈-Alkoxy,
C₃-C₁₂-Alkenoxy,
C₆-C₁₂-Aryloxy,
C₁-C₆-Alkyl-C₆-C₁₂-aryloxy,
Di-C₁-C₈-alkylamino-C₁-C₈-alkoxy,
Acylamino-C₁-C₈-alkoxy, ausgewählt aus der Gruppe
Acetylaminoethoxy,
Nicotinoylaminoethoxy und
Succinamidoethoxy,
C₁-C₈-Alkoyloxy-C₁-C₈-alkoxy und
C₆-C₁₂-Aryl-C₁-C₈-alkoxy worin die Aryl-Gruppe unsubstituiert oder mit einer bis drei der Gruppen
Nitro,
Halogen (F, Cl, Br, I),
C₁-C₄-Alkoxy,
Amino,
Hydroxy,
Hydroxy-C₂-C₈-alkoxy und
Dihydroxy-C₃-C₈-alkoxy substituiert ist; und
(o) CONR⁵R⁶
worin R⁵ und R⁶ unabhängig voneinander ausgewählt sind aus:
Wasserstoff,
C₁-C₁₀-Alkyl,
C₃-C₁₀-Alkenyl,
C₆-C₁₄-Aryl,
C₁-C₆-Alkyl-C₆-C₁₀-aryl,
wobei gegebenenfalls R⁵ und R⁶ zusammen
Trimethylen,
Tetramethylen,
Pentamethylen und
3-Oxopentamethylen bilden können; und
(vii) Q-L³-
worin Q ausgewählt ist aus:
Wasserstoff und
Q¹ und
L³ ausgewählt ist aus:
einer chemischen Bindung,
L¹ und
L;
D ausgewählt ist aus der Gruppe, bestehend aus:
R und
-(C=O)-Xaa, worin Xaa ein bis drei D- oder L-α-Aminosäurereste darstellt;
W = -R⁷-w ist, worin:
R⁷ ausgewählt ist aus:
(a) einer kovalenten Bindung,
(b) substituiertem oder unsubstituiertem Methylen und
(c) substituiertem oder unsubstituiertem Ethylen,
worin die Substituenten ausgewählt sind aus:
(i) Nitro,
(ii) Halogen (F, Cl, Br, l),
(iii) C₁-C₆-Alkyl,
(iv) (mit F, Cl, Br, I) halogeniertem C₁-C₆-Alkyl und
(v) substituiertem oder unsubstituiertem Phenyl
worin die Substituenten am Phenyl ausgewählt sind aus:
(1) C₁-C₆-Alkyl,
(2) C₁-C₆-Alkoxy,
(3) Halogen (F, Cl, Br, I) und
(4) CF₃;
w ausgewählt ist aus:
(a) -COR⁸,
(b) -SO₃R³¹,
(c) -NHSO₂R³ ,
(d) -PO(OR³¹)₂,
(e) -SO₂NHR³,
(f) -CONHOR³¹,
(g) -C(OH)R³³PO(OR³³)₂,
(h) -CN,
(i) einem -SO₂NH-Heterozyklus, worin der Heterozyklus ein 5- oder 6-gliedriger aromatischer Ring mit 1 bis 3 Heteroatomen ist, ausgewählt aus O, N und S, und worin der Heterozyklus unsubstituiert oder mit einem oder zwei Substituenten, ausgewählt aus der Gruppe:
(i) -OH,
(ii) -SH,
(iii) -(C₁-C₄-Alkyl),
(iv) -C₁-C₄-Alkoxy,
(v) CF₃,
(vi) Halogen (F, Cl, Br, I),
(vii) NO₂,
(viii) -COOH,
(ix) -COO-(C₁-C₄-Alkyl),
(x) -NH₂,
(xi) -NH(C₁-C₄-Alkyl) und
(xii) -N-(C₁-C₄-Alkyl)₂ substituiert ist;
(j) einem -CH₂SO₂NH-Heterozyklus,
(k) -SO₂NHCOR³³,
(l) -CH₂SO₂NHCOR³,
(m) -CONHSO₂R³³,
(n) -CH₂CONHSO₂R³³,
(o) -NHCONHSO₂R³³,
(p) -NHSO₂NHCOR³³,
(q) -CONHNHSO₂CF₃,
(r) -CON(OH)R³¹,
(s) -CONHCOCF₃,
(t) -CONHSO₂R⁸,
(u) -CONHSO₂R⁹,
(v) -CONHSO₂R³⁰, 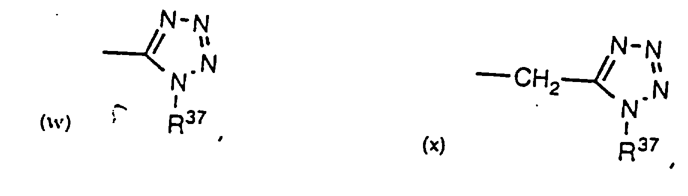 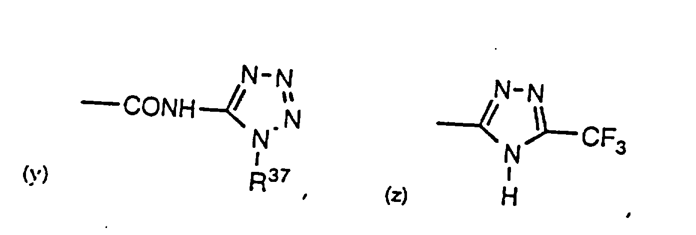 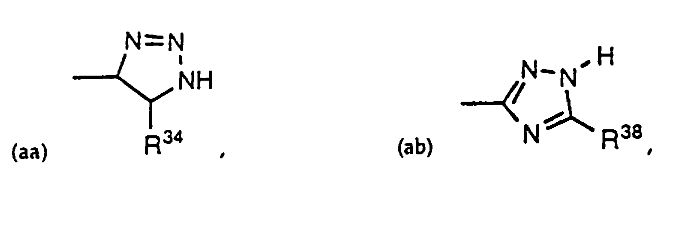 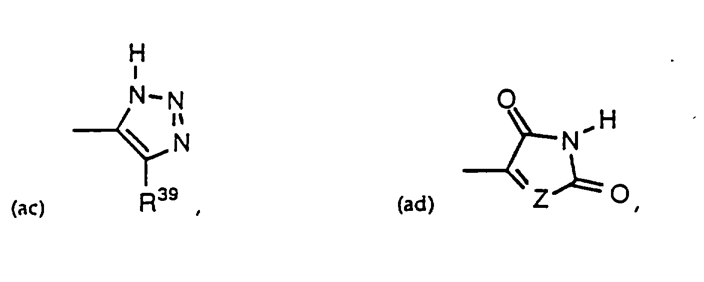 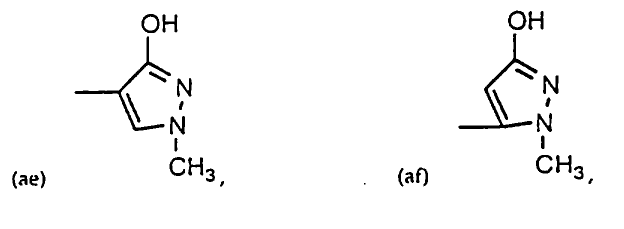 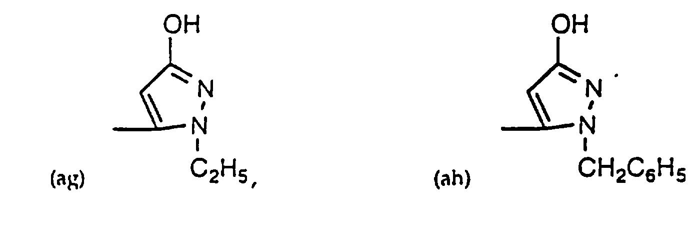 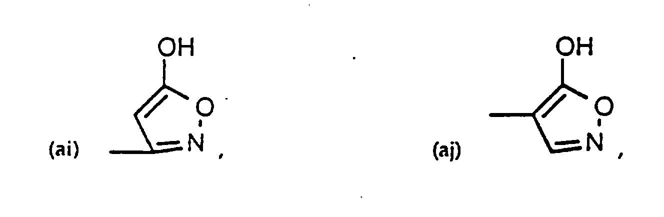 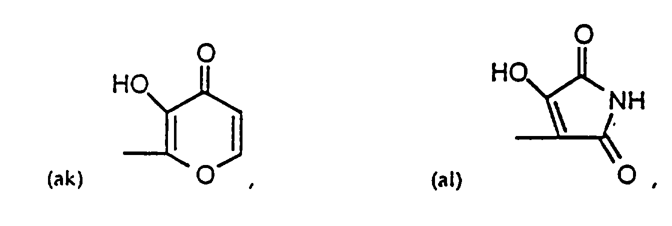 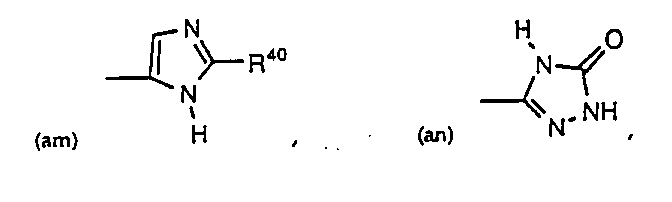 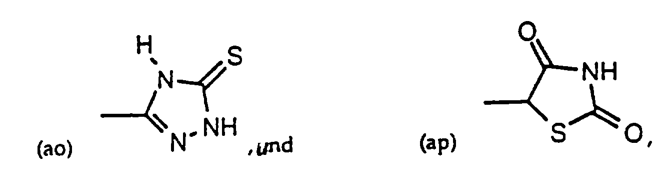
R⁸ ausgewählt ist aus der Gruppe, bestehend aus:
(a) Hydroxy,
(b) C₁-C₈-Alkoxy,
(c) C₃-C₁₂-Alkenoxy,
(d) C₆-C₁₂-Aryloxy,
(e) C₁-C₆-Alkyl-C₆-C₁₂-Aryloxy,
(f) Di-C₁-C₈-alkylamino-C₁-C₈-alkoxy,
(g) Acylamino-C₁-C₈-alkoxy, ausgewählt aus der Gruppe:
(i) Acetylaminoethoxy,
(ii) Nicotinoylaminoethoxy und
(iii) Succinamidoethoxy,
(h)C₁-C₈-Alkoyloxy-C₁-C₈-alkoxy,
(i) C₆-C₁₂-Aryl-C₁-C₈-alkoxy,
worin alle Aryl-Gruppen unsubstituiert oder mit ein bis drei der Gruppen:
(i) Nitro,
(ii) Halogen (F, Cl, Br, l),
(iii) C₁-C₄-Alkoxy und
(iv) Amino substituiert sind;
(j) Hydroxy-C₂-C₈alkoxy,
(k) Dihydroxy-C₃-C₈-alkoxy und
(l) NR⁹R³⁰;
R⁹ und R³⁰ unabhängig voneinander ausgewählt sind aus der Gruppe:
(a) Wasserstoff,
(b) C₁-C₈-Alkyl,
(c) C₃-C₈-Alkenyl,
(d) C₆-C₁₂-Aryl,
(e) C₆-C₁₂-Aryl-C₁-C₈-alkyl
worin alle Aryl-Gruppen unsubstituiert oder mit ein bis drei der Gruppen:
(i) Nitro,
(ii) Halogen (F, Cl, Br, l),
(iii)C₁-C₄-Alkoxy und
(iv) Amino substituiert sind;
R³¹ ausgewählt ist aus der Gruppe, bestehend aus:
(a) Wasserstoff,
(b) C₁-C₆-Alkyl,
(c) (mit F, Cl, Br, I) halogeniertem C₁-C₆-Alkyl,
(d) Phenyl,
(e) Benzyl und
(f) -CH₂-O-COCH₃;
R³ ausgewählt ist aus der Gruppe, bestehend aus:
(a) Wasserstoff,
(b) Benzyl und
(c) -CH(R³⁵)-O-C(O)R³⁵;
R³³ ausgewählt ist aus der Gruppe, bestehend aus:
(a) Aryl,
(b) Heteroaryl,
(c) (C₃-C₇)-Cycloalkyl,
(d) (C₁-C₄)-Alkyl, unsubstituiert oder substituiert mit einem Substituenten, ausgewählt aus der Gruppe, bestehend aus:
(i) Aryl,
(ii) Heteroaryl,
(iii) -OH,
(iv) -SH,
(v) (C₁-C₄)-Alkyl,
(vi) (C₁-C₄)-Alkoxy,
(vii) (C₁-C₄)-Alkylthio,
(viii) -CF_{3,}
(ix) Halogen (F, Cl, Br, I),
(x) -NO₂,
(xi) -CO₂H,
(xii) CO₂-(C₁-C₄)-Alkyl,
(xiii) -NH₂,
(xiv) -N[(C₁-C₄)-Alkyl]₂,
(xv) -NH[(C₁-C₄)-Alkyl],
(xvi) -PO₃H und
(xvii) PO(OH)(C₁-C₄)-Alkoxy; und
(e) (C₁-C₄)-Perfluoralkyl;
R³⁴ ausgewählt ist aus der Gruppe, bestehend aus:
(a) -CN,
(b) -NO₂,
(c) -COOR³¹,
(d) C₁-C₆-Perfluoralkyl und
(e) CF₃;
R³⁵ jeweils unabhängig voneinander ausgewählt ist aus der Gruppe, bestehend aus:
(a) Wasserstoff,
(b) gegebenenfalls substituiertem (C₁-C₆)-Alkyl,
(c) gegebenenfalls substituiertem (C₂-C₆)-Alkenyl,
(d) gegebenenfalls substituiertem (C₂-C₆)-Alkinyl und
(e) gegebenenfalls substituiertem(C₃-C₈)Cycloalkyl,
worin die Substituenten ausgewählt sind aus der Gruppe:
(i) OH,
(ii) (C₁-C₄)-Alkoxy,
(iii) CO₂R³³,
(iv) OCOR³³,
(v) CONHR³³,
(vi) CON(R³³)₂,
(vii) N(R³³)C(O)R³³,
(viii) NH₂,
(ix) (C₁-C₄)-Alkylamino,
(x) Di-[(C₁-C₄)-alkyl]amino,
(xi) Aryl und
(xii) Heteroaryl,
(f) -C(O)-Aryl,
(g) -NO₂,
(h) Halogen (Cl, Br, I, F),
(i) -OH,
(j) -OR³⁶,
(k) -(C₁-C₄)-Perfluoralkyl,
(l) -SH,
(m) -S(O)₁₋₂-(C₁-C₄)-Alkyl,
(n) -CO₂R³³,
(o) -SO₃H,
(p) -NR³³R³⁶,
(q) -NR³³C(O)R³⁶,
(r) -NR³³COOR³,
(s) -SO₂NHR³,
(t) -SO₂NR³³R³³,
(u) -NHSO₂R³,
(v) -C(O)NHSO₂R³,
(w) Aryl,
(x) Heterozyklen,
(y) Morpholin-4-yl,
(z) CONH₂ und
(aa) 1H-tetrazol-5-yl;
R³⁶ ausgewählt ist aus der Gruppe:
(a) Wasserstoff und
(b) (C₁-C₄)-Alkyl, unsubstituiert oder substituiert mit
(i) NH₂,
(ii) NH[(C₁-C₄)-Alkyl],
(iii) N[(C₁-C₄)-Alkyl]₂,
(iv) CO₂H,
(v) CO₂(C₁-C₄)-Alkyl,
(vi) OH,
(vii) SO₃H und
(viii) SO₂NH₂;
R³⁷ ausgewählt ist aus der Gruppe, bestehend aus:
(a) Wasserstoff,
(b) (C₁-C₆)-Alkyl,
(c) (C₂-C₆)-Alkenyl,
(d) (C₁-C₆)-Alkoxyalkyl,
(e) -CH₂-O-COCH₃ und
(f) -CH₂-Phenyl, worin das Phenyl unsubstituiert oder mit einem Substituenten substituiert ist, der ausgewählt ist aus:
(i) -NO₂,
(ii) -NH₂,
(iii) -OH und
(iv) -OCH₃;
R³⁸, R³⁹ und R⁴⁰ jeweils unabhängig voneinander ausgewählt sind aus:
(a) Wasserstoff,
(b) Cl,
(c) CN,
(d) NO₂,
(e) CF₃,
(f) C₂F_{S},
(g) C₃F₇,
(h) CHF₂,
(i) CH₂F,
(j) CO₂CH₃,
(k) CO₂C₂H₅,
(l) SO₂CH₃,
(m) SO₂CF₃ und
(n) SO₂C₆F_{5,}
worin Z ausgewählt ist aus: O, S, NR⁴¹ und CH₂;
R⁴¹ ausgewählt ist aus: Wasserstoff, CH₃ und CH₂C₆H₅; und
pharmazeutisch annehmbare Salze davon.

2. Verbindung nach Anspruch 1, dargestellt durch Formel (II): 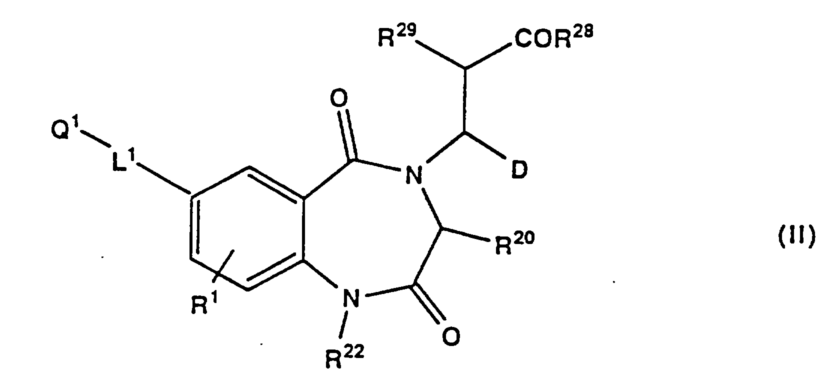
worin:
R¹ ein bis drei Gruppen darstellt, die unabhängig voneinander ausgewählt sind aus: Wasserstoff, Halogen (F, Cl, Br, I), Cyano, Carboxamido, Carbamoyloxy, Formyloxy, Formyl, Azido, Nitro, Ureido, Thioureido, Hydroxy, Mercapto, Sulfonamido und einem gegebenenfalls substituierten Rest, ausgewählt aus: C₁-C₁₂-Alkyl, C₂-C₁₂-Alkenyl, C₃-C₁₂-Alkinyl, C₃-C₁₂-Cycloalkyl, C₆-C₁₄-Aryl, C₆-C₁₀-Aryl-C₁-C₈-alkyl, C₁-C₁₂-Alkyloxy, C₆-C₁₄-Aryloxy und C₁-C₁₂-Acylamino, worin die Substituenten ausgewählt sind aus: Halogen (F, Cl, Br, l), Cyano, Azido, Nitro, Hydroxy, Mercapto, Sulfonamido, Ureido, Thioureido, Carboxamido, Carbamoyloxy, Formyloxy, Formyl, C₁-C₄-Alkyl, C₁-C₄-Alkoxy, Phenyl und Phenoxy;
Q¹ ausgewählt ist aus der Gruppe, bestehend aus:
(A) einer Amino-Gruppe, ausgewählt aus
-NH₂,
-NR³H,
-NR³R⁴ und
-NR³R⁴R⁵,
worin R³, R⁴ und R⁵ unabhängig voneinander ausgewählt sind aus: (i) einem gegebenenfalls substituierten Rest, ausgewählt aus: (a) -NR⁶R⁷, (b) -C(=NR⁸)-NR⁶R⁷, (c) -N=CR⁹-NR⁶R⁷, (d) -NR¹⁰-CR⁹=NR⁸ und (e) -NR¹⁰-C(=NR⁸)-NR⁶R⁷, worin R⁶, R⁷, R⁸, R⁹ und R¹⁰ jeweils unabhängig voneinander ausgewählt sind aus: Wasserstoff, C₁-C₄-Alkoxy, C₁-C₄-Alkyl und (mit F, Cl, Br, I) halogeniertem C₁-C₄-Alkyl, (ii) gegebenenfalls substituiertem C₁-C₁₂-Alkyl, (iii) gegebenenfalls substituiertem C₃-C₁₂-Cycloalkyl, (iv) gegebenenfalls substituiertem C₆-C₁₄-Aryl, (v) gegebenenfalls substituiertem C₁-C₆-Alkyl-C₆-C₁₄-aryl, (vi) gegebenenfalls substituiertem C₁-C₈-Alkoxy und (vii) gegebenenfalls substituiertem C₆-C₁₄-Aryloxy, worin die Substituenten ein bis drei Reste R¹¹ sind, die jeweils voneinander unabhängig ausgewählt sind aus: (a) gegebenenfalls substituiertem C₆-C₁₂-Aryloxy, (b) gegebenenfalls substituiertem C₆-C₁₂-Arylamino, (c) gegebenenfalls substituiertem C₆-C₁₂-Aroyl, worin die Substituenten ein bis drei Reste R¹ sind, die jeweils unabhängig voneinander ausgewählt sind aus: Nitro, Amino, C₁-C₈-Alkylamino, Di-(C₁-C₈)-alkylamino, Amidino, Aminomethylenimino, Imino, Imino-C₁-C₄-alkyl, Iminomethylenamino, Guanidino, C₆-C₁₀-Arylamino, C₁-C₈-Acylamino, C₁-C₄-Alkylsulfonamino, Azido, Cyano, Hydroxy, Hydroxy-C₁-C₈-alkyl, C₁-C₈-Alkoxy, Phenyloxy, C₁-C₈-Alkanoyloxy, C₁-C₈-Alkanoyl, C₆-C₁₂-Aroyl, Benzamido, Phenyl, Halogen (F, Cl, Br, I) halogeniertem C₁-C₈-Alkyl und C₁-C₈-Alkyl, (d) Halogen (F, Cl, Br, I), (e) Hydroxy, (f) Mercapto, (g) Formyl, (h) Formyloxy, (i) Carboxy, (j) Amino, (k) Ureido, (l) Amidino, (m) Guanidino, (n) Aminomethylimino, (o) Imino, (p) Glycyl, (q) Phthalimido, (r) Succinimido, (s) Morpholino und (t) C₃-C₇-Cycloalkyl, wobei gegebenenfalls R³ und R⁴ zusammen ein gegebenenfalls substituiertes Tetramethylen, Pentamethylen, 3-Oxopentamethylen und 3-Azapentamethylen bilden können, worin die Substituenten aus ein bis drei Resten R¹ ausgewählt sind,
(B) eine Amidino-Gruppe, ausgewählt aus:
-C(=NH)-NH₂,
-C(=NH)-NHR³,
-C(=NR⁴)-NHR³,
-C(=NH)-NR³R⁴ und
-C(=NR⁵)-NR³R⁴,
worin R³, R⁴ und R⁵ wie zuvor definiert sind,
(C) einer Aminoalkylenamino-Gruppe, ausgewählt aus:
-N=CH-NH₂,
-N=CH-NHR³,
-N=CH-NR³R⁴ und
-N=CR⁵-NR³R⁴,
worin R³, R⁴ und R⁵ wie zuvor definiert sind,
(D) einer Iminoalkylenamino-Gruppe, ausgewählt aus:
-NH-CH=NH,
-NH-CH=NR³,
-NH-CR⁴=NR³ und
-NR⁵-CR⁴=NR³,
worin R³, R⁴ und R⁵ wie zuvor definiert sind,
(E) einer Guanidino-Gruppe, ausgewählt aus:
-NH-C(=NH)-NH₂,
-NH-C(=NH)-NR³H,
-NH-C(=NH)-NR³R⁴,
-NH-C(=NR⁵)-NR³R⁴,
-NR³-C(=NR³)-NR³R⁴,
-NR³-C(=NH)-NR³R⁴,
-NR³-C(=NR³)-NH₂,
-NR³-C(=NH)-NH₂,
-NR³-C(=NR³)-NHR⁴ und
-NR³-C(=NH)-NHR⁴,
worin R³, R⁴ und R⁵ wie zuvor definiert sind, und
(F) einer gegebenenfalls substituierten, gesättigten, heterozyklischen Gruppe, ausgewählt aus: 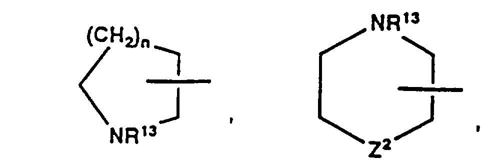 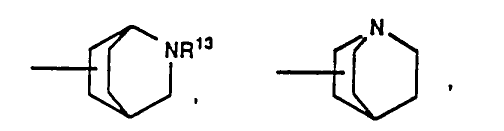 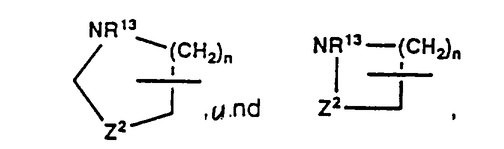
worin (1) n = 0, 1, 2 oder 3 ist, (2) R¹³ ausgewählt ist aus:; R⁶, -CR⁹=NR⁸, - CR⁹(=NR⁸)-NR⁶R⁷, -C(=NR⁸)-NR⁶R⁷, -N=CR⁹-NR⁶R⁷, -NR¹⁰-CR⁹=NR⁸ und -NR¹⁰-(C=NR⁸)-NR⁶R⁷, worin R⁶ bis R¹⁰ wie zuvor definiert sind, (3) Z O, S oder NR¹³ ist und (4) die Substituenten ein bis drei voneinander unabhängige Reste R¹ sind;
L¹ ein gegebenenfalls substituierter, zweiwertiger Rest ist, ausgewählt aus der Gruppe, bestehend aus: C₃-C₇-Alkylen, C₃-C₇-Cycloalkylen, C₃-C₇-Alkenylen, C₄-C₇-Cycloalkenylen, C₅-C₈-Cycloalkadienylen, C₃-C₇-Alkadienylen, C₃-C₇-Alkinylen, C₄-C₇-Alkeninylen, C₆-C₁₄-Arylen, C₆-C₁₄-Aryl-C₂-C₄-alkinylen, C₁-C₃-Alkyl-C₆-C₁₄-aryl-C₂-C₄-alkinylen, C₆-C₁₄-Aryl-C₂-C₄-alkenylen, C₁-C₃-Alkyl-C₆-C₁₄-arylen, C₁-C₃-Alkyl-C₆-C₁₄-aryl-C₂-C₄-alkenylen, C₆-C₁₄-Aryl-C₁-C₃-alkylen, C₆-C₁₄-Aryl-C₁-C₃-alkyloxyen, C₁-C₂-Alkyl-C₆-C₁₄-aryl-C₁-C₂-alkylen, C₁-C₃-Alkyloxy-C₆-C₁₄-arylen, C₂-C₈-Alkyloxyen, C₁-C₅-Alkyloxy-C₁-C₅-alkylen, C₆-C₁₀-Aryloxyen, C₆-C₁₀-Aryloxy-C₁-C₅-alkylen, C₆-C₁₀-Arylthio-C₁-C₅-alkylen, 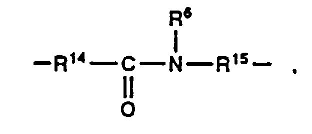 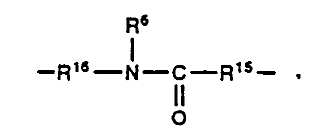 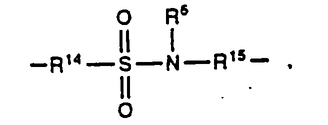 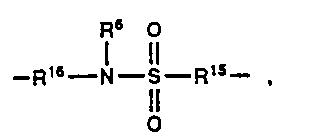 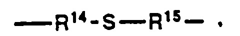 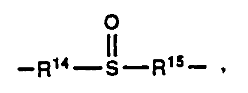 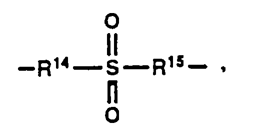 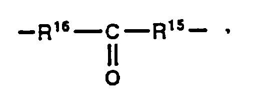 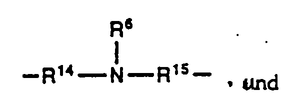 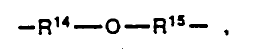 worin R¹⁴ ausgewählt ist aus: einer chemischen Bindung, C₁-C₈-Alkyl, C₃-C₇-Cycloalkyl, C₂-C₅-Alkenyl, C₃-C₅-Alkinyl, C₆-C₁₀-Aryl, C₁-C₃-Alkyl-C₆-C₁₂-aryl, C₁-C₂-Alkyl-C₆-C₁₀-aryl-C₁-C₂-alkyl und C₆-C₁₀-Aryl-C₁-C₂-alkyl, R¹⁵ ausgewählt ist aus: einer chemschen Bindung, C₁-C₄-Alkyl, C₂-C₄-Alkenyl, C₂-C₄-Alkinyl und C₆-C₁₀-Aryl, R¹⁶ ausgewählt ist aus: einer chemischen Bindung, C₁-C₅-Alkyl, C₃-C₇-Cycloalkyl, C₃-C₅-Alkenyl, C₃-C₅-Alkinyl, C₆-C₁₀-Aryl, C₁-C₃-Alkyl-C₆-C₁₂-aryl, C₁-C₃-Alkyl-C₆-C₁₂-aryl und C₆-C₁₀-Aryl-C₁-C₂-alkyl, worin die Substituenten aus ein bis drei Resten R¹ ausgewählt sind;
R⁰ ausgewählt ist aus der Gruppe: Wasserstoff, Halogen (F, Cl, Br, I), C₁-C₄-Alkoxy, C₁-C₄-Alkyl, Phenyl, Benzyl und (mit F, Cl, Br, I) halogeniertem C₁-C₄-Alkyl;
R ausgewählt ist aus der Gruppe, bestehend aus: (i) Wasserstoff, (ii) gegebenenfalls substituiertem C₁-C₁₂-Alkyl, (iii) gegebenenfalls substituiertem C₆-C₁₄-Aryl, (iv) gegebenenfalls substituiertem C₃-C₁₄-Cycloalkyl, (v) gegebenenfalls substituiertem C₁-C₁₂-Alkyl-C₆-C₁₄-aryl, (vi) gegebenenfalls substituiertem C₁-C₁₂-Alkyl-C₃-C₁₄-cycloalkyl, worin die Substituenten ausgewählt sind aus: (a) Halogen (F, Cl, Br, l), (b) Nitro, (c) Hydroxy, (d) Carboxy, (e) Tetrazole, (f) Hydroxamat, (g) Sulfonamid, (h) Trifluorimid, (i) Phosphonat, (j) C₁-C₆-Alkyl, (k) C₆-C₁₄-Aryl, (l) Benzyl, (m) C₃-C₁₄-Cycloalkyl, (n) COR⁴, worin R⁴ ausgewählt ist aus der Gruppe: C₁-C₈-Alkoxy, C₃-C₁₂-Alkenoxy, C₆-C₁₂-Aryloxy, Di-C₁-C₈-alkylamino-C₁-C₈-alkoxy, Acylamino-C₁-C₈-alkoxy, ausgewählt aus der Gruppe: Acetylaminoethoxy, Nicotinoylaminoethoxy, Succinamidoethoxy und Pivaloyloxyethoxy, und C₆-C₁₂-Aryl-C₁-C₈-alkoxy, worin die Aryl-Gruppe
unsubstituiert oder mit ein bis drei der Gruppen: Nitro, Halogen (F, Cl, Br, I), C₁-C₄-Alkoxy, Amino, Hydroxy, Hydroxy-C₂-C₈-alkoxy und Dihydroxy-C₃-C₈-alkoxy substituiert ist, (o) CONR⁵R⁶, worin R⁵ und R⁶ unabhängig voneinander ausgewählt sind aus: Wasserstoff, C₁-C₁₀-Alkyl, C₃-C₁₀-Alkenyl, C₆-C₁₄-Aryl, C₁-C₆-Alkyl-C₆-C₁₀-aryl, wobei gegebenenfalls R⁵ und R⁶ zusammen Trimethylen, Tetramethylen, Pentamethylen und 3-Oxopentamethylen bilden können, und (vii) Q-L³-, worin Q aus Wasserstoff und Q¹ ausgewählt ist und L³ ausgewählt ist aus: einer chemischen Bindung, L¹ und L, worin L ein gegebenenfalls substituierter, zweiwertiger Rest ist, ausgewählt aus der Gruppe, bestehend aus: 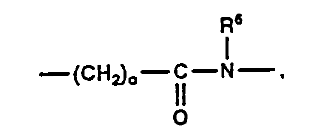 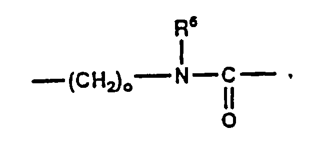 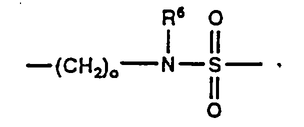 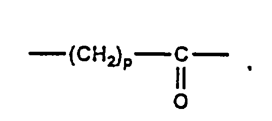 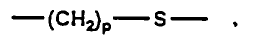 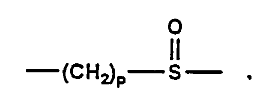 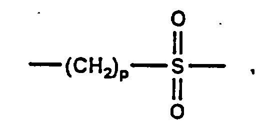 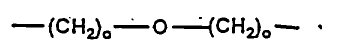 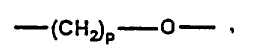 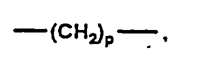 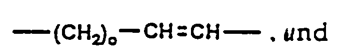 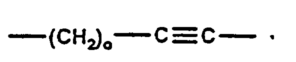 worin o = 1 oder 2 ist, p = 1, 2, 3 oder 4 ist, R⁶ wie zuvor definiert ist und worin die Substituenten aus ein bis drei Resten R¹ ausgewählt sind;
D ausgewählt ist aus der Gruppe, bestehend aus: R und -(C=O)-Xaa, worin Xaa ein bis drei D- oder L-α-Aminosäuren darstellt;
R⁸ ausgewählt ist aus der Gruppe, bestehend aus: (i) Hydroxy, (ii) C₁-C₈-Alkoxy, (iii) C₃-C₁₂-Alkenoxy, (iv) C₆-C₁₂-Aryloxy, (v) C₁-C₆-Alkyl-C₆-C₁₂-aryloxy, (vi) Di-C₁-C₈-alkylamino-C₁-C₈-alkoxy, (vii) Acylamino-C₁-C₈-alkoxy, ausgewählt aus der Gruppe: (a) Acetylaminoethoxy, (b) Nicotinoylaminoethoxy und (c) Succinamidoethoxy, (viii) C₁-C₈-Alkoyloxy-C₁-C₈-alkoxy, (ix) C₆-C₁₂-Aryl-C₁-C₈-alkoxy, worin die Aryl-Gruppe unsubstituiert oder mit ein bis drei der Gruppen: (a) nitro, (b) Halogen (F, Cl, Br, l), (c) C₁-C₄-Alkoxy und (d) Amino substituiert ist, (x) Hydroxy-C₂-C₈-alkoxy, (xi) Dihydroxy-C₃-C₈-alkoxy und (xii) NR⁹R³⁰, worin R⁹ und R³⁰ voneinander unabhängig ausgewählt sind aus der Gruppe: (a) Wasserstoff, (b) C₁-C₈-Alkyl, (c) C₃-C₈-Alkenyl, (d) C₆-C₁₂-Aryl, worin die Aryl-Gruppe unsubstituiert oder mit ein bis drei der Gruppen: Nitro, Halogen (F, Cl, Br, I), C₁-C₄-Alkoxy und Amino substituiert ist, und (e) C₆-C₁₂-Aryl-C₁-C₈-alkyl, worin die Aryl-Gruppe unsubstituiert oder mit ein bis drei der Gruppen: Nitro, Halogen (F, Cl, Br, I) und C₁-C₄-Alkoxy substituiert ist;
R⁹ eine oder zwei Gruppen darstellt, die unabhängig voneinander ausgewählt sind aus der Gruppe: (a) Wasserstoff, (b) C₁-C₈-Alkyl, (c) C₃-C₈-Alkenyl, (d) C₆-C₁₂-Aryl und (e) C₆-C₁₂-Aryl-C₁-C₈-alkyl, worin alle Aryl-Gruppen unsubstituiert oder mit ein bis drei der Gruppen: (i) Nitro, (ii) Halogen (F, Cl, Br, I), (iii) C₁-C₄-Alkoxy und (iv) Amino substituiert sind; und
pharmazeutisch annehmbare Salze davon.

3. Verbindung nach Anspruch 2, worin -L¹- ausgewählt ist aus der Gruppe, bestehend aus: 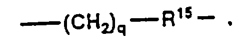 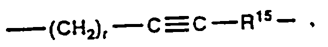 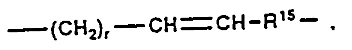 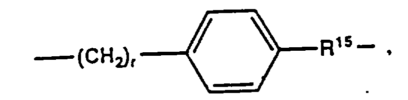 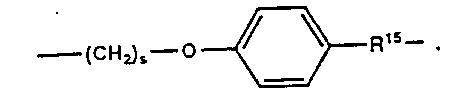 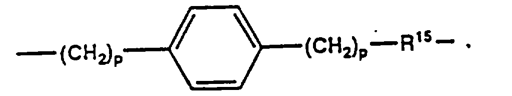 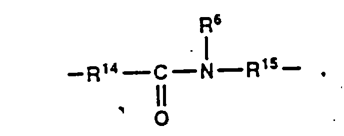 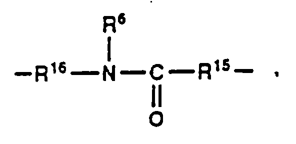 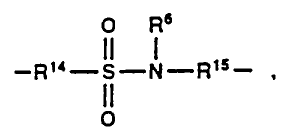 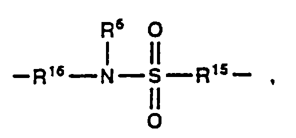 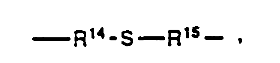 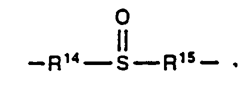 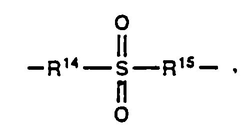 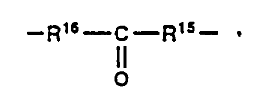 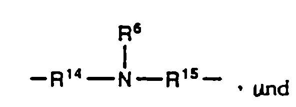 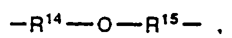
worin:
p = 1, 2, 3 oder 4 ist;
q = 3, 4, 5, 6 oder 7 ist;
r = 1, 2, 3, 4 oder 5 ist;
s = 2 oder 3 ist;
R¹⁴ ausgewählt ist aus:
C₂-C₅-Alkyl,
C₃-C₇-Cycloalkyl,
C₂-C₅-Alkenyl,
C₃-C₅-Alkinyl,
C₆-C₁₀-Aryl,
C₁-C₂-Alkyl-C₆-C₁₂-aryl,
C₁-C₂-Alkyl-C₆-C₁₀-aryl-C₁-C₂-alkyl,
C₆-C₁₀-Aryl-C₁-C₂-alkyl und
C₆-C₁₀-Aryloxy-C₁-C₂-alkyl;
R¹⁵ eine chemische Bindung ist, die L¹ mit Position 7 des Benzodiazepindions verbindet;
R¹⁶ ausgewählt ist aus:
C₂-C₅-Alkyl,
C₃-C₇-cycloalkyl,
C₃-C₅-Alkenyl,
C₃-C₅-Alkinyl,
C₆-C₁₀-Aryl,
C₁-C₃-Alkyl-C₆-C₁₂-aryl und
C₆-C₁₀-Aryl-C₁-C₂-alkyl,
wobei R¹⁴ und R¹⁶ L¹ mit Q¹ verbinden.

4. Verbindung nach Anspruch 3, worin R ausgewählt ist aus der Gruppe, bestehend aus:
Wasserstoff,
gegebenenfalls substituiertem C₁-C₆-Alkyl,
worin die Substituenten ausgewählt sind aus:
Amino,
Hydroxy,
Halogen (F, Cl, Br, l),
Carboxy und
C₁-C₄-Alkoxycarbonyl,
gegebenenfalls substituiertem C₆-C₁₀-Aryl und
gegebenenfalls substituiertem C₆-C₁₂-Aryl-C₁-C₄-alkyl,
worin die Substituenten auf allen Aryl-Gruppen ausgewählt sind aus:
Amino,
Nitro,
Halogen (F, Cl, Br, l),
(mit F, Cl, Br, l) halogeniertem C₁-C₆-Alkyl,
C₁-C₆-Alkoxy,
C₆-C₁₀-Aryloxy,
Amino-C₁-C₆-acylamino,
Amino-C₁-C₆-acyl und
Guanidino-C₆-C₁₀-aroylamino.

5. Verbindung nach Anspruch 3, worin Q¹-L¹- ausgewählt ist aus der Gruppe, bestehend aus:

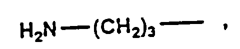

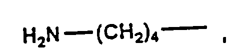

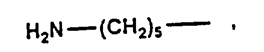

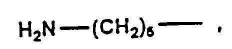

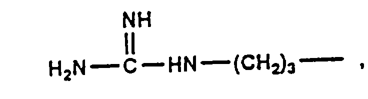

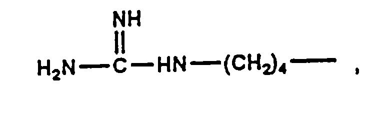

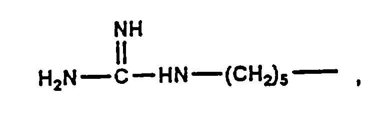

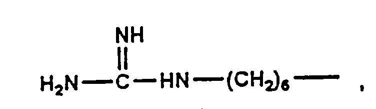

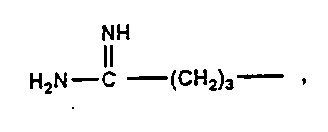

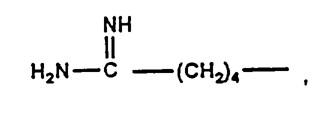

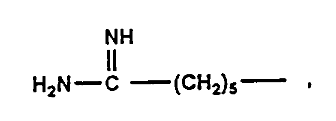

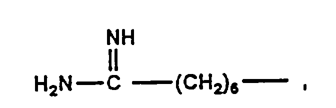

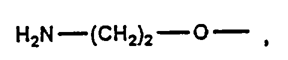

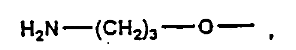

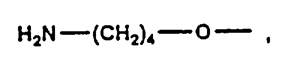

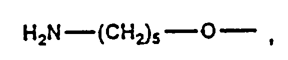

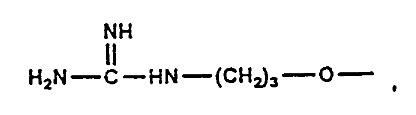

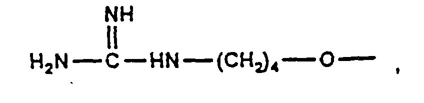

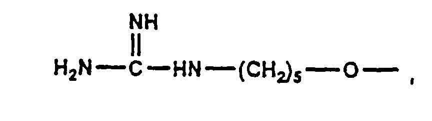

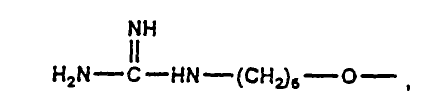

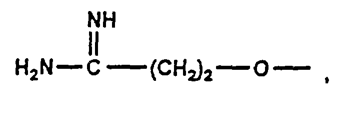

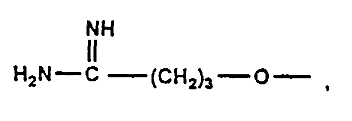

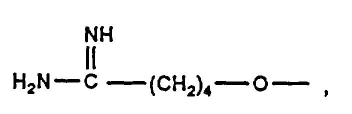

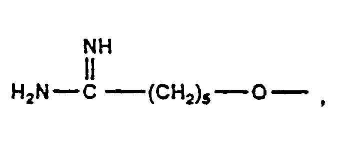

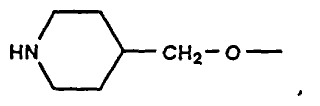

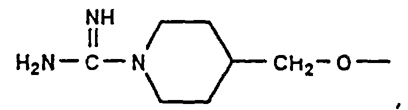

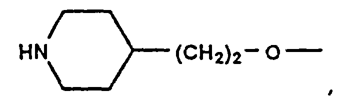

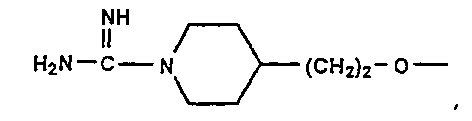

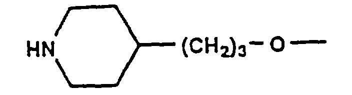

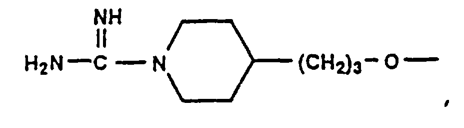

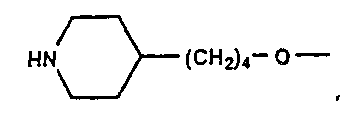

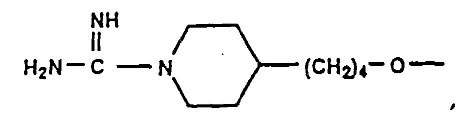

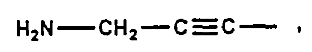

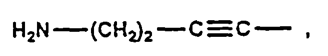

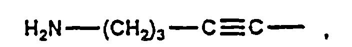

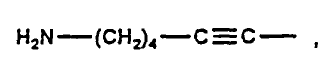

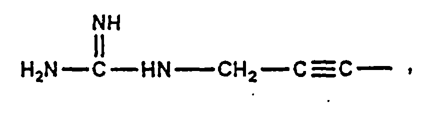

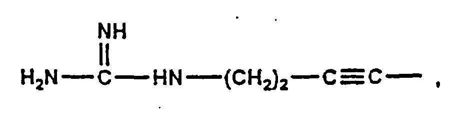

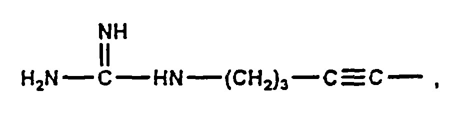

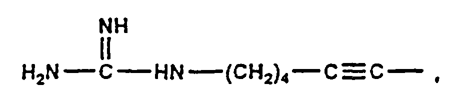

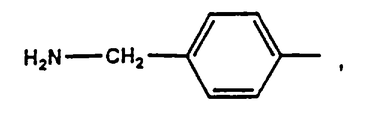

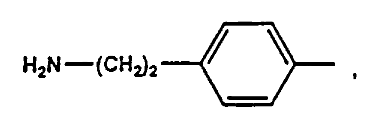

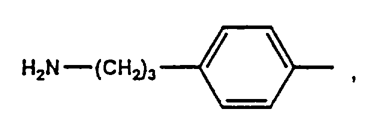

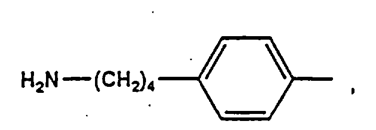

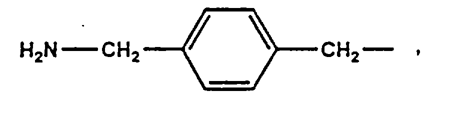

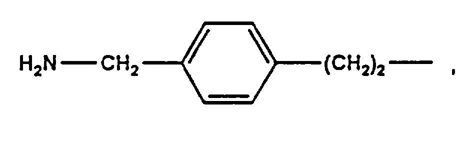

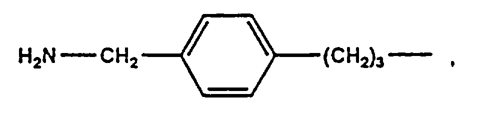

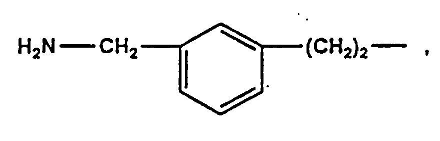

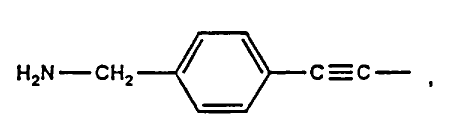

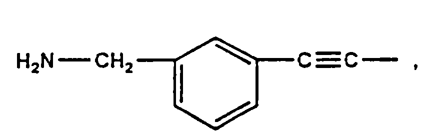

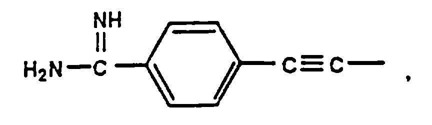

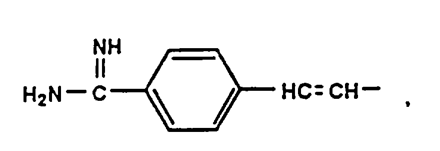

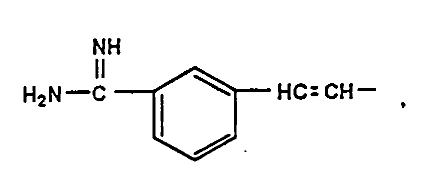

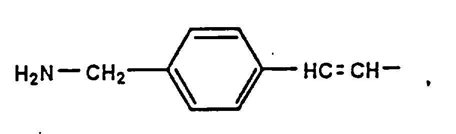

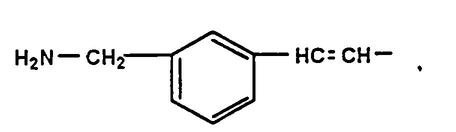

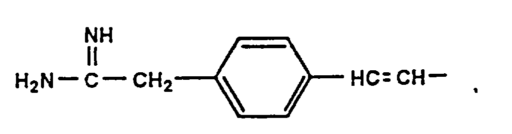

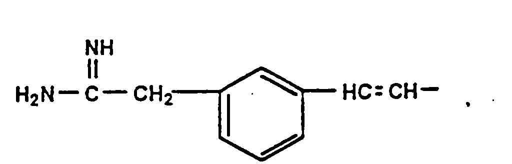

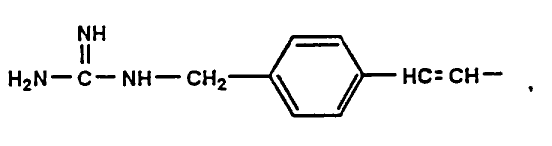

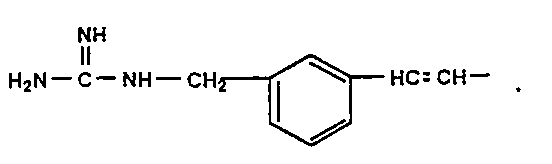

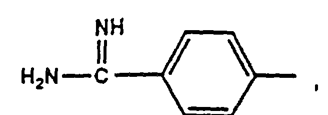

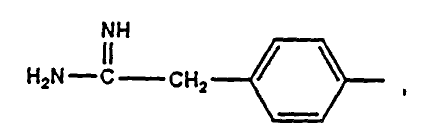

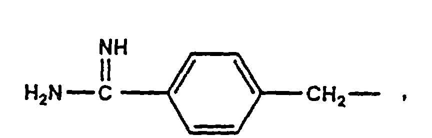

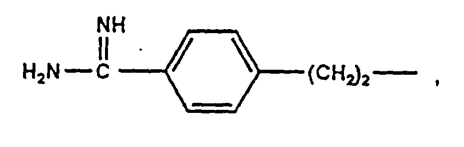

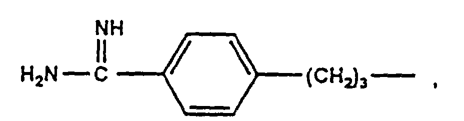

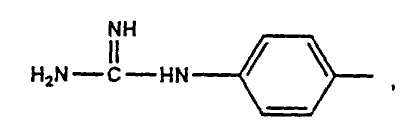

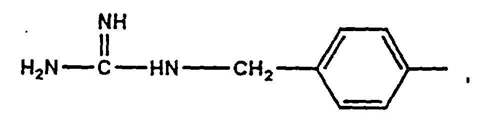

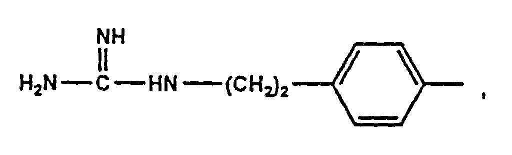

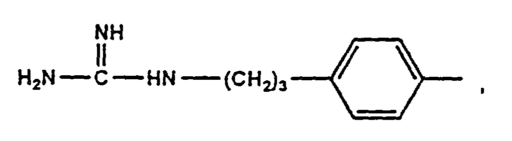

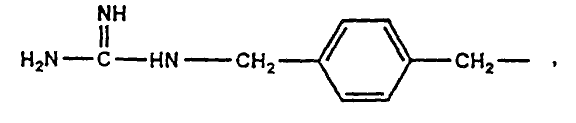

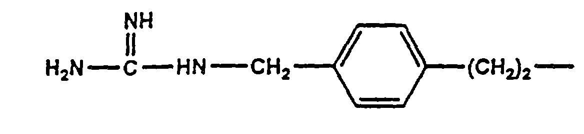

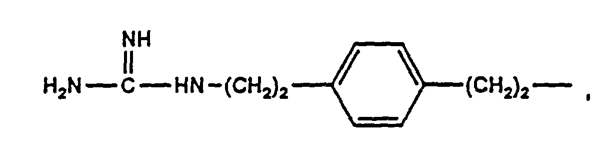

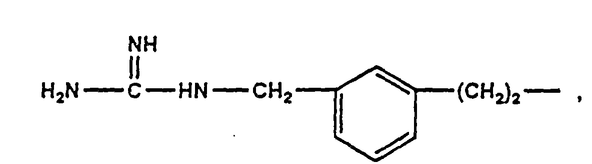

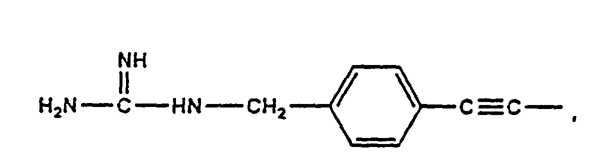

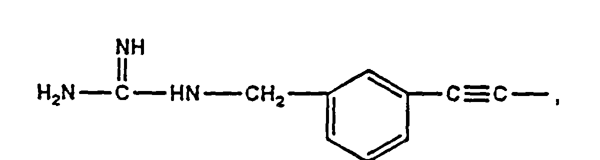

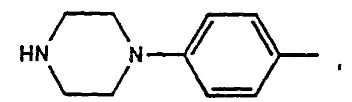

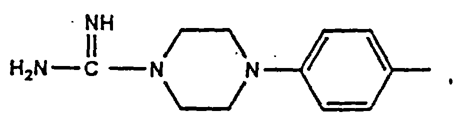

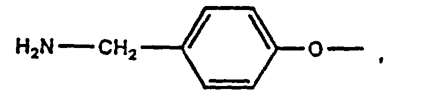

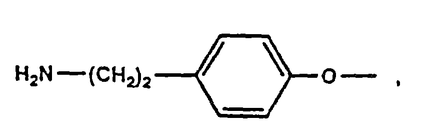

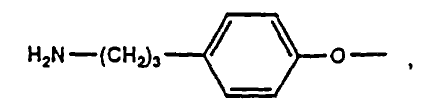

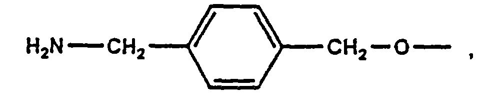

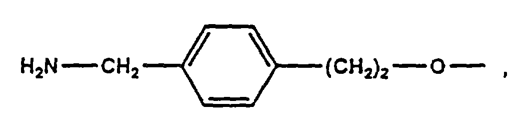

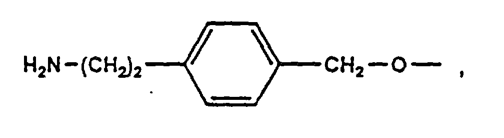

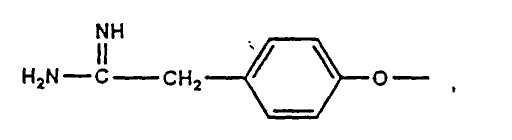

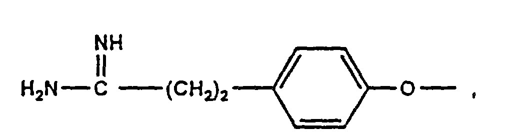

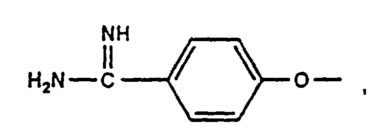

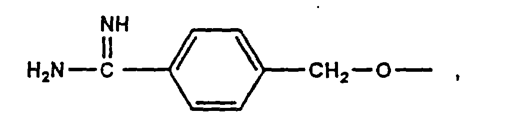

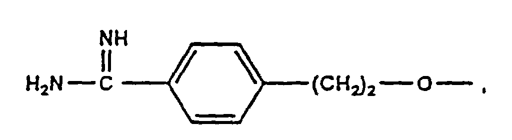

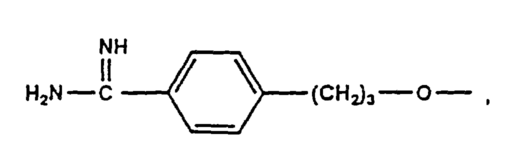

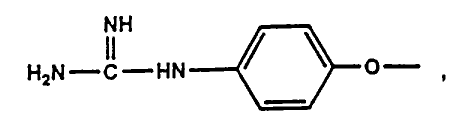

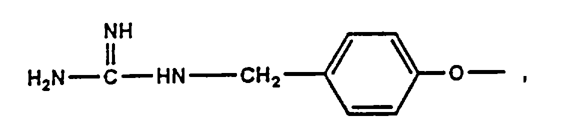

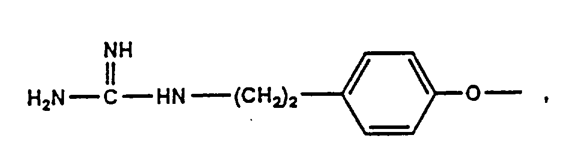

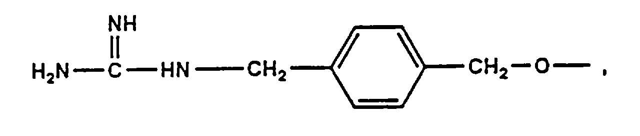

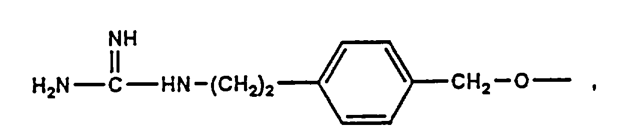

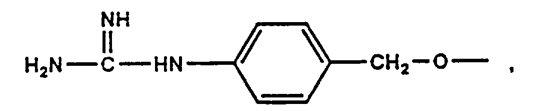

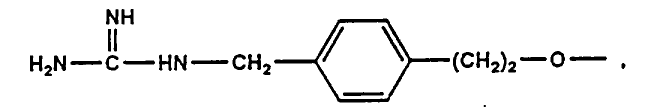

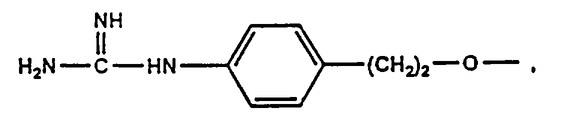

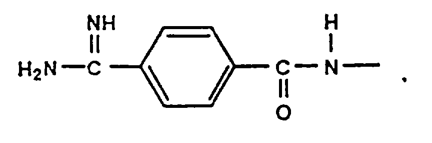

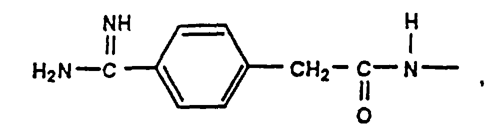

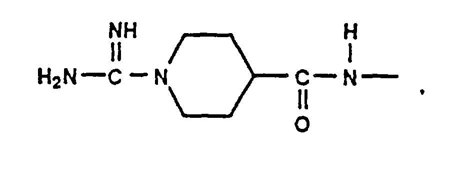

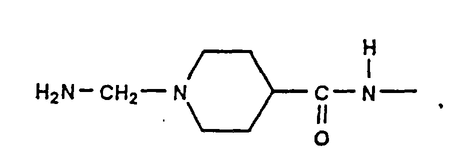

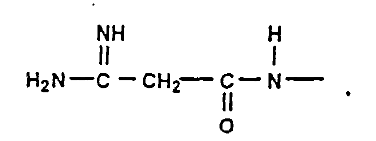

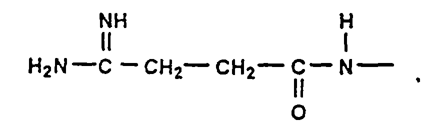

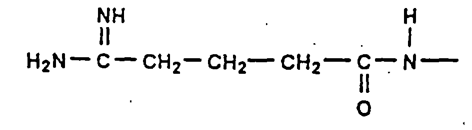

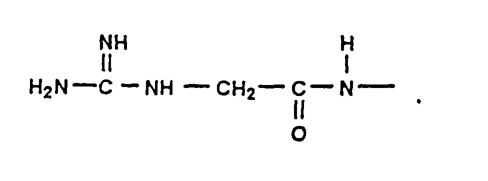

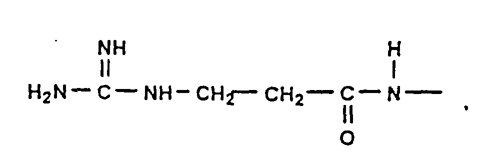

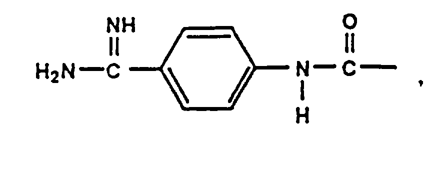

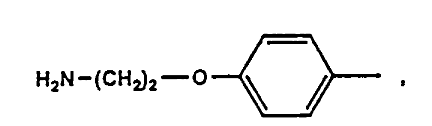

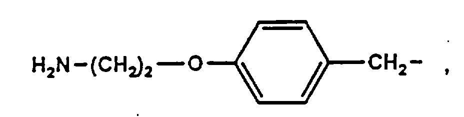

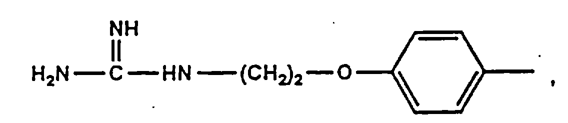

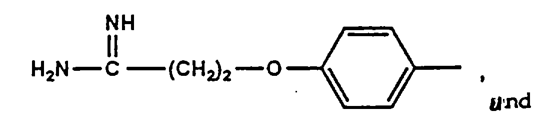

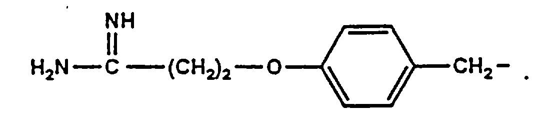

6. Verbindung nach Anspruch 2, ausgewählt aus der Gruppe, bestehend aus:;
1-(4-Chlorphenyl)methyl-4-(2-carboxyethyl)-7-(4-(1-piperizin)phenyl)-3,4-dihydro-1H-1,4-benzodiazapin-2,5-dion,
1-(Diphenyl)methyl-4-(2-carboxyethyl)-7-(4-(1-piperizin)phenyl)-3,4-dihydro-1H-1,4-benzodiazapin-2,5-dion,
1-(m-Trifluormethyl)phenyl-4-(2-carboxyethyl)-7-(4-(1-piperizin)phenyl)-3,4-dihydro-1H-1,4-benzodiazapin-2,5-dion,
1-Methyl-4-(2-carboxyethyl)-7-(4-(1-piperizin)phenyl)-3,4-dihydro-1H-1,4-benzodiazapin-2, 5-dion,
1-(1-Methyl)ethyl-4-(2-carboxyethyl)-7-(4-(1-piperizin)phenyl)-3,4-dihydro-1H-1,4-benzodiazapin-2,5-dion,
1-(2-Methyl)propyl-4-(2-carboxyethyl)-7-(4-(1-piperizin)phenyl)-3,4-dihydro-1H-1,4-benzodiazapin-2,5-dion,
1-(4-Chlorphenyl)methyl-4-(2-carboxyethyl)-7-(4-(2-aminoethoxy)phenyl)-3,4-dihydro-1H-1,4-benzodiazapin-2,5-dion,
1-(Diphenyl)methyl-4-(2-carboxyethyl)-7-(4-(2-aminoethoxy)phenyl)-3,4-dihydro-1H-1,4-benzodiazapin-2,5-dion,
1-(m-Trifluormethyl)phenyl-4-(2-carboxyethyl)-7-(4-(2-aminoethoxy)phenyl)-3,4-dihydro-1H-1,4-benzodiazapin-2,5-dion,
1-Methyl-4-(2-carboxyethyl)-7-(4-(2-aminoethoxy)phenyl)-3,4-dihydro-1H-1,4-benzodiazapin-2,5-dion,
1-(1-Methyl)ethyl-4-(2-carboxyethyl)-7-(4-(2-aminoethoxy)phenyl)-3,4-dihydro-1H-1,4-benzodiazapin-2,5-dion,
1-(2-Methyl)propyl-4-(2-carboxyethyl)-7-(4-(2-aminoethoxy)phenyl)-3,4-dihydro-1H-1,4-benzodiazapin-2,5-dion,
1-(Diphenyl)methyl-4-(2-carboxyethyl)-7-(6-aminohexyl)-3,4-dihydro-1H-1,4-benzodiazapin-2,5-dion,
1-(Diphenyl)methyl-4-(2-carboxyethyl)-7-(6-aminohex-1-inyl)-3,4-dihydro-1H-1,4-benzodiazapin-2,5-dion,
1-(Diphenyl)methyl-4-(2-carboxyethyl)-7-(5-aminopentoxy)-3,4-dihydro-1H-1,4-benzodiazapin-2,5-dion,
1-(Diphenyl)methyl-4-(2-carboxyethyl)-7-(5-guanidinopentyl)-3,4-dihydro-1H-1,4-benzodiazapin-2,5-dion,
1-(Diphenyl)methyl-4-(2-carboxyethyl)-7-(4-guanidinobutoxy)-3,4-dihydro-1H-1,4-benzodiazapin-2,5-dion,
1-(Diphenyl)methyl-4-(2-carboxyethyl)-7-(5-aminopent-1-inyl)-3,4-dihydro-1H-1,4-benzodiazapin-2,5-dion,
1-(Diphenyl)methyl-4-(2-carboxyethyl)-7-(5-guanidinopent-1-inyl)-3,4-dihydro-1H-1,4-benzodiazapin-2,5-dion,
1-(Diphenyl)methyl-4-(2-carboxyethyl)-7-(4-amidino)benzamido-3,4-dihydro-1H-1,4-benzodiazapin-2,5-dion,
1-(Diphenyl)methyl-4-(2-carboxyethyl)-7-(4-amidino)benzyloxy-3,4-dihydro-1H-1,4-benzodiazapin-2,5-dion,
1-(Diphenyl)methyl-4-(2-carboxyethyl)-7-[2-(4-amidinophenyl)]ethenyl-3,4-dihydro-1H-1,4-benzodiazapin-2,5-dion,
1-(Diphenyl)methyl-4-(2-carboxyethyl)-7-[2-(4-amidinophenyl)]ethinyl-3,4-dihydro-1H-1,4-benzodiazapin-2,5-dion,
1-(4-Chlorphenyl)methyl-4-(2-carboxyethyl)-7-(6-aminohexyl)-3,4-dihydro-1H-1,4-benzodiazapin-2,5-dion,
1-(4-Chlorphenyl)methyl-4-(2-carboxyethyl)-7-(5-aminopentoxy)-3,4-dihydro-1H-1,4-benzodiazapin-2,5-dion,
1-(4-Chlorphenyl)methyl-4-(2-carboxyethyl)-7-(4-amidino)benzamido-3,4-dihydro-1H-1,4-benzodiazapin-2,5-dion,
1-(4-Chlorphenyl)methyl-4-(2-carboxyethyl)-7-(4-amidino)benzyloxy-3,4-dihydro-1H-1,4-benzodiazapin-2,5-dion,
1-(4-Chlorphenyl)methyl-4-(2-carboxyethyl)-7-[2-(4-amidinophenyl)]ethenyl-3,4-dihydro-1H-1,4-benzodiazapin-2,5-dion,
1-(4-Chlorphenyl)methyl-4-(2-carboxyethyl)-7-[2-(4-amidinophenyl)]ethinyl-3,4-dihydro-1H-1,4-benzodiazapin-2,5-dion,
1-Methyl-4-(2-carboxyethyl)-7-(4-amidino)benzamido-3,4-dihydro-1H-1,4-benzodiazapin-2,5-dion,
1-Methyl-4-(2-carboxyethyl)-7-(4-amidino)benzyloxy-3,4-dihydro-1H-1,4-benzodiazapin-2,5-dion,
1-Methyl-4-(2-carboxyethyl)-7-[2-(4-amidinophenyl)]ethenyl-3,4-dihydro-1H-1,4-benzodiazapin-2,5-dion,
1-Methyl-4-(2-carboxyethyl)-7-[2-(4-amidinophenyl)]ethinyl-3,4-dihydro-1H-1,4-benzodiazapin-2,5-dion,
1-(1-Methyl)ethyl-4-(2-carboxyethyl)-7-(4-amidino)benzamido-3,4-dihydro-1H-1,4-benzodiazapin-2,5-dion,
1-(1-Methyl)ethyl-4-(2-carboxyethyl)-7-(4-amidino)benzyloxy-3,4-dihydro-1H-1,4-benzodiazapin-2,5-dion,
1-(1-Methyl)ethyl-4-(2-carboxyethyl)-7-[2-(4-amidinophenyl)]ethenyl-3,4-dihydro-1H-1,4-benzodiazapin-2,5-dion,
1-(2-Methyl)ethyl-4-(2-carboxyethyl)-7-[2-(4-amidinophenyl)]ethinyl-3,4-dihydro-1H-1,4-benzodiazapin-2,5-dion,
1-(2-Methyl)propyl-4-(2-carboxyethyl)-7-(4-amidino)benzamido-3,4-dihydro-1H-1,4-benzodiazapin-2,5-dion,
1-(2-Methyl)propyl-4-(2-carboxyethyl)-7-(4-amidino)benzyloxy-3,4-dihydro-1H-1,4-benzodiazapin-2,5-dion,
1-(2-Methyl)propyl-4-(2-carboxyethyl)-7-[2-(4-amidinophenyl)]ethenyl-3,4-dihydro-1H-1,4-benzodiazapin-2,5-dion,
1-(2-Methyl)propyl-4-(2-carboxyethyl)-7-[2-(4-amidinophenyl)]ethinyl-3,4-dihydro-1H-1,4-benzodiazapin-2,5-dion,
1-(2,4-Difluorphenyl)methyl-4-(2-carboxyethyl)-7-(4-(1-piperizin)phenyl)-3,4-dihydro-1H-1,4-benzodiazapin-2,5-dion,
1-(2,4-Difluorphenyl)methyl-4-(2-carboxyethyl)-7-(4-(2-aminoethoxy)phenyl)-3,4-dihydro-1H-1,4-benzodiazapin-2,5-dion,
1-(2,4-Difluorphenyl)methyl-4-(2-carboxyethyl)-7-(6-aminohexyl)-3,4-dihydro-1H-1,4-benzodiazapin-2,5-dion,
1-(2,4-Difluorphenyl)methyl-4-(2-carboxyethyl)-7-(5-aminopentoxy)-3,4-dihydro-1H-1,4-benzodiazapin-2,5-dion,
1-(2,4-Difluorphenyl)methyl-4-(2-carboxyethyl)-7-(4-amidino)benzamido-3,4-dihydro-1H-1,4-benzodiazapin-2,5-dion,
1-(2,4-Difluorphenyl)methyl-4-(2-carboxyethyl)-7-(4-amidino)benzyloxy-3,4-dihydro-1H-1,4-benzodiazapin-2,5-dion,
1-(2,4-Difluorphenyl)methyl-4-(2-carboxyethyl)-7-[2-(4-amidinophenyl)]ethenyl-3,4-dihydro-1H-1,4-benzodiazapin-2,5-dion,
1-(2,4-Difluorphenyl)methyl-4-(2-carboxyethyl)-7-[2-(4-amidinophenyl)]ethinyl-3,4-dihydro-1H-1,4-benzodiazapin-2,5-dion,
1-(4-Chlorphenyl)methyl-4-(2-carboxyethyl)-7-[2-(4-piperidinyl)ethyloxy]-3,4-dihydro-1H-1,4-benzodiazapin-2,5-dion,
1-(Diphenyl)methyl-4-(2-carboxyethyl)-7-[2-(4-piperidinyl)ethyloxy]3,4-dihydro-1H-1,4-benzodiazapin-2,5-dion,
1-(m-Trifluormethyl)phenyl-4-(2-carboxyethyl)-7-[2-(4-piperidinyl)ethyloxy]-3,4-dihydro-1H-1,4-benzodiazapin-2,5-dion,
1-Methyl-4-(2-carboxyethyl)-7-[2-(4-piperidinyl)ethyloxy]-3,4-dihydro-1H-1,4-benzodiazapin-2,5-dion,
1-(1-Methyl)ethyl-4-(2-carboxyethyl)-7-[2-(4-piperidinyl)ethyloxy]-3,4-dihydro-1H-1,4-benzodiazapin-2,5-dion,
1-(2-Methyl)propyl-4-(2-carboxyethyl)-7-[2-(4-piperidinyl)ethyloxy]-3,4-dihydro-1H-1,4-benzodiazapin-2,5-dion,
1-(2,4-Difluorphenyl)methyl-4-(2-carboxyethyl)-7-[2-(4-piperidinyl)ethyloxy]-3,4-dihydro-1H-1,4-benzodiazapin-2,5-dion,
1-(4-Chlorphenyl)methyl-4-(3-butanoyl)-7-(4-(1-piperizin)phenyl)-3,4-dihydro-1H-1,4-benzodiazapin-2,5-dion,
1-(Diphenyl)methyl-4(3-butanoyl)-7-(4-(1-piperizin)phenyl)-3,4-dihydro-1H-1,4-benzodiazapin-2,5-dion,
1-(m-Trifluormethyl)phenyl-4-(3-butanoyl)-7-(4-(1-piperizin)phenyl)-3,4-dihydro-1H-1,4-benzodiazapin-2,5-dion,
1-Methyl-4-(3-butanoyl)-7-(4-(1-piperizin)phenyl)-3,4-dihydro-1H-1,4-benzodiazapin-2,5-dion,
1-(1-Methyl)ethyl-4-(3-butanoyl)-7-(4-(1-piperizin)phenyl)-3,4-dihydro-1H-1,4-benzodiazapin-2,5-dion,
1-(2-Methyl)propyl-4-(3-butanoyl)-7-(4-(1-piperizin)phenyl)-3,4-dihydro-1H-1,4-benzodiazapin-2,5-dion,
1-(4-Chlorphenyl)methyl-4-(3-butanoyl)-7-(4-(2-aminoethoxy)phenyl)-3,4-dihydro-1H-1,4-benzodiazapin-2,5-dion,
1-(Diphenyl)methyl-4-(3-butanoyl)-7-(4-(2-aminoethoxy)phenyl)-3,4-dihydro-1H-1,4-benzodiazapin-2,5-dion,
1-(m-Trifluormethyl)phenyl-4-(3-butanoyl)-7-(4-(2-aminoethoxy)phenyl)-3,4-dihydro-1H-1,4-benzodiazapin-2,5-dion,
1-Methyl-4-(3-butanoyl)-7-(4-(2-aminoethoxy)phenyl)-3,4-dihydro-1H-1,4-benzodiazapin-2,5-dion,
1-(1-Methyl)ethyl-4-(3-butanoyl)-7-(4-(2-aminoethoxy)phenyl)-3,4-dihydro-1H-1,4-benzodiazapin-2,5-dion,
1-(2-Methyl)propyl-4-(3-butanoyl)-7-(4-(2-aminoethoxy)phenyl)-3,4-dihydro-1H-1,4-benzodiazapin-2,5-dion,
1-(Diphenyl)methyl-4-(3-butanoyl)-7-(6-aminohexyl)-3,4-dihydro-1H-1,4-benzodiazapin-2,5-dion,
1-(Diphenyl)methyl-4-(3-butanoyl)-7-(6-aminohex-1-inyl)-3,4-dihydro-1H-1,4-benzodiazapin-2,5-dion,
1-(Diphenyl)methyl-4-(3-butanoyl)-7-(5-aminopentoxy)-3,4-dihydro-1H-1,4-benzodiazapin-2,5-dion,
1-(Diphenyl)methyl-4-(3-butanoyl)-7-(5-guanidinopentyl)-3,4-dihydro-1H-1,4-benzodiazapin-2,5-dion,
1-(Diphenyl)methyl-4-(3-butanoyl)-7-(4-guanidinobutoxy)-3,4-dihydro-1H-1,4-benzodiazapin-2,5-dion,
1-(Diphenyl)methyl-4-(3-butanoyl)-7-(5-aminopent-1-inyl)-3,4-dihydro-1H-1,4-benzodiazapin-2,5-dion,
1-(Diphenyl)methyl-4-(3-butanoyl)-7-(5-guanidinopent-1-inyl)-3,4-dihydro-1H-1,4-benzodiazapin-2,5-dion,
1-(Diphenyl)methyl-4-(3-butanoyl)-7-(4-amidino)benzamido-3,4-dihydro-1H-1,4-benzodiazapin-2,5-dion,
1-(Diphenyl)methyl-4-(3-butanoyl)-7-(4-amidino)benzyloxy-3,4-dihydro-1H-1,4-benzodiazapin-2,5-dion,
1-(Diphenyl)methyl-4-(3-butanoyl)-7-[2-(4-amidinophenyl)]ethenyl-3,4-dihydro-1H-1,4-benzodiazapin-2,5-dion,
1-(Diphenyl)methyl-4-(3-butanoyl)-7-[2-(4-amidinophenyl)]ethinyl-3,4-dihydro-1H-1,4-benzodiazapin-2,5-dion,
1-(4-Chlorphenyl)methyl-4-(3-butanoyl)-7-(6-aminohexyl)-3,4-dihydro-1H-1,4-benzodiazapin-2,5-dion,
1-(4-Chlorphenyl)methyl-4-(3-butanoyl)-7-(5-aminopentoxy)-3,4-dihydro-1H-1,4-benzodiazapin-2,5-dion,
1-(4-Chlorphenyl)methyl-4-(3-butanoyl)-7-(4-amidino)benzamido-3,4-dihydro-1H-1,4-benzodiazapin-2,5-dion,
1-(4-Chlorphenyl)methyl-4-(3-butanoyl)-7-(4-amidino)benzyloxy-3,4-dihydro-1H-1,4-benzodiazapin-2,5-dion,
1-(4-Chlorphenyl)methyl-4-(3-butanoyl)-7-[2-(4-amidinophenyl)]ethenyl-3,4-dihydro-1H-1,4-benzodiazapin-2,5-dion,
1-(4-Chlorphenyl)methyl-4-(3-butanoyl)-7-[2-(4-amidinophenyl)]ethinyl-3,4-dihydro-1H-1,4-benzodiazapin-2,5-dion,
1-Methyl-4-(3-butanoyl)-7-(4-amidino)benzamido-3,4-dihydro-1H-1,4-benzodiazapin-2,5-dion,
1-Methyl-4-(3-butanoyl)-7-(4-amidino)benzyloxy-3,4-dihydro-1H-1,4-benzodiazapin-2,5-dion,
1-Methyl-4-(3-butanoyl)-7-[2-(4-amidinophenyl)]ethenyl-3,4-dihydro-1H-1,4-benzodiazapin-2,5-dion,
1-Methyl-4-(3-butanoyl)-7-[2-(4-amidinophenyl)]ethinyl-3,4-dihydro-1H-1,4-benzodiazapin-2,5-dion,
1-(1-Methyl)ethyl-4-(3-butanoyl)-7-(4-amidino)benzamido-3,4-dihydro-1H-1,4-benzodiazapin-2,5-dion,
1-(1-Methyl)ethyl-4-(3-butanoyl)-7-(4-amidino)benzyloxy-3,4-dihydro-1H-1,4-benzodiazapin-2,5-dion,
1-(1-Methyl)ethyl-4-(3-butanoyl)-7-[2-(4-amidinophenyl)]ethenyl-3,4-dihydro-1H-1,4-benzodiazapin-2,5-dion,
1-(2-Methyl)ethyl-4-(3-butanoyl)-7-[2-(4-amidinophenyl)]ethinyl-3,4-dihydro-1H-1,4-benzodiazapin-2,5-dion,
1-(2-Methyl)propyl-4-(3-butanoyl)-7-(4-amidino)benzamido-3,4-dihydro-1H-1,4-benzodiazapin-2,5-dion,
1-(2-Methyl)propyl-4-(3-butanoyl)-7-(4-amidino)benzyloxy-3,4-dihydro-1H-1,4-benzodiazapin-2,5-dion,
1-(2-Methyl)propyl-4-(3-butanoyl)-7-[2-(4-amidinophenyl)]ethenyl-3,4-dihydro-1H-1,4-benzodiazapin-2,5-dion,
1-(2-Methyl)propyl-4-(3-butanoyl)-7-[2-(4-amidinophenyl)]ethinyl-3,4-dihydro-1H-1,4-benzodiazapin-2,5-dion,
1-(2,4-Difluorphenyl)methyl-4-(3-butanoyl)-7-(4-(1-piperizin)phenyl)-3,4-dihydro-1H-1,4-benzodiazapin-2,5-dion,
1-(2,4-Difluorphenyl)methyl-4-(3-butanoyl)-7-(4-(2-aminoethoxy)phenyl)-3,4-dihydro-1H-1,4-benzodiazapin-2,5-dion,
1-(2,4-Difluorphenyl)methyl-4-(3-butanoyl)-7-(6-aminohexyl)-3,4-dihydro-1H-1,4-benzodiazapin-2,5-dion,
1-(2,4-Difluorphenyl)methyl-4-(3-butanoyl)-7-(5-aminopentoxy)-3,4-dihydro-1H-1,4-benzodiazapin-2,5-dion,
1-(2,4-Difluorphenyl)methyl-4-(3-butanoyl)-7-(4-amidino)benzamido-3,4-dihydro-1H-1,4-benzodiazapin-2,5-dion,
1-(2,4-Difluorphenyl)methyl-4-(3-butanoyl)-7-(4-amidino)benzyloxy-3,4-dihydro-1H-1,4-benzodiazapin-2,5-dion,
1-(2,4-Difluorphenyl)methyl-4-(3-butanoyl)-7-[2-(4-amidinophenyl)]ethenyl-3,4-dihydro-1H-1,4-benzodiazapin-2,5-dion,
1-(2,4-Difluorphenyl)methyl-4-(3-butanoyl)-7-[2-(4-amidinophenyl)]ethinyl-3,4-dihydro-1H-1,4-benzodiazapin-2,5-dion,
1-(4-Chlorphenyl)methyl-4-(3-butanoyl)-7-[2-(4-piperidinyl)ethyloxy]-3,4-dihydro-1H-1,4-benzodiazapin-2,5-dion,
1-(Diphenyl)methyl-4-(3-butanoyl)-7-[2-(4-piperidinyl)ethyloxy]-3,4-dihydro-1H-1,4-benzodiazapin-2,5-dion,
1-(m-Trifluormethyl)phenyl-4-(3-butanoyl)-7-[2-(4-piperidinyl)ethyloxy]-3,4-dihydro-1H-1,4-benzodiazapin-2,5-dion,
1-Methyl-4-(3-butanoyl)-7-[2-(4-piperidinyl)ethyloxy]-3,4-dihydro-1H-1,4-benzodiazapin-2,5-dion,
1-(1-Methyl)ethyl-4-(3-butanoyl)-7-[2-(4-piperidinyl)ethyloxy]-3,4-dihydro-1H-1,4-benzodiazapin-2,5-dion,
1-(2-Methyl)propyl-4-(3-butanoyl)-7-[2-(4-piperidinyl)ethyloxy]-3,4-dihydro-1H-1,4-benzodiazapin-2,5-dion,
1-(2,4-Difluorphenyl)methyl-4-(3-butanoyl)-7-[2-(4-piperidinyl)ethyloxy]-3,4-dihydro-1H-1,4-benzodiazapin-2,5-dion,
1-(4-Chlorphenyl)methyl-4-[(2-methyl)carboxyethyl]-7-(4-(1-piperizin)phenyl)-3,4-dihydro-1H-1,4-benzodiazapin-2,5-dion,
1-(Diphenyl)methyl-4-[(2-methyl)carboxyethyl]-7-(4-(1-piperizin)phenyl)-3,4-dihydro-1H-1,4-benzodiazapin-2,5-dion,
1-(m-Trifluormethyl)phenyl-4-[(2-methyl)carboxyethyl]-7-(4-(1-piperizin)phenyl)-3,4-dihydro-1H-1,4-benzodiazapin-2,5-dion,
1-Methyl-4-[(2-methyl)carboxyethyl]-7-(4-(1-piperizin)phenyl)-3,4-dihydro-1H-1,4-benzodiazapin-2,5-dion,
1-(1-Methyl)ethyl-4-[(2-methyl)carboxyethyl]-7-(4-(1-piperizin)phenyl)-3,4-dihydro-1H-1,4-benzodiazapin-2,5-dion,
1-(2-Methyl)propyl-4-[(2-methyl)carboxyethyl]-7-(4-(1-piperizin)phenyl)-3,4-dihydro-1H-1,4-benzodiazapin-2,5-dion,
1-(4-Chlorphenyl)methyl-4-[(2-methyl)carboxyethyl]-7-(4-(2-aminoethoxy)phenyl)-3,4-dihydro-1H-1,4-benzodiazapin-2,5-dion,
1-(Diphenyl)methyl-4-[(2-methyl)carboxyethyl]-7-(4-(2-aminoethoxy)phenyl)-3,4-dihydro-1H-1,4-benzodiazapin-2,5-dion,
1-(m-Trifluormethyl)phenyl-4-[(2-methyl)carboxyethyl]-7-(4-(2-aminoethoxy)phenyl)-3,4-dihydro-1H-1,4-benzodiazapin-2,5-dion,
1-Methyl-4-[(2-methyl)carboxyethyl]-7-(4-(2-aminoethoxy)phenyl)-3,4-dihydro-1H-1,4-benzodiazapin-2,5-dion,
1-(1-Methyl)ethyl-4-[(2-methyl)carboxyethyl]-7-(4-(2-aminoethoxy)phenyl)-3,4-dihydro-1H-1,4-benzodiazapin-2,5-dion,
1-(2-Methyl)propyl-4-[(2-methyl)carboxyethyl]-7-(4-(2-aminoethoxy)phenyl)-3,4-dihydro-1H-1,4-benzodiazapin-2,5-dion,
1-(Diphenyl)methyl-4-[(2-methyl)carboxyethyl]-7-(6-aminohexyl)-3,4-dihydro-1H-1,4-benzodiazapin-2,5-dion,
1-(Diphenyl)methyl-4-[(2-methyl)carboxyethyl]-7-(6-aminohex-1-inyl)-3,4-dihydro-1H-1,4-benzodiazapin-2,5-dion,
1-(Diphenyl)methyl-4-[(2-methyl)carboxyethyl]-7-(5-aminopentoxy)-3,4-dihydro-1H-1,4-benzodiazapin-2,5-dion,
1-(Diphenyl)methyl-4-[(2-methyl)carboxyethyl]-7-(5-guanidinopentyl)-3,4-dihydro-1H-1,4-benzodiazapin-2,5-dion,
1-(Diphenyl)methyl-4-[(2-methyl)carboxyethyl]-7-(4-guanidinobutoxy)-3,4-dihydro-1H-1,4-benzodiazapin-2,5-dion,
1-(Diphenyl)methyl-4-[(2-methyl)carboxyethyl]-7-(5-aminopent-1-inyl)-3,4-dihydro-1H-1,4-benzodiazapin-2,5-dion,
1-(Diphenyl)methyl-4-[(2-methyl)carboxyethyl]-7-(5-guanidinopent-1-inyl)-3,4-dihydro-1H-1,4-benzodiazapin-2,5-dion,
1-(Diphenyl)methyl-4-[(2-methyl)carboxyethyl]-7-(4-amidino)benzamido-3,4-dihydro-1H-1,4-benzodiazapin-2,5-dion,
1-(Diphenyl)methyl-4-[(2-methyl)carboxyethyl]-7-(4-amidino)benzyloxy-3,4-dihydro-1H-1,4-benzodiazapin-2,5-dion,
1-(Diphenyl)methyl-4-[(2-methyl)carboxyethyl]-7-[2-(4-amidinophenyl)]ethenyl-3,4-dihydro-1H-1,4-benzodiazapin-2,5-dion,
1-(Diphenyl)methyl-4-[(2-methyl)carboxyethyl]-7-[2-(4-amidinophenyl)]ethinyl-3,4-dihydro-1H-1,4-benzodiazapin-2,5-dion,
1-(4-Chlorphenyl)methyl-4-[(2-methyl)carboxyethyl]-7-(6-aminohexyl)-3,4-dihydro-1H-1,4-benzodiazapin-2,5-dion,
1-(4-Chlorphenyl)methyl-4-[(2-methyl)carboxyethyl]-7-(5-aminopentoxy)-3,4-dihydro-1H-1,4-benzodiazapin-2,5-dion,
1-(4-Chlorphenyl)methyl-4-[(2-methyl)carboxyethyl]-7-(4-amidino)benzamido-3,4-dihydro-1H-1,4-benzodiazapin-2,5-dion,
1-(4-Chlorphenyl)methyl-4-[(2-methyl)carboxyethyl]-7-(4-amidino)benzyloxy-3,4-dihydro-1H-1,4-benzodiazapin-2,5-dion,
1-(4-Chlorphenyl)methyl-4-[(2-methyl)carboxyethyl]-7-[2-(4-amidinophenyl)]ethenyl-3,4-dihydro-1H-1,4-benzodiazapin-2,5-dion,
1-(4-Chlorphenyl)methyl-4-[(2-methyl)carboxyethyl]-7-[2-(4-amidinophenyl)]ethinyl-3,4-dihydro-1H-1,4-benzodiazapin-2,5-dion,
1-Methyl-4-[(2-methyl)carboxyethyl]-7-(4-amidino)benzamido-3,4-dihydro-1H-1,4-benzodiazapin-2,5-dion,
1-Methyl-4-[(2-methyl)carboxyethyl]-7-(4-amidino)benzyloxy-3,4-dihydro-1H-1,4-benzodiazapin-2,5-dion,
1-Methyl-4-[(2-methyl)carboxyethyl]-7-[2-(4-amidinophenyl)]ethenyl-3,4-dihydro-1H-1,4-benzodiazapin-2,5-dion,
1-Methyl-4-[(2-methyl)carboxyethyl]-7-[2-(4-amidinophenyl)]ethinyl-3,4-dihydro-1H-1,4-benzodiazapin-2,5-dion,
1-(1-Methyl)ethyl-4-[(2-methyl)carboxyethyl]-7-(4-amidino)benzamido-3,4-dihydro-1H-1,4-benzodiazapin-2,5-dion,
1-(1-Methyl)ethyl-4-[(2-methyl)carboxyethyl]-7-(4-amidino)benzyloxy-3,4-dihydro-1H-1,4-benzodiazapin-2,5-dion,
1-(1-Methyl)ethyl-4-[(2-methyl)carboxyethyl]-7-[2-(4-amidinophenyl)]ethenyl-3,4-dihydro-1H-1,4-benzodiazapin-2,5-dion,
1-(2-Methyl)ethyl-4-[(2-methyl)carboxyethyl]-7-[2-(4-amidinophenyl)]ethinyl-3,4-dihydro-1H-1,4-benzodiazapin-2,5-dion,
1-(2-Methyl)propyl-4-[(2-methyl)carboxyethyl]-7-(4-amidino)benzamido-3,4-dihydro-1H-1,4-benzodiazapin-2,5-dion,
1-(2-Methyl)propyl-4-[(2-methyl)carboxyethyl]-7-(4-amidino)benzyloxy-3,4-dihydro-1H-1,4-benzodiazapin-2,5-dion,
1-(2-Methyl)propyl-4-[(2-methyl)carboxyethyl]-7-[2-(4-amidinophenyl)]ethenyl-3,4-dihydro-1H-1,4-benzodiazapin-2,5-dion,
1-(2-Methyl)propyl-4-[(2-methyl)carboxyethyl]-7-[2-(4-amidinophenyl)]ethinyl-3,4-dihydro-1H-1,4-benzodiazapin-2,5-dion,
1-(2,4-Difluorphenyl)methyl-4-[(2-methyl)carboxyethyl]-7-(4-(1-piperizin)phenyl)-3,4-dihydro-1H-1,4-benzodiazapin-2,5-dion,
1-(2,4-Difluorphenyl)methyl-4-[(2-methyl)carboxyethyl]-7-(4-(2-aminoethoxy)phenyl)-3,4-dihydro-1H-1,4-benzodiazapin-2,5-dion,
1-(2,4-Difluorphenyl)methyl-4-[(2-methyl)carboxyethyl]-7-(6-aminohexyl)-3,4-dihydro-1H-1,4-benzodiazapin-2,5-dion,
1-(2,4-Difluorphenyl)methyl-4-[(2-methyl)carboxyethyl]-7-(5-aminopentoxy)-3,4-dihydro-1H-1,4-benzodiazapin-2,5-dion,
1-(2,4-Difluorphenyl)methyl-4-[(2-methyl)carboxyethyl]-7-(4-amidino)benzamido-3,4-dihydro-1H-1,4-benzodiazapin-2,5-dion,
1-(2,4-Difluorphenyl)methyl-4-[(2-methyl)carboxyethyl]-7-(4-amidino)benzyloxy-3,4-dihydro-1H-1,4-benzodiazapin-2,5-dion,
1-(2,4-Difluorphenyl)methyl-4-[(2-methyl)carboxyethyl]-7-[2-(4-amidinophenyl)]ethenyl-3,4-dihydro-1H-1,4-benzodiazapin-2,5-dion,
1-(2,4-Difluorphenyl)methyl-4-[(2-methyl)carboxyethyl]-7-[2-(4-amidinophenyl)]ethinyl-3,4-dihydro-1H-1,4-benzodiazapin-2,5-dion,
1-(4-Chlorphenyl)methyl-4-[(2-methyl)carboxyethyl]-7-[2-(4-piperidinyl)ethyloxy]-3,4-dihydro-1H-1,4-benzodiazapin-2,5-dion,
1-(Diphenyl)methyl-4-[(2-methyl)carboxyethyl]-7-[2-(4-piperidinyl)ethyloxy]3,4-dihydro-1H-1,4-benzodiazapin-2,5-dion,
1-(m-Trifluormethyl)phenyl-4-[(2-methyl)carboxyethyl]-7-[2-(4-piperidinyl)ethyloxy]-3,4-dihydro-1H-1,4-benzodiazapin-2,5-dion,
1-Methyl-4-[(2-methyl)carboxyethyl]-7-[2-(4-piperidinyl)ethyloxy]-3,4-dihydro-1H-1,4-benzodiazapin-2,5-dion,
1-(1-Methyl)ethyl-4-[(2-methyl)carboxyethyl]-7-[2-(4-piperidinyl)ethyloxy]-3,4-dihydro-1H-1,4-benzodiazapin-2,5-dion,
1-(2-Methyl)propyl-4-[(2-methyl)carboxyethyl]-7-[2-(4-piperidinyl)ethyloxy]-3,4-dihydro-1H-1,4-benzodiazapin-2,5-dion,
1-(2,4-Difluorphenyl)methyl-4-[(2-methyl)carboxyethyl]-7-[2-(4-piperidinyl)ethyloxy]-3,4-dihydro-1H-1,4-benzodiazapin-2,5-dion,
1-(3-(Butyryl)aminophenyl)methyl-4-(2-carboxyethyl)-7-(6-aminohexyl)-3,4-dihydro-1H-1,4-benzodiazapin-2,5-dion,
1-(3-(Butyryl)aminophenyl)methyl-4-(2-carboxyethyl)-7-(5-guanidinopentyl)-3,4-dihydro-1H-1,4-benzodiazapin-2,5-dion,
1-(3-(4-Aminobutyryl)aminophenyl)methyl-4-(2-carboxyethyl)-7-(6-aminohexyl)-3,4-dihydro-1H-1,4-benzodiazapin-2,5-dion,
1-(3-(4-Guanidinobutyryl)aminophenyl)methyl-4-(2-carboxyethyl)-7-(5-guanidinopentyl)-3,4-dihydro-1H-1,4-benzodiazapin-2,5-dion,
1-(3-(6-Aminohexoyl)aminophenyl)methyl-4-(2-carboxyethyl)-7-(6-aminohexyl)-3,4-dihydro-1H-1,4-benzodiazapin-2,5-dion,
1-(3-(6-Guanidinohexoyl)aminophenyl)methyl-4-(2-carboxyethyl)-7-(5-guanidinopentyl)-3,4-dihydro-1H-1,4-benzodiazapin-2,5-dion,
1-(3-(6-Aminopentoyl)aminophenyl)methyl-4-(2-carboxyethyl)-7-(6-aminohexyl)-3,4-dihydro-1H-1,4-benzodiazapin-2,5-dion,
1-(3-(6-Guanidinopentoyl)aminophenyl)methyl-4-(2-carboxyethyl)-7-(5-guanidinopentyl)-3,4-dihydro-1H-1,4-benzodiazapin-2,5-dion,
1-(3-(6-Aminopropionyl)aminophenyl)methyl-4-(2-carboxyethyl)-7-(6-aminohexyl)-3,4-dihydro-1H-1,4-benzodiazapin-2,5-dion,
1-(3-(6-Guanidinopropionyl)aminophenyl)methyl-4-(2-carboxyethyl)-7-(5-guanidinopentyl)-3,4-dihydro-1H-1,4-benzodiazapin-2,5-dion,
1-(3-(6-Aminoacetyl)aminophenyl)methyl-4-(2-carboxyethyl)-7-(6-aminohexyl)-3,4-dihydro-1H-1,4-benzodiazapin-2,5-dion,
1-(3-(6-Guanidinoacetyl)aminophenyl)methyl-4-(2-carboxyethyl)-7-(5-guanidinopentyl)-3,4-dihydro-1H-1,4-benzodiazapin-2,5-dion,
1-(3-(4-Guanidinobenzoyl)aminophenyl)methyl-4-(2-carboxyethyl)-7-(6-aminohexyl)-3,4-dihydro-1H-1,4-benzodiazapin-2,5-dion,
1-(4-(6-Aminopropionyl)aminophenyl)methyl-4-(2-carboxyethyl)-7-(6-aminohexyl)-3,4-dihydro-1H-1,4-benzodiazapin-2,5-dion,
1-(3-(4-Aminobutyl)phenyl)methyl-4-(2-carboxyethyl)-7-(6-aminohexyl)-3,4-dihydro-1H-1,4-benzodiazapin-2,5-dion und
1-(4-carboxyphenyl)methyl-4-(2-carboxyethyl)-7-(6-aminohexyl)-3,4-dihydro-1H-1,4-benzodiazapin-2,5-dion.

7. Verbindung nach Anspruch 1, dargestellt durch die Formeln (III) und (IV): 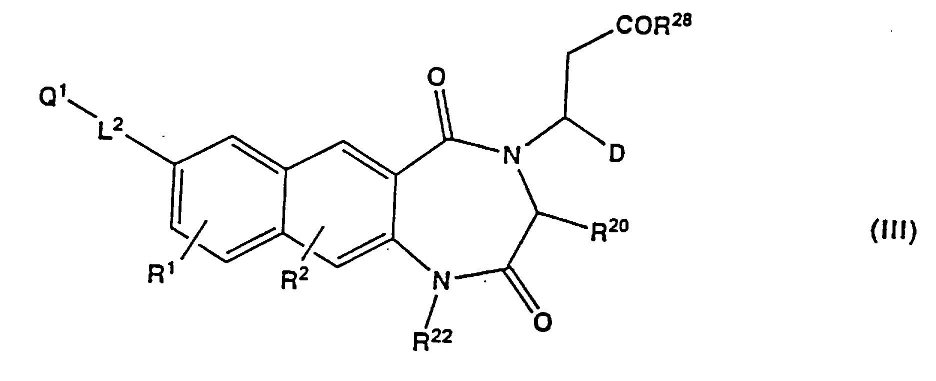 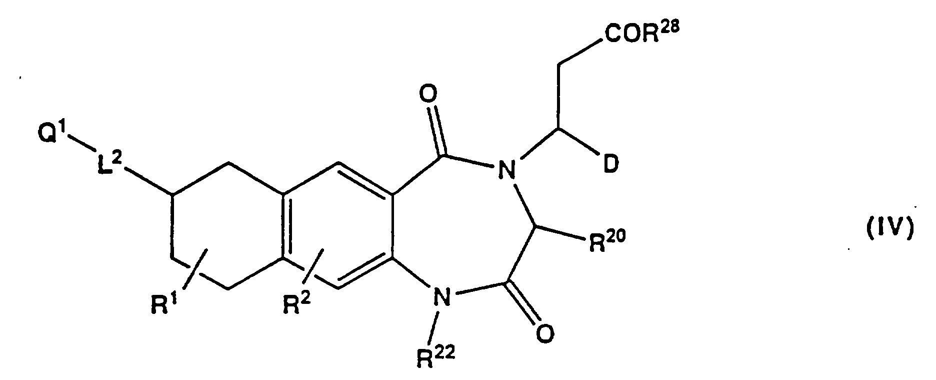
worin:
R¹ und R unabhängig voneinander ausgewählt sind aus Wasserstoff und Halogen (F, Cl, Br, l);
Q¹ ausgewählt ist aus der Gruppe, bestehend aus:
(A) einer Amino-Gruppe,
(B) einer Amidino-Gruppe,
(C) einer Aminoalkylenimino-Gruppe,
(D) einer Iminoalkylenamino-Gruppe und
(E) einer Guanidino-Gruppe;
R⁰ Wasserstoff ist;
R ausgewählt ist aus der Gruppe, bestehend aus: (i) Wasserstoff, (ii) gegebenenfalls substituiertem C₁-C₁₂-Alkyl, (iii) gegebenenfalls substituiertem C₆-C₁₄-Aryl, (iv) gegebenenfalls substituiertem C₃-C₁₄-Cycloalkyl, (v) gegebenenfalls substituiertem C₁-C₁₂-Alkyl-C₆-C₁₄-aryl, (vi) gegebenenfalls substituiertem C₁-C₁₂-Alkyl(C₁₃-C₁₄-cycloalkyl, worin die Substituenten ausgewählt sind aus: (a) Halogen (F, Cl, Br, l), (b) Nitro, (c) Hydroxy, (d) Carboxy, (e) Tetrazol, (f) Hydroxamat, (g) Sulfonamid, (h) Trifluorimid, (i) Phosphonat, (j) C₁-C₆-Alkyl, (k) C₆-C₁₄-Aryl, (l) Benzyl, (m) C₃-C₁₄-Cycloalkyl, (n) COR⁴, worin R⁴ ausgewählt ist aus der Gruppe: C₁-C₈-Alkoxy, C₃-C₁₂-Alkenoxy, C₆-C₁₂-Aryloxy, Di-C₁-C₈-alkylamino-C₁-C₈-alkoxy, Acylamino-C₁-C₈-alkoxy, ausgewählt aus der Gruppe: Acetylaminoethoxy, Nicotinoylaminoethoxy, Succinamidoethoxy und Pivaloyloxyethoxy, und C₆-C₁₂-Aryl-C₁-C₈-alkoxy, worin die Aryl-Gruppe unsubstituiert oder mit ein bis drei der Gruppen: Nitro, Halogen (F, Cl, Br, l), C₁-C₄-Alkoxy, Amino, Hydroxy, Hydroxy-C₂-C₈-alkoxy und Dihydroxy-C₃-C₈-alkoxy substituiert ist, (o) CONR⁵R⁶, worin R⁵ und R⁶ unabhängig voneinander ausgewählt sind aus:
Wasserstoff, C₁-C₁₀-Alkyl, C₃-C₁₀-Alkenyl, C₆-C₁₄-Aryl, C₁-C₆-Alkyl-C₆-C₁₀-aryl, wobei gegebenenfalls R⁵ und R⁶ zusammen Trimethylen, Tetramethylen, Pentamethylen und 3-Oxopentamethylen bilden können, und (vii) Q-L³-, worin Q aus Wasserstoff ausgewählt ist und Q¹ und L³ ausgewählt sind aus einer chemischen Bindung und L¹, worin L¹ ein gegebenenfalls substituierter, zweiwertiger Rest ist, ausgewählt aus der Gruppe, bestehend aus: C₃-C₇-Alkylen, C₃-C₇-Cycloalkylen, C₃-C₇-Alkenylen, C₄-C₇-Cycloalkenylen, C₅-C₈-Cycloalkadienylen, C₃-C₇-Alkadienylen, C₃-C₇-Alkinylen, C₄-C₇-Alkeninylen, C₆-C₁₄-Arylen, C₆-C₁₄-Aryl-C₂-C₄-alkinylen, C₁-C₃-Alkyl-C₆-C₁₄-aryl-C₂-C₄-alkinylen, C₆-C₁₄-Aryl-C₂-C₄-alkenylen, C₁-C₃-Alkyl-C₆-C₁₄-arylen, C₁-C₃-Alkyl-C₆-C₁₄-aryl-C₂-C₄-alkenylen, C₆-C₁₄-Aryl-C₁-C₃-alkylen, C₆-C₁₄-Aryl-C₁-C₃-alkyloxyen, C₁-C₂-Alkyl-C₆-C₁₄-aryl-C₁-C₂-alkylen, C₁-C₃-Alkyloxy-C₆-C₁₄-arylen, C₂-C₈-Alkyloxyen, C₁-C₅-Alkyloxy-C₁-C₅-alkylen, C₆-C₁₀-Aryloxyen, C₆-C₁₀-Aryloxy-C₁-C₅-alkylen, C₆-C₁₀-Arylthio-C₁-C₅-alkylen, 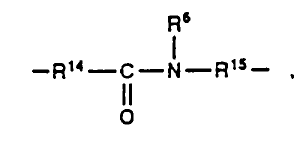 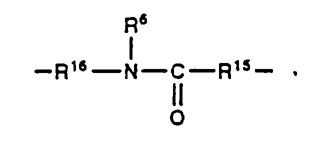 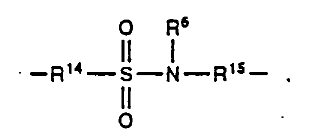 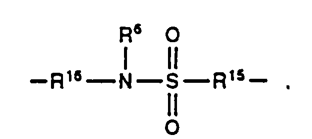 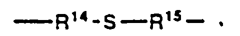 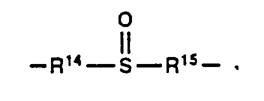 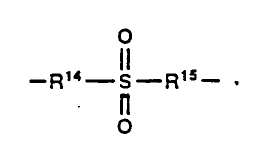 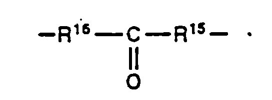 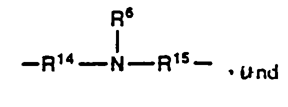 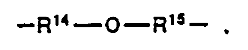
worin: R¹⁴ ausgewählt ist aus: einer chemischen Bindung, C₁-C₈-Alkyl, C₃-C₇-Cycloalkyl, C₂-c₅-Alkenyl, C₃-C₅-Alkinyl, C₆-C₁₀-Aryl, C₁-C₃-Alkyl-C₆-C₁₂-aryl, C₁-C₂-Alkyl-C₆-C₁₀-aryl-C₁-C₂-alkyl und C₆-C₁₀-Aryl-C₁-C₂-alkyl, R¹⁵ ausgewählt ist aus: einer chemischen Bindung, C₁-C₄-Alkyl, C₂-C₄-Alkenyl, C₂-C₄-Alkinyl und C₆-C₁₀-Aryl, R¹⁶ ausgewählt ist aus: einer chemischen Bindung, C₂-C₅-Alkyl, C₃-C₇-Cycloalkyl, C₃-C₅-Alkenyl, C₃-C₅-Alkinyl, C₆-C₁₀-Aryl, C₁-C₃-Alkyl-C₆-C₁₂-aryl, C₁-C₃-Alkyl-C₆-C₁₂-aryl und C₆-C₁₀-Aryl-C₁-C₂-alkyl, worin R¹⁴ oder R¹⁶ an N gebunden sind, R¹⁵ an Q gebunden ist und worin die Substituenten aus ein bis drei Resten R¹ ausgewählt sind;
L ein gegebenenfalls substituierter, zweiwertiger Rest ist, ausgewählt aus der Gruppe, bestehend aus: 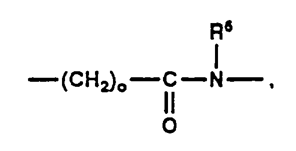 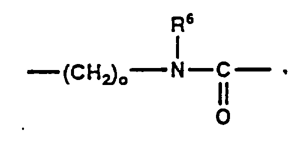 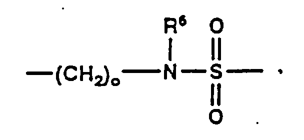 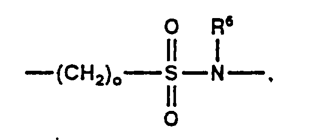 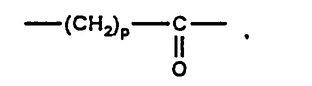 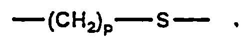 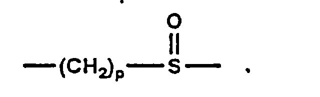 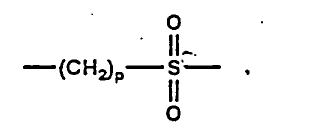 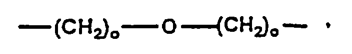 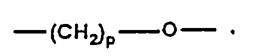 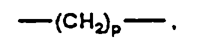 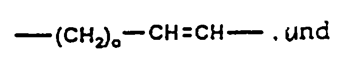 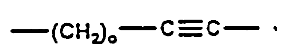 worin o = 1 oder 2 ist, p = 1, 2, 3 oder 4 ist, R⁶ wie zuvor definiert ist und worin die Substituenten aus ein bis drei Resten R¹ ausgewählt sind;
D ausgewählt ist aus der Gruppe, bestehend aus: R und -(C=O)-Xaa, worin Xaa ein bis drei D- oder L-α-Aminosäuren darstellt;
R⁸ ausgewählt ist aus der Gruppe, bestehend aus: (i) Hydroxy, (ii) C₁-C₈-Alkoxy, (iii) C₃-c₁₂-Alkenoxy, (iv) C₆₋C₁₂-Aryloxy, (v)C₁-C₆-Alkyl-C₆-C₁₂-aryloxy, (vi) Di-C₁-C₈-alkylamino-C₁-C₈-alkoxy, (vii) Acylamino-C₁-C₈-alkoxy, ausgewählt aus der Gruppe (a) Acetylaminoethoxy, (b) Nicotinoylaminoethoxy und (c) Succinamidoethoxy, (viii) C₁-C₈-Alkoyloxy-C₁-C₈-alkoxy, (ix) C₆-C₁₂-Aryl-C₁-C₈-alkoxy, worin die Aryl-Gruppe unsubstituiert oder mit ein bis drei der Gruppen: (a) Nitro, (b) Halogen (F, Cl, Br, l), (c) C₁-C₄-Alkoxy und (d) Amino substituiert ist, (x) Hydroxy-C₂-C₈-alkoxy, (xi) Dihydroxy-C₃-C₈-alkoxy und (xii) NR⁹R³⁰, worin R⁹ und R³⁰ voneinander unabhängig ausgewählt sind aus der Gruppe: (a) Wasserstoff, (b) C₁-C₈-Alkyl, (c) C₃-C₈-Alkenyl, (d) C₆-C₁₂-Aryl, worin die Aryl-Gruppe unsubstituiert oder mit ein bis drei der Gruppen: Nitro, Halogen (F, Cl, Br, l), C₁-C₄-Alkoxy und Amino substituiert ist, und (e) C₆-C₁₂-Aryl-C₁-C₈-alkyl, worin die Aryl-Gruppe unsubstituiert oder mit ein bis drei der Gruppen: Nitro, Halogen (F, Cl, Br, l) und C₁-C₄-Alkoxy substituiert ist; und
pharmazeutisch annehmbare Salze davon.

8. Verbindung nach Anspruch 1, dargestellt durch Formel (V): 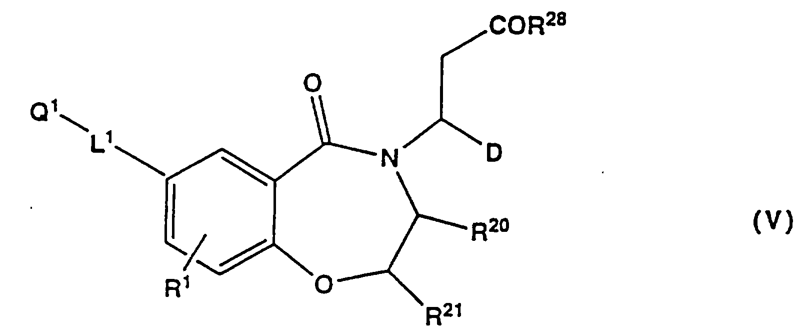
worin:
R¹ ein bis drei Gruppen darstellt, die unabhängig voneinander ausgewählt sind aus Wasserstoff und Halogen (F, Cl, Br, l);
Q¹ ausgewählt ist aus der Gruppe, bestehend aus:
(A) einer Amino-Gruppe,
(B) einer Amidino-Gruppe,
(C) einer Aminoalkylenimino-Gruppe,
(D) einer Iminoalkylenamino-Gruppe und
(E) einer Guanidino-Gruppe;
L¹ ein gegebenenfalls substituierter, zweiwertiger Rest ist, ausgewählt aus der Gruppe, bestehend aus: 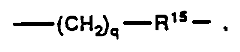 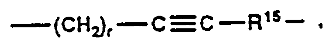 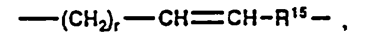 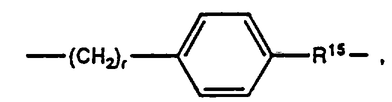 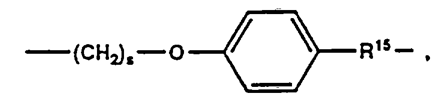 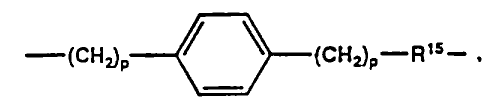 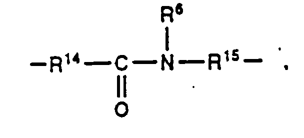 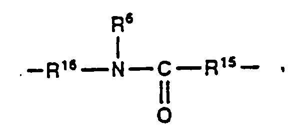 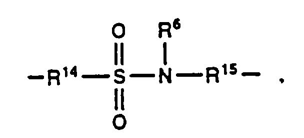 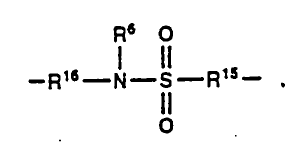 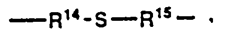 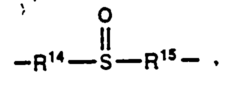 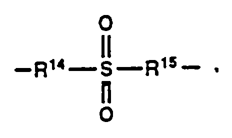 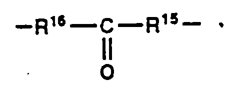 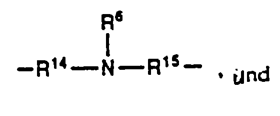 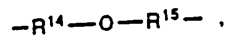
worin:
p = 1, 2, 3 oder 4 ist;
q = 3, 4, 5, 6 oder 7 ist;
r = 1, 2, 3, 4 oder 5 ist;
s = 2 oder 3 ist;
R¹⁴ ausgewählt ist aus:
C₂-C₅-Alkyl,
C₃-C₇-Cycloalkyl,
C₂-C₅-Alkenyl,
C₃-C₅-Alkinyl,
C₆-C₁₀-Aryl,
C₁-C₂-Alkyl-C₆-C₁₂-aryl,
C₁-C₂-Alkyl-C₆-C₁₀-aryl-C₁-C₂-alkyl,
C₆-C₁₀-Aryl-C₁-C₂-alkyl und
C₆-C₁₀-Aryloxy-C₁-C₂-alkyl;
R¹⁵ eine chemischen Bindung ist, die L¹ mit Position 8 des Benzazepinons verbindet;
R¹⁶ ausgewählt ist aus:
C₂-C₅-Alkyl,
C₃-C₇-Cycloalkyl,
C₃-C₅-Alkenyl,
C₃-C₅-Alkinyl,
C₆-C₁₀-Aryl,
C₁-C₃-Alkyl-C₆-C₁₂-aryl und
C₆-C₁₀-Aryl-C₁-C₂-alkyl,
worin R¹⁴ und R¹⁶ L¹ mit Q¹ verbinden und
worin die Substituenten ausgewählt sind aus der Gruppe: Wasserstoff, Halogen (F, Cl, Br, l), C₁-C₄-Alkoxy, C₁-C₄ -Alkyl, Phenyl, Benzyl und (mit F, Cl, Br, l) halogeniertem C₁-C₄-Alkyl;
R⁰ Wasserstoff ist;
R¹ ausgewählt ist aus der Gruppe, bestehend aus: (i) Wasserstoff, (ii) gegebenenfalls substituiertem C₁-C₁₂-Alkyl, (iii) gegebenenfalls substituiertem C₆-C₁₄-Aryl, (iv) gegebenenfalls substituiertem C₃-C₁₄-Cycloalkyl, (v) gegebenenfalls substituiertem C₁-C₁₂-Alkyl-C₆-C₁₄-aryl, (vi) gegebenenfalls substituiertem C₁-C₁₂-Alkyl-C₃-C₁₄-cycloalkyl, worin die Substituenten ausgewählt sind aus: (a) Halogen (F, Cl, Br, l), (b) Nitro, (c) Hydroxy, (d) Carboxy, (e) Tetrazol, (f) Hydroxamat, (g) Sulfonamid, (h) Trifluorimid, (i) Phosphonat, (j) C₁-C₆-Alkyl, (k) C₆-C₁₄-Aryl, (l) Benzyl, (m) C₃-C₁₄-Cycloalkyl, (n) COR⁴, worin R⁴ ausgewählt ist aus der Gruppe: C₁-C₈-Alkoxy, C₃-C₁₂-Alkenoxy, C₆-C₁₂-Aryloxy, Di-C₁-C₈-alkylamino-C₁-C₈-alkoxy, Acylamino-C₁-C₈-alkoxy, ausgewählt aus der Gruppe Acetylaminoethoxy, Nicotinoylaminoethoxy, Succinamidoethoxy und Pivaloyloxyethoxy, und C₆-C₁₂-Aryl-C₁-C₈-alkoxy, worin die Aryl-Gruppe
unsubstituiert oder mit ein bis drei der Gruppen: Nitro, Halogen (F, Cl, Br, l), C₁-C₄-Alkoxy, Amino, Hydroxy, Hydroxy-C₂-C₈-alkoxy und Dihydroxy-C₃-C₈-alkoxy substituiert ist, (o) CONR⁵R⁶, worin R⁵ und R⁶ unabhängig voneinander ausgewählt sind aus: Wasserstoff, C₁-C₁₀-Alkyl, C₃-C₁₀-Alkenyl, C₆-C₁₄-Aryl, C₁-C₆-Alkyl-C₆-C₁₀-aryl, wobei gegebenenfalls R⁵ und R⁶ zusammen Trimethylen, Tetramethylen, Pentamethylen und 3-Oxopentamethylen bilden können, und (vii) Q-L³-, worin Q aus Wasserstoff und Q¹ ausgewählt ist und L³ ausgewählt ist aus: einer chemischen Bindung und L¹, worin R¹⁵ L¹ mit Position 4 des Benzazepinons verbindet;
D ausgewählt ist aus der Gruppe, bestehend aus: R¹ und -(C=O)-Xaa, worin Xaa ein bis drei D- oder L-α-Aminosäuren darstellt;
R⁸ ausgewählt ist aus der Gruppe, bestehend aus: (i) Hydroxy, (ii) C₁-C₈-Alkoxy, (iii) C₃-C₁₂-Alkenoxy, (iv) C₆-C₁₂-Aryloxy, (v) C₁-C₆-Alkyl-C₆-C₁₂-aryloxy, (vi) Di-C₁-C₈-alkylamino-C₁-C₈-alkoxy, (vii) Acylamino-C₁-C₈-alkoxy, ausgewählt aus der Gruppe (a) Acetylaminoethoxy, (b) Nicotinoylaminoethoxy und (c) Succinamidoethoxy, (viii) C₁-C₈-Alkyloxy-C₁-C₈-alkoxy, (ix) C₆-C₁₂-Aryl-C₁-C₈-alkoxy, worin die Aryl-Gruppe unsubstituiert oder mit ein bis drei der Gruppen: (a) Nitro, (b) Halogen (F, Cl, Br, l), (c) C₁-C₄-Alkoxy und (d) Amino substituiert ist, (x) Hydroxy-C₂-C₈-alkoxy, (xi) Dihydroxy-C₃-C₈-alkoxy und (xii) NR⁹R³⁰, worin R⁹ und R³⁰ voneinander unabhängig ausgewählt sind aus der Gruppe: (a) Wasserstoff, (b) C₁-C₈-Alkyl, (c) C₃-C₈-Alkenyl, (d) C₆-C₁₂-Aryl, worin die Aryl-Gruppe unsubstituiert oder mit ein bis drei der Gruppen: Nitro, Halogen (F, Cl, Br, l), C₁-C₄-Alkoxy und Amino substituiert ist, und (e) C₆-C₁₂-Aryl-C₁-C₈-alkyl, worin Die Aryl-Gruppe unsubstituiert oder mit ein bis drei der Gruppen: Nitro, Halogen (F, Cl, Br, l) und C₁-C₄-Alkoxy substituiert ist; und
pharmazeutisch annehmbare Salze davon.

9. Verbindung nach Anspruch 1, dargestellt durch Formel (VI): 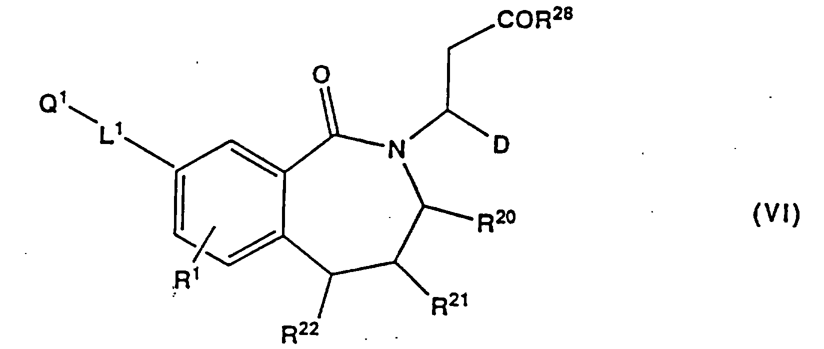
worin:
R¹ ein bis drei Gruppen darstellt, die unabhängig voneinander ausgewählt sind aus Wasserstoff und Halogen (F, Cl, Br, l);
Q¹ ausgewählt ist aus der Gruppe, bestehend aus:
(A) einer Amino-Gruppe,
(B) einer Amidino-Gruppe,
(C) einer Aminoalkylenimino-Gruppe,
(D) einer Iminoalkylenamino-Gruppe und
(E) einer Guanidino-Gruppe;
L¹ ein gegebenenfalls substituierter, zweiwertiger Rest ist, ausgewählt aus der Gruppe, bestehend aus: 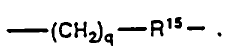 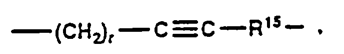 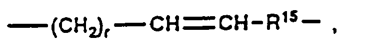 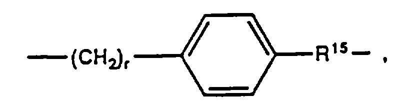 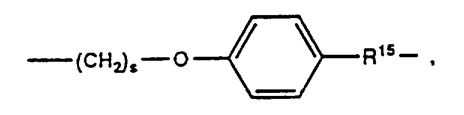 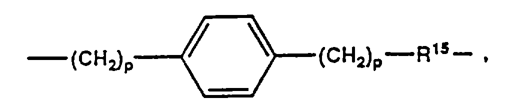 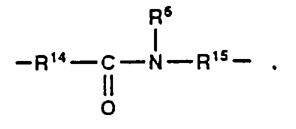 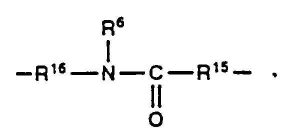 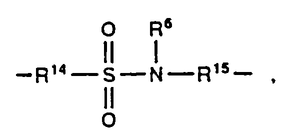 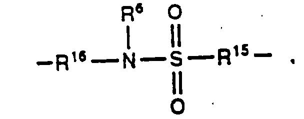 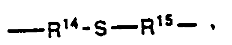 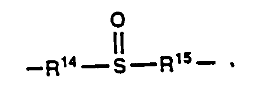 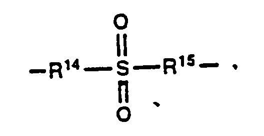 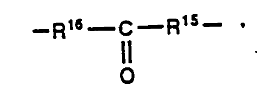 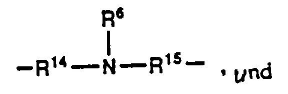 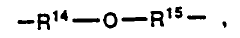
worin:
p = 1, 2, 3 oder 4 ist;
q = 3, 4, 5, 6 oder 7 ist;
r = 1, 2, 3, 4 oder 5 ist;
s = 2 oder 3 ist;
R¹⁴ ausgewählt ist aus:
C₂-C₅-Alkyl,
C₃-C₇-Cycloalkyl,
C₂-C₅-Alkenyl,
C₃-C₅-Alkinyl,
C₆-C₁₀-Aryl,
C₁-C₁₂-Alkyl-C₆-C₁₂-aryl,
C₁-C₂-Alkyl-C₆-C₁₀-aryl-C₁-C₂-alkyl,
C₆-C₁₀-Aryl-C₁-C₂-alkyl und
C₆-C₁₀-Aryloxy-C₁-C₂-alkyl;
R¹⁵ eine chemische Bindung ist, die L¹ mit Position 8 des Benzazepinons verbindet;
R¹⁶ ausgewählt ist aus:
C₂-C₅-Alkyl,
C₃-C₇-Cycloalkyl,
C₃-C₅-Alkenyl,
C₃-C₅-Alkinyl,
C₆-C₁₀-Aryl,
C₁-C₃-Alkyl-C₆-C₁₂-aryl und
C₆-C₁₀-Aryl-C₁-C₂-alkyl,
worinin R¹⁴ und R¹⁶ L¹ mit Q¹ verbinden, und
worin die Substituenten ausgewählt sind aus der Gruppe: Wasserstoff, Halogen (F, Cl, Br, l), C₁-C₄-Alkoxy, C₁-C₄ -Alkyl, Phenyl, Benzyl und (mit F, Cl, Br, l) halogeniertem C₁-C₄-Alkyl;
R⁰ Wasserstoff ist;
R¹ und R ausgewählt sind aus der Gruppe, bestehend aus: (i) Wasserstoff, (ii) gegebenenfalls substituiertem C₁-C₁₂-Alkyl, (iii) gegebenenfalls substituiertem C₆-C₁₄-Aryl, (iv) gegebenenfalls substituiertem C₃-C₁₄-Cycloalkyl, (v) gegebenenfalls substituiertem C₁-C₁₂-Alkyl-C₆-C₁₄-aryl, (vi) gegebenenfalls substituiertem C₁-C₁₂-Alkyl-C₃-C₁₄-cycloalkyl, worin die Substituenten ausgewählt sind aus: (a) Halogen (F, Cl, Br, l), (b) Nitro, (c) Hydroxy, (d) Carboxy, (e) Tetrazol, (f) Hydroxamat, (g) Sulfonamid, (h) Trifluorimid, (i) Phosphonat, (j) C₁-C₆-Alkyl, (k) C₆-C₁₄-Aryl, (l) Benzyl, (m) C₃-C₁₄-Cycloalkyl, (n) COR⁴, worin R⁴ ausgewählt ist aus der Gruppe: C₁-C₈-Alkoxy, C₃-C₁₂-Alkenoxy, C₆-C₁₂-Aryloxy, Di-C₁-C₈-alkylamino-C₁-C₈-alkoxy, Acylamino-C₁-C₈-alkoxy, ausgewählt aus der Gruppe: Acetylaminoethoxy, Nicotinoylaminoethoxy, Succinamidoethoxy und Pivaloyloxyethoxy, und C₆-C₁₂-Aryl-C₁-C₈-alkoxy, worin die Aryl-Gruppe unsubstituiert oder mit ein bis drei der Gruppen: Nitro, Halogen (F, Cl, Br, l), C₁-C₄-Alkoxy, Amino, Hydroxy, Hydroxy-C₂-C₈-alkoxy und Dihydroxy-C₃-C₈-alkoxy substituiert ist, (o) CONR⁵R⁶, worin R⁵ und R⁶ voneinander unabhängig ausgewählt sind aus: Wasserstoff, C₁-C₁₀-Alkyl, C₃-C₁₀-Alkenyl, C₆-C₁₄-Aryl, C₁-C₆-Alkyl-C₆-C₁₀-aryl, und gegebenenfalls R⁵ und R⁶ zusammen Trimethylen, Tetramethylen, Pentamethylen und 3-Oxopentamethylen bilden können, und (vii) Q-L³-, worin Q ausgewählt ist aus Wasserstoff und Q¹ und L³ ausgewählt ist aus einer chemischen Bindung und L¹, worin R¹⁵ L¹ mit Position 4 oder 5 des Benzazepinons verbindet;
D ausgewählt ist aus der Gruppe, bestehend aus: R¹ und -(C=O)-Xaa, worin Xaa ein bis drei D- oder L-α-Aminosäuren darstellt;
R⁸ ausgewählt ist aus der Gruppe, bestehend aus: (i) Hydroxy, (ii) C₁-C₈-Alkoxy, (iii) C₃-C₁₂-Alkenoxy, (iv) C₆-C₁₂-Aryloxy, (v) C₁-C₆-Alkyl-C₆-C₁₂-Aryloxy, (vi) Di-C₁-C₈-alkylamino-C₁-C₈-alkoxy, (vii) Acylamino-C₁-C₈-alkoxy, ausgewählt aus der Gruppe (a) Acetylaminoethoxy, (b) Nicotinoylaminoethoxy und (c) Succinamidoethoxy, (viii) C₁-C₈-Alkoyloxy-C₁-C₈-alkoxy, (ix) C₆-C₁₂-Aryl-C₁-C₈-alkoxy, worin die Aryl-Gruppe unsubstituiert oder mit ein bis drei der Gruppen: (a) Nitro, (b) Halogen (F, Cl, Br, l), (c) C₁-C₄-Alkoxy und (d) Amino substituiert ist, (x) Hydroxy-C₂-C₈-alkoxy, (xi) Dihydroxy-C₃-C₈-alkoxy und (xii) NR⁹R³⁰, worin R⁹ und R³⁰ voneinander unabhängig ausgewählt sind aus der Gruppe: (a) Wasserstoff, (b) C₁-C₈-Alkyl, (c) C₃-C₈-Alkenyl, (d) C₆-C₁₂-Aryl, worin die Aryl-Gruppe unsubstituiert oder mit ein bis drei der Gruppen: Nitro, Halogen (F, Cl, Br, l), C₁-C₄-Alkoxy und Amino substituiert ist, und (e) C₆-C₁₂-Aryl-C₁-C₈-alkyl, worin die Aryl-Gruppe unsubstituiert oder mit ein bis drei der Gruppen: Nitro, Halogen (F, Cl, Br, l) und C₁-C₄-Alkoxy substituiert ist; und
pharmazeutisch annehmbare Salze davon.

10. Pharmazeutische Zusammensetzung, umfassend einen pharmazeutisch annehmbaren Exzipienten und die Verbindung nach einem der Ansprüche 1 - 9.

11. Verbindung nach einem der Ansprüche 1 - 9 zur Verwendung in einem Behandlungsverfahren.

12. Verwendung einer Verbindung nach einem der Ansprüche 1 - 9 zur Herstellung eines Medikaments zur Hemmung der Blutplättchen-Aggregation.

13. Verwendung einer Verbindung nach einem der Ansprüche 1 - 9 zur Herstellung eines Medikaments zur Verringerung der Blutplättchen-Aggregation.

14. Verwendung einer Verbindung nach einem der Ansprüche 1 - 9 zur Herstellung eines Medikaments zur Behandlung eines Säugetiers mit erhöhter Neigung zu Thrombusbildung.

15. Verwendung nach Anspruch 14, worin das Medikament in Verbindung mit einem Thombolytikum verabreicht wird.

16. Verwendung nach Anspruch 14, worin das Medikament in Verbindung mit einem Antikoagulans verabreicht wird.

## Revendications

1. Composé représenté par la formule structurale (I) : 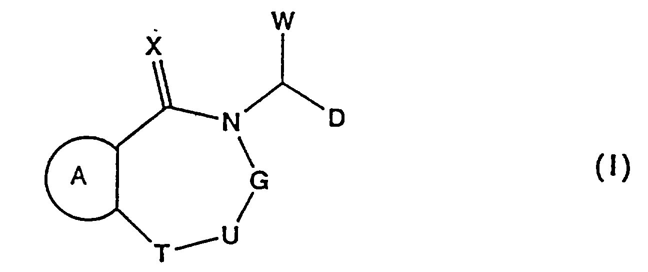
dans laquelle la structure partielle 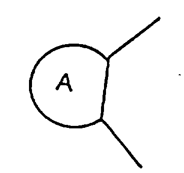
représente un groupe 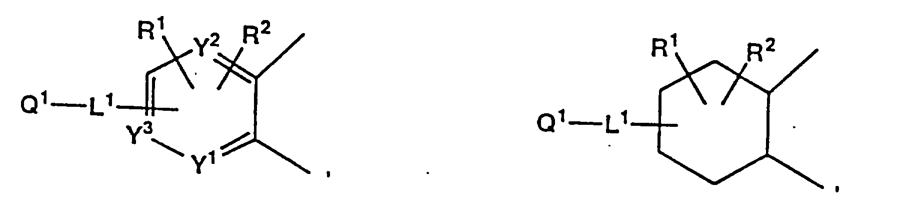 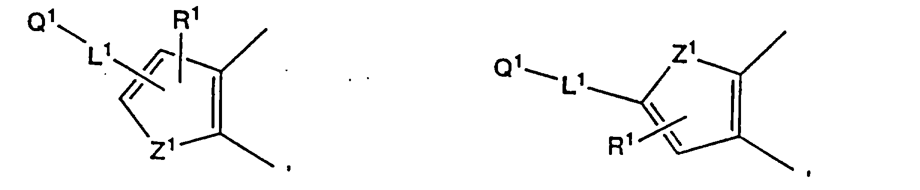 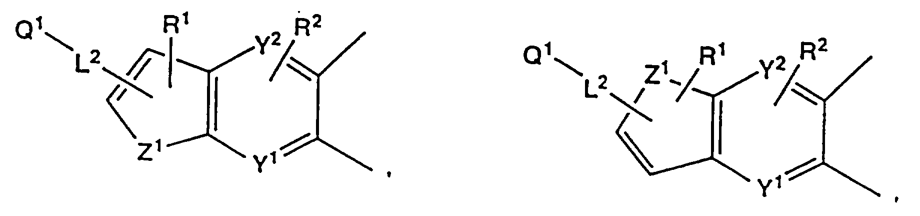 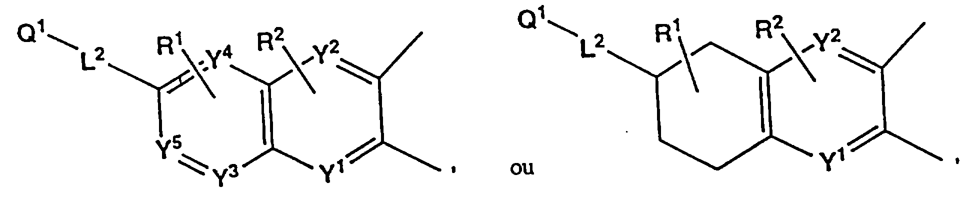
formules dans lesquelles
R¹ et R sont choisis, indépendamment, entre l'hydrogène, les groupes halogéno, (F, Cl, Br, I), cyano, carboxamido, carbamoyloxy, formyloxy, formyle, azido, nitro, uréido, thiouréido, thiocyanato, hydroxy, mercapto, sulfonamido, et un radical facultativement substitué choisi entre des radicaux alkyle en C₁ à C₁₂, alcényle en C₂ à C₁₂, alcynyle en C₃ à C₁₂, cycloalkyle en C₃ à C₁₂, aryle en C₆ à C₁₄, (aryle en C₆ à C₁₀)-(alkyle en C₁ à C₈), alkyloxy en C₁ à C₁₂, aryloxy en C₆ à C₁₄, alcylamino en C₁ à C₁₂, N,N-di(acyle en C₁ à C₁₂)amino, N-(alkyle en C₁ à C₁₂)-N-(C₁-sulfonulamino), (alkyle en C₁ à C₁₂)-thiocarbonyle, alkylthio en C₁ à C₁₂, alkylsulfinyle en C₁ à C₁₂, alkylsulfonyle en C₁ à C₁₂, alkylsulfonato en C₁ à C₁₂, N-(alkyle en C₁ à C₁₂)sulfonamido, N,N-di-(alkyle en C₁ à C₁₂) sulfonamido, N-(alkyle en C₁ à C₁₂) -N-thioformylamino, thioacylamino en C₁ à C₁₂, N-(alkyle en C₁ à C₁₂)-N-(thioacylamino en C₁ à C₁₂), alkylsulfinamido en C₁ à C₁₂, N-(alkyle en C₁ à C₁₂)-N-(alkyle en C₁ à C₁₂)sulfinylamino, carbalkoxy en C₁ à C₁₂, (alkyle en C₁ à C₁₂)-carbonyle, alcanoyloxy en C₁ à C₁₂, N-(alkyle en C₁ à C₁₂)carboxamido, N,N-di-(carboxamido en C₁ à C₁₂), N-(alkyle en C₁ à C₁₂)carbamoyloxy, N,N-di-(carbamoyloxy en C₁ à C₁₂), et hétérocycloalkyle ou hétéro-aryle ayant 1 à 3 noyaux, chaque noyau ayant 5 à 7 atomes avec 0 à 3 hétéro-atomes choisis entre N, O et S, sous réserve qu'au moins un noyau contienne un hétéro-atome, dans lesquels les substituants sont choisis entre des constituants halogéno (F, Cl, Br, l), cyano, azido, nitro, hydroxy, mercapto, sulfonamido, uréido, thiouréido, carboxamido, carbamoyloxy, formyloxy, formyle, alkyle en C₁ à C₄, alkoxy en C₁ à C₄, phényle et phénoxy, facultativement, R¹ et R, lorsqu'ils sont liés à des atomes de carbone adjacents, peuvent être réunis en formant un noyau aryle non substitué ou substitué, les substituants étant choisis entre des substituants halogéno (F, Cl, Br, I), cyano, azido, nitro, hydroxy, mercapto, sulfonamido, uréido, thiouréido, carboxamido, carbamoyloxy, formyloxy, formyle, alkyle en C₁ à C₄, alkoxy en C₁ à C₄, phényle et phénoxy ;
Y¹, Y, Y³, Y⁴ et Y⁵ sont choisis, indépendamment, entre CH, CR¹, CR et N ;
X est choisi entre O, S, =NCN et =NO(alkyle en C₁ à C₃) ;
Z¹ est choisi entre CH₂, NH, S et O ;
Q¹ est choisi dans le groupe consistant en
(A) un groupe amino choisi entre
-NH₂,
-NR³H,
-NR³R⁴, et
-NR³R⁴R⁵,
dans lesquels R³, R⁴ et R⁵ sont choisis, indépendamment, entre
(i) un groupe cyano,
(ii) un radical facultativement substitué choisi entre
(a) -NR⁶R⁷,
(b) - C=NR⁸) -NR⁶R⁷,
(c) -N=CR⁹-NR⁶R⁷,
(d) -NR¹⁰-CR⁹=NR⁸, et
(e) -NR¹⁰-C (=NR⁸) -NR⁶R⁷,
dans lesquels les groupes R⁶, R⁷, R⁸, R⁹ et R¹⁰ sont choisis chacun, indépendamment, entre
l'hydrogène,
un groupe alkoxy en C₁ à C₄,
un groupe alyle en C₁ à C₄, et
un groupe halogéno (F, Cl, Br, I)-alkyle en C₁ à C₄,
(iii) un groupe alkyle en C₁ à C₁₂, facultativement substitué,
(iv) un groupe cycloalkyle en C₃ à C₁₂ facultativement substitué,
(v) un groupe aryle en C₆ à C₁₄ facultativement substitué,
(vi) un groupe (alkyle en C₁ à C₆)-(aryle en C₆ à C₁₄) facultativement substitué,
(vii) un groupe alkoxy en C₁ à C₈ facultativement substitué,
(vii) un groupe aryloxy en C₆ à C₁₄ facultativement substitué, et
(ix) un groupe (alkoxy en C₁ à C₈)-carbonyle facultativement substitué,
dans lesquels les substituants consistent en un à trois groupes R¹¹, chaque groupe R¹¹ étant choisi indépendamment entre
(a) un groupe aryloxy en C₆ à C₁₂ facultativement substitué,
(b) un groupe arylamino en C₆ à C₁₂ facultativement substitué,
(c) un groupe aroyle en C₆ à C₁₂ facultativement substitué,
(d) un groupe alkoxy en C₁ à C₈ facultativement substitué,
(e) un groupe (alkoxy en C₁ à C₈)-alkyle facultativement substitué,
(f) un groupe (alkoxy en C₁ à C₈)-alkyloxy facultativement substitué,
(g) un groupe (alkoxy en C₁ à C₈)-carbonyle facultativement substitué,
(h) un groupe (alkyle en C₁ à C₈)-carbonyle facultativement substitué,
(i) un groupe (aralkyle en C₁ à C₈)-carbonyle facultativement substitué,
(j) un groupe (alkyle en C₁ à C₈)-thiocarbonyle facultativement substitué,
(k) un groupe (aralkyle en C₆ à C₁₂)-thiocarbonyle facultativement substitué,
(l) un groupe (alkoxy en C₂ à C₈)-thiocarbonyle facultativement substitué,
(m) un groupe aryle en C₆ à C₁₂ facultativement substitué,
(n) un groupe alkcanoylamino en C₁ à C₄ facultativement substitué,
(o) un ruope (alkoxy en C₁ à C₆)-carbonyle-(alkylamino en C₀ à C₆) facultativement substitué,
(p) un groupe alkylsulfonylamino en C₁ à C₈ facultativement substitué,
(q) un groupe (aralkyle en C₆ à C₁₀)sulfonylamino facultativement substitué,
(r) un gruope aralkyle en C₆ à C₁₀ facultativement substitué,
(s) un groupe alkaryle en C₆ à C₁₀ facultativement substitué,
(t) un groupe alkylthio en C₁ à C₈ facultativement substitué,
(u) un groupe aralkylthio en C₆ à C₁₀ facultativement substitué,
(v) un groupe alkylsulfinyle en C₁ à C₈ facultativement substitué,
(w) un groupe aralkylsulfinyle en C₆ à C₁₀ facultativement substitué,
(x) un groupe alkylsulfonyle en C₁ à C₈ facultativement substitué,
(y) un groupe aralkylsulfonyle en C₆ à C₁₀ facultativement substitué,
(z) un groupe alkylaminosulfonyle en C₁ à C₈ facultativement substitué,
(aa) un groupe aralkylsulfonylamino en C₆ à C₁₀ facultativement substitué,
(ab) un hétérocycle penta- ou hexagonal facultativement substitué contenant 1 à 4 hétéro-atomes choisis entre N, O et S, et
(ac) un groupe alcényle en C₂ à C₈ facultativement substitué,
dans lesquels les substituants consistent en un à trois R¹, chaque groupe R¹ étant choisi, indépendamment, entre des groupes
nitro,
amino,
alkylamino en C₁ à C₈,
di-(alkyle en C₁ à C₈)amino,
amidino,
aminométhylèneimino,
imino,
imino-alkyle en C₁ à C₄,
iminométhylèneamino,
guanidino,
arylamino en C₆ à C₁₀,
acylamino en C₁ à C₈,
alkylsulfonamino en C₁ à C₄,
azido,
cyano,
hydroxy,
hydroxyalkyle en C₁ à C₈,
alkoxy en C₁ à C₈,
phényloxy,
alcanoyloxy en C₁ à C₈,
alcanoyle en C₁ à C₈,
aroyle en C₆ à C₁₂,
benzamido,
phényle,
halogéno (F, Cl, Br, I),
halogénalkyle en C₁ à C₈, et
alkyle en C₁ à C₈,
(ad) aminosulfonyle
(ae) oxo,
(af) thio,
(ag) thiocarbonyle,
(ah) hydroxy,
(ai) mercapto,
(aj) formyle,
(ak) formyloxy,
(al) carboxy,
(am) amino,
(an) uréido,
(ao) amidino,
(ap) guanidino,
(aq) aminométhylèneimino,
(ar) imino,
(as) glycyle,
(at) phtalimido,
(au) succinimido,
(av) morpholino,
(aw) cycloalkyle en C₃ à C₇, et
(ax) halogéno (F, Cl, Br, I),
facultativement, R³ et R⁴, pris conjointement, peuvent former un groupe, facultativement substitué,
tétraméthylène,
pentaméthylène,
3-oxopentaméthylène, et
3-azapentaméthylène,
dans lesquels les substituants consistent en un à trois groupes R¹,
(B) un groupe amino choisi entre
-C(=NH)-NH₂
-C(=NH)-NHR³,
-C(=NR⁴)-NHR³,
-C(=NH)-NR³R⁴, et
-C(=NR⁵)-NR³R⁴,
dans lesquels R³, R⁴ et R⁵ répondent aux définitions précitées,
(C) un groupe aminoalkylèneimino choisi entre
-N=CH-NH₂,
-N=CH-NHR³,
-N=CH-NR³R⁴, et
-N=CR⁵-NR³R⁴,
dans lesquels R³, R⁴ et R⁵ répondent aux définitions précitées,
(D) un groupe iminoalkylèneamino choisi entre
-NH-CH=NH,
-NH-CH=NR³,
-NH-CR⁴=NR³, et
-NR⁵-CR⁴=NR³,
dans lesquels R³, R⁴ et R⁵ répondent aux définitions précitées,
(E) un groupe guanidino choisi entre
-NH-C (=NH) -NH₂,
-NH-C(=NH) -NR³H,
-NH-C (=NH) -NR³R⁴,
-NH-C (=NR⁵) -NR³R⁴,
-NR³-C (=NR³) -NR³R⁴,
-NR³-C(=NH) -NR³R⁴,
-NR³-C(=NR³) -NH₂,
-NR³-C (=NH) -NH₂,
-NR³-C (=NR³) -NHR⁴, et
-NR³-C (=NH) -NHR⁴,
dans lesquels R³, R⁴ et R⁵ répondent aux définitions précitées,
(F) un groupe hétérocyclique saturé facultativement substitué choisi entre 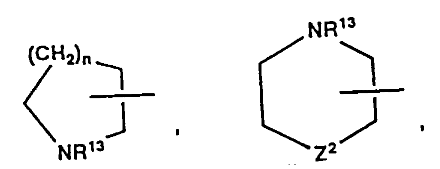 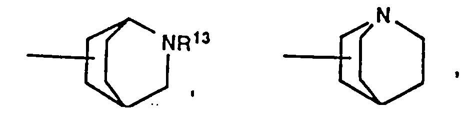 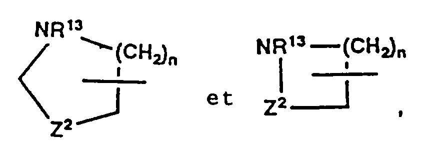 dans lesquels (1) n est égal à 0, 1, 2 ou 3, (2) R¹³ est choisi entre R⁶, -CR⁹=NR⁸, -CR⁹ (=NR⁸) -NR⁶R⁷, -C(=NR8) -NR⁶R⁷, -N=CR⁹-NR⁶R⁷, -NR¹⁰-CR⁹=NR⁸ et -NR¹⁰- (C=NR⁸) -NR⁶R⁷ dans lesquels R⁶ et R¹⁰ répondent aux définitions précitées, (3) Z représente O, S ou un groupe NR¹³, et (4) les substituants consistent indépendamment en un à trois groupes R¹ ;
(G) un groupe hérocyclyle (non aromatique insaturé facultativement substitué choisi entre 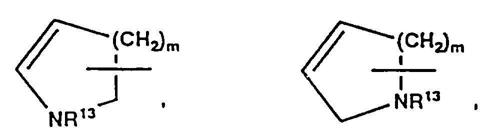 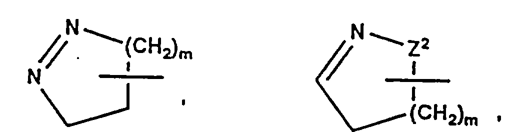 et 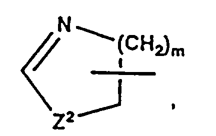 dans lesquels (1) m est égal à 1, 2 ou 3, (2) Z et R¹³ répondent aux définitions précitées et (3) les substituants consistent, indépendamment, en un à trois groupes R¹ ;
(H) un groupe hétérocyclyle (aromatique) insaturé facultativement substitué choisi entre 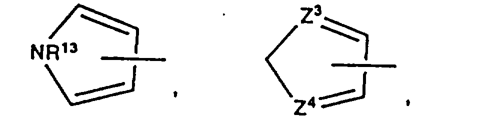 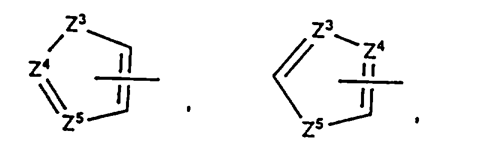 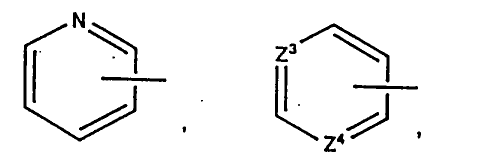 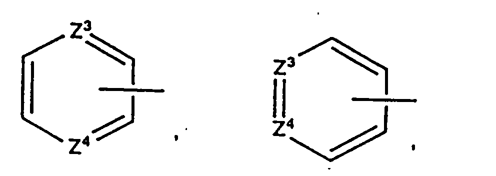 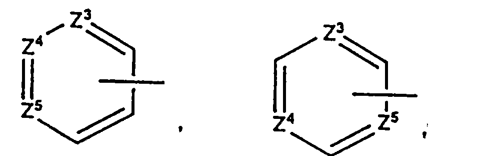 et 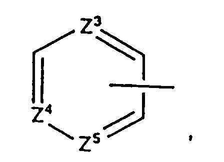 dans laquelle (1) Z³, Z⁴ et Z⁵ sont choisis entre 0, S, N et un groupe NH, sous réserve qu'au moins un groupe Z³, Z⁴ ou Z⁵ représente N ou un groupe NH, (2) R¹³ répond à la définition précitée, et les substituants consistent, indépendamment, en un à trois groupes R¹,
(I) un groupe bicyclohétérocyclique facultativement substitué choisi entre : 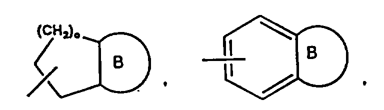 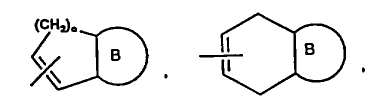 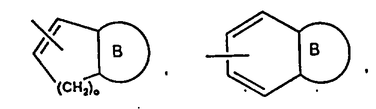 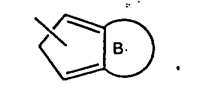 dans lesquels la structure partielle 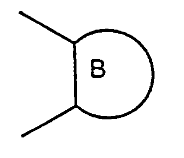 représente un groupe 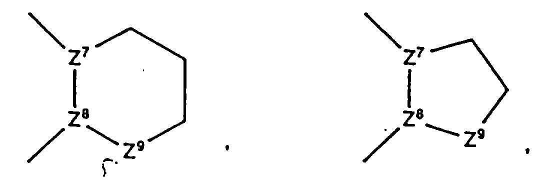 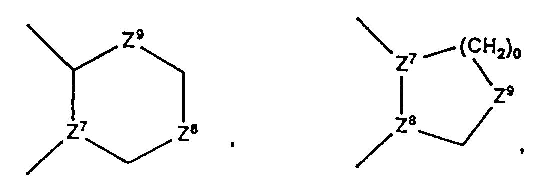 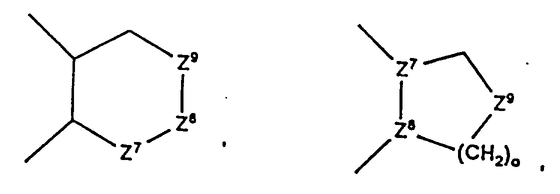 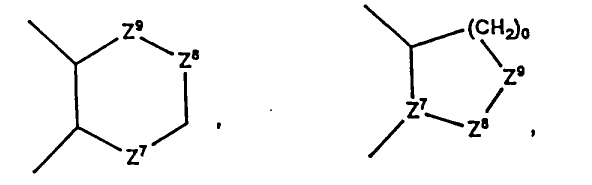 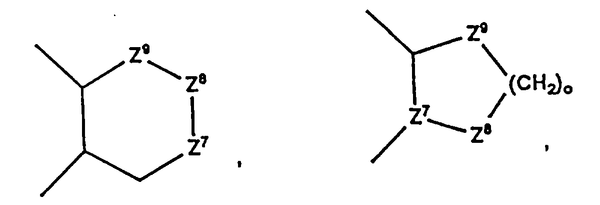 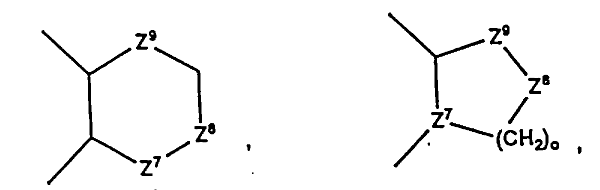 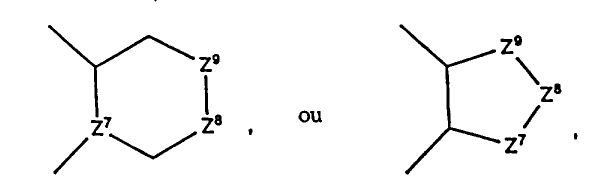 formules dans lesquelles Z⁷, Z⁸ et Z⁹ sont choisis, indépendamment, entre 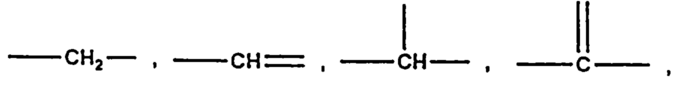 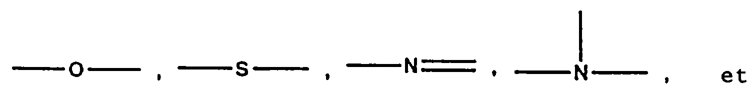 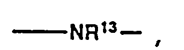 sous réserve qu'au moins un groupe Z⁷, Z⁸ et Z⁹ représente un groupe 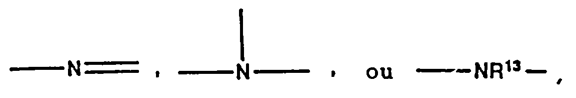 formules dans lesquelles (1) o est égal à 0, 1 ou 2, (2) R¹³ répond à la définition précitée, et (3) les substituants consistent, indépendamment, en un à trois groupes R¹ ;
L¹ représente un radical bivalent facultativement substitué choisi dans le groupe consistant en
(A) un groupe alkylène en C₃ à C₇,
(B) un groupe cycloalkylène en C₃ à C₇,
(C) un groupe alcénylène en C₃ à C₇,
(D) un groupe alcadiénylène en C₃ à C₇,
(E) un groupe alcynylène en C₃ à C₇,
(F) un groupe alcadiynylène en C₄ à C₇,
(G) un groupe alcénylène en C₄ à C₇,
(H) un groupe arylène en C₆ à C₁₄,
(I) un groupe (aryle en C₆ à C₁₄)-alcynylène en C₂ à C₄,
(J) un groupe (alkyle en C₁ à C₃)-(aryle en C₆ à C₁₄)-alcynylène en C₂ à C₄,
(K) un groupe (aryle en C₆ à C₁₄)-(alcénylène en C₂ à C₄),
(L) un groupe (alkyle en C₁ à C₃) - (arylène en C₆ à C₁₄),
(M) un groupe (alkyle en C₁ à C₃)-(aryle en C₆ à c₁₄) - (alcénylène en C₂ à C₄),
(N) un groupe (aryle en C₆ à C₁₄)-(alkylène en C₁ à C₃),
(O) un groupe (aryle en C₆ à C₁₄)-(alkyloxyène en C₁ à C₃),
(P) un groupe (alkyle en C₁ ou C₂)-(aryle en C₆ à C₁₄)-(alkylène en C₁ ou C₂),
(Q) un groupe (alkyle en C₁ à C₃)-oxy-(arylène en C₆ à C₁₄),
(R) un groupe alkyloxyène en C₂ à C₆,
(S) un groupe (alkyle en C₁ à C₅)-oxy-(alkylène en C₁ à C₅),
(T) un groupe aryloxyène en C₆ à C₁₀,
(U) un groupe (aryle en C₆ à C₁₀)-oxy-(alkylène en C₁ à C₅),
(V) un groupe alkylthioène en C₂ à C₆,
(W) un groupe (alkyle en C₁ à C₅)-thio-(alkylène en C₁ à C₅),
(X) un groupe arylthioène en C₆ à C₁₀,
(Y) un groupe (aryle en C₆ à C₁₀)-thio-(alkylène en C₁ à C₅),
(Z) un groupe (alkyle en C₁ à C₅)-sulfoxyde-(alkylène en C₁ à C₅),
(AA) un groupe (alkyle en C₁ à C₅)-sulfone-(alkylène en C₁ à C₅), 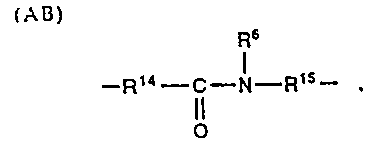 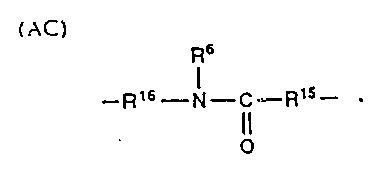 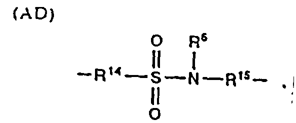 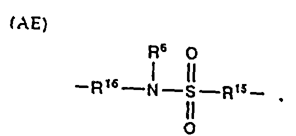 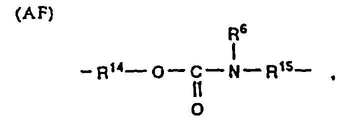 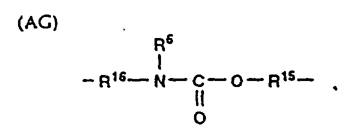 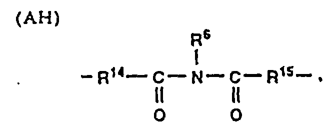 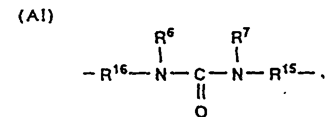 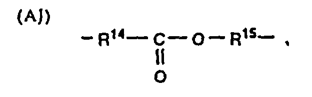 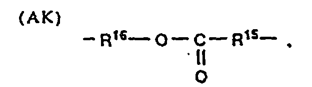 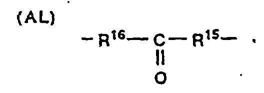 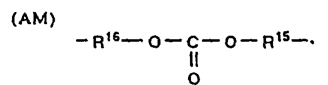 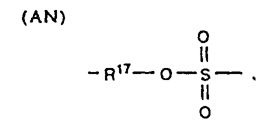 un groupe 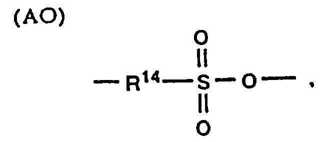 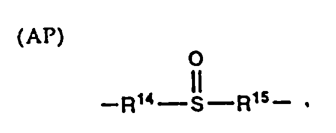 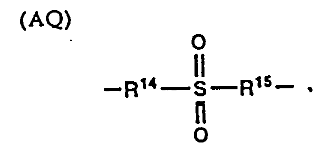 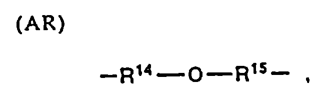 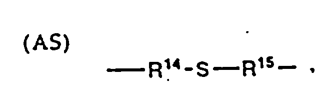 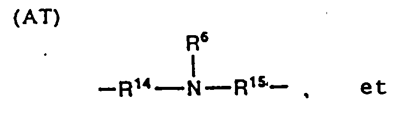 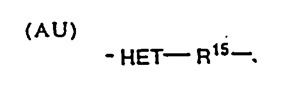
dans lesquels
R¹⁴ est choisi entre
une liaison chimique,
un groupe alkyle en C₁ à C₈,
un groupe cycloalkyle en C₃ à C₇,
un groupe alcényle en C₂ à C₅,
un groupe alcynyle en C₃ à C₅,
un groupe aryle en C₆ à C₁₀,
un groupe (alkyle en C₁ à C₃)-(aryle en C₆ à C₁₂),
un groupe (alkyle en C₁ ou C₂)-(aryle en C₆ à C₁₀)-(alkyle en C₁ ou C₂),
un groupe (aryle en C₆ à C₁₀)-(alkyle en C₁ ou C₂), et
un groupe (aryle en C₆ à C₁₀)-oxy-(alkyle en C₁ ou C₂),
R¹⁵ est choisi entre
une liaison chimique
un groupe alkyle en C₁ à C₄,
un groupe alcényle en C₂ à C₄,
un groupe alcynyle en C₂ à C₄,
un groupe aryle en C₆ à C₁₀, et
un groupe (alkyle en C₁ à C₃)-(aryle en C₆ à C₁₂),
R¹⁶ est choisi entre
une liaison chimique
un groupe alkyle en C₁ à C₅,
un groupe cycloalkyle en C₃ à C₇,
un groupe alcényle en C₃ à C₅,
un groupe alcynyle en C₃ à C₅,
un groupe aryle en C₆ à C₁₀,
un groupe (alkyle en C₁ à C₃)-(aryle en C₆ à C₁₂), et
un groupe (aryle en C₆ à C₁₀)-(alkyle en C₁ ou C₂),
R¹⁷ est choisi entre
un groupe alcényle en C₃ ou C₄,
un groupe alcynyle en C₃ ou C₄,
un groupe aryle en C₆ à C₁₀, et
un groupe benzyle, et
HET désigne un hétérocycle ayant 5 à 14 atomes dans 1 à 3 cycles, chaque cycle ayant 1 à 3 hétéro-atomes choisis entre N, O et S,
les substituants consistant en un à trois groupes R¹ ;
L représente un radical bivalent facultativement substitué choisi dans le groupe consistant en un groupe 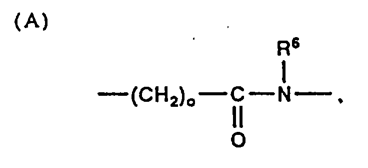 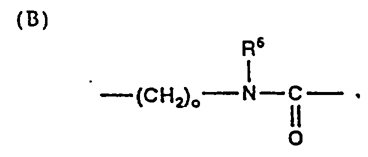 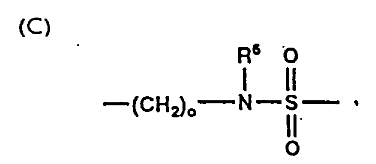 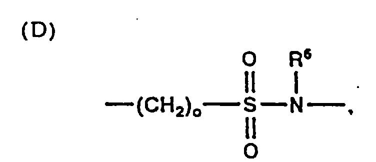 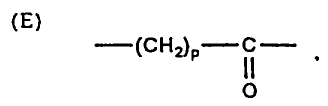 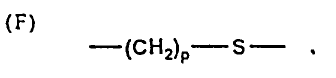 un groupe 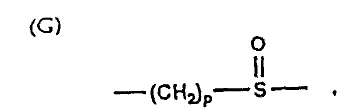 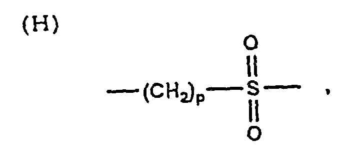 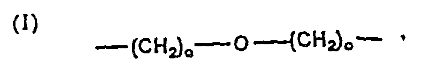 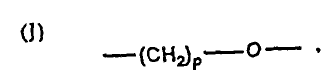 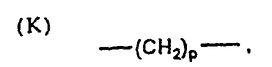 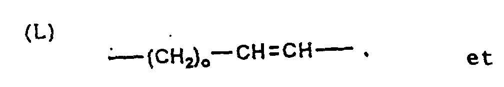 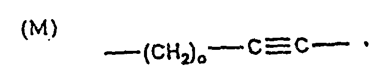
dans lesquels
o est égal à 1 ou 2,
p est égal à 1, 2, 3 ou 4,
R⁶ répond à la définition précitée, et
les substituants consistent en un ou trois groupes R¹ ;
T-UG est choisi dans le groupe consistant en un groupe 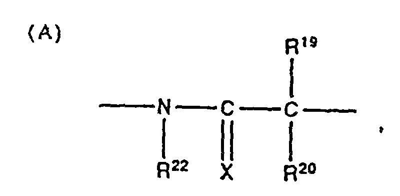 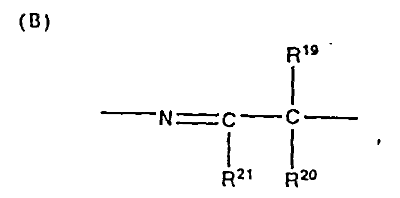 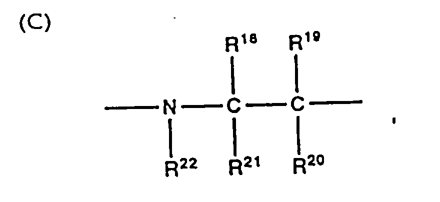 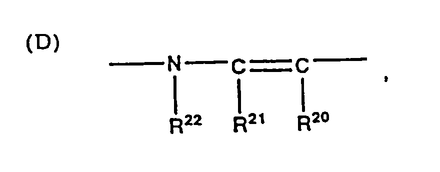 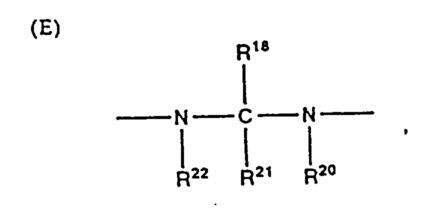 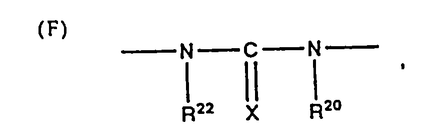 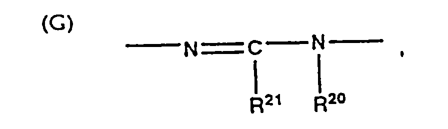 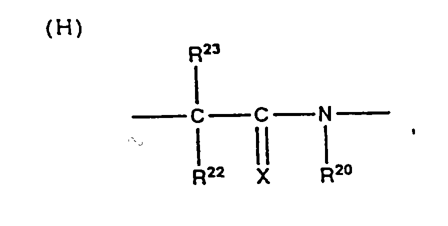 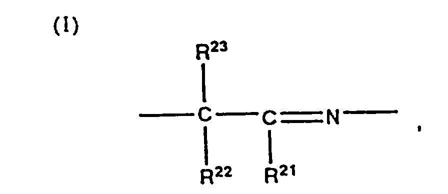 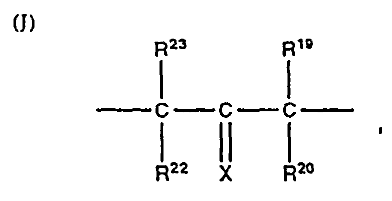 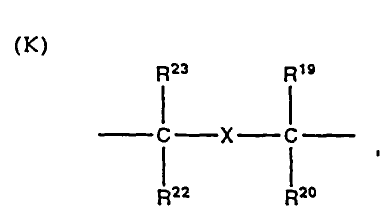 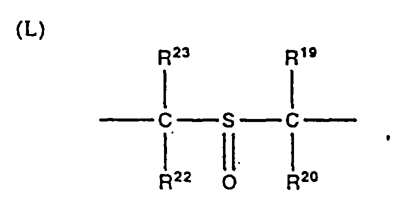 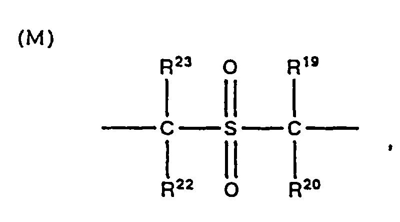 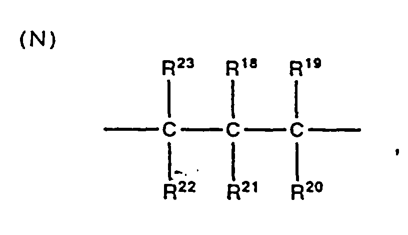 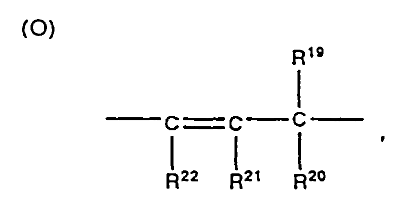 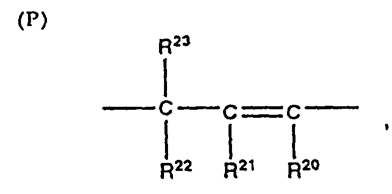 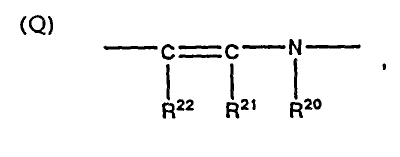 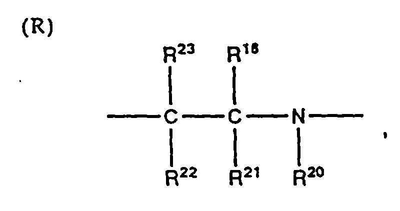 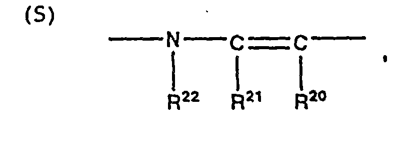 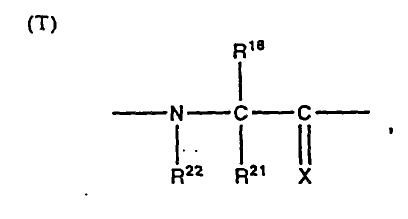 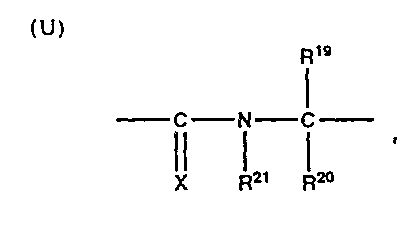 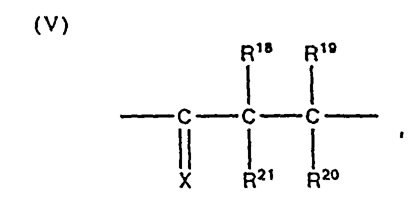 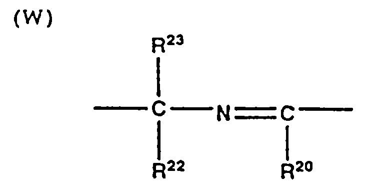 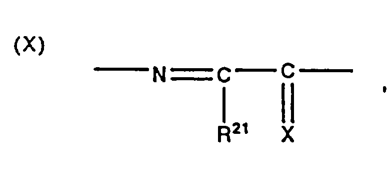 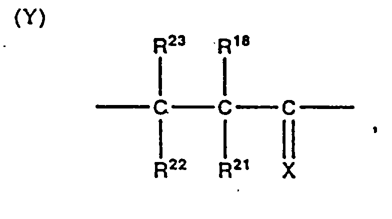 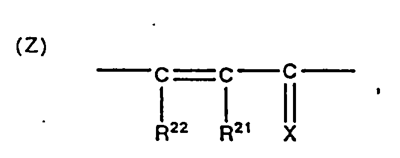 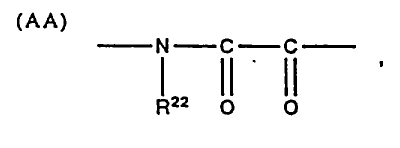 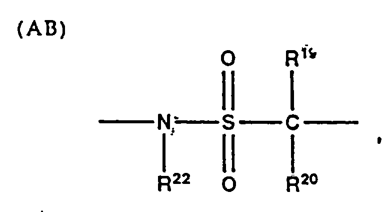 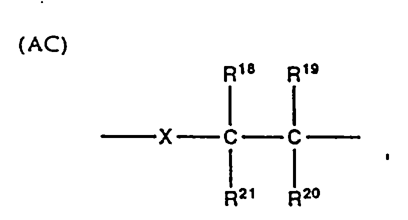 un groupe 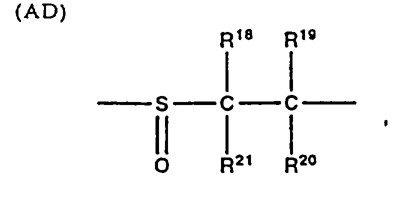 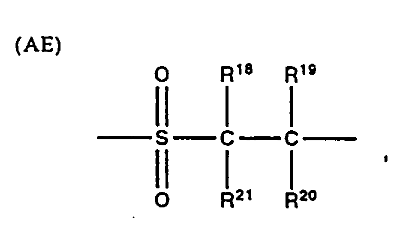 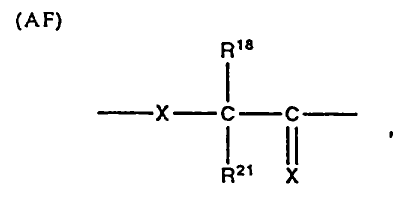 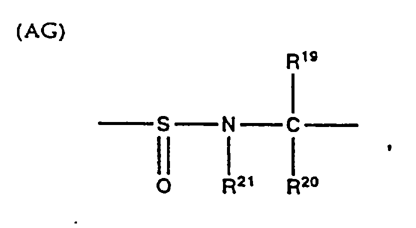 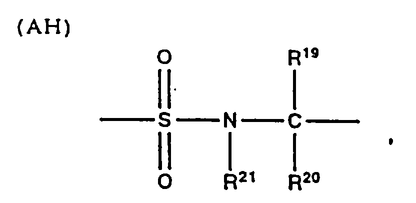 et 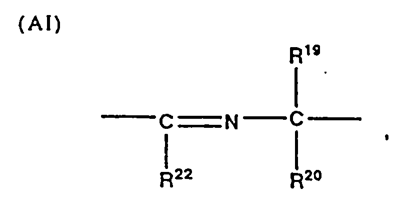
dans lesquels
X représente O ou S,
R¹⁸ et R³ sont choisis, indépendamment, dans le groupe consistant en
(i) hydrogène,
(ii) un groupe alkyle en C₁ à C₁₂ facultativement substitué,
(iii) un groupe alcényle en C₃ à C₁₂ facultativement substitué,
(iv) un groupe cycloalkyle en C₃ à C₁₄ facultativement substitué,
(v) un groupe (alkyle en C₁ à C₁₂)-(aryle en C₆ à C₁₄) facultativement substitué,
(vi) un groupe aryle en C₆ à C₁₄ facultativement substitué,
(vii) un groupe (alkyle en C₁ à C₄)-phényle facultativement substitué, et
(viii) un groupe alkoxy en C₁ à C₁₂ facultativement substitué,
R¹⁹ et R⁰ sont choisis, indépendamment, entre
(i) l'hydrogène,
(ii) un groupe halogéno (F, Cl, Br, I),
(iii) un groupe alkoxy en C₁ à C₄,
(iv) un groupe alkyle en C₁ à C₄,
(v) un groupe phényle,
(vi) un groupe benzyle, et
(vii) un groupe halogéno (F, Cl, Br, I)-(alkyle en C₁ à C₄,
R¹ et R sont choisis, indépendamment, dans le groupe consistant en
(i) l'hydrogène,
(ii) un groupe alkyle en C₁ à C₁₂ facultativement substitué,
(iii) un groupe aryle en C₆ à C₁₄ facultativement substitué,
(iv) un groupe cycloalkyle en C₃ à C₁₄ facultativement substitué,
(v) un groupe (alkyle en C₁ à C₁₂)-(aryle en C₆ à C₁₄) facultativement substitué, et
(vi) un groupe (alkyle en C₁ à C₁₂) - (cycloalkyle en C₃ à C₁₄) facultativement substitué,
dans lesquels les substituants sont choisis entre
(a) un groupe halogéno (F, Cl, Br, I),
(b) un groupe nitro,
(c) un groupe hydroxy,
(d) un groupe carboxy,
(e) un groupe tétrazole,
(f) un groupe hydroxamate,
(g) un groupe sulfamide,
(h) un groupe trifluorimide,
(i) un groupe phosphonate,
(j) un groupe alkyle en C₁ à C₆,
(k) un groupe aryle en C₆ à C₁₄,
(l) un groupe benzyle,
(m) un groupe cycloalkyle en C₃ à C₁₄,
(n) un groupe COR⁴,
dans lequel R⁴ est choisi dans le groupe consistant en
un groupe alkoxy en C₁ à C₈,
un groupe alcénoxy en C₃ à C₁₂,
un groupe aryloxy en C₆ à C₁₂,
un groupe (alkyle en C₁ à C₆)-(aryloxy en C₆ à C₁₂),
un groupe di-(alkyle en C₁ à C₈)-amino-(alkoxy en C₁ à C₈),
un groupe acylamino-(alkoxy en C₁ à C₈) choisi dans le groupe consistant en
un groupe acétylaminoéthoxy,
un groupe nicotinoylaminoéthoxy ; et
un groupe succinamidoéthoxy,
un groupe (alkoyloxy en C₁ à C₈)-oxy-(alkoxy en C₁ à C₈), et
un groupe (aryle en C₆ à C₁₂) -(alkoxy en C₁ à C₈) dans lequel le groupe aryle est non substitué ou substitué avec un à trois des groupes
nitro,
halogéno (F, Cl, Br, I),
alkoxy en C₁ à C₄,
amino,
hydroxy,
hydroxy-alkoxy en C₂ à C₈, et
dihydroxy-alkoxy en C₃ à C₈, et
(o) un groupe CONR⁵R⁶
dans lequel R⁵ et R⁶ sont choisis, indépendamment entre
l'hydrogène,
un groupe alkyle en C₁ à C₁₀,
un groupe alcényle en C₃ à C₁₀,
un groupe aryle en C₆ à C₁₄,
un groupe (alkyle en C₁ à C₆)-(aryle en C₆ à C₁₀),
facultativement, R⁵ et R⁶, conjointement, peuvent former un groupe
triméthylène,
tétraméthylène,
pentaméthylène, et
3-oxopentaméthylène, et
(vii) un groupe Q-L³
dans lequel Q est choisi entre
l'hydrogène, et
Q¹, et
L³ est choisi entre
une liaison chimique,
L¹, et
L ;
D est choisi dans le groupe consistant en
R, et
un groupe -(C=O)-Xaa, dans lequel Xaa représente un à trois résidus de D- ou L-α-amino-acides ;
W représente un groupe -R⁷-w dans lequel
R⁷ est choisi entre
(a) une liaison covalente,
(b) un groupe méthylène substitué ou non substitué, et
(c) un groupe éthylène substitué ou non substitué,
dans lesquels les substituants sont choisis entre
(i) un groupe nitro,
(ii) un groupe halogéno (F, Cl, Br, I),
(iii) un groupe alkyle en C₁ à C₆,
(iv) un groupe halogéno (F, Cl, Br, I)-(alkyle en C₁ à C₆), et
(v) un groupe phényle substitué ou non substitué,
les substituants sur le groupe phényle étant choisis entre
(1) un groupe alkyle en C₁ à C₆,
(2) un groupe alkoxy en C₁ à C₆,
(3) un groupe halogéno (F, Cl, Br, I), et
(4) un groupe CF₃ ;
w est choisi entre les groupes
(a) -COR⁸,
(b) -SO₃R³¹,
(c) -NHSO₂R³,
(d) -PO(OR³¹)₂,
(e) -SO₂NHR³,
(f) -CONHOR³¹,
(g) -C(OH)R³³)₂PO(OR³³)₂,
(h) -CN,
(i) un groupe -SO₂NH-hétérocycle dans lequel l'hétérocycle est un noyau aromatique penta- ou heptagonal contenant 1 à 3 hétéro-atomes choisis entre 0, N et S et l'hétérocycle est non substitué ou substitué avec un ou deux substituants choisis entre des groupes
(i) -OH,
(ii) -SH,
(iii) alkyle en C₁ à C₄,
(iv) alkoxy en C₁ à C₄,
(v) CF₃,
(vi) halogéno (F, Cl, Br, I),
(vii) NO₂,
(viii) -COOH,
(ix) -COO-(alkyle en C₁ à C₄),
(x) -NH₂,
(xi) -NH(alkyle en C₁ à C₄), et
(xii) -N(alkyle en C₁ à C₄)₂,
(j) -CH₂SO₂NH-hétérocycle,
(k) -SO₂NHCOR³³,
(l) -CH₂SO₂NHCOR³,
(m) -CONHSO₂R³³,
(n) -CH₂CONHSO₂R³³,
(o) -NHCONHSO₂R³³,
(p) -NHSO₂NHCOR³³,
(q) -CONHNHSO₂CF₃,
(r) -CON(OH)R³¹,
(s) -CONHCOCF₃,
(t) -CONHSO₂R⁸,
(u) -CONHSO₂R⁹,
(v) -CONHSO₂R³⁰, 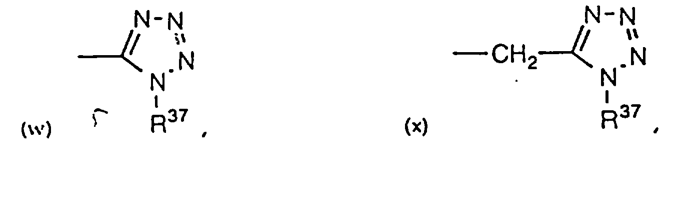 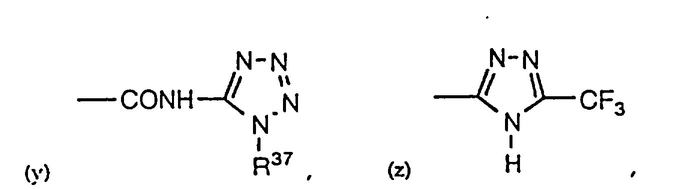 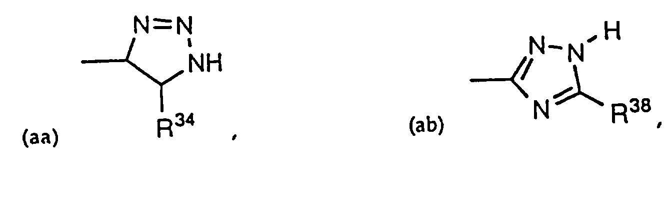 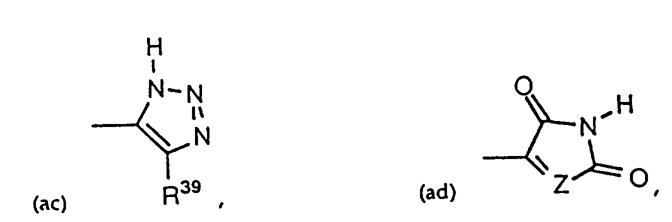 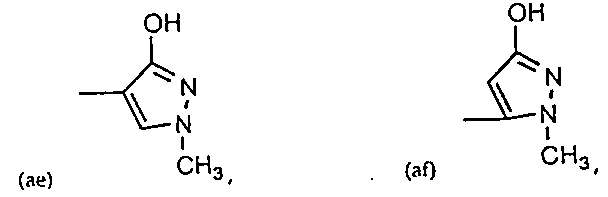 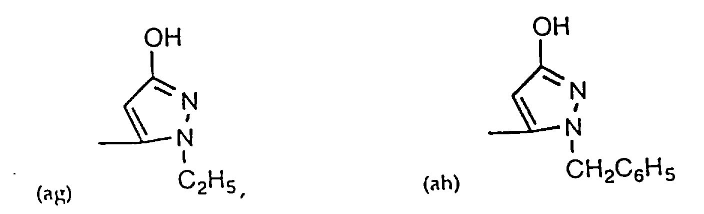 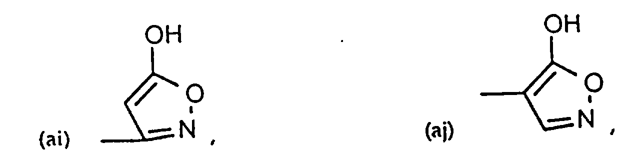 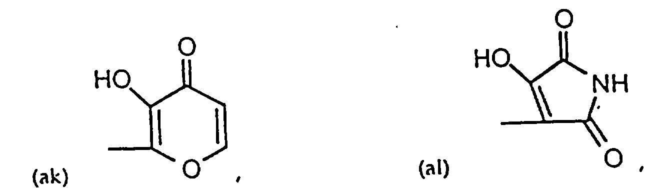 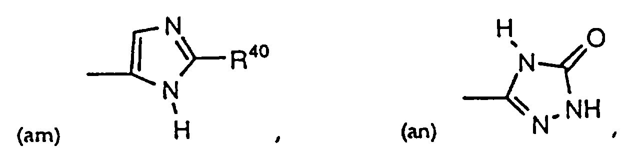 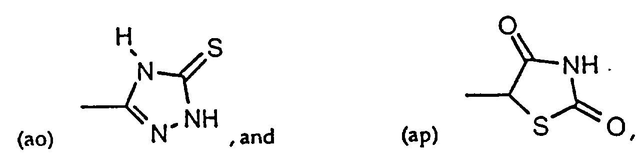
R⁸ est choisi dans le groupe consistant en
(a) un groupe hydroxy,
(b) un groupe alkoxy en C₁ à C₈,
(c) un groupe alcénoxy en C₃ à C₁₂,
(d) un groupe aryloxy en C₆ à C₁₂,
(e) un groupe (alkyle en C₁ à C₆) -(aryloxy en C₆ à C₁₂),
(f) un groupe di-(alkyle en C₁ à C₈)-amino-(alkoxy en C₁ à C₈),
(g) un groupe acylamino-(alkoxy en C₁ à C₈) choisi dans le groupe consistant en
(i) un groupe acétylaminoéthoxy,
(ii) un groupe nicotinoylaminoéthoxy, et
(iii) un groupe succinamidoéthoxy,
(h) un groupe (alkoxyle en C₁ à C₈)-oxy-(alkoxy en C₁ à C₈),
(i) un groupe (aryle en C₆ à C₁₂)-(alkoxy en C₁ à C₈),
dans lesquels n'importe quels groupes aryle sont non substitués ou substitués avec un à trois des groupes
(i) nitro,
(ii) halogéno (F, Cl, Br, I),
(iii) alkoxy en C₁ à C₄, et
(iv) amino,
(j) un groupe hydroxy-alkoxy en C₂ à C₈,
(k) un groupe dihydroxy-alkoxy en C₃ à C₈, et
(l) un groupe NR⁹R³⁰ ;
R⁹ et R³⁰ sont choisis, indépendamment, dans le groupe consistant en
(a) l'hydrogène,
(b) un groupe alkyle en C₁ à C₈,
(c) un groupe alcényle en C₃ à C₈,
(d) un groupe aryle en C₆ à C₁₂,
(e) un groupe (aryle en C₆ à C₁₂)-(alkyle en C₁ à C₈),
dans lesquels n'importe quels groupes aryle sont non substitués ou substitués avec un à trois des groupes
(i) nitro,
(ii) halogéno (F, Cl, Br, I),
(iii) alkoxy en C₁ à C₄, et
(iv) amino
R³¹ est choisi dans le groupe consistant en
(a) l'hydrogène,
(b) un groupe alkyle en C₁ à C₆,
(c) un groupe halogéno (F, Cl, Br, I)-alkyle en C₁ à C₆,
(d) un groupe phényle,
(e) un groupe benzyle, et
(f) un groupe -CH₂-O-COCH₃ ;
R³ est choisi dans le groupe consistant en
(a) l'hydrogène,
(b) un groupe benzyle, et
(c) un groupe -CH(R³⁵)-O-C(O)R³⁵ ;
R³³ est choisi dans le groupe consistant en
(a) un groupe aryle,
(b) un groupe hétéro-aryle,
(c) un groupe cycloalkyle en C₃ à C₇,
(d) un groupe alkyle en C₁ à C₄, non substitué ou substitué avec un substituant choisi dans le groupe consistant en
(i) un groupe aryle,
(ii) un groupe hétéro-aryle,
(iii) un groupe -OH,
(iv) un groupe -SH,
(v) un groupe alkyle en C₁ à C₄,
(vi) un groupe alkoxy en C₁ à C₄,
(vii) un groupe alkylthio en C₁ à C₄,
(viii) un groupe CF₃,
(ix) un groupe halogéno (F, Cl, Br, I),
(x) un groupe -NO₂,
(xi) un groupe -CO₂H,
(xii) un groupe CO₂-(alkyle en C₁ à C₄),
(xiii) un groupe -NH₂,
(xiv) un groupe -N-(alkyle en C₁ à C₄)₂,
(xv) un groupe -NH-(alkyle en C₁ à C₄),
(xvi) un groupe -PO₃H, et
(xvii) un groupe PO(OH) (alkoxy en C₁ à C₄), et
(e) un groupe perfluoralkyle en C₁ à C₄ ;
R³⁴ est choisi dans le groupe consistant en
(a) un groupe -CN,
(b) un groupe -NO₂,
(c) un groupe -COOR³¹,
(d) un groupe perfluoralkyle en C₁ à C₆,
(e) un groupe CF₃ ;
R³⁵ est choisi, indépendamment, dans le groupe consistant en
(a) l'hydrogène,
(b) un groupe alkyle en C₁ à C₆ facultativement substitué,
(c) un groupe alcényle en C₂ à C₆ facultativement substitué,
(d) un groupe alcynyle en C₂ à C₆ facultativement substitué, et
(e) un groupe cycloalkyle en C₃ à C₈ facultativement substitué,
dans lesquels les substituants sont choisis entre des groupes
(i) OH,
(ii) alkoxy en C₁ à C₄,
(iii) CO₂R³³,
(iv) OCOR³³,
(v) CONHR³³,
(vi) CON(R³³)₂,
(vii) N(R³³)C(O)R³³,
(viii) NH₂,
(ix) alkylamino en C₁ à C₄,
(x) di-(alkyle en C₁ à C₄)amino,
(xi) aryle, et
(xii) hétéro-aryle,
(f) un groupe -C(O)-aryle,
(g) un groupe -NO₂,
(h) un groupe halogéno (Cl, Br, I, F),
(i) un groupe -OH,
(j) un groupe -OR³⁶,
(k) un groupe perfluoralkyle en C₁ à C₄,
(l) un groupe -SH,
(m) un groupe -S(O)₁₋₂-alkyle en C₁ à C₄,
(n) un groupe -CO₂R³³,
(o) un groupe -SO₃H,
(p) un groupe -NR³³R³⁶,
(q) un groupe -NR³³C(O)R³⁶,
(r) un groupe NR³³COOR³,
(s) un groupe -SO₂NHR³,
(t) un groupe -SO₂NR³³R³³,
(u) un groupe -NHSO₂R³,
(v) un groupe -C(O)NHSO₂R³,
(w) un groupe aryle,
(x) un hétérocycle,
(y) un groupe morpholine-4-yle,
(z) un groupe CONH₂, et
(aa) un groupe 1H-tétrazole-5-yle ;
R³⁶ est choisi dans le groupe consistant en
(a) l'hydrogène, et
(b) un groupe alkyle en C₁ à C₄ non substitué ou substitué avec un des groupes consistant en
(i) NH₂,
(ii) NH-(alkyle en C₁ à C₄),
(iii) N-(alkyle en C₁ à C₄)₂,
(iv) CO₂H,
(v) CO₂(alkyle en C₁ à C₄),
(vi) OH,
(vii) SO₃H, et
(viii) SO₂NH₂ ;
R³⁷ est choisi dans le groupe consistant en
(a) l'hydrogène,
(b) un groupe alkyle en C₁ à C₆,
(c) un groupe alcényle en C₂ à C₆,
(d) un groupe (alkoxy en C₁ à C₆)alkyle,
(e) un groupe -CH₂-O-COCH₃, et
(f) un groupe -CH₂-phényle, dans lequel le groupe phényle est non substitué ou substitué avec un substituant choisi entre les groupes
(i) -NO₂,
(ii) -NH₂,
(iii) -OH, et
(iv) -OCH₃ ;
R³⁸, R³⁹ et R⁴⁰ sont choisis chacun, indépendamment, entre
(a) l'hydrogène,
(b) un groupe Cl,
(c) un groupe CN,
(d) un groupe NO₂,
(e) un groupe CF₃,
(f) un groupe C₂F₅,
(g) un groupe C₃F₇,
(h) un groupe CHF₂,
(i) un groupe CH₂F,
(j) un groupe CO₂CH₃,
(k) un groupe CO₂C₂H₅,
(l) un groupe SO₂CH₃,
(m) un groupe SO₂CF₃, et
(n) SO₂C₆F₅,
Z étant choisi entre O, S, un groupe NR⁴¹ et CH₂ ;
R⁴¹ est choisi entre l'hydrogène, un groupe CH₃ et un groupe CH₂C₆H₅ ; et
ses sels pharmaceutiquement acceptables.

2. Composé suivant la revendication 1, représenté par la formule II : 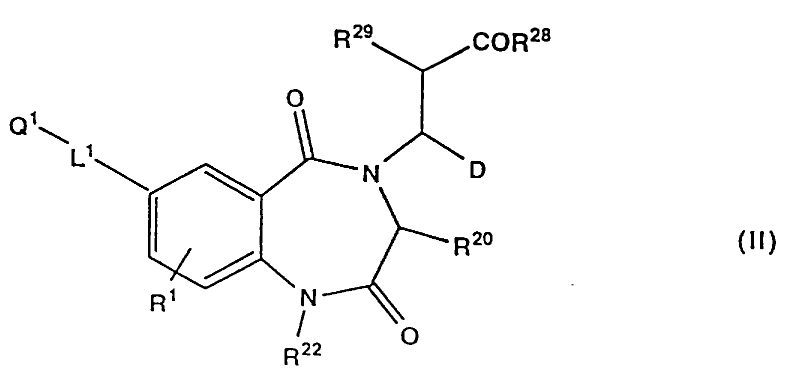
dans laquelle
R¹ représente un à trois groupes choisis, indépendamment, entre l'hydrogène, les groupes halogéno (F, Cl, Br, I), cyano, carboxamido, carbamoyloxy, formyloxy, formyle, azido, nitro, uréido, thiouréido, hydroxy, mercapto, sulfonamido, et un radical facultativement substitué choisi entre des radicaux alkyle en C₁ à C₁₂, alcényle en C₂ à C₁₂, alcynyle en C₃ à C₁₂, cycloalkyle en C₃ à C₁₂, aryle en C₆ à C₁₄, (aryle en C₆ à C₁₀)-(alkyle en C₁ à C₈), alkyloxy en C₁ à C_{12,} aryloxy en C₆ à C₁₄ et alcylamino en C₁ à C₁₂, dans lesquels les substituants sont choisis entre des substituants halogéno (F, Cl, Br, I), cyano, azido, nitro, hydroxy, mercapto, sulfonamido, uréido, thiouréido, carboxamido, carbamoyloxy, formyloxy, formyle, alkyle en C₁ à C₄, alkoxy en C₁ à C₄, phényle et phénoxy ;
Q¹ est choisi dans le groupe consistant en
(A) un groupe amino choisi entre des groupes
-NH₂,
-NR³H,
-NR³R⁴, et
-NR³R⁴R⁵,
dans lesquels R³, R⁴ et R⁵ sont choisis, indépendamment, entre (i) un radical facultativement substitué choisi entre (a) -NR⁶R⁷, (b) -C(=NR⁸)-NR⁶R⁷, (c) -N=CR⁹-NR⁶R⁷, (d) -NR¹⁰-CR⁹=NR⁸, et (e) -NR¹⁰-C(=NR⁸) -NR⁶R⁷, dans lesquels chacun des groupes R⁶, R⁷, R⁸, R⁹ et R¹⁰ est choisi, indépendamment, entre l'hydrogène, des groupes alkoxy en C₁ à C₄, alkyle en C₁ à C₄, et halogéno (F, Cl, Br, I)-alkyle en C₁ à C₄, (ii) un groupe alkyle en C₁ à C₁₂, facultativement substitué, (iii) un groupe cycloalkyle en C₃ à C₁₂ facultativement substitué, (iv) un groupe aryle en C₆ à C₁₄ facultativement substitué, (v) un groupe (alkyle en C₁ à C₆) - (aryle en C₆ à C₁₄) facultativement substitué, (vi) un groupe alkoxy en C₁ à C₈ facultativement substitué, et (vii) un groupe aryloxy en C₆ à C₁₄ facultativement substitué, dans lesquels les substituants consistent en un à trois groupes Rll, chaque groupe R¹¹ étant choisi indépendamment entre (a) un groupe aryloxy en C₆ à C₁₂ facultativement substitué, (b) un groupe arylamino en C₆ à C₁₂ facultativement substitué, (c) un groupe aroyle en C₆ à C₁₂ facultativement substitué, dans lesquels les substituants consistent en un à trois groupes R¹, chaque groupe R¹ étant choisi, indépendamment, entre des groupes nitro, amino, alkylamino en C₁ à C₈, di-(alkyle en C₁ à C₈)amino, amidino, aminométhylèneimino, imnio, imino-alkyle en C₁ à C₄, iminométhylèneamino, guanidino, arylamino en C₆ à C₁₀, acylamino en C₁ à C₈, alkylsulfonamido en C₁ à C₄, azido, cyano, hydroxy, hydroxyalkyle en C₁ à C₈, alkoxy en C₁ à C₈, phényloxy, alcanoyloxy en C₁ à C₈, alcanoyle en C₁ à C₈, aroyle en C₆ à C₁₂, benzamido, phényle, halogéno (F, Cl, Br, I), halogénalkyle en C₁ à C₈ et alkyle en C₁ à C₈, (d) un groupe halogéno (F, cl, Br, I), (e) un groupe hydroxy, (f) un groupe mercapto, (g) un groupe formyle, (h)) un groupe formyloxy, (i) un groupe carboxy, (j) un groupe amino, (k) un groupe uréido, (l) un groupe amidino, (m) un groupe guanidino, (n) un groupe aminométhyléneimino, (o) un groupe imimno, (p) un groupe glycyle, (q) un groupe phtalimido, (r) un groupe succinimido, (s) un groupe morpholino et (t) un groupe cycloalkyle en C₃ à C₇, facultativement, R³ et R⁴, pris conjointement, peuvent former un groupe tétraméthylène, pentaméthylène, 3-oxopentaméhtylène ou 3-azapentaméthylène facultativement substitué, dans lequel les substituants consistent en un à trois groupes R¹,
(B) un groupe amidino choisi entre
-C (=NH) -NH₂
-C (=NH) -NHR³,
-C (=NR⁴) -NHR³,
-C (=NH) -NR³R⁴, et
-C (=NR⁵) -NR³R⁴,
dans lesquels R³, R⁴ et R⁵ répondent aux définitions précitées,
(C) un groupe aminoalkylèneamino choisi entre les groupes
-N=CH-NH₂,
-N=CH-NHR³,
-N=CH-NR³R⁴, et
-N=CR⁵-NR³R⁴, et
-N-CR⁵-NR³R⁴,
dans lesquels R³, R⁴ et R⁵ répondent aux définitions précitées,
(D) un groupe iminoalkylèneamino choisi entre les groupes
-NH-CH=NH,
-NH-CH=NR³,
-NH-CR⁴=NR³, et
-NR⁵-CR⁴=NR³_{,}
dans lesquels R³, R⁴ et R⁵ répondent aux définitions précitées,
(E) un groupe guanidino choisi entre les groupes
-NH-C(=NH)-NH₂,
-NH-C(=NH)-NR³H,
-NH-C(=NH)-NR³R⁴,
-NH-C(=NR⁵)-NR³R⁴,
-NR³-C(=NR³)-NR³R⁴,
-NR³-C(=NH)-NR³R⁴,
-NR³-C(=NR³)-NH₂,
-NR³-C(=NH)-NH₂,
-NR³-C(=NR³)-NHR⁴, et
-NR³-C(=NH)-NHR⁴,
dans lesquels R³, R⁴ et R⁵ répondent aux définitions précitées,
(F) un groupe hétérocyclique saturé facultativement substitué choisi entre des groupes 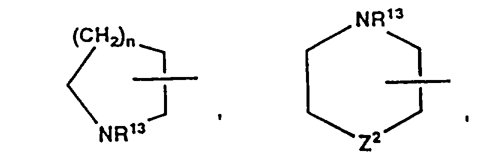 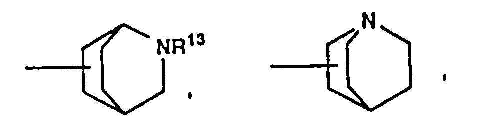 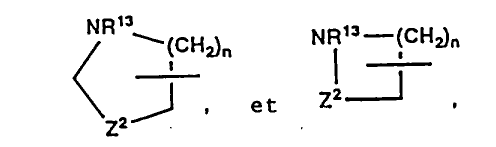
dans lesquels (1) n est égal à 0, 1, 2 ou 3, (2) R¹³ est choisi entre les groupes R⁶, -CR⁹=NR⁸, -CR⁹(=NR⁸) -NR⁶R⁷, -C(=NR⁸)-NR⁶R⁷, -N=CR⁹-NR⁶R⁷, -NR¹⁰-CR⁹=NR⁸ et -NR¹⁰-(C=NR⁸)-NR⁶R⁷ dans lesquels R⁶ et R¹⁰ répondent aux définitions précitées, (3) Z représente O, S ou un groupe NR¹³, et (4) les substituants consistent indépendamment en un à trois groupes R¹ ;
L¹ représente un radical bivalent facultativement substitué choisi dans des groupes alkylène en C₃ à C₇, cycloalkylène en C₃ à C₇, alcynylène en C₃ à C₇, cycloalcénylène en C₄ à C₇, cycloalcadiénylène en C₅ à C₈, alcadiénylène en C₃ à C₇, alcynylène en C₃ à C₇, alcénylène en C₄ à C₇, arylène en C₆ à C₁₄, (aryle en C₆ à C₁₄)-alcynylène en C₂ à C₄, (alkyle en C₁ à C₃)-(aryle en C₆ à C₁₄)-(alcynylène en C₂ à C₄), (aryle en C₆ à C₁₄)-(alcénylène en C₂ à C₄), (alkyle en C₁ à C₃)-(arylène en C₆ à C₁₄), (alkyle en C₁ à C₃)-(aryle en C₆ à C₁₄)-(alcénylène en C₂ à C₄), (aryle en C₆ à C₁₄)-(alkylène en C₁ à C₃), (aryle en C₆ à C₁₄)-(alkyloxyène en C₁ à C₃), (alkyle en C₁ ou C₂)-(aryle en C₆ à C₁₄)-alkylène en C₁ ou C₂), (alkyle en C₁ à C₃)-oxy-(arylène en C₆ à C₁₄), alkyloxyène en C₂ à C₈, (alkyle en C₁ à C₅)-oxy-(alkylène en C₁ à C₅), aryloxyène en C₆ à C₁₀, aryle (en C₆ à C₁₀)-oxy-(alkylène en C₁ à C₅), (aryle en C₆ à C₁₀)-thio-(alkylène en C₁ à C₅), 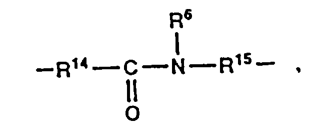 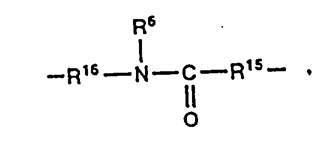 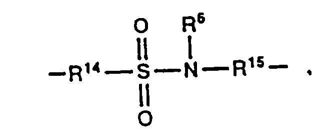 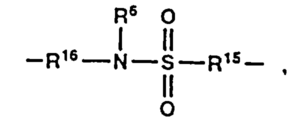 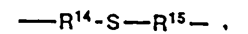 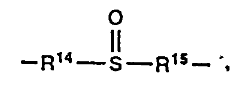 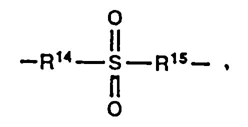 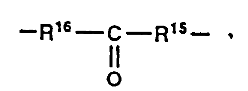 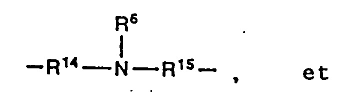 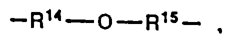
dans lesquels R¹⁴ est choisi entre une liaison chimique, des groupes alkyle en C₁ à C₈, cycloalkyle en C₃ à C₇, alcényle en C₂ à C₅, alcynyle en C₃ à C₅, aryle en C₆ à C₁₀, (alkyle en C₁ à C₃)-(aryle en C₆ à C₁₂), (alkyle en C₁ ou C₂)-(aryle en C₆ à C₁₀)-(alkyle en C₁ ou C₂), et (aryle en C₆ à C₁₀)-(alkyle en C₁ ou C₂), R¹⁵ est choisi entre une liaison chimique, des groupes alkyle en C₁ à C₄, alcényle en C₂ à C₄, alcynyle en C₂ à C₄ et aryle en C₆ à C₁₀, R¹⁶ est choisi entre une liaison chimique, des groupes alkyle en C₁ à C₅, cycloalkyle en C₃ à C₇, alcényle en C₃ à C₅, alcynyle en C₃ à C₅, aryle en C₆ à C₁₀, (alkyle en C₁ à C₃)-(aryle en C₆ à C₁₂), (alkyle en C₁ à C₃)-(aryle en C₆ à C₁₂) et (aryle en C₆ à C₁₀)-(alkyle en C₁ ou C₂), dans lesquels les substituants consistent en un à trois groupes R¹ ;
R⁰ est choisi dans le groupe consistant en l'hydrogène, des groupes halogéno (F, Cl, Br, I), alkoxy en C₁ à C₄, alkyle en C₁ à C₄, phényle, benzyle et halogéno (F, cl, Br, I)-alkyle en C₁ à C₄ ;
R est choisi dans le groupe consistant en (i) l'hydrogène, (ii) un groupe alkyle en C₁ à C₁₂ facultativement substitué, (iii) un groupe aryle en C₆ à C₁₄ facultativement substitué, (iv) un groupe cycloalkyle en C₃ à C₁₄ facultativement substitué, (v) un groupe (alkyle en C₁ à C₁₂)-(aryle en C₆ à C₁₄) facultativement substitué, (vi) un groupe (alkyle en C₁ à C₁₂)-(cycloalkyle en C₃ à C₁₄) facultativement substitué, dans lesquels les substituants sont choisis entre des groupes (a) halogéno (F, Cl, Br, I), (b) nitro, (c) hydroxy, (d) carboxy, (e) tétrazole, (f) hydroxamate, (g) sulfonamide, (h) trifluorimide, (i) phosphonate, (j) alkyle en C₁ à C₆, (k) aryle en C₆ à C₁₄, (l) benzyle, (m) cycloalkyle en C₃ à C₁₄, (n) COR⁴ dans lequel R⁴ est choisi entre des groupes alkoxy en C₁ à C₈, alcénoxy en C₃ à C₁₂, aryloxy en C₆ à C₁₂, di-(alkyle en C₁ à C₈)-amino-(alkoxy en C₁ à C₈), acylamino-(alkoxy en C₁ à C₈) choisi entre les groupes acétylaminoéthoxy, nicotinoylaminoéthoxy, succinamidoéthoxy et pivaloyloxyéthoxy, et (aryle en C₆ à C₁₂) -(alkoxy en C₁ à C₈) dans lequel le groupe aryle est non substitué ou substitué avec un à trois des groupes nitro, halogéno (F, Cl, Br, I), alkoxy en C₁ à C₄, amino, hydroxy, hydroxyalkoxy en C₂ à C₈ et dihydroxyalkoxy en C₃ à C₈, (o) CONR⁵R⁶ dans lequel R⁵ et R⁶ sont choisis, indépendamment, entre l'hydrogène, des groupes alkyle en C₁ à C₁₀, alcényle en C₃ à C₁₀, aryle en C₆ à C₁₄, (alkyle en C₁ à C₆)-(aryle en C₆ à C₁₀), facultativement R⁵ et R⁶, pris conjointement, peuvent former un groupe triméthylène, tétraméthylène, pentaméthylène ou 3-oxopentaméthylène, et (vii) un groupe Q-L³ dans lequel Q est choisi entre l'hydrogène et Q¹, et L³ est choisi entre une liaison chimique, L¹ et L, L étant un radical divalent facultativement substitué choisi entre les groupes 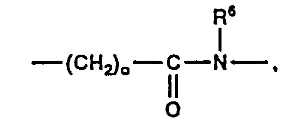 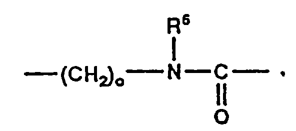 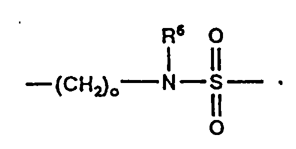 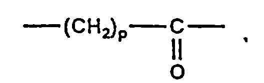 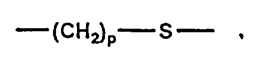 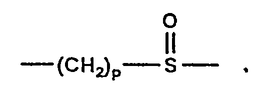 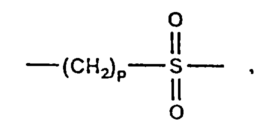 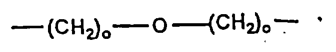 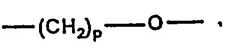 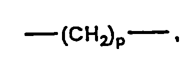 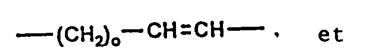 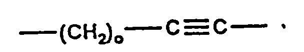
dans lesquels o est égal à 1 ou 2, p est égal à 1, 2, 3 ou 4, R⁶ répond à la définition précitée, les substituants consistant en un à trois groupes R¹ ;
D est choisi entre les groupes R et -(C=O)-Xaa, dans lequel Xaa représente un à trois D- ou L-α-amino-acides
R⁸ est choisi dans le groupe consistant en (i) un groupe hydroxy, (ii) un groupe alkoxy en C₁ à C₈, (iii) un groupe alcénoxy en C₃ à C₁₂, (iv) un groupe aryloxy en C₆ à C₁₂, (v) un groupe (alkyle en C₁ à C₆)-(aryle en C₆ à C₁₂)-oxy, (vi) un groupe di-(alkyle en C₁ à C₈)-amino-(alkoxy en C₁ à C₈), (vii) un groupe acylamino(alkoxy en C₁ à C₈) choisi entre les groupes (a) acétylaminoéthoxy, (b) nicotinyloaminoéthoxy et (c) succinamidoéthoxy, (viii) un groupe (alcoyle en C₁ à C₈)-oxy-(alkoxy en C₁ à C₈), (ix) un groupe (aryle en C₆ à C₁₂)-(alkoxy en C₁ à C₈), dans lequel le groupe aryle est non substitué ou substitué avec un à trois des groupes (a) nitro, (b) halogéno (F, C1, Br, I), (c) alkoxy en C₁ à C₄ et (d) amino, (x) un groupe hydroxy-alkoxy en C₂ à C₈, (xi) un groupe dihydroxy-alkoxy en C₃ à C₈, et (xii) un groupe NR⁹R³⁰ dans lequel R⁹ et R³⁰ sont choisis, indépendamment, dans le groupe consistant en (a) l'hydrogène, (b) un groupe alkyle en C₁ à C₈, (c) un groupe alcényle en C₃ à C₈, (d) un groupe aryle en C₆ à C₁₂, le groupe aryle étant non substitué ou substitué avec un à trois des groupes nitro, halogéno (F, Cl, Br, I), alkoxy en C₁ à C₄, et amino, et (e) un groupe (aryle en C₆ à C₁₂)-(alkyle en C₁ à C₈) dans lequel le groupe aryle est non substitué ou substitué avec un à trois des groupes nitro, halogéno (F, Cl, Br, I) et alkoxy en C₁ à C₄ ;
R⁹ représente un ou deux groupes choisis, indépendamment, dans le groupe consistant en (a) l'hydrogène, (b) un groupe alkyle en C₁ à C₈, (c) un groupe alcényle en C₃ à C₈, (d) un groupe aryle en C₆ à C₁₂, et (e) un groupe (aryle en C₆ à C₁₂)-(alkyle en C₁ à C₈), dans lesquels n'importe quels groupes aryle sont non substitués ou substitués avec un à trois des groupes (i) nitro, (ii) halogéno (F, Cl, Br, I), (iii) alkoxy en C₁ à C₄, et (iv) amino ; et
ses sels pharmaceutiquement acceptables.

3. Composé suivant la revendication 2, dans lequel L¹ est choisi dans le groupe consistant en les radicaux 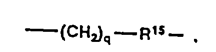 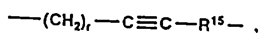 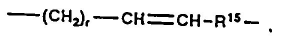 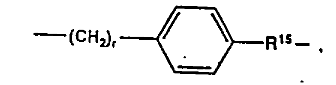 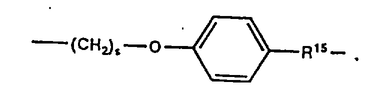 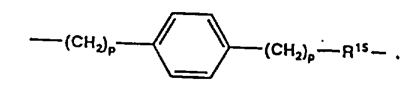 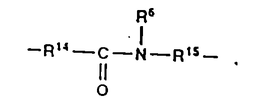 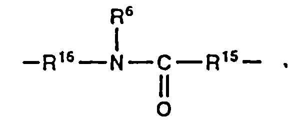 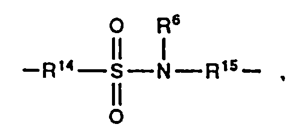 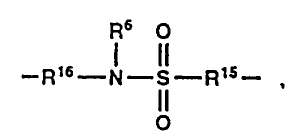 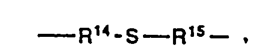 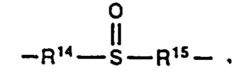 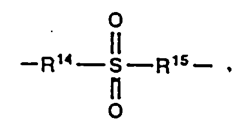 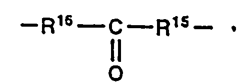 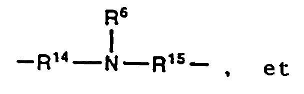 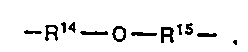
dans lesquels
p est égal à 1, 2, 3 ou 4 ;
q est égal à 3, 4, 5, 6 ou 7 ;
r est égal à 1, 2, 3, 4 ou 5 ;
s est égal à 2 ou 3 ;
R¹⁴ est choisi entre des groupes
alkyle en C₂ à C₅,
cycloalkyle en C₃ à C₇,
alcényle en C₂ à C₅,
alcynyle en C₃ à C₅,
aryle en C₆ à C₁₀,
(alkyle en C₁ à C₃)-(aryle en C₆ à C₁₂),
(alkyle en C₁ ou C₂)-(aryle en C₆ à C₁₀)-(alkyle en C₁ ou C₂),
(aryle en C₆ à C₁₀)-(alkyle en C₁ ou C₂), et
(aryle en C₆ à C₁₀)-oxy-(alkyle en C₁ ou C₂) ;
R¹⁵ représente une liaison chimique connectant L¹ à la position 7 de la benzodiazépinedione ;
R¹⁶ est choisi entre des groupes
alkyle en C₂ à C₅,
cycloalkyle en C₃ à C₇,
alcényle en C₃ à C₅,
alcynyle en C₃ à C₅,
aryle en C₆ à C₁₀,
(alkyle en C₁ à C₃)-(aryle en C₆ à C₁₂), et
(aryle en C₆ à C₁₀)-(alkyle en C₁ ou C₂)
les groupes R¹⁴ et R¹⁶ liant le groupe L¹ à Q¹.

4. Composé suivant la revendication 3, dans lequel R est choisi dans le groupe consistant en
l'hydrogène,
un groupe alkyle en C₁ à C₆ facultativement substitué,
dans lequel les substituants sont choisis entre des groupes
amino,
hydroxy,
halogéno (F, Cl, Br, I),
carboxy, et
(alkoxy en C₁ à C₄)-carbonyle,
un groupe aryle en C₆ à C₁₀ facultativement substitué, et
un groupe (aryle en C₆ à C₁₂)-(alkyle en C₁ à C₄) facultativement substitué
dans lequel les substituants sur n'importe quels groupes aryle sont choisis entre des groupes
amino,
nitro,
halogéno (F, Cl, Br, I),
halogéno (F, Cl, Br, I)-alkyle en C₁ à C₆,
alkoxy en C₁ à C₆,
aryloxy en C₆ à C₁₀,
amino-acylamino en C₁ à C₆,
amino-acyle en C₁ à C₆, et
guanidino-aroylamino en C₆ à C₁₀.

5. Composé suivant la revendication 3, dans lequel Q¹-L¹ est choisi dans le groupe consistant en les radicaux 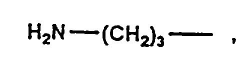 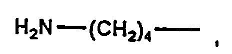 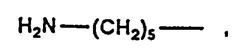 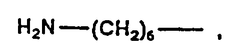 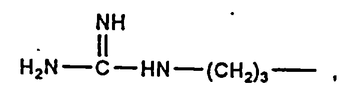 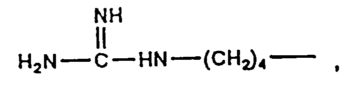 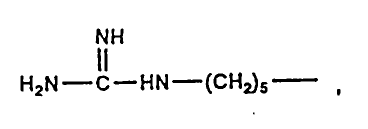 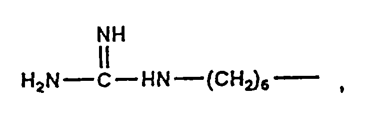 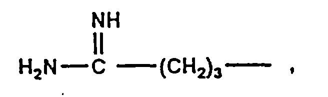 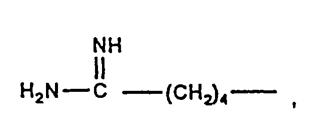 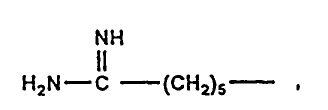 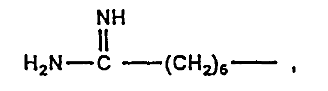 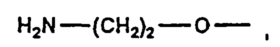 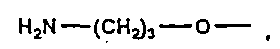 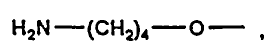 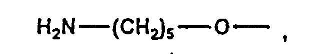 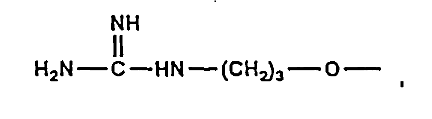 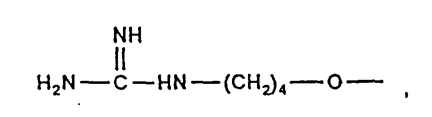 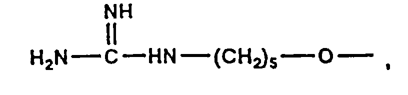 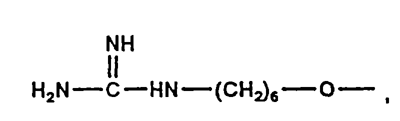 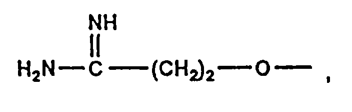 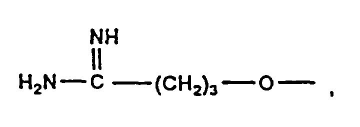 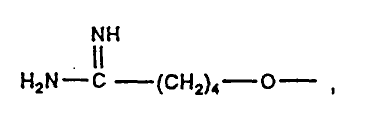 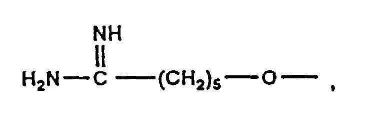 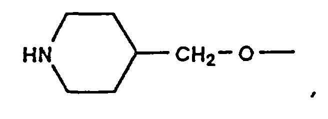 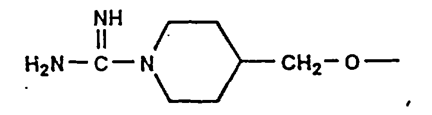 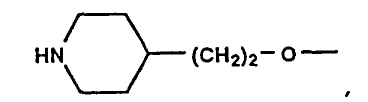 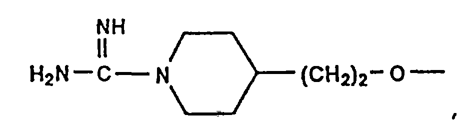 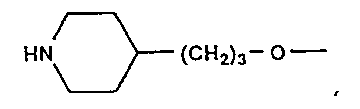 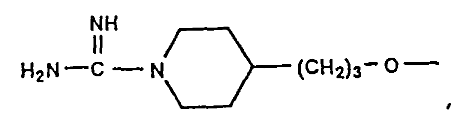 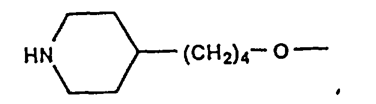 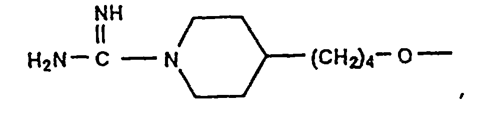 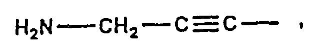 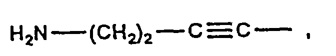 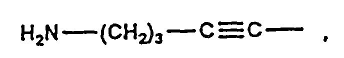 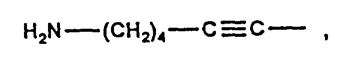 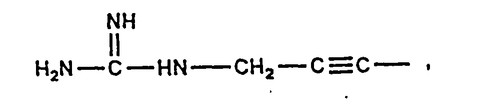 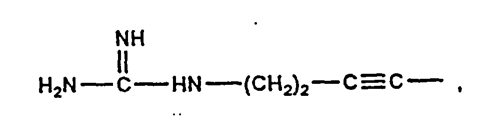 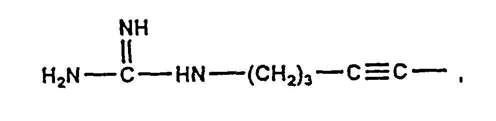 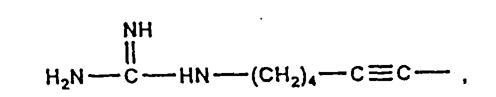 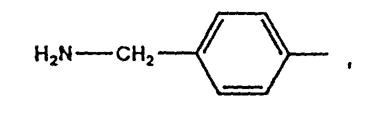 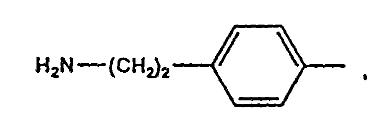 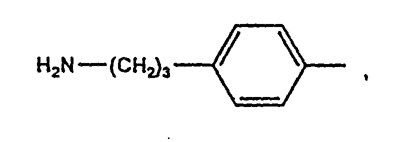 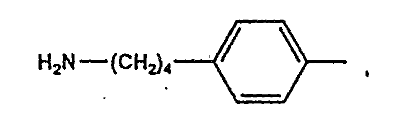 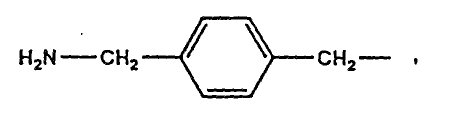 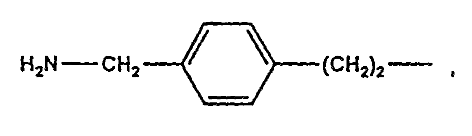 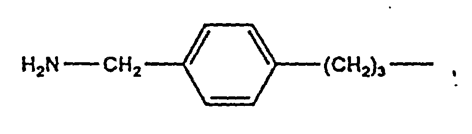 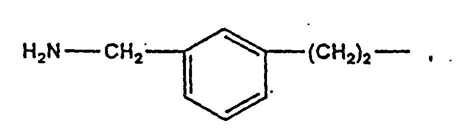 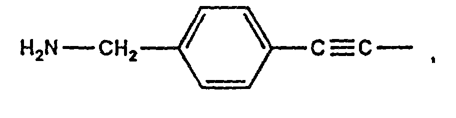 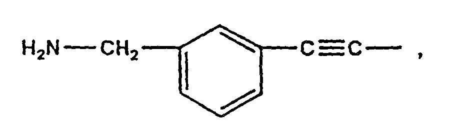 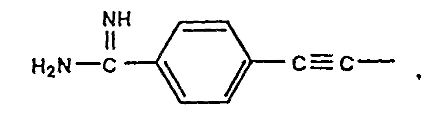 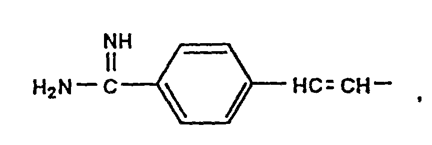 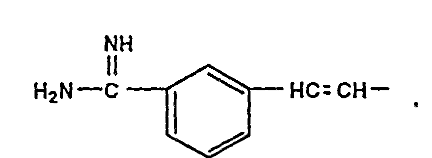 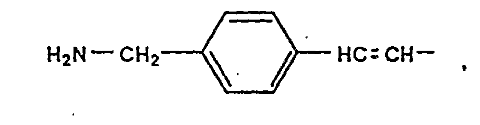 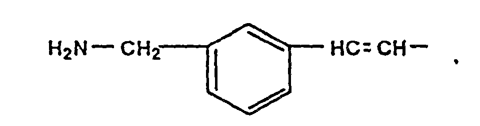 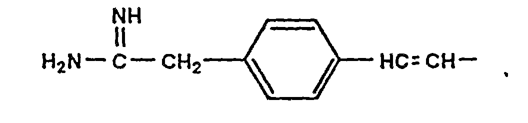 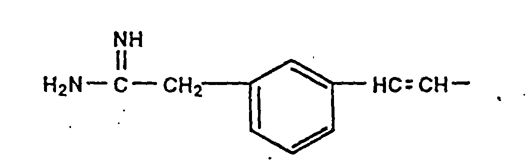 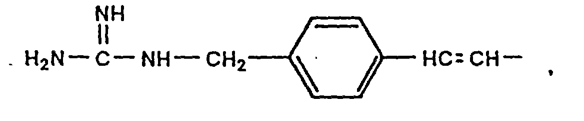 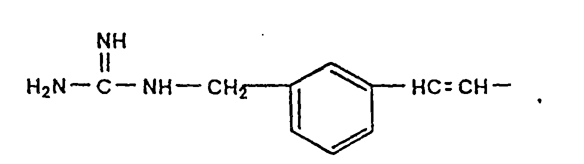 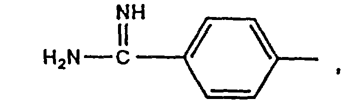 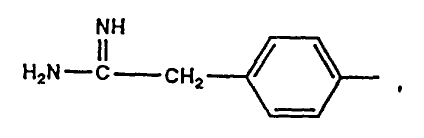 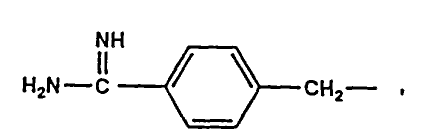 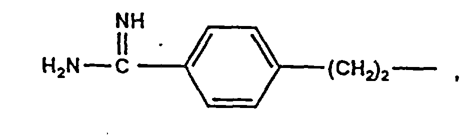 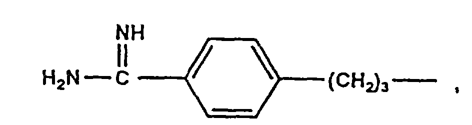 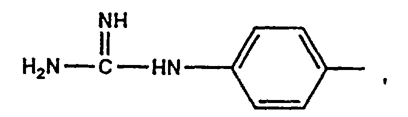 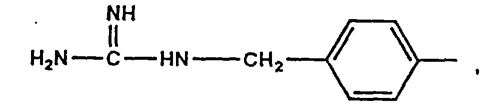 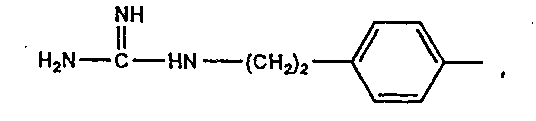 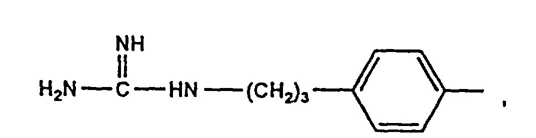 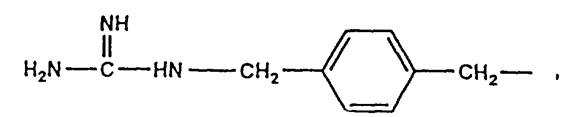 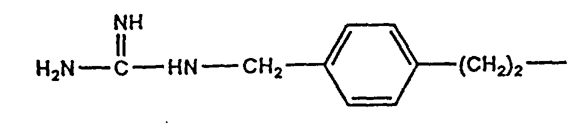 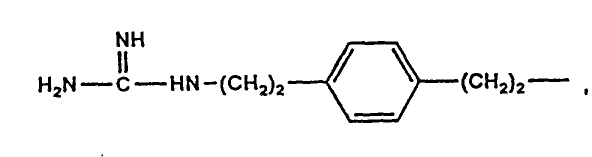 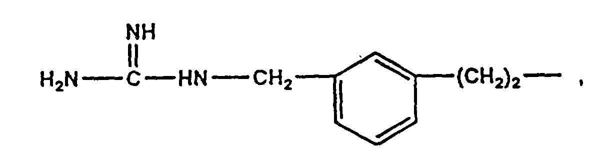 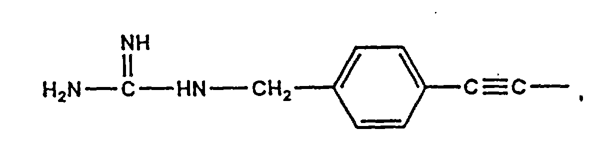 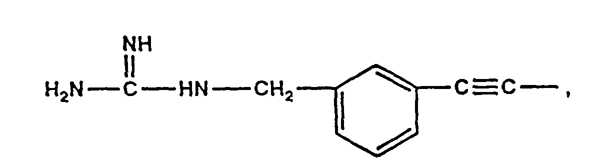 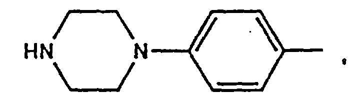 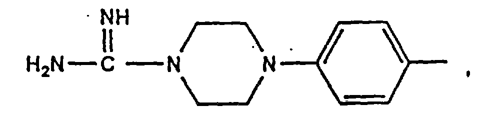 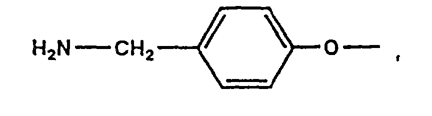 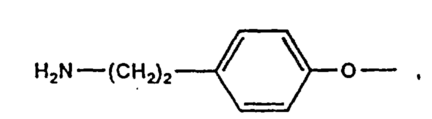 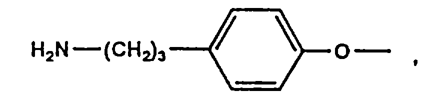 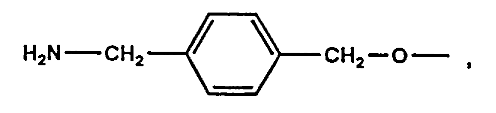 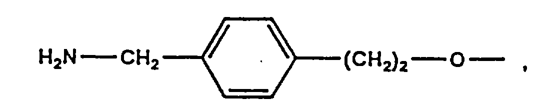 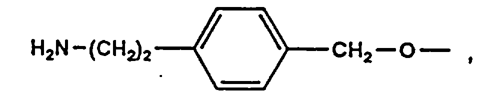 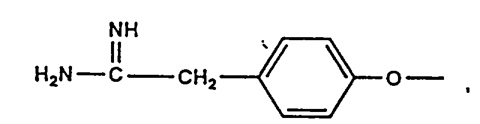 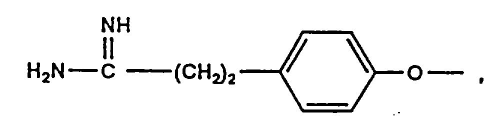 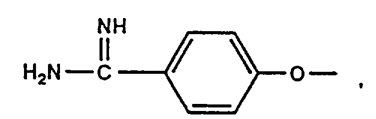 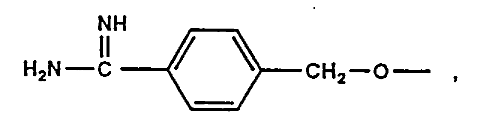 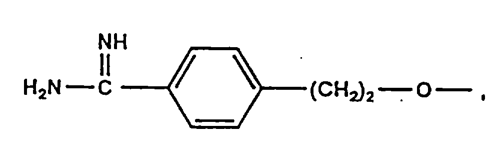 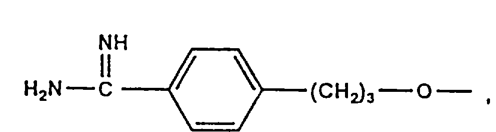 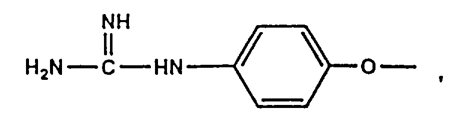 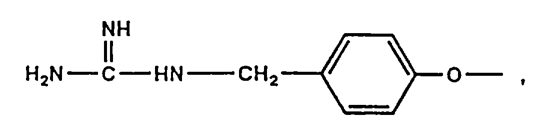 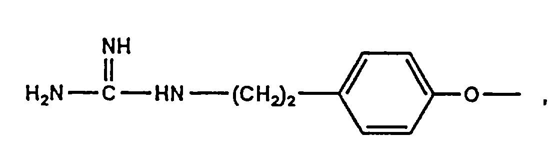 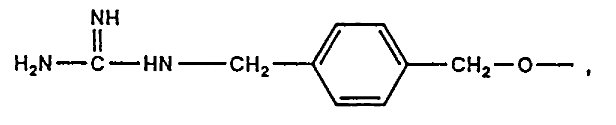 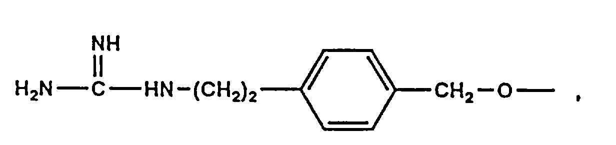 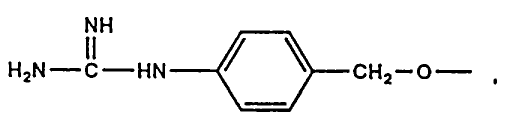 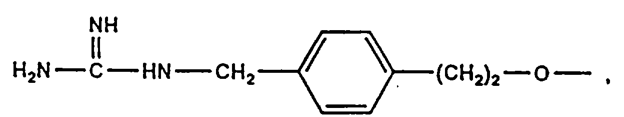 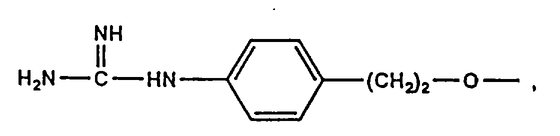 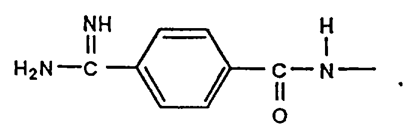 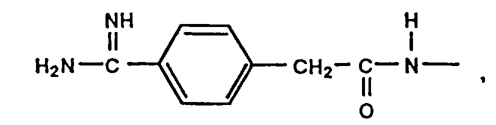 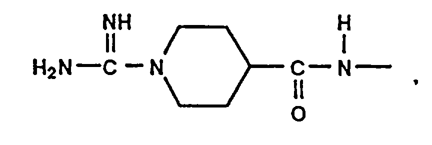 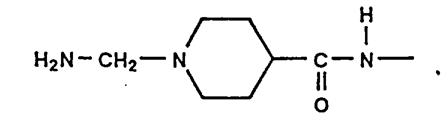 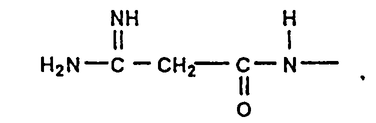 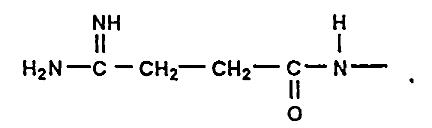 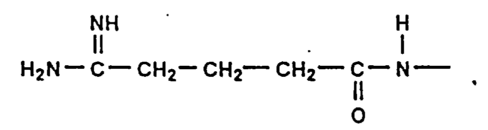 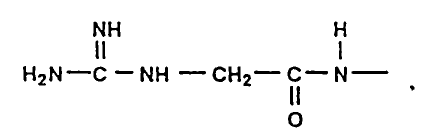 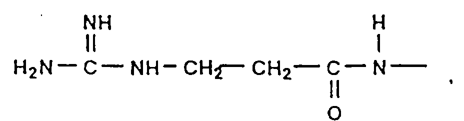 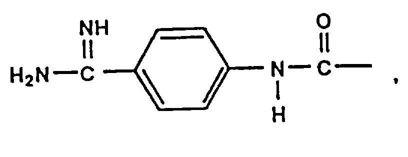 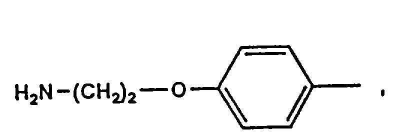 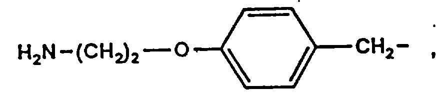 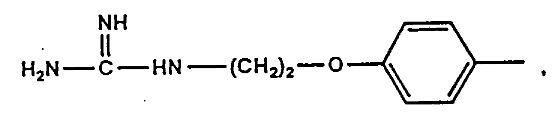 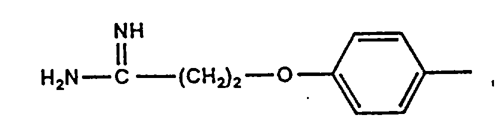 and

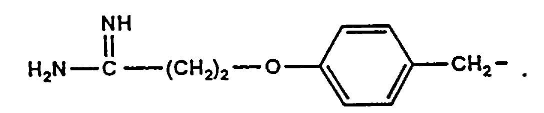

6. Composé suivant la revendication 2, choisi dans le groupe consistant en :
1-(4-chlorophényl)méthyl-4-(2-carboxyéthyl)-7-(4-(1-pipérizine)-phényl)-3,4-dihydro-1H-1,4-benzodiazapine-2,5-dione
1-(diphényl)méthyl-4-(2-carboxyéthyl)-7-(4-(1-pipérizine)-phényl)-3,4-dihydro-1H-1,4-benzodiazapine-2,5-dione,
1-(m-trifluorométhyl)phényl-4-(2-carboxyéthyl)-7-(4-(1-pipérizine)-phényl)-3,4-dihydro-1H-1,4-benzodiazapine-2,5-dione,
1-(méthyl-4-(2-carboxyéthyl)-7-(4-(1-pipérizine)-phényl)-3,4-dihydro-1H-1,4-benzodiazapine-2,5-dione,
1-(1-méthyl)éthyl-4-(2-carboxyéthyl)-7-(4-(1-pipérizine)-phényl)-3,4-dihydro-1H-1,4-benzodiazapine-2,5-dione,
1-(2-méthyl)propyl-4-(2-carboxyéthyl)-7-(4-(1-pipérizine)-phényl)-3,4-dihydro-1H-1,4-benzodiazapine-2,5-dione,
1-(4-chlorophényl)méthyl-4-(2-carboxyéthyl)-7-(4-(2-aminoéthoxy)-phényl)-3,4-dihydro-1H-1,4-benzodiazapine-2,5-dione,
1-(diphényl)méthyl-4-(2-carboxyéthyl)-7-(4-(2-aminoéthoxy)-phényl)-3,4-dihydro-1H-1,4-benzodiazapine-2,5-dione,
1-(m-trifluorométhyl)phényl-4-(2-carboxyéthyl)-7-(4-(2-aminoéthoxy)-phényl)-3,4-dihydro-1H-1,4-benzodiazapine-2,5-dione,
1-méthyl-4-(2-carboxyéthyl)-7-(4-(2-aminoéthoxy)-phényl)-3,4-dihydro-1H-1,4-benzodiazapine-2,5-dione,
1-(1-méthyl)éthyl-4-(2-carboxyéthyl)-7-(4-(2-aminoéthoxy)-phényl)-3,4-dihydro-1H-1,4-benzodiazapine-2,5-dione,
le 1-(2-méthyl)propyl-4-(2-carboxyéthyl)-7-(4-(2-aminoéthoxy)-phényl)-3,4-dihydro-1H-1,4-benzodiazapine-2,5-dione,
1-(diphényl)méthyl-4-(2-carboxyéthyl)-7-(6-aminohexyl)-3,4-dihydro-1H-1,4-benzodiazapine-2,5-dione,
1-(diphényl)méthyl-4-(2-carboxyéthyl)-7-(6-aminohex-1-ynyl)-3,4-dihydro-1H-1,4-benzodiazapine-2,5-dione,
1-(diphényl)méthyl-4-(2-carboxyéthyl)-7-(5-aminopentoxy)-3,4-dihydro-1H-1,4-benzodiazapine-2,5-dione,
1-(diphényl)méthyl-4-(2-carboxyéthyl)-7-(5-guanidinopentyl)-3,4-dihydro-1H-1,4-benzodiazapine-2,5-dione,
1-(diphényl)méthyl-4-(2-carboxyéthyl)-7-(4-guanidinobutoxy)-3,4-dihydro-1H-1,4-benzodiazapine-2,5-dione,
1-(diphényl)méthyl-4-(2-carboxyéthyl)-7-(5-aminopent-1-ynyl)-3,4-dihydro-1H-1,4-benzodiazapine-2,5-dione,
1-(diphényl)méthyl-4-(2-carboxyéthyl)-7-(5-guanidinopent-1-ynyl)-3,4-dihydro-1H-1,4-benzodiazapine-2,5-dione,
1-(diphényl)méthyl-4-(2-carboxyéthyl)-7-(4-amidino)benzamido-3,4-dihydro-1H-1,4-benzodiazapine-2,5-dione,
1-(diphényl)méthyl-4-(2-carboxyéthyl)-7-(4-amidino)benzyloxy-3,4-dihydro-1H-1,4-benzodiazapine-2,5-dione,
1-(diphényl)méthyl-4-(2-carboxyéthyl)-7-[2-(4-amidinophényl)]éthényl-3,4-dihydro-1H-1,4-benzodiazapine-2,5-dione,
1-(diphényl)méthyl-4-(2-carboxyéthyl)-7-[2-(4-amidinophényl)]éthynyl-3,4-dihydro-1H-1,4-benzodiazapine-2,5-dione,
1-(4-chlorophényl)méthyl-4-(2-carboxyéthyl)-7-(6-aminohexyl)-3,4-dihydro-1H-1,4-benzodiazapine-2,5-dione,
1-(4-chlorophényl)méthyl-4-(2-carboxyéthyl)-7-(5-aminopentoxy)-3,4-dihydro-1H-1,4-benzodiazapine-2,5-dione,
1-(4-chlorophényl)méthyl-4-(2-carboxyéthyl)-7-(4-amidino)benzamido-3,4-dihydro-1H-1,4-benzodiazapine-2,5-dione,
1-(4-chlorophényl)méthyl-4-(2-carboxyéthyl)-7-(4-amidino)benzyloxy-3,4-dihydro-1H-1,4-benzodiazapine-2,5-dione,
1-(4-chlorophényl)méthyl-4-(2-carboxyéthyl)-7-[2-(4-amidinophényl)]éthényl-3,4-dihydro-1H-1,4-benzodiazapine-2,5-dione,
1-(4-chlorophényl)méthyl-4-(2-carboxyéthyl)-7-[2-(4-amidinophényl)]éthynyl-3,4-dihydro-1H-1,4-benzodiazapine-2,5-dione,
1-méthyl-4-(2-carboxyéthyl)-7-(4-amidino)-benzamido-3,4-dihydro-1H-1,4-benzodiazapine-2,5-dione,
1-méthyl-4-(2-carboxyéthyl)-7-(4-amidino)-benzyloxy-3,4-dihydro-1H-1,4-benzodiazapine-2,5-dione,
1-méthyl-4-(2-carboxyéthyl)-7-[2-(4-amidinophényl)]éthényl-3,4-dihydro-1H-1,4-benzodiazapine-2,5-dione,
1-méthyl-4-(2-carboxyéthyl)-7-[2-(4-amidinophényl)]éthynyl-3,4-dihydro-1H-1,4-benzodiazapine-2,5-dione,
1-(1-méthyl)éthyl-4-(2-carboxyéthyl)-7-(4-amidino)benzamido-3,4-dihydro-1H-1,4-benzodiazapine-2,5-dione,
1-(1-méthyl)éthyl-4-(2-carboxyéthyl)-7-(4-amidino)benzyloxy-3,4-dihydro-1H-1,4-benzodiazapine-2,5-dione,
1-(1-méthyl)éthyl-4-(2-carboxyéthyl)-7-[2-(4-amidinophényl)]éthényl-3,4-dihydro-1H-1,4-benzodiazapine-2,5-dione,
1-(2-méthyl)éthyl-4-(2-carboxyéthyl)-7-[2-(4-amidinophényl)]éthynyl-3,4-dihydro-1H-1,4-benzodiazapine-2,5-dione,
1-(2-méthyl)propyl-4-(2-carboxyéthyl)-7-(4-amidino)benzamido-3,4-dihydro-1H-1,4-benzodiazapine-2,5-dione,
1-(2-méthyl)propyl-4-(2-carboxyéthyl)-7-(4-amidino)benzyloxy-3,4-dihydro-1H-1,4-benzodiazapine-2,5-dione,
1-(2-méthyl)propyl-4-(2-carboxyéthyl)-7-[2-(4-amidinophényl)]éthényl-3,4-dihydro-1H-1,4-benzodiazapine-2,5-dione,
1-(2-méthyl)propyl-4-(2-carboxyéthyl)-7-[2-(4-amidinophényl)]éthynyl-3,4-dihydro-1H-1,4-benzodiazapine-2,5-dione,
1-(2,4-difluorophényl)méthyl-4-(2-carboxyéthyl)-7-(4-(1-pipérizine)-phényl)-3,4-dihydro-1H-1,4-benzodiazapine-2,5-dione,
1-(2,4-difluorophényl)méthyl-4-(2-carboxyéthyl)-7-(4-(2-aminoéthoxy)-phényl)-3,4-dihydro-1H-1,4-benzodiazapine-2,5-dione,
1-(2,4-difluorophényl)méthyl-4-(2-carboxyéthyl)-7-(6-aminohexyl)-3,4-dihydro-1H-1,4-benzodiazapine-2,5-dione,
1-(2,4-difluorophényl)méthyl-4-(2-carboxyéthyl)-7-(5-aminopentoxy)-3,4-dihydro-1H-1,4-benzodiazapine-2,5-dione,
1-(2,4-difluorophényl)méthyl-4-(2-carboxyéthyl)-7-(4-amidino)benzamido-3,4-dihydro-1H-1,4-benzodiazapine-2,5-dione,
1-(2,4-difluorophényl)méthyl-4-(2-carboxyéthyl)-7-(4-amidino)benzyloxy-3,4-dihydro-1H-1,4-benzodiazapine-2,5-dione,
1-(2,4-difluorophényl)méthyl-4-(2-carboxyéthyl)-7-[2-(4-amidinophényl)]éthényl-3,4-dihydro-1H-1,4-benzodiazapine-2,5-dione,
1-(2,4-difluorophényl)méthyl-4-(2-carboxyéthyl)-7-[2-(4-amidinophényl)]éthynyl-3,4-dihydro-1H-1,4-benzo-diazapine-2,5-dione,
1-(4-chlorophényl)méthyl-4-(2-carboxyéthyl)-7-[2-(4-pipéridinyl)éthyloxy]-3,4-dihydro-1H-1,4-benzodiazapine-2,5-dione,
1-(diphényl)méthyl-4-(2-carboxyéthyl)-7-[2-(4-pipéridinyl)éthyloxy]-3,4-dihydro-1H-1,4-benzodiazapine-2,5-dione,
1-(m-trifluorométhyl)phényl)-4-(2-carboxyéthyl)-7-[2-(4-pipéridinyl)éthyloxy]-3,4-dihydro-1H-1,4-benzodiazapine-2,5-dione,
1-méthyl-4-(2-carboxyéthyl)-7-[2-(4-pipéridinyl)éthyloxy]-3,4-dihydro-1H-1,4-benzodiazapine-2,5-dione,
1-(1-méthyl)éthyl-4-(2-carboxyéthyl)-7-[2-(4-pipéridinyl) éthyloxy]-3,4-dihydro-1H-1,4-benzodiazapine-2,5-dione,
1-(2-méthyl)propyl-4-(2-carboxyéthyl)-7-[2-(4-pipéridinyl)éthyloxy]-3,4-dihydro-1H-1,4-benzodiazapine-2,5-dione,
1-(2,4-difluorophénylphényl)méthyl-4-(2-carboxyéthyl)-7-[2-(4-pipéridinyl)éthyloxy]-3,4-dihydro-1H-1,4-benzodiazapine-2,5-dione,
1-(4-chlorophényl)méthyl-4-(3-butanoyl)-7-(4-(1-pipérizine)-phényl)-3,4-dihydro-1H-1,4-benzodiazapine-2,5-dione,
1-(diphényl)méthyl-4-(3-butanoyl)-7-(4-(1-pipérizine)-phényl)-3,4-dihydro-1H-1,4-benzodiazapine-2,5-dione,
1-(m-trifluorométhyl)phényl-4-(3-butanoyl)-7-(4-(1-pipérizine)-phényl)-3,4-dihydro-1H-1,4-benzodiazapine-2,5-dione,
1-méthyl-4-(3-butanoyl)-7-(4-(1-pipérizine)-phényl)-3,4-dihydro-1H-1,4-benzodiazapine-2,5-dione,
1-(1-méthyl)éthyl-4-(3-butanoyl)-7-(4-(1-pipérizine)-phényl)-3,4-dihydro-1H-1,4-benzodiazapine-2,5-dione,
1-(2-méthyl)propyl-4-(3-butanoyl)-7-(4-(1-pipérizine)-phényl)-3,4-dihydro-1H-1,4-benzodiazapine-2,5-dione,
1-(4-chlorophényl)méthyl-4-(3-butanoyl)-7-(4-(2-aminoéthoxy)-phényl)-3,4-dihydro-1H-1,4-benzodiazapine-2,5-dione,
1-(diphényl)méthyl-4-(3-butanoyl)-7-(4-(2-aminoéthoxy)-phényl)-3,4-dihydro-1H-1,4-benzodiazapine-2,5-dione,
1-(m-trifluorométhyl)phényl-4-(3-butanoyl)-7-(4-(2-aminoéthoxy)-phényl)-3,4-dihydro-1H-1,4-benzodiazapine-2,5-dione,
1-méthyl-4-(3-butanoyl)-7-(4-(2-aminoéthoxy)-phényl)-3,4-dihydro-1H-1,4-benzodiazapine-2,5-dione,
1-(1-méthyl)éthyl-4-(3-butanoyl)-7-(4-(2-amino-éthoxy)-phényl)-3,4-dihydro-1H-1,4-benzodiazapine-2,5-dione,
1-(2-méthyl)propyl-4-(3-butanoyl)-7-(4-(2-amino-éthoxy)-phényl)-3,4-dihydro-1H-1,4-benzodiazapine-2,5-dione,
1-(diphényl)méthyl-4-(3-butanoyl)-7-(6-aminohexyl)-3,4-dihydro-1H-1,4-benzodiazapine-2,5-dione,
1-(diphényl)méthyl-4-(3-butanoyl)-7-(6-aminohex-1-ynyl)-3,4-dihydro-1H-1,4-benzodiazapine-2,5-dione,
1-(diphényl)méthyl-4-(3-butanoyl)-7-(5-aminopentoxy)-3,4-dihydro-1H-1,4-benzodiazapine-2,5-dione,
1-(diphényl)méthyl-4-(3-butanoyl)-7-(5-guanidinopentyl)-3,4-dihydro-1H-1,4-benzodiazapine-2,5-dione,
1-(diphényl)méthyl-4-(3-butanoyl)-7-(4-guanidinobutoxy)-3,4-dihydro-1H-1,4-benzodiazapine-2,5-dione,
1-(diphényl)méthyl-4-(3-butanoyl)-7-(5-aminopent-1-ynyl)-3,4-dihydro-1H-1,4-benzodiazapine-2,5-dione,
1-(diphényl)méthyl-4-(3-butanoyl)-7-(5-guanidinopent-1-ynyl)-3,4-dihydro-1H-1,4-benzodiazapine-2,5-dione,
1-(diphényl)méthyl-4-(3-butanoyl)-7-(4-amidino)-benzamido-3,4-dihydro-1H-1,4-benzodiazapine-2,5-dione,
1-(diphényl)méthyl-4-(3-butanoyl)-7-(4-amidino)-benzyloxy-3,4-dihydro-1H-1,4-benzodiazapine-2,5-dione,
1-(diphényl)méthyl-4-(3-butanoyl)-7-[2-(4-amidinophényl)] éthényl-3,4-dihydro-1H-1,4-benzodiazapine-2,5-dione,
1-(diphényl)méthyl-4-(3-butanoyl)-7-[2-(4-amidinophényl)]éthynyl-3,4-dihydro-1H-1,4-benzodiazapine-2,5-dione,
1-(4-chlorophényl)méthyl-4-(3-butanoyl)-7-(6-aminohexyl)-3,4-dihydro-1H-1,4-benzodiazapine-2,5-dione,
1-(4-chlorophényl)méthyl-4-(3-butanoyl)-7-(5-aminopentoxy)-3,4-dihydro-1H-1,4-benzodiazapine-2,5-dione,
1-(4-chlorophényl)méthyl-4-(3-butanoyl)-7-(4-amidino)benzamido-3,4-dihydro-1H-1,4-benzodiazapine-2,5-dione,
1-(4-chlorophényl)méthyl-4-(3-butanoyl)-7-(4-amidino)benzyloxy-3,4-dihydro-1H-1,4-benzodiazapine-2,5-dione,
1-(4-chlorophényl)méthyl-4-(3-butanoyl)-7-[2-(4-amidinophényl)]éthényl-3,4-dihydro-1H-1,4-benzodiazapine-2,5-dione,
1-(4-chlorophényl)méthyl-4-(3-butanoyl)-7-[2-(4-amidinophényl)]éthynyl-3,4-dihydro-1H-1,4-benzodiazapine-2,5-dione,
1-méthyl-4-(3-butanoyl)-7-(4-amidino)benzamido-3,4-dihydro-1H-1,4-benzodiazapine-2,5-dione,
1-méthyl-4-(3-butanoyl)-7-(4-amidino)benzyloxy-3,4-dihydro-1H-1,4-benzodiazapine-2,5-dione,
1-méthyl-4-(3-butanoyl)-7-[2-(4-amidinophényl)]-éthényl-3,4-dihydro-1H-1,4-benzodiazapine-2,5-dione,
1-méthyl-4-(3-butanoyl)-7-[2-(4-amidinophényl)]-éthynyl-3,4-dihydro-1H-1,4-benzodiazapine-2,5-dione,
1-(1-méthyl)éthyl-4-(3-butanoyl)-7-(4-amidino)-benzamido-3,4-dihydro-1H-1,4-benzodiazapine-2,5-dione,
1-(1-méthyl)éthyl-4-(3-butanoyl)-7-(4-amidino)-benzyloxy-3,4-dihydro-1H-1,4-benzodiazapine-2,5-dione,
1-(1-méthyl)éthyl-4-(3-butanoyl)-7-[2-(4-amidinophényl)]éthényl-3,4-dihydro-1H-1,4-benzodiazapine-2,5-dione,
1-(2-méthyl)éthyl-4-(3-butanoyl)-7-[2-(4-amidinophényl)]éthynyl-3,4-dihydro-1H-1,4-benzodiazapine-2,5-dione,
1-(2-méthyl)propyl-4-(3-butanoyl)-7-(4-amidino)-benzamido-3,4-dihydro-1H-1,4-benzodiazapine-2,5-dione,
1-(2-méthyl)propyl-4-(3-butanoyl)-7-(4-amidino)-benzyloxy-3,4-dihydro-1H-1,4-benzodiazapine-2,5-dione,
1-(2-méthyl)propyl-4-(3-butanoyl)-7-[2-(4-amidinophényl)]éthényl-3,4-dihydro-1H-1,4-benzodiazapine-2,5-dione,
1-(2-méthyl)propyl-4-(3-butanoyl)-7-[2-(4-amidinophényl)] éthynyl-3,4-dihydro-1H-1,4-benzodiazapine-2,5-dione,
1-(2,4-difluorophényl)méthyl-4-(3-butanoyl)-7-(4-(1-pipérizine)-phényl)-3,4-dihydro-1H-1,4-benzodiazapine-2,5-dione,
1-(2,4-difluorophényl)méthyl-4-(3-butanoyl)-7-(4-(2-aminoéthoxy)-phényl)-3,4-dihydro-1H-1,4-benzodiazapine-2,5-dione,
1-(2,4-difluorophényl)méthyl-4-(3-butanoyl)-7-(6-aminohexyl)-3,4-dihydro-1H-1,4-benzodiazapine-2,5-dione,
1-(2,4-difluorophényl)méthyl-4-(3-butanoyl)-7-(5-aminopentoxy)-3,4-dihydro-1H-1,4-benzodiazapine-2,5-dione,
1-(2,4-difluorophényl)méthyl-4-(3-butanoyl)-7-(4-amidino)benzamido-3,4-dihydro-1H-1,4-benzodiazapine-2,5-dione,
1-(2,4-difluorophényl)méthyl-4-(3-butanoyl)-7-(4-amidino)benzyloxy-3,4-dihydro-1H-1,4-benzodiazapine-2,5-dione,
1-(2,4-difluorophényl)méthyl-4-(3-butanoyl)-7-[2-(4-amidinophényl)]éthényl-3,4-dihydro-1H-1,4-benzodiazapine-2,5-dione,
1-(2,4-difluorophényl)méthyl-4-(3-butanoyl)-7-[2-(4-amidinophényl)]éthynyl-3,4-dihydro-1H-1,4-benzodiazapine-2,5-dione,
1-(4-chlorophényl)méthyl-4-(3-butanoyl)-7-[2-(4-pipéridinyl)éthyloxy]-3,4-dihydro-1H-1,4-benzodiazapine-2,5-dione,
1-(diphényl)méthyl-4-(3-butanoyl)-7-[2-(4-pipéridinyl)éthyloxy]-3,4-dihydro-1H-1,4-benzodiazapine-2,5-dione,
1-(m-trifluorométhyl)phényl-4-(3-butanoyl)-7-[2-(4-pipéridinyl) éthyloxy]-3,4-dihydro-1H-1,4-benzodiazapine-2,5-dione,
1-méthyl-4-(3-butanoyl)-7-[2-(4-pipéridinyl)-éthyloxy]-3,4-dihydro-1H-1,4-benzodiazapine-2,5-dione,
1-(1-méthyl)éthyl-4-(3-butanoyl)-7-[2-(4-pipéridinyl)éthyloxy]-3,4-dihydro-1H-1,4-benzodiazapine-2,5-dione,
1-(2-méthyl)propyl-4-(3-butanoyl)-7-[2-(4-pipéridinyl) éthyloxy]-3,4-dihydro-1H-1,4-benzodiazapine-2,5-dione,
1-(2,4-difluorophényl)méthyl-4-(3-butanoyl)-7-[2-(4-pipéridinyl)éthyloxy]-3,4-dihydro-1H-1,4-benzodiazapine-2,5-dione,
1-(4-chlorophényl)méthyl-4-[(2-méthyl)carboxyéthyl)]-7-(4-(1-pipérizine)-phényl)-3,4-dihydro-1H-1,4-benzo-diazapine-2,5-dione,
1-(diphényl)méthyl-4-[(2-méthyl)carboxyéthyl)]-7-(4-(1-pipérizine)-phényl)-3,4-dihydro-1H-1,4-benzodiazapine-2,5-dione,
1-(m-trifluorophényl)phényl-4-[(2-méthyl)carboxyéthyl)]-7-(4-(1-pipérizine)-phényl)-3,4-dihydro-1H-1,4-benzo-diazapine-2,5-dione,
1-méthyl-4-[(2-méthyl)carboxyéthyl)]-7-(4-(1-pipérizine)-phényl)-3,4-dihydro-1H-1,4-benzodiazapine-2,5-dione,
1-(1-méthyl)éthyl-4-[(2-méthyl)carboxyéthyl)]-7-(4-(1-pipérizine)-phényl)-3,4-dihydro-1H-1,4-benzodiazapine-2,5-dione,
1-(2-méthyl)propyl-4-[(2-méthyl)carboxyéthyl)]-7-(4-(1-pipérizine)-phényl)-3,4-dihydro-1H-1,4-benzodiazapine-2,5-dione,
1-(4-chlorophényl)méthyl-4-[(2-méthyl)carboxyéthyl)]-7-(4-(2-aminoéthoxy)-phényl)-3,4-dihydro-1H-1,4-benzodiazapine-2,5-dione,
1-(diphényl)méthyl-4-[(2-méthyl)carboxyéthyl)]-7-(4-(2-aminoéthoxy) -phényl)-3,4-dihydro-1H-1,4-benzodiazapine-2,5-dione,
1-(m-trifluorométhyl)phényl-4-[(2-méthyl)carboxyéthyl)]-7-(4-(2-aminoéthoxy)-phényl)-3,4-dihydro-1H-1,4-benzodiazapine-2,5-dione,
1-méthyl-4-[(2-méthyl)carboxyéthyl)]-7-(4-(2-aminoéthoxy)-phényl)-3,4-dihydro-1H-1,4-benzodiazapine-2,5-dione,
1-(1-méthyl)éthyl-4-[(2-méthyl)carboxyéthyl)]-7-(4-(2-aminoéthoxy)-phényl)-3,4-dihydro-1H-1,4-benzodiazapine-2,5-dione,
1-(2-tréthyl)propyl-4-[(2-méthyl)carboxyéthyl)]-7-(4-(2-aminoéthoxy)-phényl)-3,4-dihydro-1H-1,4-benzodiazapine-2,5-dione,
1-(diphényl)méthyl-4-[(2-méthyl)carboxyéthyl)]-7-(6-aminohexyl)-3,4-dihydro-1H-1,4-benzodiazapine-2,5-dione,
1-(diphényl)méthyl-4-[(2-méthyl)carboxyéthyl)]-7-(6-aminohex-1-ynyl)-3,4-dihydro-1H-1,4-benzodiazapine-2,5-dione,
1-(diphényl)méthyl-4-[(2-méthyl)carboxyéthyl)]-7-(5-aminopentoxy)-3,4-dihydro-1H-1,4-benzodiazapine-2,5-dione,
1-(diphényl)méthyl-4-[(2-méthyl)carboxyéthyl)]-7-(5-guanidinopentyl)-3,4-dihydro-1H-1,4-benzodiazapine-2,5-dione,
1-(diphényl)méthyl-4-[(2-méthyl)carboxyéthyl)]-7-(4-guanidinobutoxy)-3,4-dihydro-1H-1,4-benzodiazapine-2,5-dione,
1-(diphényl)méthyl-4-[(2-méthyl)carboxyéthyl)]-7-(5-aminopent-1-ynyl)-3,4-dihydro-1H-1,4-benzodiazapine-2,5-dione,
1-(diphényl)méthyl-4-[(2-méthyl)carboxyéthyl)]-7-(5-guanidinopent-1-ynyl)-3,4-dihydro-1H-1,4-benzodiazapine-2,5-dione,
1-(diphényl)méthyl-4-[(2-méthyl)carboxyéthyl)]-7-(4-(amidino)benzamido)-3,4-dihydro-1H-1,4-benzodiazapine-2,5-dione,
1-(diphényl)méthyl-4-[(2-méthyl)carboxyéthyl)]-7-(4-amidino)benzyloxy-3,4-dihydro-1H-1,4-benzodiazapine-2,5-dione,
1-(diphényl)méthyl-4-[(2-méthyl)carboxyéthyl)]-7-[2-(4-amidinophényl)]éthényl-3,4-dihydro-1H-1,4-benzo-diazapine-2,5-dione,
1-(diphényl)méthyl-4-[(2-méthyl)carboxyéthyl)]-7-[2-(4-amidinophényl)]éthynyl-3,4-dihydro-1H-1,4-benzodiazapine-2,5-dione,
1-(4-chlorophényl)méthyl-4-[(2-méthyl)carboxyéthyl)]-7-(6-aminohexyl)-3,4-dihydro-1H-1,4-benzodiazapine-2,5-dione,
1-(4-chlorophényl)méthyl-4-[(2-méthyl)carboxyéthyl)]-7-(5-aminopentoxy)-3,4-dihydro-1H-1,4-benzodiazapine-2,5-dione,
1-(4-chlorophényl)méthyl-4-[(2-méthyl)carboxyéthyl)]-7-(4-amidino)benzamido-3,4-dihydro-1H-1,4-benzodiazapine-2,5-dione,
1-(4-chlorophényl)méthyl-4-[(2-méthyl)carboxyéthyl)]-7-(4-amidino)benzyloxy-3,4-dihydro-1H-1,4-benzo-diazapine-2,5-dione,
1-(4-chlorophényl)méthyl-4-[(2-méthyl)carboxyéthyl)]-7-[2-(4-amidinophényl)]éthényl-3,4-dihydro-1H-1,4-benzodiazapine-2,5-dione,
1-(4-chlorophényl)méthyl-4-[(2-méthyl)carboxyéthyl)]-7-[2-(4-amidinophényl)]éthynyl-3,4-dihydro-1H-1,4-benzodiazapine-2,5-dione,
1-méthyl-4-[(2-méthyl)carboxyéthyl)]-7-(4-amidino)benzamido-3,4-dihydro-1H-1,4-benzodiazapine-2,5-dione,
1-méthyl-4-[(2-méthyl)carboxyéthyl)]-7-(4-amidino)benzyloxy-3,4-dihydro-1H-1,4-benzodiazapine-2,5-dione,
1-méthyl-4-[(2-méthyl)carboxyéthyl)]-7-[2-(4-amidinophényl)]éthényl-3,4-dihydro-1H-1,4-benzodiazapine-2,5-dione,
1-méthyl-4-[(2 -méthyl)carboxyéthyl)]-7-[2-(4-amidinophényl)]éthynyl-3,4-dihydro-1H-1,4-benzodiazapine-2,5-dione,
1-(1-méthyl)éthyl-4-[(2-méthyl)carboxyéthyl)]-7-(4-amidino)benzamido-3,4-dihydro-1H-1,4-benzodiazapine-2,5-dione,
1-(1-méthyl)éthyl-4-[(2-méthyl)carboxyéthyl)]-7-(4-amidino)benzyloxy-3,4-dihydro-1H-1,4-benzodiazapine-2,5-dione,
1-(1-méthyl)éthyl-4-[(2-méthyl)carboxyéthyl)]-7-[2-(4-amidinophényl)]éthényl-3,4-dihydro-1H-1,4-benzodiazapine-2,5-dione,
1-(2-méthyl)éthyl-4-[(2-méthyl)carboxyéthyl)]-7-[2-(4-amidinophényl)]éthynyl-3,4-dihydro-1H-1,4-benzodiazapine-2,5-dione,
1-(2-méthyl)propyl-4-[(2-méthyl)carboxyéthyl)]-7-(4-amidino)benzamido-3,4-dihydro-1H-1,4-benzodiazapine-2,5-dione,
1-(2-méthyl)propyl-4-[(2-méthyl)carboxyéthyl)]-7-(4-amidino)benzyloxy-3,4-dihydro-1H-1,4-benzodiazapine-2,5-dione,
1-(2-méthyl)propyl-4-[(2-méthyl)carboxyéthyl)]-7-[2-(4-amidinophényl)]éthényl-3,4-dihydro-1H-1,4-benzodiazapine-2,5-dione,
1-(2-méthyl)propyl-4-[(2-méthyl)carboxyéthyl)]-7-[2-(4-amidinophényl)]éthényl-3,4-dihydro-1H-1,4-benzodiazapine-2,5-dione,
1-(2,4-difluorophényl)méthyl-4-[(2-méthyl)-carboxyéthyl)]-7-(4-(1-pipérizine)-phényl)-3,4-dihydro-1H-1,4-benzodiazapine-2,5-dione,
1-(2,4-difluorophényl)méthyl-4-[(2-méthyl)-carboxyéthyl)]-7-(4-(2-aminoéthoxy)-phényl)-3,4-dihydro-1H-1,4-benzodiazapine-2,5-dione,
1-(2,4-difluorophényl)méthyl-4-[(2-méthyl)-carboxyéthyl)]-7-(6-aminohexyl)-3,4-dihydro-1H-1,4-benzodiazapine-2,5-dione,
1-(2,4-difluorophényl)méthyl-4-[(2-méthyl)-carboxyéthyl)]-7-(5-aminopentoxy)-3,4-dihydro-1H-1,4-benzodiazapine-2,5-dione,
1-(2,4-difluorophényl)méthyl-4-[(2-méthyl)-carboxyéthyl)]-7-(4-amidino)benzamido-3,4-dihydro-1H-1,4-benzodiazapine-2,5-dione,
1-(2,4-difluorophényl)méthyl-4-[(2-méthyl)-carboxyéthyl)]-7-(4-amidino)benzyloxy-3,4-dihydro-1H-1,4-benzodiazapine-2,5-dione,
1-(2,4-difluorophényl)méthyl-4-[(2-méthyl)-carboxyéthyl)]-7-[2-(4-amidinophényl)]éthényl-3,4-dihydro-1H-1,4-benzodiazapine-2,5-dione,
1-(2,4-difluorophényl)méthyl-4-[(2-méthyl)-carboxyéthyl)]-7-[2-(4-amidinophényl)]éthynyl-3,4-dihydro-1H-1,4-benzodiazapine-2,5-dione,
1-(4-chlorophényl)méthyl-4-[(2-méthyl)carboxyéthyl)-7-[2-(4-pipéridinyl)éthyloxy]-3,4-dihydro-1H-1,4-benzodiazapine-2,5-dione,
1-(diphényl)méthyl-4-[(2-méthyl)carboxyéthyl)]-7-[2-(4-pipéridinyl)éthyloxy]-3,4-dihydro-1H-1,4-benzo-diazapine-2,5-dione,
1-(m-trifluorométhyl)phényl-4-[(2-méthyl)carboxyéthyl)]-7-[2-(4-pipéridinyl)éthyloxy]-3,4-dihydro-1H-1,4-benzodiazapine-2,5-dione,
1-méthyl-4-[(2-méthyl) carboxyéthyl)]-7-[2-(4-pipéridinyl)éthyloxy]-3,4-dihydro-1H-1,4-benzodiazapine-2,5-dione,
1-(1-méthyl)éthyl-4-[(2-méthyl)carboxyéthyl)]-7-[2-(4-pipéridinyl)éthyloxy]-3,4-dihydro-1H-1,4-benzodiazapine-2,5-dione,
1-(2-méthyl)propyl-4-[(2-méthyl)carboxyéthyl)]-7-[2-(4-pipéridinyl)éthyloxy]-3,4-dihydro-1H-1,4-benzodiazapine-2,5-dione,
1-(2,4-difluorophényl)méthyl-4-[(2-méthyl)-carboxyéthyl)]-7-[2-(4-pipéridinyl)éthyloxy]-3,4-dihydro-1H-1,4-benzodiazapine-2,5-dione,
1-(3-butyryl)aminophényl)méthyl-4-(2-carboxyéthyl)-7-(6-aminohexyl)-3,4-dihydro-1H-1,4-benzodiazapine-2,5-dione,
1-(3-butyryl)aminophényl)méthyl-4-(2-carboxyéthyl)-7-(5-guanidinopentyl)-3,4-dihydro-1H-1,4-benzodiazapine-2,5-dione,
1-(3-(4-aminobutyryl)aminophényl)méthyl-4-(2-carboxyéthyl)-7-(6-aminohexyl)-3,4-dihydro-1H-1,4-benzo-diazapine-2,5-dione,
1-(3-(4-guanidinobutyryl)aminophényl)méthyl-4-(2-carboxyéthyl)-7-(5-guanidinopentyl)-3,4-dihydro-1H-1,4-benzodiazapine-2,5-dione,
1-(3-(6-aminohexoyl)aminophényl)méthyl-4-(2-carboxyéthyl)-7-(6-aminohexyl)-3,4-dihydro-1H-1,4-benzodiazapine-2,5-dione,
1-(3-(6-guanidinohexoyl)aminophényl)méthyl-4-(2-carboxyéthyl)-7-(5-guanidinopentyl)-3,4-dihydro-1H-1,4-benzodiazapine-2,5-dione,
1-(3-(6-aminopentoyl)aminophényl)méthyl-4-(2-carboxyéthyl)-7-(6-aminohexyl)-3,4-dihydro-1H-1,4-benzodiazapine-2,5-dione,
1-(3-(6-guanidinopentoyl)aminophényl)méthyl-4-(2-carboxyéthyl)-7-(5-guanidinopentyl)-3,4-dihydro-1H-1,4-benzodiazapine-2,5-dione,
1-(3-(6-aminopropionyl)aminophényl)méthyl-4-(2-carboxyéthyl)-7-(6-aminohexyl)-3,4-dihydro-1H-1,4-benzodiazapine-2,5-dione,
1-(3-(6-guanidinopropionyl)aminophényl)méthyl-4-(2-carboxyéthyl)-7-(5-guanidinopentyl)-3,4-dihydro-1H-1,4-benzodiazapine-2,5-dione,
1-(3-(6-aminoacétyl)aminophényl)méthyl-4-(2-carboxyéthyl)-7-(6-aminohexyl)-3,4-dihydro-1H-1,4-benzodiazapine-2,5-dione,
1-(3-(6-guanidinoacétyl)aminophényl)méthyl-4-(2-carboxyéthyl)-7-(5-guanidinopentyl)-3,4-dihydro-1H-1,4-benzodiazapine-2,5-dione,
1-(3-(4-guanidinobenzyl)aminophényl)méthyl-4-(2-carboxyéthyl)-7-(6-aminohexyl)-3,4-dihydro-1H-1,4-benzodiazapine-2,5-dione,
1-(4-(6-aminopropionyl)aminophényl)méthyl-4-(2-carboxyéthyl)-7-(6-aminohexyl)-3,4-dihydro-1H-1,4-benzodiazapine-2,5-dione,
1-(3-(4-aminobutyl)phényl)méthyl-4-(2-carboxyéthyl)-7-(6-aminohexyl)-3,4-dihydro-1H-1,4-benzodiazapine-2,5-dione, et
1-(4-carboxyphényl)méthyl-4-(2-carboxyéthyl)-7-(6-aminohexyl)-3,4-dihydro-1H-1,4-benzodiazapine-2,5-dione.

7. Composé suivant la revendication 1, représenté par la formule III et IV : 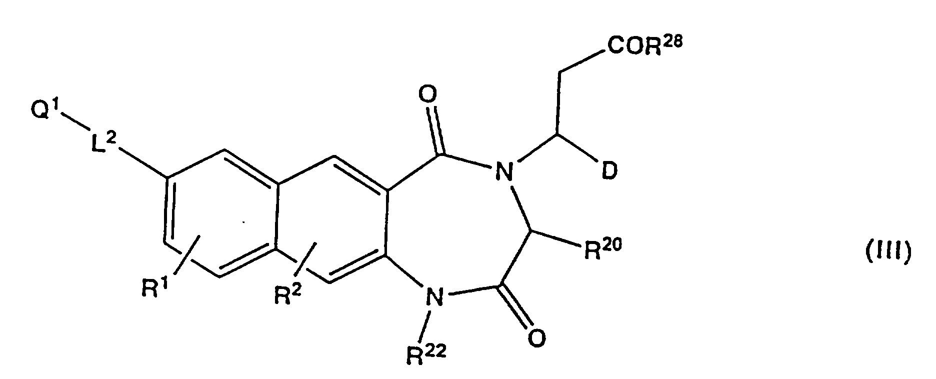 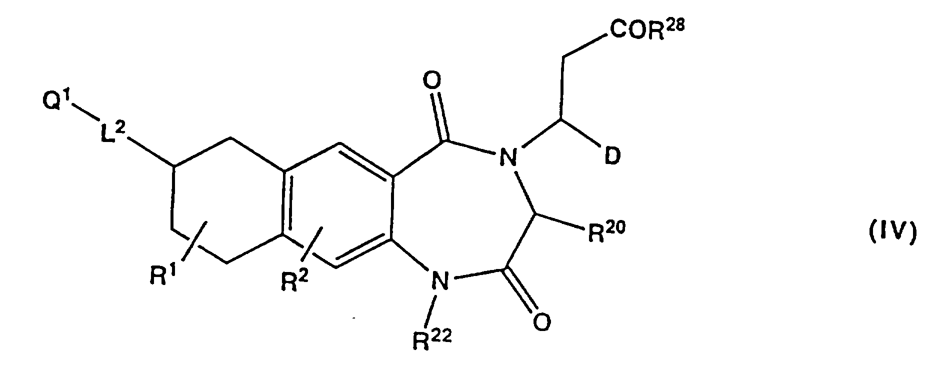
dans lesquelles
R¹ et R sont choisis, indépendamment, entre l'hydrogène et des groupes halogéno (F, Cl, Br, I) ;
Q¹ est choisi dans le groupe consistant en
(A) un groupe amino,
(B) un groupe amidino,
(C) un groupe aminoalkylèneimino,
(D) un groupe iminoalkylèneamino, et
(E) un groupe guanidino ;
R⁰ représente de l'hydrogène ;
R est choisi dans le groupe consistant en (i) l'hydrogène, (ii) un groupe alkyle en C₁ à C₁₂ facultativement substitué, (iii) un groupe aryle en C₆ à C₁₄ facultativement substitué, (iv) un groupe cycloalkyle en C₃ à C₁₄ facultativement substitué, (v) un groupe (alkyle en C₁ à C₁₂) - (aryle en C₆ à C₁₄) facultativement substitué, (vi) un groupe (alkyle en C₁ à C₁₂)-(cycloalkyle en C₃ à C₁₄) facultativement substitué, dans lesquels les substituants sont choisis entre des groupes (a) halogéno (F, Cl, Br, I), (b) nitro, (c) hydroxy, (d) carboxy, (e) tétrazole, (f) hydroxamate, (g) sulfonamide, (h) trifluorimide, (i) phosphonate, (j) alkyle en C₁ à C₆, (k) aryle en C₆ à C₁₄, (l) benzyle, (m) cycloalkyle en C₃ à C₁₄, (n) COR⁴ dans lequel R⁴ est choisi entre des groupes alkoxy en C₁ à C₈, alcénoxy en C₃ à C₁₂, aryloxy en C₆ à C₁₂, di-(alkyle en C₁ à C₈)-amino-(alkoxy en C₁ à C₈), acylamino-(alkoxy en C₁ à C₈) choisi entre les groupes acétylaminoéthoxy, nicotinoylaminoéthoxy, succinamidoéthoxy et pivaloyloxyéthoxy, et (aryle en C₆ à C₁₂)-(alkoxy en C₁ à C₈) dans lequel le groupe aryle est non substitué ou substitué avec un à trois des groupes nitro, halogéno (F, Cl, Br, I), alkoxy en C₁ à C₄, amino, hydroxy, hydroxyalkoxy en C₂ à C₈ et dihydroxyalkoxy en C₃ à C₈, (o) CONR⁵R⁶ dans lequel R⁵ et R⁶ sont choisis, indépendamment, entre l'hydrogène, des groupes alkyle en C₁ à C₁₀, alcényle en C₃ à C₁₀, aryle en C₆ à C₁₄, (alkyle en C₁ à C₆)-(aryle en C₆ à C₁₀), facultativement R⁵ et R⁶, pris conjointement, peuvent former un groupe triméthylène, tétraméthylène, pentaméthylène ou 3-oxopentaméthylène, et (vii) un groupe Q-L³ dans lequel Q est choisi entre l'hydrogène et Q¹, et L³ est choisi entre une liaison chimique et L¹, L¹ représentant un radical divalent facultativement substitué choisi entre des groupes alkylène en C₃ à C₇, cycloalkylène en C₃ à C₇, alcénylène en C₃ à C₇, cycloalcénylène en C₄ à C₇, cycloalcadiényléne en C₅ à C₈, alcadiénylène en C₃ à C₇, alcynylène en C₃ à C₇, alcénylène en C₄ à C_{7'} arylène en C₆ à C_{14'} (aryle en C₆ à C₁₄)-alcynylène en C₂ à C₄, (alkyle en C₁ à C₃)-(aryle en C₆ à C₁₄)-(alcynylène en C₂ à C₄), (aryle en C₆ à C₁₄)-(alcénylène en C₂ à C₄), (alkyle en C₁ à C₃)-(arylène en C₆ à C₁₄), (alkyle en C₁ à C₃)-(aryle en C₆ à C₁₄)-(alcénylène en C₂ à C₄), (aryle en C₆ à C₁₄)-(alkylène en C₁ à C₃), (aryle en C₆ à C₁₄)-(alkyloxyène en C₁ à C₃), (alkyle en C₁ ou C₂)-(aryle en C₆ à C₁₄)-(alkylène en C₁ ou C₂), (alkyle en C₁ à C₃)-oxy-(arylène en C₆ à C₁₄), alkyloxyène en C₂ à C₈, (alkyle en C₁ à C₅)-oxy-(alkylène en C₁ à C₅), aryloxyène en C₆ à C₁₀, aryle (en C₆ à C₁₀)-oxy-(alkylène en C₁ à C₅), (aryle en C₆ à C₁₀)-thio-(alkylène en C₁ à C₅), 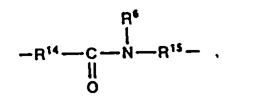 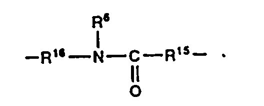 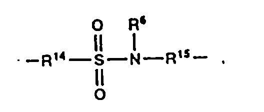 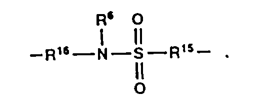 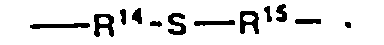 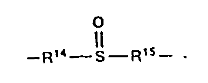 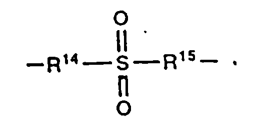 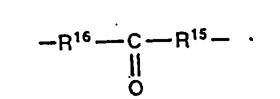 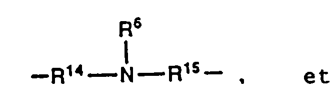 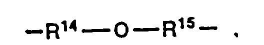
dans lesquels R¹⁴ est choisi entre une liaison chimique et des groupes alkyle en C₁ à C₈, cycloalkyle en C₃ à C₇, alcényle en C₂ à C₅, alcynyle en C₃ à C₅, aryle en C₆ à C₁₀, (alkyle en C₁ à C₃)-(aryle en C₆ à C₁₂), (alkyle en C₁ ou C₂)-(aryle en C₆ à C₁₀)-(alkyle en C₁ ou C₂ et (aryle en C₆ à C₁₀)-(alkyle en C₁ ou C₂), R¹⁵ est choisi entre une liaison chimique et des groupes alkyle en C₁ à C₄, alcényle en C₂ à C₄, alcynyle en C₂ à C₄ et aryle en C₆ à C₁₀, R¹⁶ est choisi entre une liaison chimique et des groupes alkyle en C₂ à C₅, cycloalkyle en C₃ à C₇, alcényle en C₃ à C₅, alcynyle en C₃ à C₅, aryle en C₆ à C₁₀, (alkyle en C₁ à C₃)-(aryle en C₆ à C₁₂), (alkyle en C₁ à C₃)-(aryle en C₆ à C₁₂), et (aryle en C₆ à C₁₀)-(alkyle en C₁ ou C₂), dans lesquels R¹⁴ ou R¹⁶ est lié à N et R¹⁵ est lié à Q et dans lesquels les substituants consistent en un à trois groupes R¹ ;
L représente un radical divalent facultativement substitué choisi dans le groupe consistant en les radicaux 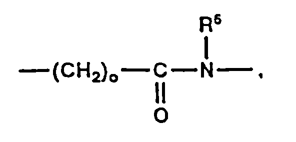 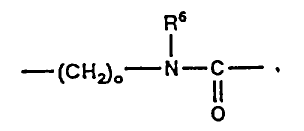 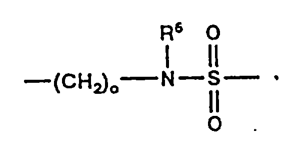 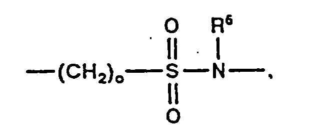 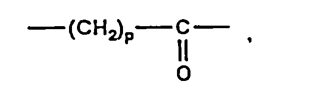 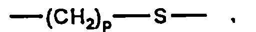 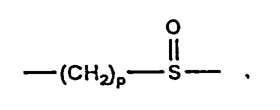 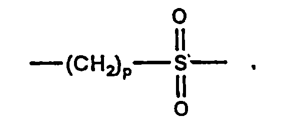 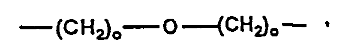 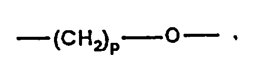 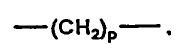 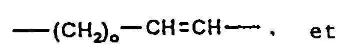 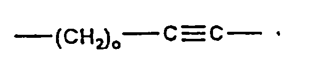 dans lesquels o est égal à 1 ou 2, p est égal à 1, 2, 3 ou 4, R⁶ répond à la définition précitée, et les substituants consistent en un à trois groupes R¹ ;
D est choisi entre le groupe consistant en R et -(C=O)-Xaa, dans lequel Xaa consiste en un à trois D- ou L-α-amino-acides ;
R⁸ est choisi dans des groupes (i) hydroxy, (ii) alkoxy en C₁ à C₈, (iii) alcénoxy en C₃ à C₁₂, (iv) aryloxy en C₆ à C₁₂, (v) (alkyle en C₁ à C₆)-(aryle en C₆ à C₁₂)-oxy, (vi) di-(alkyle en C₁ à C₈)-amino-(alkoxy en C₁ à C₈), (vii) acylamino-(alkoxy en C₁ à C₈) choisi entre les groupes (a) acétylaminoéthoxy, (b) nicotinoylaminoéthoxy et (c) succinamidoéthoxy, (viii) (alcoyle en C₁ à C₈)-oxy-(alkoxy en C₁ à C₈), (ix) (aryle en C₆ à C₁₂)-(alkoxy en C₁ à C₈), dans lequel le groupe aryle est non substitué ou substitué avec un à trois des groupes (a) nitro, (b) halogéno (F, Cl, Br, I), (c) alkoxy en C₁ à C₄ et (d) amino, (x) hydroxy-alkoxy en C₂ à C₈, (xi) dihydroxy-alkoxy en C₃ à C₈, et (xii) NR⁹R³⁰ dans lequel R⁹ et R³⁰ sont choisis, indépendamment, dans le groupe consistant en (a) l'hydrogène, (b) un groupe alkyle en C₁ à C₈, (c) un groupe alcényle en C₃ à C₈, (d) un groupe aryle en C₆ à C₁₂, dans lequel le groupe aryle est non substitué ou substitué avec un à trois des groupes nitro, halogéno (F, Cl, Br, I), alkoxy en C₁ à C₄, et amino, et (e) un groupe (aryle en C₆ à C₁₂) - (alkyle en C₁ à C₈) dans lequel le groupe aryle est non substitué ou substitué avec un à trois des groupes nitro, halogéno (F, Cl, Br, I) et alkoxy en C₁ à C₄ ;
ses sels pharmaceutiquement acceptables.

8. Composé suivant la revendication 1, représenté par la formule V : 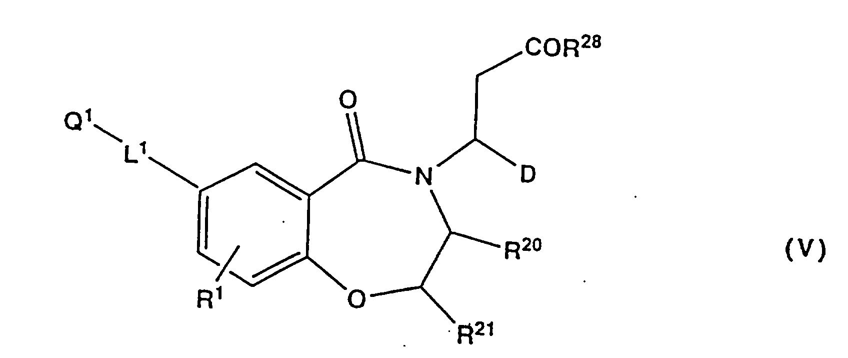
dans laquelle
R¹ représente un à trois groupes choisis indépendamment entre l'hydrogène et un halogène (F, Cl, Br, I) ;
Q¹ est choisi dans le groupe consistant en
(A) un groupe amino,
(B) un groupe amidino,
(C) un groupe aminoalkylèneimino,
(D) un groupe iminoalkylèneamino, et
(E) un groupe guanidino ;
L¹ représente un radical divalent facultativement substitué choisi dans le groupe consistant en les radicaux 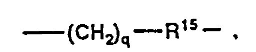 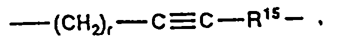 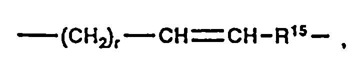 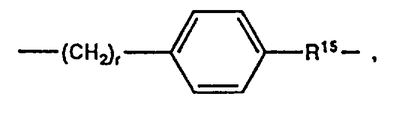 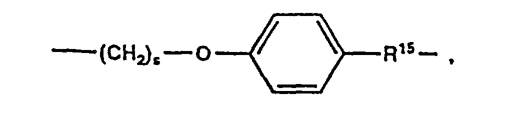 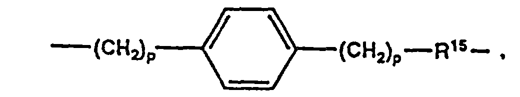 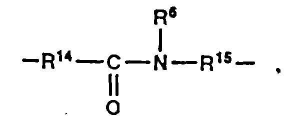 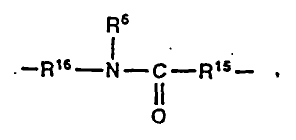 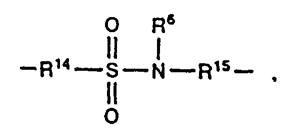 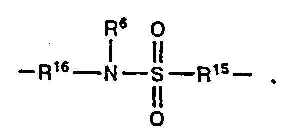 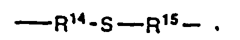 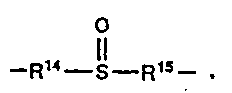 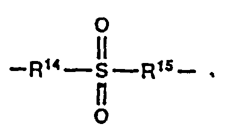 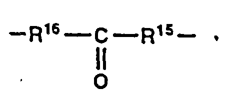 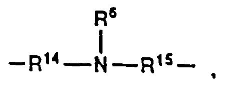 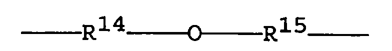
dans lesquels
p est égal à 1, 2, 3 ou 4 ;
q est égal à 3, 4, 5, 6 ou 7 ;
r est égal à 1, 2, 3, 4 ou 5 ;
s est égal 2 ou 3 ;
R¹⁴ est choisi entre des groupes
alkyle en C₂ à C₅,
cycloalkyle en C₃ à C₇,
alcényle en C₂ à C₅,
alcynyle en C₃ à C₅,
aryle en C₆ à C₁₀,
(alkyle en C₁ ou C₂)-(aryle en C₆ à C₁₂),
(alkyle en C₁ ou C₂)-(aryle en C₆ à C₁₀)-(alkyle en C₁ ou C₂),
(aryle en C₆ à C₁₀)-(alkyle en C₁ ou C₂), et
(aryle en C₆ à C₁₀)-oxy-(alkyle en C₁ ou C₂) ;
R¹⁵ représente une liaison chimique connectant L¹ à la position 8 de la benzazépine-one ;
R¹⁶ est choisi entre des groupes
alkyle en C₂ à C₅,
cycloalkyle en C₃ à C₇,
alcényle en C₃ à C₅,
alcynyle en C₃ à C₅,
aryle en C₆ à C₁₀,
(alkyle en C₁ à C₃)-(aryle en C₆ à C₁₂), et
(aryle en C₆ à C₁₀)-(alkyle en C₁ ou C₂),
R¹⁴ et R¹⁶ liant le groupe L¹ au groupe Q¹, et
les substituants étant choisis entre l'hydrogène et des groupes halogéno (F, Cl, Br, I,), alkoxy en C₁ à C₄, alkyle en C₁ à C₄, phényle, benzyle et halogéno (F, Cl, Br, I)-alkyle en C₁ à C₄ ;
R⁰ représente l'hydrogène ;
R¹ est choisi dans le groupe consistant en (i) l'hydrogène, (ii) un groupe alkyle en C₁ à C₁₂ facultativement substitué, (iii) un groupe aryle en C₆ à C₁₄ facultativement substitué, (iv) un groupe cycloalkyle en C₃ à C₁₄ facultativement substitué, (v) un groupe (alkyle en C₁ à C₁₂)- (aryle en C₆ à C₁₄) facultativement substitué, (vi) un groupe (alkyle en C₁ à C₁₂)-(cycloalkyle en C₃ à C₁₄) facultativement substitué, dans lesquels les substituants sont choisis entre des groupes (a) halogéno (F, Cl, Br, I), (b) nitro, (c) hydroxy, (d) carboxy, (e) tétrazole, (f) hydroxamate, (g) sulfonamide, (h) trifluorimide, (i) phosphonate, (j) alkyle en C₁ à C₆, (k) aryle en C₆ à C₁₄, (l) benzyle, (m) cycloalkyle en C₃ à C₁₄, (n) COR⁴ dans lequel R⁴ est choisi entre des groupes alkoxy en C₁ à C₈, alcénoxy en C₃ à C₁₂, aryloxy en C₆ à C₁₂, di-(alkyle en C₁ à C₈)-amino-(alkoxy en C₁ à C₈), acylamino-(alkoxy en C₁ à C₈) choisi entre les groupes acétylaminoéthoxy, nicotinoylaminoéthoxy, succinamidoéthoxy et pivaloyloxyéthoxy, et (aryle en C₆ à C₁₂)-(alkoxy en C₁ à C₈) dans lequel le groupe aryle est non substitué ou substitué avec un à trois des groupes nitro, halogéno (F, Cl, Br, I), alkoxy en C₁ à C₄, amino, hydroxy, hydroxyalkoxy en C₂ à C₈ et dihydroxyalkoxy en C₃ à C₈, (o) CONR⁵R⁶ dans lequel R⁵ et R⁶ sont choisis, indépendamment, entre l'hydrogène et des groupes alkyle en C₁ à C₁₀, alcényle en C₃ à C₁₀, aryle en C₆ à C₁₄, (alkyle en C₁ à C₆)-(aryle en C₆ à C₁₀), facultativement R⁵ et R⁶, pris conjointement, peuvent former un groupe triméthylène, tétraméthylène, pentaméthylène ou 3-oxopentaméthylène, et (vii) un groupe Q-L³ dans lequel Q est choisi entre l'hydrogène et Q¹, et L³ est choisi entre une liaison chimique et L¹, R¹⁵ liant L¹ à la position 4 de la benzazépine-one ;
D est choisi entre le groupe consistant en R¹ et -(C=O)-Xaa, dans lequel Xaa représente un à trois D- ou L-α-amino-acides ;
R⁸ est choisi entre des groupes (i) hydroxy, (ii) alkoxy en C₁ à C₈, (iii) alcénoxy en C₃ à C₁₂, (iv) aryloxy en C₆ à C₁₂, (v) (alkyle en C₁ à C₆)-(aryle en C₆ à C₁₂)-oxy, (vi) di-(alkyle en C₁ à C₈)-amino-(alkoxy en C₁ à C₈), (vii) acylamino-(alkoxy en C₁ à C₈) choisi entre les groupes (a) acétylaminoéthoxy, (b) nicotinoylaminoéthoxy et (c) succinamidoéthoxy, (viii) (alcoyle en C₁ à C₈)-oxy-(alkoxy en C₁ à C₈), (ix) (aryle en C₆ à C₁₂)-(alkoxy en C₁ à C₈), dans lequel le groupe aryle est non substitué ou substitué avec un à trois des groupes (a) nitro, (b) halogéno (F, Cl, Br, I), (c) alkoxy en C₁ à C₄ et (d) amino, (x) hydroxy-alkoxy en C₂ à C₈, (xi) dihydroxy-alkoxy en C₃ à C₈, et (xii) NR⁹R³⁰ dans lequel R⁹ et R³⁰ sont choisis, indépendamment, dans le groupe consistant en (a) l'hydrogène, (b) un groupe alkyle en C₁ à C₈, (c) un groupe alcényle en C₃ à C₈, (d) un groupe aryle en C₆ à C₁₂, dans lequel le groupe aryle est non substitué ou substitué avec un à trois des groupes nitro, halogéno (F, Cl, Br, I), alkoxy en C₁ à C₄, et amino, et (e) un groupe (aryle en C₆ à C₁₂)-(alkyle en C₁ à C₈) dans lequel le groupe aryle est non substitué ou substitué avec un à trois des groupes nitro, halogéno (F, Cl, Br, I) et alkoxy en C₁ à C₄ ;
ses sels pharmaceutiquement acceptables.

9. Composé suivant la revendication 1, représenté par la formule VI : 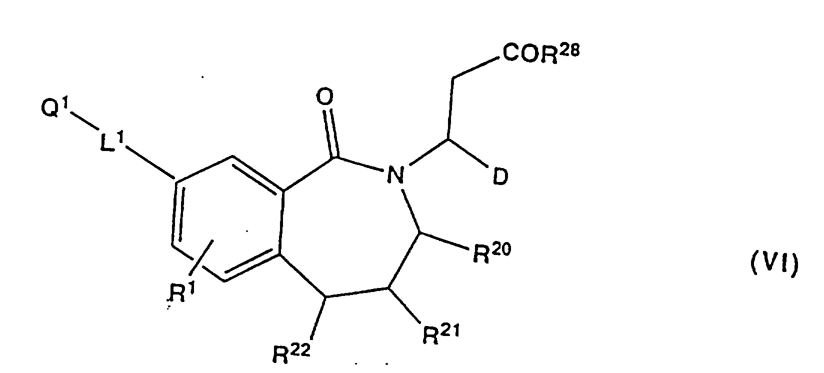
dans laquelle
R¹ représente un à trois groupes choisis indépendamment entre l'hydrogène et un halogène (F, Cl, Br, I) ;
Q¹ est choisi dans le groupe consistant en
(A) un groupe amino,
(B) un groupe amidino,
(C) un groupe aminoalkylèneimino,
(D) un groupe iminoalkylèneamino, et
(E) un groupe guanidino ;
L¹ représente un radical bivalent facultativement substitué choisi dans le groupe consistant en les radicaux 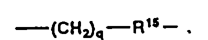 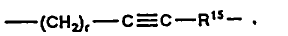 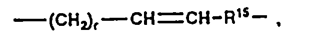 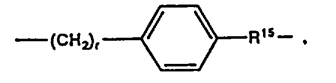 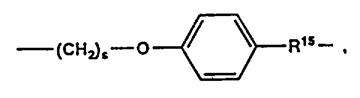 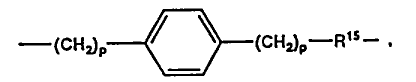 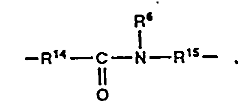 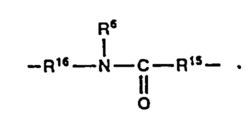 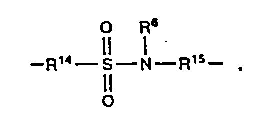 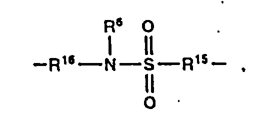 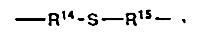 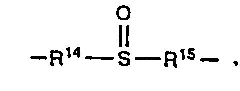 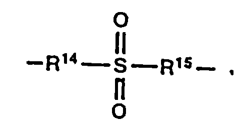 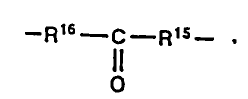 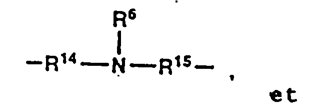 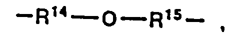
dans lesquels
p est égal à 1, 2, 3 ou 4 ;
q est égal à 3, 4, 5, 6 ou 7 ;
r est égal à 1, 2, 3, 4 ou 5 ;
s est égal 2 ou 3 ;
R¹⁴ est choisi entre des groupes
alkyle en C₂ à C₅,
cycloalkyle en C₃ à C₇,
alcényle en C₂ à C₅,
alcynyle en C₃ à C₅,
aryle en C₆ à C₁₀,
(alkyle en C₁ ou C₂)-(aryle en C₆ à C₁₂),
(alkyle en C₁ ou C₂)-(aryle en C₆ à C₁₀)-(alkyle en C₁ ou C₂),
(aryle en C₆ à C₁₀)-(alkyle en C₁ ou C₂), et
(aryle en C₆ à C₁₀)-oxy-(alkyle en C₁ ou C₂) ;
R¹⁵ représente une liaison chimique connectant L¹ à la position 8 de la benzazépine-one ;
R¹⁶ est choisi entre des groupes
alkyle en C₂ à C₅,
cycloalkyle en C₃ à C₇,
alcényle en C₃ à C₅,
alcynyle en C₃ à C₅,
aryle en C₆ à C₁₀,
(alkyle en C₁ à C₃)-(aryle en C₆ à C₁₂), et
(aryle en C₆ à C₁₀)-(alkyle en C₁ ou C₂),
R¹⁴ et R¹⁶ liant L¹ à Q¹, et
les substituants étant choisis dans le groupe consistant entre l'hydrogène et des groupes halogéno (F, Cl, Br, I,), alkoxy en C₁ à C₄, alkyle en C₁ à C₄, phényle, benzyle et halogéno (F, Cl, Br, I)-alkyle en C₁ à C₄ ;
R⁰ représente l'hydrogène ;
R¹ et R sont choisis dans le groupe consistant en (i) l'hydrogène, (ii) un groupe alkyle en C₁ à C₁₂ facultativement substitué, (iii) un groupe aryle en C₆ à C₁₄ facultativement substitué, (iv) un groupe cycloalkyle en C₃ à C₁₄ facultativement substitué, (v) un groupe (alkyle en C₁ à C₁₂)-(aryle en C₆ à C₁₄) facultativement substitué, (vi) un groupe (alkyle en C₁ à C₁₂)-(cycloalkyle en C₃ à C₁₄) facultativement substitué, dans lesquels les substituants sont choisis entre des groupes (a) halogéno (F, Cl, Br, I), (b) nitro, (c) hydroxy, (d) carboxy, (e) tétrazole, (f) hydroxamate, (g) sulfonamido, (h) trifluorimide, (i) phosphonate, (j) alkyle en C₁ à C₆, (k) aryle en C₆ à C₁₄, (l) benzyle, (m) cycloalkyle en C₃ à C₁₄, (n) COR⁴ dans lequel R⁴ est choisi entre des groupes alkoxy en C₁ à C₈, alcénoxy en C₃ à C₁₂, aryloxy en C₆ à C₁₂, di-(alkyle en C₁ à C₈)-amino-(alkoxy en C₁ à C₈), acylamino-(alkoxy en C₁ à C₈) choisi entre les groupes acétylaminoéthoxy, nicotinoylaminoéthoxy, succinamidoéthoxy et pivaloyloxyéthoxy, et (aryle en C₆ à C₁₂)-(alkoxy en C₁ à C₈) dans lequel le groupe aryle est non substitué ou substitué avec un à trois des groupes nitro, halogéno (F, Cl, Br, I), alkoxy en C₁ à C₄, amino, hydroxy, hydroxyalkoxy en C₂ à C₈ et dihydroxyalkoxy en C₃ à C₈, (o) CONR⁵R⁶ dans lequel R⁵ et R⁶ sont choisis, indépendamment, entre l'hydrogène et des groupes alkyle en C₁ à C₁₀, alcényle en C₃ à C₁₀, aryle en C₆ à C₁₄, (alkyle en C₁ à C₆)-(aryle en C₆ à C₁₀), facultativement R⁵ et R⁶, pris conjointement, peuvent former un groupe triméthylène, tétraméthylène, pentaméthylène ou 3-oxopentaméthylène, et (vii) un groupe Q-L³ dans lequel Q est choisi entre l'hydrogène et Q¹, et L³ est choisi entre une liaison chimique et L¹, R¹⁵ liant L¹ à la position 4 ou 5 de la benzazépine-one ;
D est choisi entre le groupe consistant en R¹ et -(C=O)-Xaa, dans lequel Xaa représente un à trois D- ou L-α-amino-acides ;
R⁸ est choisi entre des groupes (i) hydroxy, (ii) alkoxy en C₁ à C₈, (iii) alcénoxy en C₃ à C₁₂, (iv) aryloxy en C₆ à C₁₂, (v) (alkyle en C₁ à C₆)-(aryle en C₆ à C₁₂) -oxy, (vi) di-(alkyle en C₁ à C₈)-amino-(alkoxy en C₁ à C₈), (vii) acylamino-(alkoxy en C₁ à C₈) choisi entre les groupes (a) acétylaminoéthoxy, (b) nicotinoylaminoéthoxy et (c) succinamidoéthoxy, (viii) (alcoyle en C₁ à C₈)-oxy-(alkoxy en C₁ à C₈), (ix) (aryle en C₆ à C₁₂)-(alkoxy en C₁ à C₈), dans lequel le groupe aryle est non substitué ou substitué avec un à trois des groupes (a) nitro, (b) halogéno (F, Cl, Br, I), (c) alkoxy en C₁ à C₄ et (d) amino, (x) hydroxy-alkoxy en C₂ à C₈, (xi) dihydroxy-alkoxy en C₃ à C₈, et (xii) NR⁹R³⁰ dans lequel R⁹ et R³⁰ sont choisis, indépendamment, dans le groupe consistant en (a) l'hydrogène, (b) un groupe alkyle en C₁ à C₈, (c) un groupe alcényle en C₃ à C₈, (d) un groupe aryle en C₆ à C₁₂, dans lequel le groupe aryle étant non substitué ou substitué avec un à trois des groupes nitro, halogéno (F, Cl, Br, I), alkoxy en C₁ à C₄, et amino, et (e) un groupe (aryle en C₆ à C₁₂)-(alkyle en C₁ à C₈) dans lequel le groupe aryle est non substitué ou substitué avec un à trois des groupes nitro, halogéno (F, Cl, Br, I) et alkoxy en C₁ à C₄ ;
ses sels pharmaceutiquement acceptables.

10. Composition pharmaceutique comprenant un excipient pharmaceutiquement acceptable et le composé suivant les revendications 1 à 9.

11. Composé suivant l'une quelconque des revendications 1 à 9, destiné à être utilisé dans une méthode de traitement.

12. Utilisation d'un composé suivant l'une quelconque des revendications 1 à 9 dans la préparation d'un médicament destiné à inhiber l'agrégation plaquettaire.

13. Utilisation d'un composé suivant l'une quelconque des revendications 1 à 9 dans la préparation d'un médicament destiné à réduire l'agrégation plaquettaire.

14. Utilisation d'un composé suivant l'une quelconque des revendications 1 à 9 dans la préparation d'un médicament destiné au traitement d'un mammifère présentant une tendance accrue à la formation de thrombus.

15. Utilisation suivant la revendication 14, dans laquelle le médicament est administré en association avec un agent thrombolytique.

16. Utilisation suivant la revendication 14, dans laquelle le médicament est administré en association avec un anticoagulant.
